(19) Europäisches Patentamt
European Patent Office
Office européen des brevets

(11) EP 4 438 054 A2

# (12) EUROPEAN PATENT APPLICATION

(43) Date of publication:
**02.10.2024 Bulletin 2024/40**

(21) Application number: **24173899.6**

(22) Date of filing: **16.04.2020**

(51) International Patent Classification (IPC):
***A61K 38/13*** *(2006.01)*

(52) Cooperative Patent Classification (CPC):
(C-Sets available)
**A61K 38/1774; A61K 38/13; A61P 29/00; A61P 31/12; A61P 37/06** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**17.04.2019 US 201962835488 P**
**31.05.2019 US 201962855830 P**
**05.11.2019 US 201962931212 P**
**06.12.2019 US 201962945071 P**
**17.01.2020 US 202062962832 P**
**10.03.2020 US 202062987854 P**

(62) Document number(s) of the earlier application(s) in accordance with Art. 76 EPC:
**20724339.5 / 3 955 953**

(71) Applicant: **Alpine Immune Sciences, Inc.**
**Seattle, WA 98102 (US)**

(72) Inventors:
- **SWANSON, Ryan**
  **Seattle, 98102 (US)**
- **YANG, Jing**
  **Seattle, 98102 (US)**
- **PENG, Stanford**
  **Seattle, 98102 (US)**
- **HILLSON, Jan**
  **Seattle, 98102 (US)**

(74) Representative: **Carpmaels & Ransford LLP**
**One Southampton Row**
**London WC1B 5HA (GB)**

Remarks:
- The complete document including Reference Table(s) and the Sequence Listing(s) can be downloaded from the EPO website
- This application was filed on 02-05-2024 as a divisional application to the application mentioned under INID code 62.
- Claims filed after the date of receipt of the application (Rule 68(4) EPC).

## (54) METHODS AND USES OF VARIANT ICOS LIGAND (ICOSL) FUSION PROTEINS

(57) Provided herein are immunomodulatory proteins comprising ICOSL variants and nucleic acids encoding such proteins. The immunomodulatory proteins provide therapeutic utility for a variety of immunological and oncological conditions. Compositions and methods for making and using such proteins are provided.

**FIG. 1A**

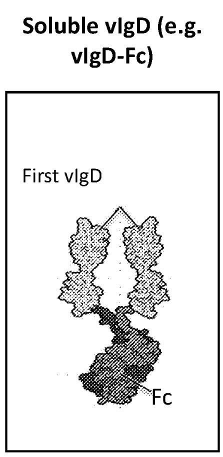




Processed by Luminess, 75001 PARIS (FR)

(Cont. next page)

(52) Cooperative Patent Classification (CPC): (Cont.)

C-Sets
**A61K 38/13, A61K 2300/00;**
**A61K 38/1774, A61K 2300/00**

## Description

### Cross-Reference to Related Applications

This application claims priority from U.S. Provisional Application No. 62/835,488, filed April 17, 2019, U.S. Provisional Application No. 62/855,830, filed May 31, 2019, U.S. Provisional Application No. 62/931,212, filed November 5, 2019, U.S. Provisional Application No. 62/945,071, filed December 6, 2019, U.S. Provisional Application No. 62/962,832, filed January 17, 2020, and U.S. Provisional Application No. 62/987,854, filed March 10, 2020, the contents of each of which are hereby incorporated by reference in their entirety.

### Incorporation by Reference of Sequence Listing

The present application is being filed along with a Sequence Listing in electronic format. The Sequence Listing is provided as a file entitled 761612003240SeqList.txt, created April 16, 2020, which is 116,543 bytes in size. The information in the electronic format of the Sequence Listing is incorporated by reference in its entirety.

### Field

The present disclosure relates to therapeutic compositions for modulating immune response in the treatment of cancer and immunological diseases. In some aspects, the present disclosure relates to particular variants of ICOS Ligand (ICOSL) that exhibit improved binding, such as improved affinity or selectivity for one or both of the cognate binding partner proteins ICOS or CD28.

### Background

Modulation of the immune response by intervening in the processes that occur in the immunological synapse (IS) formed by and between antigen-presenting cells (APCs) or target cells and lymphocytes is of increasing medical interest. Mechanistically, cell surface proteins in the IS can involve the coordinated and often simultaneous interaction of multiple protein targets with a single protein to which they bind. IS interactions occur in close association with the junction of two cells, and a single protein in this structure can interact with both a protein on the same cell (cis) as well as a protein on the associated cell (trans), likely at the same time. Although therapeutics are known that can modulate the IS, improved therapeutics are needed. Provided are immunomodulatory proteins, including soluble proteins or transmembrane immunomodulatory proteins capable of being expressed on cells, that meet such needs.

### Summary

In some of any embodiments, provided herein is a method of treating an autoimmune or inflammatory disease in a subject, the method comprising administering to a subject having an autoimmune or inflammatory disease a variant ICOSL fusion protein, said variant ICOSL fusion protein comprising a variant ICOSL polypeptide linked to a multimerization domain. In some of any embodiments, provided herein is a method of treating an autoimmune or inflammatory disease in a subject, the method comprising administering to a subject having an autoimmune or inflammatory disease a variant ICOSL fusion protein in a treatment period, said variant ICOSL fusion protein comprising a variant ICOSL polypeptide linked to a multimerization domain. In some of any embodiments, the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO: 1.

In some of any embodiments, each dose of the variant ICOSL fusion protein is administered in an amount from at or about 0.001 mg/kg to at or about 20 mg/kg. In some of any embodiments, each dose of the variant ICOSL fusion protein is administered in an amount from at or about 0.001 mg/kg to at or about 10 mg/kg. In some of any embodiments, each dose of the variant ICOSL fusion protein is administered in an amount from at or about 0.001 mg/kg to at or about 6 mg/kg. In some of any embodiments, each dose of the variant ICOSL fusion protein is administered in an amount from at or about 0.001 mg/kg to at or about 3 mg/kg. In some of any embodiments, each dose of the variant ICOSL fusion protein is administered in an amount from at or about 0.001 mg/kg to at or about 1 mg/kg. In some of any embodiments, each dose of the variant ICOSL fusion protein is administered in an amount from at or about 0.001 mg/kg to at or about 0.3 mg/kg. In some of any embodiments, each dose of the variant ICOSL fusion protein is administered in an amount from at or about 0.001 mg/kg to at or about 0.1 mg/kg. In some of any embodiments, each dose of the variant ICOSL fusion protein is administered in an amount from at or about 0.1 mg/kg to at or about 20 mg/kg. In some of any embodiments, each dose of the variant ICOSL fusion protein is administered in an amount from at or about 0.1 mg/kg to at or about 15 mg/kg. In some of any embodiments, each dose is administered in an amount from or from about 0.1 mg/kg to at or about 10 mg/kg. In some of any embodiments, each dose is administered in an amount from or from about 0.1 mg/kg to at or about 6 mg/kg. In some of any embodiments, each dose is administered in an amount from or from about 0.1 mg/kg to at or about 3 mg/kg. In some of any embodiments, each dose is administered in an amount from or from about 0.1 mg/kg to at or about 1 mg/kg. In some of any embodiments, each dose is administered in an amount from or from about 0.1 mg/kg to at or about 0.3 mg/kg. In some of any embodiments, each dose is administered in an amount from or from about 0.3 mg/kg to at or about 20 mg/kg. In some of any embodiments, each dose is administered in an amount from or from about 0.3 mg/kg to at or about 15 mg/kg. In some of any embodiments, each dose is administered in an amount from or from about 0.3 mg/kg to at or about 10 mg/kg. In some of any embodiments, each dose is administered in an amount from or from about 0.3 mg/kg to at or about 6 mg/kg. In some of any embodiments, each dose is administered in an amount from or from about 0.3 mg/kg to at or about 3 mg/kg. In some of any embodiments, each dose is administered in an amount from or from about 0.3 mg/kg to at or about 1 mg/kg. In some of any embodiments, each dose is administered in an amount from or from about 1 mg/kg to at or about 20 mg/kg. In some of any embodiments, each dose is administered in an amount from or from about 1 mg/kg to at or about 15 mg/kg. In some of any embodiments, each dose is administered in an amount from or from about 1 mg/kg to at or about 10 mg/kg. In some of any embodiments, each dose is administered in an amount from or from about 1 mg/kg to at or about 6 mg/kg. In some of any embodiments, each dose is administered in an amount from or from about 1 mg/kg to at or about 3 mg/kg. In some of any embodiments, each dose is administered in an amount from or from about 3 mg/kg to at or about 20 mg/kg. In some of any embodiments, each dose is administered in an amount from or from about 3 mg/kg to at or about 15 mg/kg. In some of any embodiments, each dose is administered in an amount from or from about 3 mg/kg to at or about 10 mg/kg. In some of any embodiments, each dose is administered in an amount from or from about 3 mg/kg to at or about 6 mg/kg. In some of any embodiments, each dose is administered in an amount from or from about 6 mg/kg to at or about 20 mg/kg. In some of any embodiments, each dose is administered in an amount from or from about 6 mg/kg to at or about 15 mg/kg. In some of any embodiments, each dose is administered in an amount from or from about 6 mg/kg to at or about 10 mg/kg. In some of any embodiments, each dose is administered in an amount from or from about 10 mg/kg to at or about 20 mg/kg. In some of any embodiments, each dose is administered in an amount from or from about 10 mg/kg to at or about 15 mg/kg.

In some of any embodiments, only a single dose of the variant ICOSL fusion protein is administered in a treatment period. In some of any embodiments, a multiple number of doses of the variant ICOSL fusion protein is administered in a treatment period. In some of any embodiments, the multiple number of doses is 2, 3, 4 or 5 doses. In some of any embodiments, the treatment period is at least 20 days. In some of any embodiments, the treatment period is 20-40 days.

In some of any embodiments, provided herein is a method of treating an autoimmune or inflammatory disease in a subject, the method comprising administering to a subject having an autoimmune or inflammatory disease a variant ICOSL fusion protein, said variant ICOSL fusion protein comprising a variant ICOSL polypeptide linked to a multimerization domain. In some of any embodiments, the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1. In some of any embodiments, the variant ICOSL fusion protein is administered in a multiple number of doses selected from 3, 4 and 5 for a treatment period of 20-40 days. In some of any embodiments, each dose is an amount from at or about 0.1 mg/kg to at or about 10 mg/kg.

In some of any embodiments, provided herein is a method of treating an autoimmune or inflammatory disease or condition in a subject, the method comprising administering to a subject having an autoimmune or inflammatory disease a variant ICOSL fusion protein, said variant ICOSL fusion protein comprising a variant ICOSL polypeptide linked to a multimerization domain. In some of any embodiments, the variant ICOSL polypeptide is an ICOSL extracellular domain or a portion thereof comprising an IgV domain or a specific binding fragment thereof and comprises one or more amino acid substitutions in a reference polypeptide corresponding to amino acid substitutions selected from N52H, N57Y and Q100R with reference to the sequence set forth in SEQ ID NO:1. In some of any embodiments, the variant ICOSL fusion protein is administered in a multiple number of doses for a treatment period of at least 20 days. In some of any embodiments, each dose is an amount from at or about 0.1 mg/kg to at or about 10 mg/kg. In some of any embodiments, each dose is for a maximum treatment period of 30 days. In some of any embodiments, each dose is at least 5 days separate each of the multiple doses from one another. In some of any embodiments, the treatment period is for at or about 21 days. In some of any embodiments, the treatment period is for at or about 28 days. In some of any embodiments, each of the multiple number of doses is administered no more than once weekly.

In some of any embodiments, provided herein is a method of treating an autoimmune or inflammatory disease in a subject, the method comprising administering to a subject having an autoimmune or inflammatory disease a variant ICOSL fusion protein, said variant ICOSL fusion protein comprising a variant ICOSL polypeptide linked to a multimerization domain. In some of any embodiments, the variant ICOSL polypeptide is an ICOSL extracellular domain or a portion thereof comprising an IgV domain or a specific binding fragment thereof and comprises one or more amino acid substitutions in a reference polypeptide corresponding to amino acid substitutions selected from N52H, N57Y and Q100R with reference to the sequence set forth in SEQ ID NO:1. In some of any embodiments, the variant ICOSL fusion protein is administered in a multiple number of doses for a treatment period of at least 4 weeks. In some of any embodiments, each dose is an amount from at or about 0.1 mg/kg to at or about 10 mg/kg and is administered no more than once weekly.

In some of any embodiments, provided herein is a method of preventing or reducing acute graft versus host disease (aGVHD), the method comprising administering to the subject one or more doses of a variant ICOSL fusion protein in a treatment period, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1, wherein the dose of the variant ICOSL fusion protein is administered in an amount from at or about 0.001 mg/kg to at or about 20 mg/kg. In some of any embodiments, the aGVHD is associated with a stem cell transplantation and at least one dose of the ICOSL fusion protein is administered to the subject prior to the subject receiving the stem cell transplantation. In some of any embodiments, the aGVHD is associated with a stem cell transplantation and at least one dose of the ICOSL fusion protein is administered to the subject prior to the subject receiving the stem cell transplantation and at least one dose of the ICOSL fusion protein is given to the subject concurrent with or subsequent to the subject receiving the stem cell transplantation. In some of any embodiments, the aGVHD is associated with stem cell transplantation and at least one dose of the ICOSL fusion protein is administered to the subject prior to the subject receiving the stem cell transplantation and at least one dose of the ICOSL fusion protein is given to the subject concurrent with the subject receiving the stem cell transplantation. In some of any embodiments, the aGVHD is associated with a stem cell transplantation and at least one dose of the ICOSL fusion protein is administered to the subject prior to the subject receiving the stem cell transplantation and at least one dose of the ICOSL fusion protein is given to the subject subsequent to the subject receiving the stem cell transplantation.

In some of any provided embodiments, provided herein is a method of treating graft versus host disease (GVHD) in a subject, the method comprising administering to the subject a single dose of a variant ICOSL fusion protein, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1, wherein the single dose of the variant ICOSL fusion protein is administered in an amount from at or about 0.001 mg/kg to at or about 20 mg/kg. In some of any of the provided embodiments, the GVHD is an acute GVHD (aGVHD). In some of any of the provided embodiments, the GVHD is a chronic GVHD.

In some of any provided embodiments, provided herein is a method of treating graft versus host disease (GVHD) in a subject, the method comprising administering to the subject a single dose of a variant ICOSL fusion protein, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1, wherein the single dose of the variant ICOSL fusion protein is administered in an amount from at or about 0.001 mg/kg to at or about 20 mg/kg, and wherein the subject is resistant or refractory to an immunosuppressant. In some embodiments, the immunosuppressant is a corticosteroid, such as a glucocorticoid. In some embodiments, the immunosuppressant is cyclosporine. In some of any of the provided embodiments, the GVHD is an acute GVHD (aGVHD). In some of any of the provided embodiments, the GVHD is a chronic GVHD.

In some of any embodiments, the treatment period is for at or about 4 weeks. In some of any embodiments, each of the multiple number of doses is administered once a week (Q1W). In some of any embodiments, each dose is an amount from at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 6 mg/kg, at or about 1 mg/kg to at or about 3 mg/kg, at or about 3 mg/kg to at or about 6 mg/kg. In some of any embodiments, dose is an amount from at or about 0.3 mg/kg to at or about 10 mg/kg, at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 10 mg/kg, at or about 1 mg/kg to at or about 6 mg/kg, at or about 1 mg/kg to at or about 3 mg/kg, at or about 3 mg/kg to at or about 10mg/kg, at or about 3 mg/kg to at or about 6 mg/kg, or at or about 6 mg/kg to at or about 10 mg/kg. In some of any embodiments, each dose is in an amount of or about 0.3 mg/kg. In some of any embodiments, each dose is in an amount of or about 1 mg/kg. In some of any embodiments, each dose is in an amount of or about 3 mg/kg. In some of any embodiments, each dose is in an amount of or about 6 mg/kg. In some of any embodiments, each dose is in an amount of or about 10 mg/kg. In some of any embodiments, at least one dose is in an amount of or about 0.3 mg/kg. In some of any embodiments, at least one dose is in an amount of or about 1 mg/kg. In some of any embodiments, at least one dose is in an amount of or about 3 mg/kg. In some of any embodiments, at least one dose is in an amount of or about 6 mg/kg. In some of any embodiments, at least one dose is in an amount of or about 10 mg/kg.

In some of any embodiments, the administration is via subcutaneous administration. In some of any embodiments, the administration is via intravenous administration. In some of any embodiments, at least one dose is administered via subcutaneous administration. In some of any embodiments, at least one dose is administerd via intravenous administration. In some of any embodiments, the treatment period is repeated. In some of any embodiments, the treatment period is repeated until remission is achieved, until partial remission is achieved or until the disease or condition does not progress in the subject.

Provided herein are methods of treating an ocular autoimmune or inflammatory disease in a subject, the method including administering intravitreally a dose of a variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and includes one or more amino acid substitutions in a reference ICOSL polypeptide with reference to the sequence set forth in SEQ ID NO:1. Provided herein are methods of reducing or preventing an ocular autoimmune or inflammatory disease in a subject, the method including administering intravitreally a dose of a variant ICOSL fusion protein including a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide with reference to the sequence set forth in SEQ ID NO:1. In some embodiments, the variant ICOSL fusion protein can include any as described herein, such as a variant ICOSL polypeptide with one or more amino acid modifications (e.g. one or more amino acid substitutions) that increase binding to CD28 and/or ICOS, and that is fused to a multimerization domain such as an Fc domain. In some embodiments, the one more more amino acid modifications are substutions N52H/N57Y/Q100R.

In some of any embodiments, the variant ICOSL polypeptide comprises the amino acid substitutions N52H/N57Y/Q100R, with reference to numbering of SEQ ID NO:1. In some of any embodiments, the variant ICOSL polypeptide exhibits increased binding affinity to the ectodomain(s) of CD28 compared to the binding of the ICOSL reference polypeptide for the same ectodomain(s). In some of any embodiments, the variant ICOSL polypeptide exhibits binding affinity to the ectodomain(s) of ICOS that is substantially the same or that is increased compared to the binding of the ICOSL reference polypeptide for the same ectodomain(s). In some of any embodiments, the binding affinity is from at or about 80% or greater of the binding affinity of the ICOSL reference polypeptide for the same ectodomain(s). In some of any embodiments, the variant ICOSL polypeptide exhibits increased binding to the ectodomain(s) of ICOS and CD28 compared to the binding of the ICOSL reference polypeptide for the same ectodomain(s). In some of any embodiments, the binding is increased more than at or about 1.2-fold, 1.5-fold, 2-fold, 3-fold, 4-fold, 5-fold, 6-fold, 7-fold, 8-fold, 9-fold, 10-fold, 20-fold, 30-fold, 40-fold, 50-fold or 60-fold.

In some of any embodiments, each dose of the variant ICOSL fusion protein achieves, or is predicted to achieve, a target binding saturation for CD28 of greater than at or about 25% at $C_{max}$; and/or each dose of the variant ICOSL fusion protein achieves, or is predicted to achieve, a target binding saturation for CD28 of greater than at or about 10% within 24 hours of the administration of the dose. In some embodiments, each dose of the variant ICOSL fusion protein achieves, or is predicted to achieve, a target binding saturation for CD28 of greater than at or about 85% within 24 hours, within 2 days or within 7 days of the administration of the dose. In some embodiments, each dose of the variant ICOSL fusion protein achieves, or is predicted to achieve, a target binding saturation for CD28 of greater than at or about 90% within 24 hours, within 2 days or within 7 days of the administration of the dose. In some embodiments, each dose of the variant ICOSL fusion protein achieves, or is predicted to achieve, a target binding saturation for CD28 of greater than at or about 95% within 24 hours, within 2 days or within 7 days of the administration of the dose. In some embodiments, each dose of the variant ICOSL fusion protein achieves, or is predicted to achieve, a target binding saturation for CD28 of greater than at or about 10% within 21 days or 28 days of the administration of the dose. In some embodiments, each dose of the variant ICOSL fusion protein achieves, or is predicted to achieve, a target binding saturation for CD28 of greater than at or about 20% within 21 days or 28 days of the administration of the dose.

In some of any embodiments, the CD28 is a human CD28. In some of any embodiments, the ICOS is a human ICOS. In some of any embodiments, the ICOSL reference polypeptide comprises the sequence of amino acids set forth in SEQ ID NO:1, a sequence of amino acids that has at least 95% sequence identity to SEQ ID NO:1; or a portion of the sequence of amino acids set forth in SEQ ID NO:1 or a sequence of amino acids that has at least 95% sequence identity to SEQ ID NO:1 comprising an IgV domain or IgC domain or specific binding fragments thereof or both. In some of any embodiments, the variant ICOSL polypeptide comprises the IgV domain or a specific binding fragment thereof. In some of any embodiments, the IgV domain or specific binding fragment thereof is the only ICOSL portion of the variant ICOSL polypeptide or of the variant ICOSL fusion protein. In some of any embodiments, the ICOSL reference polypeptide is a truncated ICOSL extracellular domain comprising a contiguous sequence of amino acids comprising amino acids 1-112 and a C-terminal truncation of at least 25 amino acids with reference to the ICOSL extracellular domain sequence set forth in SEQ ID NO:1. In some of any embodiments, the C-terminal truncation is of at least 30, at least 40, at least 50, at least 60, at least 70, at least 80, at least 90, at least 100, at least 125 amino acid residues.

In some of any embodiments, the ICOSL reference polypeptide is altered in or lacks a protease cleavage site set forth as amino acids 204-209 of SEQ ID NO:1. In some of any embodiments, the ICOSL reference polypeptide comprises the sequence of amino acids set forth in SEQ ID NO:3. In some of any embodiments, the ICOSL reference polypeptide consists of the sequence of amino acids set forth in SEQ ID NO:3. In some of any embodiments, the variant ICOSL polypeptide has the sequence set forth in SEQ ID NO:36 or a sequence that exhibits at least at or about 90%, at least at or about 91%, at least at or about 92%, at least at or about 93%, at least at or about 94%, at least at or about 95%, at least at or about 96%, at least at or about 97%, at least at or about 98%, or at least at or about 99% sequence identity to the sequence set forth in SEQ ID NO:36 and comprises the amino acid substitutions selected from N52H, N57Y and Q100R.

In some of any embodiments, the variant ICOSL polypeptide has the sequence set forth in SEQ ID NO:36. In some of any embodiments, the variant ICOSL polypeptide exhibits reduced proteolytic cleavage when expressed from a cell compared to a full-length extracellular domain of the variant ICOSL polypeptide when expressed from the same cell. In some of any embodiments, the cell is a Chinese Hamster Ovary (CHO) cell.

In some of any embodiments, the multimerization domain is or comprises an Fc region of an immunoglobulin. In some of any embodiments, the variant ICOSL polypeptide is linked via a linker to the multimerization domain. In some of any embodiments, the linker is a peptide linker. In some of any embodiments, the linker comprises 1 to 10 amino acids. In some of any embodiments, the linker is AAA. In some of any embodiments, the linker is G4S (SEQ ID NO:52). In some of any embodiments, the linker is (G4S) 2 (SEQ ID NO:53). In some of any embodiments, the linker is GSGGGGS linker (SEQ ID NO: 58).

In some of any embodiments, the variant ICOSL fusion protein is a multimer comprising a first variant ICOSL polypeptide linked to a first multimerization domain and a second variant ICOSL polypeptide linked to a second multimerization domain. In some of any embodiments, the first and second multimerization domain is the same. In some of any embodiments, the first and second multimerization domain is an Fc region of an immunoglobulin. In some of any embodiments, the multimer is a dimer. In some of any embodiments, the dimer is a homodimer.

In some of any embodiments, the Fc region is a variant Fc region that exhibits one or more reduced effector function compared to an Fc of a wildtype human immunoglobulin. In some of any embodiments, the Fc region is a variant IgG1 Fc region comprising one or more amino acid substitutions compared to the wildtype human IgG1. In some of any embodiments, the Fc region comprises a sequence that exhibits at least 90%, 91%, 92%, 93%, 94%, 95%, 96%, 97%, 98% or 99% sequence identity to SEQ ID NO:37. In some of any embodiments, the variant Fc region comprises one or more amino acid substitutions selected from N297G, E233P/L234V/L235A/G236del/S267K or L234A/L235E/G237A, wherein the residue is numbered according to the EU index of Kabat. In some of any embodiments, the variant Fc region further comprises the amino acid substitution C220S, wherein the residues are numbered according to the EU index of Kabat. In some of any embodiments, the Fc region comprises K447del, wherein the residue is numbered according to the EU index of Kabat. In some of any embdoiments, the Fc region comprises the sequence of amino acids set forth in SEQ ID NO:43 or SEQ ID NO:46. In some of any embodiments, the Fc region comprises the sequence of amino acids set forth in SEQ ID NO:44 or SEQ ID NO:47. In some of any embodiments, the Fc region comprises the sequence of amino acids set forth in SEQ ID NO:40. In some of any embodiments, the Fc region comprises the sequence of amino acids set forth in SEQ ID NO:39. In some of any embodiments, the Fc region comprises the sequence of amino acids set forth in SEQ ID NO:41. In some of any embodiments, the Fc region comprises the sequence of amino acids set forth in SEQ ID NO:42.

In some of any embodiments, the variant ICOSL fusion protein decreases an immune response in the subject. In some of any embodiments, the inflammatory or autoimmune disease or condition is an Antineutrophil cytoplasmic antibodies (ANCA)-associated vasculitis, a vasculitis, an autoimmune skin disease, transplantation, a Rheumatic disease, an inflammatory gastrointestinal disease, an inflammatory eye disease, an inflammatory neurological disease, an inflammatory pulmonary disease, an inflammatory endocrine disease, or an autoimmune hematological disease. In some of any embodiments, the inflammatory or autoimmune disease or condition disease or condition is an inflammatory bowel disease, transplant, Crohn’s disease, ulcerative colitis, multiple sclerosis, asthma, rheumatoid arthritis, psoriatic arthritis or psoriasis. In some embodiments, the inflammatory or autoimmune disease or condition is psoriatic arthritis. In some embodiments, the inflammatory or autoimmune disease or condition is rheumatoid arthritis. In some embodiments, the inflammatory or autoimmune disease or condition is Crohn’s disease. In some embodiments, the inflammatory or autoimmune disease is ulcerative colitis. In some embodiments, the inflammatory disease or autoimmune disease is uveitis. In some embodiments, the inflammatory or autoimmune disease or condition is systemic lupus erythematosus (SLE). In some embodiments, the inflammatory or autoimmune disease or condition is Sjögren’s Syndrome. In some embodiments, the inflammatory or autoimmune disease or condition is Graft Verse Host Disease (GVHD). In some embodiments, the GVHD is an acute GVHD. In some embodiments, the GVHD is a chronic GVHD. In some embodiments, the GVHD is resistant or refractory. In some embodiments, the GVHD is a chronic GVHD. In some embodiments, the GVHD is resistant or refractory to an immunosuppressant. In some embodiments, the immunosuppressant is a corticosteroid. In some embodiments, the corticosteroid is a glucocorticoid. In some embodiments, the immunosuppressant is cyclosporine.

In some of any of embodiments, the methods of treating comprises prophylactic treatment. In some of any embodiments, at least one dose is given prior to the onset of the inflammatory or autoimmune disease or condition. In some embodiments, at least one dose of the ICOSL fusion protein is administered concurrent with or subsequent to the onset of the inflammatory or autoimmune disease or condition. In some embodiments, at least one dose of the ICOSL fusion protein is administered prior to the onset of the inflammatory or autoimmune disease or condition and at least one dose of the ICOSL fusion protein is administered concurrent with or subsequent to the onset of the inflammatory or autoimmune disease or condition.

In some of any embodiments, the subject is a human.

The following are exemplary numbered embodiments of the present disclosure:

Embodiment 1. A method of preventing or reducing acute graft versus host disease (aGVHD), the method comprising administering to the subject one or more doses of a variant ICOSL fusion protein in a treatment period, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1, wherein each of the one or more doses of the variant ICOSL fusion protein is administered in an amount from at or about 0.1 mg/kg to at or about 20 mg/kg.

Embodiment 2. The method of Embodiment 1, wherein the subject has previously received an allogeneic hematopoietic stem cell transplant (HSCT) and the aGVHD occurs in the subject after receiving the allogeneic HSCT.

Embodiment 3. The method of Embodiment 1 or Embodiment 2, wherein the subject has Grade II-IV aGVHD.

Embodiment 4. The method of any one of Embodiments 1-3, wherein the aGVHD in the subject is resistant or refractory to treatment with an immunosuppressant.

Embodiment 5. The method of Embodiment 4, wherein the immunosuppressant comprises a corticosteroid.

Embodiment 6. The method of Embodiment 4 or Embodiment 5, wherein the immunosuppressant comprises a cyclosporine.

Embodiment 7. A method of preventing or reducing inflammation secondary to a viral infection, the method comprising administering to the subject one or more doses of a variant ICOSL fusion protein in a treatment period, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1, wherein each dose of the one or more doses of the variant ICOSL fusion protein is administered in an amount from at or about 0.1 mg/kg to at or about 20 mg/kg.

Embodiment 8. The method of Embodiment 7, wherein the virus is a coronavirus.

Embodiment 9. The method of Embodiment 8, wherein the coronavirus is SARS-CoV-2 and the infection is COVID-19.

Embodiment 10. The method of any of Embodiments 7-9, wherein the inflammation is associated with cytokine release syndrome (CRS).

Embodiment 11. The method of Embodiment 10, wherein the CRS is a severe CRS or a grade 3 or higher CRS.

Embodiment 12. The method of any of Embodiments 7-11, wherein at the time of or immediately prior to the administration the subject has severe pneumonia, acute respiratory distress syndrome (ARDS), sepsis or septic shock associated with or attributed to the viral infection.

Embodiment 13. A method of treating an autoimmune or inflammatory disease or condition in a subject, the method comprising administering to a subject having an autoimmune or inflammatory disease or condition one or more doses of a variant ICOSL fusion protein in a treatment period, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1, wherein each dose of the one or more doses of the variant ICOSL fusion protein is administered in an amount from at or about 0.001 mg/kg to at or about 20 mg/kg.

Embodiment 14. The method of any one of Embodiments 1-13, wherein each dose of the one or more doses is administered in an amount from or from about 0.1 mg/kg to at or about 10 mg/kg.

Embodiment 15. The method of Embodiment 14, wherein the autoimmune or inflammatory disease or condition is an acute condition.

Embodiment 16. The method of any one of Embodiments 1-15, wherein only a single dose of the variant ICOSL fusion protein is administered to the subject.

Embodiment 17. The method of Embodiment 14, wherein the autoimmune or inflammatory disease or condition is a chronic condition.

Embodiment 18. The method of Embodiment 13, Embodiment 14 or Embodiment 17, wherein the inflammatory or autoimmune disease or condition is systemic lupus erythematosus (SLE).

Embodiment 19. The method of Embodiment 13, Embodiment 14 or Embodiment 17, wherein the inflammatory or autoimmune disease or condition is Sjögren's Syndrome.

Embodiment 20. The method of Embodiment 13, Embodiment 14 or Embodiment 17, wherein the inflammatory or autoimmune disease or condition is psoriatic arthritis.

Embodiment 21. The method of Embodiment 13, Embodiment 14 or Embodiment 17, wherein the inflammatory or autoimmune disease or condition is rheumatoid arthritis.

Embodiment 22. The method of Embodiment 13, Embodiment 14 or Embodiment 17, wherein the inflammatory or autoimmune disease or condition is Crohn's disease.

Embodiment 23. The method of Embodiment 13, Embodiment 14 or Embodiment 17, wherein the inflammatory or autoimmune disease or condition is ulcerative colitis.

Embodiment 24. The method of any one of Embodiments 1-15 and 17-23, wherein a multiple number of doses of the variant ICOSL fusion protein is administered to the subject.

Embodiment 25. The method of Embodiment 24, wherein each of the multiple number of doses is administered no more than once weekly.

Embodiment 26. The method of Embodiment 24 or Embodiment 25, wherein each of the multiple number of doses is administered once a week (Q1W).

Embodiment 27. The method of Embodiment 24 or Embodiment 25, wherein each of the multiple number of doses is administered once every two weeks (Q2W).

Embodiment 28. The method of Embodiment 24 or Embodiment 25, wherein each of the multiple number of doses is administered once a month (Q4W).

Embodiment 29. The method of any one of Embodiments 1-28, wherein each dose of the one or more doses is administered in an amount from at or about 0.3 mg/kg to at or about 10 mg/kg, at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 10 mg/kg, at or about 1 mg/kg to at or about 6 mg/kg, at or about 1 mg/kg to at or about 3 mg/kg, at or about 3 mg/kg to at or about 10mg/kg, at or about 3 mg/kg to at or about 6 mg/kg, or at or about 6 mg/kg to at or about 10 mg/kg.

Embodiment 30. The method of any one of Embodiments 1-29, wherein each dose of the one or more doses is administered in an amount from at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 6 mg/kg, at or about 1 mg/kg to at or about 3 mg/kg, or at or about 3 mg/kg to at or about 6 mg/kg.

Embodiment 31. The method of any one of Embodiments 1-30, wherein each dose of the one or more doses is administered in an amount of or of about 0.3 mg/kg.

Embodiment 32. The method of any one of Embodiments 1-30, wherein each dose of the one or more doses is administered in an amount of or of about 1 mg/kg.

Embodiment 33. The method of any one of Embodiments 1-30, wherein each dose of the one or more doses is administered in an amount of or of about 3 mg/kg.

Embodiment 34. The method of any one of Embodiments 1-30, wherein each dose of the one or more doses is administered in an amount of or of about 6 mg/kg.

Embodiment 35. The method of any one of Embodiments 1-30, wherein each dose of the one or more doses is administered in an amount of or of about 10 mg/kg.

Embodiment 36. The method of any one of Embodiments 1-28, wherein each dose of the one or more doses is administered in an amount of or of about 15 mg/kg.

Embodiment 37. The method of any one of Embodiments 1-28, wherein each dose of the one or more doses is administered in an amount of or of about 20 mg/kg.

Embodiment 38. The method of any one of Embodiments 1-37, wherein the treatment period is repeated.

Embodiment 39. The method of any one of Embodiments 1-38, wherein the administration is via subcutaneous administration.

Embodiment 40. The method of any one of Embodiments 1-38, wherein the administration is via intravenous administration.

Embodiment 41. A method of treating an ocular autoimmune or inflammatory disease in a subject, the method comprising administering intravitreally a dose of a variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

Embodiment 42. The method of Embodiment 41, wherein the ocular autoimmune or inflammatory disease is uveitis.

Embodiment 43. The method of Embodiment 41 or Embodiment 42, wherein the variant ICOSL fusion protein is administered at a dose of between about 0.01 mg to 10 mg, at or about 0.05 mg to 10 mg, at or about 0.1 mg to 10 mg, at or about 0.5 mg to 10 mg, at or about 1 mg to 10 mg, at or about 1.5 mg to 10 mg, at or about 2 mg to 10 mg, at or about 3 mg to 10 mg, at or about 4 mg to 10 mg, at or about 5 mg to 10 mg, at or about 6 mg to 10 mg, at or about 7 mg to 10 mg, at or about 8 mg to 10 mg, at or about 9 mg to 10 mg, inclusive.

Embodiment 44. The method of any of Embodiments 41-43, wherein the variant ICOSL fusion protein is administered in a volume less than 0.2 mL, optionally less than 0.1 mL.

Embodiment 45. The method of any of Embodiments 41-44, wherein the variant ICOSL fusion protein is administered in a volume of at or about 0.05 mL.

Embodiment 46. The method of any one of Embodiments 1-45, wherein the ICOSL reference polypeptide comprises (i) the sequence of amino acids set forth in SEQ ID NO:32, (ii) a sequence of amino acids that has at least 95% sequence identity to SEQ ID NO:32; or (iii) a portion of (i) and/or (ii) comprising an IgV domain or an IgC domain or specific binding fragments thereof.

Embodiment 47. The method of any one of Embodiments 1-46, wherein the variant ICOSL polypeptide comprises the IgV domain or a specific binding fragment thereof.

Embodiment 48. The method of any one of Embodiments 1-47, wherein the IgV domain or specific binding fragment thereof is the only ICOSL portion of the variant ICOSL polypeptide or of the variant ICOSL fusion protein.

Embodiment 49. The method of any one of Embodiments 1-48, wherein the ICOSL reference polypeptide comprises the sequence of amino acids set forth in SEQ ID NO:3

Embodiment 50. The method of any one of Embodiments 1-49, wherein the ICOSL reference polypeptide consists of the sequence of amino acids set forth in SEQ ID NO:3

Embodiment 51. The method of any one of Embodiments 1-50, wherein the variant ICOSL polypeptide has the sequence set forth in SEQ ID NO:36 or a sequence that exhibits at least at or about 90%, at least at or about 91%, at least at or about 92%, at least at or about 93%, at least at or about 94%, at least at or about 95%, at least at or about 96%, at least at or about 97%, at least at or about 98%, or at least at or about 99% sequence identity to the sequence set forth in SEQ ID NO:36 and comprises the one or more amino acid substitutions selected from N52H, N57Y and Q100R.

Embodiment 52. The method of any one of Embodiments 1-51, wherein the variant ICOSL polypeptide has the sequence set forth in SEQ ID NO:36.

Embodiment 53. The method of any one of Embodiments 1-52, wherein the multimerization domain is or comprises an Fc region of an immunoglobulin.

Embodiment 54. The method of Embodiment 53, wherein the Fc region is a variant Fc region that exhibits reduced effector function compared to an Fc of a wildtype human immunoglobulin.

Embodiment 55. The method of Embodiment 53 or Embodiment 54, wherein the Fc region is a variant IgG1 Fc region comprising one or more amino acid substitutions compared to a wildtype human IgG1.

Embodiment 56. The method of Embodiment 54 or Embodiment 55, wherein the variant Fc region comprises one or more amino acid substitutions selected from N297G, E233P/L234V/L235A/G236del/S267K or L234A/L235E/G237A, wherein the residue is numbered according to the EU index of Kabat.

Embodiment 57. The method of any of Embodiments 54-56, wherein the variant Fc region further comprises the amino acid substitution C220S, wherein the residues are numbered according to the EU index of Kabat.

Embodiment 58. The method of any of Embodiments 53-57, wherein the Fc region comprises K447del, wherein the residue is numbered according to the EU index of Kabat.

Embodiment 59. A pharmaceutical composition comprising a variant ICOSL fusion protein for use in a method of preventing or reducing acute graft versus host disease (aGVHD) in a subject, wherein the variant ICOSL fusion protein is comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

Embodiment 60. Use of a variant ICOSL fusion protein in the formulation of a medicament for use in a method of preventing or reducing acute graft versus host disease (aGVHD), wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

Embodiment 61. The pharmaceutical composition for use of Embodiment 59 or the use of Embodiment 60, wherein the method comprises the steps of administering one or more doses of the variant ICOSL fusion protein to a subject in a treatment period, wherein each of the one or more doses of the variant ICOSL fusion protein is administered in an amount from at or about 0.1 mg/kg to at or about 20 mg/kg.

Embodiment 62. The pharmaceutical composition for use of Embodiment 59 or Embodiment 61 or use of Embodiment 60 or Embodiment 61, wherein the aGvHD is Grade II-IV aGVHD.

Embodiment 63. The pharmaceutical composition for use of any one of Embodiments 59, 61, and 62 or use of any one of Embodiments 60, 61, and 62, wherein the aGVHD is resistant or refractory to treatment with an immunosuppressant.

Embodiment 64. The pharmaceutical composition for use of any one of Embodiments 59 and 61-63, or use of any one of Embodiments 60 and 61-63, wherein the immunosuppressant comprises a corticosteroid.

Embodiment 65. The pharmaceutical composition for use of Embodiment 63 or Embodiment 64 or use of Embodiment 63 or Embodiment 64, wherein the immunosuppressant comprises a cyclosporine.

Embodiment 66. A pharmaceutical composition comprising a variant ICOSL fusion protein for use in a method of preventing or reducing inflammation secondary to a viral infection in a subject, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

Embodiment 67. Use of a variant ICOSL fusion protein in the formulation of a medicament for use in a method of preventing or reducing inflammation secondary to a viral infection, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1,.

Embodiment 68. The pharmaceutical composition for use of Embodiment 66 or use of Embodiment 67, wherein the method comprises the steps of administering one or more doses of the variant ICOSL fusion protein to a subject in a treatment period, wherein each of the one or more doses of the variant ICOSL fusion protein is administered in an amount from at or about 0.1 mg/kg to at or about 20 mg/kg.

Embodiment 69. The pharmaceutical composition for use of Embodiment 66 or Embodiment 68 or use of Embodiment 67 or Embodiment 68, wherein the virus is a coronavirus.

Embodiment 70. The pharmaceutical composition for use of Embodiment 69 or use of Embodiment 69, wherein the coronavirus is SARS-CoV-2 and the infection is COVID-19.

Embodiment 71. The pharmaceutical composition for use of any one of Embodiments 66, 68, and 69 or use of any one of Embodiments 67-69, wherein the inflammation is associated with cytokine release syndrome (CRS).

Embodiment 72. The pharmaceutical composition for use of Embodiment 71 or use of Embodiment 71, wherein the CRS is a severe CRS or a grade 3 or higher CRS.

Embodiment 73. The pharmaceutical composition for use of any one of Embodiments 68, and 69-72 or use of any one of Embodiments 68, and 69-72, wherein at the time of or immediately prior to the administration the subject has severe pneumonia, acute respiratory distress syndrome (ARDS), sepsis or septic shock associated with or attributed to the viral infection.

Embodiment 74. A pharmaceutical composition comprising a variant ICOSL fusion protein for use in treating an autoimmune or inflammatory disease or condition in a subject, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

Embodiment 75. Use of a variant ICOSL fusion protein in the formulation of a medicament for use in treating an autoimmune or inflammatory disease or condition, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

Embodiment 76. The pharmaceutical composition for use of Embodiment 74 or use of Embodiment 75, wherein the method comprises the steps of administering one or more doses of the variant ICOSL fusion protein to a subject in a treatment period, wherein each of the one or more doses of the variant ICOSL fusion protein is administered in an amount from at or about 0.001 mg/kg to at or about 20 mg/kg.

Embodiment 77. The pharmaceutical composition for use of any one of Embodiments 61-65, 68-73, and 76 or use of any one of Embodiments 61-65, 68-73, and 76, wherein each dose of the one or more doses is administered in an amount from or from about 0.1 mg/kg to at or about 10 mg/kg.

Embodiment 78. The pharmaceutical composition for use of Embodiment 77 or the use of Embodiment 77, wherein the autoimmune or inflammatory disease or condition is an acute condition.

Embodiment 79. The pharmaceutical composition for use of any one of Embodiments 61-65, 68-73, and 76-78 or use of any one of Embodiments 61-65, 68-73, and 76-78, wherein only a single dose of the variant ICOSL fusion protein is administered to the subject.

Embodiment 80. The pharmaceutical composition for use of Embodiment 77 or use of Embodiment 77, wherein the autoimmune or inflammatory disease or condition is a chronic disease or condition.

Embodiment 81. The pharmaceutical composition for use of any one of Embodiments 74, 77, and 80 or use of any one of Embodiments 75, 77 and 80, wherein the inflammatory or autoimmune disease or condition is systemic lupus erythematosus (SLE).

Embodiment 82. The pharmaceutical composition for use of any one of Embodiments 74, 77, and 80 or use of any one of Embodiments 75, 77 and 80, wherein the inflammatory or autoimmune disease or condition is Sjögren's Syndrome.

Embodiment 83. The pharmaceutical composition for use of any one of Embodiments 74, 77, and 80 or use of any one of Embodiments 75, 77 and 80, wherein the inflammatory or autoimmune disease or condition is psoriatic arthritis.

Embodiment 84. The pharmaceutical composition for use of any one of Embodiments 74, 77, and 80 or use of any one of Embodiments 75, 77 and 80, wherein the inflammatory or autoimmune disease or condition is rheumatoid arthritis.

Embodiment 85. The pharmaceutical composition for use of any one of Embodiments 74, 77, and 80 or use of any one of Embodiments 75, 77 and 80, wherein the inflammatory or autoimmune disease or condition is Crohn's disease.

Embodiment 86. The pharmaceutical composition for use of any one of Embodiments 74, 77, and 80 or use of any one of Embodiments 75, 77 and 80, wherein the inflammatory or autoimmune disease or condition is ulcerative colitis.

Embodiment 87. The pharmaceutical composition for use of any one of Embodiments 61-65, 68-73, and 76-78 or use of any one of Embodiments 61-65, 68-73, and 76-78, wherein a multiple number of doses of the variant ICOSL fusion protein is administered to the subject.

Embodiment 88. The pharmaceutical composition for use of Embodiment 87 or the use of Embodiment 87, wherein each of the multiple number of doses is administered no more than once weekly.

Embodiment 89. The pharmaceutical composition for use of Embodiment 87 or Embodiment 88 or the use of Embodiment 87 or Embodiment 88, wherein each of the multiple number of doses is administered once a week (Q1W).

Embodiment 90. The pharmaceutical composition for use of Embodiment 87 or Embodiment 88 or the use of Embodiment 87 or Embodiment 88, wherein each of the multiple number of doses is administered once every two weeks (Q2W).

Embodiment 91. The pharmaceutical composition for use of Embodiment 87 or Embodiment 88 or the use of Embodiment 87 or Embodiment 88, wherein each of the multiple number of doses is administered once a month (Q4W).

Embodiment 92. The pharmaceutical composition for use of any one of Embodiments 61-65, 68-73, 76-78, and 87-91 or use of any one of Embodiments 61-65, 68-73, 76-78, and 87-91, wherein each dose of the one or more doses is administered in an amount from at or about 0.3 mg/kg to at or about 10 mg/kg, at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 10 mg/kg, at or about 1 mg/kg to at or about 6 mg/kg, at or about 1 mg/kg to at or about 3 mg/kg, at or about 3 mg/kg to at or about 10mg/kg, at or about 3 mg/kg to at or about 6 mg/kg, or at or about 6 mg/kg to at or about 10 mg/kg.

Embodiment 93. The pharmaceutical composition for use of any one of Embodiments 61-65, 68-73, 76-78, and 87-92 or use of any one of Embodiments 61-65, 68-73, 76-78, and 87-92, wherein each dose of the one or more doses is administered in an amount from at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 6 mg/kg, at or about 1 mg/kg to at or about 3 mg/kg, or at or about 3 mg/kg to at or about 6 mg/kg.

Embodiment 94. The pharmaceutical composition for use of any one of Embodiments 61-65, 68-73, 76-78, and 87-93 or use of any one of Embodiments 61-65, 68-73, 76-78, and 87-93, wherein each dose of the one or more doses is administered in an amount of or of about 0.3 mg/kg.

Embodiment 95. The pharmaceutical composition for use of any one of Embodiments 61-65, 68-73, 76-78, and 87-93 or use of any one of Embodiments 61-65, 68-73, 76-78, and 87-93, wherein each dose of the one or more doses is administered in an amount of or of about 1 mg/kg.

Embodiment 96. The pharmaceutical composition for use of any one of Embodiments 61-65, 68-73, 76-78, and 87-93 or use of any one of Embodiments 61-65, 68-73, 76-78, and 87-93, wherein each dose of the one or more doses is administered in an amount of or of about 3 mg/kg.

Embodiment 97. The pharmaceutical composition for use of any one of 61-65, 68-73, 76-78, and 87-93 or use of any one of Embodiments 61-65, 68-73, 76-78, and 87-93, wherein each dose of the one or more doses is administered in an amount of or of about 6 mg/kg.

Embodiment 98. The pharmaceutical composition for use of any one of Embodiments 61-65, 68-73, 76-78, and 87-93 or use of any one of Embodiments 61-65, 68-73, 76-78, and 87-93, wherein each dose of the one or more doses is administered in an amount of or of about 10 mg/kg.

Embodiment 99. The pharmaceutical composition for use of any one of Embodiments 61-65, 68-73, 76-78, and 87-91 or use of any one of Embodiments 61-65, 68-73, 76-78, and 87-91, wherein each dose of the one or more doses is administered in an amount of or of about 15 mg/kg.

Embodiment 100. The pharmaceutical composition for use of any one of Embodiments 61-65, 68-73, 76-78, and 87-91 or use of any one of Embodiments 61-65, 68-73, 76-78, and 87-91, wherein each dose of the one or more doses is administered in an amount of or of about 20 mg/kg.

Embodiment 101. The pharmaceutical composition for use of any one of Embodiments 61-65, 68-73, 76-78, and 87-100 or use of any one of Embodiments 61-65, 68-73, 76-78, and 87-100, wherein the treatment period is repeated.

Embodiment 102. The pharmaceutical composition for use of any one of Embodiments 61-65, 68-73, 76-78, and 87-101 or use of any one of Embodiments 61-65, 68-73, 76-78, and 87-101, wherein the variant ICOSL fusion protein is administered subcutaneously.

Embodiment 103. The pharmaceutical composition for use of any one of Embodiments 61-65, 68-73, 76-78, and 87-101 or use of any one of Embodiments 61-65, 68-73, 76-78, and 87-101, wherein the variant ICOSL fusion protein is administered intravenously.

Embodiment 104. A pharmaceutical composition comprising a variant ICOSL fusion protein for use in a method of treating an ocular autoimmune or inflammatory disease in a subject, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

Embodiment 105. Use of a variant ICOSL fusion protein in the formulation of a medicament for use in a method of treating an ocular autoimmune or inflammatory disease in a subject, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

Embodiment 106. The pharmaceutical composition for use of Embodiment 104 or use of Embodiment 105, wherein the ocular autoimmune or inflammatory disease is uveitis.

Embodiment 107. The pharmaceutical composition for use of Embodiment 104 or Embodiment 106 or use Embodiment 105 or Embodiment 106, wherein the method comprises steps of administering one or more doses of the variant ICOSL fusion protein to a subject, wherein each of the one or more doses of the variant ICOSL fusion protein is administered at a dose of between about 0.01 mg to 10 mg, at or about 0.05 mg to 10 mg, at or about 0.1 mg to 10 mg, at or about 0.5 mg to 10 mg, at or about 1 mg to 10 mg, at or about 1.5 mg to 10 mg, at or about 2 mg to 10 mg, at or about 3 mg to 10 mg, at or about 4 mg to 10 mg, at or about 5 mg to 10 mg, at or about 6 mg to 10 mg, at or about 7 mg to 10 mg, at or about 8 mg to 10 mg, at or about 9 mg to 10 mg, inclusive.

Embodiment 108. The pharmaceutical composition for use of Embodiment 107 or use of Embodiment 107, wherein the variant ICOSL fusion protein is administered in a volume less than 0.2 mL, optionally less than 0.1 mL.

Embodiment 109. The pharmaceutical composition for use of Embodiment 107 or Embodiment 108 or use of Embodiment 107 or Embodiment 108, wherein the variant ICOSL fusion protein is administered in a volume of at or about 0.05 mL.

Embodiment 110. The pharmaceutical composition for use of any one of Embodiments 59, 61-65, 66, 68-73-, 74, 76-103, 104, and 106-109 or use of any one of Embodiments 60, 61-65, 67, 68-73, 75, 76-103, 105, and 106 107-109, wherein the ICOSL reference polypeptide comprises (i) the sequence of amino acids set forth in SEQ ID NO:32, (ii) a sequence of amino acids that has at least 95% sequence identity to SEQ ID NO:32; or (iii) a portion of (i) and/or (ii) comprising an IgV domain or an IgC domain or specific binding fragments thereof.

Embodiment 111. The pharmaceutical composition for use of any one of Embodiments 59, 61-65, 66, 68-73-, 74, 76-103, 104, and 106-110 or use of any one of Embodiments 60, 61-65, 67, 68-73, 75, 76-103, and 105-110, wherein the variant ICOSL polypeptide comprises the IgV domain or a specific binding fragment thereof.

Embodiment 112. The pharmaceutical composition for use of any one of Embodiments 59, 61-65, 66, 68-73-, 74, 76-103, 104, and 106-111 or use of any one of Embodiments 60, 61-65, 67, 68-73, 75, 76-103, and 105-111, wherein the IgV domain or specific binding fragment thereof is the only ICOSL portion of the variant ICOSL polypeptide or of the variant ICOSL fusion protein.

Embodiment 113. The pharmaceutical composition for use of any one of Embodiments 59, 61-65, 66, 68-73-, 74, 76-103, 104, and 106-112 or use of any one of Embodiments 60, 61-65, 67, 68-73, 75, 76-103, and 105-112, wherein the ICOSL reference polypeptide comprises the sequence of amino acids set forth in SEQ ID NO:3

Embodiment 114. The pharmaceutical composition for use of any one of Embodiments 59, 61-65, 66, 68-73-, 74, 76-103, 104, and 106-113 or use of any one of Embodiments 60, 61-65, 67, 68-73, 75, 76-103, and 105-113, wherein the ICOSL reference polypeptide consists of the sequence of amino acids set forth in SEQ ID NO:3

Embodiment 115. The pharmaceutical composition for use of any one of Embodiments 59, 61-65, 66, 68-73-, 74, 76-103, 104, and 106-114 or use of any one of Embodiments 60, 61-65, 67, 68-73, 75, 76-103, and 105-114, wherein the variant ICOSL polypeptide has the sequence set forth in SEQ ID NO:36 or a sequence that exhibits at least at or about 90%, at least at or about 91%, at least at or about 92%, at least at or about 93%, at least at or about 94%, at least at or about 95%, at least at or about 96%, at least at or about 97%, at least at or about 98%, or at least at or about 99% sequence identity to the sequence set forth in SEQ ID NO:36 and comprises the one or more amino acid substitutions selected from N52H, N57Y and Q 100R.

Embodiment 116. The pharmaceutical composition for use of any one of Embodiments 59, 61-65, 66, 68-73-, 74, 76-103, 104, and 106-115 or use of any one of Embodiments 60, 61-65, 67, 68-73, 75, 76-103, and 105-115, wherein the variant ICOSL polypeptide has the sequence set forth in SEQ ID NO:36.

Embodiment 117. The pharmaceutical composition for use of any one of Embodiments 59, 61-65, 66, 68-73-, 74, 76-103, 104, and 106-116 or use of any one of Embodiments 60, 61-65, 67, 68-73, 75, 76-103, and 105-116, wherein the multimerization domain is or comprises an Fc region of an immunoglobulin.

Embodiment 118. The pharmaceutical composition for use of Embodiment 117 or use of Embodiment 117, wherein the Fc region is a variant Fc region that exhibits reduced effector function compared to an Fc of a wildtype human immunoglobulin.

Embodiment 119. The pharmaceutical composition for use of Embodiment 117 or Embodiment 118 or use of Embodiment 117 or Embodiment 118, wherein the Fc region is a variant IgG1 Fc region comprising one or more amino acid substitutions compared to a wildtype human IgG1.

Embodiment 120. The pharmaceutical composition for use of Embodiment 118 or Embodiment 119 or use of Embodiment 118 or Embodiment 119, wherein the variant Fc region comprises one or more amino acid substitutions selected from N297G, E233P/L234V/L235A/G236del/S267K or L234A/L235E/G237A, wherein the residue is numbered according to the EU index of Kabat.

Embodiment 121. The pharmaceutical composition for use of any one of Embodiments 118-120 or use of any one of Embodiments 118-120, wherein the variant Fc region further comprises the amino acid substitution C220S, wherein the residues are numbered according to the EU index of Kabat.

Embodiment 122. The pharmaceutical composition for use of any one of Embodiments 117-121 or use of any one of Embodiments 117-121, wherein the Fc region comprises K447del, wherein the residue is numbered according to the EU index of Kabat.

## Brief Description Of The Drawings

**FIGS. 1A-1B** depict schematic representations of molecules. **FIG 1A** depicts a soluble molecule containing a variant IgSF domain (vIgD) fused to an Fc chain. **FIG. 1B** depicts molecules containing frequently observed mutations in the IgV domain of ICOSL.

**FIGS. 2A-2B** demonstrate, via cytokine release, the costimulatory capacity of wild-type (WT) or variant ICOSL when coimmobilized with anti-CD3. 10 nM anti-CD3 was wet coated to the wells of 96-well flat bottomed polystyrene tissue culture plates with 40 nM (arrows) or 10 nM WT or variant ICOSL. 100,000 purified $CD4^+$ and $CD8^+$ (pan) T-cells cells were added and supernatant was harvested 72 hours later for ELISA analysis for cytokine release. **FIG. 2A** shows IFN-gamma and **FIG. 2B** shows IL-17 protein levels secreted from pan T-cells. Graphs are representative of typical IFN-gamma and IL-17 responses from pan T-cell co stimulation.

**FIGS. 3A-3B** demonstrate, via proliferation, the costimulatory capacity of wild-type (WT) or variant ICOSL when coimmobilized with anti-CD3. CFSE-labeled pan T-cells were incubated in anti-CD3 and ICOSL coated plates as previously described for 72 hours. Cells were harvested, washed, stained with fluorescently conjugated anti-CD4 or anti-CD8 antibodies, and analyzed by flow cytometry. Gates and cytometer voltages were set using non-stimulated control CFSE-labeled T-cells. Proliferation was determined by CFSE dilution from control. **FIG. 3A** shows percent of total proliferating (arrows), $CD4^+$ (solid bar), and $CD8^+$ cells (hatched bar) T-cells following 40 nM ICOSL costimulation. **FIG. 3B** shows percent of total pan T-cell proliferation following 10 nM ICOSL costimulation. Graphs are representative of typical proliferative response from pan T-cell costimulation.

**FIG. 4** depicts ICOSL vIgD candidate function in a human Mixed-Lymphocyte-Reation (MLR). ICOSL variants and their mutations are listed on the x-axis, along with wild-type ICOSL, negative controls PDL2-Fc and human IgG, as well as the positive control benchmark molecule CTLA-Ig Belatacept. The line across the graph represents the baseline amount of IFN-gamma detected in the supernatants of negative control cultures. For each ICOSL variant candidate or control, three different concentrations were tested with arrows indicating the highest concentration of protein in cultures at 40nM. The majority of ICOSL variant candidates show superior antagonistic activity at all three concentrations tested compared to belatacept as reflected by the lower concentration of IFN-gamma in those cultures.

**FIGS. 5A-5D** depicts the inhibition of soluble ICOSL Fc-fusion proteins on B and T cell responses in a B-T co-culture assay. **FIG. 5A** depicts soluble ICOSL Fc-fusion proteins inhibition of T cell-driven B cell proliferation. Purified $CD4^+$ T cells and B cells from a single donor were CFSE-labeled and co-incubated at a 1:1 ration in the presence or absence of the indicated mitogens with or without the indicated ICOSL Fc-fusion proteins. Cells were stimulated with Staph enterotoxin B (SEB) at 100 ng/mL, Pokeweed mitogen (PWM) at 1 mg/mL, or both. ICOSL Fc-fusion proteins were included at a final concentration of 40 nM and cultures were incubated for 7 days and subjected to FACS analysis. The number of divided B cells was determined from the number of cells in the cultures that had diluted their CFSE. All of the ICOSL Fc-fusion proteins tested except for wild-type reduced B cell proliferation. **FIG. 5B-5D** show ICOSL Fc-fusion proteins inhibited cytokine T cell cytokine production in B-T cocultures. Supernatants from the cultures described above were harvested on day 7 and analyzed for cytokine content using a LEGENDplex Human Th Cytokine Panel (Biolegend). T cell production of IL-5 **(FIG. 5B),** IL-13 **(FIG. 5C)** and IL-21 **(FIG. 5D)** is attenuated by inclusion of ICOSL Fc-fusion proteins.

**FIGS. 6A-6F** depicts different endpoints in a mouse model of Graft Verse Host Disease (GVHD) where human PBMC cells were adoptively transferred into immunodeficient NSG murine hosts. **FIG. 6A** shows survival curves of the treated animals. Aggressive disease course and subsequent mortality was observed in the saline control animals, with similar survival observed in the animals treated with wild-type ICOSL-Fc, as well as the N52H/I143T ICOSL variant. Variant N52H/N57Y/Q100P had improved survival rates comparable to the clinical benchmark belatacept. **FIG. 6B** shows similar trends in body weight loss, with ICOSL variant N52H/N57Y/Q100P demonstrating similar weight maintenance as animals treated with belatacept, even though all other groups experienced rapid weight loss. **FIG. 6C** shows clinical scores from standardized GVHD Disease Activity Index (DAI) observations, again showing lower scores in animals treated with the ICOSL variant N52H/N57Y/Q100P that are comparable to the clinical benchmark belatacept while the other groups of animals experienced higher DAI scores. **FIG. 6D** depicts a flow cytometric measurement of CD4 and CD8 percentages in blood from experimental animals measured on day 14. The percentage of CD8 cells between experimental groups was largely the same, however, animals treated with ICOSL variant N52H/N57Y/Q100P and belatacept have lower percentages of CD4 cells compared to the other experimental groups.

**FIG. 6E** depicts survival curves from a similar experiment testing additional ICOSL variant molecules. **FIG. 6F** depicts clinical scores from a similar experiment testing additional ICOSL variant molecules.

**FIG. 7** summarizes changes in ear thickness in mice from a standard model of Delayed-Type Hypersensitivity (DTH). PBS treated animals sensitized with ovalbumin and subsequently challenged in the ear with the same protein, show the highest level of measured ear swelling. Mice treated with clinical benchmark Abatacept have slightly reduced ear swelling following ear challenge. All five ICOSL variant treatment groups demonstrated equal or improved reductions in ear swelling compared to Abatacept.

**FIG. 8** depicts the Nanostring transcriptional signature of primary human T cells when incubated 10 nM anti-CD3 with 40 nM of an Fc-control protein, wild-type ICOSL-Fc, wild-type CD80-Fc, both of these proteins, or a variant ICOSL Fc-fusion proteins with mutations as indicated. Total RNA from samples was prepared from harvested cells and the RNA was transferred to Nanostring and a Cancer Immune chip was used to quantitate transcripts of 750 gene in each sample. Altered transcripts include those whose level is above or below the diagonal line, including the noted transcripts.

**FIG. 9** depicts transcript levels of exemplary transcripts upon incubation as described in FIG. 8 for the indicated times in the presence of the various immunomodulatory proteins.

**FIGS. 10A-10G** shows SEC analysis of proteolysis in variant ICOSL Fc-fusion molecules containing mutations N52H/N57Y/Q100R/F172S generated in various reference sequences, such as truncated ICOSL ECD Fc-fusion, an ICOSL IgV domain alone Fc-fusion, and/or ICOSL variant Fc fusion proteins with mutations at N207G/L208G with reference to the reference ICOSL extracellular domain (ECD) sequence set forth in SEQ ID NO:1. Molecules were expressed using ExpiCHO-S derived cells. **FIG. 10H** shows target binding of the variant ICOSL IgV-Fc, but not control proteins, for both CD28 and ICOS. **FIG. 10I** shows the ability of variant ICOSL IgV-Fc to block ligand binding to target receptors CD28 and ICOS as measured by flow cytometry and IC50 levels. **FIG. 10J** shows the variant ICOSL IgV-Fc blocked both CD28 and ICOS costimulation.

**FIGS. 11A-11E** depicts anti-inflammatory activity of prophylactic dosing of the exemplary ICOSL IgV-Fc fusion molecule in the collagen-induced arthritis (CIA) model, including mean sum paw score **(FIG. 11A),** detected CII IgG **(FIG. 11B),** serum cytokine levels **(FIG. 11C),** CD44+ activated T cells or $T_{FH}$ cells **(FIG. 11D),** and fraction of B cells in the draining lymph node **(FIG. 11E).**

**FIGS. 12A-12D** depicts anti-inflammatory activity of delayed dosing of the exemplary ICOSL IgV-Fc fusion molecule in the collagen-induced arthritis (CIA) model, including mean sum paw score **(FIG. 12A)** and serum cytokine levels **(FIG. 12C-12D).**

**FIGS. 13A-13D** depicts anti-inflammatory activity of delayed dosing of the exemplary ICOSL IgV-Fc fusion molecule in the experimental autoimmune encephalomyelitis (EAE) model, including EAE score **(FIG. 13A),** mean % body weight over time **(FIG. 13B),** flow cytometric analysis of inguinal lymph node T cells **(FIG. 13C),** and proinflammatory cytokines **(FIG. 13D).**

**FIGS. 14A-14B** depicts survival and DAI score of Graft-versus-Host-Disease (GvHD) mice treated with various doses (20, 100, or 500 μg) of a variant ICOSL IgV-Fc molecule.

**FIGS. 15A-15F** depicts results from flow cytometric analysis of Graft-versus-Host-Disease (GvHD) ratio of human cells/mouse cells in blood collected **(FIG. 15A)** or in total T cell count **(FIG. 15B)** at the end of the study, and assessment of ICOS+ CD4+ or CD8+ cells **(FIG. 15C-15D),** or CD28+ CD4+ or CD8+ cells **(FIG. 15E-15F)** from Graft-versus-Host-Disease (GvHD) mice treated with various doses (20, 100, or 500 μg) of a variant ICOSL IgV-Fc molecule.

**FIGS. 16A-16B** depicts expression of activation or exhaustion markers of T cells from Graft-versus-Host-Disease (GvHD) mice treated with various doses (20, 100, or 500 μg) of a variant ICOSL IgV-Fc molecule.

**FIG. 16C** depicts the ratio of T effector cells (Teff) to T regulatory cells (Treg) from Graft-versus-Host-Disease (GvHD) mice treated with various doses (20, 100, or 500 μg) of a variant ICOSL IgV-Fc molecule.

**FIGS. 17A-17D** depicts serum proinflammatory cytokines from Graft-versus-Host-Disease (GvHD) mice treated with various doses (20, 100, or 500 μg) of a variant ICOSL IgV-Fc molecule.

**FIG. 17E** depicts serum exposure of variant ICOSL IgV-Fc (N52H/N57Y/Q100R) in the GVHD model compared to normal mice.

**FIG. 18A** depicts DAI results and **FIG. 18B** depicts histology results from treatment with an exemplary variant ICOSL IgV-Fc on disease activity index (DAI) calculated from body weight and stool scores in a CD4+CD45RBhigh-induced colitis model.

**FIGS. 19A-19N** depict *in vitro* stimulation of cytokine release in a Sjögren's Syndrome (SjS) model involving coculture of PBMCs from subjects with SjS or healthy donors with artificial antigen presenting cells (aAPCs) in the presence of an Fc control, prezalumab (prez), abatacept (Aba), a combination of abatacept and prezalumab (A+P) or ICOS-IgV Fc (N52H/N57Y/Q100R). **FIG. 19A** and **FIG. 19B** depict production of IL-2 from SjS PBMC and healthy donor PBMC, respectively. **FIG. 19C** and **FIG. 19D** depict product of IFN-γ from SjS PBMC and healthy donor PBMC, respectively. **FIG. 19E** and **FIG. 19F** depict IL-2 and IFN-γ respectively from SjS PBMC matched donors following co-culture with artificial APC in the presence of Fc control, abatacept or ICOS-IgV Fc (N52H/N57Y/Q100R). **FIG. 19G** to **19I** depict exemplary results for cytokine responses. **FIG. 19J** depicts gene analysis for SjS, SLE and PsA patient polulations. **FIG. 19K** depicts T cell proliferation, CD40L+ T cell recovery and B cell proliferation, and B cell differentiation as shown by the increase in percent of naive T cells during the co-culture. **FIG. 19L** depicts IC50 values for effects on cellular activation/proliferation. **FIG. 19M** depicts results for secretion of human IgG1, IgG3, IgA and IgM. **FIG. 19N** depicts next generation sequencing results for genes involved in B-T cell collaboration, sorted by level of inhibition by the variant ICOSL IgV-Fc, with degree of red reflecting higher expression values and degree of blue representing lower values for each gene.

**FIGS. 20A-20H** depict histology results of submandibular glands (SMG), organs and results for serum collected from naive mice or mice from a NOD x anti-PDL1 mouse modelof of anti-PD-L1 induced sialadenitis that had been treated with Fc control, abatacept or ICOS-IgV Fc (N52H/N57Y/Q100R). Exemplary histology images after staining with H&E are shown in **FIGS. 20A** and **20C. FIG. 20B** and **FIG. 20D** depict scores for inflammation on a scale of 0-3 for treated mice. **FIG. 20E** depicts individual SMG scores. **FIG. 20F** depicts serum autoantibodies from serum collected at day 10 and analyzed by ELISA. **FIG. 20G** depicts relative gene expression levels from RNA isolated from SMG collected at day 10. **FIG. 20H** depicts levels of GC B and CD4+Tfh cells per spleen, and decreased serum anti-dsDNA compared to Fc control in a model of SLE involving alloactivation of donor CD4+ T cells by recipient antigen-presenting cells leading to a cGvHD disease with symptoms resembling SLE.

**FIGS. 21A-21E** depict in vitro stimulation of cytokine release in a arthritis model involving coculture of PBMCs from subjects healthy donors (HD) or subjects with Rheumatoid Arthritis (RA) or Psoriatic Arthritis (PA) with artificial antigen presenting cells (aAPCs) in the presence of an Fc control, prezalumab (prez), abatacept (Aba), a combination of abatacept and prezalumab (A+P) or ICOS-IgV Fc (N52H/N57Y/Q100R). Results are shown for IL-2 **(FIG. 21A),** IFN-γ **(FIG. 21B),** TNFα **(FIG. 21C)** and IL-17A **(FIG. 21D). FIG. 21E** depicts percent inhibition of cytokine secretion relative to Fc control.

**FIGS. 22A-22D** depict results for RNA isolated from in vitro stimulation of cytokine release in a arthritis model involving co-culture of PBMCs from healthy donors (HD) or subjects with Rheumatoid Arthritis (RA) or Psoriatic Arthritis (PA) with artificial antigen presenting cells (aAPCs) in the presence of an Fc control, prezalumab (prez), abatacept (Aba), a combination of abatacept and prezalumab (A+P) or ICOS-IgV Fc (N52H/N57Y/Q100R). **FIG. 22A** depicts genes associated with disease pathogenesis that were downregulated by ICOSL IgV-Fc. Results are shown for gene reduction and/or modulation by ICOSL IgV-Fc vs comparators for Th17-associated effector molecules **(FIG. 22B),** costimulatory molecules **(FIG. 22C)** and inflammatory TH1 or TH2-associated effector molecules **(FIG. 22D).**

**FIGS. 23A-23C** depict results for human PBMCs under primary stimulation with aAPCs under conditions for no skewing (K562/OKT3/CD80 + CD86 + IL-2) or Th17 Skewing (K562/OKT3/ICOSL + Th17 skewing media. **FIG. 23A** depicts flow cytometry analysis of ICOS or CD28 four days post stimulation. Results for T cell proliferation **(FIG. 23B)** and IL-17A cytokine secretion **(FIG. 23C)** are shown.

**FIGS. 24A-24E** depict results for a collagen-induced arthritis (CIA) model for mice treated intraperitoneally (IP) every 3 days for a total of 5 doses (Q3Dx5) with vehicle (Dulbecco's phosphate buffered saline/DPBS), Fc control, abatacept (Orencia™), or exemplary variant ICOSL IgV-Fc or abatacept in a semi-therapeutic regimen, starting before administration of a collagen boost on Day 18. **FIG. 24A** depicts paw inflammation and structural damage. **FIG. 24B** depicts toluidine blue staining of decalcified paws. **FIG. 24C** depicts number of cytokines as measured by the percent of CD4+ or total live cells. **FIG. 24D** depicts the concentration of total anti-mouse Type II collagen IgG antibody. **FIG. 24E** depicts percent inhibition of cytokines determined for individual mice from the Fc control, Abatacept, or exemplary variant ICOSL IgV-Fc -treated mice relative to mice treated with vehicle (DPBS).

**FIG. 25A** shows the pharmacokinetics of an exemplary ICOSL IgV-Fc, as measured over 28 days in subjects treated with the indicated doses of an exemplary ICOSL IgV-Fc.

**FIG. 25B** depicts the percent CD4+ target saturation, as measured over 28 days in subjects treated with the indicated doses of an exemplary ICOSL IgV-Fc.

**FIGS. 26A** and **26B** depict the immunomodulatory activity of an exemplary ICOSL IgV-Fc fusion protein in human subjects. The fusion protein suppressed antibody response in subjects following exposure to an immunostimulant **(FIG. 26A)** and inhibited IL-2 production by subjects' cells when exposed *ex vivo* to staphylococcal enterotoxin B **(FIG. 26B).**

**FIGS. 27A and 27B** show the effects of exemplary variant ICOSL IgV-Fc on cytokine release from PBMCs isolated from patients with Crohn's Disease (CD) or Ulcerative Colitis (UC) and stimulated with artificial antigen presenting cells *in vitro*. **FIG. 27A** shows TNF-alpha, IFN$\gamma$, IL-6, and **IL-12p70** concentration in supernatant after 48 hours of incubation with artificial antigen presenting cells and treatment as shown. **FIG. 27B** shows percent inhibition, relative to Fc control treatment, of cytokine release for treatment as shown.

**FIG. 28** describes the efficacy of exemplary variant ICOSL IgV-Fc in an *in vivo* T cell transfer murine model of colitis. **FIG. 28A** depicts body weight, stool score, and DAI results. **FIG. 28B** shows T cell suppression via flow cytometric analyses from both the blood (left) and mesenteric lymph node (right) (legend is same as shown in **FIG. 28A). FIG. 28C** depicts serum proinflammatory cytokines from mice treated with a vehicle control (no colitis), Fc control, or exemplary variant ICOSL IgV-Fc described herein. **FIG. 28D** depicts histology results from treatment with an exemplary variant ICOSL IgV-Fc on colon weight:length ratio and combined histology score. Representative colonic tissue staining can be seen in **FIG. 28E** and at higher magnification in **FIG. 28F.**

**FIGS. 29A** and **29B** show median and mean unmasked clinical scores, respectively, for both treated and untreated eyes in an experimental model of uveitis. **FIG. 29C** shows median unmasked clinical scores on day 14 for both eyes according to treatment. **FIGS. 29D** and **29E** show median unmasked clinical scores by day for right (treated) eyes only. (8/10 or 10/12 indicates days administered).

**FIG. 30A** shows median masked OCT scores for treated and untreated eyes in an experimental model of uveitis. **FIG. 30B** shows median masked OCT scores for treated eyes. **FIG. 30C** shows median masked OCT scores for treated eyes by day. (8/10 or 10/12 indicates days administered).

**FIG. 31** shows median masked histology scores on day 14 for both treated (right) and untreated (left) eyes as indicated. (8/10 or 10/12 indicates days administered)

**FIG. 32** shows weight change from baseline across days and treatment (mean and standard deviation).

**FIG. 33** shows the pharmacokinetics of the exemplary ICOSL IgV-Fc fusion protein after a single dose. Mean concentration is plotted over time. (SC = subcutaneous; others are intravenous (i.v.))

**FIG. 34** shows mean and standard deviation target saturation on CD4+ T cells over time after a single dose of the exemplary ICOSL IgV-Fc fusion protein. (SC = subcutaneous; others are intravenous (i.v.)).

**FIG. 35** shows mean and standard deviation of anti-KLH IgG relative to baseline versus time in subjects administered a single dose of exemplary ICOSL IgV-Fc fusion protein. (SC = subcutaneous; others are intravenous (i.v.)).

**FIG. 36** shows the pharmacokinetics of the exemplary ICOSL IgV-Fc fusion protein after multiple doses (i.v.). Mean concentration is plotted over time.

**FIG. 37** shows mean and standard deviation target saturation on CD4+ T cells over time after a multiple doses (i.v.)

of exemplary ICOSL IgV-Fc fusion protein.

**FIG. 38** shows mean and standard deviation of anti-KLH IgG relative to baseline versus time in subjects administered a mutliple doses (i.v.) of the exemplary ICOSL IgV-Fc fusion protein.

## Detailed Description

Provided herein are immunomodulatory proteins that are or comprise variants or mutants of ICOS ligand (ICOSL) or specific binding fragments thereof that exhibit activity to bind to at least one target ligand cognate binding partner (also called counter-structure protein). In some embodiments, the variant ICOSL polypeptides contain one or more amino acid modifications (e.g. amino acid substitutions, deletions or additions) compared to a reference (e.g., unmodified) or wild-type ICOSL polypeptide. In some embodiments, the one or more amino acid modifications (e.g. amino acid substitutions, deletions or additions) are in an immunoglobulin superfamily (IgSF) domain (e.g. IgV) of a reference (e.g., unmodified) or wild-type ICOSL polypeptide. In some embodiments, the variant ICOSL polypeptide exhibits altered, such as increased or decreased, binding activity or affinity for at least one cognate binding partner, such as at least one of ICOS, CD28, or CTLA-4. In some embodiments, the immunomodulatory proteins are soluble. In some embodiments, also provided herein are one or more other immunomodulatory proteins that are conjugates or fusions containing a variant ICOSL polypeptide provided herein and one or more other moiety or polypeptide.

In some embodiments, the variant ICOSL polypeptides and immunomodulatory proteins modulate an immunological immune response, such as an increased or decreased immune response. In some embodiments, the variant ICOSL polypeptides and immunomodulatory proteins provided herein can be used for the treatment of diseases or conditions that are associated with a dysregulated immune response.

In some embodiments, the provided variant ICOSL polypeptides modulate T cell activation via interactions with costimulatory signaling molecules. In general, antigen specific T-cell activation requires two distinct signals. The first signal is provided by the interaction of the T-cell receptor (TCR) with major histocompatibility complex (MHC) associated antigens present on antigen presenting cells (APCs). The second signal is costimulatory to TCR engagement and necessary to avoid T-cell apoptosis or anergy.

In some embodiments, under normal physiological conditions, the T cell-mediated immune response is initiated by antigen recognition by the T cell receptor (TCR) and is regulated by a balance of co-stimulatory and inhibitory signals (e.g., immune checkpoint receptors). The immune system relies on immune checkpoint receptors to prevent autoimmunity (i.e., self-tolerance) and to protect tissues from excessive damage during an immune response, for example during an attack against a pathogenic infection. In some cases, however, these immunomodulatory proteins can be dysregulated in diseases and conditions, including tumors, as a mechanism for evading the immune system.

In some embodiments, among known T-cell costimulatory receptors is CD28, which is the T-cell costimulatory receptor for the ligands B7-1 (CD80) and B7-2 (CD86) both of which are present on APCs. These same ligands can also bind to the inhibitory T-cell receptor CTLA4 (cytotoxic T-lymphocyte-associated protein 4) with greater affinity than for CD28; the binding to CTLA-4 acts to down-modulate the immune response. ICOS (inducible costimulator) is another T-cell costimulatory receptor which binds to ICOS ligand (ICOSL) on APCs. In some cases, CD28 and CTLA-4 also are known to interact with ICOSL at a binding site that overlaps with the binding of ICOSL to the T-cell costimulatory receptor ICOS (Yao et al. (2011) Immunity, 34:729-740).

CD28 and ICOS are closely related T cells costimulatory molecules and binding by their ligands CD80 and CD86, and ICOSL, respectively, induce cell signaling that result in partially overlapping roles in immunity. Thus, although CD28 and ICOS are related CD28 family activating receptors and share some intracellular signaling motifs, costimulatory effects between CD28 and ICOS can, in some respects, differ. For example, CD28 is expressed on both unactivated and activated T cells and its signaling is important for IL-2 production and subsequent T cell effector function, and CD28 is involved in native T cell activation. The therapeutic inhibitors of the CD28 pathway (e.g. abatacept, CTLA4-Ig; and belatacept, a second generation CTLA4-Ig) have proven useful for the treatment of some inflammatory arthritis conditions (e.g. rheumatoid arthritis, juvenile idiopathic arthritis, psoriatic arthritis) and for the prevention of renal allograft rejection (Wekerle et al. Transpl. Int., 2012, 25:139-50). However, therapeutic blockage of the CD28 pathway, primarily as studied with abatacept, has proven unsuccessful for several other inflammatory diseases (e.g. Crohn's Disease, lupus nephritis, multiple sclerosis), even in such disease indications involving implication of T cells in disease pathogenesis. Even within their approved indications, treatment with CTLA4-Ig compounds does not result in complete remission and/or suppression of inflammatory activity in most patients (Masson, Cochrane Database Sys. Rev., 2014, CD010699; Singh, Cochrane Database Sys. Rev., 2017, CD012657). This is consistent with an additional pathogenic costimulatory pathway that remains unaddressed by these treatments.

ICOS is generally not expressed on the surface of T cells until after T cell activation, and signaling through ICOS on activated T cells supports specialized T cell subset differentiation. Thus, in some cases, costimulation by CD28 and ICOS yields overlapping and complementary effects. Accordingly, ICOS may be an alternative pathogenic pathway not addressed by therapeutics that only target CD28.

In some aspects, T cells express the costimulatory molecules CD28 and ICOS, which interact with CD80/CD86 and ICOSL respectively, on antigen presenting cells (APC). In lymphoid organs, professional APC (i.e. dendritic cells, macrophages, and B cells) express CD80, CD86, and ICOSL and engage CD28+/ICOS+ T cells. ICOS is not expressed in naive T cells but rapidly upregulates after activation. In some embodiments, activated T cells can then differentiate into effector cells such as CD8+ cytotoxic T cells (CTL), IL-17A/F-secreting CD4+ Th17 cells, or CD4+ follicular helper ($T_{FH}$) cells. $T_{FH}$-expressing CD40L engage B cells in lymphoid follicles and release cytokines (e.g. IL-21) inducing differentiation of B cells to antibody (Ab)-secreting plasma cells. Plasma cells can produce tissue-damaging antibodies, e.g., rheumatoid factor (RF) and anti-citrullinated peptide antibodies (ACPA) in humans, and anti-collagen (CII) antibodies in mice, which can form immune complexes and deposits in the joints and other tissues. ICOSL can also be expressed on non-professional APCs, leading to T cell activation in non-lymphoid tissues and further damage to the tissues and joints.

ICOS appears to play a role in the function of several activated and/or effector T cell subsets, such as in differentiated types 1, 2 and 17, as well as follicular helper (Wikenheiser, Front. Immunol., 2016 7:304). Indeed, activated T cells often downregulate CD28 and/or become less dependent on CD28 costimulation, and CD28-negative T cells accumulate in various inflammatory disease, correlating with disease activity and lack of responsiveness to abatacept (Garin et al., Eur J Haematol, 1996, 56:119-23; Schmidt et al., J. Clin. Invest., 1996, 97:2027-37; Yang et al., Chin J Microbiol Immunol, 2005, 25:248-251; Scarsi et al., J. Rheumatol., 2011, 38:2105-11; Mou et al., Am J Transplant, 2014, 14:2460-6; Zabinska et al., J Immunol Res., 2016, 2016:1058165; Piantoni et al., Lupus, 2018, 27:143-149). Combined inhibition of CD28 and ICOS, such as via molecules provided herein, may provide an improved therapy for autoimmune and/or inflammatory diseases.

In some aspects, CD4+Th1-, Th9- and Th17-cells, are implicated as key contributors to multiple sclerosis (MS) by increasing inflammation within the CNS in both multiple sclerosis and experimental autoimmune encephalomyelitis and CD4+ICOS+CXCR5+ T follicular helper cells are increased in PBMC in relapsing-remitting and correlate with disease progression in secondary progressive MS. In some embodiments, there is significantly increased ICOS gene expression in cerebrospinal fluid cells, in secondary progressive MS, and an increased percentage of total monocytes and monocytes expressing ICOSL is observed. ICOSL also is expressed on non-professional APCs, leading to T cell activation in non-lymphoid tissues and further tissue damage

Among the provided variant ICOSL polypeptide are polypeptides that, when modified by one or more amino acid modifications of an IgSF domain of a reference ICOSL polypeptide, exhibit enhanced binding affinity for CD28 and/or ICOS. In some cases, the overall increase in ICOS binding in provided variants is less than the increase in CD28 binding because wild-type ICOSL already demonstrates substantially more binding affinity for ICOS than CD28. Also provided are various formats of the provided variant polypeptides. For example, delivery of enhanced ICOSL proteins in soluble formats is shown herein to antagonize T cell activation by inhibiting CD28 and/or ICOS signaling.

In particular, a variant ICOSL polypeptide is provided in a format, e.g. as an Fc-fusion protein, to antagonize or block activity of its cognate binding partner, e.g. ICOS and/or CD28. In some embodiments, blocking or inhibiting costimulatory signaling via CD28 or ICOS may be useful to suppress an immune response, which can be useful in the treatment of inflammatory or autoimmune disorders (e.g., multiple sclerosis or brain inflammation), or organ transplantation.

In some embodiments, the modulation of immune signaling achieved by the provided variant ICOSL polypeptides and immunomodulatory polypeptides offers advantages for treatment of inflammatory and autoimmune disorders and other diseases and conditions compared to other treatments. In some cases, therapies to intervene and alter the costimulatory effects of both receptors are constrained by the spatial orientation requirements as well as size limitations imposed by the confines of the immunological synapse. In some aspects, existing therapeutic drugs, including antibody drugs, may not be able to interact simultaneously with the multiple target proteins involved in modulating these interactions. Additionally, pharmacokinetic differences between drugs that independently target one or the other of these two receptors can create difficulties in properly maintaining a desired blood concentration of such drug combinations throughout the course of treatment.

In some embodiments, the provided variant ICOSL polypeptides or immunomodulatory proteins modulate (e.g. increase or decrease) immunological activity induced by costimulatory receptors CD28 or ICOS. Thus, in some embodiments, the provided polypeptides overcome these constraints by providing variant ICOSL (inducible costimulator ligand) with altered (e.g. increased) binding affinities to both CD28 and ICOS, and, in some cases, CTLA-4, thereby agonizing or antagonizing the complementary effects of costimulation by receptors. Methods of making and using these variant ICOSL are also provided.

In some aspects, the provided molecules may also be more effective than other soluble therapeutic protein agents. For example, abatacept (CTLA-4-Fc) has been shown to interfere with T cell costimulation to attenuate T cell responses in autoimmune disease settings, such as for the treatment of rheumatoid arthritis, psoriatic arthritis and juvenile idiopathic arthritis, and belatacept, a variant CTLA-4-Fc molecule, for transplant rejection. These CTLA-4-Fc proteins, however, bind to CD80 and CD86 and prevent these costimulatory ligands from engaging and triggering only CD28. Variant ICOSL polypeptides provided herein, in some cases, exhibit binding affinity and enhanced activity for both CD28 and ICOS.

All publications, including patents, patent applications scientific articles and databases, mentioned in this specification are herein incorporated by reference in their entirety for all purposes to the same extent as if each individual publication, including patent, patent application, scientific article or database, were specifically and individually indicated to be incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

## I. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The terms used throughout this specification are defined as follows unless otherwise limited in specific instances. As used in the specification and the appended claims, the singular forms "a," "an," and "the" include plural referents unless the context clearly dictates otherwise. Unless defined otherwise, all technical and scientific terms, acronyms, and abbreviations used herein have the same meaning as commonly understood by one of ordinary skill in the art to which the invention pertains. Unless indicated otherwise, abbreviations and symbols for chemical and biochemical names is per IUPAC-IUB nomenclature. Unless indicated otherwise, all numerical ranges are inclusive of the values defining the range as well as all integer values in-between.

The term "affinity modified" as used in the context of an immunoglobulin superfamily domain, means a mammalian immunoglobulin superfamily (IgSF) domain having an altered amino acid sequence (relative to the corresponding wild-type parental or unmodified IgSF domain) such that it has an increased or decreased binding affinity or avidity to at least one of its cognate binding partners (alternatively "counter-structures") compared to the parental wild-type or unmodified (i.e., non-affinity modified) IgSF control domain. Included in this context is an affinity modified ICOSL IgSF domain. In some embodiments, the affinity-modified IgSF domain can contain 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more amino acid differences, such as amino acid substitutions, in a reference (e.g., unmodified) or wild-type IgSF domain. An increase or decrease in binding affinity or avidity can be determined using well known binding assays such as flow cytometry. Larsen et al., American Journal of Transplantation, Vol 5: 443-453 (2005). See also, Linsley et al., Immunity, Vol 1: 793-801 (1994). An increase in a protein's binding affinity or avidity to its cognate binding partner(s) is to a value at least 10% greater than that of the wild-type IgSF domain control and in some embodiments, at least 20%, 30%, 40%, 50%, 100%, 200%, 300%, 500%, 1000%, 5000%, or 10000% greater than that of the wild-type IgSF domain control value. A decrease in a protein's binding affinity or avidity to at least one of its cognate binding partner is to a value no greater than 90% of the control but no less than 10% of the wild-type IgSF domain control value, and in some embodiments no greater than 80%, 70% 60%, 50%, 40%, 30%, or 20% but no less than 10% of the wild-type IgSF domain control value. An affinity-modified protein is altered in primary amino acid sequence by substitution, addition, or deletion of amino acid residues. The term "affinity modified IgSF domain" is not be construed as imposing any condition for any particular starting composition or method by which the affinity-modified IgSF domain was created. Thus, the affinity modified IgSF domains of the present invention are not limited to wild type IgSF domains that are then transformed to an affinity modified IgSF domain by any particular process of affinity modification. An affinity modified IgSF domain polypeptide can, for example, be generated starting from wild type mammalian IgSF domain sequence information, then modeled in silico for binding to its cognate binding partner, and finally recombinantly or chemically synthesized to yield the affinity modified IgSF domain composition of matter. In but one alternative example, an affinity modified IgSF domain can be created by site-directed mutagenesis of a wild-type IgSF domain. Thus, affinity modified IgSF domain denotes a product and not necessarily a product produced by any given process. A variety of techniques including recombinant methods, chemical synthesis, or combinations thereof, may be employed.

The term "allogeneic" as used herein means a cell or tissue that is removed from one organism and then infused or adoptively transferred into a genetically dissimilar organism of the same species. In some embodiments of the invention, the species is murine or human.

The term "autologous" as used herein means a cell or tissue that is removed from the same organism to which it is later infused or adoptively transferred. An autologous cell or tissue can be altered by, for example, recombinant DNA methodologies, such that it is no longer genetically identical to the native cell or native tissue which is removed from the organism. For example, a native autologous T-cell can be genetically engineered by recombinant DNA techniques to become an autologous engineered cell expressing a transmembrane immunomodulatory protein and/or chimeric antigen receptor (CAR), which in some cases involves engineering a T-cell or TIL (tumor infiltrating lymphocyte). The engineered cells are then infused into a patient from which the native T-cell was isolated. In some embodiments, the organism is human or murine.

The terms "binding affinity," and "binding avidity" as used herein means the specific binding affinity and specific binding avidity, respectively, of a protein for its counter-structure under specific binding conditions. In biochemical kinetics avidity refers to the accumulated strength of multiple affinities of individual non-covalent binding interactions, such as between ICOSL and its counter-structures ICOS and/or CD28. As such, avidity is distinct from affinity, which describes the strength of a single interaction. An increase or attenuation in binding affinity of a variant ICOSL containing an affinity modified ICOSL IgSF domain to its counter-structure is determined relative to the binding affinity of the unmodified ICOSL, such as an unmodified ICOSL containing the native or wild-type IgSF domain, such as IgV domain. Methods for determining binding affinity or avidity are known in art. See, for example, Larsen et al., American Journal of Transplantation, Vol 5: 443-453 (2005). In some embodiments, a variant ICOSL of the invention (i.e. a ICOSL protein containing an affinity modified IgSF domain) specifically binds to CD28 and/or ICOS measured by flow cytometry with a binding affinity that yields a Mean Fluorescence Intensity (MFI) value at least 10%, 20%, 30%, 40%, 50%, 60%, 70%, 80%, 90%, or 100% greater than a wild-type ICOSL control in a binding assay such as described in Example 6.

The term "biological half-life" refers to the amount of time it takes for a substance, such as an immunomodulatory polypeptide comprising a variant ICOSL of the present invention, to lose half of its pharmacologic or physiologic activity or concentration. Biological half-life can be affected by elimination, excretion, degradation (e.g., enzymatic) of the substance, or absorption and concentration in certain organs or tissues of the body. In some embodiments, biological half-life can be assessed by determining the time it takes for the blood plasma concentration of the substance to reach half its steady state level ("plasma half-life"). Conjugates that can be used to derivatize and increase the biological half-life of polypeptides of the invention are known in the art and include, but are not limited to, polyethylene glycol (PEG), hydroxyethyl starch (HES), XTEN (extended recombinant peptides; see, WO2013130683), human serum albumin (HSA), bovine serum albumin (BSA), lipids (acylation), and poly-Pro-Ala-Ser (PAS), polyglutamic acid (glutamylation).

The term "chimeric antigen receptor" or "CAR" as used herein refers to an artificial (i.e., man-made) transmembrane protein expressed on a mammalian cell comprising at least an ectodomain, a transmembrane, and an endodomain. Optionally, the CAR protein includes a "spacer" which covalently links the ectodomain to the transmembrane domain. A spacer is often a polypeptide linking the ectodomain to the transmembrane domain via peptide bonds. The CAR is typically expressed on a mammalian lymphocyte. In some embodiments, the CAR is expressed on a mammalian cell such as a T-cell or a tumor infiltrating lymphocyte (TIL). A CAR expressed on a T-cell is referred to herein as a "CAR T-cell" or "CAR-T." In some embodiments the CAR-T is a T helper cell, a cytotoxic T-cell, a natural killer T-cell, a memory T-cell, a regulatory T-cell, or a gamma delta T-cell. When used clinically in, e.g. adoptive cell transfer, a CAR-T with antigen binding specificity to the patient's tumor is typically engineered to express on a native T-cell obtained from the patient. The engineered T-cell expressing the CAR is then infused back into the patient. The CAR-T is thus often an autologous CAR-T although allogeneic CAR-T are included within the scope of the invention. The ectodomain of a CAR comprises an antigen binding region, such as an antibody or antigen binding fragment thereof (e.g. scFv), that specifically binds under physiological conditions with a target antigen, such as a tumor specific antigen. Upon specific binding a biochemical chain of events (i.e., signal transduction) results in modulation of the immunological activity of the CAR-T. Thus, for example, upon specific binding by the antigen binding region of the CAR-T to its target antigen can lead to changes in the immunological activity of the T-cell activity as reflected by changes in cytotoxicity, proliferation or cytokine production. Signal transduction upon CAR-T activation is achieved in some embodiments by the CD3-zeta chain ("CD3-z") which is involved in signal transduction in native mammalian T-cells. CAR-Ts can further comprise multiple signaling domains such as CD28, 41BB or OX40. to further modulate immunomodulatory response of the T-cell. CD3-z comprises a conserved motif known as an immunoreceptor tyrosine-based activation motif (ITAM) which is involved in T-cell receptor signal transduction.

The term "collectively" or "collective" when used in reference to cytokine production induced by the presence of two or more variant ICOSL of the invention in an in vitro assay, means the overall cytokine expression level irrespective of the cytokine production induced by individual variant ICOSL. In some embodiments, the cytokine being assayed is IFN-gamma in an in vitro primary T-cell assay.

The term "cognate binding partner" (used interchangeably with "counter-structure") in reference to a polypeptide, such as in reference to an IgSF domain of a variant ICOSL, refers to at least one molecule (typically a native mammalian protein) to which the referenced polypeptide specifically binds under specific binding conditions. In some aspects, a variant ICOSL containing an affinity modified IgSF domain specifically binds to the counter-structure of the corresponding native or wild-type ICOSL but with increased or attenuated affinity. A species of ligand recognized and specifically binding to its cognate receptor under specific binding conditions is an example of a counter-structure or cognate binding partner of that receptor. A "cognate cell surface binding partner" is a cognate binding partner expressed on a mammalian cell surface. A "cell surface molecular species" is a cognate binding partner of ligands of the immunological synapse (IS), expressed on and by cells, such as mammalian cells, forming the immunological synapse.

As used herein, "conjugate," "conjugation" or grammatical variations thereof refers the joining or linking together of two or more compounds resulting in the formation of another compound, by any joining or linking methods known in the art. It can also refer to a compound which is generated by the joining or linking together two or more compounds. For example, a variant ICOSL polypeptide linked directly or indirectly to one or more chemical moieties or polypeptide is an exemplary conjugate. Such conjugates include fusion proteins, those produced by chemical conjugates and those produced by any other methods.

The term "competitive binding" as used herein means that a protein is capable of specifically binding to at least two cognate binding partners but that specific binding of one cognate binding partner inhibits, such as prevents or precludes, simultaneous binding of the second cognate binding partner. Thus, in some cases, it is not possible for a protein to bind the two cognate binding partners at the same time. Generally, competitive binders contain the same or overlapping binding site for specific binding but this is not a requirement. In some embodiments, competitive binding causes a measurable inhibition (partial or complete) of specific binding of a protein to one of its cognate binding partner due to specific binding of a second cognate binding partner. A variety of methods are known to quantify competitive binding such as ELISA (enzyme linked immunosorbent assay) assays.

The term "conservative amino acid substitution" as used herein means an amino acid substitution in which an amino acid residue is substituted by another amino acid residue having a side chain R group with similar chemical properties (e.g., charge or hydrophobicity). Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Conservative amino acids substitution groups are: valine-leucine-isoleucine, phenylalanine-tyrosine, lysine-arginine, alanine-valine, glutamate-aspartate, and asparagine-glutamine.

The term, "corresponding to" with reference to positions of a protein, such as recitation that nucleotides or amino acid positions "correspond to" nucleotides or amino acid positions in a disclosed sequence, such as set forth in the Sequence listing, refers to nucleotides or amino acid positions identified upon alignment with the disclosed sequence based on structural sequence alignment or using a standard alignment algorithm, such as the GAP algorithm. For example, corresponding residues can be determined by alignment of a reference sequence with the sequence set forth in SEQ ID NO: 1 (ECD domain) or set forth in SEQ ID NOs: 2 or 3 (IgV domain) by structural alignment methods as described herein. By aligning the sequences, one skilled in the art can identify corresponding residues, for example, using conserved and identical amino acid residues as guides.

The terms "decrease" or "attenuate" "or suppress" as used herein means to decrease by a statistically significant amount. A decrease can be at least 10%, 20%, 30%, 40%, 50%, 60%, 70%, 80%, 90%, or 100% of a control value, such as a non-zero control value.

The terms "decreased" or "reduced" as used herein in the context of decreasing immunological activity of a mammalian lymphocyte means to decrease one or more activities of the lymphocyte, as compared to a control, such as an untreated control or a control in which a treatment using an unmodified or non-variant control was employed under the same conditions. A decreased activity can refer to one or more of cell cycle inhibition, reduced cell survival, reduced cell proliferation, reduced cytokine production, or reduced T-cell cytotoxicity, such as by a statistically significant amount. In some embodiments, reference to reduced immunological activity means to reduce interferon gamma (IFN-gamma) production compared to in the absence of treatment, such as by a statistically significant amount. In some embodiments, the immunological activity can be assessed in a mixed lymphocyte reaction (MLR) assay. Methods of conducting MLR assays are known in the art. Wang et al., Cancer Immunol Res. 2014 Sep: 2(9):846-56. Other methods of assessing activities of lymphocytes are known in the art, including any assay as described herein. In some embodiments an enhancement can be a decrease by at least 5%, 10%, 15%, 20%, 25%, 30%, 35%, 40%, 45%, 50%, 60%, 70%, 80%, 90%, or 100%, as compared to a control value, such as an untreated control value or a non-zero control value.

The terms "derivatives" or "derivatized" refer to modification of a protein by covalently linking it, directly or indirectly, to a composition so as to alter such characteristics as biological half-life, bioavailability, immunogenicity, solubility, toxicity, potency, or efficacy while retaining or enhancing its therapeutic benefit. Derivatives of immunomodulatory polypeptides of the invention are within the scope of the invention and can be made by, for example, glycosylation, pegylation, lipidation, or Fc-fusion.

As used herein, domain (typically a sequence of three or more, generally 5 or 7 or more amino acids, such as 10 to 200 amino acid residues) refers to a portion of a molecule, such as a protein or encoding nucleic acid, that is structurally and/or functionally distinct from other portions of the molecule and is identifiable. For example, domains include those portions of a polypeptide chain that can form an independently folded structure within a protein made up of one or more structural motifs and/or that is recognized by virtue of a functional activity, such as binding activity. A protein can have one, or more than one, distinct domains. For example, a domain can be identified, defined or distinguished by homology of the primary sequence or structure to related family members, such as homology to motifs. In another example, a domain can be distinguished by its function, such as an ability to interact with a biomolecule, such as a cognate binding partner. A domain independently can exhibit a biological function or activity such that the domain independently or fused to another molecule can perform an activity, such as, for example binding. A domain can be a linear sequence of amino acids or a non-linear sequence of amino acids. Many polypeptides contain a plurality of domains. Such domains are known, and can be identified by those of skill in the art. For exemplification herein, definitions are provided, but it is understood that it is well within the skill in the art to recognize particular domains by name. If needed appropriate software can be employed to identify domains.

The term "ectodomain" as used herein refers to the region of a membrane protein, such as a transmembrane protein, that lies outside the vesicular membrane. Ectodomains often comprise binding domains that specifically bind to ligands or cell surface receptors, such as via a binding domain that specifically binds to the ligand or cell surface receptor. The ectodomain of a cellular transmembrane protein is alternately referred to as an extracellular domain.

The terms "effective amount" or "therapeutically effective amount" refer to a quantity and/or concentration of a therapeutic composition of the invention, including a protein composition or cell composition, that when administered ex vivo (by contact with a cell from a patient) or in vivo (by administration into a patient) either alone (i.e., as a monotherapy) or in combination with additional therapeutic agents, yields a statistically significant decrease in disease progression as, for example, by ameliorating or eliminating symptoms and/or the cause of the disease. An effective amount may be an amount that relieves, lessens, or alleviates at least one symptom or biological response or effect associated with a disease or disorder, prevents progression of the disease or disorder, or improves physical functioning of the patient. In the case of cell therapy, the effective amount is an effective dose or number of cells administered to a patient by adoptive cell therapy. In some embodiments the patient is a mammal such as a non-human primate or human patient.

The term "endodomain" as used herein refers to the region found in some membrane proteins, such as transmembrane proteins, that extends into the interior space defined by the cell surface membrane. In mammalian cells, the endodomain is the cytoplasmic region of the membrane protein. In cells, the endodomain interacts with intracellular constituents and can be play a role in signal transduction and thus, in some cases, can be an intracellular signaling domain. The endodomain of a cellular transmembrane protein is alternately referred to as a cytoplasmic domain, which, in some cases, can be a cytoplasmic signaling domain.

The terms "enhanced" or "increased" as used herein in the context of increasing immunological activity of a mammalian lymphocyte means to increase one or more activities the lymphocyte, as compared to a control, such as an untreated control or a control in which a treatment using an unmodified or non-variant control was employed under the same conditions. An increased activity can be one or more of increase cell survival, cell proliferation, cytokine production, or T-cell cytotoxicity, such as by a statistically significant amount. In some embodiments, reference to increased immunological activity means to increase interferon gamma (IFN-gamma) production, such as by a statistically significant amount. In some embodiments, the immunological activity can be assessed in a mixed lymphocyte reaction (MLR) assay. Methods of conducting MLR assays are known in the art. Wang et al., Cancer Immunol Res. 2014 Sep: 2(9):846-56. Other methods of assessing activities of lymphocytes are known in the art, including any assay as described herein. In some embodiments an enhancement can be an increase of at least 10%, 20%, 30%, 40%, 50%, 75%,100%, 200%, 300%, 400%, or 500% greater than a non-zero control value.

The term "engineered cell" as used herein refers to a mammalian cell that has been genetically modified by human intervention such as by recombinant DNA methods or viral transduction. In some embodiments, the cell is an immune cell, such as a lymphocyte (e.g. T cell, B cell, NK cell) or an antigen presenting cell (e.g. dendritic cell). The cell can be a primary cell from a patient or can be a cell line. In some embodiments, an engineered cell of the invention comprises a variant ICOSL provided herein. In some embodiments, the variant ICOSL is a transmembrane immunomodulatory protein (hereinafter referred to as "TIP") that is expressed on the engineered cell. In some embodiments, the TIP contains the extracellular domain or a portion thereof containing the IgV domain linked to a transmembrane domain (e.g., a ICOSL transmembrane domain) and, optionally, an intracellular signaling domain. In some cases, the TIP is formatted as a chimeric receptor containing a heterologous cytoplasmic signaling domain or endodomain. In some embodiments, an engineered cell is capable of expressing and secreting a immunomodulatory protein as described herein. Among provided engineered cells also are cells further containing an engineered T-cell receptor (TCR) or chimeric antigen receptor (CAR).

The term "engineered T-cell" as used herein refers to a T-cell such as a T helper cell, cytotoxic T-cell (alternatively, cytotoxic T lymphocyte or CTL), natural killer T-cell, regulatory T-cell, memory T-cell, or gamma delta T-cell, that has been genetically modified by human intervention such as by recombinant DNA methods or viral transduction methods. An engineered T-cell comprises a variant ICOSL transmembrane immunomodulatory protein (TIP) or secreted immunodulatory protein (SIP) of the present invention that is expressed on the T-cell and is engineered to modulate immunological activity of the engineered T-cell itself, or a mammalian cell to which the variant ICOSL expressed on the T-cell specifically binds. An engineered T-cell can comprise a variant ICOSL secreted immunomodulatory protein (SIP) of the present invention that is expressed by and/or secreted by the T-cell and is engineered to modulate immunological activity of the engineered T-cell itself, or a mammalian cell to which the variant ICOSL when secreted by the T-cell, specifically binds.

The term "engineered T-cell receptor" or "engineered TCR" refers to a T-cell receptor (TCR) engineered to specifically bind with a desired affinity to a major histocompatibility complex (MHC)/peptide target antigen that is selected, cloned, and/or subsequently introduced into a population of T-cells, often used for adoptive immunotherapy. In contrast to engineered TCRs, CARs are engineered to bind target antigens in a MHC independent manner.

The term "expressed on" as used herein is used in reference to a protein expressed on the surface of a cell, such as a mammalian cell. Thus, the protein is expressed as a membrane protein. In some embodiments, the expressed protein is a transmembrane protein. In some embodiments, the protein is conjugated to a small molecule moiety such as a drug or detectable label. Proteins expressed on the surface of a cell can include cell-surface proteins such as cell surface receptors that are expressed on mammalian cells.

The term "half-life extending moiety" refers to a moiety of a polypeptide fusion or chemical conjugate that extends the half-life of a protein circulating in mammalian blood serum compared to the half-life of the protein that is not so conjugated to the moiety. In some embodiments, half-life is extended by greater than or greater than about 1.2-fold, 1.5-fold, 2.0-fold, 3.0-fold, 4.0-fold, 5.0-fold, or 6.0-fold. In some embodiments, half-life is extended by more than 6 hours, more than 12 hours, more than 24 hours, more than 48 hours, more than 72 hours, more than 96 hours or more than 1 week after *in vivo* administration compared to the protein without the half-life extending moiety. The half-life refers to the amount of time it takes for the protein to lose half of its concentration, amount, or activity. Half-life can be determined for example, by using an ELISA assay or an activity assay. Exemplary half-life extending moieties include an Fc domain, a multimerization domain, polyethylene glycol (PEG), hydroxyethyl starch (HES), XTEN (extended recombinant peptides; see, WO2013130683), human serum albumin (HSA), bovine serum albumin (BSA), lipids (acylation), and poly-Pro-Ala-Ser (PAS), and polyglutamic acid (glutamylation).

The term "immunological synapse" or "immune synapse" as used herein means the interface between a mammalian cell that expresses MHC I (major histocompatibility complex) or MHC II, such as an antigen-presenting cell or tumor cell, and a mammalian lymphocyte such as an effector T cell or Natural Killer (NK) cell.

An Fc (fragment crystallizable) region or domain of an immunoglobulin molecule (also termed an Fc polypeptide) corresponds largely to the constant region of the immunoglobulin heavy chain, and is responsible for various functions, including the antibody's effector function(s). The Fc domain contains part or all of a hinge domain of an immunoglobulin molecule plus a CH2 and a CH3 domain. The Fc domain can form a dimer of two polypeptide chains joined by one or more disulfide bonds. In some embodiments, the Fc is a variant Fc that exhibits reduced (e.g. reduced greater than 30%, 40%, 50%, 60%, 70%, 80%, 90% or more) activity to facilitate an effector function. In some embodiments, reference to amino acid substitutions in an Fc region is by EU numbering system unless described with reference to a specific SEQ ID NO. EU numbering is known and is according to the most recently updated IMGT Scientific Chart (IMGT®, the international ImMunoGeneTics information system® http://www.imgt.org/IMGTScientificChart/Numbering/Hu_IGHGnber.html (created: 17 May 2001, last updated: 10 Jan 2013) and the EU index as reported in Kabat, E.A. et al. Sequences of Proteins of Immunological interest. 5th ed. US Department of Health and Human Services, NIH publication No. 91-3242 (1991).

An immunoglobulin Fc fusion ("Fc-fusion"), such as an immunomodulatory Fc fusion protein, is a molecule comprising one or more polypeptides (or one or more small molecules) operably linked to an Fc region of an immunoglobulin. An Fc-fusion may comprise, for example, the Fc region of an antibody (which facilitates effector functions and pharmacokinetics) and a variant ICOSL. An immunoglobulin Fc region may be linked indirectly or directly to one or more variant ICOSL or small molecules (fusion partners). Various linkers are known in the art and can optionally be used to link an Fc to a fusion partner to generate an Fc-fusion. Fc-fusions of identical species can be dimerized to form Fc-fusion homodimers, or using non-identical species to form Fc-fusion heterodimers. In some embodiments, the Fc is a mammalian Fc such as a murine or human Fc. In some embodiments, the Fc is an effectorless Fc.

The term "host cell" refers to a cell that can be used to express a protein encoded by a recombinant expression vector. A host cell can be a prokaryote, for example, E. coli, or it can be a eukaryote, for example, a single-celled eukaryote (e.g., a yeast or other fungus), a plant cell (e.g., a tobacco or tomato plant cell), an animal cell (e.g., a human cell, a monkey cell, a hamster cell, a rat cell, a mouse cell, or an insect cell) or a hybridoma. Examples of host cells include Chinese hamster ovary (CHO) cells or their derivatives such as Veggie CHO and related cell lines which grow in serum-free media or CHO strain DX-B 11, which is deficient in DHFR. In some embodiments, a host cell is a mammalian cell (e.g., a human cell, a monkey cell, a hamster cell, a rat cell, a mouse cell, or an insect cell).

The term "immunoglobulin" (abbreviated "Ig") as used herein refers to a mammalian immunoglobulin protein including any of the five human classes of antibody: IgA (which includes subclasses IgA1 and IgA2), IgD, IgE, IgG (which includes subclasses IgG1, IgG2, IgG3, and IgG4), and IgM. The term is also inclusive of immunoglobulins that are less than full-length, whether wholly or partially synthetic (e.g., recombinant or chemical synthesis) or naturally produced, such as antigen binding fragment (Fab), variable fragment (Fv) containing $V_H$ and $V_L$, the single chain variable fragment (scFv) containing $V_H$ and $V_L$ linked together in one chain, as well as other antibody V region fragments, such as Fab',

$F(ab)_2$, $F(ab')_2$, dsFv diabody, Fc, and Fd polypeptide fragments. Bispecific antibodies, homobispecific and heterobispecific, are included within the meaning of the term.

The term "immunoglobulin superfamily" or "IgSF" as used herein means the group of cell surface and soluble proteins that are involved in the recognition, binding, or adhesion processes of cells. Molecules are categorized as members of this superfamily based on shared structural features with immunoglobulins (i.e., antibodies); they all possess a domain known as an immunoglobulin domain or fold. Members of the IgSF include cell surface antigen receptors, co-receptors and co-stimulatory molecules of the immune system, molecules involved in antigen presentation to lymphocytes, cell adhesion molecules, certain cytokine receptors and intracellular muscle proteins. They are commonly associated with roles in the immune system. Proteins in the immunological synapse are often members of the IgSF. IgSF can also be classified into "subfamilies" based on shared properties such as function. Such subfamilies typically consist of from 4 to 30 IgSF members.

The terms "IgSF domain" or "immunoglobulin domain" or "Ig domain" as used herein refers to a structural domain of IgSF proteins. Ig domains are named after the immunoglobulin molecules. They contain about 70-110 amino acids and are categorized according to their size and function. Ig-domains possess a characteristic Ig-fold, which has a sandwich-like structure formed by two sheets of antiparallel beta strands. Interactions between hydrophobic amino acids on the inner side of the sandwich and highly conserved disulfide bonds formed between cysteine residues in the B and F strands, stabilize the Ig-fold. One end of the Ig domain has a section called the complementarity determining region that is important for the specificity of antibodies for their ligands. The Ig like domains can be classified (into classes) as: IgV, IgC1, IgC2, or IgI. Most Ig domains are either variable (IgV) or constant (IgC). IgV domains with 9 beta strands are generally longer than IgC domains with 7 beta strands. Ig domains of some members of the IgSF resemble IgV domains in the amino acid sequence, yet are similar in size to IgC domains. These are called IgC2 domains, while standard IgC domains are called IgC1 domains. T-cell receptor (TCR) chains contain two Ig domains in the extracellular portion; one IgV domain at the N-terminus and one IgC1 domain adjacent to the cell membrane. ICOSL contains two Ig domains: IgV and IgC.

The term "IgSF species" as used herein means an ensemble of IgSF member proteins with identical or substantially identical primary amino acid sequence. Each mammalian immunoglobulin superfamily (IgSF) member defines a unique identity of all IgSF species that belong to that IgSF member. Thus, each IgSF family member is unique from other IgSF family members and, accordingly, each species of a particular IgSF family member is unique from the species of another IgSF family member. Nevertheless, variation between molecules that are of the same IgSF species may occur owing to differences in post-translational modification such as glycosylation, phosphorylation, ubiquitination, nitrosylation, methylation, acetylation, and lipidation. Additionally, minor sequence differences within a single IgSF species owing to gene polymorphisms constitute another form of variation within a single IgSF species as do wild type truncated forms of IgSF species owing to, for example, proteolytic cleavage. A "cell surface IgSF species" is an IgSF species expressed on the surface of a cell, generally a mammalian cell.

The term "immunological activity" as used herein in the context of mammalian lymphocytes such as T-cells refers to one or more cell survival, cell proliferation, cytokine production (e.g. interferon-gamma), or T-cell cytotoxicity activities. In some cases, an immunological activity can mean the cell expression of cytokines, such as chemokines or interleukins. Assays for determining enhancement or suppression of immunological activity include the MLR (mixed lymphocyte reaction) assays measuring interferon-gamma cytokine levels in culture supernatants (Wang et al., Cancer Immunol Res. 2014 Sep: 2(9):846-56), SEB (staphylococcal enterotoxin B) T cell stimulation assay (Wang et al., Cancer Immunol Res. 2014 Sep: 2(9):846-56), and anti-CD3 T cell stimulation assays (Li and Kurlander, J Transl Med. 2010: 8: 104). Since T cell activation is associated with secretion of IFN-gamma cytokine, detecting IFN-gamma levels in culture supernatants from these in vitro human T cell assays can be assayed using commercial ELISA kits (Wu et al, Immunol Lett 2008 Apr 15; 117(1): 57-62). Induction of an immune response results in an increase in immunological activity relative to quiescent lymphocytes. An immunomodulatory protein, such as a variant ICOSL polypeptide containing an affinity modified IgSF domain, as provided herein can in some embodiments increase or, in alternative embodiments, decrease IFN-gamma (interferon-gamma) expression in a primary T-cell assay relative to a wild-type IgSF member or IgSF domain control. Those of skill will recognize that the format of the primary T-cell assay used to determine an increase in IFN-gamma expression will differ from that employed to assay for a decrease in IFN-gamma expression. In assaying for the ability of an immunomodulatory protein or affinity modified IgSF domain of the invention to decrease IFN-gamma expression in a primary T-cell assay, a Mixed Lymphocyte Reaction (MLR) assay can be used as described in Example 6. Conveniently, a soluble form of an affinity modified IgSF domain of the invention can be employed to determine its ability to antagonize and thereby decrease the IFN-gamma expression in a MLR as likewise described in Example 6. Alternatively, in assaying for the ability of an immunomodulatory protein or affinity modified IgSF domain of the invention to increase IFN-gamma expression in a primary T-cell assay, a co-immobilization assay can be used. In a co-immobilization assay, a T-cell receptor signal, provided in some embodiments by anti-CD3 antibody, is used in conjunction with a co-immobilized affinity modified IgSF domain, such as variant ICOSL, to determine the ability to increase IFN-gamma expression relative to a wild-type IgSF domain control. Methods to assay the immunological activity of engineered cells, including to evaluate the activity of a variant ICOSL transmembrane immunomodulatory protein, are known in the art and include, but are not limited to, the ability to expand T cells following antigen stimulation, sustain T cell expansion in the absence of re- stimulation, and anti-cancer activities in appropriate animal models. Assays also include assays to assess cytotoxicity, including a standard $^{51}$Cr-release assay (see e.g. Milone et al., (2009) Molecular Therapy 17: 1453-1464) or flow based cytotoxicity assays, or an impedance based cytotoxicity assay (Peper et al. (2014) Journal of Immunological Methods, 405:192-198).

An "immunomodulatory polypeptide" or "immunomodulatory protein" is a polypeptide or protein molecule that modulates immunological activity. By "modulation" or "modulating" an immune response is meant that immunological activity is either increased or decreased. An immunomodulatory protein can be a single polypeptide chain or a multimer (dimers or higher order multimers) of at least two polypeptide chains covalently bonded to each other by, for example, interchain disulfide bonds. Thus, monomeric, dimeric, and higher order multimeric polypeptides are within the scope of the defined term. Multimeric polypeptides can be homomultimeric (of identical polypeptide chains) or heteromultimeric (of non-identical polypeptide chains). An immunomodulatory protein of the invention comprises a variant ICOSL.

The term "increase" as used herein means to increase by a statistically significant amount. An increase can be at least 5%, 10%, 20%, 30%, 40%, 50%, 75%, 100%, or greater than a non-zero control value.

An "isoform" of ICOSL (inducible costimulator ligand; CD275) is one of a plurality of naturally occurring ICOSL polypeptides that differ in amino acid sequence. Isoforms can be the product of splice variants of an RNA transcript expressed by a single gene, or the expression product of highly similar but different genes yielding a functionally similar protein such as may occur from gene duplication. As used herein, the term "isoform" of ICOSL also refers to the product of different alleles of an ICOSL gene (e.g., ICOSLG).

The term "lymphocyte" as used herein means any of three subtypes of white blood cell in a mammalian immune system. They include natural killer cells (NK cells) (which function in cell-mediated, cytotoxic innate immunity), T cells (for cell-mediated, cytotoxic adaptive immunity), and B cells (for humoral, antibody-driven adaptive immunity). T cells include: T helper cells, cytotoxic T-cells, natural killer T-cells, memory T-cells, regulatory T-cells, or gamma delta T-cells. Innate lymphoid cells (ILC) are also included within the definition of lymphocyte.

The terms "mammal," or "patient" specifically includes reference to at least one of a: human, chimpanzee, rhesus monkey, cynomolgus monkey, dog, cat, mouse, or rat.

The term "membrane protein" as used herein means a protein that, under physiological conditions, is attached directly or indirectly to a lipid bilayer. A lipid bilayer that forms a membrane can be a biological membrane such as a eukaryotic (e.g., mammalian) cell membrane or an artificial (i.e., man-made) membrane such as that found on a liposome. Attachment of a membrane protein to the lipid bilayer can be by way of covalent attachment, or by way of non-covalent interactions such as hydrophobic or electrostatic interactions. A membrane protein can be an integral membrane protein or a peripheral membrane protein. Membrane proteins that are peripheral membrane proteins are non-covalently attached to the lipid bilayer or non-covalently attached to an integral membrane protein. A peripheral membrane protein forms a temporary attachment to the lipid bilayer such that under the range of conditions that are physiological in a mammal, peripheral membrane protein can associate and/or disassociate from the lipid bilayer. In contrast to peripheral membrane proteins, integral membrane proteins form a substantially permanent attachment to the membrane's lipid bilayer such that under the range of conditions that are physiological in a mammal, integral membrane proteins do not disassociate from their attachment to the lipid bilayer. A membrane protein can form an attachment to the membrane by way of one layer of the lipid bilayer (monotopic), or attached by way of both layers of the membrane (polytopic). An integral membrane protein that interacts with only one lipid bilayer is an "integral monotopic protein". An integral membrane protein that interacts with both lipid bilayers is an "integral polytopic protein" alternatively referred to herein as a "transmembrane protein".

The terms "modulating" or "modulate" as used herein in the context of an immune response, such as a mammalian immune response, refer to any alteration, such as an increase or a decrease, of existing or potential immune responses that occurs as a result of administration of an immunomodulatory polypeptide comprising a variant ICOSL of the present invention or as a result of administration of engineered cells expresses an immunomodulatory protein, such as a variant ICOSL transmembrane immunomodulatory protein of the present invention. Thus, it refers to an alteration, such as an increase or decrease, of an immune response as compared to the immune response that occurs or is present in the absence of the administration of the immunomodulatory protein comprising the variant ICOSL or cells expressing such an immunomodulatory polypeptide. Such modulation includes any induction, activation, suppression or alteration in degree or extent of immunological activity of an immune cell. Immune cells include B cells, T cells, NK (natural killer) cells, NK T cells, professional antigen-presenting cells (APCs), and non-professional antigen-presenting cells, and inflammatory cells (neutrophils, macrophages, monocytes, eosinophils, and basophils). Modulation includes any change imparted on an existing immune response, a developing immune response, a potential immune response, or the capacity to induce, regulate, influence, or respond to an immune response. Modulation includes any alteration in the expression and/or function of genes, proteins and/or other molecules in immune cells as part of an immune response. Modulation of an immune response or modulation of immunological activity includes, for example, the following: elimination, deletion, or sequestration of immune cells; induction or generation of immune cells that can modulate the functional capacity of other cells such as autoreactive lymphocytes, antigen presenting cells, or inflammatory cells; induction of an unresponsive state in immune cells (i.e., anergy); enhancing or suppressing the activity or function of immune cells, including but not limited to altering the pattern of proteins expressed by these cells. Examples include altered production and/or secretion of certain classes of molecules such as cytokines, chemokines, growth factors, transcription factors, kinases, costimulatory molecules, or other cell surface receptors or any combination of these modulatory events. Modulation can be assessed, for example, by an alteration in IFN-gamma (interferon gamma) expression relative to the wild-type ICOSL control in a primary T cell assay (see, Zhao and Ji, Exp Cell Res. 2016 Jan1; 340(1) 132-138). Modulation can be assessed, for example, by an alteration of an immunological activity of engineered cells, such as an alteration in in cytotoxic activity of engineered cells or an alteration in cytokine secretion of engineered cells relative to cells engineered with a wild-type ICOSL transmembrane protein.

The term "molecular species" as used herein means an ensemble of proteins with identical or substantially identical primary amino acid sequence. Each mammalian immunoglobulin superfamily (IgSF) member defines a collection of identical or substantially identical molecular species. Thus, for example, human ICOSL is an IgSF member and each human ICOSL molecule is a molecule species of ICOS. Variation between molecules that are of the same molecular species may occur owing to differences in post-translational modification such as glycosylation, phosphorylation, ubiquitination, nitrosylation, methylation, acetylation, and lipidation. Additionally, minor sequence differences within a single molecular species owing to gene polymorphisms constitute another form of variation within a single molecular species as do wild type truncated forms of a single molecular species owing to, for example, proteolytic cleavage. A "cell surface molecular species" is a molecular species expressed on the surface of a mammalian cell. Two or more different species of protein, each of which is present exclusively on one or exclusively the other (but not both) of the two mammalian cells forming the IS, are said to be in "cis" or "cis configuration" with each other. Two different species of protein, the first of which is exclusively present on one of the two mammalian cells forming the IS and the second of which is present exclusively on the second of the two mammalian cells forming the IS, are said to be in "trans" or "trans configuration." Two different species of protein each of which is present on both of the two mammalian cells forming the IS are in both cis and trans configurations on these cells.

The term, a "multimerization domain" refers to a sequence of amino acids that promotes stable interaction of a polypeptide molecule with one or more additional polypeptide molecules, each containing a complementary multimerization domain (e.g. a first multimerization domain and a second multimerization domain), which can be the same or a different multimerization domain. The interactions between complementary multimerization domains, e.g. interaction between a first multimerization domain and a second multimerization domain, form a stable protein-protein interaction to produce a multimer of the polypeptide molecule with the additional polypeptide molecule. In some cases, the multimerization domain is the same and interacts with itself to form a stable protein-protein interaction between two polypeptide chains. Generally, a polypeptide is joined directly or indirectly to the multimerization domain. Exemplary multimerization domains include the immunoglobulin sequences or portions thereof, leucine zippers, hydrophobic regions, hydrophilic regions, and compatible protein-protein interaction domains. The multimerization domain, for example, can be an immunoglobulin constant region or domain, such as, for example, the Fc domain or portions thereof from IgG, including IgG1, IgG2, IgG3 or IgG4 subtypes, IgA, IgE, IgD and IgM and modified forms thereof.

The terms "nucleic acid" and "polynucleotide" are used interchangeably to refer to a polymer of nucleic acid residues (e.g., deoxyribonucleotides or ribonucleotides) in either single- or double-stranded form. Unless specifically limited, the terms encompass nucleic acids containing known analogues of natural nucleotides and that have similar binding properties to it and are metabolized in a manner similar to naturally-occurring nucleotides. Unless otherwise indicated, a particular nucleic acid sequence also implicitly encompasses conservatively modified variants thereof (e.g., degenerate codon substitutions) and complementary nucleotide sequences as well as the sequence explicitly indicated (a "reference sequence"). Specifically, degenerate codon substitutions may be achieved by generating sequences in which the third position of one or more selected (or all) codons is substituted with mixed-base and/or deoxyinosine residues. The term nucleic acid or polynucleotide encompasses cDNA or mRNA encoded by a gene.

The term "non-competitive binding" as used herein means the ability of a protein to specifically bind simultaneously to at least two cognate binding partners. Thus, the protein is able to bind to at least two different cognate binding partners at the same time, although the binding interaction need not be for the same duration such that, in some cases, the protein is specifically bound to only one of the cognate binding partners. In some embodiments, the binding occurs under specific binding conditions. In some embodiments, the simultaneous binding is such that binding of one cognate binding partner does not substantially inhibit simultaneous binding to a second cognate binding partner. In some embodiments, non-competitive binding means that binding a second cognate binding partner to its binding site on the protein does not displace the binding of a first cognate binding partner to its binding site on the protein. Methods of assessing non-competitive binding are well known in the art such as the method described in Perez de La Lastra et al., Immunology, 1999 Apr: 96(4): 663-670. In some cases, in non-competitive interactions, the first cognate binding partner specifically binds at an interaction site that does not overlap with the interaction site of the second cognate binding partner such that binding of the second cognate binding partner does not directly interfere with the binding of the first cognate binding partner. Thus, any effect on binding of the cognate binding partner by the binding of the second cognate binding partner is through a mechanism other than direct interference with the binding of the first cognate binding partner. For example, in the context of enzyme-substrate interactions, a non-competitive inhibitor binds to a site other than the active site of the enzyme. Non-competitive binding encompasses uncompetitive binding interactions in which a second cognate binding partner specifically binds at an interaction site that does not overlap with the binding of the first cognate binding partner but binds to the second interaction site only when the first interaction site is occupied by the first cognate binding partner.

The term "pharmaceutical composition" refers to a composition suitable for pharmaceutical use in a mammalian subject, often a human. A pharmaceutical composition typically comprises an effective amount of an active agent (e.g., an immunomodulatory polypeptide comprising a variant ICOSL or engineered cells expressing a variant ICOSL transmembrane immunomodulatory protein) and a carrier, excipient, or diluent. The carrier, excipient, or diluent is typically a pharmaceutically acceptable carrier, excipient or diluent, respectively.

The terms "polypeptide" and "protein" are used interchangeably herein and refer to a molecular chain of two or more amino acids linked through peptide bonds. The terms do not refer to a specific length of the product. Thus, "peptides," and "oligopeptides," are included within the definition of polypeptide. The terms include post-translational modifications of the polypeptide, for example, glycosylations, acetylations, phosphorylations and the like. The terms also include molecules in which one or more amino acid analogs or non-canonical or unnatural amino acids are included as can be synthesized, or expressed recombinantly using known protein engineering techniques. In addition, proteins can be derivatized.

The term "primary T-cell assay" as used herein refers to an in vitro assay to measure interferon-gamma ("IFN-gamma") expression. The assay used can be an anti-CD3 coimmobilizaton assay. In this assay, primary T cells are stimulated by anti-CD3 immobilized with or without additional recombinant proteins. Culture supernatants are harvested at timepoints, usually 24-72 hours. In another embodiment, the assay used is the MLR. In this assay, primary T cells are stimulated with allogeneic APC. Culture supernatants are harvested at timepoints, usually 24-72 hours. Human IFN-gamma levels are measured in culture supernatants by standard ELISA techniques. Commercial kits are available from vendors and the assay is performed according to manufacturer's recommendation.

The term "purified" as applied to nucleic acids, such as encoding immunomodulatory proteins of the invention, generally denotes a nucleic acid or polypeptide that is substantially free from other components as determined by analytical techniques well known in the art (e.g., a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or protein of the invention is at least about 50% pure, usually at least about 75%, 80%, 85%, 90%, 95%, 96%, 99% or more pure (e.g., percent by weight or on a molar basis).

The term "recombinant" indicates that the material (e.g., a nucleic acid or a polypeptide) has been artificially (i.e., non-naturally) altered by human intervention. The alteration can be performed on the material within, or removed from, its natural environment or state. For example, a "recombinant nucleic acid" is one that is made by recombining nucleic acids, e.g., during cloning, affinity modification, DNA shuffling or other well-known molecular biological procedures. A "recombinant DNA molecule," is comprised of segments of DNA joined together by means of such molecular biological techniques. The term "recombinant protein" or "recombinant polypeptide" as used herein refers to a protein molecule which is expressed using a recombinant DNA molecule. A "recombinant host cell" is a cell that contains and/or expresses a recombinant nucleic acid or that is otherwise altered by genetic engineering, such as by introducing into the cell a nucleic acid molecule encoding a recombinant protein, such as a transmembrane immunomodulatory protein provided herein. Transcriptional control signals in eukaryotes comprise "promoter" and "enhancer" elements. Promoters and enhancers consist of short arrays of DNA sequences that interact specifically with cellular proteins involved in transcription. Promoter and enhancer elements have been isolated from a variety of eukaryotic sources including genes in yeast, insect and mammalian cells and viruses (analogous control elements, i.e., promoters, are also found in prokaryotes). The selection of a particular promoter and enhancer depends on what cell type is to be used to express the protein of interest. The terms "in operable combination," "in operable order" and "operably linked" as used herein refer to the linkage of nucleic acid sequences in such a manner or orientation that a nucleic acid molecule capable of directing the transcription of a given gene and/or the synthesis of a desired protein molecule is produced.

The term "recombinant expression vector" as used herein refers to a DNA molecule containing a desired coding sequence and appropriate nucleic acid sequences necessary for the expression of the operably linked coding sequence in a particular host cell. Nucleic acid sequences necessary for expression in prokaryotes include a promoter, optionally an operator sequence, a ribosome binding site and possibly other sequences. Eukaryotic cells are known to utilize promoters, enhancers, and termination and polyadenylation signals. A secretory signal peptide sequence can also, optionally, be encoded by the recombinant expression vector, operably linked to the coding sequence for the recombinant protein, such as a recombinant fusion protein, so that the expressed fusion protein can be secreted by the recombinant host cell, for easier isolation of the fusion protein from the cell, if desired. The term includes the vector as a self-replicating nucleic acid structure as well as the vector incorporated into the genome of a host cell into which it has been introduced. Among the vectors are viral vectors, such as lentiviral vectors.

The term "selectivity" refers to the preference of a subject protein, or polypeptide, for specific binding of one substrate, such as one cognate binding partner, compared to specific binding for another substrate, such as a different cognate binding partner of the subject protein. Selectivity can be reflected as a ratio of the binding activity (e.g. binding affinity) of a subject protein and a first substrate, such as a first cognate binding partner, (e.g., $K_{d1}$) and the binding activity (e.g. binding affinity) of the same subject protein with a second cognate binding partner (e.g., $K_{d2}$).

The term "sequence identity" as used herein refers to the sequence identity between genes or proteins at the nucleotide or amino acid level, respectively. "Sequence identity" is a measure of identity between proteins at the amino acid level and a measure of identity between nucleic acids at nucleotide level. The protein sequence identity may be determined by comparing the amino acid sequence in a given position in each sequence when the sequences are aligned. Similarly, the nucleic acid sequence identity may be determined by comparing the nucleotide sequence in a given position in each sequence when the sequences are aligned. Methods for the alignment of sequences for comparison are well known in the art, such methods include GAP, BESTFIT, BLAST, FASTA and TFASTA. The BLAST algorithm calculates percent sequence identity and performs a statistical analysis of the similarity between the two sequences. The software for performing BLAST analysis is publicly available through the National Center for Biotechnology Information (NCBI) website.

The term "soluble" as used herein in reference to proteins, means that the protein is not a membrane protein. In general, a soluble protein contains only the extracellular domain of an IgSF family member receptor, or a portion thereof containing an IgSF domain or domains or specific-binding fragments thereof, but does not contain the transmembrane domain and/or is not capable of being expressed on the surface of a cell. In some cases, solubility of a protein can be improved by linkage or attachment, directly or indirectly via a linker, to an Fc domain, which, in some cases, also can improve the stability and/or half-life of the protein. In some aspects, a soluble protein is an Fc fusion protein.

The term "species" as used herein with respect to polypeptides or nucleic acids means an ensemble of molecules with identical or substantially identical sequences. Variation between polypeptides that are of the same species may occur owing to differences in post-translational modification such as glycosylation, phosphorylation, ubiquitination, nitrosylation, methylation, acetylation, and lipidation. Slightly truncated sequences of polypeptides that differ (or encode a difference) from the full length species at the amino-terminus or carboxy-terminus by no more than 1. 2, or 3 amino acid residues are considered to be of a single species. Such microheterogeneities are a common feature of manufactured proteins.

The term "specific binding fragment" as used herein in reference to a full-length wild-type mammalian ICOSL polypeptide or an IgV or an IgC domain thereof, means a polypeptide having a subsequence of an IgV and/or IgC domain and that specifically binds in vitro and/or in vivo to a mammalian ICOS and/or mammalian CD28 such as a human or murine ICOS or CD28. In some embodiments, the specific binding fragment of ICOSL IgV or ICOSL IgC is at at least 60%, 70%, 75%, 80%, 85%, 90%, 95%, 96%, 97%, 98%, or 99% the sequence length of the full-length wild-type sequence. The specific binding fragment can be altered in sequence to form a variant ICOSL of the invention.

The term "specifically binds" as used herein means the ability of a protein, under specific binding conditions, to bind to a target protein such that its affinity or avidity is at least 5 times as great, but optionally at least 10, 20, 30, 40, 50, 100, 250 or 500 times as great, or even at least 1000 times as great as the average affinity or avidity of the same protein to a collection of random peptides or polypeptides of sufficient statistical size. A specifically binding protein need not bind exclusively to a single target molecule but may specifically bind to a non-target molecule due to similarity in structural conformation between the target and non-target (e.g., paralogs or orthologs). Those of skill will recognize that specific binding to a molecule having the same function in a different species of animal (i.e., ortholog) or to a non-target molecule having a substantially similar epitope as the target molecule (e.g., paralog) is possible and does not detract from the specificity of binding which is determined relative to a statistically valid collection of unique non-targets (e.g., random polypeptides). Thus, a polypeptide of the invention may specifically bind to more than one distinct species of target molecule due to cross-reactivity. Solid-phase ELISA immunoassays or Biacore measurements can be used to determine specific binding between two proteins. Generally, interactions between two binding proteins have dissociation constants ($K_d$) less than $1\times10^{-5}$ M, and often as low as $1 \times 10^{-12}$ M. In certain embodiments of the present disclosure, interactions between two binding proteins have dissociation constants of $1\times10^{-6}$ M, $1\times10^{-7}$ M, $1\times10^{-8}$ M, $1\times10^{-9}$ M, $1\times10^{-10}$ M or $1\times10^{-11}$ M.

The terms "surface expresses", "surface expression" or "expressed on the surface" in reference to a mammalian cell expressing a polypeptide means that the polypeptide is expressed as a membrane protein. In some embodiments, the membrane protein is a transmembrane protein.

As used herein, "synthetic," with reference to, for example, a synthetic nucleic acid molecule or a synthetic gene or a synthetic peptide refers to a nucleic acid molecule or polypeptide molecule that is produced by recombinant methods and/or by chemical synthesis methods.

The term "targeting moiety" as used herein refers to a composition that is covalently or non-covalently attached to, or physically encapsulates, a polypeptide comprising a variant ICOSL of the present invention. In some embodiments, the targeting moiety has specific binding affinity for a target molecule such as a target molecule expressed on a cell. Typically, the target molecule is localized on a specific tissue or cell-type. Targeting moieties include: antibodies, antigen binding fragment (Fab), variable fragment (Fv) containing $V_H$ and $V_L$, the single chain variable fragment (scFv) containing $V_H$ and $V_L$ linked together in one chain, as well as other antibody V region fragments, such as Fab', $F(ab)_2$, $F(ab')_2$, dsFv diabody, nanobodies, soluble receptors, receptor ligands, affinity matured receptors or ligands, as well as small molecule (<500 dalton) compositions (e.g., specific binding receptor compositions). Targeting moieties can also be attached covalently or non-covalently to the lipid membrane of liposomes that encapsulate a polypeptide of the present invention.

The term "transmembrane protein" as used herein means a membrane protein that substantially or completely spans a lipid bilayer such as those lipid bilayers found in a biological membrane such as a mammalian cell, or in an artificial construct such as a liposome. The transmembrane protein comprises a transmembrane domain ("transmembrane domain") by which it is integrated into the lipid bilayer and by which the integration is thermodynamically stable under physiological conditions. Transmembrane domains are generally predictable from their amino acid sequence via any number of commercially available bioinformatics software applications on the basis of their elevated hydrophobicity relative to regions of the protein that interact with aqueous environments (e.g., cytosol, extracellular fluid). A transmembrane domain is often a hydrophobic alpha helix that spans the membrane. A transmembrane protein can pass through the both layers of the lipid bilayer once or multiple times. A transmembrane protein includes the provided transmembrane immunomodulatory proteins described herein. In addition to the transmembrane domain, a transmembrane immunomodulatory protein of the invention further comprises an ectodomain and, in some embodiments, an endodomain.

The terms "treating," "treatment," or "therapy" of a disease or disorder as used herein mean slowing, stopping or reversing the disease or disorders progression, as evidenced by decreasing, cessation or elimination of either clinical or diagnostic symptoms, by administration of a therapeutic composition (e.g. containing an immunomodulatory protein or engineered cells) of the invention either alone or in combination with another compound as described herein. "Treating," "treatment," or "therapy" also means a decrease in the severity of symptoms in an acute or chronic disease or disorder or a decrease in the relapse rate as for example in the case of a relapsing or remitting autoimmune disease course or a decrease in inflammation in the case of an inflammatory aspect of an autoimmune disease. As used herein in the context of cancer, the terms "treatment" or, "inhibit," "inhibiting" or "inhibition" of cancer refers to at least one of: a statistically significant decrease in the rate of tumor growth, a cessation of tumor growth, or a reduction in the size, mass, metabolic activity, or volume of the tumor, as measured by standard criteria such as, but not limited to, the Response Evaluation Criteria for Solid Tumors (RECIST), or a statistically significant increase in progression free survival (PFS) or overall survival (OS). "Preventing," "prophylaxis," or "prevention" of a disease or disorder as used in the context of this invention refers to the administration of an immunomodulatory polypeptide or engineered cells of the invention, either alone or in combination with another compound, to prevent the occurrence or onset of a disease or disorder or some or all of the symptoms of a disease or disorder or to lessen the likelihood of the onset of a disease or disorder.

The term "tumor specific antigen" or "TSA" as used herein refers to a counter-structure that is present primarily on tumor cells of a mammalian subject but generally not found on normal cells of the mammalian subject. A tumor specific antigen need not be exclusive to tumor cells but the percentage of cells of a particular mammal that have the tumor specific antigen is sufficiently high or the levels of the tumor specific antigen on the surface of the tumor are sufficiently high such that it can be targeted by anti-tumor therapeutics, such as immunomodulatory polypeptides of the invention, and provide prevention or treatment of the mammal from the effects of the tumor. In some embodiments, in a random statistical sample of cells from a mammal with a tumor, at least 50% of the cells displaying a TSA are cancerous. In other embodiments, at least 60%, 70%, 80%, 85%, 90%, 95%, or 99% of the cells displaying a TSA are cancerous.

The term "variant" (also "modified" or mutant") as used in reference to a variant ICOSL means an ICOSL, such as a mammalian (e.g., human or murine) ICOSL created by human intervention. The variant ICOSL is a polypeptide having an altered amino acid sequence, relative to a reference (e.g. unmodified) or wild-type ICOSL. The variant ICOSL is a polypeptide which differs from a reference ICOSL, such as a wild-type ICOSL isoform sequence, by one or more modifications, such as one or more amino acid substitutions, deletions, additions, or combinations thereof. For purposes herein, the variant ICOSL contains at least one affinity modified domain, whereby one or more of the amino acid differences occurs in an IgSF domain (e.g. IgV domain). A variant ICOSL can contain 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more amino acid differences, such as amino acid substitutions. A variant ICOSL polypeptide generally exhibits at least 50%, 60%, 70%, 80%, 85%, 86%, 87%, 88%, 89%, 90%, 91%, 92%, 93%, 94%, 95%, 96%, 97%, 98%, 99% or more sequence identity to a corresponding reference (e.g. unmodified ICOSL) or wild-type, such as to the sequence of SEQ ID NO:4, a mature sequence thereof or a portion thereof containing the extracellular domain or an IgSF domain thereof. In some embodiments, a variant ICOSL polypeptide exhibits at least 50%, 60%, 70%, 80%, 85%, 86%, 87%, 88%, 89%, 90%, 91%, 92%, 93%, 94%, 95%, 96%, 97%, 98%, 99% or more sequence identity to a corresponding reference (e.g. unmodified) or wild-type ICOSL, such as a reference ICOSL set forth in SEQ ID NO:1 or SEQ ID NOs: 2 or 3. Non-naturally occurring amino acids as well as naturally occurring amino acids are included within the scope of permissible substitutions or additions. A variant ICOSL is not limited to any particular method of making and includes, for example, de novo chemical synthesis, de novo recombinant DNA techniques, or combinations thereof. A variant ICOSL of the invention specifically binds to CD28, ICOS, and/or CTLA-4 of a mammalian species. In some embodiments, the altered amino acid sequence results in an an altered (i.e., increased or decreased) binding affinity or avidity to ICOS and/or CD28 compared to the reference (e.g. unmodified) or wild-type ICOSL protein. An increase or decrease in binding affinity or avidity can be determined using well known binding assays such as flow cytometry. Larsen et al., American Journal of Transplantation, Vol 5: 443-453 (2005). See also, Linsley et al., Immunity, Vol. 1(9): 793-801 (1994). An increase in variant ICOSL binding affinity or avidity to ICOS and/or CD28 is to a value at least 5% greater than that of the reference (e.g. unmodified) or wild-type ICOSL and in some embodiments, at least 10%, 15%, 20%, 30%, 40%, 50%, 100% greater than that of the reference (e.g. unmodified) or wild-type ICOSL control value. A decrease in ICOSL binding affinity or avidity to ICOS and/or CD28 is to a value no greater than 95% of the of the wild-type control values, and in some embodiments no greater than 80%, 70% 60%, 50%, 40%, 30%, 20%, 10%, 5%, or no detectable binding affinity or avidity of the wild-type ICOS and/or CD28 control values. A variant ICOSL is altered in primary amino acid sequence by substitution, addition, or deletion of amino acid residues. The term "variant" in the context of variant ICOSL is not be construed as imposing any condition for any particular starting composition or method by which the variant ICOSL is created. A variant ICOSL can, for example, be generated starting from a reference ICOSL or wild type mammalian ICOSL sequence information, then modeled in silico for binding to ICOS and/or CD28, and finally recombinantly or chemically synthesized to yield a variant ICOSL of the present invention. In but one alternative example, a variant ICOSL can be created by site-directed mutagenesis of a reference (e.g. unmodified) or wild-type ICOSL. Thus, variant ICOSL denotes a composition and not necessarily a product produced by any given process. A variety of techniques including recombinant methods, chemical synthesis, or combinations thereof, may be employed.

The term "wild-type" or "natural" or "native" as used herein is used in connection with biological materials such as nucleic acid molecules, proteins (e.g., ICOSL), IgSF members, host cells, and the like, refers to those which are found in nature and not modified by human intervention.

Further, various embodiments of the invention as discussed below are frequently provided within the meaning of a defined term as disclosed above. The embodiments described in a particular definition are therefore to be interpreted as being incorporated by reference when the defined term is utilized in discussing the various aspects and attributes described herein. Thus, the headings, the order of presentation of the various aspects and embodiments, and the separate disclosure of each independent attribute is not meant to be a limitation to the scope of the present disclosure.

## II. VARIANT ICOSL POLYPEPTIDE FUSION PROTEINS

Provided herein are fusion proteins containing a variant ICOSL polypeptide and a multimerization domain, such as an Fc domain. In some embodiments, the fusion proteins containing the variant ICOSL polypeptides exhibit altered (e.g. increased) binding activity or affinity for one or more of an ICOSL cognate binding partner. In some embodiments, the ICOSL cognate binding partner is one or both of CD28 or ICOS.

### A. Variant ICOSL Polypeptides

In some embodiments, the variant ICOSL polypeptide contains one or more amino acids modifications, such as one or more substitutions (alternatively, "mutations" or "replacements"), deletions or addition, in an immunoglobulin superfamily (IgSF) domain (IgD) relative to a wild-type or unmodified ICOSL polypeptide or a portion of a wild-type or unmodified ICOSL containing an immunoglobulin superfamily (IgSF) domain or a specific binding fragment thereof. Thus, a provided variant ICOSL polypeptide is or comprises a variant IgD (hereinafter called "vIgD") in which the one or more amino acid modifications (e.g. substitutions) is in an IgD.

In some embodiments, the IgD comprises an IgV domain or an IgC (e.g. IgC2) domain or specific binding fragment of the IgV domain or the IgC (e.g. IgC2) domain, or combinations thereof. In some embodiments, the IgD can be an IgV only, the combination of the IgV and IgC, including the entire extracellular domain (ECD), or any combination of Ig domains of ICOSL. Table 1 provides exemplary residues that correspond to IgV or IgC regions of ICOSL. In some embodiments, the variant ICOSL polypeptide contains an IgV domain or an IgC domain or specific binding fragments thereof in which the at least one of the amino acid modifications (e.g. substitutions) is in the IgV domain or IgC domain or a specific binding fragment thereof. In some embodiments, by virtue of the altered binding activity or affinity, the IgV domain or IgC domain is an affinity-modified IgSF domain.

Provided herein are variant ICOSL polypeptides containing at least one affinity-modified IgSF domain (e.g., IgV or IgC) or a specific binding fragment thereof in an IgSF domain contained in a reference (e.g., unmodified) or wild-type ICOSL polypeptide such that the variant ICOSL polypeptide exhibits altered (e.g. increased) binding activity or affinity for one or more ligands ICOS and/or CD28 compared to a reference (e.g., unmodified) or wild-type ICOSL polypeptide. In some embodiments, a variant ICOSL polypeptide has a binding affinity for CD28 and/or ICOS that differs from that of a reference (e.g., unmodified) or wild-type ICOSL polypeptide control sequence as determined by, for example, solid-phase ELISA immunoassays, flow cytometry or Biacore assays. In some embodiments, the variant ICOSL polypeptide has an increased binding affinity for CD28 and/or ICOS, relative to a reference (unmodified) or wild-type ICOSL polypeptide. The CD28 and/or ICOS can be a mammalian protein, such as a human protein or a murine protein.

Binding affinities for each of the cognate binding partners are independent; that is, in some embodiments, a variant ICOSL polypeptide has an increased binding affinity for one or both of CD28 and/or ICOS, relative to a reference (e.g., unmodified) or wild-type ICOSL polypeptide. In some embodiments, a variant ICOSL polypeptide has an increased binding affinity for both of CD28 and ICOS, relative to a reference (e.g., unmodified) or wild-type ICOSL polypeptide.

In some embodiments, the variant ICOSL polypeptide has an increased binding affinity for CD28, relative to a reference (e.g., unmodified) or wildtype ICOSL polypeptide. In some embodiments, the variant ICOSL polypeptide has an increased binding affinity for ICOS, relative to a reference (e.g., unmodified) or wild-type ICOSL polypeptide. In some embodiments, the variant ICOSL polypeptide has an increased binding affinity for CD28 and ICOS, relative to a reference (e.g., unmodified) or wild-type ICOSL polypeptide.

In some embodiments, a variant ICOSL polypeptide with increased or greater binding affinity to CD28 and/or ICOS will have an increase in binding affinity relative to the reference (e.g., unmodified) or wild-type ICOSL polypeptide control of at least about 5%, such as at least about 10%, 15%, 20%, 25%, 35%, or 50% for the CD28 and/or ICOS. In some embodiments, the increase in binding affinity relative to the reference (e.g., unmodified) or wild-type ICOSL polypeptide is more than 1.2-fold, 1.5-fold, 2-fold, 3-fold, 4-fold, 5-fold, 6-fold, 7-fold, 8-fold, 9-fold, 10-fold, 20-fold, 30-fold 40-fold or 50-fold. In such examples, the reference (e.g., unmodified) or wild-type ICOSL polypeptide has the same sequence as the variant ICOSL polypeptide except that it does not contain the one or more amino acid modifications (e.g., substitutions).

In some embodiments, the equilibrium dissociation constant ($K_d$) of any of the foregoing embodiments to CD28 and/or ICOS can be less than $1\times10^{-5}$ M, $1\times10^{-6}$ M, $1\times10^{-7}$ M, $1\times10^{-8}$ M, $1\times10^{-9}$ M, $1\times10^{-10}$ M or $1\times10^{-11}$M, or $1\times10^{-12}$ M.

In some embodiments, a variant ICOSL polypeptide has an increased or greater binding affinity to CD28. In some embodiments, a variant ICOSL polypeptide with increased or greater binding affinity to CD28 will have an increase in binding affinity relative to the reference (e.g., unmodified) or wild-type ICOSL polypeptide control of at least about 25%, such as at least about 30%, 40%, 50%, or 60% for CD28. In some embodiments, a variant ICOSL polypeptide with increased or greater binding affinity to CD28 has an equilibrium dissociation constant ($K_d$) of less than 200 pM, 300 pM, 400 pM, 500 pM, or 600 pM for CD28.

In some embodiments, a variant ICOSL polypeptide has an increased or greater binding affinity to ICOS. In some embodiments, a variant ICOSL polypeptide with increased or greater binding affinity to ICOS will have an increase in binding affinity relative to the reference (e.g., unmodified) or wild-type ICOSL polypeptide control of at least about 10%, such as at least about 15%, 20%, 25%, 30%, 40%, 50%, or 60% for CD28. In some embodiments, a variant ICOSL polypeptide with increased or greater binding affinity to ICOS has an equilibrium dissociation constant ($K_d$) of less than 200 pM, 300 pM, 400 pM, 500 pM, or 600 pM for ICOS.

In some embodiments, the variant ICOSL polypeptide has one or more amino acid modification, e.g. substitution in a reference (e.g., unmodified) or wild-type ICOSL sequence. The one or more amino acid modification, e.g. substitution, can be in the ectodomain (extracellular domain) of the reference (e.g., unmodified) or wild-type ICOSL sequence. In some embodiments, the one or more amino acid modification, e.g. substitution is in the IgV domain or specific binding fragment thereof.

In some embodiments, the variant ICOSL polypeptide has one or more amino acid modification, e.g. substitution in a reference ICOSL or specific binding fragment thereof corresponding to position(s) 52, 57, and/or 100, with reference to numbering of SEQ ID NO:1. In some embodiments, the variant ICOSL polypeptide has one or more amino acid modification, e.g. substitution, selected from N52H, N57Y and/or Q100R, or a conservative amino acid modification, e.g. substitution thereof, with reference to numbering of SEQ ID NO:1.

A conservative amino acid modification, e.g. substitution is any amino acid that falls in the same class of amino acids as the substituted amino acids, other than the reference (e.g., unmodified) or wild-type amino acid. The classes of amino acids are aliphatic (glycine, alanine, valine, leucine, and isoleucine), hydroxyl or sulfur-containing (serine, cysteine, threonine, and methionine), cyclic (proline), aromatic (phenylalanine, tyrosine, tryptophan), basic (histidine, lysine, and arginine), and acidic/amide (aspartate, glutamate, asparagine, and glutamine).

In some embodiments, the one or more amino acid modification, e.g. substitution is N52H/N57Y/Q100R, with reference to numbering of SEQ ID NO:1.

Unless stated otherwise, as indicated throughout the present disclosure, the amino acid modification (s) are designated by amino acid position number corresponding to the numbering of positions of the reference ECD sequence set forth in SEQ ID NO:1. It is within the level of a skilled artisan to identify the corresponding position of a modification, e.g. amino acid substitution, in an ICOSL polypeptide, including portion thereof containing an IgSF domain (e.g. IgV) thereof, such as by alignment of a reference sequence (e.g. SEQ ID NO:2, 3, 5-33) with SEQ ID NO:1. In the listing of modifications throughout this disclosure, the amino acid position is indicated in the middle, with the corresponding reference (e.g. unmodified or wild-type) amino acid listed before the number and the identified variant amino acid substitution listed after the number. If the modification is a deletion of the position a "del" is indicated and if the modification is an insertion at the position an "ins" is indicated. In some cases, an insertion is listed with the amino acid position indicated in the middle, with the corresponding reference amino acid listed before and after the number and the identified variant amino acid insertion listed after the unmodified (e.g. wild-type) amino acid.

In some embodiments, the variant is modified in one more IgSF domains relative to the sequence of a reference (e.g., unmodified) ICOSL sequence. In some embodiments, the reference (e.g., unmodified) ICOSL sequence is a wild-type ICOSL. In some embodiments, the reference (e.g., unmodified) or wild-type ICOSL has the sequence of a native ICOSL or an ortholog thereof. In some embodiments, the reference (e.g., unmodified) or wild-type ICOSL is or comprises the extracellular domain (ECD) of ICOSL or a portion thereof containing one or more IgSF domain (see Table 1). In some embodiments, the extracellular domain of a reference (e.g., unmodified) or wild-type ICOSL polypeptide comprises an IgV domain and an IgC domain or domains. However, the variant ICOSL polypeptide need not comprise both the IgV domain and the IgC domain or domains. In some embodiments, the variant ICOSL polypeptide comprises or consists essentially of the IgV domain or a specific binding fragment thereof. In some embodiments, the variant ICOSL is soluble and lacks a transmembrane domain.

In some embodiments, the reference (e.g., unmodified) or wild-type ICOSL sequence is a mammalian ICOSL sequence. In some embodiments, the reference (e.g., unmodified) or wild-type ICOSL sequence can be a mammalian ICOSL that includes, but is not limited to, human, mouse, cynomolgus monkey, or rat. In some embodiments, the reference (e.g., unmodified) or wildtype ICOSL sequence is human.

Various features of a reference (e.g., unmodified) or wild-type ICOSL sequence are set forth in Table 1. The first column of Table 1 provides the name and, optionally, the name of some possible synonyms for that particular IgSF member. The second column provides the protein identifier of the UniProtKB database, a publicly available database accessible via the internet at uniprot.org or, in some cases, the GenBank Number. The Universal Protein Resource (UniProt) is a comprehensive resource for protein sequence and annotation data. The UniProt databases include the UniProt Knowledgebase (UniProtKB). UniProt is a collaboration between the European Bioinformatics Institute (EMBL-EBI), the SIB Swiss Institute of Bioinformatics and the Protein Information Resource (PIR) and supported mainly by a grant from the U.S. National Institutes of Health (NIH). GenBank is the NIH genetic sequence database, an annotated collection of all publicly available DNA sequences (Nucleic Acids Research, 2013 Jan;41(D1):D36-42). The third column provides the region where the indicated IgSF domain is located. The region is specified as a range where the domain is inclusive of the residues defining the range. Column 3 also indicates the IgSF domain class for the specified IgSF region. Column 4 provides the region where the indicated additional domains are located (signal peptide, S; extracellular domain, E; transmembrane domain, T; cytoplasmic domain, C). It is understood that description of domains can vary depending on the methods used to identify or classify the domain, and may be identified differently from different sources. The description of residues corresponding to a domain in Table 1 is for exemplification only and can be several amino acids (such as one, two, three or four) longer or shorter. Column 5 indicates some of its cognate cell surface binding partners.

**TABLE 1. IgSF members according to the present disclosure.**

| IgSF Member (Synonyms) | NCBI Protein Accession Number/ UniProtKB Protein Identifier | IgSF Region & Domain Class | Other Domains | Cognate Cell Surface Binding Partners | IgSF Member Amino Acid Sequence (SEQ ID NO) | | |
|---|---|---|---|---|---|---|---|
| | | | | | Precursor (mature residues) | Mature | ECD |
| ICOSLG (B7RP1, CD275, ICOSL, B7-H2) | 075144.2 | 19-129 IgV, 141-227 IgC2 | S: 1-18, E: 19-256, T: 257-277, C: 278-302 | ICOS, CD28, CTLA4 | SEQ ID NO: 4 (19-302) | SEQ ID NO: 68 | SEQ ID NO: 1 |

In some embodiments, the reference (e.g., unmodified) or wild-type ICOSL sequence has (i) the sequence of amino acids set forth in SEQ ID NO:4 or a mature form thereof lacking the signal sequence, (ii) a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO:4 or the mature form thereof, or (iii) is a portion of (i) or (ii) containing an IgV domain or IgC domain or specific binding fragments thereof.

In some embodiments, the reference ICOSL sequence is or comprises an extracellular domain of the ICOSL or a portion thereof. In some embodiments, the reference or wild-type ICOSL polypeptide comprises the amino acid sequence set forth in SEQ ID NO:1, or an ortholog thereof.

DTQEKEVRAMVGSDVELSCACPEGSRFDLNDVYVYWQTSESKTVVTYHIPQNSSLEN
VDSRYRNRALMSPAGMLRGDFSLRLFNVTPQDEQKFHCLVLSQSLGFQEVLSVEVTL
HVAANFSVPVVSAPHSPSQDELTFTCTSINGYPRPNVYWINKTDNSLLDQALQNDTVF
LNMRGLYDVVSVLRIARTPSVNIGCCIENVLLQQNLTVGSQTGNDIGERDKITENPVS
TGEKNAAT (SEQ ID NO:1)

In some cases, the reference (e.g., unmodified) or wild-type ICOSL polypeptide can comprise (i) the sequence of amino acids set forth in SEQ ID NO:1, (ii) a sequence of amino acids that has at least about 85%, 86%, 87%, 88%, 89%, 90%, 91%, 92%, 93%, 94%, 95%, 96%, 97%, 98%, 99% sequence identity to SEQ ID NO: 1, or (iii) is a specific binding fragment of the sequence of(i) or (ii) comprising an IgV domain or an IgC domain.

In some embodiments, the reference ICOSL polypeptide comprises a truncated extracellular domain comprising a C-terminal truncation with reference to the reference ICOSL extracellular domain sequence set forth in SEQ ID NO:1. In some embodiments, the C-terminal truncation is of at least 10, at least 20, at least 30, at least 40, at least 50, at least 60, at least 70, at least 80, at least 90, at least 100, at least 125 amino acid residues. In some embodiments, the C-terminal truncation is of at least 1, at least 5 at least 10, at least 15, at least 20, at least 25, at least 30, at least 35 amino acid residues. In some embodiments, the ICOSL polypeptide comprising a C-terminal truncation does not contain, beyond the C-terminus of the truncation point, contiguous amino acid residues of a wild-type ICOSL. Hence, among provided ICOSL reference sequences are those that are shorter than the full extracellular domain of a wild-type ICOSL, e.g. set forth in SEQ ID NO: 1. In some embodiments, the ICOSL polypeptide comprising a C-terminal truncation does not contain or is not fused to amino acid residues of an ICOSL domain beyond the extracellular domain.

In some embodiments, the ICOSL reference polypeptide is altered, such as mutated or deleted, in one or more protease cleavage site. As found herein, wild-type ICOSL polypeptide contains a protease cleavage site that, in some cases, results in cleavage of the protein upon expression in cells, e.g. Chinese Hamster Ovary cells, thereby resulting in a heterogeneous product of multiple species, including species of different lengths or sizes. For example, cleavage of the ICOSL polypeptide may occur at the LQQN/LT protease cleavage site between residues 207 and 208 of SEQ ID NO: 1 ("/" indicates potential cleavage site). In some embodiments, the ICOSL reference polypeptide is altered in or lacks a protease cleavage site set forth as amino acids 204-209 of SEQ ID NO:1. In some embodiments, a truncated ICOSL polypeptide is more resistant to protease cleavage compared to a wild-type or non-truncated ICOSL polypeptide. Exemplary truncated ICOSL polypeptide ECD truncations lacking all or a portion of the LQQN/LT protease cleavage site (designated Truncations #2, #3, #4, #5, #6, #7, or #8) are provided in SEQ ID NOs: 5-11.

Truncation #2:

DTQEKEVRAMVGSDVELSCACPEGSRFDLNDVYVYWQTSESKTVVTYHIPQNSSLENVDSRY
RNRALMSPAGMLRGDFSLRLFNVTPQDEQKFHCLVLSQSLGFQEVLSVEVTLHVAANFSVPV
VSAPHSPSQDELTFTCTSINGYPRPNVYWINKTDNSLLDQALQNDTVFLNMRGLYDVVSVLRI
ARTPSVNIGCCIENVLLQQNL (SEQ ID NO: 5)

Truncation #3:

DTQEKEVRAMVGSDVELSCACPEGSRFDLNDVYVYWQTSESKTVVTYHIPQNSSLENVDSRY
RNRALMSPAGMLRGDFSLRLFNVTPQDEQKFHCLVLSQSLGFQEVLSVEVTLHVAANFSVPV
VSAPHSPSQDELTFTCTSINGYPRPNVYWINKTDNSLLDQALQNDTVFLNMRGLYDVVSVLRI
ARTPSVNIGCCIENVLLQQNLTVGSQ (SEQ ID NO: 6)

Truncation #4:

DTQEKEVRAMVGSDVELSCACPEGSRFDLNDVYVYWQTSESKTVVTYHIPQNSSLENVDSRY RNRALMSPAGMLRGDFSLRLFNVTPQDEQKFHCLVLSQSLGFQEVLSVEVTLHVAANFSVPV VSAPHSPSQDELTFTCTSINGYPRPNVYWINKTDNSLLDQALQNDTVFLNMRGLYDVVSVLRI ARTPSVNIGCCIENVLLQQN (SEQ ID NO: 7)

Truncation #5:

DTQEKEVRAMVGSDVELSCACPEGSRFDLNDVYVYWQTSESKTVVTYHIPQNSSLENVDSRY RNRALMSPAGMLRGDFSLRLFNVTPQDEQKFHCLVLSQSLGFQEVLSVEVTLHVAANFSVPV VSAPHSPSQDELTFTCTSINGYPRPNVYWINKTDNSLLDQALQNDTVFLNMRGLYDVVSVLRI ARTPSVNIGCCIENVLLQQ (SEQ ID NO: 8)

Truncation #6:

DTQEKEVRAMVGSDVELSCACPEGSRFDLNDVYVYWQTSESKTVVTYHIPQNSSLENVDSRY RNRALMSPAGMLRGDFSLRLFNVTPQDEQKFHCLVLSQSLGFQEVLSVEVTLHVAANFSVPV VSAPHSPSQDELTFTCTSINGYPRPNVYWINKTDNSLLDQALQNDTVFLNMRGLYDVVSVLRI ARTPSVNIGCCIENVLL (SEQ ID NO: 9)

Truncation #7:

DTQEKEVRAMVGSDVELSCACPEGSRFDLNDVYVYWQTSESKTVVTYHIPQNSSLENVDSRY RNRALMSPAGMLRGDFSLRLFNVTPQDEQKFHCLVLSQSLGFQEVLSVEVTLHVAANFSVPV VSAPHSPSQDELTFTCTSINGYPRPNVYWINKTDNSLLDQALQNDTVFLNMRGLYDVVSVLRI ARTPSVNIGCCIEN (SEQ ID NO: 10)

Truncation #8:

DTQEKEVRAMVGSDVELSCACPEGSRFDLNDVYVYWQTSESKTVVTYHIPQNSSLENVDSRY RNRALMSPAGMLRGDFSLRLFNVTPQDEQKFHCLVLSQSLGFQEVLSVEVTLHVAANFSVPV VSAPHSPSQDELTFTCTSINGYPRPNVYWINKTDNSLLDQALQNDTVFLNMRGLYDVVSVLRI ARTPSVNIGCCIENVLLQQNLT (SEQ ID NO: 11)

In some embodiments, the ICOSL reference polypeptide is altered in one or more amino acids corresponding to amino acids 204-209 with reference to SEQ ID NO:1. In some embodiments, the variant ICOSL polypeptide has one or more amino acid modification, e.g., substitution in a reference ICOSL or specific binding fragment thereof corresponding to position(s) 207 and/or 208 with reference to numbering of SEQ ID NO:1. In some embodiments, the variant ICOSL polypeptide has one or more amino acid modification, e.g., substitution, selected from N207A, N207G, L208G, or a conservative amino acid modification, e.g., substitution thereof. In some embodiments, the one or more amino acid modification, e.g., substitution is N207A/L208G or N207G/L208G. In some embodiments, the full length reference ECDs or truncated reference ECDs of the variant ICOSL polypeptide are modified to contain one or more amino acid modifications, e.g., substitutions, selected from N207A, N207G, L208G, or a conservative amino acid modification. Exemplary full length or truncated reference ECDs with one or more modifications are set forth in SEQ ID NOs: 12-33. Exemplary reference sequences containing mutations at cleavage site N207 and/or L208 with reference to positions are set forth in SEQ ID NO: 1 are set forth in SEQ ID NOs: 29-33. In some cases, the provided modifications may reduce protease cleavage of the ICOSL polypeptide, such as cleavage that may occur at the LQQN/LT protease cleavage site.

In some embodiments, combinations of the above truncation and modification strategies can be employed in a reference ICOSL ECD sequence. In some embodiments, the provided modifications e.g., substitutions, are made in a truncated reference ICOSL polypeptide such as exemplary reference ICOSL sequence set forth in SEQ ID NOs: 5-11. Exemplary variant ICOSL polypeptide sequences with modifications at the potential protease cleavage site(s) N207 and/or L208 are set forth in SEQ ID NOs: 12-33. In some embodiments, the variant ICOSL polypeptide exhibits decreased protease cleavage compared to wild-type ICOSL polypeptide, such as containing the ECD sequence set forth in SEQ ID NO:1.

In some embodiments, the reference (e.g., unmodified) or wildtype ICOSL polypeptide comprises an IgV domain or an IgC domain, or a specific binding fragment thereof. In some embodiments, an ICOSL reference polypeptide containing an IgV domain comprises the amino acid sequence set forth in SEQ ID NO: 2 (corresponding to amino acid residues 19-129 of SEQ ID NO:4), or an ortholog thereof.

DTQEKEVRAMVGSDVELSCACPEGSRFDLNDVYVYWQTSESKTVVTYHIPQNSSLENVDSRY
RNRALMSPAGMLRGDFSLRLFNVTPQDEQKFHCLVLSQSLGFQEVLSVE (SEQ ID NO:2)

In some embodiments, the reference ICOSL polypeptide containing the IgV domain contains at least amino acids 1-112, 1-113, 1-114, 1-115, 1-116, 1-117, 1-118, 1-119, 1-120, 1-121, 1-122, with reference to numbering set forth in SEQ ID NO:1. In some embodiments, an ICOSL reference polypeptide containing an IgV domain comprises the amino acid sequence set forth in SEQ ID NO: 3 (corresponding to amino acid residues 19-140 of SEQ ID NO:4), or an ortholog thereof. In some embodiments, the IgV domain is the only IgSF domain of the ICOSL reference polypeptide.

DTQEKEVRAMVGSDVELSCACPEGSRFDLNDVYVYWQTSESKTVVTYHIPQNSSLENVDSRY
RNRALMSPAGMLRGDFSLRLFNVTPQDEQKFHCLVLSQSLGFQEVLSVEVTLHVAANFSV
(SEQ ID NO: 3)

In some embodiments, the IgV domain of the reference (e.g., unmodified) or wild-type ICOSL polypeptide can contain (i) the sequence of amino acids set forth in SEQ ID NO: 2, (ii) a sequence of amino acids that has at least about 85%, 86%, 87%, 88%, 89%, 90%, 91%, 92%, 93%, 94%, 95%, 96%, 97%, 98%, 99% sequence identity to SEQ ID NO: 2, or (iii) a specific binding fragment of the sequence of amino acids set forth in SEQ ID NO: 2 or a specific binding fragment of a sequence of (i) or (ii). In some embodiments, the reference (e.g., unmodified) IgV domain is capable of binding one or more ICOSL cognate binding proteins, such as one or both of CD28 and ICOS.

In some embodiments, the IgV domain of the reference (e.g., unmodified) or wild-type ICOSL polypeptide can contain (i) the sequence of amino acids set forth in SEQ ID NO: 3, (ii) a sequence of amino acids that has at least about 85%, 86%, 87%, 88%, 89%, 90%, 91%, 92%, 93%, 94%, 95%, 96%, 97%, 98%, 99% sequence identity to SEQ ID NO: 3, or (iii) a specific binding fragment of the sequence of amino acids set forth inSEQ ID NO: 3 or a specific binding fragment of a sequence of (i) or (ii). In some embodiments, the reference (e.g., unmodified) IgV domain is capable of binding one or more ICOSL cognate binding proteins, such as one or both of CD28 and ICOS.

In some embodiments, the reference (e.g., unmodified) or wild-type ICOSL polypeptide contains a specific binding fragment of ICOSL, such as a specific binding fragment of the IgV domain. In some embodiments the specific binding fragment can bind CD28 and/or ICOS. The specific binding fragment can have an amino acid length of at least 50 amino acids, such as at least 60, 70, 80, 90, 100, or 110 amino acids. In some embodiments, the specific binding fragment of the IgV domain contains an amino acid sequence that is at least about 85%, 86%, 87%, 88%, 89%, 90%, 91%, 92%, 93%, 94%, 95%, 96%, 97%, 98%, 99% of the length of the IgV domain set forth as amino acids 19-129 of SEQ ID NO: 4.

In some embodiments, the variant ICOSL polypeptide comprises the ECD domain, a truncated ECD domain, or a portion thereof comprising one or more affinity modified IgSF domains. In some embodiments, the variant ICOSL polypeptides can comprise an IgV domain, in which the IgV domain or a specific binding fragment of the IgV domain contains the one or more amino acid modifications (e.g. substitutions). In some embodiments, the variant ICOSL polypeptides can comprise an IgV domain and an IgC domain, or a specific binding fragment of the IgV domain and a specific binding fragment of the IgC domain. In some embodiments, the variant ICOSL polypeptide comprises a full-length IgV domain. In some embodiments, the variant ICOSL polypeptide comprises a full-length IgC domain. In some embodiments, the variant ICOSL polypeptide comprises a specific binding fragment of the IgV domain. In some embodiments, the variant ICOSL polypeptide comprises a specific binding fragment of the IgC domain. In some embodiments, the variant ICOSL polypeptide comprises a full-length IgV domain and a full-length IgC domain. In some embodiments, the variant ICOSL polypeptide comprises a full-length IgV domain and a specific binding fragment of an IgC domain. In some embodiments, the variant ICOSL polypeptide comprises a specific binding fragment of an IgV domain and a full-length IgC domain. In some embodiments, the variant ICOSL polypeptide comprises a specific binding fragment of an IgV domain and a specific binding fragment of an IgC domain.

In any of such embodiments, the one or more amino acid modifications (e.g., substitutions) of the variant ICOSL polypeptides can be located in any one or more of the ICOSL polypeptide domains. For example, in some embodiments, one or more amino acid substitutions are located in the extracellular domain (ECD) of the variant ICOSL polypeptide, such as set forth in SEQ ID NO: 1. In some embodiments, one or more amino acid substitutions are located in the IgV domain or specific binding fragment of the IgV domain.

In some embodiments, the variant ICOSL polypeptide has up to 1, 2, 3, 4, 5, 6, 7, 8, 9, 10, 11, 12, 13, 14, 15, 16, 17, 18, 19, or 20 amino acid modification(s), e.g. substitution. The modification, e.g. substitution can be in the IgV domain or the IgC domain. In some embodiments, the variant ICOSL polypeptide has up to 1, 2, 3, 4, 5, 6, 7, 8, 9, 10, 11, 12, 13, 14, 15, 16, 17, 18, 19, or 20 amino acid substitutions in the IgV domain or specific binding fragment thereof. In some embodiments, the variant ICOSL polypeptide has up to 1, 2, 3, 4, 5, 6, 7, 8, 9, 10, 11, 12, 13, 14, 15, 16, 17, 18, 19, or 20 amino acid substitutions in the IgC domain or specific binding fragment thereof. In some embodiments, the variant ICOSL polypeptide has at least about 85%, 86%, 86%, 88%, 89%, 90%, 91%, 92%, 93%, 94%, 95%, 96%, 97%, 98%, or 99% sequence identity with the reference (e.g., unmodified) or wild-type ICOSL polypeptide or specific binding fragment thereof, such as with the amino acid sequence of SEQ ID NO: 1, 2 or 3.

In some embodiments, the variant ICOSL polypeptide has one or more amino acid modifications, e.g. substitutions in a reference ICOSL or specific binding fragment thereof corresponding to position(s) 10, 11, 13, 16, 18, 20, 25, 27, 30, 33, 37, 42, 43, 47, 52, 54, 57, 61, 62, 67, 71, 72, 74, 77, 78, 75, 80, 84, 89, 90, 92, 93, 94, 96, 97, 98, 99, 100, 102, 103, 107, 109, 110, 111, 113, 115, 116, 117, 119, 120, 121, 122, 126, 129, 130, 132, 133, 135, 138, 139, 140, 142, 143, 144, 146, 151, 152, 153, 154, 155, 156, 158, 161, 166, 168, 172, 173, 175, 190, 192, 193, 194, 198, 201, 203, 207, 208, 210, 212, 217, 218, 220, 221, 224, 225, or 227 with reference to numbering of SEQ ID NO:1. In some embodiments, the variant ICOSL polypeptide has one or more amino acid modifications, e.g. substitutions in a reference ICOSL or specific binding fragment there of corresponding to position(s) 10, 11, 13, 16, 18, 20, 25, 26, 27, 30, 33, 37, 38, 42, 43, 47, 52, 54, 57, 61, 62, 67, 71, 72, 74, 75, 77, 78, 80, 84, 89, 90, 92, 93, 94, 96, 97, 98, 99, 100, 102, 103, 107, 109, 110, 111, 113, 115, 116, 117, 119, 120, 121, 122, 126, 129, 130, 132, 133, 135, 137, 138, 139, 140, 142, 143, 144, 146, 151, 152, 153, 154, 155, 156, 158, 161, 164, 166, 168, 172, 173, 175, 190, 192, 193, 194, 198, 201, 203, 207, 208, 210, 212, 217, 218, 220, 221, 224, 225, or 227 with reference to numbering of SEQ ID NO:1.

In some embodiments, the variant ICOSL polypeptide has one or more amino acid modification, e.g. substitution selected from M10V, M10I, V11E, S13G, E16V, S18R, A20V, S25G, F27S, F27C, N30D, Y33del, Q37R, K42E, Y47H, T43A, N52A, N52C, N52D, N52G, N52H, N52L, N52K, N52M, N52P, N52Q, N52R, N52S, N52T, N52V, N52Y, S54A, S54P, N57A, N57E, N57F, N57H, N57K, N57L, N57M, N57P, N57Q, N57S, N57T, N57V, N57W, N57Y, R61S, R61C, Y62F, L67P, A71T, G72R, L74Q, R75Q, D77G, F78L, L80P, N84Q, D89G, E90A, K92R, F93L, H94E, H94D, L96F, L96I, V97A, L98F, S99G, Q100A, Q100D, Q100E, Q100G, Q100K, Q100L, Q100M, Q100N, Q100R, Q100P, Q100S, Q100T, Q100V, L102R, G103E, V107A, V107I, S109G, S109N, V110D, V110N, V110A, E111del, T113E, H115R, H115Q, V116A, A117T, N119Q, F120I, F120S, S121G, V122A, V122M, S126T, S126R, H129P, S130G,S132F, Q133H, E135K, F138L, T139S, C140D, C140del, S142F,I143V, I143T, N144D, Y146C, V151A, Y152C, Y152H,W153R, I154F, N155H, N155Q, K156M, D158G, L161P, L161M, L166Q, N168Q, F172S, L173S, M175T, T190S, T190A, S192G, V193M, N194D, C198R, N201S, L203P, L203F, N207Q, L208P, V210A, S212G, D217V, I218T, I218N, E220G, R221G, R221I, I224V, T225A, N227K or a conservative amino acid modification, e.g. substitution thereof. In some embodiments, the variant ICOSL polypeptide has one or more amino acid modification, e.g. substitution selected from M10V, M10I, V11E, S13G, E16V, S18R, A20T, A20V, S25G, R26S, F27C, F27S, N30D, Y33del, Q37R, T38P, K42E, T43A, Y47H, N52A, N52C, N52D, N52G, N52H, N52K, N52L, N52M, N52P, N52Q, N52R, N52S, N52T, N52V, N52Y, S54A, S54F, S54P, N57A, N57D, N57E, N57F, N57H, N57K, N57L, N57M, N57P, N57Q, N57S, N57T, N57V, N57W, N57Y, R61C, R61S, Y62F, L67P, A71T, G72R, L74Q, R75Q, D77G, F78L, L80P, N84Q, D89G, E90A, K92R, F93L, H94D, H94E, L96F, L96I, V97A, L98F, S99G, Q100A, Q100D, Q100E, Q100G, Q100K, Q100L, Q100M, Q100N, Q100P, Q100R, Q100S, Q100T, Q100V, L102R, G103E, V107A, V107I, S109G, S109N, V110A, V110D, V110N, E111del, T113E, H115Q, H115R, V116A, A117T, N119Q, F120I, F120S, S121G, V122A, V122M, S126R,S126T, H129P, S130G, S132F, Q133H, E135K, T137A, F138L, T139S, C140del, C140D, S142F, I143T, I143V, N144D, Y146C, V151A, Y152C, Y152H, W153R, I154F, N155H, N155Q, K156M, D158G, L161M, L161P, Q164L, L166Q, N168Q, F172S, L173S, M175T, T190A, T190S, S192G, V193A, V193M, N194D, C198R, N201S, L203F, L203P, N207Q, L208P, V210A, S212G, D217G, D217V, I218N, I218T, E220G, R221G, R221I, R221K, I224V, T225A, T225S, N227K, or a conservative amino acid substitution thereof.

In some embodiments, the variant ICOSL polypeptide has one or more amino acid modification, e.g. substitution selected from M10V, M10I, V11E, S13G, E16V, S18R, A20V, S25G, F27S, F27C, N30D, Y33del, Q37R, K42E, T43A, Y47H, N52A, N52C, N52D, N52G, N52H, N52L, N52K, N52M, N52P, N52Q, N52R, N52S, N52T, N52V, N52Y, S54A, S54P, N57A, N57E, N57F, N57H, N57K, N57L, N57M, N57P, N57Q, N57S, N57T, N57V, N57W, N57Y,R61S, R61C, Y62F, L67P, A71T, G72R, L74Q, R75Q, D77G, F78L, L80P, N84Q, D89G, E90A, K92R, F93L, H94E, H94D, L96F, L96I, V97A, L98F, S99G, Q100A, Q100D, Q100E, Q100G, Q100K, Q100L, Q100M, Q100N, Q100R, Q100P, Q100S, Q100T, Q100V, G103E, L102R, V107A, V107I, S109G, S109N, V110D, V110N, V110A, E111del, T113E, H115R, H115Q, V116A, A117T, N119Q, F120I, F120S, S121G, V122A, V122M, S126T, S126R, H129P, S130G,S132F, Q133H, E135K, F138L, T139S, C140D, C140del, S142F, I143V, I143T, N144D, Y146C, V151A, Y152C, Y152H,W153R, I154F, N155H, N155Q, K156M, D158G, L161P, L161M, L166Q, N168Q, F172S, L173S, M175T, T190A, T190S, S192G, V193M, N194D, C198R, N201S, L203P, L203F, N207Q, L208P, V210A, S212G, D217V, I218T, I218N, E220G, R221G, R221I, I224V, T225A, or N227K. In some embodiments, the variant ICOSL polypeptide has one or more amino acid modification, e.g. substitution selected from M10V, M10I, V11E, S13G, E16V, S18R, A20T, A20V, S25G, R26S, F27C, F27S, N30D, Y33del, Q37R, T38P, K42E, T43A, Y47H, N52A, N52C, N52D, N52G, N52H, N52K, N52L, N52M, N52P, N52Q, N52R, N52S, N52T, N52V, N52Y, S54A, S54F, S54P, N57A, N57D, N57E, N57F, N57H, N57K, N57L, N57M, N57P, N57Q, N57S, N57T, N57V, N57W, N57Y, R61C, R61S, Y62F, L67P, A71T, G72R, L74Q, R75Q, D77G, F78L, L80P, N84Q, D89G, E90A, K92R, F93L, H94D, H94E, L96F, L96I, V97A, L98F, S99G, Q100A, Q100D, Q100E, Q100G, Q100K, Q100L, Q100M, Q100N, Q100P, Q100R, Q100S, Q100T, Q100V, L102R, G103E, V107A, V107I, S109G, S109N, V110A, V110D, V110N, E111del, T113E, H115Q, H115R, V116A, A117T, N119Q, F120I, F120S, S121G, V122A, V122M, S126R,S126T, H129P, S130G, S132F, Q133H, E135K, T137A, F138L, T139S, C140del, C140D, S142F, I143T, I143V, N144D, Y146C, V151A, Y152C, Y152H, W153R, I154F, N155H, N155Q, K156M, D158G, L161M, L161P, Q164L, L166Q, N168Q, F172S, L173S, M175T, T190A, T190S, S192G, V193A, V193M, N194D, C198R, N201S, L203F, L203P, N207Q, L208P, V210A, S212G, D217G, D217V, I218N, I218T, E220G, R221G, R221I, R221K, I224V, T225A, T225S, N227K, or a conservative amino acid substitution thereof.

In some embodiments, the one or more amino acid modification, e.g. substitution is N52Y/N57Y/F138L/L203P, N52H/N57Y/Q100P, N52S/Y146C/Y152C, N52H/C198R, N52H/C140D/T225A, N52H/C198R/T225A, N52H/K92R, N52H/S99G, N57Y/Q100P, N52S/G103E, N52S/S130G/Y152C, N52S/Y152C, N52S/C198R, N52Y/N57Y/Y152C, N52Y/N57Y/H129P/C198R, N52H/L161P/C198R, N52S/T113E, N52D/S54P, N52K/L208P, N52S/Y152H, N52D/V151A, N52H/I143T, N52S/L80P, F120S/Y152H/N201S, N52S/R75Q/L203P, N52S/D158G, N52D/Q133H, N52S/N57Y/H94D/L96F/L98F/Q100R, N52S/N57Y/H94D/L96F/L98F/Q100R/G103E/F120S, N52H/F78L/Q100R, N52H/N57Y/Q100R/V110D, N52H/N57Y/R75Q/Q100R/V110D, N52H/N57Y/Q100R, N52H/N57Y/L74Q/Q100R/V110D, N52H/Q100R, N52H/S121G, A20V/N52H/N57Y/Q100R/S109G, N52H/N57Y/Q100P, N52H/N57Y/R61S/Q100R/V110D/L173S, N52H/N57Y/Q100R/V122A, N52H/N57Y/Q100R/F172S, N52H/N57Y, N52S/F120S, N52S/V97A, N52S/G72R, N52S/A71T/A117T, N52S/E220G, Y47H/N52S/V107A/F120S, N52H/N57Y/Q100R/V110D/S132F/M175T, E 16V/N52H/N57Y/Q100R/V 110D/H 115R/Y 152C/K156M/C 198R, Q37R/N52H/N57Y/Q100R/V 110N/S 142F/C 198R/D217V/R221G, N52H/N57Y/Q100R/V110D/C198R, N52H/N57Y/Q100R/V 110D/V 116A/L161M/F172S/S 192G/C 198R, F27S/N52H/N57Y/V 110N, N52S/H94E/L96I/S109N/L166Q, S18R/N52S/F93L/I143V/R221G, A20T/N52D/Y146C/Q164L, V11E/N30D/N52H/N57Y/H94E/L96I/L98F/N194D/V210A/I218T, N52S/H94E/L96I/V122M, N52H/N57Y/H94E/L96I/F120I/S126T/W153R/I218N, M10V/S18R/N30D/N52S/S126R/T139S/L203F, S25G/N30D/N52S/F120S/N227K, N30D/N52S/L67P/Q100K/D217G/R221K/T225S, N52H/N57Y/Q100R/V110D/A117T/T190S/C198R, N52H/N57Y/Q100R/V110D/F172S/C198R, S25G/F27C/N52H/N57Y/Q100R/V110D/E135K/L173S/C198R, N52H/N57Y/V110A/C198R/R221I, M10I/S13G/N52H/N57Y/D77G/V110A/H129P/I143V/F172S/V193M,C198R, N52H/N57Y/R61C/Y62F/Q100R/V110N/F120S/C198R, N52H/N57Y/Q100R/V 110D/H 115R/C 198R, N52H/N57Y/Q100R/V110D/N144D/F172S/C198R, N52S/H94E/L98F/Q100R, N52S/E90A, N30D/K42E/N52S, N52S/F120S/I143V/I224V, N52H/N57Y/Q100R/V110D/C198R/S212G, N52H/N57Y/Q100R/C198R, N52S/N194D, N52H/N57Y/Q100R/L102R/V110D/H115R/C198R, N52S/S54P, T38P/N52S/N57D, N52H/C140del/T225A, N52H/F78L/Q100R/C198R, N52H/N57Y/R75Q/Q100P/V110D, N52H/N57Y/L74Q/V110D/S192G, N52H/S121G/C198R, N52S/F120S/N227K, N52S/A71T/A117T/T190A/C198R, T43A/N52H/N57Y/L74Q/D89G/V110D/F172S, N52H/N57Y/Q100R/V110D/S132F/M175T, N52H/N57Y/Q100R/V107I/V110D/I154F/C198R/R221G, Q100R, F138L/L203P, N57Y/F138L/L203P, N57Y/Q100R/C198R, N57Y/F138L/L203P, Q100R/F138L, L203P, N52H/N57Y/Q100R/H115R/C198R, N52H/N57Y/Q100R/F172S/C198R, N52H/N57Y/Q100R/H115R/F172S/C198R, N52H/N57Y/Q100R/H115R/I143V/F172S/C198R, N52H/N57Y/Q100R/L102R/H115R/F172S/C198R, N52H/V122A/F172S/C198R, N52H/N57Y/Q100R/H115R/F172S/N194D, N52H/N57Y/H115R/F172S/C198R, N52H/N57Y/Q100R/H115R/C198R, N52H/N57Y/H115R, N52H/N57Y/Q100R/H115R, N52H/N57Y/Q100R/H115R/F172S/I224V, N52H/N57Y/Q100R/H115R/F172S, N52H/N57Y/Q100R/F172S, N52H/Q100R/H115R/I143T/F172S, N52H/N57Y/Q100P/H115R/F172S, N52Y/N57Y/Q100P/F172S, E16V/N52H/N57Y/Q100R/V110D/H115R/C198R, E16V/N52H/N57Y/Q100R/V110D/H115R/Y152C/K156M/F172S/C198R, N52S/E90A/H115R, N30D/K42E N52S/H115R, N30D/K42E/N52S/H115R/C198R/R221I, N30D/K42E/N52S/H115R/C198R, N30D/K42E/N52S/H115R/F172S/N194D, N52S/H115R/F120S/I143V/C198R, N52S/H115R/F172S/C198R, N52H/N57Y/Q100P/C198R, N52H/N57Y/Q100P H115R/F172S/C198R, N52H/N57Y/Q100P/F172S/C198R, N52H/N57Y/Q100P/H115R, N52H/N57Y/Q100P/H115R/C198R, N52H/Q100R/C198R, N52H/Q100R/H115R/F172S, N52H/Q100R/F172S/C198R, N52H/Q100R/H115Q/F172S/C198R, N52H/N57Y/Q100R /F172S/C198R, N52Q/N207Q, N168Q/N207Q, N52Q/N168Q, N84Q/N207Q, N155Q/N207Q, N119Q/N168Q, N119Q/N207Q, N119Q/N155Q, N52Q/N84Q, N52Q/N119Q, N84Q/N119Q, N52Q/N84Q/N168Q, N52Q/N84Q/N207Q, N84Q/N155Q/N168Q, N84Q/N168Q/N207Q, N84Q/N155H/N207Q, N155Q/N168Q/N207Q, N119Q/N155Q/N168Q, N119Q/N168Q/N207Q, N84Q/N119Q/N207Q, N119Q/N155H/N207Q, N84Q/N119Q/N155Q, N52Q/N119Q/N155Q, N52H/N84Q/N119Q, N52H/N84Q, N52H/N84Q/N168Q, N52H/N84Q/N207Q, N52H/N84Q/N168Q/N207Q, N52Q/N84Q/N155Q, N52Q/N84Q/N168Q, N52Q/N84Q/N155Q/N168Q, N52Q/N84Q/N119Q/N168Q, N84Q/N119Q/N155Q/N168Q, N84Q/N155Q/N168Q/N207Q, N84Q/N119Q/N155Q/N207Q, N52Q/N84Q/N119Q/N207Q, N52Q/N84Q/N119Q/N155Q, N52Q/N84Q/N119Q/N155Q/N207Q, N84Q/N119Q/N155Q/N168Q/N207Q, N52A/N57F/Q100S, N52A/N57H/Q100S, N52A/N57Y/Q100A, N52D/N57A/Q100A, N52D/Q100S, N52G/Q100A, N52H/Q100A, N52M/N57H/Q100S, N52M/N57W/Q100P, N52Q/N57F, N52Q/N57S/Q100A, N52R/N57L/Q100A, N52R/N57Y/Q100P, N52R/N57Y/Q100S, N52S/N57A/Q100A, N52S/N57H/Q100E, N52S/N57L/Q100S, N52S/N57M/Q100S, N52S/N57Y/Q100S, N52S/N57Y/Q100M, N52S/N57Y/Q100V, N52T/N57H/Q100S, N52T/N57H/Q100A, N52T/N57Y/Q100A, N52V/N57L/Q100A, N52H/N57Y/Q100K, N52K/N57Y/Q100R, N52L/N57H/Q100R, N52R/N57F/Q100N, N52R/N57F/Q100P, N52R/N57F/Q100R, N52R/N57F/Q100T, N52R/N57H/Q100K, N52R/N57L/Q100S, N52R/N57W/Q100K, N52R/N57W, N52R/N57Y/Q100R, N52C/N57E/Q100S, N52G/N57P/Q100D, N52G/N57V/Q100G, N52G/N57V, N52L/N57V, N52P/N57P, N52P/N57S/Q100G, N52S/N57L/Q100G, N52T/N57K/Q100P, N52V/N57T/Q100L, or N57Q/Q100P.

In some embodiments, the one or more amino acid modifications are selected from among N52Y/N57Y/F138L/L203P, N52H/N57Y/Q100P, N52S/Y146C/Y152C, N52H/C198R, N52H/C140D/T225A, N52H/C198R/T225A, N52H/K92R, N52H/S99G, N57Y/Q100P, N52S/S130G/Y152C, N52S/Y152C, N52S/C198R, N52Y/N57Y/Y152C, N52Y/N57Y/H129P/C198R, N52H/L161P/C198R, N52S/T113E, N52D/S54P, N52K/L208P, N52S/Y152H, N52D/V151A, N52H/I143T, N52S/L80P, F120S/Y152H/N201S, N52S/R75Q/L203P, N52S/D158G, N52D/Q133H, N52S/N57Y/H94D/L96F/L98F/Q100R, N52S/N57Y/H94D/L96F/L98F/Q100R/G103E/F120S, N52S/G103E, N52H/F78L/Q100R, N52H/N57Y/Q100R/V110D, N52H/N57Y/R75Q/Q100R/V110D, N52H/N57Y/Q100R, N52H/N57Y/L74Q/Q100R/V110D, N52H/Q100R, N52H/S121G, A20V/N52H/N57Y/Q100R/S109G, N52H/N57Y/R61S/Q100R/V110D/L173S, N52H/N57Y/Q100R/V122A, N52H/N57Y/Q100R/F172S, N52H/N57Y, N52S/F120S, N52S/V97A, N52S/G72R, N52S/A71T/A117T, N52S/E220G, Y47H/N52S/V107A/F120S, N52H/N57Y/Q100R/V110D/S132F/M175T, E 16V/N52H/N57Y/Q100R/V 110D/H 115R/Y 152C/K156M/C 198R, Q37R/N52H/N57Y/Q100R/V 110N/S 142F/C 198R/D217V/R221G, N52H/N57Y/Q100R/V110D/C198R, N52H/N57Y/Q100R/V 110D/V 116A/L161M/F172S/S 192G/C 198R, F27S/N52H/N57Y/V 110N, N52S/H94E/L96I/S109N/L166Q, S18R/N52S/F93L/I143V/R221G, A20T/N52D/Y146C/Q164L, V11E/N30D/N52H/N57 Y/H94E/L96 VL98F/N194D/V210A/I218T, N52S/H94E/L96EV 122M, N52H/N57Y/H94E/L96I/F120I/S126T/W153R/I218N, M10V/S18R/N30D/N52S/S126R/T139S/L203F, S25G/N30D/N52S/F120S/N227K, N30D/N52S/L67P/Q100K/D217G/R221K/T225S, N52H/N57Y/Q100R/V110D/A117T/T190S/C198R, N52H/N57Y/Q100R/V110D/F172S/C198R, S25G/F27C/N52H/N57Y/Q100R/V110D/E135K/L173S/C198R, N52H/N57Y/V110A/C198R/R221I, M10ES13G/N52H/N57Y/D77G/V110A/H129P/I143V/F172S/V193M,C198R, N52H/N57Y/R61C/Y62F/Q100R/V110N/F120S/C198R, N52H/N57Y/Q100R/V 110D/H 115R/C 198R, N52H/N57Y/Q100R/V110D/N144D/F172S/C198R, N52S/H94E/L98F/Q100R, N52S/E90A, N30D/K42E/N52S, N52S/F120S/I143V/I224V, N52H/N57Y/Q100R/V110D/C198R/S212G, N52H/N57Y/Q100R/C198R, N52S/N194D, N52H/N57Y/Q100R/L102R/V110D/H115R/C198R, N52H/N57Y/Q100R/V110D/C198R/S212G, N52H/N57Y/Q100R/C198R, N52S/N194D, N52H/N57Y/Q100R/L102R/V110D/H115R/C198R, N52S/S54P, T38P/N52S/N57D, N52H/C140del/T225A, N52H/F78L/Q100R/C198R, N52H/N57Y/R75Q/Q100P/V110D, N52H/N57Y/L74Q/V110D/S192G, N52H/S121G/C198R, N52S/F120S/N227K, N52S/A71T/A117T/T190A/C198R, T43A/N52H/N57Y/L74Q/D89G/V110D/F172S, N52H/N57Y/Q100R/V 110D/S 132F/M175T, N52H/N57Y/Q100R/V107I/V110D/I154F/C198R/R221G, N52Q/N207Q, N168Q/N207Q, N52Q/N168Q, N84Q/N207Q, N155Q/N207Q, N119Q/N168Q, N119Q/N207Q, N119Q/N155Q, N52Q/N84Q, N52Q/N119Q, N84Q/N119Q, N52Q/N84Q/N168Q, N52Q/N84Q/N207Q, N84Q/N155Q/N168Q, N84Q/N168Q/N207Q, N84Q/N155H/N207Q, N155Q/N168Q/N207Q, N119Q N155Q/N168Q, N119Q/N168Q/N207Q, N84Q/N119Q/N207Q, N119Q/N155H/N207Q, N84Q/N119Q/N155Q, N52Q/N119Q/N155Q, N52H/N84Q/N119Q, N52H/N84Q, N52H/N84Q/N168Q/N207Q, N52Q/N84Q/N155Q/N168Q, N52Q/N84Q/N119Q/N168Q, N84Q/N119Q/N155Q/N168Q, N84Q/N155Q/N168Q/N207Q, N84Q/N119Q/N155Q/N207Q, N52Q/N84Q/N119Q/N207Q, N52Q/N84Q/N119Q/N155Q, N52Q/N84Q/N119Q/N155Q/N207Q, N84Q/N119Q/N155Q/N168Q/N207Q, F138L/L203P, N52Y/F138L/L203P, N57Y/Q100R/C198R, N57Y/F138L/L203P, Q100R/F138L, N52H/N57Y/Q100R/H115R/C198R, N52H/N57Y/Q100R/F172S/C198R, N52H/N57Y/Q100R/H115R/F172S/C198R, N52H/N57Y/Q100R/H115R/I143V/F172S/C198R, N52H/N57Y/Q100R/L102R/H115R/F172S/C198R, N52H/V122A/F172S/C198R, N52H/N57Y/Q100R/H115R/F172S/N194D, N52H/N57Y/H115R/F172S/C198R, N52H/N57Y/H115R, N52H/N57Y/Q100R/H115R, N52H/N57Y/Q100R/H115R/F172S/I224V, N52H/N57Y/Q100R/H115R/F172S, N52H/N57Y/Q100R/F172S, N52H/Q100R/H115R/I143T/F172S, N52H/N57Y/Q100P/H115R/F172S, N52Y/N57Y/Q100P/F172S, E16V/N52H/N57Y/Q100R/V110D/H115R/C198R, E16V/N52H/N57Y/Q100R/V110D/H115R/Y152C/K156M/F172S/C198R, N52S/E90A/H115R, N30D/K42E N52S/H115R, N30D/K42E/N52S/H115R/C198R/R221I, N30D/K42E/N52S/H115R/C198R, N30D/K42E/N52S/H115R/F172S/N194D, N52S/H115R/F120S/I143V/C198R, N52S/H115R/F172S/C198R, N52H/N57Y/Q100P/C198R, N52H/N57Y/Q100P/H115R/F172S/C198R, N52H/N57Y/Q100P/F172S/C198R, N52H/N57Y/Q100P/H115R, N52H/N57Y/Q100P/H115R/C198R, N52H/Q100R/C198R, N52H/Q100R/H115R/F172S, N52H/Q100R/F172S/C198R, N52H/Q100R/H115R/F172S/C198R, N52H/N57Y/Q100R/F172S/C198R, N52A/N57F/Q100S, N52A/N57H/Q100S, N52A/N57Y/Q100A, N52D/N57A/Q100A, N52D/Q100S, N52G/Q100A, N52H/Q100A, N52M/N57H/Q100S, N52M/N57W/Q100P, N52Q/N57F, N52Q/N57S/Q100A, N52R/N57L/Q100A, N52R/N57Y/Q100P, N52R/N57Y/Q100S, N52S/N57A/Q100A, N52S/N57H/Q100E, N52S/N57L/Q100S, N52S/N57M/Q100S, N52S/N57Y/Q100S, N52S/N57Y/Q100M, N52S/N57Y/Q100V, N52T/N57H/Q100S, N52T/N57H/Q100A, N52T/N57Y/Q100A, N52V/N57L/Q100A, N52H/N57Y/Q100K, N52K/N57Y/Q100R, N52L/N57H/Q100R, N52R/N57F/Q100N, N52R/N57F/Q100P, N52R/N57F/Q100R, N52R/N57F/Q100T, N52R/N57H/Q100K, N52R/N57L/Q100S, N52R/N57W/Q100K, N52R/N57W, N52R/N57Y/Q100R, N52C/N57E/Q100S, N52G/N57P/Q100D, N52G/N57V/Q100G, N52G/N57V, N52L/N57V, N52P/N57P, N52P/N57S/Q100G, N52S/N57L/Q100G, N52T/N57K/Q100P, N52V/N57T/Q100L, N57Q/Q100P, S54F/V193A or R26S/N52H/N57Y/V110D/T137A/C198R.

In some embodiments, the one or more amino acid modification, e.g. substitution, is N52H/N57Y/Q100R, with reference to numbering of SEQ ID NO:1. In some embodiments, the one or more amino acid modification, e.g. substitution, is N52L/N57H/Q100R, with reference to numbering of SEQ ID NO:1. In some embodiments, the one or more amino acid modification, e.g. substitution, is N52H/Q100R, with reference to numbering of SEQ ID NO:1. In some embodiments, the one or more amino acid modification, e.g. substitution, is N52D, with reference to numbering of SEQ ID NO: 1.

Generally, each of the various attributes of polypeptides are separately disclosed (e.g., affinity of ICOSL for CD28 and/or ICOS, number of variations per polypeptide chain, number of linked polypeptide chains, the number and nature of amino acid alterations per variant ICOSL, etc.). However, as will be clear to the skilled artisan, any particular polypeptide can comprise a combination of these independent attributes. It is understood that reference to amino acids, including to a specific sequence set forth as a SEQ ID NO used to describe domain organization of an IgSF domain are for illustrative purposes and are not meant to limit the scope of the embodiments provided. It is understood that polypeptides and the description of domains thereof are theoretically derived based on homology analysis and alignments with similar molecules. Thus, the exact locus can vary, and is not necessarily the same for each protein. Hence, the specific IgSF domain, such as specific IgV domain or IgC domain, can be several amino acids (such as one, two, three or four) longer or shorter.

The reference (e.g., unmodified) or wild-type ICOSL sequence does not necessarily have to be used as a starting composition to generate variant ICOSL polypeptides described herein. Therefore, use of the term "modification", such as "substitution" does not imply that the present embodiments are limited to a particular method of making variant ICOSL polypeptides. Variant ICOSL polypeptides can be made, for example, by *de novo* peptide synthesis and thus does not necessarily require a modification, such as a "substitution", in the sense of altering a codon to encode for the modification, e.g. substitution. This principle also extends to the terms "addition" and "deletion" of an amino acid residue which likewise do not imply a particular method of making. The means by which the variant ICOSL polypeptides are designed or created is not limited to any particular method. In some embodiments, however, a reference (e.g., unmodified) or wild-type ICOSL encoding nucleic acid is mutagenized from reference (e.g., unmodified) or wild-type ICOSL genetic material and screened for desired specific binding affinity and/or modulation of IFN-gamma expression or other functional activity. In some embodiments, a variant ICOSL polypeptide is synthesized *de novo* utilizing protein or nucleic acid sequences available at any number of publicly available databases and then subsequently screened. The National Center for Biotechnology Information provides such information and its website is publicly accessible via the internet as is the UniProtKB database as discussed previously.

In some embodiments, the variant ICOSL polypeptide comprises the mutations listed in Table 2. Table 2 also provides exemplary sequences by reference to SEQ ID NO for the extracellular domain (ECD) or IgV domain of the wild-type ICOSL (i.e. reference (e.g., unmodified)) or exemplary variant ICOSL polypeptides. As indicated, the exact locus or residues corresponding to a given domain can vary, such as depending on the methods used to identify or classify the domain. Also, in some cases, adjacent N- and/or C-terminal amino acids of a given domain (e.g. IgV) also can be included in a sequence of a variant IgSF polypeptide, such as to ensure proper folding of the domain when expressed. Thus, it is understood that the exemplification of the SEQ ID NOs in Table 2 is not to be construed as limiting. For example, the particular domain, such as the ECD domain, of a variant ICOSL polypeptide can be several amino acids longer or shorter, such as 1-10, e.g., 1, 2, 3, 4, 5, 6 or 7 amino acids longer or shorter, than the sequence of amino acids set forth in the respective SEQ ID NO.

| **TABLE 2: Exemplary variant ICOSL polypeptides** | | |
|---|---|---|
| **Mutation(s)** | **ECD SEQ ID NO** | **IgV SEQ ID NO** |
| Wild-type | 1 | 2, 3 |
| N52H/N57Y/Q100R | 34 | 35, 36 |

The variant ICOSL polypeptide can contain any of the amino acid substitutions (mutations) described herein. In some embodiments, the variant ICOSL polypeptide comprises any of the mutations listed in Table 2. In some examples, the mutations are made in a reference ICOSL containing the sequence of amino acids set forth in SEQ ID NO: 1, a reference ICOSL that contains the IgV domain of ICOSL set forth in SEQ ID NOs: 2 or 3, or a reference ICOSL that is truncated and/or modified containing the sequence of amino acids set forth in any of SEQ ID NOs: 5-33.

In some embodiments, the variant ICOSL polypeptide comprises an extracellular domain (ECD) sequence set forth in SEQ ID NO: 34. In some embodiments, the variant ICOSL polypeptide comprises a polypeptide sequence that exhibits at least 90% identity, at least 91% identity, at least 92% identity, at least 93% identity, at least 94% identity, at least 95% identity, such as at least 96% identity, 97% identity, 98% identity, or 99% identity to an extracellular domain (ECD) sequence set forth in SEQ ID NO:34 and contains the amino acid modification(s), e.g. substitution(s) not present in the reference (e.g., unmodified) or wild-type ICOSL, e.g. N52H/N57Y/Q100R. In some embodiments, the variant ICOSL polypeptide comprises a specific binding fragment of the extracellular domain (ECD) sequence set forth in SEQ ID NO: 34 and contains the amino acid modification(s), e.g. substitution (s) not present in the reference (e.g., unmodified) or wild-type ICOSL, e.g. N52H/N57Y/Q100R.

In some embodiments, the variant ICOSL polypeptide comprises an IgV sequence set forth in SEQ ID NO: 35. In some embodiments, the variant ICOSL polypeptide comprises a polypeptide sequence that exhibits at least 90% identity, at least 91% identity, at least 92% identity, at least 93% identity, at least 94% identity, at least 95% identity, such as at least 96% identity, 97% identity, 98% identity, or 99% identity to an IgV sequence set forth in SEQ ID NO: 35 and contains the amino acid modification(s), e.g. substitution(s) not present in the reference (e.g., unmodified) or wild-type ICOSL, e.g. N52H/N57Y/Q100R. In some embodiments, the variant ICOSL polypeptide comprises a specific binding fragment of a IgV sequence set forth in SEQ ID NO: 35 and contains the amino acid substitution(s) not present in the reference (e.g., unmodified) or wild-type ICOSL, e.g. N52H/N57Y/Q100R.

In some embodiments, the variant ICOSL polypeptide comprises an IgV sequence set forth in SEQ ID NO: 36. In some embodiments, the variant ICOSL polypeptide comprises a polypeptide sequence that exhibits at least 90% identity, at least 91% identity, at least 92% identity, at least 93% identity, at least 94% identity, at least 95% identity, such as at least 96% identity, 97% identity, 98% identity, or 99% identity to an IgV sequence set forth in SEQ ID NO: 36 and contains the amino acid modification(s), e.g. substitution(s) not present in the reference (e.g., unmodified) or wild-type ICOSL, e.g. N52H/N57Y/Q100R. In some embodiments, the variant ICOSL polypeptide comprises a specific binding fragment of a IgV sequence set forth in SEQ ID NO: 36 and contains the amino acid substitution(s) not present in the reference (e.g., unmodified) or wild-type ICOSL, e.g. N52H/N57Y/Q100R..

In some embodiments, the variant ICOSL polypeptide exhibits increased affinity for the ectodomain of CD28 compared to the reference (e.g., unmodified) or wild-type ICOSL polypeptide, such as compared to the reference (e.g., unmodified) or wild-type ICOSL polypeptide comprising the sequence set forth in SEQ ID NO: 1, 2, or 3. In some embodiments, the ICOSL polypeptide exhibits increased affinity for the ectodomain of ICOS compared to the reference (e.g., unmodified) or wild-type ICOSL, such as compared to the reference (e.g., unmodified) or wild-type ICOSL polypeptide comprising the sequence set forth in SEQ ID NO: 1, 2, or 3. In some embodiments, the ICOSL polypeptide exhibits increased affinity for the ectodomain of CD28 and the ectodomain of ICOS compared to the reference (e.g., unmodified) or wild-type ICOSL, such as compared to the reference (e.g., unmodified) or wild-type ICOSL polypeptide comprising the sequence set forth in SEQ ID NO: 1, 2, or 3.

**B. Multimerization Domains**

The variant ICOSL polypeptide fusion proteins comprising a variant ICOSL polypeptide provided herein in which is contained a vIgD can be formatted in a variety of ways as a soluble protein. In some embodiments, the variant ICOSL fusion protein contains a multimerization domain. In some cases, a variant ICOSL fusion protein can antagonize or block activity of its cognate binding partner, e.g. CD28 and/or ICOSL. In some embodiments, antagonism of CD28 and/or ICOSL may be useful to treat inflammation or autoimmunity. Those of skill will appreciate that cell surface proteins typically have an intracellular, transmembrane, and extracellular domain (ECD) and that a soluble form of such proteins can be made using the extracellular domain or an immunologically active subsequence thereof. Thus, in some embodiments, the immunomodulatory protein containing a variant ICOSL polypeptide lacks a transmembrane domain or a portion of the transmembrane domain. In some embodiments, the immunomodulatory protein containing a variant ICOSL lacks the intracellular (cytoplasmic) domain or a portion of the intracellular domain. In some embodiments, the immunomodulatory protein containing the variant ICOSL polypeptide only contains the vIgD portion containing the ECD domain or a portion thereof containing an IgV domain and/or IgC (e.g. IgC2) domain or domains or specific binding fragments thereof containing the amino acid modification(s).

In some embodiments, the conjugate is a fusion protein of a variant ICOSL polypeptide linked, directly or via a linker, to another protein or polypeptide moiety. In some embodiments the fusion protein is an ICOSL-Fc variant fusion, in which any two or more of the foregoing variant polypeptides can be attached to an Fc. In some embodiments, a variant ICOSL polypeptide has the structure of a soluble protein as set forth in FIG. 1

In some embodiments, a fusion protein comprising a variant ICOSL can include one or more variant ICOSL polypeptides of the invention. In some embodiments a polypeptide of the invention will comprise exactly 1, 2, 3, 4, 5 variant ICOSL sequences. In some embodiments, at least two of the variant ICOSL sequences are identical variant ICOSL sequences.

In some embodiments, the provided immunomodulatory polypeptide comprises two or more vIgD sequences of ICOSL. Multiple variant ICOSL polypeptides within the polypeptide chain can be identical (i.e., the same species) to each other or be non-identical (i.e., different species) variant ICOSL sequences. In addition to single polypeptide chain embodiments, in some embodiments two, three, four, or more of the polypeptides of the invention can be covalently or non-covalently attached to each other. Thus, monomeric, dimeric, and higher order (e.g., 3, 4, 5, or more) multimeric proteins are provided herein. For example, in some embodiments exactly two polypeptides of the invention can be covalently or non-covalently attached to each other to form a dimer. In some embodiments, attachment is made via interchain cysteine disulfide bonds. Compositions comprising two or more polypeptides of the invention can be of an identical species or substantially identical species of polypeptide (e.g., a homodimer) or of non-identical species of polypeptides (e.g., a heterodimer). A composition having a plurality of linked polypeptides of the invention can, as noted above, have one or more identical or non-identical variant ICOSL polypeptides of the invention in each polypeptide chain.

In some embodiments, the immunomodulatory protein containing a variant ICOSL is multivalent, such as bivalent. In aspects, the immunomodulatory protein is linked, directly or indirectly via a linker, to a multimerization domain. In some aspects, the mutlimerization domain increase half-life of the molecule.

Interaction of two or more variant ICOSL polypeptides can be facilitated by their linkage, either directly or indirectly, to any moiety or other polypeptide that are themselves able to interact to form a stable structure. For example, separate encoded variant ICOSL polypeptide chains can be joined by multimerization, whereby multimerization of the polypeptides is mediated by a multimerization domain. Typically, the multimerization domain provides for the formation of a stable protein-protein interaction between a first variant ICOSL polypeptide and a second variant ICOSL polypeptide. Homo- or heteromultimeric polypeptides can be generated from co-expression of separate variant ICOSL polypeptides. The first and second variant ICOSL polypeptides can be the same or different.

In some embodiments, a multimerization domain includes any capable of forming a stable protein-protein interaction. The multimerization domains can interact via an immunoglobulin sequence (e.g. Fc domain; see e.g., International Patent Pub. Nos. WO 93/10151 and WO 2005/063816 US; U.S. Pub. No. 2006/0024298; U.S. Pat. No. 5,457,035); leucine zipper (e.g. from nuclear transforming proteins fos and jun or the proto-oncogene c-myc or from General Control of Nitrogen (GCN4)) (ee e.g., Busch and Sassone-Corsi (1990) Trends Genetics, 6:36-40; Gentz et al., (1989) Science, 243:1695-1699); a hydrophobic region; a hydrophilic region; or a free thiol which forms an intermolecular disulfide bond between the chimeric molecules of a homo- or heteromultimer. In addition, a multimerization domain can include an amino acid sequence comprising a protuberance complementary to an amino acid sequence comprising a hole, such as is described, for example, in U.S. Pat. No. 5,731,168; International Patent Pub. Nos. WO 98/50431 and WO 2005/063816; Ridgway et al. (1996) Protein Engineering, 9:617-621. Such a multimerization region can be engineered such that steric interactions not only promote stable interaction, but further promote the formation of heterodimers over homodimers from a mixture of chimeric monomers. Generally, protuberances are constructed by replacing small amino acid side chains from the interface of the first polypeptide with larger side chains (e.g., tyrosine or tryptophan). Compensatory cavities of identical or similar size to the protuberances are optionally created on the interface of the second polypeptide by replacing large amino acid side chains with smaller ones (e.g., alanine or threonine). Exemplary multimerization domains are described below.

The variant ICOSL polypeptide can be joined anywhere, but typically via its N- or C-terminus, to the N- or C-terminus of a multimerization domain to form a chimeric polypeptide. The linkage can be direct or indirect via a linker. Also, the chimeric polypeptide can be a fusion protein or can be formed by chemical linkage, such as through covalent or non-covalent interactions. For example, when preparing a chimeric polypeptide containing a multimerization domain, nucleic acid encoding all or part of a variant ICOSL polypeptide can be operably linked to nucleic acid encoding the multimerization domain sequence, directly or indirectly or optionally via a linker domain. In some cases, the construct encodes a chimeric protein where the C-terminus of the variant ICOSL polypeptide is joined to the N-terminus of the multimerization domain. In some instances, a construct can encode a chimeric protein where the N-terminus of the variant ICOSL polypeptide is joined to the N- or C-terminus of the multimerization domain.

A polypeptide multimer contains two chimeric proteins created by linking, directly or indirectly, two of the same or different variant ICOSL polypeptides directly or indirectly to a multimerization domain. In some examples, where the multimerization domain is a polypeptide, a gene fusion encoding the variant ICOSL polypeptide and multimerization domain is inserted into an appropriate expression vector. The resulting chimeric or fusion protein can be expressed in host cells transformed with the recombinant expression vector, and allowed to assemble into multimers, where the multimerization domains interact to form multivalent polypeptides. Chemical linkage of multimerization domains to variant ICOSL polypeptides can be effected using heterobifunctional linkers.

The resulting chimeric polypeptides, such as fusion proteins, and multimers formed therefrom, can be purified by any suitable method such as, for example, by affinity chromatography over Protein A or Protein G columns. Where two nucleic acid molecules encoding different polypeptides are transformed into cells, formation of homo- and heterodimers will occur. Conditions for expression can be adjusted so that heterodimer formation is favored over homodimer formation.

In some embodiments, the immunomodulatory protein comprises a variant ICOSL polypeptide attached to an Fc region of an immunoglobulin (yielding an "immunomodulatory Fc fusion," such as an "ICOSL-Fc variant fusion," also termed an ICOSL vIgD-Fc fusion). In some embodiments, the ICOSL-Fc variant fusion also comprises one or more additional IgSF domain(s), such as one or more additional vIgD linked to a vIgD of ICOSL. In some embodiments, the attachment of the variant ICOSL polypeptide or additional IgSF domain is at the N-terminus of the Fc. In some embodiments, the attachment of the variant ICOSL or additional IgSF domain polypeptide is at the C-terminus of the Fc. In some embodiments, two or more ICOSL or additional IgSF domain variant polypeptides (the same or different) are independently attached at the N-terminus and at the C-terminus.

In some embodiments, the Fc is murine or human Fc. In some embodiments, the Fc is a mammalian or human IgGl, IgG2, IgG3, or IgG4 Fc regions. In some embodiments, the Fc is derived from IgG1, such as human IgG1. In some embodiments, the Fc comprises the amino acid sequence set forth in SEQ ID NO: 37 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 37.

In some embodiments, the Fc region contains one more modifications to alter (e.g. reduce) one or more of its normal functions. In general, the Fc region is responsible for effector functions, such as complement-dependent cytotoxicity (CDC) and antibody-dependent cell cytotoxicity (ADCC), in addition to the antigen-binding capacity, which is the main function of immunoglobulins. Additionally, the FcRn sequence present in the Fc region plays the role of regulating the IgG level in serum by increasing the in vivo half-life by conjugation to an in vivo FcRn receptor. In some embodiments, such functions can be reduced or altered in an Fc for use with the provided Fc fusion proteins.

In some embodiments, one or more amino acid modifications may be introduced into the Fc region of an ICOSL-Fc variant fusion provided herein, thereby generating an Fc region variant. In some embodiments, the Fc region variant has decreased effector function. There are many examples of changes or mutations to Fc sequences that can alter effector function. For example, WO 2000/42072, WO2006/019447, WO2012/125850, WO2015/107026, US2016/0017041 and Shields et al. J Biol. Chem. 9(2): 6591-6604 (2001) describe exemplary Fc variants with improved or diminished binding to FcRs. The contents of those publications are specifically incorporated herein by reference.

In some embodiments, the provided variant ICOSL-Fc fusions comprise an Fc region that exhibits reduced effector functions (also called inert Fc or effectorless Fc), which makes it a desirable candidate for applications in which the half-life of the ICOSL-Fc variant fusion *in vivo* is important yet certain effector functions (such as CDC and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the ICOSL-Fc variant fusion lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Pat. No. 5,500,362 (see, *e.g.* Hellstrom, I. et al. Proc. Nat'l Acad. Sci. USA 83:7059-7063 (1986)) and Hellstrom, I et al., Proc. Nat'l Acad. Sci. USA 82:1499-1502 (1985); U.S. Pat. No. 5,821,337 (see Bruggemann, M. et al., J. Exp. Med. 166:1351-1361 (1987)). Alternatively, non-radioactive assay methods may be employed (see, for example, ACTI™ non-radioactive cytotoxicity assay for flow cytometry (CellTechnology, Inc. Mountain View, Calif.; and CytoTox 96™ non-radioactive cytotoxicity assay (Promega, Madison, Wis.). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the ICOSL-Fc variant fusion is unable to bind C1q and hence lacks CDC activity. See, *e.g.,* C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M. S. et al., Blood 101:1045-1052 (2003); and Cragg, M. S. and M. J. Glennie, Blood 103:2738-2743 (2004)). FcRn binding and *in vivo* clearance/half life determinations can also be performed using methods known in the art (see, *e.g.,* Petkova, S. B. et al., Int'l. Immunol. 18(12):1759-1769 (2006)).

ICOSL-Fc variant fusions with reduced effector function include those with substitution of one or more of Fc region residues 238, 265, 269, 270, 297, 327 and 329 by EU numbering (U.S. Pat. No. 6,737,056). Such Fc mutants include Fc mutants with substitutions at two or more of amino acid positions 265, 269, 270, 297 and 327 by EU numbering, including the so-called "DANA" Fc mutant with substitution of residues 265 and 297 to alanine (U.S. Pat. No. 7,332,581).

In some embodiments, the Fc region of ICOSL-Fc variant fusions has an Fc region in which any one or more of amino acids at positions 234, 235, 236, 237, 238, 239, 270, 297, 298, 325, and 329 (indicated by EU numbering) are substituted with different amino acids compared to the native Fc region. Such alterations of Fc region are not limited to the above-described alterations, and include, for example, alterations such as deglycosylated chains (N297A and N297Q), IgG1-N297G, IgG1-L234A/L235A, IgG1-L234A/L235E/G237A, IgG1-A325A/A330S/P331S, IgG1-C226S/C229S, IgG1-C226S/C229S/E233P/L234V/L235A, IgG1-E233P/L234V/L235A/G236del/S267K, IgG1-L234F/L235E/P331S, IgG1-S267E/L328F, IgG2-V234A/G237A, IgG2-H268Q/V309L/A330S/A331S, IgG4-L235A/G237A/E318A, and IgG4-L236E described in Current Opinion in Biotechnology (2009) 20 (6), 685-691; alterations such as G236R/L328R, L235G/G236R, N325A/L328R, and N325LL328R described in WO 2008/092117; amino acid insertions at positions 233, 234, 235, and 237 (indicated by EU numbering); and alterations at the sites described in WO 2000/042072.

Certain Fc variants with improved or diminished binding to FcRs are described. (See, *e.g.,* U.S. Pat. No. 6,737,056; WO 2004/056312, WO2006/019447 and Shields et al., J. Biol. Chem. 9(2): 6591-6604 (2001).)

In some embodiments, there is provided a ICOSL-Fc variant fusion comprising a variant Fc region comprising one or more amino acid substitutions which increase half-life and/or improve binding to the neonatal Fc receptor (FcRn). Antibodies with increased half-lives and improved binding to FcRn are described in US2005/0014934A1 (Hinton et al.) or WO2015107026. Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434 by EU numbering, e.g., substitution of Fc region residue 434 (U.S. Pat. No. 7,371,826).

In some embodiments, the Fc region of a ICOSL-Fc variant fusion comprises one or more amino acid substitution E356D and M358L by EU numbering. In some embodiments, the Fc region of an ICOSL-Fc variant fusion comprises one or more amino acid substitutions C220S, C226S, and/or C229S by EU numbering. In some embodiments, the Fc region of a ICOSL variant fusion comprises one or more amino acid substitutions R292C and V302C. *See* also Duncan & Winter, Nature 322:738-40 (1988); U.S. Pat. No. 5,648,260; U.S. Pat. No. 5,624,821; and WO 94/29351 concerning other examples of Fc region variants.

In some embodiments, the wild-type IgG1 Fc can be the Fc set forth in SEQ ID NO: 37 having an allotype containing residues Glu (E) and Met (M) at positions 356 and 358 by EU numbering (e.g., f allotype). In other embodiments, the wild-type IgG1 Fc contains amino acids of the human G1m1 allotype, such as residues containing Asp (D) and Leu (L) at positions 356 and 358, e.g. as set forth in SEQ ID NO: 38. Thus, in some cases, an Fc provided herein can contain amino acid substitutions E356D and M358L to reconstitute residues of allotype G1 m1 (e.g., alpha allotype). In some aspects, a wild-type Fc is modified by one or more amino acid substitutions to reduce effector activity or to render the Fc inert for Fc effector function. Exemplary effectorless or inert mutations include those described herein. Among effectorless mutations that can be included in an Fc of constructs provided herein are L234A, L235E and G237A by EU numbering. In some embodiments, a wild-type Fc is further modified by the removal of one or more cysteine residue, such as by replacement of the cysteine residues to a serine residue at position 220 (C220S) by EU numbering. Exemplary inert Fc regions having reduced effector function are set forth in SEQ ID NO: 39 or 40 and SEQ ID NO: 41 or 42, which are based on allotypes set forth in SEQ ID NO: 37 or SEQ ID NO: 38, respectively. In some embodiments, an Fc region used in a construct provided herein can further lack a C-terminal lysine residue.

In some embodiments, alterations are made in the Fc region that result in diminished C1q binding and/or Complement Dependent Cytotoxicity (CDC), *e.g.,* as described in U.S. Pat. No. 6,194,551, WO 99/51642, and Idusogie et al., J. Immunol. 164: 4178-4184 (2000).

In some embodiments, there is provided a ICOSL-Fc variant fusion comprising a variant Fc region comprising one or more amino acid modifications, wherein the variant Fc region is derived from IgG1, such as human IgG1. In some embodiments, the variant Fc region is derived from the amino acid sequence set forth in SEQ ID NO: 37. In some embodiments, the Fc exhibits reduced effector function. In some embodiments, the Fc contains at least one amino acid substitution that is N82G by numbering of SEQ ID NO: 37 (corresponding to N297G by EU numbering). In some embodiments, the Fc further contains at least one amino acid substitution that is R77C or V87C by numbering of SEQ ID NO: 37 (corresponding to R292C or V302C by EU numbering). In some embodiments, the variant Fc region further comprises a C5S amino acid modification by numbering of SEQ ID NO: 37 (corresponding to C220S by EU numbering). For example, in some embodiments, the variant Fc region comprises the following amino acid modifications: V297G and one or more of the following amino acid modifications C220S, R292C or V302C by EU numbering (corresponding to N82G and one or more of the following amino acid modifications C5S, R77C or V87C with reference to SEQ ID NO:37), e.g. the Fc region comprises the sequence set forth in SEQ ID NO:43. In some embodiments, the variant Fc region comprises one or more of the amino acid modifications C220S, L234A, L235E or G237A, e.g. the Fc region comprises the sequence set forth in SEQ ID NO:40. In some embodiments, the variant Fc region comprises one or more of the amino acid modifications C220S, E233P, L234V, L235A, G236del or S267K, e.g. the Fc region comprises the sequence set forth in SEQ ID NO:44. In some embodiments, the variant Fc comprises one or more of the amino acid modifications C220S, L234A, L235E, G237A, E356D or M358L, e.g. the Fc region comprises the sequence set forth in SEQ ID NO:41.

In some embodiments, the Fc region lacks the C-terminal lysine corresponding to position 232 of the reference (e.g., unmodified) or wild-type Fc set forth in SEQ ID NO: 45 (corresponding to K447del by EU numbering). In some embodiments, because the C-terminal lysine may be differentially removed during biosynthesis, removal of the C-terminal lysine residue results in a more homogenous product when the protein is expressed in cells. In some aspects, such an Fc region can additionally include one or more additional modifications, e.g. amino acid substitutions, such as any as described. Exemplary of such an Fc region is set forth in SEQ ID NO: 46, 39, 47, or 42.

In some embodiments, there is provided a ICOSL-Fc variant fusion comprising a variant Fc region in which the variant Fc comprises the sequence of amino acids set forth in any of SEQ ID NOS:41, 43, 40, 44, 48, 46, 39, 47, or 42 or a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 41, 43, 40, 44, 48, 46, 39, 47, or 42. In some embodiments, the Fc exhibits reduced effector function.

In some embodiments, the Fc is derived from IgG2, such as human IgG2. In some embodiments, the Fc comprises the amino acid sequence set forth in SEQ ID NO: 49 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 49.

In some embodiments, the Fc comprises the amino acid sequence set forth in SEQ ID NO: 50 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 50. In some embodiments, the IgG4 Fc is a stabilized Fc in which the CH3 domain of human IgG4 is substituted with the CH3 domain of human IgG1 and which exhibits inhibited aggregate formation, an antibody in which the CH3 and CH2 domains of human IgG4 are substituted with the CH3 and CH2 domains of human IgG1, respectively, or an antibody in which arginine at position 409 indicated in the EU index proposed by Kabat et al. of human IgG4 is substituted with lysine and which exhibits inhibited aggregate formation (see e.g. U.S. Patent No. 8,911,726). In some embodiments, the Fc is an IgG4 containing the S228P mutation, which has been shown to prevent recombination between a therapeutic antibody and an endogenous IgG4 by Fab-arm exchange (see e.g. Labrijin et al. (2009) Nat. Biotechnol., 27(8)767-71.) In some embodiments, the Fc comprises the amino acid sequence set forth in SEQ ID NO: 51 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 51.

In some embodiments, the variant ICOSL polypeptide is directly linked to the Fc sequence. In some embodiments, the variant ICOSL polypeptide is indirectly linked to the Fc sequence, such as via a linker. In some embodiments, one or more "peptide linkers" link the variant ICOSL polypeptide and the Fc domain. In some embodiments, a peptide linker can be a single amino acid residue or greater in length. In some embodiments, the peptide linker has at least one amino acid residue but is no more than 20, 19, 18, 17, 16, 15, 14, 13, 12, 11, 10, 9, 8, 7, 6, 5, 4, 3, 2, or 1 amino acid residues in length. In some embodiments, the linker is three alanines (AAA). In some embodiments, the linker is (in one-letter amino acid code): GGGGS ("4GS" ; SEQ ID NO:52) or multimers of the 4GS linker, such as repeats of 2, 3, 4, 5 or 6 4GS linkers, such as set forth in SEQ ID NO: 53 (2xGGGGS) or SEQ ID NO: 54 (3xGGGGS). In some embodiments, the linker is a rigid linker. For example, the linker is an $\alpha$-helical linker. In some embodiments, the linker is (in one-letter amino acid code): EAAAK or multimers of the EAAAK linker, such as repeats of 2, 3, 4, or 5 4GS linkers, such as set forth in SEQ ID NO: 55 (EAAAK) or SEQ ID NO: 56 (3xEAAAK) or SEQ ID NO: 57 (5xEAAAK). In some embodiments, linkers start with one or more EAAAK units and can be lengthened by addition of A, AA, AAA, AAAA, EAAAA and EAAAK sequences. In some embodiments, the linker can further include amino acids introduced by cloning and/or from a restriction site, for example the linker can include the amino acids GS (in one-letter amino acid code) as introduced by use of the restriction site BAMHI. In some embodiments, the linker (in one-letter amino acid code) is GSGGGGS (SEQ ID NO: 58). In some examples, the linker is a 2xGGGGS followed by three alanines (GGGGSGGGGSAAA; SEQ ID NO: 59).

In some embodiments, the variant ICOSL-Fc fusion protein is a dimer formed by two variant ICOSL Fc polypeptides linked to an Fc domain. In some specific embodiments, identical or substantially identical species (allowing for 3 or fewer N-terminus or C-terminus amino acid sequence differences) of ICOSL-Fc variant fusion polypeptides will be dimerized to create a homodimer. In some embodiments, the dimer is a homodimer in which the two variant ICOSL Fc polypeptides are the same. Alternatively, different species of ICOSL-Fc variant fusion polypeptides can be dimerized to yield a heterodimer. Thus, in some embodiments, the dimer is a heterodimer in which the two variant ICOSL Fc polypeptides are different.

In some embodiments, provided is a variant ICOSL-Fc fusion protein containing a variant ICOSL polypeptide that includes one or more amino acid modifications in a reference ICOSL as described herein that is linked, directly or indirectly, to an Fc region. In some cases, the C-terminus of the variant ICOSL polypeptide is joined to the N-terminus of the Fc region. In some embodiments, the variant ICOSL of an ICOSL-Fc fusion contains one or more amino acid modifications in the sequence of amino acids the reference IgV domain set forth in SEQ ID NO:3. In particular cases, such an immunomodulatory protein contains variant ICOSL polypeptide containing an IgV domain, such as an IgV domain set forth in SEQ ID NO: 35, or an IgV domain that has at least 85%, 86%, 87%, 88%, 89%, 90%, 91%, 92%, 93%, 94%, 95%, 96%, 97%, 98%, 99% to SEQ ID NO: 35 and contains the one or more amino acid modifications of the respective SEQ ID NO, e.g. N52H/N57Y/Q100R. In particular cases, such an immunomodulatory protein contains variant ICOSL polypeptide containing an IgV domain, such as an IgV domain set forth in SEQ ID NO: 36, or an IgV domain that has at least 85%, 86%, 87%, 88%, 89%, 90%, 91%, 92%, 93%, 94%, 95%, 96%, 97%, 98%, 99% to SEQ ID NO: 36 and contains the one or more amino acid modifications of the respective SEQ ID NO, e.g. N52H/N57Y/Q100R. In some embodiments, the variant ICOSL polypeptide contains the amino acid modifications N52H/N57Y/Q100R (e.g. is or includes an IgV domain set forth in SEQ ID NO: 36).

In particular embodiments of such variant ICOSL-Fc fusion proteins, the Fc polypeptide is a variant of a human IgG1 Fc region that exhibits reduced effector functions, such as any as described. In some embodiments, the Fc region is a human IgG1 that contains the amino acid modifications N297G, E233P/L234V/L235A/G236del/S267K or L234A/L235E/G237A, wherein the residue is numbered according to the EU index of Kabat. In some embodiments, the variant IgG1 Fc region further contains the amino acid substitution C220S, wherein the residues are numbered according to the EU index of Kabat. In some embodiments, the Fc region contains K447del, wherein the residue is numbered according to the EU index of Kabat. In some aspects, the Fc region contains the sequence of amino acid sequence set forth in any of SEQ ID NOS: 41, 43, 40, 44, 39 or 42 or a sequence of amino acids that exhibits at least 85%, 90%, 91%, 92%, 93%, 94%, 95%, 96%, 97%, 98%, 99% or more sequence identity to any ofSEQ ID NOS: 41, 43, 40, 44, 39 or 42 and contains the amino acid substitutions of the respective SEQ ID NO. The linkage between the variant ICOSL IgSF (e.g. IgV) polypeptide and the Fc can be via a peptide linker, such as any as described. In some embodiments, the linker is GGGGS ("4GS"; SEQ ID NO: 52), SEQ ID NO: 53 (2xGGGGS) or SEQ ID NO: 54 (3xGGGGS). In particular examples, the C-terminus of the variant ICOSL polypeptide is joined to the N-terminus of the Fc region, such that the order of components is variant ICOSL-linker-Fc.

In some embodiments, there is provided a variant ICOSL-Fc fusion protein, e.g. variant ICOSL-linker-Fc, containing a variant ICOSL IgV domain set forth in SEQ ID NO:36, a linker set forth in SEQ ID NO:52 and an Fc polypeptide set forth in SEQ ID NO: 42. In some embodiments, there is provided a variant ICOSL-Fc fusion protein, e.g. variant ICOSL-linker-Fc, containing a variant ICOSL IgV domain set forth in SEQ ID NO:36, a linker set forth in SEQ ID NO:52 and an Fc polypeptide set forth in SEQ ID NO: 41. In some embodiments, there is provided a variant ICOSL-Fc fusion protein, e.g. variant ICOSL-linker-Fc, containing a variant ICOSL IgV domain set forth in SEQ ID NO:36, a linker set forth in SEQ ID NO:52 and an Fc polypeptide set forth in SEQ ID NO: 40. In some embodiments, there is provided a variant ICOSL-Fc fusion protein, e.g. variant ICOSL-linker-Fc, containing a variant ICOSL IgV domain set forth in SEQ ID NO:36, a linker set forth in SEQ ID NO:52 and an Fc polypeptide set forth in SEQ ID NO: 39.

In some embodiments, there is provided a variant ICOSL-Fc fusion protein, e.g. variant ICOSL-linker-Fc, containing a variant ICOSL IgV domain set forth in SEQ ID NO:36, a linker set forth in SEQ ID NO: 53 and an Fc polypeptide set forth in SEQ ID NO: 42. In some embodiments, there is provided a variant ICOSL-Fc fusion protein, e.g. variant ICOSL-linker-Fc, containing a variant ICOSL IgV domain set forth in SEQ ID NO:36, a linker set forth in SEQ ID NO:53 and an Fc polypeptide set forth in SEQ ID NO: 41. In some embodiments, there is provided a variant ICOSL-Fc fusion protein, e.g. variant ICOSL-linker-Fc, containing a variant ICOSL IgV domain set forth in SEQ ID NO:36, a linker set forth in SEQ ID NO: 53 and an Fc polypeptide set forth in SEQ ID NO: 40. In some embodiments, there is provided a variant ICOSL-Fc fusion protein, e.g. variant ICOSL-linker-Fc, containing a variant ICOSL IgV domain set forth in SEQ ID NO:36, a linker set forth in SEQ ID NO:53 and an Fc polypeptide set forth in SEQ ID NO: 39.

In some embodiments, there is provided a variant ICOSL-Fc fusion protein, e.g. variant ICOSL-linker-Fc, containing a variant ICOSL IgV domain set forth in SEQ ID NO:36, a linker set forth in SEQ ID NO:54 and an Fc polypeptide set forth in SEQ ID NO: 42. In some embodiments, there is provided a variant ICOSL-Fc fusion protein, e.g. variant ICOSL-linker-Fc, containing a variant ICOSL IgV domain set forth in SEQ ID NO:36, a linker set forth in SEQ ID NO: 54 and an Fc polypeptide set forth in SEQ ID NO: 41. In some embodiments, there is provided a variant ICOSL-Fc fusion protein, e.g. variant ICOSL-linker-Fc, containing a variant ICOSL IgV domain set forth in SEQ ID NO:36, a linker set forth in SEQ ID NO:54 and an Fc polypeptide set forth in SEQ ID NO: 40. In some embodiments, there is provided a variant ICOSL-Fc fusion protein, e.g. variant ICOSL-linker-Fc, containing a variant ICOSL IgV domain set forth in SEQ ID NO: 36, a linker set forth in SEQ ID NO:54 and an Fc polypeptide set forth in SEQ ID NO: 39.

In some embodiments, there is provided a variant ICOSL IgSF Fc fusion protein that has the sequence of amino acids set forth in SEQ ID NO: 60, or a sequence of amino acids that exhibits at least 85%, 90%, 91%, 92%, 93%, 94%, 95%, 96%, 97%, 98%, 99% to SEQ ID NO:60. In some embodiments, the variant ICOSL IgSF Fc fusion protein binds to CD28 and ICOS, such as with increased binding affinity compared to reference (wild-type) ICOSL-Fc fusion protein. In some embodiments, the variant ICOSL IgSF Fc fusion exhibits reduced Fc effector function compared to fusion with an Fc of a wild-type human IgG1.

## III. NUCLEIC ACIDS, VECTORS AND METHODS FOR PRODUCING THE POLYPEPTIDES OR CELLS

Provided herein are isolated or recombinant nucleic acids collectively referred to as "nucleic acids" which encode any of the various provided embodiments of the variant ICOSL polypeptides or immunomodulatory fusion polypeptides provided herein. In some embodiments, nucleic acids provided herein, including all described below, are useful in recombinant production (e.g., expression) of variant ICOSL polypeptides or immunomodulatory fusion polypeptides provided herein. The nucleic acids provided herein can be in the form of RNA or in the form of DNA, and include mRNA, cRNA, recombinant or synthetic RNA and DNA, and cDNA. The nucleic acids provided herein are typically DNA molecules, and usually double-stranded DNA molecules. However, single-stranded DNA, single-stranded RNA, double-stranded RNA, and hybrid DNA/RNA nucleic acids or combinations thereof comprising any of the nucleotide sequences of the invention also are provided.

Also provided herein are recombinant expression vectors and recombinant host cells useful in producing the variant ICOSL polypeptides or immunomodulatory fusion polypeptides provided herein.

In any of the above provided embodiments, the nucleic acids encoding the variant polypeptides or immunomodulatory fusion polypeptides provided herein can be introduced into cells using recombinant DNA and cloning techniques. To do so, a recombinant DNA molecule encoding an immunomodulatory polypeptide is prepared. Methods of preparing such DNA molecules are well known in the art. For instance, sequences coding for the peptides could be excised from DNA using suitable restriction enzymes. Alternatively, the DNA molecule could be synthesized using chemical synthesis techniques, such as the phosphoramidite method. Also, a combination of these techniques could be used. In some instances, a recombinant or synthetic nucleic acid may be generated through polymerase chain reaction (PCR). In some embodiments, a DNA insert can be generated encoding one or more variant ICOSL polypeptides containing at least one affinity-modified IgSF domain in accord with the provided description. This DNA insert can be cloned into an appropriate transduction/transfection vector as is known to those of skill in the art. Also provided are expression vectors containing the nucleic acid molecules.

In some embodiments, the expression vectors are capable of expressing the immunomodulatory proteins in an appropriate cell under conditions suited to expression of the protein. In some aspects, nucleic acid molecule or an expression vector comprises the DNA molecule that encodes the immunomodulatory protein operatively linked to appropriate expression control sequences. Methods of effecting this operative linking, either before or after the DNA molecule is inserted into the vector, are well known. Expression control sequences include promoters, activators, enhancers, operators, ribosomal binding sites, start signals, stop signals, cap signals, polyadenylation signals, and other signals involved with the control of transcription or translation.

In some embodiments, expression of the immunomodulatory protein is controlled by a promoter or enhancer to control or regulate expression. The promoter is operably linked to the portion of the nucleic acid molecule encoding the variant polypeptide or immunomodulatory protein. In some embodiments, an inducible promoter is operatively linked to the nucleic acid molecule encoding the variant polypeptide or immunomodulatory protein such that expression of the nucleic acid is controllable by controlling the presence or absence of the appropriate inducer of transcription. For example, the promoter can be a regulated promoter and transcription factor expression system, such as the published tetracycline-regulated systems or other regulatable systems (see, e.g. published International PCT Appl. No. WO 01/30843), to allow regulated expression of the encoded polypeptide. An exemplary regulatable promoter system is the Tet-On (and Tet-Off) system available, for example, from Clontech (Palo Alto, CA). This promoter system allows the regulated expression of the transgene controlled by tetracycline or tetracycline derivatives, such as doxycycline. Other regulatable promoter systems are known (see e.g., published U.S. Application No. 2002-0168714, entitled "Regulation of Gene Expression Using Single-Chain, Monomeric, Ligand Dependent Polypeptide Switches," which describes gene switches that contain ligand binding domains and transcriptional regulating domains, such as those from hormone receptors).

The resulting recombinant expression vector having the DNA molecule thereon is used to transform an appropriate host. This transformation can be performed using methods well known in the art. In some embodiments, a nucleic acid provided herein further comprises nucleotide sequence that encodes a secretory or signal peptide operably linked to the nucleic acid encoding an immunomodulatory polypeptide such that a resultant soluble immunomodulatory polypeptide is recovered from the culture medium, host cell, or host cell periplasm. Furthermore, commercially available kits as well as contract manufacturing companies can also be utilized to make engineered cells or recombinant host cells provided herein.

In some embodiments, the resulting expression vector having the DNA molecule thereon is used to transform, such as transduce, an appropriate cell. The introduction can be performed using methods well known in the art. Exemplary methods include those for transfer of nucleic acids encoding the receptors, including via viral, e.g., retroviral or lentiviral, transduction, transposons, and electroporation. In some embodiments, the expression vector is a viral vector. In some embodiments, the nucleic acid is transferred into cells by lentiviral or retroviral transduction methods.

Any of a large number of publicly available and well-known mammalian host cells can be used in the preparing the polypeptides. The selection of a cell is dependent upon a number of factors recognized by the art. These include, for example, compatibility with the chosen expression vector, toxicity of the peptides encoded by the DNA molecule, rate of transformation, ease of recovery of the peptides, expression characteristics, bio-safety and costs. A balance of these factors must be struck with the understanding that not all cells can be equally effective for the expression of a particular DNA sequence.

In some embodiments, the host cells can be a variety of eukaryotic cells, such as in yeast cells, or with mammalian cells such as Chinese hamster ovary (CHO) or HEK293 cells. In some embodiments, the host cell is a suspension cell and the polypeptide is engineered or produced in cultured suspension, such as in cultured suspension CHO cells, e.g. CHO-S cells. In some examples, the cell line is a CHO cell line that is deficient in DHFR (DHFR-), such as DG44 and DUXB11. In some embodiments, the cell is deficient in glutamine synthase (GS), e.g. CHO-S cells, CHOK1 SV cells, and CHOZN((R)) GS-/- cells. In some embodiments, the CHO cells, such as suspension CHO cells, may be CHO-S-2H2 cells, CHO-S-clone 14 cells, or ExpiCHO-S cells.

In some embodiments, expressing the provided ICOSL polypeptides from CHO cells results in a more homogenous composition of produced proteins. In some embodiments, the provided ICOSL polypeptides results in a more homogenous product when the proteins are expressed from CHO cells compared to ICOSL polypeptides containing the full ECD reference sequence and/or containing the protease cleavage site (e.g., LQQN/LT). In some embodiments, at least 91%, 92%, 93%, 94%, 95%, 96%, 97%, 98%, or 99% of the composition of produced proteins containing an ICOSL variant polypeptide produced herein, have the same amino acid length or are the same size. Techniques to assess homogeneity of size include high performance liquid chromatography (HPLC), size exclusion chromatography, SDS page, or sequencing.

In some embodiments, host cells can also be prokaryotic cells, such as with *E. coli.* The transformed recombinant host is cultured under polypeptide expressing conditions, and then purified to obtain a soluble protein. Recombinant host cells can be cultured under conventional fermentation conditions so that the desired polypeptides are expressed. Such fermentation conditions are well known in the art. Finally, the polypeptides provided herein can be recovered and purified from recombinant cell cultures by any of a number of methods well known in the art, including ammonium sulfate or ethanol precipitation, acid extraction, anion or cation exchange chromatography, phosphocellulose chromatography, hydrophobic interaction chromatography, and affinity chromatography. Protein refolding steps can be used, as desired, in completing configuration of the mature protein. Finally, high performance liquid chromatography (HPLC) can be employed in the final purification steps.

In some embodiments, the recombinant vector is a viral vector. Exemplary recombinant viral vectors include a lentiviral vector genome, poxvirus vector genome, vaccinia virus vector genome, adenovirus vector genome, adenovirus-associated virus vector genome, herpes virus vector genome, and alpha virus vector genome. Viral vectors can be live, attenuated, replication conditional or replication deficient, non-pathogenic (defective), replication competent viral vector, and/or is modified to express a heterologous gene product, e.g., the variant immunomodulatory polypeptides provided herein. Vectors for generation of viruses also can be modified to alter attenuation of the virus, which includes any method of increasing or decreasing the transcriptional or translational load.

Exemplary viral vectors that can be used include modified vaccinia virus vectors (see, e.g., Guerra et al., J. Virol. 80:985-98 (2006); Tartaglia et al., AIDS Research and Human Retroviruses 8: 1445-47 (1992); Gheradi et al., J. Gen. Virol. 86:2925-36 (2005); Mayr et al., Infection 3:6-14 (1975); Hu et al., J. Virol. 75: 10300-308 (2001); U.S. Patent Nos. 5,698,530, 6,998,252, 5,443,964, 7,247,615 and 7,368,116); adenovirus vector or adenovirus-associated virus vectors (see., e.g., Molin et al., J. Virol. 72:8358-61 (1998); Narumi et al., Am J. Respir. Cell Mol. Biol. 19:936-41 (1998); Mercier et al., Proc. Natl. Acad. Sci. USA 101:6188-93 (2004); U.S. Patent Nos. 6,143,290; 6,596,535; 6,855,317; 6,936,257; 7,125,717; 7,378,087; 7,550,296); retroviral vectors including those based upon murine leukemia virus (MuLV), gibbon ape leukemia virus (GaLV), ecotropic retroviruses, simian immunodeficiency virus (SIV), human immunodeficiency virus (HIV), and combinations (see, e.g., Buchscher et al., J. Virol. 66:2731-39 (1992); Johann et al., J. Virol. 66: 1635-40 (1992); Sommerfelt et al., Virology 176:58-59 (1990); Wilson et al., J. Virol. 63:2374-78 (1989) ; Miller et al., J. Virol. 65:2220-24 (1991); Miller et al., Mol. Cell Biol. 10:4239 (1990) ; Kolberg, NIH Res. 4:43 1992; Cometta et al., Hum. Gene Ther. 2:215 (1991)); lentiviral vectors including those based upon Human Immunodeficiency Virus (HIV-1), HIV-2, feline immunodeficiency virus (FIV), equine infectious anemia virus, Simian Immunodeficiency Virus (SIV), and maedi/visna virus (see, e.g., Pfeifer et al., Annu. Rev. Genomics Hum. Genet. 2: 177-211 (2001); Zufferey et al., J. Virol. 72: 9873, 1998; Miyoshi et al., J. Virol. 72:8150, 1998; Philpott and Thrasher, Human Gene Therapy 18:483, 2007; Engelman et al., J. Virol. 69: 2729, 1995; Nightingale et al., Mol. Therapy, 13: 1121, 2006; Brown et al., J. Virol. 73:9011 (1999); WO 2009/076524; WO 2012/141984; WO 2016/011083; McWilliams et al., J. Virol. 77: 11150, 2003; Powell et al., J. Virol. 70:5288, 1996) or any, variants thereof, and/or vectors that can be used to generate any of the viruses described above. In some embodiments, the recombinant vector can include regulatory sequences, such as promoter or enhancer sequences, that can regulate the expression of the viral genome, such as in the case for RNA viruses, in the packaging cell line (see, e.g., U.S. Patent Nos.5,385,839 and 5,168,062).

In some embodiments, polypeptides provided herein can also be made by synthetic methods. Solid phase synthesis is the preferred technique of making individual peptides since it is the most cost-effective method of making small peptides. For example, well known solid phase synthesis techniques include the use of protecting groups, linkers, and solid phase supports, as well as specific protection and deprotection reaction conditions, linker cleavage conditions, use of scavengers, and other aspects of solid phase peptide synthesis. Peptides can then be assembled into the polypeptides as provided herein.

## IV. METHODS OF ASSESSING ACTIVITY IMMUNE MODULATION OF VARIANT ICOSL POLYPEPTIDES AND IMMUNOMODULATORY PROTEINS

In some embodiments, the variant ICOSL polypeptides provided herein (e.g. full-length and/or specific binding fragments or immunomodulatory fusion thereof) exhibit immunomodulatory activity to modulate T cell activation. In some embodiments, ICOSL polypeptides modulate IFN-gamma expression in a primary T cell assay relative to a reference (e.g., unmodified) or wild-type ICOSL control. In some cases, modulation of IFN-gamma expression can increase or decrease IFN-gamma expression relative to the control. Assays to determine specific binding and IFN-gamma expression are well-known in the art and include the MLR (mixed lymphocyte reaction) assays measuring interferon-gamma cytokine levels in culture supernatants (Wang et al., Cancer Immunol Res. 2014 Sep: 2(9):846-56), SEB (staphylococcal enterotoxin B) T cell stimulation assay (Wang et al., Cancer Immunol Res. 2014 Sep: 2(9):846-56), and anti-CD3 T cell stimulation assays (Li and Kurlander, J Transl Med. 2010: 8: 104).

In some embodiments, a variant ICOSL polypeptide can in some embodiments increase or, in alternative embodiments, decrease IFN-gamma (interferon-gamma) expression in a primary T-cell assay relative to a wild-type ICOSL control. In some embodiments of the provided polypeptides containing a soluble variant ICOSL sequence, the polypeptide can increase IFN-gamma expression and, in alternative embodiments, decrease IFN-gamma expression in a primary T-cell assay relative to a wild-type ICOSL control. In some embodiments of the provided polypeptides containing multiple variant ICOSL sequences, the polypeptide can increase IFN-gamma expression and, in alternative embodiments, decrease IFN-gamma expression in a primary T-cell assay relative to a wild-type ICOSL control.

Those of skill will recognize that the format of the primary T-cell assay used to determine an increase in IFN-gamma expression can differ from that employed to assay for a decrease in IFN-gamma expression. In assaying for the ability of a variant ICOSL to decrease IFN-gamma expression in a primary T-cell assay, a Mixed Lymphocyte Reaction (MLR) assay can be used. In some cases, a soluble form of a variant ICOSL can be employed to determine the ability of the variant ICOSL to antagonize and thereby decrease the IFN-gamma expression in a MLR. Alternatively, in assaying for the ability of a variant ICOSL to increase IFN-gamma expression in a primary T-cell assay, a co-immobilization assay can be used. In a co-immobilization assay, a TCR signal, provided in some embodiments by anti-CD3 antibody, is used in conjunction with a co-immobilized variant ICOSL to determine the ability to increase IFN-gamma expression relative to an ICOSL control.

In some embodiments, in assaying for the ability of a variant ICOSL to modulate an increase or decrease IFN-gamma expression a T cell reporter assay can be used. In some embodiments, the T cell is a Jurkat T cell line or is derived from Jurkat T cell lines. In reporter assays, the reporter cell line (e.g. Jurkat reporter cell) also is generated to overexpress a cognate binding partner of the variant IgSF domain polypeptide. In some embodiments, the reporter T cells also contain a reporter construct containing an inducible promoter responsive to T cell activation operably linked to a reporter. In some embodiments, the reporter is a fluorescent or luminescent reporter. In some embodiments, the reporter is luciferase. In some embodiments, the promoter is responsive to CD3 signaling. In some embodiments, the promoter is an NFAT promoter. In some embodiments, the promoter is responsive to costimulatory signaling, e.g. CD28 costimulatory signaling. In some embodiments, the promoter is an IL-2 promoter.

In aspects of a reporter assay, a reporter cell line is stimulated, such as by co-incubation with antigen presenting cells (APCs) expressing the wild-type ligand of the inhibitory receptor, e.g. ICOSL. In some embodiments, the APCs are artificial APCs. Artificial APCs are well known to a skilled artisan. In some embodiments, artificial APCs are derived from one or more mammalian cell line, such as K562, CHO or 293 cells.

In some embodiments, the Jurkat reporter cells are co-incubated with artificial APCs overexpressing the ligand in the presence of the variant IgSF domain molecule or immunomodulatory protein, e.g., variant ICOSL polypeptide or immunomodulatory protein. In some embodiments, reporter expression is monitored, such as by determining the luminescence or fluorescence of the cells. In some embodiments, normal interactions between its receptor and ligand result in a repression of or decrease in the reporter signal, such as compared to control, e.g. reporter expression by co-incubation of control T cells and APCs in which the receptor and ligand interaction is not present, e.g. APCs that do not overexpress ICOSL. In some embodiments, a variant ICOSL polypeptide or immunomodulatory protein provided herein antagonizes the interaction, e.g. when provided in soluble form as a variant ICOSL-Fc, thereby resulting in an increase in the reporter signal compared to the absence of the variant ICOSL polypeptide or immunomodulatory protein.

Use of proper controls is known to those of skill in the art, however, in the aforementioned embodiments, the control typically involves use of the reference ICOSL, such as a wild-type of native ICOSL isoform from the same mammalian species from which the variant ICOSL was derived or developed. Irrespective of whether the binding affinity to either one or both of ICOS and CD28 is increased or decreased, a variant ICOSL in some embodiments will increase IFN-gamma expression and, in alternative embodiments, decrease IFN-gamma expression in a primary T-cell assay relative to a wild-type ICOSL control.

In some embodiments, a variant ICOSL increases IFN-gamma expression (i.e., protein expression) relative to a reference (e.g., unmodified) or wild-type ICOSL control by at least: 5%, 10%, 20%, 30%, 40%, 50%, 60%, 70%, 80%, 90%, or higher. In other embodiments, a variant ICOSL decreases IFN-gamma expression (i.e. protein expression) relative to a wild-type or unmodified ICOSL control by at least: 5%, 10%, 20%, 30%, 40%, 50%, 60%, 70%, 80%, 90%, or higher. In some embodiments, the wild-type ICOSL control is murine ICOSL, such as would typically be used for a variant ICOSL altered in sequence from that of a wild-type murine ICOSL sequence. In some embodiments, the wild-type ICOSL control is human ICOSL, such as would typically be used for a variant ICOSL altered in sequence from that of a wild-type human ICOSL sequence such as an ICOSL sequence comprising the sequence of amino acids of SEQ ID NO:1 or SEQ ID NO:2 or 3.

## V. PHARMACEUTICAL FORMULATIONS

Provided herein are compositions containing any of the variant ICOSL polypeptides, such as immunodulatory fusion proteins described herein. The pharmaceutical composition can further comprise a pharmaceutically acceptable excipient. For example, the pharmaceutical composition can contain one or more excipients for modifying, maintaining or preserving, for example, the pH, osmolarity, viscosity, clarity, color, isotonicity, odor, sterility, stability, rate of dissolution or release, adsorption, or penetration of the composition. In some aspects, a skilled artisan understands that a pharmaceutical composition containing cells may differ from a pharmaceutical composition containing a protein.

In some embodiments, the pharmaceutical composition is a solid, such as a powder, capsule, or tablet. For example, the components of the pharmaceutical composition can be lyophilized. In some embodiments, the solid pharmaceutical composition is reconstituted or dissolved in a liquid prior to administration.

In some embodiments, the pharmaceutical composition is a liquid, for example variant ICOSL polypeptides dissolved in an aqueous solution (such as physiological saline or Ringer's solution). In some embodiments, the pH of the pharmaceutical composition is between about 4.0 and about 8.5 (such as between about 4.0 and about 5.0, between about 4.5 and about 5.5, between about 5.0 and about 6.0, between about 5.5 and about 6.5, between about 6.0 and about 7.0, between about 6.5 and about 7.5, between about 7.0 and about 8.0, or between about 7.5 and about 8.5).

In some embodiments, the pharmaceutical composition comprises a pharmaceutically-acceptable excipient, for example a filler, binder, coating, preservative, lubricant, flavoring agent, sweetening agent, coloring agent, a solvent, a buffering agent, a chelating agent, or stabilizer. Examples of pharmaceutically-acceptable fillers include cellulose, dibasic calcium phosphate, calcium carbonate, microcrystalline cellulose, sucrose, lactose, glucose, mannitol, sorbitol, maltol, pregelatinized starch, corn starch, or potato starch. Examples of pharmaceutically-acceptable binders include polyvinylpyrrolidone, starch, lactose, xylitol, sorbitol, maltitol, gelatin, sucrose, polyethylene glycol, methyl cellulose, or cellulose. Examples of pharmaceutically-acceptable coatings include hydroxypropyl methylcellulose (HPMC), shellac, corn protein zein, or gelatin. Examples of pharmaceutically-acceptable disintegrants include polyvinylpyrrolidone, carboxymethyl cellulose, or sodium starch glycolate. Examples of pharmaceutically-acceptable lubricants include polyethylene glycol, magnesium stearate, or stearic acid. Examples of pharmaceutically-acceptable preservatives include methyl parabens, ethyl parabens, propyl paraben, benzoic acid, or sorbic acid. Examples of pharmaceutically-acceptable sweetening agents include sucrose, saccharine, aspartame, or sorbitol. Examples of pharmaceutically-acceptable buffering agents include carbonates, citrates, gluconates, acetates, phosphates, or tartrates.

In some embodiments, the pharmaceutical composition further comprises an agent for the controlled or sustained release of the product, such as injectable microspheres, bio-erodible particles, polymeric compounds (polylactic acid, polyglycolic acid), beads, or liposomes.

In some embodiments, the pharmaceutical composition is sterile. Sterilization may be accomplished by filtration through sterile filtration membranes or radiation. Where the composition is lyophilized, sterilization using this method may be conducted either prior to or following lyophilization and reconstitution. The composition for parenteral administration may be stored in lyophilized form or in solution. In addition, parenteral compositions generally are placed into a container having a sterile access port, for example, an intravenous solution bag or vial having a stopper pierceable by a hypodermic injection needle.

Such a formulation may, for example, be in a form suitable for intravenous infusion. A pharmaceutically acceptable carrier may be a pharmaceutically acceptable material, composition, or vehicle that is involved in carrying or transporting cells of interest from one tissue, organ, or portion of the body to another tissue, organ, or portion of the body. For example, the carrier may be a liquid or solid filler, diluent, excipient, solvent, or encapsulating material, or some combination thereof. Each component of the carrier must be "pharmaceutically acceptable" in that it must be compatible with the other ingredients of the formulation. It also must be suitable for contact with any tissue, organ, or portion of the body that it may encounter, meaning that it must not carry a risk of toxicity, irritation, allergic response, immunogenicity, or any other complication that excessively outweighs its therapeutic benefits.

In some embodiments, the pharmaceutical composition is formulated to contain an amount of a variant ICOSL polypeptide, e.g. variant ICOSL IgSF-Fc, of from at or about 1 mg to at or about 100 mg, such as from at or about 1 mg to at or about 75 mg, from at or about 1 mg to at or about 50 mg, from at or about 1 mg to at or about 25 mg, from at or about 1 mg to at or about 10 mg, from at or about 1 mg to at or about 5 mg, from at or about 5 mg to at or about 100 mg, from at or about 5 mg to at or about 75 mg, from at or about 5 mg to at or about 50 mg, from at or about 5 mg to at or about 25 mg, from at or about 5 mg to at or about 10 mg, from at or about 10 mg to at or about 100 mg, from at or about 10 mg to at or about 75 mg, from at or about 10 mg to at or about 50 mg, from at or about 10 mg to at or about 25 mg, from at or about 25 mg to at or about 100 mg, from at or about 25 mg to at or about 75 mg, from at or about 25 mg to at or about 50 mg, from at or about 50 mg to at or about 100 mg, from at or about 50 mg to at or about 75 mg or from at or about 75 mg to at or about 100 mg. In some embodiments, the pharmaceutical composition is formulated to contain an amount of a variant ICOSL polypeptide, e.g. variant ICOSL IgSF-Fc, that is at or about 10 mg, at or about 25 mg, at or about 50 mg, at or about 75 mg or at or about 100 mg.

In some embodiments, the pharmaceutical composition is formulated in a volume that is from at or about 0.5 mL to at or about 10 mL, such as from at or about 0.5 mL to at or about 5 mL, from at or about 0.5 mL to at or about 2 mL, from at or about 0.5 mL to at or about 1 mL, from at or about 1 mL to at or about 10 mL, from at or about 1 mL to at or about 5 mL or from at or about 5 mL to at or about 10 mL. In some embodiments, the pharmaceutical composition is formulated in a volume that is at or about 0.5 mL, at or about 1 mL, at or about 2 mL, at or about 2.5 mL, at or about 3 mL, at or about 4 mL, at or about 5 mL, at or about 6 mL, at or about 7 mL, at or about 8 mL, at or about 9 mL or at or about 10 mL. In some embodiments, the concentration of the composition is from at or about 1 mg/mL to at or about 50 mg/mL, such as from at or about 1 mg/mL to at or about 25 mg/mL, from at or about 1 mg/mL to at or about 15 mg/mL, from at or about 1 mg mL to at or about 5 mg/mL, from at or about 5 mg/mL to at or about 50 mg/mL, from at or about 5 mg/mL to at or about 25 mg/mL, from at or about 5 mg/mL to at or about 15 mg/mL, from at or about 15 mg/mL to at or about 50 mg/mL, from at or about 15 mg/mL to at or about 25 mg/mL or from at or about 25 mg/mL to at or about 50 mg/mL. In some embodiments, the concentration of the composition is from at or about 1 mg/mL, at or about 5 mg/mL, at or about 10 mg/mL, at or about 15 mg/mL, at or about 20 mg/mL, at or about 25 mg/mL, at or about 30 mg/mL, at or about 40 mg/mL or at or about 50 mg/mL. Provided herein are any of such compositions contained in a container such as a vial. In particular aspects, the container, such as vial, is sterile.

In some embodiments, the pharmaceutical composition is administered to a subject. Generally, dosages and routes of administration of the pharmaceutical composition are determined according to the size and condition of the subject, according to standard pharmaceutical practice. For example, the therapeutically effective dose can be estimated initially either in cell culture assays or in animal models such as mice, rats, rabbits, dogs, pigs, or monkeys. An animal model may also be used to determine the appropriate concentration range and route of administration. Such information can then be used to determine useful doses and routes for administration in humans. The exact dosage will be determined in light of factors related to the subject requiring treatment. Dosage and administration are adjusted to provide sufficient levels of the active compound or to maintain the desired effect. Factors that may be taken into account include the severity of the disease state, the general health of the subject, the age, weight, and gender of the subject, time and frequency of administration, drug combination(s), reaction sensitivities, and response to therapy.

Long-acting pharmaceutical compositions may be administered every 3 to 4 days, every week, or biweekly depending on the half-life and clearance rate of the particular formulation. The frequency of dosing will depend upon the pharmacokinetic parameters of the molecule in the formulation used. Typically, a composition is administered until a dosage is reached that achieves the desired effect. The composition may therefore be administered as a single dose, or as multiple doses (at the same or different concentrations/dosages) over time, or as a continuous infusion. Further refinement of the appropriate dosage is routinely made. Appropriate dosages may be ascertained through use of appropriate dose-response data. A number of biomarkers or physiological markers for therapeutic effect can be monitored including T cell activation or proliferation, cytokine synthesis or production (e.g., production of TNF-α, IFN-γ, IL-2), induction of various activation markers (e.g., CD25, IL-2 receptor), inflammation, joint swelling or tenderness, serum level of C-reactive protein, anti-collagen antibody production, and/or T cell-dependent antibody response(s).

In some embodiments, the pharmaceutical composition is administered to a subject through any route, including orally, transdermally, by inhalation, intravenously, intra-arterially, intramuscularly, direct application to a wound site, application to a surgical site, intraperitoneally, by suppository, subcutaneously, intradermally, transcutaneously, by nebulization, intrapleurally, intraventricularly, intra-articularly, intraocularly, or intraspinally. In some embodiments, the pharmaceutical composition is administered to a subject intravitreally.

In some embodiments, the dosage of the pharmaceutical composition is a single dose or a repeated dose. In some embodiments, the doses are given to a subject once per day, twice per day, three times per day, or four or more times per day. In some embodiments, about 1 or more (such as about 2 or more, about 3 or more, about 4 or more, about 5 or more, about 6 or more, or about 7 or more) doses are given in a week. In some embodiments, multiple doses are given over the course of days, weeks, months, or years. In some embodiments, a course of treatment is about 1 or more doses (such as about 2 or more does, about 3 or more doses, about 4 or more doses, about 5 or more doses, about 7 or more doses, about 10 or more doses, about 15 or more doses, about 25 or more doses, about 40 or more doses, about 50 or more doses, or about 100 or more doses).

In some embodiments, an administered dose of the pharmaceutical composition is about 1 μg of protein per kg subject body mass or more (such as about 2 μg of protein per kg subject body mass or more, about 5 μg of protein per kg subject body mass or more, about 10 μg of protein per kg subject body mass or more, about 25 μg of protein per kg subject body mass or more, about 50 μg of protein per kg subject body mass or more, about 100 μg of protein per kg subject body mass or more, about 250 μg of protein per kg subject body mass or more, about 500 μg of protein per kg subject body mass or more, about 1 mg of protein per kg subject body mass or more, about 2 mg of protein per kg subject body mass or more, or about 5 mg of protein per kg subject body mass or more). In some embodiments, an administered dose of the pharmaceutical composition is or is about 0.3 mg of protein per kg subject body mass. In some embodiments, an administered dose of the pharmaceutical composition is or is about 1 mg of protein per kg subject body mass. In some embodiments, an administered dose of the pharmaceutical composition is or is about 3 mg of protein per kg subject body mass. In some embodiments, an administered dose of the pharmaceutical composition is or is about 6 mg of protein per kg subject body mass. In some embodiments, an administered dose of the pharmaceutical composition is or is about 10 mg of protein per kg subject body mass. In some embodiments, an administered dose of the pharmaceutical composition is or is about 15 mg of protein per kg subject body mass. In some embodiments, an administered dose of the pharmaceutical composition is or is about 20 mg of protein per kg subject body mass.

A variety of means are known for determining if administration of a therapeutic composition of the invention sufficiently modulates immunological activity by eliminating, sequestering, or inactivating immune cells mediating or capable of mediating an undesired immune response; inducing, generating, or turning on immune cells that mediate or are capable of mediating a protective immune response; changing the physical or functional properties of immune cells; or a combination of these effects. Examples of measurements of the modulation of immunological activity include, but are not limited to, examination of the presence or absence of immune cell populations (using flow cytometry, immunohistochemistry, histology, electron microscopy, polymerase chain reaction (PCR)); measurement of the functional capacity of immune cells including ability or resistance to proliferate or divide in response to a signal (such as using T-cell proliferation assays and pepscan analysis based on 3H-thymidine incorporation following stimulation with anti-CD3 antibody, anti-T-cell receptor antibody, anti-CD28 antibody, calcium ionophores, PMA (phorbol 12-myristate 13-acetate) antigen presenting cells loaded with a peptide or protein antigen; B cell proliferation assays); measurement of the ability to kill or lyse other cells (such as cytotoxic T cell assays); measurements of the cytokines, chemokines, cell surface molecules, antibodies and other products of the cells (e.g., by flow cytometry, enzyme-linked immunosorbent assays, Western blot analysis, protein microarray analysis, immunoprecipitation analysis); measurement of biochemical markers of activation of immune cells or signaling pathways within immune cells (e.g., Western blot and immunoprecipitation analysis of tyrosine, serine or threonine phosphorylation, polypeptide cleavage, and formation or dissociation of protein complexes; protein array analysis; DNA transcriptional, profiling using DNA arrays or subtractive hybridization); measurements of cell death by apoptosis, necrosis, or other mechanisms (e.g., annexin V staining, TUNEL assays, gel electrophoresis to measure DNA laddering, histology; fluorogenic caspase assays, Western blot analysis of caspase substrates); measurement of the genes, proteins, and other molecules produced by immune cells (e.g., Northern blot analysis, polymerase chain reaction, DNA microarrays, protein microarrays, 2-dimensional gel electrophoresis, Western blot analysis, enzyme linked immunosorbent assays, flow cytometry); and measurement of clinical symptoms or outcomes such as improvement of autoimmune, neurodegenerative, and other diseases involving self-proteins or self-polypeptides (clinical scores, requirements for use of additional therapies, functional status, imaging studies) for example, by measuring relapse rate or disease severity (using clinical scores known to the ordinarily skilled artisan) in the case of multiple sclerosis, measuring blood glucose in the case of type I diabetes, or joint inflammation in the case of rheumatoid arthritis.

## VI. ARTICLES OF MANUFACTURE AND KITS

Also provided herein are articles of manufacture comprising the pharmaceutical compositions described herein (including pharmaceutical composition comprising the variant ICOSL IgSF domain fusion proteins) in suitable packaging. Among suitable packaging for articles of manufacture include one or more containers, typically a plurality of containers, packaging material, and a label or package insert on or associated with the container or containers and/or packaging, generally including instructions for administration of the composition to a subject. Suitable containers for packaging for compositions described herein are known in the art, and include, for example, vials (such as sealed vials), vessels, ampules, bottles, jars, flexible packaging (e.g., sealed Mylar or plastic bags), and the like. These articles of manufacture may further be sterilized and/or sealed.

The article of manufacture may further include a package insert or label with one or more pieces of identifying information and/or instructions for use. In some embodiments, the information or instructions indicates that the contents can or should be used to treat a particular condition or disease, and/or providing instructions therefor. The label or package insert may indicate that the contents of the article of manufacture are to be used for treating the disease or condition. In some embodiments, the label or package insert provides instructions to treat a subject, e.g., according to any of the embodiments of the provided methods. In some embodiments, the instructions specify administering one or more of the unit doses to the subject.

Further provided are kits comprising the pharmaceutical compositions (or articles of manufacture) described herein, which may further comprise instruction(s) on methods of using the composition, such as uses described herein. The kits described herein may also include other materials desirable from a commercial and user standpoint, including other buffers, diluents, filters, needles, syringes, and package inserts with instructions for performing any methods described herein.

## VII. THERAPEUTIC APPLICATIONS

Provided herein are methods for using and uses of the provided molecules containing a variant ICOSL IgSF domain fusion protein described herein and pharmaceutical compositions containing the same. Such methods and uses include methods for modulating an immune response, including in connection with treating a disease or condition in a subject, such as in a human patient. Included among such molecules in the methods for using and uses herein are immunomodulatory fusion protein formats in which an extracellular domain or portion thereof of a ICOSL variant polypeptide containing an affinity modified IgSF domain (e.g. IgV) is linked, directly or indirectly, to a multimerization domain, e.g. an Fc domain or region. In some embodiments, such a therapeutic agent is a variant ICOSL-Fc fusion protein, such as a variant ICOSL IgV-Fv fusion protein.In some embodiments the pharmaceutical composition is used to treat inflammatory or autoimmune disorders, or in connection with an organ transplantation in a mammal.

In some aspects, the pharmaceutical composition can modulate (e.g. decrease) an immune response to treat the disease. A variety of means are known for determining if administration of a therapeutic composition of the invention sufficiently modulates immunological activity by decreasing or reducing activity of immune cells; changing the physical or functional properties of immune cells; or a combination of these effects. Examples of measurements of the modulation of immunological activity include, but are not limited to, examination of the presence or absence of immune cell populations (using flow cytometry, immunohistochemistry, histology, electron microscopy, polymerase chain reaction (PCR)); measurement of the functional capacity of immune cells including ability or resistance to proliferate or divide in response to a signal (such as using T-cell proliferation assays and pepscan analysis based on 3H-thymidine incorporation following stimulation with anti-CD3 antibody, anti-T-cell receptor antibody, anti-CD28 antibody, calcium ionophores, PMA (phorbol 12-myristate 13-acetate) antigen presenting cells loaded with a peptide or protein antigen; B cell proliferation assays); measurement of the ability to kill or lyse other cells (such as cytotoxic T cell assays); measurements of the cytokines, chemokines, cell surface molecules, antibodies and other products of the cells (e.g., by flow cytometry, enzyme-linked immunosorbent assays, Western blot analysis, protein microarray analysis, immunoprecipitation analysis); measurement of biochemical markers of activation of immune cells or signaling pathways within immune cells (e.g., Western blot and immunoprecipitation analysis of tyrosine, serine or threonine phosphorylation, polypeptide cleavage, and formation or dissociation of protein complexes; protein array analysis; DNA transcriptional, profiling using DNA arrays or subtractive hybridization); measurements of cell death by apoptosis, necrosis, or other mechanisms (e.g., annexin V staining, TUNEL assays, gel electrophoresis to measure DNA laddering, histology; fluorogenic caspase assays, Western blot analysis of caspase substrates); measurement of the genes, proteins, and other molecules produced by immune cells (e.g., Northern blot analysis, polymerase chain reaction, DNA microarrays, protein microarrays, 2- dimensional gel electrophoresis, Western blot analysis, enzyme linked immunosorbent assays, flow cytometry); and measurement of clinical symptoms or outcomes for example, by measuring relapse rate or disease severity (using clinical scores known to the ordinarily skilled artisan).

Among the provided methods and uses include therapeutic methods and uses, for example, involving administration of the molecules, or compositions containing the same, to a subject having a disease, condition, or disorder in need of treatment thereof. In some embodiments, the provided methods also include prophylactic treatment involving administration of the molecules, or compositions containing the same, to a subject prior to the onset or likely onset of a disease, condition or disorder. The pharmaceutical compositions described herein (including pharmaceutical composition comprising the variant ICOSL IgSF domain fusion proteins) can be used in a variety of therapeutic or prophylactic treatment applications, such as described, including for the treatment of an autoimmune or inflammatory disease or disorder. In some embodiments, the molecule is administered in an effective amount to effect treatment of the disease or disorder. Uses include uses of molecules containing a variant ICOSL polypeptide, such as immunomodulatory fusion protein, in such methods and treatments, and in the preparation of a medicament in order to carry out such therapeutic methods. In some embodiments, the methods are carried out by administering a variant ICOSL polypeptide, such as immunomodulatory fusion protein, or compositions comprising the same, to the subject having or suspected of having the disease or condition. In some embodiments, the methods thereby treat the disease or condition or disorder in the subject.

In some embodiments, the provided methods are applicable to therapeutic administration of variant ICOSL polypeptides, such as the immunomodulatory fusion proteins, described herein. In some embodiments, the pharmaceutical composition suppresses an immune response, which can be useful in the treatment of inflammatory or autoimmune disorders, or organ transplantation. In some embodiments, the pharmaceutical composition contains a variant ICOSL polypeptide in a format that exhibits antagonist activity of its cognate binding partner CD28 or ICOS and/or that blocks or inhibits costimulatory signaling via CD28 or ICOS. Exemplary formats of an ICOSL polypeptide for use in connection with such therapeutic applications include, for example, a variant ICOSL polypeptide that is soluble (e.g. variant ICOSL-Fc fusion protein).

The provided methods and uses include treatment of diseases or conditions in which the CD28 and/or ICOS pathway is involved. In some embodiments, the provided methods and uses include treatment of disease or conditions in which the CD28 and ICOS pathways are involved. CD28 and ICOS provide costimulatory signals required for optimal T cell activation when bound to their respective ligands, CD80 (B7-1) and CD86 (B7-2), and ICOSL. CD28 is involved in initiation of the pathogenic process in certain inflammatory diseases, such as Graft Versus Host Disease (GVHD). In some aspects, targeting CD28 by the use of CD28 pathway inhibitors may provide therapeutic utility against such diseases and conditions, including for prophylaxis and/or treatment. However, in some cases, CD28 pathway inhibition alone may not be sufficient to control established disease in many patients. In this context, it is recognized that following initial activation, CD28 is often down-regulated while ICOS, its most closely related family member, is upregulated, providing additional T cell costimulation that may sustain diseases, such as GVHD including gastrointestinal manifestations (Adorn et al. Blood 2018; 132-355). Provided embodiments are based on superior utility of variant ICOSL molecules, e.g. variant ICOSL vIgD-Fc fusion proteins, to exhibit combined blockade of CD28 and ICOS compared to agents that exhibit isolated blockage of the CD28 or ICOS pathways alone. As described herein, variant ICOSL molecules, e.g. variant ICOSL vIgD-Fc fusion proteins, are provided that exhibit high affinity binding to CD28 and ICOS, potent T cell activation, and suppression of disease activity in a variety of inflammatory animal models, such as a human xenogeneic model of GVHD in mice, among others. In such examples, such effects can be seen after only a single dose of the variant ICOSL molecule.

### A. Diseases or Conditions

In some embodiments, the provided methods are for treating an inflammatory or autoimmune disorder, such as any described herein. In some embodiments, treatment is administered at or about the time of onset of symptoms of the disorder. In some embodiments, treatment is administered at a time that is delayed relative to the onset of the disease and/or the onset of symptoms. It should be appreciated that delay of treatment administration may also be relative to transplanation, for example a transplantion as decribed herein. In some embodiments, the amount of delay is, is about, or is at least 1 week, 2 weeks, 3 weeks, 4, weeks, 5 weeks, 6 weeks, 7 weeks, 8 weeks or more from disease and/or symptom onset. In some embodiments, the amount of delay is 2 weeks from disease and/or symptom onset. In some embodiments, the amount of delay is, is about, or is at least 3 months, 6 months, 1 year, 2 years, 3 years, 4 years, 5 years, 6 years, 7 years, 8 years, 9 years, 10 years, or more from disease and/or symptom onset. In some embodiments, treatment administered following any time delay, for example as described herein, is administered according to any treatment regime, dosing amount, or dosing scheduled described herein.

#### *1. Inflammatory or Autoimmune Disorders*

In some embodiments, the provided methods include prophylactic treatment of a subject who is likely or suspected to develop an inflammatory or autoimmune disorder, such as any described herein. For example, development of certain autoimmune or inflammatory disoders, such as acute GVHD, are associated with or can be developed following hematopoietic cell transplant. In some cases, a prophylactic treatment as described herein minizimizes or reduces the risk of the subject developing the inflammatory or autoimmune disorder.

In some embodiments, the inflammatory or autoimmune disorder is antineutrophil cytoplasmic antibodies (ANCA)-associated vasculitis, a vasculitis, an autoimmune skin disease, transplantation, a Rheumatic disease, an inflammatory gastrointestinal disease, an inflammatory eye disease, an inflammatory neurological disease, an inflammatory pulmonary disease, an inflammatory endocrine disease, or an autoimmune hematological disease.

In some embodiments, the inflammatory and autoimmune disorders that can be treated by the pharmaceutical composition described herein is Addison's Disease, allergies, alopecia areata, Alzheimer's, antineutrophil cytoplasmic antibodies (ANCA)-associated vasculitis, ankylosing spondylitis, antiphospholipid syndrome (Hughes Syndrome), asthma, atherosclerosis, rheumatoid arthritis, autoimmune hemolytic anemia, autoimmune hepatitis, autoimmune inner ear disease, autoimmune lymphoproliferative syndrome, autoimmune myocarditis, autoimmune oophoritis, autoimmune orchitis, azoospermia, Behcet's Disease, Berger's Disease, bullous pemphigoid, cardiomyopathy, cardiovascular disease, celiac Sprue/coeliac disease, chronic fatigue immune dysfunction syndrome (CFIDS), chronic idiopathic polyneuritis, chronic inflammatory demyelinating, polyradicalneuropathy (CIDP), chronic relapsing polyneuropathy (Guillain-Barré syndrome), Churg-Strauss Syndrome (CSS), cicatricial pemphigoid, cold agglutinin disease (CAD), COPD (chronic obstructive pulmonary disease), CREST syndrome, Crohn's disease, dermatitis, herpetiformus, dermatomyositis, diabetes, discoid lupus, eczema, epidermolysis bullosa acquisita, essential mixed cryoglobulinemia, Evan's Syndrome, exopthalmos, fibromyalgia, Goodpasture's Syndrome, Graves' Disease, Hashimoto's thyroiditis, idiopathic pulmonary fibrosis, idiopathic thrombocytopenia purpura (ITP), IgA nephropathy, immunoproliferative disease or disorder, inflammatory bowel disease (IBD), interstitial lung disease, juvenile arthritis, juvenile idiopathic arthritis (JIA), Kawasaki's Disease, Lambert-Eaton Myasthenic Syndrome, lichen planus, lupus nephritis, lymphocytic hypophysitis, Ménière's Disease, Miller Fish Syndrome/acute disseminated encephalomyeloradiculopathy, mixed connective tissue disease, multiple sclerosis (MS), muscular rheumatism, myalgic encephalomyelitis (ME), myasthenia gravis, ocular inflammation, pemphigus foliaceus, pemphigus vulgaris, pernicious anaemia, polyarteritis nodosa, polychondritis, polyglandular syndromes (Whitaker's syndrome), polymyalgia rheumatica, polymyositis, primary agammaglobulinemia, primary biliary cirrhosis/autoimmune cholangiopathy, psoriasis, psoriatic arthritis, Raynaud's Phenomenon, Reiter's Syndrome/reactive arthritis, restenosis, rheumatic fever, rheumatic disease, sarcoidosis, Schmidt's syndrome, scleroderma, Sjörgen's Syndrome, stiff-man syndrome, systemic lupus erythematosus (SLE), systemic scleroderma, Takayasu arteritis, temporal arteritis/giant cell arteritis, thyroiditis, Type 1 diabetes, ulcerative colitis, uveitis, vasculitis, vitiligo, interstitial bowel disease or Wegener's Granulomatosis. In some embodiments, the inflammatory or autoimmune disorder is selected from interstitial bowel disease, transplant, Crohn's disease, ulcerative colitis, multiple sclerosis, asthma, rheumatoid arthritis, and psoriasis.

In some embodiments, the inflammatory or autoimmune disease or condition is a connective tissue disease. Connective tissue diseases include diseases that have in common widespread immunologic and inflammatory alterations of connective tissue. Common findings include arthritis or synovitis, pleuritis, myocarditis, endocarditis, pericarditis, peritonitis, vasculitis, myositis, dermatitis, nephritis, and alteration of connective tissues. Connective tissue disease involves virtually any organ system. In particular, provided methods include methods for treating connective tissue diseases involving inflammation of skin, joints and soft tissues due to altered patterns of immunoregulation, such as rheumatoid arthritis, systemic lupus erythematosus, progressive systemic sclerosis, Sjogren's syndrome, dermatomyositis and mixed connective tissue disease.

In some embodiments, the inflammatory or autoimmune disorder is a disorder secondary to an infection. In some embodiments, the infection is a viral infection. In some embodiments, the infection is a bacterial infection. In some embodiments, the inflammatory or autoimmune disorder is a disorder that occurs in response to treatment with a drug or therapy. In some embodiments, the treatment is an immunotherapy. In some embodiments, the immunotherapy is adoptive cell transfer of autologous T cells engineered to express chimeric antigen receptors, e.g., CAR-T cell therapy. In some embodiments, the immunotherapy is treatment with checkpoint inhibitors. Examples of inflammatory or autoimmune disorders secondary to infections, e.g., viral infections, or drug treatment or therapy, include, but are not limited to cytokine storm, cytokine release syndrome, systemic inflamamotry response syndrome, macrophage activation syndrome (MAS), and hemophagocytic lymphohistiocytosis (e.g., secondary hemophagocytic lymphohistiocytosis).

In some embodiments, the inflammatory or autoimmune disorder is cytokine storm. In some embodiments, the inflammatory or autoimmune disorder is cytokine release syndrome (CRS). In some embodiments, the inflammatory or autoimmune disorder is systemic inflammatory response syndrome. In some embodiments, the inflammatory or autoimmune disorder is macrophage activation syndrome (MAS). In some embodiments, the inflammatory or autoimmune disorder is hemophagocytic lymphohistiocytosis. In some embodiments, the inflammatory or autoimmune disorder is secondary hemophagocytic lymphohistiocytosis. In some embodiments, cytokine release syndrome, systemic inflammatory response syndrome, macrophage activation syndrome (MAS), and hemophagocytic lymphohistiocytosis (e.g.,

secondary hemophagocytic lymphohistiocytosis) are subsets of cytokine storm.

Exemplary outcomes associated with cytokine storm, e.g., cytokine release syndrome (CRS), include fever, rigors, chills, hypotension, dyspnea, acute respiratory distress syndrome (ARDS), encephalopathy, ALT/AST elevation, renal failure, cardiac disorders, hypoxia, neurologic disturbances, and death. Neurological complications include delirium, seizure-like activity, confusion, word-finding difficulty, aphasia, and/or becoming obtunded. Other outcomes include fatigue, nausea, headache, seizure, tachycardia, myalgias, rash, acute vascular leak syndrome, liver function impairment, and renal failure. In some aspects, cytokine storm, e.g., CRS, is associated with an increase in one or more factors such as serum-ferritin, d-dimer, aminotransferases, lactate dehydrogenase and triglycerides, or with hypofibrinogenemia or hepatosplenomegaly. Other exemplary signs or symptoms associated with cytokine storm, e.g., CRS, include hemodynamic instability, febrile neutropenia, increase in serum C-reactive protein (CRP), changes in coagulation parameters (for example, international normalized ratio (INR), prothrombin time (PTI) and/or fibrinogen), changes in cardiac and other organ function, and/or absolute neutrophil count (ANC).

In some embodiments, outcomes associated with cytokine storm, e.g., CRS, include one or more of: persistent fever, e.g., fever of a specified temperature, e.g., greater than at or about 38 degrees Celsius, for two or more, e.g., three or more, e.g., four or more days or for at least three consecutive days; fever greater than at or about 38 degrees Celsius; elevation of cytokines, such as a max fold change, e.g., of at least at or about 75, compared to pre-treatment levels of at least two cytokines (e.g., at least two of the group consisting of interferon gamma (IFNγ), GM-CSF, IL-6, IL-10, Flt-3L, fracktalkine, and IL-5, and/or tumor necrosis factor alpha (TNFα)), or a max fold change, e.g., of at least at or about 250 of at least one of such cytokines; and/or at least one clinical sign of toxicity, such as hypotension (e.g., as measured by at least one intravenous vasoactive pressor); hypoxia (e.g., plasma oxygen ($PO_2$) levels of less than at or about 90%); and/or one or more neurologic disorders (including mental status changes, obtundation, and seizures).

Exemplary cytokine storm-related, e.g., CRS-related, outcomes include increased or high serum levels of one or more factors, including cytokines and chemokines and other factors associated with cytokine storm. Exemplary outcomes further include increases in synthesis or secretion of one or more of such factors. Such synthesis or secretion can be by the T cell or a cell that interacts with the T cell, such as an innate immune cell or B cell.

In some embodiments, the cytokine storm-associated, e.g., CRS, serum factors or related outcomes include inflammatory cytokines and/or chemokines, including interferon gamma (IFN-γ), TNF-a, IL-1β, IL-2, IL-6, IL-7, IL-8, IL-10, IL-12, sIL-2Ra, granulocyte macrophage colony stimulating factor (GM-CSF), macrophage inflammatory protein (MIP)-1, tumor necrosis factor alpha (TNFα), IL-6, and IL-10, IL-1β, IL-8, IL-2, MIP-1, Flt-3L, fracktalkine, and/or IL-5. In some embodiments, the factor or outcome includes C reactive protein (CRP). In addition to being an early and easily measurable risk factor, CRP also is a marker for cell expansion. In some embodiments, subjects that are measured to have high levels of CRP, such as ≥ 15 mg/dL, have cytokine storm, e.g., CRS. In some embodiments, subjects that are measured to have high levels of CRP do not have cytokine storm, e.g., CRS. In some embodiments, a measure of cytokine storm includes a measure of CRP and another factor indicative of cytokine storm, e.g., CRS.

In some embodiments, one or more inflammatory cytokines or chemokines are monitored to assess cytokine storm. In some aspects, the one or more cytokines or chemokines include IFN-γ, TNF-α, IL-2, IL-1β, IL-6, IL-7, IL-8, IL-10, IL-12, sIL-2Rα, granulocyte macrophage colony stimulating factor (GM-CSF), or macrophage inflammatory protein (MIP). In some embodiments, IFN-γ, TNF-α, and IL-6 are monitored.

In some embodiments, the inflammatory or autoimmune disorder is CRS. In some embodiments, the inflammation is associated with CRS. CRS criteria that appear to correlate with the onset of CRS to predict which patients are more likely to be at risk for developing CRS have been developed. Factors include fevers, hypoxia, hypotension, neurologic changes, elevated serum levels of inflammatory cytokines, such as a set of seven cytokines (IFNγ, IL-5, IL-6, IL-10, Flt-3L, fractalkine, and GM-CSF). In some embodiments, the criteria reflective of CRS grade are those detailed in **Table A** below.

| **Table A:** | **Exemplary Grading Criteria for CRS** |
|---|---|
| **Grade** | **Description of Symptoms** |
| 1<br>Mild | Not life-threatening, require only symptomatic treatment such as antipyretics and anti-emetics (e.g., fever, nausea, fatigue, headache, myalgias, malaise) |
| 2<br>Moderate | Require and respond to moderate intervention:<br>• Oxygen requirement < 40%, or<br>• Hypotension responsive to fluids or low dose of a single vasopressor, or<br>• Grade 2 organ toxicity (by CTCAE v4.0) |
| 3<br>Severe | Require and respond to aggressive intervention:<br>• Oxygen requirement ≥ 40%, or |

(continued)

| Table A: | Exemplary Grading Criteria for CRS |
|---|---|
| Grade | Description of Symptoms |
| | • Hypotension requiring high dose of a single vasopressor (e.g., norepinephrine ≥ 20 μg/kg/min, dopamine ≥ 10 μg/kg/min, phenylephrine ≥ 200 μg/kg/min, or epinephrine ≥ 10 μg/kg/min), or<br>• Hypotension requiring multiple vasopressors (e.g., vasopressin + one of the above agents, or combination vasopressors equivalent to ≥ 20 μg/kg/min norepinephrine), or<br>• Grade 3 organ toxicity or Grade 4 transaminitis (by CTCAE v4.0) |
| 4<br>Life-threatening | Life-threatening:<br>• Requirement for ventilator support, or<br>• Grade 4 organ toxicity (excluding transaminitis) |
| 5<br>Fatal | Death |

In some embodiments, the CRS is severe CRS or a grade 3 or higher CRS. In some embodiments, outcomes associated with severe CRS or grade 3 CRS or greater, such as grade 4 or greater, include one or more of: persistent fever, e.g., fever of a specified temperature, e.g., greater than at or about 38 degrees Celsius, for two or more, e.g., three or more, e.g., four or more days or for at least three consecutive days; fever greater than at or about 38 degrees Celsius; elevation of cytokines, such as a max fold change, e.g., of at least at or about 75, compared to baseline levels of at least two cytokines (e.g., at least two of the group consisting of interferon gamma (IFNγ), GM-CSF, IL-6, IL-10, Flt-3L, fracktalkine, and IL-5, and/or tumor necrosis factor alpha (TNFα)), or a max fold change, e.g., of at least at or about 250 of at least one of such cytokines; and/or at least one clinical sign of toxicity, such as hypotension (e.g., as measured by at least one intravenous vasoactive pressor); hypoxia (e.g., plasma oxygen ($PO_2$) levels of less than at or about 90%); and/or one or more neurologic disorders (including mental status changes, obtundation, and seizures). In some embodiments, severe CRS includes CRS that requires management or care in the intensive care unit (ICU).

In some embodiments, severe CRS or grade 3 CRS encompasses an increase in alanine aminotransferase, an increase in aspartate aminotransferase, chills, febrile neutropenia, headache, left ventricular dysfunction, encephalopathy, hydrocephalus, and/or tremor.

In some embodiments, the cytokine storm, e.g., CRS, occurs in response to or is secondary to an infection. In some embodiments, the infection is a viral infection or a bacterial infection. In some embodiments, the the viral infection is a coronavirus infection. In some embodiments, the the viral infection is a SARS-CoV-2 infection. In some embodiments, the viral infection is a SARS-CoV infection. In some embodiments, the viral infection is a MERS-CoV infection.

In some embodiments, the cytokine storm occurs or is secondary to an infection, a condition, or a disease. For example, the cytokine storm, e.g., CRS, may occur during the course of the infection, condition, or disease. In some embodiments, the cytokine storm, e.g., CRS, occurs in coronavirus 2019. In some embodiments, the cytokine storm occurs in pneumonia. In some embodiments, the cytokine storm occurs in shock. In some embodiments, the cytokine storm occurs in sepsis. In some embodiments, the cytokine storm occurs in multiple organ system dysfunction. In some embodiments, the cytokine storm occurs in acute respiratory distress syndrome (ARDS).

In some embodiments, cytokine storm occurs in response to treatment with a drug or therapy. In some embodiments, the cytokine storm occurs in response to treatment with an immunotherapy. For example, in some cases, the cytokine storm occurs following adoptive cell transfer of autologous T cells engineered to express chimeric antigen receptors, e.g., CAR-T cell therapy. In some embodiments, the cytokine storm occurs following treatment with checkpoint inhibitors.

In some embodiments, a pharmaceutical composition provided herein, such as a variant ICOSL IgSF (e.g. IgV) Fc fusion protein provided herein, is used to treat cytokine storm. In some embodiments, the cytokine storm to be treated is secondary to SARS-CoV-2 infection. In some embodiments, infection with SARS-CoV-2 is confirmed by a positive RT-PCR test. In some embodiments, a subject positive for SARS-CoV-2 to be treated further exhibits severe pneumonia, acute respiratory distress syndrome (ARDS), sepsis, septic shock, or multiple organ system dysfunction attributed to SARS-CoV-2 infection.

In some embodiments, the inflammatory or autoimmune disorder is a chronic autoimmune disease. In some embodiments, the inflammatory or autoimmune disorder is Sjogren's Syndrome (pSS) or Systemic Lupus Erythematosus (SLE). In some embodiments, the inflammatory or autoimmune disorder is an inflammatory bowel disease (IBD). In some embodiments, the IBD is chronic IBD. In some examples, the inflammatory or autoimmune disorder is Crohn's Disease. In some examples, the inflammatory or autoimmune disorder is ulcerative colitis. In some embodiments, the inflammatory or autoimmune disorder is an IBD-related disease or disorder, e.g. interstitial lung disease (ILD). In some embodiments, the inflammatory or autoimmune disorder is psoriatic arthritis or rheumatoid arthritis.

In some embodiments, the pharmaceutical composition is administered to modulate an autoimmune condition. For example, suppressing an immune response can be beneficial in methods for inhibiting rejection of a tissue, cell, or organ transplant from a donor by a recipient. Accordingly, in some embodiments, the pharmaceutical compositions described herein are used to limit or prevent graft-related or transplant related diseases or disorders, such as graft versus host disease (GvHD). In some embodiments, the pharmaceutical compositions are used to suppress autoimmune rejection of transplanted or grafted bone marrow, organs, skin, muscle, neurons, islets, or parenchymal cells.

In some embodiments, the inflammatory or autoimmune disorder is associated with sialadenitis. In some cases, sialadenitis is an infection of the salivary glands caused by a virus or bacteria. In some embodiments, the parotid or submandibular glands are affected by sialadenitis. In some embodiments, sialadenitis is associated with pain, tenderness, redness and gradual localized sqelling of the affected area.

In some embodiments, the inflammatory or immune disorder is associated with insulitis. In some embodiments, insulitis involves inflammation of the islets of Langerhans in the pancreas. In some embodiments, the islets containing pancreatic β-cells are infiltrated by B and T lymphocytes, macrophages or dendritic cells.

In some embodiments, a pharmaceutical composition provided herein, such as a variant ICOSL IgSF (e.g. IgV) Fc fusion protein provided herein, is used to treat psoriatic arthritis (PsA). In some cases, the PsA affects one or more joints, such as fingers, toes, arms or legs, including elbows, wrists, hands and feet, or sacroliliac joint. In some cases, the PsA is mild and/or affects four or less joints. In some cases the PsA is moderate and/or affects four or more joints. In some cases, a subject with PsA may exhibit pain, stiffness or inflammation in the spine or neck, or in the one more joints.

In some embodiments, a pharmaceutical composition provided herein, such as a variant ICOSL IgSF (e.g. IgV) Fc fusion protein provided herein, is used to treat rheumatoid arthritis (RA). In some cases, RA affects joints, lining of joints, and/or non-joint structures in the body (e.g., skin, eyes, lungs, heart, kidneys, salivary glands, nerve tissue, bone marrow or blood vessels). In some embodiments, RA or RA symptoms are chronic.

In some embodiments, the inflammatory or autoimmune disorder is a chronic disorder. In some embodiments, the inflammatory or autoimmune disorder is an acute disorder.

In some cases, the pharmaceutical composition provided herein, such as a variant ICOSL IgSF (e.g. IgV) Fc fusion protein provided herein, may be used to treat chronic inflammatory or autoimmune disorders. In some embodiments, treatment of chronic inflammatory or autimmune disorders with the pharmaceutical composition provided herein, such as a variant ICOSL IgSF (e.g. IgV) Fc fusion protein provided herein, includes continuous administration to a subject. In some embodiments, continuous administration is a repeated administration. In some cases, a treatment period including a treatement regimen is repeated. As described herein, in some embodiments, the treatment period is repeated. In some embodiments, the repeated treatment period occurs immediately following a prior treatment period. In some embodiments, administration is repeated until any time as desired by a skilled practitioner. For example, treatment of a chronic inflammatory or autoimmune disorder, e.g., with a pharmaceutical composition provided herein, such as a variant ICOSL IgSF (e.g. IgV) Fc fusion protein provided herein, may include administering treatment to the subject indefinitely.

In some cases, treatment with a pharmaceutical composition provided herein, such as a variant ICOSL IgSF (e.g. IgV) Fc fusion protein provided herein, to treat a chronic inflammatory or autoimmune disorder includes intermittent administration. For example, treatment of a chronic inflammatory or autoimmune disorder, e.g., with a pharmaceutical composition provided herein, such as a variant ICOSL IgSF (e.g. IgV) Fc fusion protein provided herein, may include administering a treatment in a cyclic manner, such that a particular treatment regimen, for example a treatment regimen in a treatment period, is administered repeatedly any number of times to a subject. In some embodiments, the treatment period and treatment regimen are repeated with a delay between the treatment periods. In some embodiment, the treatment period is repeated indefinitely. In some embodiments, treatment is administered to a subject any number of times as desired by a skilled practitioner.

In some embodiments, treatment is administered to a subject to maintain a desired effect. For example, in some embodiments, when the inflammatory or autoimmune disorder is a chronic inflammatory or autoimmune disease, treatment with a pharmaceutical composition provided herein, such as a variant ICOSL IgSF (e.g. IgV) Fc fusion protein provided herein, is continued following remission or partial remission of the disease and/or a reduction or amelioration in signs and/or symptoms of a disease, such as a reduction of one or more signs of inflammation in a subject having the chronic inflammatory or autoimmune disorder. In some embodiments, treatment may be continued so as to prevent resurgence of symptoms and/or maintain remission or reduction of symptoms of the chronic inflammatory or autoimmune disorder. In some embodiments, administration continues until any time as desired by a skilled practitioner. In some embodiments, the continued administration is in the form of repeated treatment periods as described herein.

Any of the chronic inflammatory or autoimmune disorders described herein, for example, chronic GvHD, chronic Inflammatory Bowel Disease, Sjogren's Syndrome (pSS) or Systemic Lupus Erythematosus (SLE), COPD, Crohn's Disease, ulcerative colitis, interstitial lung disease (ILD), psoriatic arthritis, rheumatoid arthritis, or any chronic forms of inflammatory or autoimmune diseases or disorders described herein may be treated by administering treatment according to the chronic treatment described above.

In some cases, the pharmaceutical composition provided herein, such as a variant ICOSL IgSF (e.g. IgV) Fc fusion protein provided herein, may be used to treat acute inflammatory or autoimmune disorders. For example, treatment of an acute inflammatory or autoimmune disorder, e.g., with a pharmaceutical composition provided herein, such as a variant ICOSL IgSF (e.g. IgV) Fc fusion protein provided herein, may include administering treatment to the subject for a defined or limited time period. In some embodiments, the administration is for a single treatment period which is not repeated. In some embodiments, the administration is a single dose. In some embodiments, the duration or treatment period is set as desired by a skilled practitioner.

In some embodiments, when the inflammatory or autoimmune disorder is an acute inflammatory or autoimmune disorder, treatment with a pharmaceutical composition provided herein, such as a variant ICOSL IgSF (e.g. IgV) Fc fusion protein provided herein, is discontinued following remission or partial remission of the disease and/or a reduction or amelioration in signs and/or symptoms of a disease, such as a reduction of one or more signs of inflammation in a subject having an acute inflammatory or autoimmune disorder. In some embodiments, administration is discontinued at any time as desired by a skilled practitioner.

Any of the acute inflammatory or autoimmune disorders described herein, for example, acute GvHD, or any acute forms of inflammatory or autoimmune disorders described herein may be treated by administering treatment according to the above. In some embodiments, the cytokine storm is an acute inflammatory or autoimmune disease. In some embodiments, the CRS is an acute inflammatory or autoimmune disease.

In some embodiments, a pharmaceutical composition provided herein, such as a variant ICOSL IgSF (e.g. IgV) Fc fusion protein provided herein, is used to treat GVHD.

In some embodiments, the GVHD is chronic GVHD. In some embodiments, a pharmaceutical composition provided herein, such as a variant ICOSL IgSF (e.g. IgV) Fc fusion protein provided herein, is used to treat GVHD. In some embodiments, the GVHD is chronic GVHD. In some cases, chronic GVHD occurs after allogeneic hematopoietic stem cell transplant (HSCT) and/or a reaction of donor immune cells against host tissues. In some case, the chronic GVHD manifests in the skin, liver or gastrointestinal tract. In some embodiments, the chronic GVHD is resistant or refractory. In some embodiments, the chronic GVHD is resistant or refractory to an immunosuppressant. In some cases the immunosuppressant is a corticosteroid and/or cyclosporine. In some cases, the corticosteroid is a glucocorticoid.

In some embodiments, the GVHD is acute GVHD (aGVHD). In some cases, aGVHD occurs after allogeneic hematopoietic stem cell transplant (HSCT) and/or a reaction of donor immune cells against host tissues. In some embodiments, the aGVHD is resistant or refractory. In some embodiments, the aGVHD is resistant or refractory to an immunosuppressant. In some case, the aGVHD manifests in the skin, liver or gastrointestinal tract.

In some embodiments, a pharmaceutical composition provided herein, such as a variant ICOSL IgSF (e.g. IgV) Fc fusion protein provided herein, is used to treat GVHD. In some embodiments, the GVHD is acute GVHD (aGVHD). In some cases, aGVHD occurs after allogeneic hematopoietic stem cell transplant (HSCT) and/or a reaction of donor immune cells against host tissues. In some case, the aGVHD manifests in the skin, liver or gastrointestinal tract. In some embodiments, the aGVHD is resistant or refractory. In some embodiments, the aGVHD is resistant or refractory to an immunosuppressant. In some cases the immunosuppressant is a corticosteroid and/or cyclosporine. In some cases, the corticosteroid is a glucocorticoid.

In some embodiments, resistant or refractory aGvHD includes progression of aGVHD within 5 days following initation of treatment with ≥ 2 mg/kg/day of prednisone or an equivalent thereof. In some embodiments, the progression is or includes a worsening of GVHD in at least one organ with or without amelioration in any organ. In some embodiments, resistant or refractory aGVHD includes failure to improve within 7 days following initiation of treatment with ≥ 2 mg/kg/day of prednisone or an equivalent thereof. In some embodiments, the failure to improve is or includes a worsening of GVHD in at least one organ with or without amelioration in any organ or maintining the same grade of GVHD or progression of GVHD in any organ or death, or the addition of secondary GVHD therapy beyond a planned salvage therapy. In some embodiments, resistant or refractory aGVHD includes an incomplete response after 28 days of immunosuppressive treatment including steroids, e.g., treatment with ≥ 2 mg/kg/day of prednisone or an equivalent thereof. In some embodiments, the incomplete response is or includes a failure to reach a complete resolution of aGVHD symptoms in all organs, without secondary GVHD therapy beyond a planned salvage therapy. In some embodiments, resistant or refractory aGVHD includes any of the conditions described herein.

In some cases, the aGVHD is graded or staged according to Mount Sinai Acute GVHD International Consortium (MAGIC) Guideline. **Table B** provides exemplary GVHD target organ staging. In some embodiments, a pharmaceutical composition provided herein, such as a variant ICOSL IgSF (e.g. IgV) Fc fusion protein provided herein, is used to treat grade II-IV aGVHD.

**Table B:** GVHD target organ staging.

| Stage | Skin (active erythema only) | Liver (bilirubin) | Upper GI | Lower GI (stool output/day) |
|---|---|---|---|---|
| **0** | No active (erythematous) GVHD rash | < 2 mg/dL | No or intermittent nausea. vomiting: or anorexia | Adult: < 500 ml/day or <3 episodes/day<br>Child: < 10 ml/kg/day or <4 episodes/day |
| **1** | Maculopapular rash <25% BSA | 2-3 mg/dL | Persistent nausea, vomiting or anorexia | Adult: 500-999 ml/day or 3-4 episodes/day<br>Child: 10-19.9 ml/kg/day or 4-6 episodes/day |
| **2** | Maculopapular rash 25 - 50% BSA | 3.1-6 mg/dL | - | Adult: 1000-1500 ml/day or 5-7 episodes/day<br>Child: 20-30 ml/kg/day or 7-10 episodes/day |
| **3** | Maculopapular rash > 50% BSA | 6.1-15 mg/dL | - | Adult: >1500 ml/day or >7 episodes/day<br>Child: >30 ml/kg/day or >10 episodes/day |
| **4** | Generalized erythroderma (>50% BSA) plus bullous formation and desquamation >5% BSA | >15 mg/dL | - | Severe abdominal pain with or without ileus, or grossly bloody stool (regardless of stool volume.). |

**Overall clinical grade (based upon most severe target organ involvement):**

- Grade 0: No stage 1-4 of any organ
- Grade I: Stage 1-2 skin without liver, upper GI or lower GI involvement
- Grade II: Stage 3 rash and/or stage 1 liver and/or stage 1 upper GI and/or stage 1 lower GI
- Grade III: Stage 2-3 liver and/or stage 2-3 lower GI, with stage 0-3 skin and/or stage 0-1 upper GI
- Grade IV: Stage 4 skin, liver or lower GI involvement, with stage 0-1 upper GI

In some embodiments, a pharmaceutical composition provided herein, such as a variant ICOSL IgSF (e.g. IgV) Fc fusion protein provided herein, is used to treat a subject with aGVHD having a Karnofsky grade of 40 or greater at commencement of treatment. **Table C** shows the Karnofsky grading scheme.

**Table C:** Karnofsky status.

| Karnofsky Status | Karnofsky Grade | Reference ECOG Grade |
|---|---|---|
| Normal, no complaints | 100 | 0 |
| Able to carry on normal activities. Minor signs or symptoms of disease | 90 | 1 |
| Normal activity with effort | 80 | 1 |
| Care for self. Unable to carry on normal activity or to do active work | 70 | 2 |
| Requires occasional assistance, but able to care for most of his needs | 60 | 2 |

(continued)

| Karnofsky Status | Karnofsky Grade | Reference ECOG Grade |
|---|---|---|
| Requires considerable assistance and frequent medical care | 50 | 3 |
| Disabled. Requires special care and assistance | 40 | 3 |
| Severely disabled. Hospitalisation indicated though death nonimminent | 30 | 4 |
| Very sick. Hospitalisation necessary. Active supportive treatment necessary | 20 | 4 |
| Moribund | 10 | 4 |
| Dead | 0 | 5 |

In some embodiments, a pharmaceutical composition provided herein, such as a variant ICOSL IgSF (e.g. IgV) Fc fusion protein provided herein, is used to treat a subject with grade II-IV resistant or refractory aGvHD, as described herein, having a Karnofsky grade of 40 or greater at the onset of treatment, e.g., first dose. In some embodiments, the subject must be at least 18 years of age or older. In some embodiments, the subject may be further treated with a salvage therapy. In some embodiments, the salvage therapy is initiated at least 2 days prior to administering a pharmaceutical composition provided herein, such as a variant ICOSL IgSF (e.g. IgV) Fc fusion protein provided herein. In some embodiments, the subject receiving salvage therapy must be administered at a stable dose for at least two days prior to administration of a pharmaceutical composition provided herein, such as a variant ICOSL IgSF (e.g. IgV) Fc fusion protein provided herein. In some embodiments, salvage therapy may not be initiated following administration of a pharmaceutical composition provided herein, such as a variant ICOSL IgSF (e.g. IgV) Fc fusion protein provided herein.

In some embodiments, a pharmaceutical composition provided herein, such as a variant ICOSL IgSF (e.g. IgV) Fc fusion protein provided herein, is used to prevent or lower the risk of an inflammatory or autoimmune condition associated with a stem cell transplant (*e.g.,* a hematopoietic stem cell transplant, such as from an allogenic donor), such as by prophylactic treatment. In some embodiments, prophylactic treatment comprises administering at least one dose of a variant ICOSL IgSF (e.g. IgV) Fc fusion protein provided herein prior to a subject receiving a stem cell transplant. In some embodiments, prophylactic treatment comprises administering at least one dose of a variant ICOSL IgSF (e.g. IgV) Fc fusion protein provided herein prior to a subject receiving a stem cell transplant and administering at least one dose concurrent with the subject receiving the stem cell transplant. In some embodiments, the inflammatory or autoimmune condition associated with a stem cell transplant is acute GVHD. In some embodiments, a pharmaceutical composition provided herein, such as a variant ICOSL IgSF (e.g. IgV) Fc fusion protein provided herein, is used to prophylactically treat moderate aGVHD. In some embodiments, a pharmaceutical composition provided herein, such as a variant ICOSL IgSF (e.g. IgV) Fc fusion protein provided herein, is used to prophylactically treat, or prevent, severe aGVHD. In some embodiments, prophylactic treatment with a pharmaceutical composition provided herein, such as a variant ICOSL IgSF (e.g. IgV) Fc fusion protein provided herein, prevents or limits moderate or severe aGVHD. In some embodiments, the pharmaceutical compositions described herein are used to limit or prevent graft-related or transplant related diseases or disorders, such as aGVHD that is resistant or refractory to an immunosuppressant, such as a corticosteroid (*e.g.*, a glucocorticoid) or cyclosporine.

In some embodiments, treating the inflammatory or autoimmune condition associated with a stem cell transplant (*e.g.,* a hematopoietic stem cell transplant, such as from an allogenic donor) includes prophylactic treatment of (*e.g.* preventing) the condition. In some embodiments, prophylactic treatment includes peri-transplant treatment. In some cases, peri-transplant treatment prevents or lowers the risk of GVHD associated with stem cell transplant. In some embodiments, peri-transplant treatment includes administering treatment prior to a stem cell transplant and administering treatment concurrent with or subsequent to the stem cell transplant. In some embodiments, the treatment administration is delayed relative to transplantation. In some embodiments, the amount of delay is, is about, or is at least 1 week, 2 weeks, 3 weeks, 4, weeks, 5 weeks, 6 weeks, 7 weeks, 8 weeks or more from the time of transplantation. In some embodiments, the amount of delay is less than 100 days from transplantation. In some embodiments, the GVHD is acute graft versus host disease (aGVHD).

In some embodiments, a pharmaceutical composition provided herein, such as a variant ICOSL IgSF (e.g. IgV) Fc fusion protein provided herein, is used to treat an autoimmune condition associated with an organ transplant. In some cases, treating the autoimmune condition associated with an organ transplant may prolong the survival of the host and transplanted organ. In some embodiments, treating the autoimmune condition associated with an organ transplant includes prophylaxis of or inhibiting or preventing transplant rejections by a subject that is the recipient of the organ transplant.

In some embodiments, treating the inflammatory or autoimmune condition associated with an organ transplant includes peri-transplant treatment. In some cases, peri-transplant treatment prevents or lowers the risk of transplant rejection by a subject that is the recipient of the organ transplant. In some embodiments, peri-transplant treatment includes administering treatment prior to a transplant and administering treatment concurrent with or subsequent to the transplant. In some embodiments, the treatment administration is delayed relative to transplantation. In some embodiments, the amount of delay is, is about, or is at least 1 week, 2 weeks, 3 weeks, 4, weeks, 5 weeks, 6 weeks, 7 weeks, 8 weeks or more from the time of transplantation. In some embodiments, the amount of delay is less than 100 days from transplantation.

In some embodiments, a pharmaceutical composition provided herein, such as a variant ICOSL IgSF (e.g. IgV) Fc fusion protein provided herein, is used to treat an inflammatory bowel disease (IBD). In some embodiments, IBD is chronic IBD. In some embodiments, a pharmaceutical composition provided herein, such as a variant ICOSL IgSF (e.g. IgV) Fc fusion protein provided herein, is used to treat Crohn's disease. In some embodiments, the Crohn's disease can include a subtype from Crohn's colitis, Crohn's enteritis, Crohn's iletis or Crohn's enterocolitis.

In some embodiments, the inflammatory or autoimmune disease to be treated with a pharmaceutical composition provided herein, such as a variant ICOSL IgSF (e.g. IgV) Fc fusion protein provided herein, is an Inflammatory Bowel Disease (IBD). In some cases, IBD encompasses disorders that involves chronic inflammation of all or a part of the digestive tract. In some embodiments, IBD is chronic IBD. In some embodiments, types of IBD (e.g., chronic IBD) include ulcerative colitis and Crohn's disease. In some embodiments, chronic IBD includes Crohn's disease and ulcerative colitis. In some embodiments, symptoms of IBD (e.g., chronic IBD) include diarrhea, fever and fatigue, abdominal pain and cramping, blood in stool, reduced appetite, and unintended weight loss. In some embodiments, the symptoms of IBD are common to both Crohn's Disease and ulcerative colitis.

In some embodiments, a pharmaceutical composition provided herein, such as a variant ICOSL IgSF (e.g. IgV) Fc fusion protein provided herein, is used to treat Crohn's Disease (CD). In some cases, CD results in inflammation of any or all of the gastrointestinal tract, from mouth to anus. In some cases, CD is a chronic inflammatory bowel disease that affects the lining of the digestive tract. In some cases, the CD affects bowel movements, including persistent diarrhea, urgency, senstation of incomplete evacuation, and/or constipation. In some cases, patients with CD experience abdominal cramps and pain. In some cases, patients with CD experience rectal bleeding. In some cases, the symptoms of CD vary between patients. In some cases, patients with CD develop complications such as fissures, fistula, and strictures. In some cases, systemic symptoms of CD include redness or pain in eyes, changes in vision, mouth sores, swollen and/or painful joints, skin complications (e.g., rashes, bumps, sores), fever, loss of appetite, weight loss, anemia, fatigue, night sweats, loss of normal menstrual cycle, osteoporosis, kidney stones, and liver complications (e.g., primary sclerosing cholangitis, cirrhosis).

In some embodiments, a pharmaceutical composition provided herein, such as a variant ICOSL IgSF (e.g. IgV) Fc fusion protein provided herein, is used to treat ulcerative colitis (UC). In some cases, UC results in long-lasting inflammation and/or ulcers in the digestive tract. In some cases UC affected the innermost lining of the large intestine (colon) and rectum. In some cases, symptoms of UC develop over time. In some cases, the UC affects bowel movements, including diarrhea with blood or pus, urgency, and/or inability to defecate despite urgency. In some cases, patients with UC experience rectal bleeding weight loss, fatigue, fever, and in children a failure to grow. In some cases, symptoms are mild to moderate in intesitiy. In some cases, patients with UC may experience long periods of remission. In some embodiments, the UC is ulcerative proctitis. In some embodiments, ulcerative proctitis is confined to the area closest to the anus (rectum). In some cases, patients with ulcerative proctitis experience rectal bleeding. In some embodiments, the UC is proctosigmoiditis. In some cases, proctosigmoiditis affects the rectum and sigmoid colon. In some cases, patients with proctosigmoiditis experience bloody diarrhea, abdominal cramps and pain, and an inability to move the bowels despite urgency (tenesmus). In some cases, the UC is left-sided colitis. In some embodiments, left-sided colitis results in inflammation extending from the rectum to the sigmoid colon and descending colon. In some cases, patients with left-sided colitis experience bloody diarrhea, abdominal pain and cramping on the left side, and unintended weight loss. In some cases, the UC is pancolitis. In some embodiments, pancolitis affects the entire colon. In some cases, patients with pancolitis experience bouts of bloody diarrhea, abdominal cramps and pain, fatigue, and significant weight loss. In some cases the UC is acute severe UC. In some embodiments, acute severe UC is a rare from of UC that affects the entire colon. In some cases, patients with acute severe UC experience severe pain, profuse diarrhea, bleeding, fever, and an inability to eat.

In some embodiments, a pharmaceutical composition provided herein, such as a variant ICOSL IgSF (e.g. IgV) Fc fusion protein provided herein, is used to treat systemic lupus erythematosus (SLE). In some embodiments, a pharmaceutical composition provided herein, such as a variant ICOSL IgSF (e.g. IgV) Fc fusion protein provided herein, is used to treat Sjogren's Syndrome.

## Dosage and Administration

In some embodiments, a pharmaceutical composition described herein (including pharmaceutical composition comprising the variant ICOSL IgSF domain fusion proteins) is administered to a subject. Generally, dosages and routes of administration of the pharmaceutical composition are determined according to the size and condition of the subject, according to standard pharmaceutical practice. In some embodiments, the condition of the subject includes whether the disorder treated is a chronic or an acute disorder. For example, the therapeutically effective dose can be estimated initially either in cell culture assays or in animal models such as mice, rats, rabbits, dogs, pigs, or monkeys. An animal model may also be used to determine the appropriate concentration range and route of administration. Such information can then be used to determine useful doses and routes for administration in humans. The exact dosage can be determined in light of factors related to the subject requiring treatment. Dosage and administration can be adjusted to provide sufficient levels of the active compound or to maintain the desired effect. Factors that may be taken into account include the severity of the disease state, the general health of the subject, the age, weight, and gender of the subject, time and frequency of administration, drug combination(s), reaction sensitivities, and response to therapy.

In some embodiments, modeling and simulation of pharmacokinetic (PK) and pharmacodynamic (PD) profiles observed in control animals and animal models of disease (e.g., cancer models) can be used to predict or determine patient dosing. For example, PK data from non-human primates (e.g., cynomolgus monkeys) can be used to estimate human PK. Similarly, mouse PK and PD data can be used to predict human dosing. The observed animal data can be used to inform computational models which can be used to simulate human dose response.

In some embodiments, methods provided herein including administering a pharmaceutical composition described herein (including pharmaceutical composition comprising the variant ICOSL IgSF domain fusion proteins) in an amount in which a dose is known or predicted to achieve target binding saturation to its cognate ligands, e.g. CD28 and/or ICOS, sufficient for a therapeutic effect. Target binding saturation for binding to cognate ligands can be determined experimentally or empirically. In some embodiments, target binding saturation can be determined from in vitro binding data to mammalian cells that express the cognate ligand, such as a human CD28 or human ICOS.

In some embodiments, the dose of the variant ICOSL IgSF fusion protein administered is in an amount to exhibit, or predicted to exhibit, a target binding saturation for binding to CD28 of greater than at or about 25% at $C_{max}$, such as greater than at or about 30% at $_{max,}$, greater than at or about 35% at$C_{max}$, greater than at or about 40% at $C_{max}$, greater than at or about 50% at $C_{max}$ or greater. In some embodiments, the dose of the variant ICOSL IgSF fusion protein administered is in an amount to exhibit, or predicted to exhibit, a target binding saturation for binding to CD28 of, of at least, or about about 85% at $C_{max}$. In some embodiments, the dose of the variant ICOSL IgSF fusion protein administered is in an amount to exhibit, or predicted to exhibit, a target binding saturation for binding to CD28 of greater than at or about 10% within 24 hours of the administration of the dose, such as greater than at or about 15%, greater than at or about 20%, greater than at or about 25%, greater than at or about 30%, greater than at or about 50%, greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to CD28 of greater than at or about 50% within 2 days of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to CD28 of greater than at or about 50% within 7 days (one week) of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to CD28 of greater than at or about 50% within 14 days (2 weeks) of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to CD28 of greater than at or about 50% within 21 days of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to CD28 of greater than at or about 50% within 28 days of the administration of the dose, such as greater than at or about 60%, greater than at or about 70%, greater than at or about 80%, greater than at or about 85%, greater than at or about 90%, greater than at or about 95%, or at or about 100%.

In some embodiments, the dose of the variant ICOSL IgSF fusion protein administered is in an amount to exhibit, or predicted to exhibit, a target binding saturation for binding to ICOS of greater than at or about 25% at $C_{max}$,

such as greater than at or about 30% at $_{max,}$, greater than at or about 35% at$C_{max}$, greater than at or about 40% at $C_{max}$, greater than at or about 50% at $C_{max}$ or greater. In some embodiments, the dose of the variant ICOSL IgSF fusion protein administered is in an amount to exhibit, or predicted to exhibit, a target binding saturation for binding to ICOS of greater than at or about 10% within 24 hours of the administration of the dose, such as greater than at or about 15%, greater than at or about 20%, greater than at or about 25%, greater than at or about 30%, greater than at or about 50%, greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to ICOS of greater than at or about 50% within 2 days of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to ICOS of greater than at or about 50% within 7 days (one week) of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to ICOS of greater than at or about 50% within 14 days (2 weeks) of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to ICOS of greater than at or about 50% within 21 days of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to ICOS of greater than at or about 50% within 28 days of the administration of the dose, such as greater than at or about 60%, greater than at or about 70%, greater than at or about 80%, greater than at or about 85%, greater than at or about 90%, greater than at or about 95%, or at or about 100%.

In some embodiments, the dose of the variant ICOSL IgSF fusion protein administered is in an amount to exhibit, or predicted to exhibit, a target binding saturation for binding to ICOS of greater than at or about 25%, 30%, 40%, 50%, 60%, 70%, 80%, 90%, 95%, or more for a duration of at least or about 24 hours, 36 hours, 48 hours, 60 hours, 72 hours, or more. In some embodiments, the dose of the variant ICOSL IgSF fusion protein administered is in an amount to exhibit, or predicted to exhibit, a target binding saturation for binding to ICOS of greater than at or about 50%, 60%, 70%, 80%, 90%, 95%, or more for a duration of at least or about 24 hours, 36 hours, 48 hours, 60 hours, 72 hours, or more. In some embodiments, the dose of the variant ICOSL IgSF fusion protein administered is in an amount to exhibit, or predicted to exhibit, a target binding saturation for binding to ICOS of greater than at or about 50% for a duration of at or about 24 hours.

The pharmaceutical composition described herein (including pharmaceutical composition comprising the variant ICOSL IgSF domain fusion proteins) may be administered every 3 to 4 days, every week, biweekly, every three weeks, once a month, etc. depending on the half-life and clearance rate of the particular formulation. The frequency of dosing will depend upon the pharmacokinetic parameters of the molecule in the formulation used. Typically, a composition is administered until a dosage is reached that achieves the desired effect. The composition may therefore be administered as a single dose, or as multiple doses (at the same or different concentrations/dosages) over time, or as a continuous infusion. Further refinement of the appropriate dosage is routinely made. Appropriate dosages may be ascertained through use of appropriate dose-response data. In some cases, for example when a chronic inflammatory or autoimmune disorder is treated with a pharmaceutical composition provided herein, such as a variant ICOSL IgSF (e.g. IgV) Fc fusion protein provided herein, the composition is administered continuously, e.g., repeatedly, over time or intermittently over time. For example, treatment of a chronic inflammatory or autoimmune disorder, e.g., with a pharmaceutical composition provided herein, such as a variant ICOSL IgSF (e.g. IgV) Fc fusion protein provided herein, may include administering the treatment to a subject indefinitely. In some embodiments, when the inflammatory or autoimmune disorder is a chronic inflammatory or autoimmune disorder, treatment with a pharmaceutical composition provided herein, such as a variant ICOSL IgSF (e.g. IgV) Fc fusion protein provided herein, is continued following remission or partial remission of the disease and/or a reduction or amelioration in signs and/or symptoms of a disease, such as a reduction of one or more signs of inflammation in a subject having the chronic inflammatory or autoimmune disorder. In some embodiments, administration continues until any time as desired by a skilled practitioner. In some cases, for example when an acute inflammatory or autoimmune disorder is treated with a pharmaceutical composition provided herein, such as a variant ICOSL IgSF (e.g. IgV) Fc fusion protein provided herein, the composition is administered for a defined or limited period of time. In some embodiments, when the inflammatory or autoimmune disorder is an acute inflammatory or autoimmune disorder, treatment with a pharmaceutical composition provided herein, such as a variant ICOSL IgSF (e.g. IgV) Fc fusion protein provided herein, is discontinued following remission or partial remission of the disease and/or a reduction or amelioration in signs and/or symptoms of a disease, such as a reduction of one or more signs of inflammation in a subject having the acute inflammatory or autoimmune disorder. In some embodiments, administration is discontinued at any time as desired by a skilled practitioner.

Typically, precise amounts of the compositions provided herein to be administered can be determined by a physician with consideration of individual differences in age, weight, tumor size, extent of infection or metastasis, and condition of the patient (subject). In some embodiments, when referencing dosage based on mg/kg of the subject, an average human subject is considered to have a mass of about 70 kg-75 kg, such as 70 kg and a body surface area (BSA) of 1.73 $m^2$.

In some embodiments, the dosage, such as to achieve a therapeutically effective amount, of the pharmaceutical composition (including pharmaceutical composition comprising the variant ICOSL IgSF domain fusion proteins) is a single dose or a repeated dose in a treatment period, such as via administration of a multiple number of doses in the treatment period. In some embodiments, a single dose of a pharmaceutical composition provided herein containing a variant ICOSL IgSF domain fusion protein is administered during a treatment period. In some embodiments, multiple repeated doses of a pharmaceutical composition provided herein containing a variant ICOSL IgSF domain fusion protein is administered during a treatment period.

In some embodiments, the treatment period is repeated. For example, in cases where the disorder to be treated is a chronic inflammatory or autoimmune disorder, the treatment period including a specific treatment regimen, for example as described herein, may be repeated any number of times. In some embodiment, the treatment period is repeated indefinitely. In some embodiments, the treatment periods are repeated for at or about 2, 3, 4, 5, 6, or more years. In some embodiments, the treatment periods are repeated for at or about 6 years. In some embodiments, the treatment periods are repeated for at or about 3 years. In some embodiments, the number of repeats of a treatment period is any number desired by a skilled practitioner. In some embodiments, repeated treatment periods may be considered continuous treatment. For example, repeated treatment may be continuous when administration occurs once a week in a treatment period of 4 weeks, and the treatment period is repeated immediately following the end of the prior treatment period, and the treatment period is repeated indefinitely. In some embodiments, repeated treatment periods may be considered intermittent treatment. For example, repeated treatment may be intermittent when administration occurs once a week in a treatment period of 4 weeks, and the treatment period is repeated two weeks after the end of the first treatment period, and this cycle is repeated, for example indefinitely. In some embodiments, intermittent treatment is continuous treatment.

In some embodiments, about 1 or more (such as about 2 or more, about 3 or more, about 4 or more, about 5 or more, about 6 or more, or about 7 or more) multiple doses are given in a treatment period. In some embodiments, the treatment period is for greater than 20 days, such as 20 days to 40 days, such as for at or about 21 days, 22 days, 23 days, 24 days, 25 days, 26 days, 27 days, 28 days, 29 days, 30 days, 31 days, 32 days, 33 days, 34 days, 35 days, 36 days, 37 days, 38 days, 39 days or 40 days. In some embodiments, the treatment period is, is at least, or is about 41, 42, 43, 44, 45, 46, 47, 48, 49, or 50 days. In some embodiments, the treatment period is from at or about 28 to at or about 42 days. In some embodiments, the treatment period is at or about 42 days (at or about 6 weeks). In some embodiments, the treatment period is from at or about 21 days to at or about 28 days. In some embodiments, the treatment period is at or about 21 days. In some embodiments the treatment period is at or about 28 days (4 weeks). In some embodiments, the treatment period is 6 months or more. In some embodiments, the treatment period is 1, 2, 3, 4, 5, 6, 7, 8, 9, 10 years or more. In some embodiments, the treatment period is about 1year to about 3 years. In some embodiments, the treatment period is about 3 years to about 6 years. In some embodiments, the treatment period is repeated. In some embodiments, the treatment period is repeated. In some embodiments, the treatment period is repeated. In some embodiments, the treatment period is repeated indefinitely. For example, indefinite treatment may be used to treat a chronic inflammatory or autoimmune disorder. In some embodiments, the treatment period is repeated immediately following a prior treatment period. In some embodiments, the treatment period is repeated with a time delay following a prior treatment period. In some embodiments, the delay is, is about, or is at least 1, 2, 3, 4, 5, 6, 7, 8 weeks. In some embodiments, the delay is, is about, or is at least 1 month, 2 months, 3 months, 4 months, 5 months, 6 months, or up to a year.

In some embodiments, more than one treatment period and/or regimen may be combined. For example, in some cases, an initial treatment period may be four weeks and include administration every two weeks, and a second treatment period may be four weeks with admisntrartion every fourth week. In some embodiments, the first or second treatment period may be repeated. For example, in some embodiments, the second treatment period of four weeks with administration every fouth week may be repeated, for example, as many times as desired by a skilled practitioner.

In some embodiments, each dose of the multiple number of doses is administered twice a week in the treatment period. In particular embodiments, each dose of the multiple number of doses is administered no more than once weekly in the treatment period. In some embodiments, each does of the multiple number of doses is administered once a week (Q1W) in the treatment period. In some embodiments, the dosing regimen includes administration of 4 doses given once a week for 4 weeks. In some embodiments, the dosing regimen includes an initial administration, administration at 2 and 4 weeks following the initital administration, and every 4 weeks thereafter.

In some embodiments, each dose of a multiple number of doses is administered daily. This may occur, for example, in cases where the inflammatory or autimmune disease is an acute disease, and the subject is treated in a hospital or other health care facility.

In some embodiments, an administered dose of the pharmaceutical composition (including pharmaceutical composition comprising the variant ICOSL IgSF domain fusion proteins) is in an amount of from at or about 0.001 mg/kg to at or about 10 mg/kg. In some embodiments, an administered dose of the pharmaceutical composition (including pharmaceutical composition comprising the variant ICOSL IgSF domain fusion proteins) is in an amount of from at or about 0.001 mg/kg to at or about 20 mg/kg. In some embodiments, an administered dose of the pharmaceutical composition (including pharmaceutical composition comprising the variant ICOSL IgSF domain fusion proteins) is in an amount of from at or about 0.1 mg/kg to at or about 10 mg/kg. In some embodiments, an administered dose of the pharmaceutical composition (including pharmaceutical composition comprising the variant ICOSL IgSF domain fusion proteins) is in an amount of from at or about 0.1 mg/kg to at or about 20 mg/kg. In some embodiments, an administered dose of the pharmaceutical composition (including pharmaceutical composition comprising the variant ICOSL IgSF domain fusion proteins) is in an amount from at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 6 mg/kg, at or about 1 mg/kg to at or about 3 mg/kg, at or about 3 mg/kg to at or about 6 mg/kg. In some embodiments, an administered dose of the pharmaceutical composition (including pharmaceutical composition comprising the variant ICOSL IgSF domain fusion proteins) is in an amount from at or about 0.1 mg/kg to at or about 20 mg/kg, at or about 0.1 mg/kg to at or about 10 mg/kg, at or about 0.1 mg/kg to at or about 6 mg/kg, at or about 0.1 mg/kg to at or about 3 mg/kg, at or about 0.1 mg/kg to at or about 1 mg/kg, at or about 1 mg/kg to at or about 20 mg/kg, at or about 1 mg/kg to at or about 10 mg/kg, at or about 1 mg/kg to at or about 6 mg/kg, at or about 1 mg/kg to at or about 3 mg/kg, at or about 3 mg/kg to at or about 20 mg/kg, at or about 3 mg/kg to at or about 10 mg/kg, at or about 3 mg/kg to at or about 6 mg/kg, at or about 6 mg/kg to at or about 20 mg/kg, at or about 6 mg/kg to at or about 10 mg/kg, or at or about 10 mg/kg to at or about 20 mg/kg. In some embodiments, an administered dose of the pharmaceutical composition (including pharmaceutical composition comprising the variant ICOSL IgSF domain fusion proteins) is in an amount of at or about 0.3 mg/kg, at or about 1 mg/kg, at or about 3 mg/kg or at or about 6 mg/kg. In some embodiments, an administered dose of the pharmaceutical composition (including pharmaceutical composition comprising the variant ICOSL IgSF domain fusion proteins) is in an amount of at or about 10 mg/kg.

In some embodiments, the pharmaceutical composition (including pharmaceutical composition comprising the variant ICOSL IgSF domain fusion proteins) is administered to a subject through any route, including orally, transdermally, by inhalation, intravenously, intra-arterially, intramuscularly, direct application to a wound site, application to a surgical site, intraperitoneally, by suppository, subcutaneously, intradermally, transcutaneously, by nebulization, intrapleurally, intraventricularly, intra-articularly, intraocularly, intraspinally, intratumorally or systemically.

In some embodiments, the pharmaceutical composition (including pharmaceutical composition comprising the variant ICOSL IgSF domain fusion proteins) is administered parenterally. Examples provided herein demonstrate that particularly suitable routes of administration include intravenous, subcutaneous or intratumoral administration. In some embodiments, the pharmaceutical composition is in a form suitable for administration by injection, such as by bolus injection. In some embodiments, the pharmaceutical composition is in a form suitable for infusion injection, for example by intravenous injection. In some embodiments, the infusion duration is, is at least, or is about 30 minutes, 40 minutes, 50 minutes, 1 hour, 1.5 hours, 2 hours, 3 hours, 4 hours, 5 hours or 6 hours. In some embodiments the infusion duration is between about 30 minutes and 6 hours. In some embodiments, the infusion duration is between about 30 minutes and 5 hours. In some embodiments, the infusion duration is between about 30 minutes and 4 hours. In some embodiments, the infusion duration is between about 30 minutes and 3 hours. In some embodiments, the infusion duration is between about 30 minutes and 2 hours. In some embodiments, the infusion duration is between about 30 minutes and 1 hour. In some embodiments, the infusion duration is or is about 30 minutes.

In some embodiments, a dose regimen as described herein is administered to achieve a therapeutically effective amount.

It is contemplated that dosing (e.g., multiple doses), can continue until any time as desired by a skilled practitioner. For example, dosing may continue until a desirable disease response is achieved, such as in remission or partial remission of the disease and/or a reduction or amelioration in signs and/or symptoms of a disease, such as a reduction of one or more signs of inflammation in the subject. In some embodiments, the dosing is continued following remission or partial remission of the disease and/or a reduction or amelioration in signs and/or symptoms of a disease, such as a reduction of one or more signs of inflammation in the subject.

In some embodiments of the methods, the treating can result in a clinical remission. In some aspects, the treating can result in a clinical remission without about 1 week, about 2 weeks, about 3 weeks, about 4 weeks, about 5 weeks, about 6 weeks, about 7 weeks, about 9 weeks, about 10 weeks, about 11 weeks, about 12 weeks, about 14 weeks, about 16 weeks, about 18 weeks, about 20 weeks, about 22 weeks, about 24 weeks, about 26 weeks, about 28 weeks, about 30 weeks, about 32 weeks, about 34 weeks, about 36 weeks, about 38 weeks, about 40 weeks, about 42 weeks, about 44 weeks, about 46 weeks, about 48 weeks, about 50 weeks, about 52 weeks, about 54 weeks, about 56 weeks, about 58 weeks, about 60 weeks, about 62 weeks, about 64 weeks, about 66 weeks, about 68 weeks, about 70 weeks, about 72 weeks, about 74 weeks, about 76 weeks, about 78 weeks, or about 80 weeks from the first dose. In some embodiments, the treating results in a clinical remission within about 10 weeks from the first dose. In some embodiments, the treating results in a clinical remission within about 6 weeks from the first dose. In some embodiments, the treating results in a clinical remission at about 6 weeks from the first dose and at about 10 weeks from the first dose.

In some embodiments of any of the preceding methods, the clinical remission is a sustained remission. For example, in some embodiments, the sustained remission is a clinical remission at about 10 weeks, about 15 weeks, about 20 weeks, about 25 weeks, about 30 weeks, about 35 weeks, about 40 weeks, about 45 weeks, about 50 weeks, about 52 weeks, about 55 weeks, about 60 weeks, about 65 weeks, about 70 weeks, about 72 weeks, about 75 weeks, about 80 weeks, about 85 weeks, about 90 weeks, about 95 weeks, about 100 weeks, about 102 weeks, about 105 weeks, or about 110 weeks from the first dose. In some embodiments, the sustained remission is a clinical remission at about ten weeks from the first dose and at about 30 weeks from the first dose. In some embodiments, the sustained remission has a length of at least about 30 weeks, or at least about 7, about 8, about 9, about 10, about 11, or about 12 months. In some embodiments of any of the preceding aspects, the amelioration of one or more symptoms of the disease or condition, clinical remission, and/or clinical response is maintained at least one month (e.g., at least one month, at least two months, at least three months, at least four months, at least five months, at least six months, at least seven months, at least eight months, at least nine months, at least ten months, at least eleven months, at least twelve months, or longer) after the end of treatment.

*2. Ocular Inflammatory or Autoimmune Diseases*

In some embodiments, the provided compositions and methods are used for treating an inflammatory or autoimmune disorder that affects the eye. In some embodiments, systemic autoimmune diseases, such as rheumatoid arthritis, Juvenile rheumatoid arthritis, Sjögren's syndrome, Enteropathic arthritis, Psoriatic arthritis, Mutliple Sclerosis, Giant cell arteritis, Graves' disease, Myasthenia gravis, Sarcoidosis, Takayasu's arteritis, Dermatomyositis, systemic lupus erythematosus, polyarteritis nodosa, relapsing polychondritis, Granulomatosis with Polyangiitis (formerly called Wegener's), scleroderma, Behcet's disease, Reactive Arthritis (formerly called Reiter's syndrome), inflammatory bowel disease (ulcerative colitis and Crohn's disease), Sjogren's syndrome, and ankylosing spondylitis, include the eye as a target of immune inflammatory attack, e.g., by white blood cells. In some case, the ocular manifestation of the systemic inflammatory or autoimmune disease is keratoconjunctivitis sicca, scleritis, episcleritis, keratitis, ulcerative keratitis, choroiditis, retinal vasculitis, episcleral nodules, retinal detachments, macular edemauveitis, episcleritis, peripheral ulcerative keratitis, conjunctivitis, uveitis, scleritis, reinal hemorrages, proliferative retinopathy, ischemic optic neuopathy, hemianopia, amaurosis, internuclear ophthalmoplegia, pupillary abnormalities, oculomotor abnormalities, visual hallucinations, retrobulbar neuritis, visual field defects, internuclear ophthalmoplegia, dysmetria, nystagmus, cranial nerve palsies, Amaurosis fugax, diplopia, vision loss, proptosis/exophthalmos, lid lag and retraction, decreased visual acuity, reduced visual fields, relative afferent pupillary defect, loss of color vision, diplopia, eye ptosis, conjunctival nodules, cranial nerve palsies, enlarged lacrimal glands, orbital cellulitis, corneal ulcers, hypopyon, vaso-occlusive retinopathy, cataracts, and/or eyelid/conjunctival edeam. In some cases, the ocular manifestation is specific to the systemic inflammatory or autoimmune disease.

In some cases, the eye is the specific and only target, e.g., eye-specific, affected by an autoimmune disease. Examples of eye-specific autoimmune diseases include, but are not limited to, ocular cicatricial pemphigoid, Mooren's corneal ulcer, and uveitis.

Local ocular administration of corticosteroids, for example by intravitreal injection, is generally considered the standard of care for inflammatory or autoimmune diseases affecting the eye, such as uveitis. Use of corticosteroids, however, carries a risk of side effects including, for example, cataracts and glaucoma. The compositions and methods provided herein are contemplated for use in treating inflammatory or autoimmune diseases affecting the eye. In some embodiments, the compositions and methods provided herein treat systemic autoimmune disease affecting the eye or eye-specific autoimmune diseases without the side effects associated with the use of steroids. In some embodiments, the compositions and methods provided herein are administered as a prophylactic, for example to prevent an eye-specific inflammatory or autoimmune disease. In some embodiments, the compositions and methods provided herein are administered as a prophylactic, for example to prevent an ocular manifestation of a systemic inflammatory or autoimmune disease. In some embodiments, the compositions and methods provided herein are administered as a therapeutic treatment, for example to treat an eye-specific inflammatory or autoimmune disease. In some embodiments, the compositions and methods provided herein are administered as a therapeutic treatment, for example to treat an ocular manifestation of a systemic inflammatory or autoimmune disease. In some embodiments, treatment includes reducing or amelioriating symptoms of the autoimmune or inflammaotory disease affecting the eye.

In some embodiments, the inflammatory or autoimmune disease to be treated is a systemic autoimmune disease that affects the eye, e.g., results in an ocular manifestation. In some embodiments, the inflammatory or autoimmune disease to be treated is rheumatoid arthritis that affects the eye. In some embodiments, the inflammatory or autoimmune disease to be treated is juvenile rheumatoid arthritis that affects the eye. In some embodiments, the inflammatory or autoimmune disease to be treated is Sjögren's syndrome that affects the eye. In some embodiments, the inflammatory or autoimmune disease to be treated is psoriatic arthritis that affects the eye. In some embodiments, the inflammatory or autoimmune disease to be treated is systemic lupus erythematosus that affects the eye. In some embodiments, the inflammatory or autoimmune disease to be treated is sarcoidosis that affects the eye. In some embodiments, the inflammatory or autoimmune disease to be treated is dermatomyositis that affects the eye. In some embodiments, the inflammatory or autoimmune disease to be treated is polyarteritis nodosa that affects the eye. In some embodiments, the inflammatory or autoimmune disease to be treated is relapsing polychondritis that affects the eye. In some embodiments, the inflammatory or autoimmune disease to be treated is Granulomatosis with Polyangiitis that affects the eye. In some embodiments, the inflammatory or autoimmune disease to be treated is scleroderma that affects the eye. In some embodiments, the inflammatory or autoimmune disease to be treated is Behcet's disease that affects the eye. In some embodiments, the inflammatory or autoimmune disease to be treated is Reactive Arthritis that affects the eye. In some embodiments, the inflammatory or autoimmune disease to be treated is inflammatory bowel disease (ulcerative colitis and Crohn's disease) that affects the eye. In some embodiments, the inflammatory or autoimmune disease to be treated is ankylosing spondylitis that affects the eye. In some embodiments, the systemic autoimmune or inflammatory disease affects the eye resulting in an ocular manifestation, for example as described above. In some embodiments, inflammatory or autoimmune disease affects the eye by generating an inflammatory response in the eye. In some embodiments, the inflammatory or autoimmune disease affects the eye by casuing infiltration of immune cells into the eye. In some embodiments, the treatment is a prophylactic treatment. For example, a subject having a systemic autoimmune or inflammatory disease may be treated with a composition provided herein prior to showing symptoms of eye inflammation (e.g., ocular manifestation). In some embodiments, the subject having the systemic autoimmune disease or inflammatory disease may be suspected of developing an ocular manifestation, e.g., inflammatory response. In some embodiments, the treatment is a therapeutic treatment, delievered following the onset of eye symptoms.

In some embodiments, the inflammatory or autoimmune disease to be treated is ocular cicatricial pemphigoid. In some embodiments, the inflammatory or autoimmune disease to be treated is Mooren's corneal ulcer. In some embodiments, the inflammatory or autoimmune disease to be treated is uveitis or a specific type thereof. In some embodiments, the inflammatory or autoimmune disease affects the eye by generating an inflammatory response in the eye. In some embodiments, the inflammatory or autoimmune disease affects the eye by casuing infiltration of immune cells into the eye. In some embodiments, the treatment is a prophylactic treatment. For example, a subject suspected of having an eye-specific autoimmune or inflammatory disease, e.g., positive for one or more biomarkers of an eye-specific autimmune or inflammatory disease may be treated with a composition provided herein prior to showing symptoms, e.g., eye inflammation. In some embodiments, the treatment is a therapeutic treatment, delivered following the onset of eye symptoms, e.g., inflammation.

In some embodiments, the inflammatory or autoimmune disease to be treated is uveitis. In some cases, uveitis results in inflammation of the middle layer of tissue in the eye wall (uvea). In some cases, uveitis involves inflammation of the iris. In some embodiments, uveitis involves inflammation of the ciliary body. In some embodiments, uveitis involves inflammation of the choroid. In some embodiments, the uveitis involves inflammation of the retina. In some embodiments, uveitis involves inflammation of all layers of the uvea. In some embodiments, uveitis is associated with an inflamed vitreous and/or infiltration of the vitreous with inflammatory cells. In some embodiments, uveitis affects one or both eyes.

In some embodiments, a pharmaceutical composition provided herein, such as a variant ICOSL IgSF (e.g. IgV) Fc fusion protein provided herein, is used to treat uveitis. In some embodiments, the uveitis is iritis (anterior uveitis). In some embodiments, the uveitis is cyclitis (intermediate uveitis). In some embodiments, the uveitis is choroiditis. In some embodiments, the uveitis is retinitus. In some embodiments, the uveitis is diffuse uveitis (panuveitis). In some embodiments, uveitis is associated with an inflammed vitreous. In some embodiments, uveitis is associated with infiltration of inflammatory cells into the vitreous. In some embodiments, uvieitis affects one or both eyes. In some embodiments, uveitis is associated with eye redness, eye pain, light sensitivity, blurred vision, dark and/or floating spots in the field of view, and/or decreased vision. In some embodiments, uveitis is caused by sarcoidosis. In some embodiments, uveitis is caused by ankylosing spondylitis. In some embodiments, uveitis is caused by Crohn's disease. In some embodiments, uveitis is caused by ulcerative colitis. In some embodiments, uveitis is caused by rheumatoid arthritis. In some embodiments, uveitis is caused by juvenile rheumatoid arthritis. In some embodiments, uveitis is caused by reactive arthritis. In some embodiments, uveitis is caused by enteropathic arthritis. In some embodiments, uveitis is caused by psoriatic arthritis. In some embodiments, uveitis is caused by systemic lupus erythematosus. In some embodiments, uveitis is caused by Bechet's syndrome. In some embodiments, uveitis is caused by dermatomyositis. In some embodiments, uveitis is caused by Granulomatosis with Polyangiitis. In some embodiments, uveitis is caused by an infection, such as cat-scratch disease, herpes zoster, syphilis, toxoplasmosis, tuberculosis, Lyme disease or West Nile virus. In some embodiments, uveitis is caused by a cancer that affects the eye, such as lymphoma. In some embodiments, the uveitis is an eye-specific autoimmune or inflammatory disease, e.g., not cause by a systemic autoimmune or inflammatory disease.

In some cases, the ocular inflammatory or autoimmune disease is a chronic disease. For example, the ocular inflammatory or autoimmune disease may be considered a chronic disease when the disease is an ocular manifestation of a chronic inflammatory or autoimmune disorder, for example as described herein (see, e.g., Section VII-A-1), or is a chronic form of an eye-specific inflammatory disease described above. In some cases, the ocular inflammatory or autoimmune disease is an acute disease. For example, the ocular inflammatory or autoimmune disease may be considered an acute disease when the disease is an ocular manifestation of an acute inflammatory or autoimmune disorder, for example as described herein (see, e.g., Section VII-A-1) or is an acute form of an eye-specific inflammatory disease described above.

In some cases, the pharmaceutical composition provided herein, such as a variant ICOSL IgSF (e.g. IgV) Fc fusion protein provided herein, may be used to treat chronic ocular inflammatory or autoimmune diseases. In some embodiments, treatment of chronic ocular inflammatory or autimmune diseases with the pharmaceutical composition provided herein, such as a variant ICOSL IgSF (e.g. IgV) Fc fusion protein provided herein, includes continuous administration to a subject. In some embodiments, continuous administration is a repeated administration. In some cases, a treatment period including a treatment regimen is repeated. As described herein, in some embodiments, the treatment period is repeated. In some embodiments, the repeated treatment period occurs immediately following a prior treatment period. In some embodiments, administration is repeated until any time as desired by a skilled practitioner. For example, treatment of a chronic ocular inflammatory or autoimmune disease, e.g., with a pharmaceutical composition provided herein, such as a variant ICOSL IgSF (e.g. IgV) Fc fusion protein provided herein, may include administering treatment to the subject indefinitely.

In some cases, treatment with a pharmaceutical composition provided herein, such as a variant ICOSL IgSF (e.g. IgV) Fc fusion protein provided herein, to treat a chronic ocular inflammatory or autoimmune disease includes intermittent administration. For example, treatment of a chronic ocular inflammatory or autoimmune disease, e.g., with a pharmaceutical composition provided herein, such as a variant ICOSL IgSF (e.g. IgV) Fc fusion protein provided herein, may include administering a treatment in a cyclic manner, such that a particular treatment regimen, for example a treatment regimen in a treatment period, is administered repeatedly any number of times to a subject. In some embodiments, the treatment period and treatment regimen are repeated with a delay between the treatment periods. In some embodiment, the treatment period is repeated indefinitely. In some embodiments, treatment is administered to a subject any number of times as desired by a skilled practitioner.

In some embodiments, treatment is administered to a subject to maintain a desired effect. For example, in some embodiments, when the ocular inflammatory or autoimmune disease is a chronic ocular inflammatory or autoimmune disease, treatment with a pharmaceutical composition provided herein, such as a variant ICOSL IgSF (e.g. IgV) Fc fusion protein provided herein, is continued following remission or partial remission of the disease and/or a reduction or amelioration in signs and/or symptoms of a disease, such as a reduction of one or more signs of inflammation in a subject having the chronic ocular inflammatory or autoimmune disorder. In some embodiments, treatment may be continued so as to prevent resurgence of symptoms and/or maintain remission or reduction of symptoms of the chronic ocular inflammatory or autoimmune disorder. In some embodiments, administration continues until any time as desired by a skilled practitioner. In some embodiments, the continued administration is in the form of repeated treatment periods as described herein.

In some cases, the pharmaceutical composition provided herein, such as a variant ICOSL IgSF (e.g. IgV) Fc fusion protein provided herein, may be used to treat acute ocular inflammatory or autoimmune disease. For example, treatment of an acute ocular inflammatory or autoimmune disease, e.g., with a pharmaceutical composition provided herein, such as a variant ICOSL IgSF (e.g. IgV) Fc fusion protein provided herein, may include administering the treatment to the subject for a defined or limited time period. In some embodiments, the administration is for a single treatment period which is not repeated. In some embodiments, the duration or treatment period is set as desired by a skilled practitioner.

In some embodiments, when the inflammatory or autoimmune disorder is an acute ocular inflammatory or autoimmune disease, treatment with a pharmaceutical composition provided herein, such as a variant ICOSL IgSF (e.g. IgV) Fc fusion protein provided herein, is discontinued following remission or partial remission of the disease and/or a reduction or amelioration in signs and/or symptoms of a disease, such as a reduction of one or more signs of inflammation in a subject having an acute disease or disorder. In some embodiments, administration is discontinued at any time as desired by a skilled practitioner.

## Dosage and Administration

Methods for treating ocular inflammatory and autoimmune diseases, such as described herein, may include systemic administration, local administration, or both. The symptoms presented and/or the underlining cause of the eye disease can inform the method of administration. In some embodiments, the symptoms presented and/or the underlining cause of the eye disease can inform whether acute or chronic treatment is administered.

In some embodiments, a pharmaceutical composition described herein (including pharmaceutical composition comprising the variant ICOSL IgSF domain fusion proteins) is administered to a subject having an ocular inflammatory or autoimmune disease systemically. For example, systemic administration can occur according to any of the embodiments described herein. In some embodimetns, systemic administration is accomplished according to the methods described in Section VII-A-1. In some embodiments, systemic administration is used when the subject has a systemic autoimmune or inflammatory disease, e.g., as described above, that can or presents with an ocular manifestation, e.g., eye inflammation. In some embodiments, systemic administration is used when the subject has an eye-specific autoimmune or inflammatory disease, e.g., as described above.

In some embodiments, a pharmaceutical composition described herein (including pharmaceutical composition comprising the variant ICOSL IgSF domain fusion proteins) is locally, e.g., intraocularly, administered to a subject having an ocular inflammatory or autoimmune disease. In some embodiments, administration is intravitreal.

In some embodiments, modeling and simulation of pharmacokinetic (PK) and pharmacodynamic (PD) profiles observed in control animals and animal models of disease (e.g., uveitis models) following intravitreal administration can be used to predict or determine patient dosing. For example, PK data from non-human primates (e.g., cynomolgus monkeys) can be used to estimate human PK. Similarly, mouse and/or rat PK and PD data can be used to predict human dosing. The observed animal data can be used to inform computational models which can be used to simulate human dose response.

In some embodiments, methods provided herein including administering a pharmaceutical composition described herein (including pharmaceutical composition comprising the variant ICOSL IgSF domain fusion proteins) in an amount in which a dose is known or predicted to achieve target binding saturation to its cognate ligands, e.g. CD28 and/or ICOS, sufficient for a therapeutic effect. Target binding saturation for binding to cognate ligands can be determined experimentally or empirically. In some embodiments, target binding saturation can be determined from in vitro binding data to mammalian cells that express the cognate ligand, such as a human CD28 or human ICOS.

In some embodiments, the dose of the variant ICOSL IgSF fusion protein administered is in an amount to exhibit, or predicted to exhibit, a target binding saturation for binding to CD28 of greater than at or about 25% at $C_{max}$, such as greater than at or about 30% at $_{max,}$, greater than at or about 35% at$C_{max}$, greater than at or about 40% at $C_{max}$, greater than at or about 50% at $C_{max}$ or greater. In some embodiments, the dose of the variant ICOSL IgSF fusion protein administered is in an amount to exhibit, or predicted to exhibit, a target binding saturation for binding to CD28 of, of at least, or about about 85% at $C_{max}$. In some embodiments, the dose of the variant ICOSL IgSF fusion protein administered is in an amount to exhibit, or predicted to exhibit, a target binding saturation for binding to CD28 of greater than at or about 10% within 24 hours of the administration of the dose, such as greater than at or about 15%, greater than at or about 20%, greater than at or about 25%, greater than at or about 30%, greater than at or about 50%, greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to CD28 of greater than at or about 50% within 2 days of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to CD28 of greater than at or about 50% within 7 days (one week) of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to CD28 of greater than at or about 50% within 14 days (2 weeks) of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to CD28 of greater than at or about 50% within 21 days of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to CD28 of greater than at or about 50% within 28 days of the administration of the dose, such as greater than at or about 60%, greater than at or about 70%, greater than at or about 80%, greater than at or about 85%, greater than at or about 90%, greater than at or about 95%, or at or about 100%.

In some embodiments, the dose of the variant ICOSL IgSF fusion protein administered is in an amount to exhibit, or predicted to exhibit, a target binding saturation for binding to ICOS of greater than at or about 25% at $C_{max}$, such as greater than at or about 30% at $_{max,}$, greater than at or about 35% at$C_{max}$, greater than at or about 40% at $C_{max}$, greater than at or about 50% at $C_{max}$ or greater. In some embodiments, the dose of the variant ICOSL IgSF fusion protein administered is in an amount to exhibit, or predicted to exhibit, a target binding saturation for binding to ICOS of greater than at or about 10% within 24 hours of the administration of the dose, such as greater than at or about 15%, greater than at or about 20%, greater than at or about 25%, greater than at or about 30%, greater than at or about 50%, greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to ICOS of greater than at or about 50% within 2 days of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to ICOS of greater than at or about 50% within 7 days (one week) of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to ICOS of greater than at or about 50% within 14 days (2 weeks) of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to ICOS of greater than at or about 50% within 21 days of the administration of the dose, such as greater than at or about 60%, greater than at or about 70%, greater than at or about 80%, greater than at or about 85%, greater than at or about 90%, greater than at or about 95%, or at or about 100%. In some embodiments, the dose of the variant ICOSL IgSF fusion protein administered is in an amount to exhibit, or predicted to exhibit, a target binding saturation for binding to ICOS of greater than at or about 50% within 28 days of the administration of the dose, such as greater than at or about 60%, greater than at or about 70%, greater than at or about 80%, greater than at or about 85%, greater than at or about 90%, greater than at or about 95%, or at or about 100%.

In some embodiments, the dose of the variant ICOSL IgSF fusion protein administered is in an amount to exhibit, or predicted to exhibit, a target binding saturation for binding to ICOS of greater than at or about 25%, 30%, 40%, 50%, 60%, 70%, 80%, 90%, 95%, or more for a duration of at least or about 24 hours, 36 hours, 48 hours, 60 hours, 72 hours, or more. In some embodiments, the dose of the variant ICOSL IgSF fusion protein administered is in an amount to exhibit, or predicted to exhibit, a target binding saturation for binding to ICOS of greater than at or about 50%, 60%, 70%, 80%, 90%, 95%, or more for a duration of at least or about 24 hours, 36 hours, 48 hours, 60 hours, 72 hours, or more. In some embodiments, the dose of the variant ICOSL IgSF fusion protein administered is in an amount to exhibit, or predicted to exhibit, a target binding saturation for binding to ICOS of greater than at or about 50% for a duration of at or about 24 hours.

The pharmaceutical composition described herein (including pharmaceutical composition comprising the variant ICOSL IgSF domain fusion proteins) may be administered every 3 to 4 days, every week, biweekly, every three weeks, once a month, etc. depending on the half-life and clearance rate of the particular formulation. The frequency of dosing will depend upon the pharmacokinetic parameters of the molecule in the formulation used. Typically, a composition is administered until a dosage is reached that achieves the desired effect. The composition may therefore be administered as a single dose, or as multiple doses (at the same or different concentrations/dosages) over time. Further refinement of the appropriate dosage is routinely made. Appropriate dosages may be ascertained through use of appropriate dose-response data. In some cases, for example when a chronic ocular inflammatory or autoimmune disease is treated with a pharmaceutical composition provided herein, such as a variant ICOSL IgSF (e.g. IgV) Fc fusion protein provided herein, the composition is administered continuously, e.g., repeatedly, over time or intermittently over time. For example, treatment of a chronic ocular inflammatory or autoimmune disease, e.g., with a pharmaceutical composition provided herein, such as a variant ICOSL IgSF (e.g. IgV) Fc fusion protein provided herein, may include administering the treatment to the subject indefinitely. In some embodiments, when the ocular inflammatory or autoimmune disease is a chronic ocular inflammatory or autoimmune disease, treatment with a pharmaceutical composition provided herein, such as a variant ICOSL IgSF (e.g. IgV) Fc fusion protein provided herein, is continued following remission or partial remission of the disease and/or a reduction or amelioration in signs and/or symptoms of a disease, such as a reduction of one or more signs of inflammation in a subject having the chronic ocular inflammatory or autoimmune disease. In some embodiments, administration continues until any time as desired by a skilled practitioner. In some cases, for example when an acute ocular inflammatory or autoimmune disease is treated with a pharmaceutical composition provided herein, such as a variant ICOSL IgSF (e.g. IgV) Fc fusion protein provided herein, the composition is administered for a defined or limited period of time. In some embodiments, when the ocular inflammatory or autoimmune disease is an acute disease, treatment with a pharmaceutical composition provided herein, such as a variant ICOSL IgSF (e.g. IgV) Fc fusion protein provided herein, is discontinued following remission or partial remission of the disease and/or a reduction or amelioration in signs and/or symptoms of the disease, such as a reduction of one or more signs of inflammation in a subject having the acute disease. In some embodiments, administration is discontinued at any time as desired by a skilled practitioner.

Typically, precise amounts of the compositions provided herein to be administered can be determined by a physician, e.g., an ophthalmologist, with consideration of individual differences in condition of the patient (subject).

In some embodiments, the dosage, such as to achieve a therapeutically effective amount, of the pharmaceutical composition (including pharmaceutical composition comprising the variant ICOSL IgSF domain fusion proteins) is a single dose or a repeated dose in a treatment period, such as via administration of a multiple number of doses in the treatment period. In some embodiments, a single dose of a pharmaceutical composition provided herein containing a variant ICOSL IgSF domain fusion protein is administered during a treatment period. In some embodiments, multiple repeated doses of a pharmaceutical composition provided herein containing a variant ICOSL IgSF domain fusion protein is administered during a treatment period.

In some embodiments, the treatment period is repeated. For example, in cases where the disorder to be treated is a chronic ocular inflammatory or autoimmune disease, the treatment period including a specific treatment regimen, for example as described herein, may be repeated any number of times. In some embodiment, the treatment period is repeated indefinitely. In some embodiments, the treatment periods are repeated for at or about 2, 3, 4, 5, 6, or more years. In some embodiments, the treatment periods are repeated for at or about 6 years. In some embodiments, the treatment periods are repeated for at or about 3 years. In some embodiments, the number of repeats of a treatment period is any number desired by a skilled practitioner. In some embodiments, repeated treatment periods may be considered continuous treatment. For example, repeated treatment may be continuous when administration occurs once a week in a treatment period of 4 weeks, and the treatment period is repeated immediately following the end of the prior treatment period, and the treatment period is repeated indefinitely. In some embodiments, repeated treatment periods may be considered intermittent treatment. For example, repeated treatment may be intermittent when administration occurs once a week in a treatment period of 4 weeks, and the treatment period is repeated two weeks after the end of the first treatment period, and this cycle is repeated, for example indefinitely. In some embodiments, intermittent treatment is continuous treatment.

In some embodiments, about 1 or more (such as about 2 or more, about 3 or more, about 4 or more, about 5 or more, about 6 or more, or about 7 or more) multiple doses are given in a treatment period. In some embodiments, the treatment period is for greater than 20 days, such as 20 days to 40 days, such as for at or about 21 days, 22 days, 23 days, 24 days, 25 days, 26 days, 27 days, 28 days, 29 days, 30 days, 31 days, 32 days, 33 days, 34 days, 35 days, 36 days, 37 days, 38 days, 39 days or 40 days. In some embodiments, the treatment period is, is at least, or is about 41, 42, 43, 44, 45, 46, 47, 48, 49, or 50 days. In some embodiments, the treatment period is from at or about 28 to at or about 42 days. In some embodiments, the treatment period is at or about 42 days (at or about 6 weeks). In some embodiments, the treatment period is from at or about 21 days to at or about 28 days. In some embodiments, the treatment period is at or about 21 days. In some embodiments the treatment period is at or about 28 days (4 weeks). In some embodiments, each dose of the multiple number of doses is administered twice a week in the treatment period. In particular embodiments, each dose of the multiple number of doses is administered no more than once weekly in the treatment period. In some embodiments, each does of the multiple number of doses is administered once a week (Q1W) in the treatment period. In some embodiments, the dosing regimen includes administration of 4 doses given once a week for 4 weeks. In some embodiments, each dose of the multiple dose is administered twice a week (BIW). In some embodiments, the treatment period is 6 months or more. In some embodiments, the treatment period is 1, 2, 3, 4, 5, 6, 7, 8, 9, 10 years or more. In some embodiments, the treatment period is about 1year to about 3 years. In some embodiments, the treatment period is about 3 years to about 6 years. In some embodiments, the treatment period is repeated. In some embodiments, the treatment period is repeated. In some embodiments, the treatment period is repeated. In some embodiments, the treatment period is repeated indefinitely. For example, indefinite treatment may be used to treat a chronic ocular inflammatory or autoimmune disease. In some embodiments, the treatment period is repeated immediately following a prior treatment period. In some embodiments, the treatment period is repeated with a time delay following a prior treatment period. In some embodiments, the delay is, is about, or is at least 1, 2, 3, 4, 5, 6, 7, 8 weeks. In some embodiments, the delay is, is about, or is at least 1 month, 2 months, 3 months, 4 months, 5 months, 6 months, or up to a year.

In some embodiments, more than one treatment period and/or regimen may be combined. For example, in some cases, an initial treatment period may be four weeks and include administration every two weeks, and a second treatment period may be four weeks with administrartion every fourth week. In some embodiments, the first or second treatment period may be repeated. For example, in some embodiments, the second treatment period of four weeks with administration every fouth week may be repeated, for example, as many times as desired by a skilled practitioner.

In some embodiments, each dose of the multiple number of doses is administered twice a week in the treatment period. In particular embodiments, each dose of the multiple number of doses is administered no more than once weekly in the treatment period. In some embodiments, each does of the multiple number of doses is administered once a week (Q1W) in the treatment period. In some embodiments, the dosing regimen includes administration of 4 doses given once a week for 4 weeks. In some embodiments, the dosing regimen includes an initial administration, administration at 2 and 4 weeks following the initital administration, and every 4 weeks thereafter.

In some embodiments, each dose of a multiple number of doses is administered daily. This may occur, for example, in cases where the inflammatory or autimmune disease is an acute disease, and the subject is treated in a hospital or other health care facility.

In some embodiments, an administered dose of the pharmaceutical composition (including pharmaceutical composition comprising the variant ICOSL IgSF domain fusion proteins) is in an amount from at or about 0.1 mg/kg to at or about 20 mg/kg, at or about 0.1 mg/kg to at or about 10 mg/kg, at or about 0.1 mg/kg to at or about 6 mg/kg, at or about 0.1 mg/kg to at or about 3 mg/kg, at or about 0.1 mg/kg to at or about 1 mg/kg, at or about 1 mg/kg to at or about 20 mg/kg, at or about 1 mg/kg to at or about 10 mg/kg, at or about 1 mg/kg to at or about 6 mg/kg, at or about 1 mg/kg to at or about 3 mg/kg, at or about 3 mg/kg to at or about 20 mg/kg, at or about 3 mg/kg to at or about 10 mg/kg, at or about 3 mg/kg to at or about 6 mg/kg, at or about 6 mg/kg to at or about 20 mg/kg, at or about 6 mg/kg to at or about 10 mg/kg, or at or about 10 mg/kg to at or about 20 mg/kg. In some embodiments, an administered dose of the pharmaceutical composition (including pharmaceutical composition comprising the variant ICOSL IgSF domain fusion proteins) is in an amount of at or about 0.3 mg/kg, at or about 1 mg/kg, at or about 3 mg/kg or at or about 6 mg/kg. In some embodiments, an administered dose of the pharmaceutical composition (including pharmaceutical composition comprising the variant ICOSL IgSF domain fusion proteins) is in an amount of at or about 10 mg/kg.

In some embodiments, an administered dose of the pharmaceutical composition (including pharmaceutical composition comprising the variant ICOSL IgSF domain fusion proteins) is in an amount of at, about, or at least 0.01, 0.05, 0.1, 0.5, 1, 2.5, 5, 10, 15, 20, 30, 40, or 50 mg. In some embodiments, an administered dose of the pharmaceutical composition (including pharmaceutical composition comprising the variant ICOSL IgSF domain fusion proteins) is in an amount of from at or about 0.01 mg to 10 mg, at or about 0.05 mg to 10 mg, at or about 0.1 mg to 10 mg, at or about 0.5 mg to 10 mg, at or about 1 mg to 10 mg, at or about 1.5 mg to 10 mg, at or about 2 mg to 10 mg, at or about 3 mg to 10 mg, at or about 4 mg to 10 mg, at or about 5 mg to 10 mg, at or about 6 mg to 10 mg, at or about 7 mg to 10 mg, at or about 8 mg to 10 mg, at or about 9 mg to 10 mg. In some embodiments, an administered dose of the pharmaceutical composition (including pharmaceutical composition comprising the variant ICOSL IgSF domain fusion proteins) is in an amount of at, about, or at least 0.5, 1, 2, 3, 4, or 5 mg.

In some embodiments, the dose administered is related directly or indirectly to the volume of the eye. In some embodiments, the dose administered is related directly or indirectly to the surface area of the eye, for example the inner surface area.

In some embodiments, the dose of the pharmaceutical composition (including pharmaceutical composition comprising the variant ICOSL IgSF domain fusion proteins) is administered in a volume that minimally and/or transiently affects intraocular pressure. In some embodiments, the volume administered is determined by the volume of the eye. In some embodiments, the volume administered is or is about 0.05 mL. In some embodiments, the volume administered is or is about 0.1 mL or less. In some embodiments, the volume administered is or is about 0.2 mL or less.

In some embodiments, a dose regimen as described herein is administered to achieve a therapeutically effective amount. In some embodiments, a dose regimen as described herein is administered to achieve a prophylactically effective amount.

It is contemplated that dosing (e.g., multiple doses), can continue until any time as desired by a skilled practitioner. For example, dosing may continue until a desirable disease response is achieved, such as in remission or partial remission of the disease and/or a reduction or amelioration in signs and/or symptoms of a disease, such as a reduction of one or more signs of inflammation in the subject. In some embodiments, the dosing is continued following remission or partial remission of the disease and/or a reduction or amelioration in signs and/or symptoms of a disease, such as a reduction of one or more signs of inflammation in the subject.

In some embodiments of the methods, the treating can result in a clinical remission. In some aspects, the treating can result in a clinical remission without about 1 week, about 2 weeks, about 3 weeks, about 4 weeks, about 5 weeks, about 6 weeks, about 7 weeks, about 9 weeks, about 10 weeks, about 11 weeks, about 12 weeks, about 14 weeks, about 16 weeks, about 18 weeks, about 20 weeks, about 22 weeks, about 24 weeks, about 26 weeks, about 28 weeks, about 30 weeks, about 32 weeks, about 34 weeks, about 36 weeks, about 38 weeks, about 40 weeks, about 42 weeks, about 44 weeks, about 46 weeks, about 48 weeks, about 50 weeks, about 52 weeks, about 54 weeks, about 56 weeks, about 58 weeks, about 60 weeks, about 62 weeks, about 64 weeks, about 66 weeks, about 68 weeks, about 70 weeks, about 72 weeks, about 74 weeks, about 76 weeks, about 78 weeks, or about 80 weeks from the first dose. In some embodiments, the treating results in a clinical remission within about 10 weeks from the first dose. In some embodiments, the treating results in a clinical remission within about 6 weeks from the first dose. In some embodiments, the treating results in a clinical remission at about 6 weeks from the first dose and at about 10 weeks from the first dose.

In some embodiments of any of the preceding methods, the clinical remission is a sustained remission. For example, in some embodiments, the sustained remission is a clinical remission at about 10 weeks, about 15 weeks, about 20 weeks, about 25 weeks, about 30 weeks, about 35 weeks, about 40 weeks, about 45 weeks, about 50 weeks, about 52 weeks, about 55 weeks, about 60 weeks, about 65 weeks, about 70 weeks, about 72 weeks, about 75 weeks, about 80 weeks, about 85 weeks, about 90 weeks, about 95 weeks, about 100 weeks, about 102 weeks, about 105 weeks, or about 110 weeks from the first dose. In some embodiments, the sustained remission is a clinical remission at about ten weeks from the first dose and at about 30 weeks from the first dose. In some embodiments, the sustained remission has a length of at least about 30 weeks, or at least about 7, about 8, about 9, about 10, about 11, or about 12 months. In some embodiments of any of the preceding aspects, the amelioration of one or more symptoms of the disease or condition, clinical remission, and/or clinical response is maintained at least one month (e.g., at least one month, at least two months, at least three months, at least four months, at least five months, at least six months, at least seven months, at least eight months, at least nine months, at least ten months, at least eleven months, at least twelve months, or longer) after the end of treatment.

### B. Determining or Predicting Effectiveness of Treatment

In some embodiments, the method includes assaying a subject with a disease or condition to predict a response and or outcome. Particular embodiments contemplate that the expression of one or more gene products, e.g., a gene expression profile, of a sample is predictive of, correlated with, and/or associated with effectiveness of response to the variant ICOSL polypeptides (e.g. variant ICOSL IgV-Fc).

In some embodiments, the method includes assessment of one or more biomarkers associated with activity of the provided variant ICOSL polypeptides (e.g. variant ICOSL IgV-Fc) to inhibit or reduce activity of ICOS and CD28. In some embodiments, the one or more biomarkers is gene product that is CCL17, CXCR5, GATA3, IL12RB2, IL18R1, IL18RAP, KITLG, TTA, PVR, SOCS3, TFRC, TNFRSF8, BATF3, CTLA4, FLT1, FOSL1, IL12, IL17F, IL1R2, IL23R, IL4, IL5 or VEGFA, CCL20, CD40LG, CSF2, ICOS, ID2, IFNG, IGSF3, IL17A, IL1R1, IL2, IL21, IL24, IL3, IL31, IL6, IL9, LIF, NFKBID, PDCD1 or TNF, or a combination of any of the foregoing. In some embodiments, the one or more biomarkers is gene product that is CCL17, CXCR5, GATA3, IL12RB2, IL18R1, IL18RAP, KITLG, TTA, PVR, SOCS3, TFRC or TNFRSF8, or a combination of any of the foregoing. In some embodiments, the one or more biomarkers is a gene product that is BATF3, CTLA4, FLT1, FOSL1, IL12, IL17F, IL1R2, IL23R, IL4, IL5 or VEGFA, or a combination of any of the foregoing. In some embodiments, the one or more biomarkers is a gene product that is CCL20, CD40LG, CSF2, ICOS, ID2, IFNG, IGSF3, IL17A, IL1R1, IL2, IL21, IL24, IL3, IL31, IL6, IL9, LIF, NFKBID, PDCD1 or TNF, or a combination of any of the foregoing. In some of any of the embodiments, the biomarker is a protein. In some embodiments, the biomarker is a nucleic acid, such as RNA, for example mRNA.

In some embodiments, 1, 2, 3, 4, 5, 6, 7, 8, 9, 10, 11, 12, 13, 14, 15, 16, 17, 18, 19, 20, 21, 22, 23, 34, 25 or more of any of the above gene products are assessed, measured, detected, and/or quantified. In some embodiments, provided herein are panels, profiles, and/or arrays for use in the measurement, assessment, and/or determination of one any one or more of the above gene products in a sample. In particular embodiments, the panels, profiles, and/or arrays include the measurements, assessments, and/or quantifications of at least one, two, three, four, five, six, seven, eight, nine, ten, more than ten, or more than twenty gene products.

In certain embodiments, the expression of one or more biomarkers or genes are measured, assessed, and/or determined in a sample. In some embodiments, a gene product is any biomolecule that is assembled, generated, and/or synthesized with information encoded by a gene, and may include polynucleotides and/or polypeptides. In particular embodiments, assessing, measuring, and/or determining gene expression is or includes determining or measuring the level, amount, or concentration of the gene product. In certain embodiments, the level, amount, or concentration of the gene product may be transformed (e.g., normalized) or directly analyzed (e.g., raw). In some embodiments, the gene product is a protein that is encoded by the gene. In certain embodiments, the gene product is a polynucleotide, e.g., an mRNA or a protein, that is encoded by the gene.

In some embodiments, the gene product is a polynucleotide that is expressed by and/or encoded by the gene. In certain embodiments, the polynucleotide is an RNA. In some embodiments, the gene product is a messenger RNA (mRNA), a transfer RNA (tRNA), a ribosomal RNA, a small nuclear RNA, a small nucleolar RNA, an antisense RNA, long noncoding RNA, a microRNA, a Piwi-interacting RNA, a small interfering RNA, and/or a short hairpin RNA. In particular embodiments, the gene product is an mRNA.

In particular embodiments, the amount or level of a polynucleotide in a sample may be assessed, measured, determined, and/or quantified by any suitable means known in the art. For example, in some embodiments, the amount or level of a polynucleotide gene product can be assessed, measured, determined, and/or quantified by polymerase chain reaction (PCR), including reverse transcriptase (rt) PCR, droplet digital PCR, real-time and quantitative PCR (qPCR) methods (including, e.g., TAQMAN®, molecular beacon, LIGHTUP™, SCORPION™, SIMPLEPROBES®; see, e.g., U.S. Pat. Nos.5,538,848; 5,925,517; 6,174,670; 6,329,144; 6,326,145 and 6,635,427); northern blotting; Southern blotting, e.g., of reverse transcription products and derivatives; array based methods, including blotted arrays, microarrays, or in situ-synthesized arrays; and sequencing, e.g., sequencing by synthesis, pyrosequencing, dideoxy sequencing, or sequencing by ligation, or any other methods known in the art, such as discussed in Shendure et al., Nat. Rev. Genet. 5:335-44 (2004) or Nowrousian, Euk. Cell 9(9): 1300-1310 (2010), including such specific platforms as HELICOS®, ROCHE® 454, ILLUMINA®/SOLEXA®, ABI SOLiD®, and POLONATOR® sequencing. In particular embodiments, the levels of nucleic acid gene products are measured by quantitative PCR (qPCR) methods, such qRT-PCR. In some embodiments, the qRT-PCR uses three nucleic acid sets for each gene, where the three nucleic acids comprise a primer pair together with a probe that binds between the regions of a target nucleic acid where the primers bind- known commercially as a TAQMAN® assay.

In particular embodiments, assessing, measuring, determining, and/or quantifying amount or level of an RNA gene product includes a step of generating, polymerizing, and/or deriving a cDNA polynucleotide and/or a cDNA oligonucleotide from the RNA gene product. In certain embodiments, the RNA gene product is assessed, measured, determined, and/or quantified by directly assessing, measuring, determining, and/or quantifying a cDNA polynucleotide and/or a cDNA oligonucleotide that is derived from the RNA gene product.

In some embodiments, the expression of one or more gene products, e.g., polynucleotide gene products, is determined by sequencing the gene product and/or by sequencing a cDNA polynucleotide that is derived from the from the gene product. In some embodiments, the sequencing is performed by a non-Sanger sequencing method and/or a next generation sequencing (NGS) technique. Examples of Next Generation Sequencing techniques include, but are not limited to Massively Parallel Signature Sequencing (MPSS), Polony sequencing, pyrosequencing, Reversible dye-terminator sequencing, SOLiD sequencing, Ion semiconductor sequencing, DNA nanoball sequencing, Helioscope single molecule sequencing, Single molecule real time (SMRT) sequencing, Single molecule real time (RNAP) sequencing, and Nanopore DNA sequencing.

In some embodiments, the NGS technique is RNA sequencing (RNA-Seq). In particular embodiments, the expression of the one or more polynucleotide gene products is measured, determined, and/or quantified by RNA-Seq. RNA-Seq, also called whole transcriptome shotgun sequencing determines the presence and quantity of RNA in a sample. RNA sequencing methods have been adapted for the most common DNA sequencing platforms [HiSeq systems (Illumina), 454 Genome Sequencer FLX System (Roche), Applied Biosystems SOLiD (Life Technologies), IonTorrent (Life Technologies)]. These platforms require initial reverse transcription of RNA into cDNA. Conversely, the single molecule sequencer HeliScope (Helicos BioSciences) is able to use RNA as a template for sequencing. A proof of principle for direct RNA sequencing on the PacBio RS platform has also been demonstrated (Pacific Bioscience). In some embodiments, the one or more RNA gene products are assessed, measured, determined, and/or quantified by RNA-seq.

In some embodiments, the gene product is or includes a protein, i.e., a polypeptide, that is encoded by and/or expressed by the gene. In particular embodiments, the gene product encodes a protein that is localized and/or exposed on the surface of a cell. In some embodiments, the protein is a soluble protein. In certain embodiments, the protein is secreted by a cell.

In particular embodiments, the gene expression is the amount, level, and/or concentration of a protein that is encoded by the gene. In certain embodiments, one or more protein gene products are measured by any suitable means known in the art. Suitable methods for assessing, measuring, determining, and/or quantifying the level, amount, or concentration or more or more protein gene products include, but are not limited to detection with immunoassays, nucleic acid-based or protein-based aptamer techniques, HPLC (high precision liquid chromatography), peptide sequencing (such as Edman degradation sequencing or mass spectrometry (such as MS/MS), optionally coupled to HPLC), and microarray adaptations of any of the foregoing (including nucleic acid, antibody or protein-protein (i.e., non- antibody) arrays). In some embodiments, the immunoassay is or includes methods or assays that detect proteins based on an immunological reaction, e.g., by detecting the binding of an antibody or antigen binding antibody fragment to a gene product. Immunoassays include, but are not limited to, quantitative immunocytochemisty or immunohistochemisty, ELISA (including direct, indirect, sandwich, competitive, multiple and portable ELISAs (see, e.g., U.S. Patent No. 7,510,687), western blotting (including one, two or higher dimensional blotting or other chromatographic means, optionally including peptide sequencing), enzyme immunoassay (EIA), RIA (radioimmunoassay), and SPR (surface plasmon resonance).

In provided embodiments, the sample is a biological sample that is taken, collected, and/or obtained from a subject that has received, will receive, or is a candidate to receive administration of the variant ICOSL polypeptides (e.g. variant ICOSL IgV-Fc). In some embodiments, the subject has received or been administered the variant ICOSL polypeptides (e.g. variant ICOSL IgV-Fc). In particular embodiments, the sample is a blood or serum sample. In particular embodiments, the sample is a blood sample. In particular embodiments, the sample is taken, collected, and/or obtained prior to treatment or administration with the therapy. In some embodiments, the sample is taken, collected, and/or obtained after treatment or administration with the therapy. In some embodiments, the sample is collected within or about within or about 1 hours, 2 hours, 6 hours, 12 hours, 24 hours, 2 days, 3 days, 4 days, 5 days, 6 days, 7 days, 2 weeks, 3 weeks, one month or more following initiation of administration of the variant ICOSL polypeptides (e.g. variant ICOSL IgV-Fc).

In some embodiments, the method includes assessment of the level, amount or concentration of one or more gene products in the sample to threshold value of the gene product. In some embodiments, the threshold value is the level, amount or concentration of the gene product in a healthy subject, or is the median or mean level, amount or concentration of the gene product in a plurality of healthy subject, in which such healthy subjects that have not been treated. In some embodiments, the threshold value is the level, amount or concentration of the gene product in a diseased subject, or is the median or mean level, amount or concentration of the gene product in a plurality of diseased subject, such as subjects having the same disease but in which such subjects have not been treated. In some embodiments, a reduction in the level, amount or concentration of the gene product compared to the threshold value indicates or predicts that the treatment with the variant ICOSL polypeptides (e.g. variant ICOSL IgV-Fc) is effective.

In some embodiments, the assessment of the level, amount or concentration of the gene product is compared to a baseline value. In some embodiments, the baseline value is derived from the same patient 1 day, 2 days, 3 days, 4 days, or 5 days prior to treatment. In some embodiments, the baseline value is derived from the same patient 1 hour, 2 hours, 3 hours, 4, hours, 5 hours, 6 hours, 7 hours, 8 hours, 9 hours, 10 hours, 11 hours, 12 hours or 24 hours prior to initiation of treatment. In some embodiments, a reduction in the level, amount or concentration of the gene product compared to the baseline value indicates or predicts that the treatment with the variant ICOSL polypeptides (e.g. variant ICOSL IgV-Fc) is effective.

In some embodiments, the response is predicted to be effective if it is determined there is a 2 fold, 3 fold, 4 fold, 5 fold, 6 fold, 7 fold, 8 fold, 9 fold, 10 fold or more than 10 fold reduction in gene product. In some embodiments, the response is predicted to be effective if it is determined there is a 2 fold, 3 fold, 4 fold, 5 fold, 6 fold, 7 fold, 8 fold, 9 fold, 10 fold or more than 10 fold reduction in gene expression.

## VIII. EXEMPLARY EMBODIMENTS

Among the provided embodiments are:

1. A method of treating an autoimmune or inflammatory disease or condition in a subject, the method comprising administering to a subject having an autoimmune or inflammatory disease or condition one or more doses of a variant ICOSL fusion protein in a treatment period, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1, wherein each dose of the variant ICOSL fusion protein is administered in an amount from at or about 0.001 mg/kg to at or about 20 mg/kg.
2. The method of embodiment 1, wherein each dose is administered in an amount from or from about 0.001 mg/kg to at or about 20 mg/kg, from about 0.001 mg/kg to at or about 10 mg/kg, from about 0.001 mg/kg to at or about 6 mg/kg, from about 0.001 mg/kg to at or about 3 mg/kg, from about 0.001 mg/kg to at or about 1 mg/kg, from about 0.001 mg/kg to at or about 0.1 mg/kg, from about 0.1 mg/kg to at or about 20 mg/kg, from or from about 0.1 mg/kg to at or about 10 mg/kg, from or from about 0.1 mg/kg to at or about 6 mg/kg, from about 0.1 mg/kg to at or about 3 mg/kg, from about 0.1 mg/kg to at or about 1 mg/kg, from about 1 mg/kg to at or about 20 mg/kg, from or from about 1 mg/kg to at or about 10 mg/kg, from or from about 1 mg/kg to at or about 6 mg/kg, from about 1 mg/kg to at or about 3 mg/kg, from about 3 mg/kg to at or about 20 mg/kg, from or from about 3 mg/kg to at or about 10 mg/kg, from or from about 3 mg/kg to at or about 6 mg/kg, from about 6 mg/kg to at or about 20 mg/kg, from or from about 6 mg/kg to at or about 10 mg/kg, or from or from about 10 mg/kg to at or about 20 mg/kg.
3. The method of embodiment 1 or embodiment 2, wherein each dose is administered in an amount from at or about 0.001 mg/kg to at or about 10 mg/kg.
4. The method of any one of embodiments 1-3, wherein each dose is administered in an amount from or from about 0.1 mg/kg to at or about 10 mg/kg.
5. The method of any one of embodiments 1-4, wherein only a single dose of the variant ICOSL fusion protein is administered in a treatment period.
6. The method of any one of embodiments 1-4, wherein a multiple number of doses of the variant ICOSL fusion protein is administered in a treatment period, optionally wherein the multiple number of doses is 2, 3, 4 or 5 doses.
7. The method of any one of embodiments 1-6, wherein the treatment period is at least 20 days
8. The method of any one of embodiments 1-7, wherein the treatment period is 20-40 days.
9. A method of treating an autoimmune or inflammatory disease or condition in a subject, the method comprising administering to a subject having an autoimmune or inflammatory disease or condition a variant ICOSL fusion protein, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1,
wherein the variant ICOSL fusion protein is administered in a multiple number of doses selected from 3, 4 and 5 within a treatment period of 20-40 days, wherein each dose is administered in an amount from at or about 0.1 mg/kg to at or about 10 mg/kg.

10. A method of treating an autoimmune or inflammatory disease or condition in a subject, the method comprising administering to a subject having an autoimmune or inflammatory disease or condition a variant ICOSL fusion protein, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference polypeptide corresponding to amino acid substitutions selected from N52H, N57Y and Q100R with reference to the sequence set forth in SEQ ID NO:1,
wherein the variant ICOSL fusion protein is administered in a multiple number of doses within a treatment period of at least 20 days, wherein each dose is administered in an amount from at or about 0.1 mg/kg to at or about 10 mg/kg, optionally for a maximum treatment period of 30 days, and further optionally wherein at least 5 days separate each of the multiple doses from one another.

11. The method of any one of embodiments 1-10, wherein the treatment period is at or about 21 days.

12. The method of any one of embodiments 1-10, wherein the treatment period is at or about 28 days.

13. The method of any one of embodiments 6-12, wherein each of the multiple number of doses is administered no more than once weekly.

14. A method of treating an autoimmune or inflammatory disease or condition in a subject, the method comprising administering to a subject having an autoimmune or inflammatory disease or condition a variant ICOSL fusion protein, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference polypeptide corresponding to amino acid substitutions selected from N52H, N57Y and Q100R with reference to the sequence set forth in SEQ ID NO:1,
wherein the variant ICOSL fusion protein is administered in a multiple number of doses within a treatment period of at least 4 weeks, wherein each dose is administered in an amount from at or about 0.1 mg/kg to at or about 10 mg/kg and is administered no more than once weekly.

15. The method of embodiment 14, wherein the treatment period is at or about 4 weeks.

16. The method of any one of embodiments 6-15, wherein each of the multiple number of doses is administered once a week (Q1W).

17. A method of preventing or reducing acute graft versus host disease (aGVHD), the method comprising administering to the subject one or more doses of a variant ICOSL fusion protein in a treatment period, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1, wherein the dose of the variant ICOSL fusion protein is administered in an amount from at or about 0.001 mg/kg to at or about 20 mg/kg.

18. The method of embodiment 17, wherein the aGVHD is associated with a stem cell transplantation and at least one dose of the ICOSL fusion protein is administered to the subject prior to the subject receiving the stem cell transplantation.

19. The method of embodiment 17 or embodiment 18, wherein the aGVHD is associated with a stem cell transplantation and at least one dose of the ICOSL fusion protein is administered to the subject prior to the subject receiving the stem cell transplantation and at least one dose of the ICOSL fusion protein is given to the subject concurrent with or subsequent to the subject receiving the stem cell transplantation.

20. The method of any one of embodiments 17-19, wherein the aGVHD is associated with a stem cell transplantation and at least one dose of the ICOSL fusion protein is administered to the subject prior to the subject receiving the stem cell transplantation and at least one dose of the ICOSL fusion protein is given to the subject concurrent with the subject receiving the stem cell transplantation.

21. The method of any one of embodiments 17-19, wherein the aGVHD is associated with a stem cell transplantation and at least one dose of the ICOSL fusion protein is administered to the subject prior to the subject receiving the stem cell transplantation and at least one dose of the ICOSL fusion protein is given to the subject subsequent to the subject receiving the stem cell transplantation.

22. A method of treating graft versus host disease (GVHD) in a subject, the method comprising administering to the subject a single dose of a variant ICOSL fusion protein, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1, wherein the single dose of the variant ICOSL fusion protein is administered in an amount from at or about 0.001 mg/kg to at or about 20 mg/kg.

23. A method of treating graft versus host disease (GVHD) in a subject, the method comprising administering to the subject a single dose of a variant ICOSL fusion protein, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1, wherein the single dose of the variant ICOSL fusion protein is administered in an amount from at or about 0.001 mg/kg to at or about 20 mg/kg, and wherein the subject is resistant or refractory to an immunosuppressant, optionally a corticosteroid and/or cyclosporine.

24. The method of embodiment 22 or embodiment 23, wherein the GVHD is an acute GVHD (aGVHD).

25. The method of embodiment 22 or embodiment 23, wherein the GVHD is a chronic GVHD.

26. The method of any one of embodiments 1-25, wherein each dose is an amount from at or about 0.3 mg/kg to at or about 10 mg/kg, at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 10 mg/kg, at or about 1 mg/kg to at or about 6 mg/kg, at or about 1 mg/kg to at or about 3 mg/kg, at or about 3 mg/kg to at or about 10mg/kg, at or about 3 mg/kg to at or about 6 mg/kg, or at or about 6 mg/kg to at or about 10 mg/kg.

27. The method of any one of embodiments 1-26, wherein each dose is in an amount from at or about 0.3 mg/kg to at or about 6 mg/kg, at or about 0.3 mg/kg to at or about 3 mg/kg, at or about 0.3 mg/kg to at or about 1 mg/kg,at or about 1 mg/kg to at or about 6 mg/kg, at or about 1 mg/kg to at or about 3 mg/kg, or at or about 3 mg/kg to at or about 6 mg/kg.

28. The method of any one of embodiments 1-27, wherein each dose is administered in an amount of or of about 0.3 mg/kg.

29. The method of any one of embodiments 1-27, wherein each dose is administered in an amount of or of about 1 mg/kg.

30. The method of any one of embodiments 1-27, wherein each dose is administered in an amount of or of about 3 mg/kg.

31. The method of any one of embodiments 1-27, wherein each dose is administered in an amount of or of about 6 mg/kg.

32. The method of any one of embodiments 1-27, wherein each dose is administered in an amount of or of about 10 mg/kg.

33. The method of any one of embodiments 1-27, wherein at least one dose is administered in an amount of or about 0.3 mg/kg.

34. The method of any one of embodiments 1-27, wherein at least one dose is administered in an amount of or about 1 mg/kg.

35. The method of any one of embodiments 1-7, wherein at least one dose is administered in an amount of or about 3 mg/kg.

36. The method of any one of embodiments 1-27, wherein at least one dose is administered in an amount of or about 6 mg/kg.

37. The method of any one of embodiments 1-27, wherein at least one dose is administered in an amount of or about 10 mg/kg.

38. The method of any one of embodiments 1-37, wherein at least one dose is administered via subcutaneous administration.

39. The method of any one of embodiments 1-37, wherein at least one dose is administered via intravenous administration.

40. The method of any one of embodiments 1-37, wherein the administration is via subcutaneous administration.

41. The method of any one of embodiments 1-37, wherein the administration is via intravenous administration.

42. The method of any one of embodiments 1-16, wherein the autoimmune or inflammatory disease is an ocular autoimmune or inflammatory condition.

43. The method of embodiment 42, wherein the administration is via intravitreally administration.

44. The method of any one of embodiments 1-43, wherein the treatment period is repeated, optionally wherein the treatment period is repeated until remission is achieved, until partial remission is achieved or until the disease or condition does not progress in the subject.

45. A method of treating an ocular autoimmune or inflammatory disease in a subject, the method comprising administering intravitreally a dose of a variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide with reference to the sequence set forth in SEQ ID NO:1.

46. A method of reducing or preventing an ocular autoimmune or inflammatory disease in a subject, the method comprising administering intravitreally a dose of a variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide with reference to the sequence set forth in SEQ ID NO:1.

47. The method of embodiment 42 or 44, wherein the ocular autoimmune or inflammatory disease is uveitis.

48. The method of any of embodiments 42-47, wherein the ocular autoimmune or inflammatory disease is caused by a systemic autoimmune of inflammatory disease.

49. The method of embodiment 48, where in the systemic autoimmune or inflammatory disease is ankylosing spondylitis, Crohn's disease, ulcerative colitis, rheumatoid arthritis, juvenile rheumatoid arthritis, reactive arthritis, enteropathic arthritis, psoriatic arthritis, systemic lupus erythematosus, Bechet's syndrome, dermatomyositis, or Granulomatosis with Polyangiitis.

50. The method of any of embodiments 42-47, wherein the ocular autoimmune or inflammatory disease is an eye-specific autoimmune or inflammatory disease.

51. The method of any of embodiments 42-47 and 50, wherein the eye-specific autoimmune or inflammatory disease is ocular cicatricial pemphigoid, Mooren's corneal ulcer, or uveitis.

52. The method of any of embodiments 42-51, wherein the ICOSL fusion protein is administered at a dose of at least 0.01, 0.05, 0.1, 0.5, 1, 2.5, 5, 10, 15, 20, 30, 40, or 50 mg, optionally 1, 2, 3, 4, or 5 mg.

53. The method of any of embodiments 42-51, wherein the ICOSL fusion protein is administered at a dose of between about 0.01 mg to 10 mg, at or about 0.05 mg to 10 mg, at or about 0.1 mg to 10 mg, at or about 0.5 mg to 10 mg, at or about 1 mg to 10 mg, at or about 1.5 mg to 10 mg, at or about 2 mg to 10 mg, at or about 3 mg to 10 mg, at or about 4 mg to 10 mg, at or about 5 mg to 10 mg, at or about 6 mg to 10 mg, at or about 7 mg to 10 mg, at or about 8 mg to 10 mg, at or about 9 mg to 10 mg, inclusive.

54. The method of any of embodiments 42-53, wherein the ICOSL fusion protein is administered in a volume less than 0.2 mL, optionally less than 0.1 mL.

55. The method of any of embodiments 42-54, wherein the ICOSL fusion protein is administered in a volume about 0.05 mL.

56. The method of any one of embodiments 1-55, wherein the variant ICOSL polypeptide comprises the amino acid substitutions N52H/N57Y/Q100R, with reference to numbering of SEQ ID NO:1.

57. The method of any one of embodiments 1-56, wherein the variant ICOSL polypeptide exhibits increased binding affinity to the ectodomain(s) of CD28 compared to the binding of the ICOSL reference polypeptide for the same ectodomain(s).

58. The method of any one of embodiments 1-57, wherein the variant ICOSL polypeptide exhibits binding affinity to the ectodomain(s) of ICOS that is substantially the same or that is increased compared to the binding of the ICOSL reference polypeptide for the same ectodomain(s), optionally wherein the binding affinity is from at or about 80% or greater of the binding affinity of the ICOSL reference polypeptide for the same ectodomain(s).

59. The method of any one of embodiments 1-58, wherein the variant ICOSL polypeptide exhibits increased binding to the ectodomain(s) of ICOS and CD28 compared to the binding of the ICOSL reference polypeptide for the same ectodomain(s).

60. The method of embodiment 58 or embodiment 59, wherein the binding is increased more than at or about 1.2-fold, 1.5-fold, 2-fold, 3-fold, 4-fold, 5-fold, 6-fold, 7-fold, 8-fold, 9-fold, 10-fold, 20-fold, 30-fold, 40-fold, 50-fold or 60-fold.

61. The method of any one of embodiments 1-60, wherein:

each dose of the variant ICOSL fusion protein achieves, or is predicted to achieve, a target binding saturation for CD28 of greater than at or about 25% at $C_{max}$; and/or
each dose of the variant ICOSL fusion protein achieves, or is predicted to achieve, a target binding saturation for CD28 of greater than at or about 10% within 24 hours of the administration of the dose.

62. The method of any one of embodiments 1-61, wherein each dose of the variant ICOSL fusion protein achieves, or is predicted to achieve, a target binding saturation for CD28 of greater than at or about 85% within 24 hours, within 2 days or within 7 days of the administration of the dose.

63. The method of any one of embodiments 1-62, wherein each dose of the variant ICOSL fusion protein achieves, or is predicted to achieve, a target binding saturation for CD28 of greater than at or about 90% within 24 hours, within 2 days or within 7 days of the administration of the dose.

64. The method of any one of embodiments 1-63, wherein each dose of the variant ICOSL fusion protein achieves, or is predicted to achieve, a target binding saturation for CD28 of greater than at or about 95% within 24 hours, within 2 days or within 7 days of the administration of the dose.

65. The method of any one of embodiments 1-64, wherein each dose of the variant ICOSL fusion protein achieves, or is predicted to achieve, a target binding saturation for CD28 of greater than at or about 10% within 21 days or 28 days of the administration of the dose.

66. The method of any one of embodiments 1-65, wherein each dose of the variant ICOSL fusion protein achieves, or is predicted to achieve, a target binding saturation for CD28 of greater than at or about 20% within 21 days or 28 days of the administration of the dose.

67. The method of any one of embodiments 57-66, wherein the CD28 is a human CD28.

68. The method of any one of embodiments 58-67, wherein the ICOS is a human ICOS.

69. The method of any one of embodiments 1-68, wherein the ICOSL reference polypeptide comprises (i) the sequence of amino acids set forth in SEQ ID NO:32, (ii) a sequence of amino acids that has at least 95% sequence identity to SEQ ID NO:32; or (iii) a portion of (i) and/or (ii) comprising an IgV domain or an IgC domain or specific binding fragments thereof.

70. The method of any one of embodiments 1-69, wherein the variant ICOSL polypeptide comprises the IgV domain or a specific binding fragment thereof.

71. The method of any one of embodiments 1-70, wherein the IgV domain or specific binding fragment thereof is the only ICOSL portion of the variant ICOSL polypeptide or of the variant ICOSL fusion protein.

72. The method of any one of embodiments 1-71, wherein the ICOSL reference polypeptide is a truncated ICOSL extracellular domain comprising a contiguous sequence of amino acids comprising amino acids 1-112 and a C-terminal truncation of at least 25 amino acids with reference to the ICOSL extracellular domain sequence set forth in SEQ ID NO:1.

73. The method of embodiment 72, wherein the C-terminal truncation is of at least 30, at least 40, at least 50, at least 60, at least 70, at least 80, at least 90, at least 100, or at least 125 amino acid residues.

74. The method of any one of embodiments 1-73, wherein the ICOSL reference polypeptide is altered in or lacks a protease cleavage site set forth as amino acids 204-209 of SEQ ID NO:1.

75. The method of any one of embodiments 1-74, wherein the ICOSL reference polypeptide comprises the sequence of amino acids set forth in SEQ ID NO:3.

76. The method of any one of embodiments 1-75, wherein the ICOSL reference polypeptide consists of the sequence of amino acids set forth in SEQ ID NO:3.

77. The method of any one of embodiments 1-76, wherein the variant ICOSL polypeptide has the sequence set forth in SEQ ID NO:36 or a sequence that exhibits at least at or about 90%, at least at or about 91%, at least at or about 92%, at least at or about 93%, at least at or about 94%, at least at or about 95%, at least at or about 96%, at least at or about 97%, at least at or about 98%, or at least at or about 99% sequence identity to the sequence set forth in SEQ ID NO:36 and comprises the one or more amino acid substitutions selected from N52H, N57Y and Q 100R.

78. The method of any one of embodiments 1-77, wherein the variant ICOSL polypeptide has the sequence set forth in SEQ ID NO:36.

79. The method of any one of embodiments 1-78, wherein the variant ICOSL polypeptide exhibits reduced proteolytic cleavage when expressed from a cell compared to a full-length extracellular domain of the variant ICOSL polypeptide when expressed from the same cell, optionally wherein the cell is a Chinese Hamster Ovary (CHO) cell.

80. The method of any one of embodiments 1-79, wherein the multimerization domain is or comprises an Fc region of an immunoglobulin.

81. The method of any one of embodiments 1-80, wherein the variant ICOSL polypeptide is linked via a linker to the multimerization domain, optionally wherein the linker is a peptide linker.

82. The method of embodiment 81, wherein the linker comprises 1 to 10 amino acids.

83. The method of embodiment 81 or embodiment 82, wherein the linker is AAA.

84. The method of embodiment 81 or embodiment 82, wherein the linker is G4S (SEQ ID NO:52).

85. The method of embodiment 81 or embodiment 82, wherein the linker is (G4S)2 (SEQ ID NO:53).

86. The method of embodiment 81 or embodiment 82, wherein the linker is GSGGGGS linker (SEQ ID NO: 58).

87. The method of any one of embodiments 1-86, wherein the variant ICOSL fusion protein is a multimer comprising a first variant ICOSL polypeptide linked to a first multimerization domain and a second variant ICOSL polypeptide linked to a second multimerization domain, optionally wherein the first and second multimerization domain are the same, optionally wherein the first and second multimerization domain are an Fc region of an immunoglobulin.

88. The method of embodiment 87, wherein the multimer is a dimer.

89. The method of embodiment 88, wherein the dimer is a homodimer.

90. The method of any one of embodiments 80-89, wherein the Fc region is a variant Fc region that exhibits reduced effector function compared to an Fc of a wildtype human immunoglobulin.

91. The method of any one of embodiments 80-90, wherein the Fc region is a variant IgG1 Fc region comprising one or more amino acid substitutions compared to a wildtype human IgG1.

92. The method of any one of embodiments 80-91, wherein the Fc region comprises a sequence that exhibits at least 90%, 91%, 92%, 93%, 94%, 95%, 96%, 97%, 98% or 99% sequence identity to SEQ ID NO:37.

93. The method of any one of embodiments 80-92, wherein the variant Fc region comprises one or more amino acid substitutions selected from N297G, E233P/L234V/L235A/G236del/S267K or L234A/L235E/G237A, wherein the residue is numbered according to the EU index of Kabat.

94. The method of embodiment 93, wherein the variant Fc region further comprises the amino acid substitution C220S, wherein the residues are numbered according to the EU index of Kabat.

95. The method of embodiment 93 or embodiment 94, wherein the Fc region comprises K447del, wherein the residue is numbered according to the EU index of Kabat.

96. The method of any one of embodiments 93-95, wherein the Fc region comprises the sequence of amino acids set forth in SEQ ID NO:43 or SEQ ID NO:46.

97. The method of anyone one of embodiments 93-95, wherein the Fc region comprises the sequence of amino acids set forth in SEQ ID NO:44 or SEQ ID NO:47.

98. The method of any one of embodiments 93-95, wherein the Fc region comprises the sequence of amino acids set forth in SEQ ID NO:40.

99. The method of any one of embodiments 93-95, wherein the Fc region comprises the sequence of amino acids set forth in SEQ ID NO:39.

100. The method of any one of embodiments 93-95, wherein the Fc region comprises the sequence of amino acids set forth in SEQ ID NO:41.

101. The method of any one of embodiments 93-95, wherein the Fc region comprises the sequence of amino acids set forth in SEQ ID NO:42.

102. The method of any one of embodiments 1-101, wherein the variant ICOSL fusion protein decreases an immune response in the subject.

103. The method of any one of embodiments 1-16, 26-41, and 56-102, wherein the inflammatory or autoimmune disease or condition is an Antineutrophil cytoplasmic antibodies (ANCA)-associated vasculitis, a vasculitis, an autoimmune skin disease, transplantation, a Rheumatic disease, an inflammatory gastrointestinal disease, an inflammatory eye disease, an inflammatory neurological disease, an inflammatory pulmonary disease, an inflammatory endocrine disease, or an autoimmune hematological disease.

104. The method of any one of embodiments 1-16, 26-41, and 56-103, wherein the inflammatory or autoimmune disease or condition is an inflammatory bowel disease, optionally Crohn's disease or ulcerative colitis; transplantation; multiple sclerosis; asthma; rheumatoid arthritis; psoriatic arthritis or psoriasis.

105. The method of embodiment 104, wherein the inflammatory bowel disease is a chronic inflammatory bowel disease.

106. The method of any one of embodiments 1-16, 26-41, and 56-104, wherein the inflammatory or autoimmune disease or condition is psoriatic arthritis.

107. The method of any one of embodiments 1-16, 26-41, and 56-104, wherein the inflammatory or autoimmune disease or condition is rheumatoid arthritis.

108. The method of any one of embodiments 1-16, 26-41, and 56-105, wherein the inflammatory or autoimmune disease or condition is Crohn's disease.

109. The method of any one of embodiments 1-16, 26-41, and 56-105, wherein the inflammatory or autoimmune disease or condition is ulcerative colitis.

110. The method of any one of embodiments 1-15, 26-41, and 56-102, wherein the inflammatory or autoimmune disease or condition is an ocular inflammatory disease, optionally uveitis.

111. The method of any one of embodiments 1-16, 26-41, and 56-103, wherein the inflammatory or autoimmune disease or condition is systemic lupus erythematosus (SLE).

112. The method of any one of embodiments 1-16, 26-41, and 56-103, wherein the inflammatory or autoimmune disease or condition is Sjögren's Syndrome.

113. The method of any one of embodiments 1-16, 26-41, and 56-103, wherein the inflammatory or autoimmune disease or condition is Graft Versus Host Disease (GVHD).

114. The method of embodiment 113, wherein the GVHD is an acute GVHD (aGVHD).

115. The method of embodiment 113, wherein the GVHD is a chronic GVHD.

116. The method of any one of embodiments 113-115, wherein the GVHD is resistant or refractory to an immunosuppressant, optionally a corticosteroid and/or cyclosporine.

117. The method of any one of embodiments 1-114, wherein the method of treating comprises prophylactic treatment.

118. The method of any one of embodiments 1-16, 26-103, 116, and 117, wherein at least one dose of the ICOSL fusion protein is administered prior to the onset of the inflammatory or autoimmune disease or condition.

119. The method of any one of embodiments 1-16, and 26-118, wherein at least one dose of the ICOSL fusion protein is administered concurrent with or subsequent to the onset of the inflammatory or autoimmune disease or condition.

120. The method of any one of embodiments 1-16, 26-114, and 116-119, wherein at least one dose of the ICOSL fusion protein is administered prior to the onset of the inflammatory or autoimmune disease or condition and at least one dose of the ICOSL fusion protein is administered concurrent with or subsequent to the onset of the inflammatory or autoimmune disease or condition.

121. The method of any of embodiments 1-120, wherein the subject is a human.

## IX. EXAMPLES

The following examples are included for illustrative purposes only and are not intended to limit the scope of the invention.

## EXAMPLE 1

### Assessment of Binding and Activity of ICOSL IgSF Domain Variants

Variant ICOSL polypeptides with altered binding to binding partners ICOSL, CD28 and/or CTLA-4, were selected. The variant ICOSL polypeptides were selected by screening a degenerate yeast display library generated based on a wildype human ICOSL sequence set forth in SEQ ID NO:1 containing the ECD domain as follows:

DTQEKEVRAMVGSDVELSCACPEGSRFDLNDVYVYWQTSESKTVVTYHIPQNSSLENVDSRY
RNRALMSPAGMLRGDFSLRLFNVTPQDEQKFHCLVLSQSLGFQEVLSVEVTLHVAANFSVPV
VSAPHSPSQDELTFTCTSINGYPRPNVYWINKTDNSLLDQALQNDTVFLNMRGLYDVVSVLRI
ARTPSVNIGCCIENVLLQQNLTVGSQTGNDIGERDKITENPVSTGEKNAAT

Vector DNA encoding mutated library members were introduced into yeast competent cells by electroporation. Yeast cells from the library were thawed and then grown in the presence of induction media (5.4 grams $Na_2HPO_4$, 8.56 grams of $NaH_2PO_4$•$H_20$, 20 grams galactose, 2.0 grams dextrose, 6.7 grams Difco yeast nitrogen base, and 1.6 grams of yeast synthetic drop out media supplement without leucine dissolved in water and sterilized through a 0.22 μm membrane filter device) and the culture was grown for two days at 20°C to induce expression of library proteins on the yeast cell surface. Cells were processed with magnetic beads (New England Biolabs, USA) to reduce non-binders and enrich for ICOSL variants with the ability to bind the exogenous recombinant counter-structure proteins. Target ligand proteins were sourced from R&D Systems (USA) as follows: human rCD28.Fc (i.e., recombinant CD28-Fc fusion protein), rCTLA4.Fc and rICOS.Fc. This was then followed by two to three rounds of flow cytometry sorting using exogenous counter-structure protein staining to enrich the fraction of yeast cells that displays improved binders. Magnetic bead enrichment and selections by flow cytometry are essentially as described in Miller, K.D. Current Protocols in Cytometry 4.7.1-4.7.30, July 2008.

Yeast outputs from the flow cytometric sorts were assayed for higher specific binding affinity. Sort output yeast were expanded and re-induced to express the particular IgSF affinity modified domain variants they encode. This population then was compared to the parental, wild-type yeast strain, or, in some cases, another selected outputs, such as the bead output yeast population, by flow cytometry.

Selected variant ICOSL ECD domains were further formatted as fusion proteins and tested for binding and functional activity as described below. Plasmid DNA from each output cell from flow cytometric sorts was isolated using a yeast plasmid DNA isolation kit (Zymo Research, USA). For Fc fusions, PCR primers with added restriction sites suitable for cloning into the Fc fusion vector were used to batch-amplify from the plasmid DNA preps the coding DNA's for the mutant target ECD's. After restriction digestion, the PCR products were ligated into an appropriate Fc fusion vector followed by chemical transformation into strain *E. coli* XL1 Blue (Agilent, USA) or NEB5alpha (New England Biolabs, USA) as directed by supplier. Aliquots of transformed samples were submitted for DNA sequencing of the ECD insert in order to identify the mutation(s) in all clones. In some instances, resequencing was performed to verify mutations. After analysis of the Genewiz-generated DNA sequencing data, clones of interest were recovered from master plate. Clones of interest were then identified using the following criteria: 1.) identical clone occurs at least two times in the alignment and 2.) a mutation occurs at least two times in the alignment and preferably in distinct clones. Clones that meet at least one of these criteria were clones that have been enriched by the sorting process most likely due to improved binding.

To generate recombinant immunomodulatory proteins that are Fc fusion proteins containing an ECD of ICOSL with at least one affinity-modified domain (e.g. variant ICOSL ECD-Fc), the encoding nucleic acid molecule was generated to encode a protein designed as follows: signal peptide followed by variant (mutant) ICOSL ECD followed by a linker of three alanines (AAA) followed by a human IgG1 Fc containing the mutation N82G with reference to wild-type human IgG1 Fc set forth in SEQ ID NO: 37 (corresponding to N297G by EU numbering). This exemplary Fc also contained stabilizing cysteine mutations R77C and V87C and replacement of the cysteine residue to a serine residue at position 220 (C220S) by EU numbering (corresponding to position 5 (C5S) with reference with reference to wild-type human IgG1 Fc set forth in SEQ ID NO:37 (corresponding to R292C, V302C and C220S, respectively, by EU numbering). In some cases, the NotI cloning site which contributes to the AAA linker sequence was deleted to generate a direct fusion of the ICOSL ECD and the beginning of the Fc. Since the construct does not include any antibody light chains that can form a covalent bond with a cysteine, the human IgG1 Fc also contained replacement of the cysteine residues to a serine residue at position 5 (C5S) compared to the wild-type or unmodified Fc set forth in SEQ ID NO: 37.

Recombinant variant Fc fusion proteins were produced from suspension-adapted human embryonic kidney (HEK) 293 cells using the Expi293 expression system (Invitrogen, USA). Proteins were purified from supernatants using a high throughput 96 well Protein A purification kit using the manufacturer's protocol (Catalog number 45202, Life Technologies, USA). A schematic of an exemplary recombinant variant Fc fusion protein is shown in **FIG. 1A.**

### A. Binding and Functional Characterization

Binding studies were performed to assess specificity and affinity of ICOSL domain variant immunomodulatory proteins for cognate binding partners. Binding studies were carried out on transfected HEK293 cells generated to express the full-length mammalian (human) surface ligands, CD28, ICOS or CTLA-4, using a transient transfection system (Life Technologies, USA). As a control, binding to mock (non-transfected) cells also was assessed. For staining by flow cytometry, 200,000 cells of appropriate transient transfection or negative control (mock) were plated in 96 well round bottom plates, and, then incubated in staining buffer (PBS, phosphate buffered saline; 1% BSA, bovine serum albumin; and 0.1% sodium azide) for 20 minutes to block non-specific binding. Cells were resuspended in staining buffer containing 100 nM to 1nM variant immunomodulatory protein in 50 $\mu$L. Mean Fluorescence Intensity (MFI) was calculated for each transfectant and negative parental line with Cell Quest Pro software (Becton Dickinson, USA) or a Hypercyt flow cytometer (Intellicyte, USA).

Bioactivity of the ECD ICOSL variants also was assessed in an anti-CD3 coimmobilization assay or human Mixed Lymphocyte Reaction (MLR), For the MLR assay, about 10,000 matured primary human dendritic cells were co-cultured with 100,000 purified allogeneic CD4+ T cells (BenTech Bio, USA) in the presence of ICOSL variant Fc fusion proteins and controls in 96 well round bottom plates in 200 $\mu$l final volume of Ex-Vivo 15 media. On days 4-5, IFN-gamma secretion in culture supernatants was analyzed using the Human IFN-gamma Duoset ELISA kit (R&D Systems, USA).

For the anti-CD3 coimmobilization assay, 10 nM mouse anti-human CD3 (OKT3, Biolegends, USA) was diluted in PBS with 40nM rICOSL.Fc variant proteins. This mixture was added to tissue culture treated flat bottom 96 well plates (Corning, USA) overnight to facilitate adherence of the stimulatory proteins to the wells of the plate. The next day, unbound protein was washed off the plates and 100,000 purified human pan T cells (BenTech Bio, US) or human T cell clone BC3 (Astarte Biologies, USA) were added to each well in a final volume of 200 $\mu$l of Ex-Vivo 15 media (Lonza, Switzerland). In some instances, human pan T cells were labeled with 0.25 $\mu$M carboxyfluorescein succinimidyl ester (CFSE, ThermoFisher Scientific, USA). Cells were cultured 3 days before harvesting culture supernatants and measuring human IFN-gamma levels with Duoset ELISA kit (R&D Systems, USA). Cellular proliferation was determined by the percent of input cells that entered division as measured by CFSE dilution on cells stained with fluorescently-conjugated anti-CD4, anti-CD8 antibodies (BD, USA) or total T cells via flow cytometric analysis on an LSR II (BD, USA),

Table 3 depicts exemplary results for selected ICOSL IgSF domain variants for binding to cell-expressed counter structures and bioactivity in the anti-CD3 coimmobilization assay or MLR assay. The exemplary amino acid substitutions depicted in Table 3 are designated by amino acid position number corresponding to the respective reference (e.g., unmodified) ICOSL ECD sequence set forth in SEQ ID NO:1. The amino acid position is indicated in the middle, with the corresponding unmodified (e.g. wild-type) amino acid listed before the number and the identified variant amino acid substitution listed after the number.

The results in Table 3 depict binding activity as measured by the Mean Fluorescence Intensity (MFI) value for binding of each variant Fc-fusion molecule to cells engineered to express the cognate counter structure ligand and the ratio of the MFI compared to the binding of the corresponding reference (e.g., unmodified) ECD-Fc fusion molecule not containing the amino acid substitution(s) to the same cell-expressed counter structure ligand. The functional activity of the variant Fc-fusion molecules to modulate the activity of T cells also is shown based on the calculated levels of IFN-gamma in culture supernatants (pg/mL) generated with either i) the indicated variant ECD-Fc fusion molecule coimmoblized with anti-CD3 or ii) the indicated variant ECD-Fc fusion molecule in an MLR assay. The Table also depicts the ratio of IFN-gamma produced by each variant ECD-Fc compared to the corresponding unmodified (parental) ECD-Fc in both functional assays.

The results show altered, including increased, binding affinity of affinity-modified ICOSL IgSF domain variants for at least one cognate counter structure ligand and/or improved immunological activity. The selections resulted in the identification of a number of ICOSL IgSF domain variants that were affinity-modified to exhibit increased binding for at least one, and in some cases, more than one, cognate counter structure ligand. Because the affinity of native ICOSL for ICOS is relatively high compared to the affinity of native ICOSL to CD28, the differential of increased binding affinity per round of selection was more modest than the increase in CD28 binding, which initially bound wildtype ICOSL very poorly. Selections for elevated CD28 binding concomitantly yielded elevated binding to CTLA4 as well, despite the fact there was no specific selection in favor of this interaction. This observation suggests selection for variants that more effectively interacted with the highly conserved sequence in CTLA4 and CD28 responsible for binding to the ICOSL-related proteins CD80 and CD86.

**Table 3: ICOSL variants: binding data and costimulatory bioactivity data.**

| ICOSL mutation(s) | ICOS tfxn MFI (parental ratio) | CD28 tfxn MFI (parental ratio) | CTLA-4 tfxn MFI (parental ratio) | Anti-CD3 IFN-gamma Coimmobili zation Assay pg/mL (parental ratio) | MLR IFN-gamma pg/mL (parental ratio) |
|---|---|---|---|---|---|
| N52H, F78L, Q100R, C198R | 9568 (0.12) | 1966 (0.24) | 1454 (0.12) | 130 (0.31) | 5927 (1.84) |
| N52H, N57Y, Q100R, V110D, C198R, S212G | 9418 (1.16) | 136665 (16.55) | 115352 (9.59) | 944 (2.21) | 821 (0.25) |
| N52H, N57Y, R75Q, Q100P, V110D | 5558 (0.07) | 7465 (0.90) | 4689 (0.39) | 122 (0.28) | 1136 (0.35) |
| N52H, N57Y, Q100R, C198R | 9148 (1.13) | 134923 (16.33) | 83241 (6.92) | 1060 (2.48) | 375 (0.12) |
| N52H, N57Y, L74Q, V110D, S192G | 9448 (1.17) | 128342 (15.54) | 123510 (10.26) | 1137 (2.66) | 889 (0.28) |
| N52H, Q100R | 9478 (1.17) | 151977 (18.40) | 133929 (11.13) | 972 (2.28) | 794 (0.25) |
| N52H, S121G, C198R | 9128 (1.13) | 124732 (15.10) | 182607 (15.18) | 827 (1.94) | 1257 (0.39) |
| A20V, N52H, N57Y, Q100R, S109G | 5828 (0.72) | 76973 (9.32) | 73640 (6.12) | 447 (1.05) | 2283 (0.71) |
| N52H, N57Y, Q100P, C198R | 9548 (1.18) | 130676 (15.82) | 81966 (6.81) | 1125 (2.64) | 643 (0.20) |
| N52H, N57Y, R61S, Q100R, V110D, L173S | 1018 (0.13) | 9129 (1.11) | 5790 (0.48) | 109 (0.25) | 5094 (1.58) |
| N52H, N57Y, Q100R, V122A | 9978 (1.23) | 137372 (16.63) | 70764 (5.88) | 1316 (3.08) | 473 (0.15) |
| N52H, N57Y, Q100R, F172S | 1028 (1.27) | 135821 (16.44) | 73320 (6.09) | 1561 (3.66) | 486 (0.15) |

(continued)

| Table 3: ICOSL variants: binding data and costimulatory bioactivity data. | | | | | |
|---|---|---|---|---|---|
| ICOSL mutation(s) | ICOS tfxn MFI (parental ratio) | CD28 tfxn MFI (parental ratio) | CTLA-4 tfxn MFI (parental ratio) | Anti-CD3 IFN-gamma Coimmobili zation Assay pg/mL (parental ratio) | MLR IFN-gamma pg/mL (parental ratio) |
| N52H, N57Y, Q100R | 9858 (1.22) | 140612 (17.02) | 75106 (6.24) | 1648 (3.86) | 778 (0.24) |
| N52S, F120S, N227K | 9438 (1.17) | 67796 (8.21) | 82370 (6.85) | 1157 (2.71) | 1626 (0.50) |
| N52S, N194D | 9798 (1.21) | 59431 (7.19) | 74502 (6.19) | 1671 (3.91) | 1690 (0.52) |
| N52S, V97A | 3138 (0.04) | 1733 (0.21) | 1541 (0.13) | 84 (0.20) | 3858 (1.20) |
| N52S, F120S | 9068 (1.12) | 67233 (8.14) | 97880 (8.13) | 1178 (2.76) | 2814 (0.87) |
| N52S, G72R | 9288 (1.15) | 51638 (6.25) | 62339 (5.18) | 1161 (2.72) | 2947 (0.91) |
| N52S, A71T, A117T, T190A, C198R | 8918 (1.10) | 44044 (5.33) | 56646 (4.71) | 1076 (2.52) | 4031 (1.25) |
| N52S, E220G | 3878 (0.05) | 2047 (0.25) | 1796 (0.15) | 122 (0.29) | 1927 (0.60) |
| Y47H, N52S, V107A, F120S | 3268 (0.04) | 2562 (0.31) | 2104 (0.17) | 334 (0.78) | 4390 (1.36) |
| WT ICOSL | 8088 (1.00) | 8260 (1.00) | 12033 (1.00) | 427 (1.00) | 3226 (1.00) |
| T43A, N52H, N57Y, L74Q, D89G, V110D, F172S | 2821 (0.02) | 2180 (0.49) | 2051 (0.12) | 184 (0.75) | |
| N52H, N57Y, Q100R, V107I, V110D, S132F, I154F, C198R, R221G | 174586 (0.97) | 122383 (27.24) | 76202 (4.31) | 985 (4.01) | 1037 (0.36) |
| E16V, N52H, N57Y, Q100R, V110D, H115R, Y152C, K156M, C198R | 190765 (1.05) | 129070 (28.73) | 68488 (3.87) | 4288 (17.46) | 1225 (0.43) |
| Q37R, N52H, N57Y, Q100R, V110N, S142F, C198R, D217V, R221G | 148638 (0.82) | 91104 (20.28) | 13498 (0.76) | 62 (0.25) | 7643 (2.68) |
| N52H, N57Y, Q100R, V110D, C198R | 179194 (0.99) | 123312 (27.45) | 84136 (4.76) | 762 (3.10) | 1342 (0.47) |
| N52H, N57Y, Q100R, V110D, V116A, L161M, F172S, S192G, C198R | 5236 (0.03) | 4160 (0.93) | 3305 (0.19) | 49 (0.20) | 2039 (0.72) |
| F27S, N52H, N57Y, V110N | 20154 (0.11) | 8613 (1.92) | 3903 (0.22) | 83 (0.34) | 7522 (2.64) |
| F27S, N52H, N57Y, V110N | 5236 (0.03) | 4160 (0.93) | 2957 (0.17) | 40 (0.16) | - |

(continued)

| Table 3: ICOSL variants: binding data and costimulatory bioactivity data. | | | | | |
|---|---|---|---|---|---|
| ICOSL mutation(s) | ICOS tfxn MFI (parental ratio) | CD28 tfxn MFI (parental ratio) | CTLA-4 tfxn MFI (parental ratio) | Anti-CD3 IFN-gamma Coimmobili zation Assay pg/mL (parental ratio) | MLR IFN-gamma pg/mL (parental ratio) |
| N52S, H94E, L96I, S109N, L166Q, | 198604 (1.10) | 100361 (22.34) | 102892 (5.82) | 1253 (5.10) | 5645 (1.98) |
| S18R, N52S, F93L, I143V, R221G | 154561 (0.85) | 7625 (1.70) | 4254 (0.24) | 203 (0.83) | 5239 (1.84) |
| A20T, N52D, Y146C, Q164L | 149661 (0.83) | 9073 (2.02) | 6901 (0.39) | 287 (1.17) | 4829 (1.69) |
| V11E, N30D, N52H, N57Y, H94E, L961, L98F, N194D, V210A, I218T | 180016 (1.00) | 120230 (26.76) | 62809 (3.55) | 2218 (9.03) | 7283 (2.56) |
| N52S, H94E, L96I, V122M | 198717 (1.10) | 88901 (19.79) | 94231 (5.33) | 590 (2.40) | 618 (0.22) |
| N52H, N57Y, H94E, L96I, F1201, S126T, W153R, I218N | 87711 (0.48) | 42035 (9.36) | 31798 (1.80) | 67 (0.27) | 2500 (0.88) |
| M10V, S18R, N30D, N52S, S126R, T139S, L203F | 180665 (1.00) | 64929 (14.45) | 48362 (2.73) | 1193 (4.86) | 13647 (4.79) |
| S25G, N30D, N52S, F120S, N227K | 178834 (0.99) | 66127 (14.72) | 46631 (2.64) | 1246 (5.07) | 2202 (0.77) |
| N30D, N52S, L67P, Q100K, D217G, R221K, T225S | 18630 (0.10) | 1986 (0.44) | 1940 (0.11) | 54 (0.22) | 2752 (0.97) |
| WT ICOSL | 180900 (1.00) | 4493 (1.00) | 17685 (1.00) | 246 (1.00) | 2850 (1.00) |
| R26S, N52H, N57Y, V110D, T137A, C198R | N/A | N/A | N/A | N/A | N/A |
| N52H, N57Y, Q100R, V110D, A117T, T190S, C198R | 2831 (0.04) | 2881 (0.57) | 2464 (0.23) | 59 (0.08) | - |
| N52H, N57Y, Q100R, V110D, F172S, C198R | 58478 (0.79) | 74031 (14.75) | 56850 (5.33) | 712 (0.96) | 1093 (0.23) |
| S25G, F27C, N52H, N57Y, Q100R, V110D, E135K, L173S, C198R | 22514 (0.30) | 21320 (4.25) | 20450 (1.92) | 353 (0.48) | 5765 (1.21) |
| N52H, N57Y, V110A, C198R, R221I | 84236 (1.14) | 81842 (16.31) | 121519 (11.39) | 4593 (6.18) | 1137 (0.24) |
| M10I, S13G, N52H, N57Y, D77G, V110A, H129P, I143V, F172S, V193M, C198R | 6362 (0.09) | 6001 (1.20) | 4834 (0.45) | 141 (0.19) | 4326 (0.91) |

(continued)

| Table 3: ICOSL variants: binding data and costimulatory bioactivity data. | | | | | |
|---|---|---|---|---|---|
| ICOSL mutation(s) | ICOS tfxn MFI (parental ratio) | CD28 tfxn MFI (parental ratio) | CTLA-4 tfxn MFI (parental ratio) | Anti-CD3 IFN-gamma Coimmobili zation Assay pg/mL (parental ratio) | MLR IFN-gamma pg/mL (parental ratio) |
| N52H, N57Y, R61C, Y62F, Q100R, V110N, F120S, C198R | 4355 (0.06) | 4316 (0.86) | 3430 (0.32) | 110 (0.15) | 6854 (1.44) |
| N52H, N57Y, Q100R, L102R, V110D, H115R, C198R | 96736 (1.31) | 77881 (15.52) | 148012 (13.88) | 8765 (11.79) | 630 (0.13) |
| N52H, N57Y, Q100R, V110D, N144D, F172S, C198R | 67578 (0.91) | 64953 (12.94) | 95731 (8.98) | 1672 (2.52) | 1490 (0.31) |
| N52S, H94E, L98F, Q100R, | 80690 (1.09) | 78750 (15.69) | 148160 (13.89) | 3564 (4.80) | 1497 (0.32) |
| N52S, E90A | 108908 (1.47) | 31086 (6.19) | 108866 (10.21) | 4564 (6.14) | 3927 (0.83) |
| N30D, K42E, N52S | 85726 (1.16) | 4293 (0.86) | 10755 (1.01) | 5211 (7.01) | 5656 (1.19) |
| N52S, F120S, I143V, I224V | 90862 (1.23) | 28443 (5.67) | 105229 (9.87) | 4803 (6.46) | 4357 (0.92) |
| WT ICOSL | 73964 (1.00) | 5018 (1.00) | 10665 (1.00) | 743 (1.00) | 4748 (1.00) |

**B. ICOSL Protein Structure with Mutations**

The selection of ICOSL variants with increased affinity for both ICOS and CD28, e.g. as shown in Table 3, revealed several amino acid substitutions that appeared to be conserved across multiple clones and appeared to be present in the ICOS/CD28 binding IgV domain of ICOSL. To further elucidate the role of these residues, recurring mutations were mapped onto the structure of ICOSL. The structure for the ICOSL-ICOS complex was modeled using the CD80-CTLA4 X-ray crystal structure (PDB 1D:118L) as the template for homology modeling using the SWISS-MODEL server (Biasini et al., 2014 Nucleic Acids Res. 42:p.W252-8). The sequences of ICOSL and ICOS were aligned to CD80 and CTLA4 structures respectively. Two models were generated independently: (1) the homo-dimer model for ICOSL, and (2) the bound hetero-complex between a single ICOSL unit and ICOS. Next, the full homology model was constructed by superimposing the ICOSL of the hetero-complex to each monomeric unit of the homo-dimer ICOSL model using PyMOL Molecular Graphics System. This produced the full ICOSL homo-dimer bound to ICOS, which was then subjected to a final round of energy minimization to produce the final homology model using Swiss-PDB Viewer.

As shown in **FIG. 1B** except for C198, frequently observed mutations were confined to the IgV domain of ICOSL (see left panel for location of CTLA4 as proxy for position of ICOS and compare to right panel). In contrast, residues found to be less frequently mutated in selected variants were distributed across ICOSL IgV and IgC domains with some bias towards the IgC domain. These results are consistent with an observation that recurrently mutated residues in the IgV domain affect the ICOSL/ICOS interface, while mutations in the IgC domain are less frequent across different clones, and thus, may be more likely non-functional "bystander" mutations that are products of library generation by random mutagenesis. Only the recurrent mutation at position 98 affected an ICOSL amino acid residue within 5 angstroms of the predicted ICOS site of interaction with ICOS. The other mutated residue within 5 angstroms of the ICOS binding site is at position 96 and only infrequently observed across clones. However, the proximity of residues 96 and 98 suggests residue 96 may be another residue that is part of the binding interface. Consistent with this notion, the CD80 residues structurally equivalent to residues 96 and 98 are contact residues for CTLA4 (Peach et al. 1994 J Exp Med, 180:2049-58). Strongly selected mutations at positions other than position 96 (and perhaps 98) in ICOSL are not predicted to be in the predicted ICOS-binding interface. Without wishing to be bound by theory, mutations outside of the binding interface may modulate ligand binding indirectly through allosteric effects transmitted through the IgV domain and into the interface with ICOS as predicted from the modeled structure. Notably, residue 52 is predicted to be glycosylated and removal of a glycan through the mutation of the asparagine may impact binding through alteration of ICOSL tertiary structure.

The results are consistent with an observation that strongly selected mutations affect the ICOS interface either directly or indirectly. This interface is analogous to the CD80/CTLA4 interface (Chattopadhyay et al. 2006 J Immunol, 177:3920-9e) in which the CD28 binding epitope on CD80 overlaps the CTLA4 binding epitope and both ligands utilize similar sequence features to contact CD80 (Peach et al. 1994). Results from this structure/function analysis established why improved binding of ICOS may be associated with improved binding to CD28, even though mutants were selected with either ICOS-Fc or CD28-Fc as selection agents.

### B. Cytokine Production in Anti-CD3 Costimulation Assays

Exemplary variant ECD ICOSL Fc-fusion molecules described above were further assessed for stimulation of cytokines IL-17 in the anti-CD3 costimulatory (coimmobilization) bioactivity assay described above. A mixture of 10 nM plate-bound anti-CD3 and 40 nM ICOSL Fc variant proteins were cultured with human T cells. Supernatants were collected and IL-17 levels were determined by ELISA. The amount of IL-17 in culture supernatants (pg/mL) generated with the indicated variant ECD-Fc fusion molecule and corresponding unmodified (parental) ECD-Fc coimmobilized with anti-CD3 was measured. For comparison, also shown in this Table are the results for production of IFN-gamma in the same assay as depicted in Table 3 for the exemplary variants.

Results are shown in Table 4, which depict the pg/mL of IL-17 measured in the supernatant as well as the ratio (fold increase) of IL-17 produced by each variant ECD-Fc compared to the corresponding unmodified (wild-type) ECD-Fc. Similar results are shown for IFN-gamma. Also shown is the percent of total IL-17 or IFN-gamma cytokine produced by cells. The results showed that affinity modification of the variant molecules exhibited altered functional T cell activity to increase IL-17 in addition to IFN-gamma in the costimulation assay.

**Table 4: Costimulatory Bioreactivity Data for ICOSL IgSF Domain Variants**

| ICOSL mutation(s) | IL-17A [pg/mL] | IL-17A Fold ↑WT | IFN-g [pg/mL] | IFN-g Fold ↑WT | Total Fold ↑WT | % of Total Cytokine Produced | | % Total IL-17 + IFN-g |
|---|---|---|---|---|---|---|---|---|
| | | | | | | % IL-17 | % IFN-g | |
| N52H, N57Y, Q100R, C198R | 617 | 7.93 | 1060 | 2.48 | 10.42 | 5.51 | 0.77 | 6.28 |
| N52H, N57Y, Q100R, V122A | 647 | 8.33 | 1316 | 3.08 | 11.41 | 5.79 | 0.96 | 6.75 |
| N52H, N57Y, Q100R, F172S | 549 | 7.06 | 1561 | 3.66 | 10.72 | 4.91 | 1.14 | 6.05 |
| N52Y, N57Y, F138L, L203P | 90 | 1.05 | 1999 | 2.69 | 3.74 | 0.81 | 2.91 | 3.72 |
| V11E, N30D, N52H, N57Y, H94E, L96I, L98F, N194D, V210A, I218T | 319 | 3.16 | 2218 | 9.03 | 12.19 | 2.85 | 3.23 | 6.08 |
| N52H, N57Y, Q100R, L102R, V110D, H115R, C198R | 510 | 5.90 | 8765 | 11.79 | 17.70 | 4.56 | 12.78 | 17.33 |
| N52H, N57Y, Q100R | 473 | 6.08 | 1648 | 3.86 | 9.94 | 4.23 | 1.20 | 5.43 |
| N52H, Q100R | 358 | 4.60 | 972 | 7.01 | 11.62 | 3.20 | 0.71 | 3.91 |
| N52H, N57Y, Q100R, V110D, C198R, S212G | 124 | 1.60 | 944 | 2.21 | 3.81 | 1.11 | 0.69 | 1.80 |
| N52H, N57Y, Q100P | 127 | 1.47 | 4922 | 6.62 | 8.09 | 1.14 | 7.17 | 8.31 |
| E16V, N52H, N57Y, Q100R, V110D, H115R, Y152C, K156M, C198R | 22 | 7.11 | 130 | 17.46 | 24.57 | 6.41 | 6.25 | 12.66 |
| N30D, K42E, N52S | 349 | 4.04 | 5211 | 7.01 | 11.05 | 3.12 | 7.60 | 10 .71 |

(continued)

**Table 4: Costimulatory Bioreactivity Data for ICOSL IgSF Domain Variants**

| ICOSL mutation(s) | IL-17A [pg/mL] | IL-17A Fold ↑WT | IFN-g [pg/mL] | IFN-g Fold ↑WT | Total Fold ↑WT | % of Total Cytokine Produced | | % Total IL-17 + IFN-g |
|---|---|---|---|---|---|---|---|---|
| | | | | | | % IL-17 | % IFN-g | |
| N52S, F120S, I143V, I224V | 292 | 3.39 | 4803 | 6.46 | 9.85 | 2.61 | 7.00 | 9.62 |
| N52S, E90A | 306 | 3.54 | 4564 | 6.14 | 9.68 | 2.73 | 6.65 | 9.39 |
| N52H, N57Y, V110A, C198R, R221I | 290 | 3.35 | 4593 | 6.18 | 9.53 | 2.59 | 6.69 | 9.28 |
| N52S, N194D | 428 | 5.50 | 1671 | 3.90 | 9.4 | 1.52 | 5.19 | 5.40 |
| N52H, I143T | 84 | - | 1727 | - | 3.30 | 0.75 | 2.52 | 3.27 |
| N52D | 126 | - | 1447 | - | 3.41 | 1.13 | 2.11 | 3.23 |

## EXAMPLE 2

### Purification and Assessment of Purified of ICOSL IgSF Domain Variants

A purification strategy was employed for exemplary candidate hits described above. Human cells derived from the 293 cell line (Expi293) were transiently transfected with expression construct and the ECD ICOSL Fc fusion molecule was expressed in the cells. The Fc fusion proteins were then purified from supernatants with Protein A by affinity chromatograpy (MabSelect SuRe). This initial purification step was then followed by a preparative size exclusion chromatography (SEC) step to further purify the proteins (Superdex200 16x60). Samples from both purification steps were retained and compared by analytic SEC. The concentration of the protein was determined after Protein A purification. The resulting purified proteins also were analyzed by analytic SEC on a high performance liquid chromatography (HPLC) to assess purity.

The percent main peak in the purified samples was determined and compared to protein purified in the initial Protein A step (% Main Peak Prot A pool) versus protein purified with Protein A followed by preparative SEC (% Main Peak SEC pool T=D0). As shown in Table 5, the additional SEC step substantially increased protein purity of purified proteins. To further assess stability of the proteins, proteins purified by preparative SEC were left at room temperature for 24 hours and then % Main Peak by HPLC (% Main Peak SEC pool T=D24) was assessed and compared to D0 sample. The change in % Main Peak at D0 versus D24 was determined (. % Main Peak SEC pool). As shown in Table 5, most of the tested exemplary variant ECD ICOSL Fc fusion molecules exhibited little change in % Main Peak at this time, indicating minimal aggregation of the protein variants had occurred.

**Table 5: Purification of ICOSL Protein Variants**

| **ICOSL mutation(s)** | **Expi293 Prod. Prot A mg/L** | **% Main Peak Prot A pool** | **% Main Peak SEC pool T=D0** | **% Main Peak SEC pool T=D24** | **▲% Main Peak SEC pool** |
|---|---|---|---|---|---|
| N52S, N194D | 120 | 87.9 | 93.5 | 92 | 1.5 |
| N52H, N57Y, Q100R, F172S | 217 | 86.9 | 97.4 | 95.6 | 1.8 |
| N52S, E90A | 128 | 86.5 | 89.5 | 88.3 | 1.2 |
| N52H, Q100R | 176 | 85.9 | 97.5 | 96.1 | 1.4 |
| N52H, N57Y, Q100R | 186 | 85.1 | 97.6 | 95.7 | 1.9 |
| N52S, F120S, I143V, I224V | 87 | 83.2 | 88.9 | 88.3 | 0.6 |
| N52H, N57Y, Q100R, C198R | 204 | 82.9 | 95.8 | 92.3 | 3.5 |
| N52H, N57Y, Q100P | 63 | 80.5 | 94.5 | 88.5 | 6 |

(continued)

| Table 5: Purification of ICOSL Protein Variants | | | | | |
|---|---|---|---|---|---|
| ICOSL mutation(s) | Expi293 Prod. Prot A mg/L | % Main Peak Prot A pool | % Main Peak SEC pool T=D0 | % Main Peak SEC pool T=D24 | ▲% Main Peak SEC pool |
| N30D, K42E, N52S | 81 | 80 | 95.4 | 91.3 | 4.1 |
| N52H, N57Y, Q100R, L102R, V110D, H115R, C198R | 141 | 78.9 | 96 | 92.9 | 3.1 |
| N52H, N57Y, Q100R, V122A | 260 | 77.6 | 96.4 | 95.2 | 1.2 |
| N52Y/N57Y/F138L/L203P | 40 | 75.6 | 96.8 | 94.8 | 2 |
| E16V, N52H, N57Y, Q100R, V110D, H115R, Y152C, K156M, C198R | 60 | 73.8 | 97.1 | 95.8 | 1.3 |
| N52H, N57Y, V110A, C198R, R221I | 95 | 65.4 | 90.9 | 86 | 4.9 |
| N52H, N57Y, Q100R, V110D, C198R, S212G | 73 | 50.6 | 87.9 | 78.6 | 9.3 |
| V11E, N30D, N52H, N57Y, H94E, L96I, L98F, N194D, V210A, I218T | 58 | - | - | - | - |
| N52H, I143T | 134 | 93.2 | 96 | 92.7 | 3.3 |
| N52D | 136 | 90.4 | 95.5 | 93.3 | 2.2 |

## EXAMPLE 3

### Assessment of Costimulatory Bioactivity of Purified ICOSL IgSF Domain Variant Hits

Exemplary ECD ICOSL Fc fusion molecules purified as described in Example 2 were assessed for bioactivity by MLR substantially as described in Example 1. A mixture of 10 nM or 40 nM ICOSL Fc variant proteins was bound overnight to 96-well plates in the presence of 10 nM anti-CD3. The plates were washed and 100,000 CFSE labelled pan T cells were added for 96 hours. Supernatants were collected, and IFN-gamma and IL-17 levels were measured by ELISA.

Results for the cytokine secretion induced by anti-CD3 costimulation with exemplary tested variants (10 nM and 40 nM ICOSL Fc) are shown in **FIGS. 2A and 2B,** which indicate exemplary IgSF domain amino acid substitutions (replacements) in the ECD of ICOSL. The bar graphs in **FIGS. 2A and 2B** depict the amount of secreted IFN-gamma and IL-17, respectively, by ELISA in the supernatants (pg/mL). The level of cytokine release induced by anti-CD3 costimulation with the tested variants compared to the level induced by anti-CD3 costimulation with WT ICOSL is indicated by the horizontal line. The results showed that affinity modification of the variant molecules exhibited activity to modulate functional T cell activity, including to substantially increase IFN-gamma and IL-17 secretion in the costimulation assay. Increased immunological activity was observed with some variants.

## EXAMPLE 4

### Assessment of Proliferation of Purified ICOSL IgSF Domain Variant Hits

Exemplary variant ECD ICOSL Fc fusion molecules purified as described in Example 2 were assessed for ability to costimulate anti-CD3-induced proliferation of T cells.

Primary T cells were labeled with carboxyfluorescein succinmidyl ester (CFSE). A mixture of 10 nM or 40 nM variant ECD ICOSL Fc or wild-type ECD ICOSL proteins were bound overnight to 96-well plates in the presence of 10 nM anti-CD3, and then labeled T cells were added and incubated for 3 days. As a control, proliferation also was assessed in the presence of bound anti-CD3 and IgG or IgG alone. Cells were stained for CD4 or CD8 surface markers and proliferation of total T cells, CD4+ T cells or CD8+ T cells was determined by assessing CFSE dilution by flow cytometry.

The results are set forth in **FIGS. 3A and FIG. 3B** for exemplary variants tested at 40 nM and 10 nM ICOSL, respectively. As shown in **FIG. 3A,** nearly all tested variant ECD ICOSL Fc fusion molecules induced proliferation greater than WT control. As shown in **FIG. 3B,** differences in proliferation were more apparent at 10 nM, with certain variants providing maximal proliferation even at this lower concentration.

## EXAMPLE 5

### Assessment of Binding and Activity of Purified ICOSL IgSF Domain Variant Hits

Exemplary variant ECD ICOSL Fc fusion molecules purified as described in Example 2 were assessed for binding and functional activities using methods substantially as described in Example 1.

### A. Flow Cytometric Binding Assays

Human cells derived from the 293 cell line (Expi293) were transfected with CD28, CTLA-4, or ICOS, or mock transfected. Cells were then incubated with ECD ICOSL Fc fusion molecules or wild-type ECD ICOSL-Fc molecules that were titrated from 100,000 pM to 46 pM, and binding was observed by flow cytometry as described in Example 1. Binding was assessed by flow cytometry and mean fluorescence intensity (MFI) and percent (%) of cells positive for signal was determined using Cell Quest Pro software (Becton Dickinson, USA). The concentration of ICOSL-Fc that gave a half-maximal MFI response (MFI EC50) or % positive cells (% (+) EC50) was determined.

Table 6 sets forth the results. The ICOSL amino acid substitutions depicted in Table 6 are designated by amino acid position number corresponding to the respective reference (e.g., unmodified) ICOSL ECD sequence set forth in SEQ ID NO:1. For some values (e.g. WT binding to CD28) it was not possible to obtain an EC50, therefore 1,000,000 pM was arbitrarily piced for data formatting purposes. Similar to results obtained from previous binding assays as described in Example 1 above, altered binding affinity of variant ICOSL ECD-Fc fusion molecule for at least one cognate counter structure ligand was observed.

**Table 6: Flow Cytometric EC50s for ICOSL variants**

| ICOSL mutation(s) | CD28 MFI EC50 [pM] | CD28 % (+) EC50 [pM] | CTLA-4 MFI EC50 [pM] | CTLA-4 % (+) EC50 [pM] | ICOS MFI EC50 [pM] | ICOS % (+) EC50 [pM] |
|---|---|---|---|---|---|---|
| WT ICOSL | 1000000 | 1000000 | 1000000 | 1000000 | 10543 | 762 |
| N52H, I143T | 19147 | 567 | 20259 | 1891 | 2666 | 286 |
| N52H, N57Y, Q100R, C198R | 950 | 159 | 73548 | 422 | 1032 | 179 |
| N52H, N57Y, Q100R, V122A | 29701 | 152 | 1008 | 293 | 302 | 64 |
| N52H, N57Y, Q100R, F172S | 1006 | 231 | 1332 | 396 | 779 | 130 |
| N52Y/N57Y/F138L/L203P | 7844 | 386 | 7457 | 994 | 3104 | 408 |
| V11E, N30D, N52H, N57Y, H94E, L96I, L98F, N194D, V210A, I218T | 5961 | 595 | 6909 | 1026 | 5514 | 852 |
| N52H, N57Y, Q100R, L102R, V110D, H115R, C198R | 1034 | 307 | 23328 | 579 | 3172 | 347 |
| N52H, N57Y, Q100R | 1665 | 238 | 11002 | 533 | 383 | 131 |
| N52H, Q100R | 1305 | 274 | 8593 | 1997 | 702 | 167 |
| N52H, N57Y, Q100R, V110D, C198R, S212G | 4987 | 594 | 30382 | 922 | 50219 | 814 |
| N52H, N57Y, Q100P | 21137 | 402 | 22651 | 758 | 4090 | 320 |
| E16V, N52H, N57Y, Q100R, V110D, H115R, Y152C, K156M, C198R | 2508 | 387 | 5399 | 806 | 2381 | 421 |
| N30D, K42E, N52S | - | 3683800 | 8593 | 1997 | 3251 | 558 |
| N52S, F120S, I143V, I224V | 902400 | 9060 | 28126 | 2948 | 4366 | 245 |

(continued)

| Table 6: Flow Cytometric EC50s for ICOSL variants | | | | | | |
|---|---|---|---|---|---|---|
| ICOSL mutation(s) | | | | | | |
| | CD28 MFI EC50 [pM] | CD28 % (+) EC50 [pM] | CTLA-4 MFI EC50 [pM] | CTLA-4 % (+) EC50 [pM] | ICOS MFI EC50 [pM] | ICOS % (+) EC50 [pM] |
| N52S, E90A | 1339700 | 31302 | 31419 | 5828 | 5225 | 473 |
| N52H, N57Y, V110A, C198R, R221I | 1809 | 426 | 7201 | 841 | 1293 | 433 |
| N52S, N194D | 944669 | 11876 | 1254880 | 5170 | 473 | 206 |
| N52D | 288617 | 17793 | 396841 | 3891 | 2642 | 137 |

**B. ForteBio Binding Assay**

Protein-protein interactions between the receptors and ICOSL domain variant immunomodulatory proteins were further assessed using ForteBio binding assays. ICOS, CD28, and CTLA-4 receptors were loaded individually onto anti-human capture sensors (ForteBio Octet AHC) and wildtype unmodified ICOSL ECD-Fc fusion molecules, wildtype PD-L2 ED-Fc fusion molecules or variant ICOSL Fc-fusion molecules were bound to the receptors in 4 point titrations. Each titration was globally fit to calculate the association ($k_{on}$) and dissociation ($K_{dis}$) of each protein. Loading response of anti-human capture sensors of each receptor being tested with the variant ICOSL ECD-Fc fusion molecule was determined. The dissociation constant (KD) was calculated and compared to wildtype to determine a fold improvement value (fold imp.).

Binding results to ICOS are set forth in Table 7, to CD28 are set forth in Table 8 and to CTLA-4 are set forth in Table 9. The exemplary amino acid substitutions depicted in Table 7-9 are designated by amino acid position number corresponding to the respective reference unmodified ICOSL ECD sequence set forth in SEQ ID NO:1.

| Table 7: ICOS ForteBio Binding Assay | | | | | | |
|---|---|---|---|---|---|---|
| ICOSL mutation(s) | Response | KD (M) | $K_{on}$ (1/Ms) | $K_{dis}$ (1/s) | Full $R^2$ | Fold Imp. |
| WT ICOSL | 0.73 | 8.83E-10 | 1.78E+05 | 1.58E-04 | 0.9908 | - |
| N52H, I143T | 0.87 | 3.32E-10 | 3.13E+05 | 1.04E-04 | 0.9683 | 2.7 |
| N52H, N57Y, Q100R, C198R | 0.74 | 4.92E-10 | 3.85E+05 | 1.89E-04 | 0.9882 | 1.8 |
| N52H, N57Y, Q100R, V122A | 0.67 | 4.72E-10 | 3.77E+05 | 1.78E-04 | 0.9775 | 1.9 |
| N52H, N57Y, Q100R, F172S | 0.68 | 4.20E-10 | 4.34E+05 | 1.82E-04 | 0.9545 | 2.1 |
| N52Y/N57Y/F138L/L203P | 0.64 | 7.69E-10 | 2.22E+05 | 1.71E-04 | 0.9782 | 1.1 |
| V11E, N30D, N52H, N57Y, H94E, L961, L98F, N194D, V210A, I218T | 0.67 | 3.62E-10 | 3.55E+05 | 1.29E-04 | 0.9687 | 2.4 |
| N52H, N57Y, Q100R, L102R, V110D, H115R, C198R | 0.76 | 4.77E-10 | 3.29E+05 | 1.57E-04 | 0.9616 | 1.9 |
| N52H, N57Y, Q100R | 0.74 | 3.69E-10 | 2.87E+05 | 1.06E-04 | 0.9817 | 2.4 |

(continued)

| Table 7: ICOS ForteBio Binding Assay | | | | | | |
|---|---|---|---|---|---|---|
| **ICOSL mutation(s)** | **Response** | **KD (M)** | **$K_{on}$ (1/Ms)** | **$K_{dis}$ (1/s)** | **Full $R^2$** | **Fold Imp.** |
| N52H, Q100R | 0.79 | 3.73E-10 | 4.45E+05 | 1.66E-04 | 0.968 | 2.4 |
| N52H, N57Y, Q100R, V110D, C198R, S212G | 0.60 | 1.29E-09 | 1.66E+05 | 2.15E-04 | 0.9846 | 0.7 |
| N52H, N57Y, Q100P | 0.73 | 3.82E-10 | 3.71 E+05 | 1.42E-04 | 0.9729 | 2.3 |
| E16V, N52H, N57Y, Q100R, V110D, H115R, Y152C, K156M, C198R | 0.75 | 5.43E-10 | 2.65E+05 | 1.44E-04 | 0.9848 | 1.6 |
| N30D, K42E, N52S | 0.80 | 3.71E-10 | 4.48E+05 | 1.66E-04 | 0.9651 | 2.4 |
| N52S, F120S, I143V, I224V | 0.80 | 3.11E-10 | 5.03E+05 | 1.56E-04 | 0.9673 | 2.8 |
| N52S, E90A | 0.88 | 3.40E-10 | 4.85E+05 | 1.65E-04 | 0.9792 | 2.6 |
| N52H, N57Y, V110A, C198R, R221I | 0.68 | 4.77E-10 | 3.15E+05 | 1.50E-04 | 0.976 | 1.9 |
| N52S, N194D | 0.88 | 3.37E-10 | 3.38E+05 | 1.14E-04 | 0.9723 | 2.6 |
| N52D | 0.87 | 3.38E-10 | 3.91E+ 05 | 1.32E-04 | 0.9792 | 2.6 |
| Wildtype PD-L2 ED-Fc | 0.03 | | | | | |

| Table 8: CD28 ForteBio Binding Assay | | | | | | |
|---|---|---|---|---|---|---|
| **ICOSL mutation(s)** | **Response** | **KD (M)** | **$K_{on}$ (1/Ms)** | **$K_{dis}$ (1/s)** | **Full $R^2$** | **Fold Imp.** |
| WT ICOSL | 0.33 | 1.39E-08 | 6.69E+04 | 9.29E-04 | 0.9715 | - |
| N52H, I143T | 0.95 | 5.25E-10 | 4.27E+05 | 2.24E-04 | 0.9877 | 26.5 |
| N52H, N57Y, Q100R, C198R | 1.14 | 4.47E-10 | 4.12E+05 | 1.84E-04 | 0.9877 | 31.0 |
| N52H, N57Y, Q100R, V122A | 1.04 | 3.90E-10 | 4.07E+05 | 1.59E-04 | 0.9878 | 35.6 |
| N52H, N57Y, Q100R, F172S | 1.06 | 2.93E-10 | 4.26E+05 | 1.25E-04 | 0.9836 | 47.3 |
| N52Y/N57Y/F138L/L203P | 0.86 | 7.83E-10 | 1.79E+05 | 1.40E-04 | 0.993 | 17.7 |
| V11E, N30D, N52H, N57Y, H94E, L96I, L98F, N194D, V210A, I218T | 0.92 | 5.53E-10 | 2.54E+05 | 1.40E-04 | 0.9906 | 25.1 |
| N52H, N57Y, Q100R, L102R, V110D, H115R, C198R | 1.10 | 3.66E-10 | 3.41 E+05 | 1.25E-04 | 0.986 | 37.9 |

(continued)

| Table 8: CD28 ForteBio Binding Assay | | | | | | |
|---|---|---|---|---|---|---|
| ICOSL mutation(s) | Response | KD (M) | $K_{on}$ (1/Ms) | $K_{dis}$ (1/s) | Full $R^2$ | Fold Imp. |
| N52H, N57Y, Q100R | 1.04 | 3.68E-10 | 3.72E+05 | 1.37E-04 | 0.983 | 37.7 |
| N52H, Q100R | 1.09 | 4.01E-10 | 5.0SE+05 | 2.02E-04 | 0.9938 | 34.7 |
| N52H, N57Y, Q100R, V110D, C198R, S212G | 0.94 | 8.96E-10 | 1.78E+05 | 1.60E-04 | 0.9961 | 15.5 |
| N52H, N57Y, Q100P | 0.99 | 4.36E-10 | 3.29E+05 | 1.43E-04 | 0.9835 | 31.8 |
| E16V, N52H, N57Y, Q100R, V110D, H115R, Y152C, K156M, C198R | 1.06 | 5.03E-10 | 3.06E+05 | 1.54E-04 | 0.9872 | 27.6 |
| N30D, K42E, N52S | 0.54 | 1.95E-09 | 2.74E+05 | 5.33E-04 | 0.9772 | 7.1 |
| N52S, F120S, I143V, I224V | 0.84 | 9.10E-10 | 4.51 E+05 | 4.10E-04 | 0.9742 | 15.3 |
| N52S, E90A | 0.94 | 9.69E-10 | 4.74E+05 | 4.59E-04 | 0.978 | 14.3 |
| N52H, N57Y, V110A, C198R, R221I | 0.94 | 5.63E-10 | 2.63E+05 | 1.48E-04 | 0.9781 | 24.7 |
| N52S, N194D | 0.82 | 1.04E-09 | 3.53E+05 | 3.68E-04 | 0.9887 | 13.3 |
| N52D | 0.86 | 1.16E-09 | 3.36E+05 | 3.90E-04 | 0.989 | 11.9 |
| wildtype PD-L2 ED-Fc | -0.04 | | | | | |

| Table 9: CTLA-4 ForteBio Binding Assay | | | | | | |
|---|---|---|---|---|---|---|
| ICOSL mutation(s) | Response | KD (M) | $K_{on}$ (1/Ms) | $K_{dis}$ (1/s) | Full $R^2$ | Fold Imp. |
| WT ICOSL | 0.21 | 7.71E-08 | 1.92E+04 | 1.48E-03 | 0.8919 | - |
| N52H, I143T | 0.96 | 6.78E-10 | 7.26E+05 | 4.92E-04 | 0.9641 | 113.8 |
| N52H, N57Y, Q100R, C198R | 1.57 | 6.45E-10 | 4.79E+05 | 3.09E-04 | 0.9875 | 119.6 |
| N52H, N57Y, Q100R, V122A | 1.43 | 5.76E-10 | 4.73E+05 | 2.72E-04 | 0.9926 | 133.9 |
| N52H, N57Y, Q100R, F172S | 1.47 | 5.36E-10 | 5.13E+05 | 2.75E-04 | 0.9924 | 144.0 |
| N52Y/N57Y/F138L/L203P | 1.33 | 8.33E-10 | 3.45E+05 | 2.87E-04 | 0.9943 | 92.6 |
| V11E, N30D, N52H, N57Y, H94E, L961, L98F, N194D, V210A, I218T | 1.50 | 6.48E-10 | 3.12E+05 | 2.02E-04 | 0.9943 | 119.0 |
| N52H, N57Y, Q100R, L102R, V110D, H115R, C198R | 1.60 | 8.64E-10 | 4.79E+05 | 4.14E-04 | 0.9825 | 89.2 |
| N52H, N57Y, Q100R | 1.65 | 7.19E-10 | 4.28E+05 | 3.08E-04 | 0.9895 | 107.2 |
| N52H, Q100R | 1.17 | 5.92E-10 | 8.37E+05 | 4.96E-04 | 0.9629 | 130.3 |
| N52H, N57Y, Q100R, V110D, C198R, S212G | 1.32 | 1.47E-09 | 2.34E+05 | 3.44E-04 | 0.9937 | 52.6 |

(continued)

| Table 9: CTLA-4 ForteBio Binding Assay | | | | | | |
|---|---|---|---|---|---|---|
| ICOSL mutation(s) | Response | KD (M) | $K_{on}$ (1/Ms) | $K_{dis}$ (1/s) | Full $R^2$ | Fold Imp. |
| N52H, N57Y, Q100P | 1.51 | 6.47E-10 | 3.61 E+05 | 2.33E-04 | 0.9911 | 119.2 |
| E16V, N52H, N57Y, Q100R, V110D, H115R, Y152C, K156M, C198R | 1.58 | 1.06E-09 | 4.24E+05 | 4.49E-04 | 0.9779 | 72.8 |
| N30D, K42E, N52S | 0.42 | 2.81E-09 | 2.42E+05 | 6.81E-04 | 0.9676 | 27.4 |
| N52S, F120S, I143V, I224V | 0.58 | 1.20E-09 | 3.10E+05 | 3.72E-04 | 0.9283 | 64.3 |
| N52S, E90A | 0.64 | 1.12E-09 | 3.28E+05 | 3.68E-04 | 0.9184 | 68.7 |
| N52H, N57Y, V110A, C198R, R221I | 1.44 | 1.07E-09 | 4.0SE+05 | 4.32E-04 | 0.9811 | 72.3 |
| N52S, N194D | 0.59 | 2.52E-09 | 2.66E+05 | 6.69E-04 | 0.9643 | 30.6 |
| N52D | 0.62 | 1.52E-09 | 4.16E+05 | 6.32E-04 | 0.9234 | 50.7 |
| wildtype PD-L2 ED-Fc | 0.00 | | | | | |

### C. Coimmobilization Assay

Costimulatory bioactivity of ICOSL fusion variants was determined in anti-CD3 coimmobilization assays substantially as described in Example 1. Approximately 0.37 nM, 1.3 nM or 10n M mouse anti-human CD3 (OKT3, Biolegend, USA) was diluted in PBS with 10 nM or 40 nM variant ICOSL ECD Fc or wild-type ICOSL ECD-Fc. This mixture was added to tissue culture treated flat bottom 96 well plates overnight to facilitate adherence of the stimulatory proteins to the wells of the plate. The next day, unbound protein was washed off the plates and 100,000 purified human pan T cells were added to each well. Cells were cultured 3 days before harvesting culture supernatants and measuring human IFN-gamma levels with an ELISA kit.

Table 10 sets fort the amount of IFN-gamma (pg/mL) produced by cells under the various conditions in the anti-CD3 coimmobilization assay. In the Table, the amino acid substitutions of exemplary variant ECD ICOSL-Fc fusions are designated by amino acid position number corresponding to the unmodified ICOSL ECD sequence set forth in SEQ ID NO: 1. The ratio of IFN-gamma produced in the presence of each variant ICOSL ECD-Fc in the functional assay compared to in the presence of the corresponding unmodified (wildtype) ECD-Fc is shown (Fold ↑WT). As shown, costimulatory signaling of the variant ICOSL-ECD-Fc molecules were substantially greater compared to wildtype ICOSL.

| Table 10: Assessment of IFN-gamma Responses in Co-stimulation Assay | | | | | | | |
|---|---|---|---|---|---|---|---|
| ICOSL mutation(s) | IFN-gamma [pg/mL] | | | | | | |
| | 40nM Ligand | | 10nM Ligand | | 40nM Ligand | | |
| | 10mM OKT3 | Fold ↑WT | 10mM OKT3 | Fold ↑WT | 10mM OKT3 | 1.1 nM OKT3 | 0.37 nM OKT3 |
| E16V, N52H, N57Y, Q100R, V110D, H115R, Y152C, K156M, C198R | 14372 | 17.3 | 4903 | 29.9 | 8379.2 | 7422.8 | 2893.7 |
| N52H, N57Y, Q100R, V122A | 10640 | 12.8 | 6456 | 39.4 | 5636.2 | 4724.2 | 2246.3 |
| N52H, N57Y, Q100R, F172S | 10379 | 12.5 | 3741 | 22.8 | 3979.7 | 4067.7 | 1415.5 |
| N52H, N57Y, Q100R, C198R | 9590 | 11.5 | 4048 | 24.7 | 4215.8 | 2787.1 | 1072.4 |
| N52H, N57Y, Q100R | 9568 | 11.5 | 3270 | 19.9 | 4412.3 | 3862.0 | 1820.0 |
| N52H, N57Y, Q100R, L102R, V110D, H115R, C198R | 6939 | 8.4 | 3234 | 19.7 | 5495.2 | 4081.6 | 1442.8 |
| N52S, F120S, I143V, I224V | 6567 | 7.9 | 717 | 4.4 | 2145.4 | 2185.7 | 646.1 |
| N52S, N194D | 5690 | 6.8 | 272 | 1.7 | 2315.1 | 1485.0 | 1140.6 |

(continued)

| Table 10: Assessment of IFN-gamma Responses in Co-stimulation Assay | | | | | | | |
|---|---|---|---|---|---|---|---|
| ICOSL mutation(s) | IFN-gamma [pg/mL] | | | | | | |
| | 40nM Ligand | | 10nM Ligand | | 40nM Ligand | | |
| | 10mM OKT3 | Fold ↑WT | 10mM OKT3 | Fold ↑WT | 10mM OKT3 | 1.1 nM OKT3 | 0.37 nM OKT3 |
| V11E, N30D, N52H, N57Y, H94E, L961, L98F, N194D, V210A, I218T | 5345 | 6.4 | 1152 | 7.0 | 2747.0 | 3383.4 | 1701.2 |
| N52S, E90A | 5097 | 6.1 | 706 | 4.3 | 5019.8 | 3036.4 | 1482.4 |
| N52H, N57Y, V110A, C198R, R221I | 4737 | 5.7 | 520 | 3.2 | 2501.5 | 1632.1 | 937.5 |
| N52H, Q100R | 4122 | 5.0 | 1466 | 8.9 | 5782.1 | 2861.4 | 967.5 |
| N30D, K42E, N52S | 4080 | 4.9 | 273 | 1.7 | 1336.8 | 1260.7 | 541.1 |
| N52H, N57Y, Q100P | 3344 | 4.0 | 229 | 1.4 | 2525.4 | 2439.5 | 1233.9 |
| N52H, N57Y, Q100R, V110D, C198R, S212G | 3064 | 3.7 | 1471 | 9.0 | 2699.5 | 2629.9 | 678.2 |
| N52Y, N57Y, F138L, L203P | 2177 | 2.6 | 200 | 1.2 | 1889.5 | 1757.9 | 808.8 |
| N52H, I143T | 1906 | 2.3 | 138 | 0.8 | 1417.1 | 1367.9 | 275.2 |
| WT ICOSL | 831 | 1.0 | 164 | 1.0 | 558.8 | 377.7 | 152.0 |
| N52D | 88 | 0.1 | 231 | 1.4 | 1288.9 | 1737.9 | 289.0 |

**D. Mixed Lymphocyte Reaction for Assessment of Bioreactivity Suppression**

Modulation of T cell activity by fusion variants was determined in a mixed lymphocyte reaction (MLR) substantially as described in Example 1. Human monocytes were incubated for 6 days in the presence of IL-4 and GM-CSF and matured to dendritic cells with the additional of LPS for the final 24 hours. 1 × $10^4$ dendritic cells and 1 × $10^5$ human CFSE- labeled T cells were plated per well and incubated for 4 days in the presence of three different concentrations (40 nM, 13.3 nM or 4.4 nM) of wildtype or recombinant variant ICOSL ECD-Fc molecules diluted in PBS. The same concentrations of human IgG, PD-L2-Fc or Belatacept (CTLA4-Fc containing L104E and A29Y mutations) were used as controls. Supernatants were harvested and IFN-gamma responses were characterized by ELISA.

**FIG. 4** depicts the IFN-gamma production under the various conditions. The levels of IFN-gamma produced by cells in the presence of wild-type ICOSL is set forth by the horizontal line. No suppression of IFN-gamma production was observed in the presence of negative control protein PD-L2-Fc. In contrast, most of the tested ICOSL variants exhibited some degree of inhibition of IFN-gamma production in the MLR. Certain variants exhibited substantial inhibition of IFN-gamma with very low to no detectable IFN-gamma produced in the cultures, even at the lowest concentration of 4.4 nM tested. The percent MLR suppression in the presence of 4.4 nM of variant of variant ECD ICOSL-Fc is set forth in Table 11. In the Table, the negative values indicate an inflammatory effect in the assay.

| Table 11: Costimulatory bioactivity data for ICOSL in MLR | |
|---|---|
| ICOSL mutation(s) | % MLR Suppresion (4.4nM) |
| N52H, N57Y, Q100R, C198R | 93.6 |
| N52H, N57Y, Q100R, V122A | 94.4 |
| N52H, N57Y, Q100R, F172S | 100.0 |
| N52Y/N57Y/F138L/L203P | 100.0 |
| V11E, N30D, N52H, N57Y, H94E, L96I, L98F, N194D, V210A, I218T | 100.0 |
| N52H, N57Y, Q100R, L102R, V110D, H115R, C198R | 100.0 |

(continued)

| Table 11: Costimulatory bioactivity data for ICOSL in MLR | |
|---|---|
| ICOSL mutation(s) | % MLR Suppresion (4.4nM) |
| N52H, N57Y, Q100R | 98.2 |
| N52H, Q100R | 97.5 |
| N52H, N57Y, Q100R, V110D, C198R, S212G | 90.4 |
| N52H, N57Y, Q100P | 100.0 |
| E16V, N52H, N57Y, Q100R, V110D, H115R, Y152C, K156M, C198R | 100.0 |
| N30D, K42E, N52S | -38.8 |
| N52S, F120S, I143V, I224V | -44.2 |
| N52S, E90A | -30.4 |
| N52H, N57Y, V110A, C198R, R221I | 100.0 |
| N52S, N194D | -22.3 |
| N52H, I143T | -78.0 |
| N52D | 0.5 |

**E. Assessment of Proliferation and Intracellular Cytokine Markers by Flow**

**Cytometry**

Carboxyfluorescein succinmidyl ester (CFSE) labeled pan T cells from MLR studies as described above that had been incubated for 4 days in the presence of wildtype or recombinant variant ICOSL ECD-Fc molecules were further tested for cytokine levels by restimulation with phorbol myristate acetate (PMA)/Ionomycin for 6 hours in the presence of a golgi inhibitor (Golgi/Block/Plug). T cells from the MLR study that had been incubated with human IgG, anti-CD28, anti-ICOSL, PD-L2-Fc, or Belatacept (CTLA4-Fc containing L104E and A29Y mutations) also were restimulated. T cells were stained for CD4 or CD8 surface markers, fixed, permeabilized, and intracellularly stained for various cytokines as set forth in Tables 12 and 13.

The percent (%) of CD4+ and CD8+ T cells that were positive for specific intracellular cytokines are shown in Tables 12 and 13, respectively. The results showed that a number of the variant ICOSL ECD-Fc molecules were able to suppress one or more cytokines, including, in some cases, a majority of cytokines. A total score and mean score were calculated to assess the sum effects of individual molecules tested over the parameters examined in this assay. Proliferation was also assessed and the percentage of cells that divided as determined by CFSE dilution is also shown in Table 12 and 13. Among the provided results, the results show that certain variants show comparable or better activity than Belatacept, particularly from the CD8+ cells.

**Table 12. Assessment of Proliferation and Intracellular Cytokine levels of CD4+ T cells**

| | | % Cytokine + | | | | | | | | | |
|---|---|---|---|---|---|---|---|---|---|---|---|
| **Variant (ECD) or Protein Mutations** | **Proli f** | **% IFNg +** | **%IL4 +** | **% IL21 +** | **% IL22 +** | **%TNF +** | **%IL2 +** | **%IL10 +** | **% IL17A +** | **Tota l Scor e** | **Mea n Scor e** |
| V11E, N30D, N52H, N57Y, H94E, L96I, L98F, N194D, V210A, I218T | 3.0 | 9.9 | 3.0 | 1.0 | 1.3 | 34.9 | 31.3 | 0.1 | 0.2 | 41.0 | 4.6 |
| E16V, N52H, N57Y, Q100R, V110D, H115R, Y152C, K156M, C198R | 2.7 | 11.1 | 3.4 | 1.1 | 1.4 | 38.0 | 35.0 | 0.0 | 0.1 | 63.0 | 7.0 |
| N52H, N57Y, V110A, C198R, R221I | 2.9 | 10.9 | 3.3 | 1.1 | 1.5 | 37.3 | 34.1 | 0.1 | 0.2 | 65.0 | 7.2 |
| N52H, N57Y, Q100R, F172S | 3.2 | 8.1 | 2.5 | 0.6 | 1.1 | 27.9 | 24.1 | 0.9 | 1.3 | 66.0 | 7.3 |
| N52H, N57Y, Q100R | 3.3 | 9.2 | 3.0 | 0.8 | 1.4 | 31.1 | 26.3 | 0.8 | 1.3 | 70.0 | 7.8 |
| N52H, N57Y, Q100R,V110D, C198R, S212G | 3.4 | 10.9 | 3.3 | 1.0 | 1.6 | 36.5 | 32.3 | 0.5 | 0.9 | 89.0 | 9.9 |
| N207Q | 3.6 | 9.5 | 3.1 | 0.9 | 1.5 | 33.8 | 29.4 | 0.8 | 1.4 | 92.0 | 10.2 |
| N52H, N57Y, Q100R, L102R, V110D, H115R, C198R | 2.8 | 12.0 | 3.5 | 1.1 | 1.6 | 40.9 | 38.5 | 0.1 | 0.3 | 92.0 | 10.2 |
| CTLA-4-Ig: L104E, A29Y (Belatacept) | 10.7 | 10.5 | 2.7 | 2.4 | 2.0 | 24.5 | 19.6 | 0.6 | 1.4 | 99.0 | 11.0 |
| N52Y/N57Y/ F138L/L203P | 3.5 | 12.0 | 3.8 | 1.3 | 1.6 | 41.3 | 36.7 | 0.2 | 0.4 | 109. 0 | 12.1 |
| N52H, Q100R | 4.4 | 9.8 | 3.2 | 1.2 | 1.7 | 32.7 | 29.3 | 0.9 | 1.4 | 114. 0 | 12.7 |
| N52H/N57Y/Q100 P | 3.0 | 13.2 | 4.1 | 1.2 | 1.8 | 43.2 | 39.8 | 0.1 | 0.2 | 115. 0 | 12.8 |
| N52H, N57Y, Q100R, C198R | 3.6 | 11.3 | 3.9 | 1.0 | 2.0 | 39.1 | 34.6 | 0.8 | 1.3 | 118. 0 | 13.1 |
| WT ICOSL | 12.7 | 16.7 | 5.8 | 4.9 | 4.7 | 36.2 | 29.2 | 0.2 | 0.4 | 127. 0 | 14.1 |
| N52H/N57Y/Q100 P | 3.5 | 12.3 | 3.9 | 1.1 | 1.8 | 39.2 | 37.5 | 0.4 | 0.6 | 127. 0 | 14.1 |
| N52S, N194D | 10.9 | 16.0 | 7.2 | 4.9 | 5.4 | 40.6 | 33.0 | 0.1 | 0.2 | 135. 0 | 15.0 |
| N52H, N57Y, Q100R, V110D, N144D, F172S, C198R | 10.6 | 16.7 | 6.0 | 4.3 | 5.0 | 41.3 | 37.3 | 0.2 | 0.3 | 146. 0 | 16.2 |
| mIgG ct1 | 15.5 | 15.6 | 5.9 | 5.2 | 4.6 | 31.7 | 26.0 | 0.4 | 1.7 | 146. 0 | 16.2 |
| PDL2 | 12.3 | 17.9 | 5.7 | 4.7 | 5.2 | 41.4 | 36.0 | 0.3 | 0.6 | 163. 0 | 18.1 |
| N52S, E90A | 11.9 | 17.7 | 6.2 | 5.2 | 5.3 | 42.4 | 37.5 | 0.2 | 0.4 | 167. 0 | 18.6 |
| WT ICOSL | 12.8 | 17.4 | 6.3 | 5.4 | 5.6 | 38.9 | 32.1 | 0.3 | 0.6 | 167. 0 | 18.6 |

(continued)

Table 12. Assessment of Proliferation and Intracellular Cytokine levels of CD4+ T cells

| | | % Cytokine + | | | | | | | | | |
|---|---|---|---|---|---|---|---|---|---|---|---|
| Variant (ECD) or Protein Mutations | Proli f | % IFNg + | %IL4 + | % IL21 + | % IL22 + | %TNF + | %IL2 + | %IL10 + | % IL17A + | Tota l Scor e | Mea n Scor e |
| Anti-ICOSL | 15.5 | 16.3 | 6.5 | 5.2 | 5.5 | 35.1 | 29.1 | 0.7 | 2.0 | 168. 0 | 18.7 |
| N52H/I143T | 12.6 | 17.4 | 6.5 | 5.4 | 4.9 | 44.3 | 37.2 | 0.4 | 0.5 | 179. 0 | 19.9 |
| HuIgG | 12.7 | 17.1 | 6.3 | 5.9 | 4.9 | 41.1 | 32.4 | 0.7 | 1.3 | 181. 0 | 20.1 |
| Anti-CD28 | 88.2 | 42.9 | 5.0 | 5.8 | 5.4 | 43.5 | 25.5 | 0.4 | 1.4 | 183. 0 | 20.3 |
| N52S, F120S, I143V, I224V | 12.7 | 18.7 | 6.5 | 5.4 | 5.6 | 44.2 | 40.0 | 0.1 | 0.3 | 186. 0 | 20.7 |
| N52D | 13.7 | 18.3 | 6.8 | 5.9 | 6.1 | 42.1 | 35.2 | 0.3 | 0.5 | 194. 0 | 21.6 |

**Table 13. Assessment of Proliferation and Intracellular Cytokine levels of CD8+ T cells**

| | | % Cytokine + | | | | | | | | | |
|---|---|---|---|---|---|---|---|---|---|---|---|
| **Variant (ECD) or Protein Mutations** | **Proli f** | **% IFNg +** | **%IL4 +** | **% IL21 +** | **% IL22 +** | **%TNF +** | **%IL2 +** | **%IL10 +** | **% IL17A +** | **Tota l Scor e** | **Mea n Scor e** |
| V11E, N30D, N52H, N57Y, H94E, L96I, L98F, N194D, V210A, I218T | 4.2 | 8.4 | 1.6 | 2.2 | 47.2 | 7.6 | 8.0 | 0.2 | 0.1 | 67.0 | 7.4 |
| E16V, N52H, N57Y, Q100R, V110D, H115R, Y152C, K156M, C198R | 3.8 | 8.5 | 2.0 | 2.2 | 47.2 | 8.1 | 9.1 | 0.1 | 0.0 | 69.0 | 7.7 |
| N52H, N57Y, V110A, C198R, R221I | 3.9 | 8.8 | 2.0 | 1.7 | 45.6 | 8.4 | 9.0 | 0.1 | 0.1 | 64.0 | 7.1 |
| N52H, N57Y, Q100R, F172S | 3.8 | 7.0 | 1.4 | 1.8 | 46.6 | 5.9 | 5.9 | 1.1 | 1.1 | 78.0 | 8.7 |
| N52H, N57Y, Q100R | 4.3 | 8.4 | 1.9 | 1.7 | 50.2 | 7.1 | 6.6 | 1.1 | 1.1 | 98.0 | 10.9 |
| N52H, N57Y, Q100R,V110D, C198R, S212G | 4.1 | 9.0 | 1.8 | 1.8 | 46.4 | 8.2 | 8.5 | 0.7 | 0.8 | 87.0 | 9.7 |
| N207Q | 4.1 | 7.9 | 1.8 | 1.8 | 49.8 | 7.3 | 7.2 | 1.0 | 1.2 | 93.0 | 10.3 |
| N52H, N57Y, Q100R, L102R, V110D, H115R, C198R | 3.5 | 9.0 | 1.7 | 2.0 | 47.3 | 8.6 | 10.4 | 0.2 | 0.2 | 78.0 | 8.7 |
| CTLA-4-Ig: L104E, A29Y (Belatacept) | 12.3 | 14.5 | 2.0 | 3.2 | 38.1 | 11.2 | 8.8 | 0.9 | 1.6 | 121. 0 | 13.4 |
| N52Y/N57Y/ F138L/L203P | 4.2 | 9.6 | 1.9 | 1.8 | 40.4 | 9.4 | 9.9 | 0.3 | 0.3 | 81.0 | 9.0 |
| N52H, Q100R | 5.4 | 9.5 | 2.0 | 2.7 | 44.2 | 7.8 | 8.4 | 1.2 | 1.3 | 112. 0 | 12.4 |
| N52H/N57Y/Q100 P | 3.7 | 9.5 | 1.9 | 1.3 | 44.4 | 9.4 | 10.5 | 0.1 | 0.1 | 62.0 | 6.9 |
| N52H, N57Y, Q100R, C198R | 4.1 | 9.6 | 2.3 | 1.8 | 46.8 | 9.0 | 9.3 | 0.9 | 1.0 | 122. 0 | 13.6 |
| ICOSL | 17.2 | 22.3 | 4.9 | 6.4 | 46.4 | 22.0 | 15.7 | 0.4 | 0.7 | 181. 0 | 20.1 |
| N52H/N57Y/Q100 P | 4.2 | 9.5 | 2.0 | 2.1 | 45.6 | 8.9 | 10.7 | 0.5 | 0.5 | 110. 0 | 12.2 |
| N52S, N194D | 14.5 | 19.4 | 5.6 | 4.8 | 48.4 | 19.2 | 13.8 | 0.1 | 0.2 | 142. 0 | 15.8 |
| N52H, N57Y, Q100R, V110D, N144D, F172S, C198R | 13.4 | 18.9 | 4.3 | 5.0 | 46.3 | 18.4 | 15.4 | 0.3 | 0.6 | 138. 0 | 15.3 |
| mIgG ct1 | 20.2 | 25.0 | 4.4 | 4.1 | 35.5 | 24.6 | 15.8 | 0.7 | 1.8 | 174. 0 | 19.3 |
| PDL2 | 15.6 | 21.2 | 4.1 | 4.8 | 44.1 | 20.7 | 15.6 | 0.5 | 0.8 | 147. 0 | 16.3 |
| N52S, E90A | 15.4 | 20.8 | 4.7 | 5.6 | 44.9 | 20.6 | 17.1 | 0.2 | 0.5 | 149. 0 | 16.6 |
| WT ICOSL | 17.5 | 22.1 | 5.5 | 4.6 | 45.0 | 21.0 | 12.8 | 0.2 | 0.4 | 148. 0 | 16.4 |

(continued)

**Table 13. Assessment of Proliferation and Intracellular Cytokine levels of CD8+ T cells**

| | | % Cytokine + | | | | | | | | | |
|---|---|---|---|---|---|---|---|---|---|---|---|
| **Variant (ECD) or Protein Mutations** | **Proli f** | **% IFNg +** | **%IL4 +** | **% IL21 +** | **% IL22 +** | **%TNF +** | **%IL2 +** | **%IL10 +** | **% IL17A +** | **Tota l Scor e** | **Mea n Scor e** |
| Anti-ICOSL | 21.5 | 26.4 | 4.8 | 5.3 | 33.4 | 26.9 | 19.0 | 1.0 | 2.3 | 198. 0 | 22.0 |
| N52H/I143T | 17.2 | 22.2 | 4.7 | 6.7 | 39.4 | 22.5 | 18.1 | 0.8 | 0.9 | 178. 0 | 19.8 |
| HuIgG | 15.9 | 21.5 | 4.4 | 6.4 | 41.6 | 21.4 | 15.1 | 1.4 | 1.7 | 179. 0 | 19.9 |
| Anti-CD28 | 60.6 | 44.3 | 3.5 | 2.1 | 38.6 | 32.5 | 16.0 | 1.2 | 1.6 | 182. 0 | 20.2 |
| N52S, F120S, I143V, 1224V | 16.5 | 22.0 | 4.9 | 5.4 | 44.0 | 21.9 | 18.5 | 0.2 | 0.6 | 161. 0 | 17.9 |
| N52D | 17.7 | 22.8 | 5.3 | 6.1 | 45.4 | 22.9 | 16.7 | 0.4 | 0.6 | 183. 0 | 20.3 |

## EXAMPLE 6

### Assessment of Cytokine Production in B-T cell Co-culture

B cells and CD4+ T cells were purified from the same donor and labeled with CSFE and plated in 96 well plates in 1:1 cellular ratios at 5 x $10^4$ cells of each per well. Variant ICOSL ECD-Fc fusion molecules or Belatacept were added at a final concentration of 40 nM per well. Cells were either unstimulated or incubated with 100 ng/mL of Staphylococcus enterotoxin B (SEB), 1 μg/mL of Pokeweed Mitogen (PWM) or both for 7 days at 37°C in a final volume of 200 μl/well.

Cells were harvested and surface stained for the following B and T cell lineage markers (IgM, IgD, CD38, CD138, CD27, CD19, CD4, CD3). Proliferation was assessed by flow cytometry and culture supernatants were analyzed for IL-5, IL-13 or IL-21 cytokines using a LEGENDplex human Th cytokine detection kit (Biolegend, USA).

As shown in **FIG. 5A,** the number of dividing B cells was reduced in B/T cell cocultures when incubated in the presence of variant ICOSL ECD Fc-fusion molecules compared to no protein controls. The degree of antagonistic effect for the tested exemplary variants was similar to CTLA-4-Ig Belatacept (L104E, A29Y). Likewise, as shown in **FIGS. 5B-5D,** variant ICOSL ECD Fc-fusion molecules inhibited cytokine production in primary human B cell/T cell coculture *in vitro* compared to no protein controls as well as cultures containing wild-type ICOSL control. Compared to Belatacept, exemplary tested variant ICOSL ECD Fc-fusion molecules were more effective in blocking cytokine production in some cases.

## EXAMPLE 7

### Assessment of Survival and Disease Activity in Graft-Versus-Host-Disease (GvHD) Model

Exemplary ICOSL variant ECD-Fc proteins were assessed for activity in a graft-versus-host-disease (GvHD) model. Female NSG mice (n = 10 per group) were irradiated (100 rad) and administered 10 mg of gamma globulin subcutaneously on Day -1. On Day 0, the mice received 10 million human peripheral blood mononuclear cells (PBMCs) and intraperitoneal injection dosing of either 100 μg of WT-ICOSL ECD Fc, a variant ICOSL ECD Fc molecule N52H/I143T (ECD set forth in SEQ ID NO: 61), a variant ICOSL ECD Fc molecule N52H/N57Y/Q100P (ECD set forth in SEQ ID NO: 62), 75μg of Belatacept (CTLA-4-Ig L104E/A29Y; U.S. Patent Application Publication Number US20160271218) or saline as control. On Day 15, engrafted human CD45+ cells were phenotyped by flow cytometry. After the study was terminated on Day 35, endpoint measurements of survival, body weight loss, and disease activity were evaluated.

**FIG. 6A** shows the survival of GVHD mice treated with saline, WT ICOSL-ECD Fc, the variant ICOSL ECD-Fc molecules, or Belatacept. A significant difference in the survival of mice administered variant ICOSL ECD- Fc N52H/N57Y/Q100P (ECD set forth in SEQ ID NO: 62) compared to mice administered saline or WT ICSOL ECD-Fc was observed ($p < 0.0001$ by Mantel-Cox and Gehan-Breslow-Wilcoxon tests). **FIG. 6B** shows similar differences between the body weight loss of mice treated with saline, WT ICOSL ECD- Fc, the variant ICOSL ECD-Fc molecules, or Belatacept over the course of the study.

A disease activity index (DAI) was determined three times a week during the study and evaluated the scoring of weight loss, posture, activity, appearance of the hair coat and skin of the mice. The grade of disease over the course of the study is shown in **FIG. 6C.** Treatment groups that received ICOSL Fc variant N52H/N57Y/Q100P (ECD set forth SEQ ID NO: 62) or Belatacept showed significantly improved DAI scores. The percentage of human T cells in the peripheral blood on day 14 of the study was also assessed by flow cytometry. Measurements were averaged by treatment group and error bars represent standard error of the mean (SEM). **FIG. 6D** shows the percent of live CD3+/CD4+ or CD3+/CD8+ cells in the blood. Treatment groups that received variant ICOSL ECD Fc with N52H/N57Y/Q100P (ECD set forth SEQ ID NO: 62) or Belatacept showed significantly different levels of CD4+ T cells compared to saline treatment group ($p = 0.008$ and 0.006, respectively, by unpaired t-test). This study demonstrates the therapeutic effect of the variant ICOSL Fc variant protein on human primary T cells and GVHD during *in vivo* modeling.

A similar study with additional variant ICOSL ECD-Fc fusion molecules was carried out including variant ICOSL ECD- Fc N52H/N57Y/Q100R/C198R (ECD set forth in SEQ ID NO: 63), N52H/N57Y/Q100R/F172S(ECD set forth in SEQ ID NO:64 ), and N52H/N57Y/Q100P (ECD set forth in SEQ ID NO: 65). N52H/Q100R (ECD set forth in SEQ ID NO: 66), which had reduced activity in the in vitro MLR studies as shown above. NSG mice (n=9/group) were treated as described above. In this study, dosing with the ICOSL-Fc or Belatacept continued 3 times per week from day 0 through day 37 and surviving mice were terminated on day 49. To determine statistical differences in survival proportions among groups, data were analyzed using the Mantel-Cox (log-rank) test. The resulting survival curves are shown in **FIG. 6E.** Belatacept and ICOSL-Fc variants N52H/N57Y/Q100R/C198R (ECD set forth in SEQ ID NO: 63), N52H/N57Y/Q100R/F172S (ECD set forth in SEQ ID NO: 64), and N52H/N57Y/Q100P (ECD set forth in SEQ ID NO: 65) significantly prolonged survival as compared to saline and WT ICOSL-Fc ($p<0.001$). The mean DAI scores were plotted for the time course of the experiment, with the last observation (i.e. mean scores collected on days of termination) carried forward on the graph for those groups terminated prior to the last planned study day (Day 49). Significant differences among groups for data over time (i.e. DAI scores) were determined using 2-way repeated measures ANOVA for 'treatment' effects. The resulting DAI scores are shown in **FIG. 6F.** Belatacept and ICOSL-Fc variants N52H/N57Y/Q100R/C198R (ECD set forth in SEQ ID NO: 63), N52H/N57Y/Q100R/F172S (ECD set forth in SEQ ID NO: 64), and N52H/N57Y/Q100P (ECD set forth in SEQ ID NO: 65) significantly reduced DAI scores as compared to saline and WT ICOSL-Fc (p<0.001) , and between Belatacept and ICOSL-Fc variant with N52H/N57Y/Q100R/C198R by 2-way ANOVA for DAI scores (p = 0.035).

The activity of the variant ICOSL-Fc, in some cases, was improved compared to treatment with belatacept. Administration of ICOSL-Fc variants protected from effects of GVHD, as evidenced by enhanced survival and attenuated disease development, at levels comparable to or better than belatacept. The activity in this model correlated with in vitro activity, since WT ICOSL-Fc and variant ICOSL-Fc with mutations N52H/Q100R (ECD set forth in SEQ ID NO: 66) were not as effective in this model.

**EXAMPLE** 8

**Assessment of Delayed Type Hypersensitivity *in vivo***

Variant ICOSL ECD-Fc fusion molecules were assessed for anti-inflammatory activity in vivo in the mouse delayed-type hypersensitivity (DTH) model. Delayed-type hypersensitivity immune reactions were elicited in ovalbumin (OVA)-sensitized mice and response after challenge was assessed. The variant ICOSL ECD-Fc fusion molecules tested contained a variant ECD with the following amino acid substitutions: N52H/N57Y/Q100P (ECD set forth in SEQ ID NO: 62), N52H/Q100R (ECD set forth in SEQ ID NO: 66), or N52H/N57Y/Q100R/F172S (ECD set forth in SEQ ID NO: 64). The variants were fused to either an Fc backbone containing mutations C220S/L234A/L235E/G237A by EU numbering (designated Fc#1) set forth in SEQ ID NO: 40 or an Fc backbone containing mutations C220S/E233P/L234V/L235A/G236del/S267K by EU numbering (designated Fc#2) set forth in SEQ ID NO:44 either with or without a G4S (GGGGS; SEQ ID NO: 52) linker. Table 14 sets forth the tested constructs:

| Table 14: ICOSL ECD-Fc Fusion Constructs | | |
|---|---|---|
| | G4S Linker | Fc (SEQ ID NO) |
| N52H/N57Y/Q100P (G4S)-Fc #1 | + | 40 |
| N52H/Q100R (G4S)-Fc #1 | + | 40 |
| N52H/N57Y/Q100R/F172S (G4S)-Fc | + | 44 |
| N52H/N57Y/Q100R/F172S (G4S)-Fc | + | 40 |
| N52H/N57Y/Q100R/F172S-Fc | - | 40 |

For sensitization, 8-week old female BALB/c mice were injected subcutaneously with 100 μg of OVA emulsified in Sigma Adjuvant (100 μL; catalog number S6322-1VL) at the base of the tail on day 0. On days 1 and 4, the mice were administered variant ICOSL ECD-Fc fusion proteins, 75 μg of CTLA-4 Fc (abatacept), or PBS as negative control by intraperitoneal injection. On day 7 at two to three hours prior to OVA challenge, the mice were further administered PBS as a control, 75μg of CTLA-4 Fc (abatacept from Orencia), or the indicated variant ICOSL polypeptide by intraperitoneal injection. Abatacept and variant ICOSL-Fc fusion molecules were dosed at molar equivalents.

For OVA challenge, an intradermal injection of 10 μg OVA in the left ear pinnae in a 10 μL volume of PBS was delivered under gas isoflurane anesthesia 2-3 hours following therapeutic treatment. Baseline ear thickness was measured prior to OVA challenge.

On day 8, ear thickness was measured under isoflurane anesthesia using Mitutoyo calipers and the change in ear thickness before and after OVA challenge was determined. As shown in **FIG. 7,** mice treated with the indicated variant ICOSL ECD-Fc fusion molecules showed significantly less OVA-induced ear swelling as compared to PBS control (< 0.0001 by 1-way ANOVA). There were no significant differences between ear thickness for mice treated with abatacept compared to any of the indicated variant ICOSL ECD-Fc fusion molecules tested or between the variant ICOSL treatments. These results demonstrate that variant ICOSL molecules can reduce immune responses *in vivo*.

## EXAMPLE 9

### Nanostring Transcriptional Signature of Primary Human T cells

Tissue culture plates were coated with 10 nM anti-CD3 with 40 nM of an Fc-control protein, wild-type ICOSL-Fc, wild-type CD80-Fc, both of these proteins, or variant ICOSL Fc-fusion proteins with mutations as indicated. Purified human T cells were then plated on the protein coated plates and incubated at 37°C. Cultures from each treatment group described above were harvested at 24, 48 and 72 hours and total RNA was prepared from each cell sample. The RNA was transferred to Nanostring and a Cancer Immune chip was used to quantitate transcripts of 750 gene in each sample. Transcript values were normalized using Nanostring's proprietary software allowing comparison of transcript levels between treatment groups and over the various time points. As shown in **FIG. 8** and **FIG. 9,** the variant ICOSL ECD-Fc polypeptides tested show altered inflammatory activity compared to wildtype CD80 ECD-Fc, wildtype ICOSL ECD-Fc, or a combination of both.

## EXAMPLE 10

### Generation and Assessment of Fc-Fusion Immunomodulatory Proteins

Variant ICOSL IgSF (e.g. ECD) domain-containing molecules were formatted as Fc-fusion proteins substantially as described in Example 1, except using various linkers and Fc molecules. To generate immunomodulatory proteins that are Fc fusion proteins containing an ECD of ICOSL with at least one affinity-modified domain (e.g. variant ICOSL ECD-Fc), the encoding nucleic acid molecule was generated to encode a protein designed as follows: variant (mutant) ECD linked directly or indirectly via a linker to an inert human IgG1 Fc. Specifically, the generated immunomodulatory proteins either did not contain a linker (none) or contained a AAA or a G4S (SEQ ID NO: 52) linker. The inert human IgG1 Fc contained mutations, by EU numbering, as follows: C220S/R292C/N297G/V302C (SEQ ID NO: 43), C220S/E233P/L234V/L235A/G236del/S267K (SEQ ID NO: 44), C220S/L234A/L235E/G237A (SEQ ID NO: 40), or allotypes thereof. The replacement C220S was included because the resulting proteins do not include an antibody light chain that can form a covalent bond with a cysteine. The recombinant variant Fc fusion proteins were produced in 293 cells and purified with Protein A substantially as described in Example 1.

The variant ICOSL Fc-fusion immunomodulatory proteins were assessed in binding studies to assess binding for cognate binding partners. Expi293 cells transfected with cognate binding partners, human CD28, ICOS and CTLA4, were used as target cells in binding studies. MFI of binding of variant ICOSL Fc fusion immunomodulatory proteins for target cell expressing each binding partner was determined and compared to the binding of the corresponding unmodified (wildtype) ICOSL ECD-Fc to the same target cells. Modulation of T cell activity by the variant ICOSL Fc-fusion immunomodulatory proteins was also determined using a mixed lymphocyte reaction (MLR) substantially as described in Example 1.

Results for the binding of exemplary variant ICOSL ECD-Fc fusion immunomodulatory proteins containing various linkers and Fc regions are shown in Table 15. The Table indicates amino acid substitutions in the ECD of the variant ICOSL as designated by amino acid position number corresponding to amino acid positions in the respective reference (e.g., unmodified) ICOSL extracellular domain (ECD) sequence set forth in SEQ ID NO:1. Column 2 indicates the linker used in the Fc fusion protein and the SEQ ID NO identifier for the linker. Column 3 sets forth the mutations in the Fc by EU numbering and the SEQ ID NO identifier for the Fc contained in the variant ICOSL Fc fusion protein.

As shown in Table 15, similar results were observed for binding to cognate binding partners among the tested variant ICOSL Fc fusion proteins. These results indicate that the format of the Fc fusion with different Fc molecules or different linkers did not impact the binding of the ICOSL IgSF domain variants for their cognate binding partner. In addition, all Fc fusion formats, when provided as bivalent Fc molecules in solution in an MLR reaction, exhibited an antagonist activity to decrease T cell activation compared to the reference (e.g. unmodified or wildtype) ECD-Fc molecule not containing the amino acid substitution. In some cases, no detectable IFN-gamma was measured in the supernatant consistent with complete blocking of interactions of costimulatory ligand cognate binding partners with their ligands to induce IFN-gamma secretion.

| Table 15: Molecule sequences, binding data, and costimulatory bioactivity data of variant ICOSL ECD-Fc molecules | | | | | | |
|---|---|---|---|---|---|---|
| ICOSL Mutation (s) | Linker (SEQ ID NO) | Fc Mutations (SEQ ID NO) | Binding | | | MLR |
| | | | CD28 | CTLA-4 | ICOS | IFN-gamma |
| | | | MFI (ΔWT) | MFI (ΔWT) | MFI (ΔWT) | pg/mL (ΔWT) |
| N52H/N57Y/Q100R/F172S | AAA | C220S/R292C/N297G/V302C (43) | 67870 (13.0) | 213333 (22.7) | 120042 (1.5) | 3 (0.02) |
| N52H/N57Y/Q100R/F172S) | AAA | C220S/E233P/L234V/L235A/G236del /S267K (44) | 57272 (10.9) | 192595 (20.5) | 103983 (1.3) | 0 (0.00) |
| N52H/N57Y/Q100R/F172S | AAA | C220S/L234A/L235E/G237A (40) | 65506 (12.5) | 193704 (20.6) | 105432 (1.3) | 0 (0.00) |
| N52H/N57Y/Q100R/F172S | G4S (52) | C220S/E233P/L234V/L235A/G236del /S267K (44) | | | | |
| N52H/N57Y/Q100R/F172S | G4S (52) | C220S/L234A/L235E/G237A (40) | 67596 (12.9) | 212875 (22.7) | 106576 (1.4) | 0 (0.00) |
| N52H/N57Y/Q100R/F172S | none | C220S/E233P/L234V/L235A/G236del /S267K (44) | - | - | - | - |
| N52H/N57Y/Q100R/F172S | none | C220S/L234A/L235E/G237A (40) | 59987 (11.5) | 210061 (22.4) | 106405 (1.4) | 0 (0.00) |
| N52H/Q100R | AAA | C220S/R292C/N297G/V302C (43) | 57419 (11.0) | 190012 (20.2) | 86522 (1.1) | 30 (0.26) |
| N52H/Q100R | AAA | C220S/E233P/L234V/L235A/G236del /S267K (44) | | | | |
| N52H/Q100R | AAA | C220S/L234A/L235E/G237A (40) | 58772 (11.2) | 211494 (22.5) | 88969 (1.1) | 25 (0.22) |
| N52H/Q100R | G4S (52) | C220S/E233P/L234V/L235A/G236del /S267K (44) | 62331 (11.9) | 207285 (22.1) | 110512 (1.4) | 31 (0.28) |
| N52H/Q100R | G4S (52) | C220S/L234A/L235E/G237A (40) | 70142 (13.4) | 187699 (20.0) | 125505 (1.6) | 49 (0.44) |
| N52H/Q100R | none | C220S/E233P/L234V/L235A/G236del /S267K (44) | 58726 (11.2) | 206110 (21.9) | 110721 (1.4) | 54 (0.48) |
| N52H/Q100R | none | C220S/L234A/L235E/G237A (40) | 62746 (12.0) | 198281 (21.1) | 96948 (1.2) | 16 (0.14) |
| N52H/N57Y/Q100P | AAA | C220S/R292C/N297G/V302C (43) | 79792 (15.3) | 193633 (20.6) | 91384 (1.2) | 1 (0.01) |
| N52H/N57Y/Q100P | AAA | C220S/E233P/L234V/L235A/G236del /S267K (44) | 69603 (13.3) | 314593 (33.5) | 103387 (1.3) | 0 (0.00) |
| N52H/N57Y/Q100P | AAA | C220S/L234A/L235E/G237A (40) | 68729 (13.1) | 171223 (18.2) | 97068 (1.2) | 0 (0.00) |
| N52H/N57Y/Q100P | G4S (52) | C220S/E233P/L234V/L235A/G236del /S267K (44) | 67753 (13.0) | 188192 (20.0) | 93424 (1.2) | 1 (0.01) |
| N52H/N57Y/Q100P | G4S (52) | C220S/L234A/L235E/G237A (40) | 69887 (13.4) | 160705 (17.1) | 104124 (1.3) | 0 (0.00) |
| N52H/N57Y/Q100P | None | C220S/E233P/L234V/L235A/G236del /S267K (44) | 68979 (13.2) | 184726 (19.7) | 98512 (1.3) | 0 (0.00) |
| N52H/N57Y/Q100P | none | C220S/L234A/L235E/G237A (40) | 67863 (13.0) | 154563 (16.5) | 97714 (1.2) | 0 (0.00) |
| WT | AAA | C220S/R292C/N297G/V302C (43) | 5232 (1.0) | 9394 (1.0) | 78795 (1.0) | 113 (1.00) |

## EXAMPLE 11

### Expression of Variant ICOSL Molecules in CHO cells

As an alternative to expressing variant ICOSL Fc fusion proteins in Expi293 cells as described in Example 2, suspension Chinese hamster ovary cells (ExpiCHO-S) cells were used to produce various ICOSL molecules. A DNA construct encoding the exemplary variant ICOSL IgSF (e.g. ECD) Fc-fusion proteins containing the variant (mutant) ECD N52H/N57Y/Q100R/F172S (SEQ ID NO:64) linked to an inert Fc containing mutations C220S/L234A/L235E/G237A by EU numbering set forth in SEQ ID NO: 40 or an allotype thereof set forth in SEQ ID NO: 42, with a GSGGGGS linker (SEQ ID NO: 58) was used to transfect cells.

ExpiCHO-S cells, and reagents for transfection using the ExpiCHO™ Expression System, were purchased from ThermoFisher Scientific (Cat #A29133). The cells were thawed and expanded per manufacture's recommended protocol. After at least 2 passages, the cells were split 24 hours pre-transfection and allowed to expand to high density. Cells were then diluted to the number of cells for transfection, DNA complex was formed with the ExpiFectamine™ CHO reagent and added to the cells. One day post DNA complex addition the ExpiCHO™ feed and ExpiFectamine™ CHO Enhancer were added to the culture, which was then placed in a 32 degree Celsius incubator. The cell viability and cell mass were monitored and the culture was harvested when the viability fell below 80%. The culture was then centrifuged at low speed to remove the cell pellet, and the cleared supernatant was 0.2 μm sterile filtered. Protein was purified as described in Example 1.

### A. Protein Analysis

The purified variant ICOSL Fc fusion protein was run on SDS-PAGE and analyzed by protein staining. Multiple bands were observed in cells produced from CHO cells but not from 293 cells, which is consistent with an observation that proteolysis clipping of the ICOSL was occurring when expressed in CHO cells. Table 16 depicts the molecular weight of intact, single-clipped, and double clipped proteins calculated based on amino acid sequences and potential carbohydrates as observed by SDS-PAGE. Proteolysis of the ICOSL Fc-fusion proteins expressed in ExpiCHO-S derived cells was observed, as indicated by the presence of both reduced/non-reduced clipped species with lower molecular weight (single and double clipping). Based on the size of the observed bands and Mass Spectromety analysis, these results are consistent with a potential cleavage site in ICOSL ECD corresponding to the sequence LQQN/LT ("/" indicates potential cleavage site), thereby resulting in cleavage before the stalk region of the ECD and removal of the Fc portion of the sequence in one or both chains of the Fc fusion protein. The observed protease cleavage may result in a heterogenous protein product when produced in CHO cells. Also, for formats expressed as transmembrane immunomodulatory proteins, protease cleavage, occurring in certain cells, could lead to release of soluble protein from cells, thereby reducing cell surface-expressed forms of the variant protein on engineered cells.

**Table 16: Reduced/Non-Reduced Species Detected After Capture and Elution from Protein A Column Chromatography Using SDS-PAGE to Assay Proteolysis**

| | Calculated MW | | Observed (Apparent) MW[1] | |
|---|---|---|---|---|
| | **Non Reduced** | **Reduced** | **Non Reduced** | **Reduced** |
| Intact | 105.6 kD aa<br>~29 kD carb<br>135 kD total | 52.8 kD aa<br>~14.5 kD carb<br>67.3 kD total | 150 kD | 80 kD |
| Single Clip | 78.8 kD aa<br>~16.5 kD carb<br>95.3 kD total | 52.8 kD aa<br>~14.5 kD carb<br>67.3 kD total<br>26 kD aa<br>~2 kd carb<br>28 kD total | 100 kD | 80 kD<br>37 kD |
| Double Clip | 52.1 kD aa<br>~4 kD carb<br>56.1 kdD total | 26 kD aa<br>~2 kd carb<br>28 kd total | 65 Kd | 37 kD |

(continued)

| Table 16: Reduced/Non-Reduced Species Detected After Capture and Elution from Protein A Column Chromatography Using SDS-PAGE to Assay Proteolysis | | | | |
|---|---|---|---|---|
| | **Calculated MW** | | **Observed (Apparent) MW[1]** | |
| | **Non Reduced** | **Reduced** | **Non Reduced** | **Reduced** |
| [1]Estimated MW from SDS-PAGE relative to Protein MW Markers | | | | |

**EXAMPLE 12**

**Generation of Proteolysis-Resistant Variants of ICOSL IgSF Domain-Containing Molecules**

To render variant ICOSL polypeptides resistant to proteolysis upon expression in cells, such as in CHO cells, various additional forms of variant ICOSL polypeptides were generated. The following additional modified reference sequences of the ICOSL ECD were generated : (1) various ECD truncations lacking all or a portion of the LQQN/LT protease cleavage site (designated Trunc #4, #5, #6, #7, or #8); (2) ICOSL variant reference sequences containing mutations at cleavage site N207 and/or L208 with reference to positions set forth in SEQ ID NO:1; or an ICOSL alone IgV reference sequence containing the IgV domain as the only IgSF domain of the molecule (set forth in SEQ ID NO: 3, corresponding to amino acids 1-122 of SEQ ID NO:1). In some cases, combinations of the above strategies were employed in a ICOSL ECD reference sequence. Table 17 below sets forth various generated reference sequences.

The exemplary mutations N52H/N57Y/Q100R/F172S, with reference to numbering set forth in SEQ ID NO: 1, were introduced into the various reference sequences. Because the reference ICOSL IgV set forth in SEQ ID NO: 3 does not contain a position corresponding to F172S, the variant ICOSL IgV did not contain the mutation F172S. The generated variant ICOSL polypeptides were formatted as an Fc fusion protein containing the generated reference ICOSL IgSF domain linked via a $(G_4S)2$ linker (SEQ ID NO:53) to an inert Fc containing mutations C220S/L234A/L235E/G237A/K447del by EU numbering set forth in SEQ ID NO: 39, or an allotype thereof set forth in SEQ ID NO:42.

| Table 17: Exemplary ICOSL IgSF-containing domain reference sequences | |
|---|---|
| **Reference ICOSL Sequence (SEQ ID NO)** | ...--------- **ECD** ---------------\|-------- *Stalk* ------------------------------------- |
| Full ECD (1) | **...VNIGCCIENVLLQQNLT***VGSQTVGNDIGERDKITENPVSTGEKNAAT* |
| Truncation #2 (5) | **...VNIGCCIENVLLQQNL** |
| Truncation #3 (6) | ...**VNIGCCIENVLLQQNLT***VGSQ* |
| Truncation #4 (7) | **...VNIGCCIENVLLQQN** |
| Truncation #5 (8) | **...VNIGCCIENVLLQQ** |
| Truncation #6 (9) | **...VNIGCCIENVLL** |
| Truncation #7 (10) | **...VNIGCCIEN** |
| Truncation #8 with N207G/L208G (28) | **...VNIGCCIENVLLQQGGT** |
| ECD with N207A (29) | ...**VNIGCCIENVLLQQALTV***GSQTGNDIGERDKITENPVSTGEKNAAT* |
| ECD with N207G/L208G (33) | **...VNIGCCIENVLLQQGGTV***GSQTGNDIGERDKITENPUSTGEKNAAT* |
| IgV (3) | **...HVAANFSV** |

**A. Proteolysis Assessment**

DNA constructs encoding the variant ICOSL Fc-fusion molecules described above were transfected into Chinese hamster ovary cells (ExpiCHO-S). The ICOSL Fc-fusion proteins were then purified from supernatants with Protein A by affinity chromatograpy as described in Example 2. Purified protein was analyzed by analytical SEC.

By SEC, intact protein displayed as a single peak while clipped protein displayed as multiple peaks, including lower molecular weight species. Consistent with the SDS-PAGE results described in Example 11, proteolysis as assessed by SEC was observed when variant ICOSL ECD Fc-fusion protein was expressed in ExpiCHO-S derived cells, as indicated by the multiple peaks shown in **FIG. 10A.** As shown in **FIGS. 10B-10G,** single peaks were observed by SEC analysis of the variant ICOSL Fc fusion proteins generated using modified reference ICOSL polypeptides in which the putative ECD protease cleavage site was removed or mutated, indicating reduced cleavage of the proteins occurred. In one purification lot, however, lower molecular weight species were observed by SEC analysis of the variant ICOSL Fc fusion protein generated using the reference ICOSL polypeptide set forth in SEQ ID NO: 9 (Trunc. #5), although it was not clear the reason for the presence of these species in this lot. As shown in **FIG. 10G,** the generation of lower molecular weight species, and hence proteolysis, also was not observed by SEC analysis of the variant ICOSL Fc-fusion proteins generated using the ICOSL IgV alone reference sequence.

**B. Binding and Activity**

Binding and activity of protein produced and purified following transfection of DNA constructs encoding the variant ICOSL Fc-fusion immunomodulatory proteins in various reference sequences described above in CHO cells was compared. In some cases, purified clones as assessed below were later found to contain additional mutations beyond those described above, which were not believed to impact the immunomodulatory activity of the tested proteins.

The resulting purified variant ICOSL Fc-fusion immunomodulatory proteins were assessed for binding to cognate binding partners and for modulation of T cell activity using a mixed lymphocyte reaction (MLR) substantially as described above. Table 18 indicates amino acid substitutions in the ICOSL reference sequence as designated by amino acid position number corresponding to amino acid positions in the respective reference (e.g., unmodified) ICOSL extracellular domain (ECD) sequence set forth in SEQ ID NO: 1, and sets forth the SEQ ID NO identifier for each ICOSL reference sequence. As shown, the binding and MLR antagonist activity was generally similar for all tested formats.

**Table 18: Molecule sequences, binding data, and costimulatory bioactivity data of variant ICOSL molecules**

| Description | Reference Sequence SEQ ID NO | Binding | | | MLR |
|---|---|---|---|---|---|
| | | CD28 | CTLA-4 | ICOS | IFN-gamma |
| | | MFI (ΔWT) | MFI (ΔWT) | MFI (ΔWT) | pg/mL (ΔWT) |
| ICOSL ECD Truncation #7 with N52H/N57Y/Q100R/F172S and F83S | 10 | 88329 (51.1) | 206566 (27.3) | 106493 (1.1) | 12 (0.02) |
| ICOSL ECD Truncation #6 with N52H/N57Y/Q100R/F172S | 9 | 91273 (52.8) | 239746 (31.6) | 90074 (0.9) | 14 (0.02) |
| ICOSL ECD Truncation #4 with N52H/N57Y/Q100R/F172S and E200G | 7 | 80555 (46.6) | 320229 (42.3) | 107957 (1.1) | 9 (0.01) |
| ICOSL ECD Truncation #4 with N52H/N57Y/Q100R/F172S | 7 | 68599 (39.7) | 377254 (49.8) | 132880 (1.3) | 2 (0.00) |
| ICOSL ECD Truncation #8 with N207G/L208G and N52H/N57Y/Q100R/F172S | 11 | 107837 (62.4) | 308427 (40.7) | 132654 (1.3) | 8 (0.01) |
| ICOSL IgV with N52H/N57Y/Q100R and H48R/S54P | 3 | 75304 (43.6) | 321613 (42.4) | 143141 (1.4) | 995 (1.31) |
| ICOSL ECD with N52H/N57Y/Q100R/F172S | 1 | 110407 (63.9) | 323219 (42.6) | 136060 (1.4) | 0 (0.00) |
| ICOSL ECD Truncation #7 with N52H/N57Y/Q100R/C198R and E90K/E111G | 10 | 38876 (22.5) | 83695 (11.0) | 54596 (0.5) | 761 (1.00) |
| ICOSL ECD Truncation #6 with N52H/N57Y/Q100R/C198R | 9 | 84566 (49.0) | 236011 (31.1) | 91357 (0.9) | 7 (0.01) |
| ICOSL ECD Truncation #5 with N52H/N57Y/Q100R/C198R | 8 | 86289 (50.0) | 216071 (28.5) | 110188 (1.1) | 9 (0.01) |
| ICOSL ECD Truncation #8 with N207G/L208G and N52H/N57Y/Q100R/C198R and Y151H | 11 | 94156 (54.5) | 368471 (48.6) | 142900 (1.4) | 2 (0.00) |

(continued)

| Table 18: Molecule sequences, binding data, and costimulatory bioactivity data of variant ICOSL molecules | | | | | |
|---|---|---|---|---|---|
| Description | Reference Sequence SEQ ID NO | Binding | | | MLR |
| | | CD28 | CTLA-4 | ICOS | IFN-gamma |
| | | MFI (ΔWT) | MFI (ΔWT) | MFI (ΔWT) | pg/mL (ΔWT) |
| ICOSL IgV with N52H/N57Y/Q100R | 3 | 84594 (49.0) | 204840 (27.0) | 117707 (1.2) | 0 (0.00) |
| ICOSL ECD with N52H/N57Y/Q100R/C198R | 1 | 59179 (34.3) | 132894 (17.5) | 138555 (1.4) | 0 (0.00) |
| Wildtype ICOSL ECD | 1 | 1727 (1.0) | 7579 (1.0) | 100466 (1.0) | 757 (1.00) |

**C. Binding and Activity of Proteins Expressed in 293 (Expi293) or CHO cells**

The variant ICOSL Fc fusion proteins, generated based on ICOSL reference sequences described above, were assessed for binding and activity following expression in 293 (Expi293) or CHO cells. In addition, a DNA construct encoding exemplary IgSF domain ICOSL variants N52H/N57Y/Q100R/C198R or N52H/Q100R, in exemplary ICOSL reference sequences as set forth in Table 19A, also were linked to an inert Fc containing mutations C220S/L234A/L235E/G237A by EU numbering set forth in SEQ ID NO: 40, and were produced and purified following transfection of 293 or CHO cells with the DNA constructs. In addition, an exemplary variant immunomodulatory protein was generated as a monomer in which cells were transfected with a DNA construct encoding the variant in the variant ICOSL ECD reference sequence but without fusion with an Fc sequence.

The resulting purified variant ICOSL Fc-fusion proteins or variant ICOSL monomer were assessed for binding to cognate binding partners and for modulation of T cell activity using a mixed lymphocyte reaction (MLR) substantially as described above. Table 19A indicates amino acid substitutions in the reference sequence of the variant ICOSL as designated by amino acid position number corresponding to amino acid positions in the respective reference (e.g., unmodified) ICOSL extracellular domain (ECD) sequence set forth in SEQ ID NO:1, and sets forth the SEQ ID NO identifier for each reference ICOSL sequence. Column 3 indicates the cell type (ExpiCHO-S or Expi293) used to produce the ICOSL protein. As shown in Table 19A, the results indicate substantially similar binding and activity, whether or not the variant ICOSL immunomodulatory protein was produced in CHO or 293 cells.

| Table 19A: Molecule sequences, binding data, and costimulatory bioactivity data of variant ICOSL ECD Fc-fusion molecules produced using various cells | | | | | | |
|---|---|---|---|---|---|---|
| Description | Referenc e Sequence SEQ ID NO | CHO or 293 Material | Binding | | | MLR |
| | | | CD28 | CTLA-4 | ICOS | IFN-gamma |
| | | | MFI (ΔWT) | MFI (ΔWT) | MFI (ΔWT) | pg/mL (ΔWT) |
| ICOSL ECD with N52H/N57Y/Q100R/C198R | 1 | 293 | 13710 (3.9) | 8715 (0.7) | 16746 (1.4) | 20 (0.02) |
| ICOSL ECD with N52H/N57Y/Q100R/C198R | 1 | CHO | 12876 (3.6) | 8750 (0.7) | 7700 (0.7) | 16 (0.02) |
| ICOSCL ECD with N52H/Q100R | 1 | 293 | 11664 (3.3) | 13429 (1.1) | 10284 (0.9) | 168 (0.19) |
| ICOSCL ECD with N52H/N57Y/Q100R/F172S | 1 | 293 | 12900 (3.6) | 8179 (0.7) | 15956 (1.4) | 14 (0.02) |
| ICOSCL ECD with N52H/N57Y/Q100R/F172S | 1 | CHO | 14437 (4.1) | 8708 (0.7) | 12610 (1.1) | 21 (0.02) |

(continued)

**Table 19A: Molecule sequences, binding data, and costimulatory bioactivity data of variant ICOSL ECD Fc-fusion molecules produced using various cells**

| Description | Referenc e Sequence SEQ ID NO | CHO or 293 Material | Binding | | | MLR |
|---|---|---|---|---|---|---|
| | | | CD28 | CTLA-4 | ICOS | IFN-gamma |
| | | | MFI (ΔWT) | MFI (ΔWT) | MFI (ΔWT) | pg/mL (ΔWT) |
| ICOSCL IgV with N52H/N57Y/Q100R | 3 | 293 | 16618 (4.7) | 9674 (0.8) | 9377 (0.8) | 10 (0.01) |
| ICOSCL IgV with N52H/N57Y/Q100R | 3 | CHO | 17343 (4.9) | 9039 (0.7) | 8673 (0.7) | 14 (0.01) |
| ICOSL ECD Truncation #4 with N52H/N57Y/Q100R/F172S | 7 | 293 | 14710 (4.1) | 8841 (0.7) | 6893 (0.6) | 21 (0.02) |
| ICOSL ECD Truncation #4 with N52H/N57Y/Q100R/F172S | 7 | CHO | 12743 (3.6) | 9000 (0.7) | 7606 (0.7) | 21 (0.02) |
| ICOSL ECD Truncation #7 with N52H/N57Y/Q100R/F172S | 10 | 293 | 12017 (3.4) | 9674 (0.8) | 7599 (0.7) | 15 (0.02) |
| ICOSL ECD Truncation #7 with N52H/N57Y/Q100R/F172S | 10 | CHO | 13043 (3.7) | 9039 (0.7) | 8077 (0.7) | 7 (0.01) |
| ICOSL ECD Monomer with N52H/N57Y/Q100R no Fc | 1 | 293 | 20575 (5.8) | 19978 (1.6) | 11989 (1.0) | 133 (0.15) |
| ICOSL ECD Dimer with N52H/N57Y/Q100R no Fc | 1 | 293 | 18477 (5.2) | 22361 (1.8) | 12913 (1.1) | 119 (0.13) |
| Wildtype ICOSL ECD | 1 | 293 | 3556 (1.0) | 12121 (1.0) | 11690 (1.0) | 905 (1.00) |

**D. Ligand Binding and Blocking**

Ligand binding was assessed using CHO cells expressing CD28 or ICOS incubated with fixed amounts of CD86 or ICOSL labeled with AlexaFluor-647, and in the presence of escalating concentrations of (100 pM to1,000,000 pM) of wild type ICOSL-Fc, the variant ICOSL IgV-Fc (containing a variant ICOSL IgV with N52H/N57Y/Q100R as set forth in SEQ ID NO: 36, fused to an inert Fc, e.g. containing mutations L234A, L235E and G237Ain a human IgG1 Fc, e.g. set forth in SEQ ID NO:42) or, for comparison, the CD28-only inhibitor, abatacept, the ICOS pathway inhibitor, prezalumab (AMG-557; anti-ICOSL, Creative Biolabs), or belatacept. Bound protein was detected with anti-human IgG-PE and measured by flow cytometry **(FIG. 10H),** which shows high target binding of the variant ICOSL IgV-Fc, but not control proteins, for both CD28 and ICOS. The IC50 for ligand binding is shown in **Table 19B.1.** The variant ICOSL IgV-Fc was confirmed to block ligand binding to both the target receptor CD28 and ICOS as measured by flow cytometry and IC50 levels determined with Graphpad Prism **(FIG. 10I**).

To assess impacts on costimulation activity, PBMCs from healthy donors and from subjects with Sjögren's Syndrome (SjS) were stimulated with artificial antigen presenting cells (K562 cells expressing CD80, CD86, ICOS ligand, and anti-CD3 (OKT3)), at a 20:1 ratio of cells to artificial APCs and in the presence of 100 nM of the variant ICOSL IgV-Fc or, for comparison, the CD28-only inhibitor, abatacept, the ICOS pathway inhibitor, prezalumab (AMG-557; anti-ICOSL, Creative Biolabs), or a combination of abatacept and prezalumab. Fc alone was used as a control.

To evaluate effects on CD28 costimulation activity, Jurkat effector cells expressing IL-2-luciferase reporter drived from IL-2 pomoter (Promega) were co-cultred with artificial APC (aAPC) expressing anti-CD3 OKT3 + human CD86 (CD28 costimulation blockade). To evaluate effects on ICOS costimulation activity, Jurkat/IL-2 reporter cells were further transduced with extracellular domain of human ICOS and intracellular domain of CD28 and co-cultured with aAPC expressing anti-CD3 OKT3 + human ICOSL (ICOS costimulation blockade). Titrating amounts of (100 pM to1,000,000 pM) of the variant ICOSL IgV-Fc was added to co-cultures. For comparison of blocking effects on CD28 costimulation activity, abatacept or belatacept were added as controls. For comparison of blocking effects on ICOS costimulation activity, prezalumab was added as a control. Costimulation activity was assessed by monitoring relative luminescence signal after the start of incubation. As shown in **FIG. 10J,** the variant ICOSL IgV-Fc blocked both CD28 and ICOS costimulation.

| | **Table 19B.1. Ligand Binding IC50s (nM).** | | | |
|---|---|---|---|---|
| | **Cell surface Expressed Target** | **CD28** | | **ICOS** |
| | **Ligand** | **CD80** | **CD86** | **ICOSL** |
| **Inhibitor** | **WT** | - | - | 17.2 |
| | **Prezalumab** | - | - | 23.3 |
| | **Abatacept** | 5.4 | 12.9 | - |
| | **Belatacept** | 2.5 | 2.8 | - |
| | **variant ICOSL IgV-Fc** | 1.0 | 0.7 | 4.3 |

### E. Comparison of Target Binding

Target binding of the exemplary variant ICOSL vIgD-Fc fusion molecule, containing a variant ICOSL IgV with N52H/N57Y/Q100R as set forth in SEQ ID NO: 36, fused to an inert Fc (containing mutations L234A, L235E and G237Ain a human IgG1 Fc, e.g. set forth in SEQ ID NO:42), was assessed across different species. Binding was assessed substantially as described in Example 1 on transfected HEK293 cells generated to express the full-length surface ligands CD28, ICOS or CTLA-4 from human, cynomolgus monkey, rat or mouse. Transfection was carried out using a transient transfection system (Life Technologies, USA). As a control, binding to mock (non-transfected) cells also was assessed. Results are set forth in Table 19B.2. As shown, the exemplary variant ICOSL vIgD-Fc fusion molecule exhibited binding to both human ICOS and CD28 with high affinity. Comparable binding was observed for cynomolgus monkey, but lower affintity to rodent counterparts was observed. With respect to CTLA-4 binding, bynomolgus moneky and rat are comparable to human whereas mouse has lower affinity. In contrast, wild-type ICOSL-Fc exhibited detectable binding to ICOS, but little detectable or quantifiable binding to CD28 was observed (data not shown).

| **Table 19B.2. Comparison of target binding across species.** | | | |
|---|---|---|---|
| **Species** | **CD28 $EC_{50}$ (pM)** | **ICOS $EC_{50}$ (pM)** | **CTLA-4 $EC_{50}$ (pM)** |
| Human | 648 | 633 | 680 |
| Cynomolgus monkey | 440 | 579 | 704 |
| Rat | 1,064 | 1,028 | 793 |
| Mouse | 1,153 | 1,428 | 1,112 |

## EXAMPLE 13

### Assessment of Dosing and *in vivo* Effects of ICOSL IgV-Fc Fusion Molecules in a CIA Model

Variant ICOSL IgV-Fc fusion molecules were assessed for anti-inflammatory activity in the collagen-induced arthritis (CIA) model with either prophylactic or therapeutic dosing. The variant ICOSL IgV-Fc fusion molecule was dosed a maximum of 4 times either prior to or just after disease onset. The tested variant ICOSL IgV-Fc fusion molecule contained a variant ICOSL IgV with N52H/N57Y/Q100P as set forth in SEQ ID NO: 67 or N52H/N57Y/Q100R as set forth in SEQ ID NO: 36, fused to an inert Fc (containing mutations L234A, L235E and G237A in a human IgG1 Fc, e.g.

set forth in SEQ ID NO:42).

For induction of joint inflammation, mice were injected on day -18 or -21 with a chick or bovine collagen II/CFA emulsion in the tail and with a chick or bovine collagen II/IFA emulsion ('boost') on day 0. For prophylactic dosing, mice were dosed with the variant ICOSL IgV-Fc fusion molecule (N52H/N57Y/Q100P) with four doses beginning on the day of the boost, before the onset of disease. For therapeutic/delayed treatment, mice were dosed with the variant ICOSL IgV-Fc fusion molecule (N52H/N57Y/Q100R) started when the observed paw score was greater than one, and dosing occurred every two days for a total of four doses. As a control, Fc only molecules and a CTLA-4-Fc (abatacept) molecule were also tested. Paw score based on redness or swelling was determined. Serum also was collected to measure anti-collagen (CII IgG) antibodies and IL-6 and TNF$\alpha$ proinflammatory cytokines. Cells from draining lymph nodes were collected, stained for CD4, CD8, CD44 or markers of T follicular helper ($T_{FH}$) cells (CD25-CD4+PD-1+CXCR5+). **FIGS. 11A-11D** show results for prophylactic dosing. Mice treated with the variant ICOSL IgV-Fc fusion molecule on the prophylactic dosing treatment showed suppressed disease in the CIA mouse model of rheumatoid arthritis as shown by a lower mean sum paw score **(FIG. 11A),** and decreased detected CII IgG **(FIG. 11B).** * p<0.05 for ICOSL IgV-Fc vs. abatacept ** p<0.001 for ICOSL IgV-Fc vs. PBS (by 2-way repeated-measures ANOVA). Lower levels of serum cytokines **(FIG. 11C)** and CD44+ activated T cells or $T_{FH}$ cells **(FIG. 11D)** were also observed in mice treated with the variant ICOSL IgV-Fc fusion molecule compared to Fc control; * p< 0.05, ** p< 0.01, *** p< 0.001, **** p< 0.0001 (by 1-way ANOVA). The fraction of B cells in the draining lymph node was also significantly reduced in the ICOSL IgV-Fc treated group vs. the Fc control group (p<0.05) **(FIG. 11E).**

**FIGS. 12A-12D** show results for delayed dosing. The variant ICOSL-IgV Fc resulted in the lowest mean sum paw score **(FIG. 12A)** and greatest percent change in body weight **(FIG. 12B)** compared to other groups, including the abatacept control. As shown in **FIG. 12C** and **FIG. 12D,** serum cytokines also were suppressed in the therapeutic CIA model in mice treated with the variant ICOS IgV-Fc. Statistical significance between groups: *p<0.05; **p<0.01; ***p<0.001 by Student's unpaired t-test. In **FIG. 12C** and **FIG. 12D,** the dotted horizontal lines indicate the assay lower limit of quantification (LLOQ) for each cytokine.

Together, these data evidence that the CD28 and ICOS pathways play important roles in inflammatory arthritis. In particular, the superior activity of the variant ICOSL dual CD28/ICOS antagonist is consistent with an observation that blockade of both pathways is necessary and that only partial benefit is achieved by a single pathway blockade.

## EXAMPLE 14

### Assessment of *in vivo* Effects of ICOSL IgV-Fc Fusion Molecules in a EAE Model

A variant ICOSL IgV-Fc fusion molecule was assessed for anti-inflammatory activity in an adoptive transfer experimental autoimmune encephalomyelitis (EAE) model. The tested variant ICOSL IgV-Fc fusion molecule containing a variant ICOSL IgV (N52H/ N57Y/Q100R; SEQ ID NO: 36) fused to an inert Fc (containing mutations L234A, L235E and G237A in a human IgG1 Fc).

Female C57BL/6 mice were subcutaneously injected with a $MOG_{35-55}$/CFA emulsion. After 11 days, spleen cells were obtained and cultured with $MOG_{35-55}$ peptide, IL-12, and anti-IFN$\gamma$. Three days post culture, encephalitogenic T cells were delivered via intraperitoneal injection (Day 0). Mice were dosed with variant ICOSL IgV-Fc fusion molecule every other day starting on Day 0 for a total of five doses. As a control, Fc only molecules and a CTLA-4-Fc molecule (abatacept) were also tested. For 20 days post injection of the T cells, mice were weighed, monitored and assessed for EAE score as described in Table 20. At end of study, serum was collected for analysis of pro-inflammatory cytokines, and cells from draining lymph nodes were collected for flow cytometric analysis.

| **Table 20. EAE Scoring** | |
|---|---|
| Score | Clinical Observations |
| 0 | No obvious changes in motor functions |
| 1 | Limp tail |
| 2 | Limp tail and weakness of hind legs |
| 3 | Limp tail and complete paralysis of hind legs, OR Limp tail with paralysis of one front and one hind leg, OR ALL of: 1) Severe head tilting, 2) Walking only along the edges of the cage, 3) Pushing against the cage wall, 4) Spinning when picked up by the tail. |
| 4 | Limp tail, complete hind leg and partial front leg paralysis |

(continued)

| Table 20. EAE Scoring | |
|---|---|
| Score | Clinical Observations |
| 5 | Complete hind and complete front leg paralysis, no movement; OR Mouse is spontaneously rolling in the cage; OR Mouse found dead due to paralysis. |

As shown in **FIG. 13A,** mice treated with the variant ICOSL IgV-Fc fusion molecule suppressed disease in the EAE mouse model as shown by a lower EAE score, *p<0.0001 by 1-way ANOVA Area Under the Curve (AUC); variant ICOSL IgV-Fc fusion molecule compared to controls. **FIG. 13B** shows change in mean body weight over time.

For flow cytometric analysis of inguinal lymph node T cells, the cells were stained with viability dye & analyzed with anti-CD44, anti-CD62L, anti-CD4, anti-CD8 antibodies and assessed for percentage of viable naive (CD62L+CD44-) and $T_{effector}$ memory (Tern) (CD62L-CD44+) CD4+ and CD8+ T cells. As shown in **FIG. 13C,** CD4+ and CD8+ Tern cells were reduced with treatment with the variant ICOSL IgV-Fc fusion molecule (**** p<0.0001; *** p<0.001 by 1-way ANOVA).

Serum cytokines were assessed on Day 0 (2 hours post 1$^{st}$ dose) and on Day 6 (1 hour before 4$^{th}$ dose). As shown in **FIG. 13D,** the tested variant ICOSL IgV-Fc fusion molecule resulted in reduction of pro-inflammatory cytokines in serum on Day 0, including IL-5, IL-10, IL-12p70 and TNFα. At day 6, serum levels of IFN-gamma and IL-6 were reduced by treatment with the variant ICOSL IgV-Fc fusion molecule compared to Fc control.

## EXAMPLE 15

### Dose Ranging Study of Variant ICOSL-IgV Fc in Graft-Versus-Host-Disease (GvHD) Model

A dose ranging study was conducted with 20, 100, or 500 μg of a variant ICOSL IgV-Fc molecule, containing a variant ICOSL IgV (N52H/N57Y/Q100R; SEQ ID NO: 36) fused to an inert Fc (containing mutations L234A, L235E and G237A in a human IgG1 Fc, e.g. set forth in SEQ ID NO:42), in a mouse model of graft-versus-host-disease (GVHD). The activity of the variant ICOSL IgV-Fc molecule was compared to belatacept (CTLA-4-FcL104E/A29Y; U.S. Patent Application Publication Number US2016/0271218).

Female NSG mice (n = 5 per group for Group 1, no treatment; n= 10 per group for treatment Groups 2-7) were administered 10 mg of gamma globulin subcutaneously and then irradiated (100 cGy/rad) on Day -1. On Day 0 (within 24 hours post-irradiation), the mice in Groups 2-7 were dosed with test articles as set forth in **Table 21,** and then all mice received 1 x $10^7$ human PBMCs injected IV via tail vein post-dosing.

A disease activity index (DAI) was determined by evaluating the mice three times a week during the study and scoring disease based on body weight loss, posture, activity, appearance of the fur and skin of the mice. After the study was terminated on Day 42, endpoint measurements of survival, body weight loss, and disease activity were evaluated. Kaplan-Meier survival plots representing the percentage of animals surviving to the study endpoint were generated and survival curve comparisons were analyzed by the Mantel-Cox and Gehan-Breslow-Wilcox tests (95% CI). Blood/serum samples were collected from surviving mice at the end of the study (Day 42) and cells were assessed by flow cytometry for markers of T cells, including mouse or human markers, CD4, CD8, CD28, ICOS, activation or exhaustion markers (PD-1, Ki67) and FoxP3 (a marker of Tregs). The levels of serum pro-inflammatory cytokines (e.g. IFN-gamma, IL-10, IL-12(p70), IL-17A, IL-4, IL-5 and TNFα) also were assessed.

| Table 21: Dosing Schedule | | | | | | | |
|---|---|---|---|---|---|---|---|
| **Group** | **N** | **Test Articles** | **Dose (μg)** | **Dose Volume (μl)** | **Route** | **Schedule** | **Start of Dosing** |
| 1 | 5 | No treatment | n/a | n/a | n/a | n/a | n/a |
| Survival/DAI | | | | | | | |
| 2 | 10 | Saline | n/a | 100 | IP | 3x weekly 4 weeks | Day 0 |
| 3 | 10 | variant ICOSL IgV-Fc | 500 | 100 | IP | 3x weekly 4 weeks | Day 0 |
| 4 | 10 | variant ICOSL IgV-Fc | 100 | 100 | IP | 3x weekly 4 weeks | Day 0 |

(continued)

| | | | | | | | |
|---|---|---|---|---|---|---|---|
| Survival/DAI | | | | | | | |
| 5 | 10 | variant ICOSL IgV-Fc | 20 | 100 | IP | 3x weekly 4 weeks | Day 0 |
| 6 | 10 | Belatacept | 100 | 100 | IP | 3x weekly 4 weeks | Day 0 |
| PK/Survival//DAI | | | | | | | |
| 7 | 9 | variant ICOSL IgV-Fc | 100 | 100 | IP | Single Day 1 | Day 1 |

**FIGS. 14A-14B** show the survival and DAI scores of GVHD mice treated in accord with each dosing schedule. As shown, the tested variant ICOSL IgV-Fc (N52H/N57Y/Q100R) at all dose levels tested significantly enhanced survival **(FIG. 14A)** and reduced disease scores **(FIG. 14B)** compared to mice treated with belatacept (i.e. 100% vs. 40% survival at Day 42, respectively; p <0.01 by Mantel-Cox log rank test). Notably, single dose (100 μg) administration of variant ICOSL IgV-Fc (N52H/N57Y/Q100R) resulted in similar protection from disease as repeat dosing of 100 μg belatacept.

Flow cytometric analysis of blood collected at the end of the study demonstrated that the tested variant ICOSL IgV-Fc (N52H/N57Y/Q100R) effectively suppressed expansion of transferred human T cells as observed by a reduced ratio of human cells/mouse cells **(FIG. 15A)** and the greatly reduced total T cell counts **(FIG. 15B).** As shown in **FIGS. 15C-15F,** flow cytometry staining of blood collected at end of the study of CD4+ and CD8+ T cells co-stained for ICOS **(FIGS. 15C-15D)** and CD28 **(FIGS. 15E-15F)** demonstrated essentially no staining in groups treated with variant ICOSL IgV-Fc (N52H/N57Y/Q100R), although CD4+ and CD8+ T cells expressing ICOS were readily detectable in belatacept-treated mice. These results are consistent with the lack of T cells remaining in the ICOSL IgV-Fc-treated mice, and also with the ability of the variant ICOSL IgV-Fc (N52H/N57Y/Q100R) to bind its target molecules CD28 and ICOS and block their detection by the flow cytometry antibodies. Binding of variant ICOSL IgV-Fc (N52H/N57Y/Q100R) on the few remaining human T cells was confirmed by detection using anti-Human IgG Fc. Notably, while most of the transferred human T cells initially expressed CD28 and just 10-20% were ICOS+, the activated T cells remaining in the saline-or belatacept-treated mice at termination/end of study were >80% ICOS+.

The presence of activation or exhaustion markers of T cells also was suppressed in groups treated with variant ICOSL IgV-Fc (N52H/N57Y/Q100R), as evidenced by lower expression of PD1 in CD4+ and CD8+ T cells and decreased Ki67 expression in CD4+ T cells, **(FIGS. 16A-16B).** The ratio of effector T cells to Tregs remained stable in groups treated with variant ICOSL IgV-Fc (N52H/N57Y/Q100R) compared to belatacept **(FIG. 16C).** Serum proinflammatory cytokines also were suppressed in groups treated with variant ICOSL IgV-Fc (N52H/N57Y/Q100R) compared to belatacept **(FIGS. 17A-17D).**

Pharmacokinetic (PK) analysis also was carried out to monitor serum exposure of variant ICOSL IgV-Fc (N52H/N57Y/Q100R). Test article concentrations were measured in mouse serum samples using a quantitative PK ELISA using an anti-human ICOSL mAb capture antibody and an Fc-specific mouse anti-human IgG as the detection reagent.

The observed serum exposure of variant ICOSL IgV-Fc (N52H/N57Y/Q100R) in the GVHD model was 45% lower than that of normal mice, determined in a separate study **(FIG. 17E).** The longer terminal half-life of variant ICOSL IgV-Fc (N52H/N57Y/Q100R) in the GVHD model may due to reduced target (CD28, ICOS) at later time points in GVHD (as the human T cells disappear), and/or to anti-drug antibody (ADA) formation in normal mice, which can interfere with drug exposure. The observation that variant ICOSL IgV-Fc (N52H/N57Y/Q100R) had lower serum exposure compared to normal mice may be due to target mediated drug disposition (TMDD) in the GVHD model (i.e. its higher affinity for human CD28 and ICOS as compared to the mouse orthologues), and/or to lack of FcRn in the NOD/SCID (NSG) mice used in this model.

Together, these results are consistent with an observation that variant ICOSL IgV-Fc (N52H/N57Y/Q100R) exhibits potent antagonist activity, even with only a single dose, and superior activity to belatacept. This observation may be attributable to the variant ICOSL IgV-Fc (N52H/N57Y/Q100R) exhibiting superior control of ICOS+ T cells, which otherwise escape single ICOS or CD28 pathway blockade, such as that achieved with the CD28 pathway antagonist belatacept.

## EXAMPLE 16

### Assessment of Variant ICOSL-IgV Fc in CD4+CD45RBhigh-induced Colitis Model

The effect of the exemplary variant ICOSL IgV-Fc containing a variant ICOSL IgV (N52H/ N57Y/Q100R; SEQ ID NO: 36) fused to an inert Fc (containing mutations L234A, L235E and G237A in a human IgG1 Fc, e.g. set forth in SEQ ID NO:42) on disease development in CD4+CD45RBhigh-induced colitis model was evaluated.

CD4+CD45RBhigh donor cells were enriched by negative selection from spleen cell suspensions obtained from 15 male BALB/C donor mice. On Day 0, 0.3 million CD4+CD25-CD45RBhigh (Treg depleted) donor cells were injected intravenously into immunodeficient C.B17 (SCID) mice (n=12 or 21 per group) to induce colitis. As a control, 0.3 million CD4+ cells (containing Treg cells), which do not induce development of colitis in this model, were injected into SCID mice recipients (n=12). On the day of cell transfer, mice in each group were dosed with variant ICOSL-IgV Fc or Fc only or vehicle controls. **Table 22** summarizes the treatment regimen for tested groups.

| Table 22: Treatment Regimens | | | | | | |
|---|---|---|---|---|---|---|
| **Group** | **# mice** | **Cells injected** | **Treatment** | **Dose** | **Route** | **Frequency** |
| 1 | 12 | CD4+ | PBS (sterile) | - | i.p. | 3x/week (M,W,F) |
| 2 | 12 | CD4+CD45RBhigh | PBS (sterile) | - | i.p. | 3x/week (M,W,F) |
| 3 | 21 | CD4+CD45RBhigh | Fc control | 300 μg | i.p. | 3x/week (M,W,F) |
| 5 | 12 | CD4+CD45RBhigh | Variant ICOSL IgV-Fc | 400 μg | i.p. | 3x/week (M,W,F) |

Body weight (taken starting on Day 0) and stool consistency score (taken starting on Day 10), were evaluated three times per week. Daily disease activity index (DAI) was calculated from body weight and stool scores. After the study was terminated on Day 42, colon was collected for determination of length and weight and histological analysis. Statistical analysis of end body weight and end colon weight and length of variant ICOSL-IgV Fc treated group to vehicle group was assessed using two-tailed Student's t-test. Stool scores and DAI scores were compared using Wilcoxon's non-parametric T test.

DAI results are shown in Tables 23 (DAI) and **FIG. 18A,** colon measurements are shown in Table 24, and colon histology results are shown in Table 25 and **FIG. 18B.** As shown tested variant ICOSL IgV-Fc (N52H/N57Y/Q100R) exhibited significantly reduced development of colitis in this model, which is consistent with the utility of this dual CD28/ICOS antagonist to effectively treat inflammatory bowel disease (IBD).

| Table 23: Disease Activity Score | | | | | | |
|---|---|---|---|---|---|---|
| **Treatment** | **End % body weight +/- SD** | **p value** | **End stool score +/- SD** | **p value** | **End DAI score +/- SD** | **p value** |
| Colitis not induced/Vehicle | 112.3% +/- 3.9% | | 0.3 +/- 0.5 | | 0.3 +/- 0.5 | |
| Vehicle | 92.7% +/- 11.7% | <0.0001^ | 1.3 +/- 1.3 | 0.0016^ | 3.2 +/- 2.6 | 0.0003^ |
| Fc control, 300 μg | 96.2% +/- 5.4% | 0.2656* | 1.4+/- 1.4 | 0.8836* | 2.5 +/- 1.7 | 0.6948* |
| ICOSL IgV-Fc (N52H/N57Y/Q100R), 400 μg | 109.6% +/- 4.0% | <0.0001~ | 0.1 +/- 0.3 | 0.0014~ | 0.1 +/- 0.3 | <0.0001~ |
| ^ Compared to Colitis not induced/Vehicle<br>* Compared to Vehicle<br>- Compared to Fc control, 300 μg | | | | | | |

| Table 24: Colon Measurements | | | | | | |
|---|---|---|---|---|---|---|
| **Treatment** | **End colon weight (mg) +/- SD** | **p value** | **End colon length (mm) +/- SD** | **p value** | **End Colon weight/length +/- SD** | **p value** |
| Colitis not induced/ Vehicle | 175.3 +/- 16.9 | | 82.8 +/- 5.2 | | 2.1 +/- 0.2 | |
| Vehicle | 323.1 +/- 78.0 | <0.0001^ | 77.7 +/- 7.5 | 0.0668^ | 4.2 +/- 1.2 | <0.0001^ |
| Fc control, 300 μg | 276.7 +/- 92.4 | 0.1602* | 77.6 +/- 8.9 | 0.9911 * | 3.6 +/- 1.2 | 0.1843* |
| ICOSL IgV-Fc (N52H/N57Y/Q100R), 400 μg | 160.8 +/- 24.4 | 0.0004~ | 79.4 +/- 6.1 | 0.5738~ | 2.0 +/- 0.3 | 0.0003~ |
| ^ Compared to Colitis not induced/Vehicle<br>* Compared to Vehicle<br>- Compared to Fc control, 300 μg | | | | | | |

| Table 25: Histology Results | | | | | | | |
|---|---|---|---|---|---|---|---|
| | | | | Colon (swiss roll) | | | |
| **Group** | **Cage #** | **Mouse #** | **Mouse ID#** | **Mucosa** | **Submucosa** | **Muscularis** | **Total score** |
| | 1 | 1 | 1-1 | 0 | 0 | 0 | 0.0 |
| Colitis not induced/ Vehicle | | 2 | 1-2 | 0 | 0 | 0 | 0.0 |
| | | 3 | 1-3 | 0 | 0 | 0 | 0.0 |
| | | 4 | 1-4 | 0 | 0 | 0 | 0.0 |
| | 2 | 1 | 2-1 | 1 | 1 | 0 | 2.0 |
| | | 2 | 2-2 | 0 | 0 | 0 | 0.0 |
| | | 3 | 2-3 | - | - | - | - |
| | | 4 | 2-4 | - | - | - | - |
| | 3 | 1 | 3-1 | - | - | - | - |
| | | 2 | 3-2 | - | - | - | - |
| | | 3 | 3-3 | - | - | - | - |
| | | 4 | 3-4 | - | - | - | - |
| | | | Average | 0.2 | 0.2 | 0.0 | 0.3 |
| | | | Std Dev | 0.4 | 0.4 | 0.0 | 0.8 |
| | | | SEM | 0.2 | 0.2 | 0.0 | 0.3 |

(continued)

| Table 25: Histology Results | | | | | | | |
|---|---|---|---|---|---|---|---|
| | | | | Colon (swiss roll) | | | |
| **Group** | **Cage #** | **Mouse #** | **Mouse ID#** | **Mucosa** | **Submucosa** | **Muscularis** | **Total score** |
| Vehicle | 4 | 1 | 4-1 | 3 | 3 | 1 | 7.0 |
| | | 2 | 4-2 | 1 | 1 | 0 | 2.0 |
| | | 3 | 4-3 | 2 | 2 | 0 | 4.0 |
| | | 4 | 4-4 | 2 | 2 | 0 | 4.0 |
| | 5 | 1 | 5-1 | 2 | 3 | 0 | 5.0 |
| | | 2 | 5-2 | 2 | 2 | 0 | 4.0 |
| | | 3 | 5-3 | - | - | - | - |
| | | 4 | 5-4 | - | - | - | - |
| | 6a | 1 | 6a-1 | - | - | - | - |
| | | 2 | 6a-2 | - | - | - | - |
| | | 3 | 6a-3 | - | - | - | - |
| | 6b | 1 | 6b-1 | - | - | - | - |
| Average | | | | 2.0 | 2.2 | 0.2 | 4.3 |
| Std Dev | | | | 0.6 | 0.8 | 0.4 | 1.6 |
| SEM | | | | 0.3 | 0.3 | 0.2 | 0.7 |
| T-test vs colitis not induced/Vehicle | | | | 0.0001 | 0.0002 | 0.3409 | 0.0003 |

(continued)

| Table 25: Histology Results | | | | | | | |
|---|---|---|---|---|---|---|---|
| | | | | Colon (swiss roll) | | | |
| Group | Cage # | Mouse # | Mouse ID# | Mucosa | Submucosa | Muscularis | Total score |
| Fc control, 300 µg | 6b | 2 | 6b-2 | 2 | 1 | 0 | 3.0 |
| | | 3 | 6b-3 | 3 | 3 | 1 | 7.0 |
| | 6c | 1 | 6c-1 | 3 | 3 | 0 | 6.0 |
| | | 2 | 6c-2 | 2 | 2 | 2 | 6.0 |
| | | 3 | 6c-3 | 3 | 2 | 1 | 6.0 |
| | 7 | 1 | 7-1 | - | - | - | - |
| | | 2 | 7-2 | 2 | 2 | 1 | 5.0 |
| | | 3 | 7-3 | 2 | 1 | 0 | 3.0 |
| | | 4 | 7-4 | 2 | 3 | 0 | 5.0 |
| | 8 | 1 | 8-1 | 2 | 1 | 0 | 3.0 |
| | | 2 | 8-2 | 2 | 1 | 0 | 3.0 |
| | | 3 | 8-3 | - | - | - | - |
| | | 4 | 8-4 | 1 | 1 | 0 | 2.0 |
| | 9 | 1 | 9-1 | - | - | - | - |
| | | 2 | 9-2 | - | - | - | - |
| | | 3 | 9-3 | - | - | - | - |
| | | 4 | 9-4 | - | - | - | - |
| | 10 | 1 | 10-1 | - | - | - | - |
| | | 2 | 10-2 | - | - | - | - |
| | | 3 | 10-3 | - | - | | - |
| | | 4 | 10-4 | - | - | - | - |
| Average | | | | 2.2 | 1.8 | 0.5 | 4.5 |
| Std Dev | | | | 0.6 | 0.9 | 0.7 | 1.7 |
| SEM | | | | 0.2 | 0.3 | 0.2 | 0.5 |
| T-test vs Vehicle | | | | 0.5676 | 0.4240 | 0.3663 | 0.8885 |

(continued)

| Table 25: Histology Results | | | | | | | |
|---|---|---|---|---|---|---|---|
| | | | | Colon (swiss roll) | | | |
| Group | Cage # | Mouse # | Mouse ID# | Mucosa | Submucosa | Muscularis | Total score |
| ICOSL IgV -Fc (N52H/N57Y/Q100R) , 400 μg | 14 | 1 | 14-1 | 0 | 0 | 0 | 0.0 |
| | | 2 | 14-2 | 0 | 0 | 0 | 0.0 |
| | | 3 | 14-3 | 0 | 0 | 0 | 0.0 |
| | | 4 | 14-4 | 0 | 0 | 0 | 0.0 |
| | 15 | 1 | 15-1 | 0 | 0 | 0 | 0.0 |
| | | 2 | 15-2 | 0 | 0 | 0 | 0.0 |
| | | 3 | 15-3 | - | - | - | - |
| | | 4 | 15-4 | - | - | - | - |
| | 16 | 1 | 16-1 | - | - | - | - |
| | | 2 | 16-2 | - | - | - | - |
| | | 3 | 16-3 | - | - | - | - |
| | | 4 | 16-4 | - | - | - | - |
| Average | | | | 0.0 | 0.0 | 0.0 | 0.0 |
| Std Dev | | | | 0.0 | 0.0 | 0.0 | 0.0 |
| SEM | | | | 0.0 | 0.0 | 0.0 | 0.0 |
| T-test vs Fc control | | | | 0.0000 | 0.0002 | 0.1315 | 0.0000 |

## EXAMPLE 17

### In vivo assessment of ICOSL IgV-fc fusion proteins toxicology and toxicokinetic profiles

Toxicology and toxicokinetic profiles (TK) were evaluated in rats and cynomolgus monkeys injected intravenously (IV) or subcutaneously (SC) with an exemplary ICOSL IgV-Fc fusion protein once a week, with repeat doses over a period of 4-5 weeks. Recovery of animals over 4 months also was monitored.

Exemplary recombinant ICOSL vIgD-Fc fusion proteins were generated from suspension-adapted Chinese hamster ovary cells (ExpiCHO-S) cells substantially as described in Example 11. ExpiCHO-S cells were transfected with DNA constructs encoding exemplary variant ICOSL vIgD-Fc fusion molecules containing a variant ICOSL IgV with N52H/N57Y/Q100P as set forth in SEQ ID NO: 67 or N52H/N57Y/Q100R as set forth in SEQ ID NO: 36, fused to an inert Fc (containing mutations L234A, L235E and G237Ain a human IgG1 Fc, e.g. set forth in SEQ ID NO:42).

Sprague-Dawley rats were administered the exemplary ICOSL vIgD-Fc fusion protein once weekly at 10, 100 or 200 mg/kg subcutaneously (SC), or 200 mg/kg by intravenous (IV) bolus for one month (5 doses, e.g. days 1, 8, 15, 22 and 29) followed by a 4 month recovery.

Cynomolgus monkey were administered the exemplary ICOSL vIgD-Fc fusion protein once weekly at 10, 100, or 150 mg/kg SC or 150 mg/kg IV bolus for one month (5 doses, e.g. days 1, 8, 15, 22 and 29) followed by a 4 month recovery.

Standard toxicologic assessments including clinical signs (as determined by cage side, detailed and postdose observations), body weights, qualitative food evaluation, clinical pathology parameters (hematology, coagulation, clinical chemistry, and urinalysis), gross necropsy findings, organ weights, and histopathologic examinations, as well as toxiokinetic (TK) evaluations and antibody analysis were evaluated through the end of the dosing phase (Day 30). In cynomolgus monkeys, additional assessments included ophthalmic exams, electrocardiology, flow cytometry immunophenotyping, Staphylococcal Enterotoxin B (SEB) ex vivo, and serum cytokine analysis assays. Toxicokinetic (TK) parameters, such as Tmax, Cmax, AUC0-7d and t1/2, were estimated using non-compartmental analysis methods.

### A. Sprague Dawley Rats

In the study in Sprague Dawley rats, following once weekly administration at 100 and 200 mg/kg SC and 200 mg/kg IV bolus, exposure was maintained through Day 30 (Table 26). At the 10 mg/kg dose level, ICOSL vIgD-Fc fusion protein exposure was reduced or lost after repeat dosing in many of the animals. Following SC dosing, ICOSL vIgD-Fc fusion protein exposure based on mean Cmax and AUC0-7day increased in a less than dose proportional manner between 10, 100, and 200 mg/kg dose levels.Bioavailability at the 200 mg/kg dose level was approximately 18%. Day 22 mean ARCmax and ARAUC values ranged from 0.856 to 1.52. There was little to no accumulation of ICOSL vIgD-Fc fusion proteins observed with repeat once weekly SC and IV dosing. Where it could be measured, the t1/2 of ICOSL vIgD-Fc fusion protein estimated within the dosing interval was approximately 1 to 3 days.

Administration of the ICOSL vIgD-Fc fusion protein at all dose levels was well tolerated. No ICOSL vIgD-Fc fusion protein related signs, or changes in body weights, quantitative food consumption, ophthalmology or organ weights we observed. Related findings included decreased lymphocyte cellularity in lymph nodes at all dose levels, consistent with expected pharmacology; as well as mild, non-adverse injection site changes (red discoloration, microscopic hemorrhage, mixed cell infiltrates). Mild, transient, and non-adverse clinical pathology changes were noted including minimal increases in neutrophils, lymphocytes, monocytes, and large unstained cells, bilirubin and fibrinogen; and minimal decreases in albumin. Minimally decreased urine volume and increased urine specific gravity was noted without clinical chemistry or microscopic correlates.

The no-observed adverse effect level (NOAEL), determined to be the highest dose evaluated at 200 mg/kg SC and IV ICOSL vIgD-Fc fusion protein, corresponds respectively to an AUC0-7d of 219 μg•day/mL, and a Cmax of 53.4 μg/mL; and an AUC0-7d of 1250 μg•hr/mL, and a Cmax of 4120 μg/mL.

**Table 26: Summary of Repeat-dose Mean Toxicokinetic Parameters of ICOSL vIgD-Fc Fusion Proteins in Sprage-Dawley Rats.**

| Dose (mg/kg) | Route | Day 1 (Dose 1) | | | | Day 22 (Dose 4) | | | | | |
|---|---|---|---|---|---|---|---|---|---|---|---|
| | | $T_{max}$ (day) | $C_{max}$ (μg/mL) | $AUC_{0-7d}$ (μg*day/mL) | $t_{1/2}$ (day) | $T_{max}$ (day) | $C_{max}$ (μg/mL) | $AUC_{0-7d}$ (μg*day/mL) | $t_{1/2}$ (day) | $AR_{Cmax}$ | $AR_{AUC}$ |
| 10 | SC | 1.40 | 6.48 | 26.2 | NA | 0.9 | 10.2 | 24.8 | 0.623 | 1.5 | 0.856 |
| 100 | SC | 1.00 | 33.7 | 133 | 1.01 | 0.8 | 50.9 | 188 | 0.892 | 1.52 | 1.42 |
| 200 | SC | 1.19 | 53.4 | 219 | 2.77 | 0.875 | 67.9 | 252 | 1.3 | 1.29 | 1.16 |
| | IV | 0.00412 | 4120 | 1250 | 3.2 | 0.00346 | 4810 | 1630 | 2.83 | 1.18 | 1.32 |

**B. Cynomolgus monkeys**

In the study in cynomolgus monkeys, following once weekly administration at 10 mg/kg, 100 and 150 mg/kg SC and 150 mg/kg IV bolus, exposure was maintained through Day 30 **(Table 27)**. After SC administration, ICOSL vIgD-Fc fusion protein exposure based on mean maximum observed concentration increased in an approximately dose-proportional manner between the 10, 100, and 150 mg/kg doses. Mean $C_{max}$ values increased in a ratio of 1:9.33:16.8, mean $AUC_{0-7day}$ values increased in a ratio of 1:7.36:10.4. SC bioavailability at 150 mg/kg was approximately 39%. There was little to no accumulation with repeat once weekly SC or IV dosing. The apparent terminal elimination half-life ($t_{1/2}$) of ICOSL vIgD-Fc fusion protein estimated within the dosing interval was approximately 2 days for all dose levels.

In cynomoglus monkeys, all animals survived until the scheduled necropsy on study day 30. Administration of the ICOSL vIgD-Fc fusion protein was well tolerated at all dose levels. ICOSL vIgD-Fc fusion protein related changes included decreased lymphocyte cellularity in lymph nodes and spleen at all doses, correlating with decreased spleen weights in some animals. No other ICOSL vIgD-Fc fusion protein-related effects were observed on mortality, clinical observations, body weights, food consumption, ophthalmic exams, electrocardiology, vital signs, clinical pathology parameters, macroscopic evaluations, or serum cytokines (IL-1β, IL-1ra, IL-2, IL-5, IL-6, IL-8, IL-10 IL-12/23 (p40), IL-13, IL-17A, MCP-1, MIP-1β, IFN-γ, TNF-α, G-CSF, and GM-CSF).

The NOAEL was considered the highest dose evaluated, 150 mg/kg SC and IV, which corresponds respectively to an $AUC_{0-7d}$ of 480 μg•hr/mL, and a Cmax of 204 μg/mL; and an $AUC_{0-7d}$ of 1320 μg•hr/mL, and a Cmax of 3040 μg/mL.

**TABLE 27. Summary of Group Mean Repeatdose Toxicokinetic Parameters of ICOSL vIgD-Fc fusion proteins in Cynomolgus Monkeys**

| Dose (mg/kg) | Route | Day 1 (Dose 1) | | | | Day 22 (Dose 4) | | | | | |
|---|---|---|---|---|---|---|---|---|---|---|---|
| | | $T_{max}$ (day) | $C_{max}$ (μg/mL) | $AUC_{0-7d}$ (μg*day/mL) | $t_{1/2}$ (day) | $T_{max}$ (day) | $C_{max}$ (μg/mL) | $AUC_{0-7d}$ (μg*day/mL) | $t_{1/2}$ (day) | $AR_{Cmax}$ | $AR_{AUC}$ |
| 10 | SC (N=6) | 0.750 | 14.9 | 49.7 | 2.13 | 0.611 | 17.2 | 61.0 | 2.29 | 1.21 | 1.24 |
| 100 | SC (N=6) | 0.778 | 139 | 366 | 2.01 | 0.750 | 132 | 381 | 2.10 | 0.950 | 1.05 |
| 150 | SC (N=10) | 0.667 | 250 | 516 | 1.89 | 0.800 | 204 | 480 | 2.12 | 0.819 | 0.923 |
| | IV (N=10) | 0.0208 | 2950 | 1310 | 1.96 | 0.0292 | 3040 | 1320 | 2.14 | 1.03 | 1.01 |

## C. Summary

Together, these data are accordant with a biphasic elimination profile for serum concentration over time, which is consistent with the profile for a monoclonal antibody or antibody-like drug.

## EXAMPLE 18

### Determination of Dosage and Treatment by Human Pharmacokinetic Modeling for ICOSL vIgD-Fc fusion proteins

Human pharmacokinetic models (PK) for the exemplary ICOSL vIgD-Fc fusion protein, N52H/N57Y/Q100R as set forth in SEQ ID NO: 36, fused to an inert Fc (containing mutations L234A, L235E and G237A in a human IgG1 Fc, e.g. set forth in SEQ ID NO:42), was constructed based on modeling PK data and allometric scaling from the cynomoglus monkey study and Sprague Dawley rat study as described in Example 17. Linear PK at different dose levels was assumed to predict the human exposure. The dose levels selected were modeled based on in vitro binding data of the ICOSL vIgD-Fc fusion protein consistent with observations that CD28 receptor occupancy is positively correlated with immune inhibitory activity.

### A. PK Modeling

A lower starting dose level of 0.001 mg/kg was determined to be the Minimum Anticipated Biological Effect Level (MABEL) based on a predicted target saturation of 26% at $C_{max}$ decreasing rapidly below 10% by 24 hours post-dose. The maximum dose level was determined to be 10 mg/kg based on safety margins comparing the exposure at NOAEL dose of 150 mg/kg in cynomolgus monkeys and 200 mg/kg in rats. The predicted human $C_{max}$ and AUC at 10 mg/mkg IV doses was predicted to be 9-fold and 18-fold lower than the observed $C_{max}$ and AUC at the monkey NOAEL dose of 150 mg/kg. The predicted human $C_{max}$ and AUC at 10 mg/kg IV dose was predicted to be 9-fold and 26-fold lower than the observed Cmax and AUC at the rat NOAEL dose of 200 mg/kg.

Table 28 illustrates the predicted target saturation after a single dose of the ICOSL vIgD-Fc fusion protein. Table 29 illustrates the predicted human exposure and safety margins after a single IV dose of the ICOSL vIgD-Fc fusion protein. The target saturation and predicted exposure for SC doses (single and multiple doses) are predicted to be within the range of IV doses levels with an assumption of 70% bioavailability.

**Table 28: Predicted Target Saturation (%) After a Single IV Dose [a, b]**

| Time post-dose (day) | 0.001 mg/kg | 0.003 mg/kg | 0.01 mg/kg | 0.03 mg/kg | 0.1 mg/kg | 0.3 mg/kg | 1 mg/kg | 3 mg/kg | 6 mg/kg | 10 mg/kg |
|---|---|---|---|---|---|---|---|---|---|---|
| End of infusion | 26 | 52 | 78 | 91 | 97 | 99 | 100 | 100 | 100 | 100 |
| 0.5 | 15 | 34 | 63 | 84 | 95 | 98 | 99 | 100 | 100 | 100 |
| 1 | 7 | 18 | 43 | 69 | 88 | 96 | 99 | 100 | 100 | 100 |
| 2 | 2 | 6 | 18 | 39 | 68 | 86 | 96 | 98 | 99 | 100 |
| 7 | 0 | 1 | 5 | 13 | 32 | 59 | 83 | 93 | 97 | 98 |
| 14 | 0 | 0 | 1 | 3 | 11 | 26 | 54 | 78 | 88 | 92 |
| 21 | 0 | 0 | 0 | 1 | 3 | 8 | 23 | 47 | 64 | 74 |
| 28 | 0 | 0 | 0 | 0 | 1 | 2 | 7 | 18 | 30 | 42 |

a Target saturation was calculated based on human CD28 binding affinity $EC_{50}$= 648 pM (52 ng/mL).
b Predicted PK and target saturation are based on IV infusion of over 30 min.
$EC_{50}$ - 50% Effective Concentration

| Table 29: Predicted Human Exposure and Safety Margins after a Single IV Dose | | | | | | | | |
|---|---|---|---|---|---|---|---|---|
| | **Predicted human exposure[1]** | | **Monkey NOAEL (150 mg/kg IV weekly for 1 month)[2]** | | | **Rat NOAEL (200 mg/kg IV weekly for 1 month)[3]** | | |
| Proposed IV Dose level (mg/kg) | AUC $_{0-7d}$ (μg•day/mL) | C $_{max}$ (μg/mL) | Safety Margin by AUC | Safety Margin by C $_{max}$ | Safety Margin by Dose | Safety Margin by AUC | Safety Margin by C $_{max}$ | Safety Margin by Dose |
| 0.001 | 0.0146 | 0.0209 | 89818 | 140861 | 150000 | 85704 | 196727 | 200000 |
| 0.003 | 0.0438 | 0.0628 | 29939 | 46954 | 50000 | 28568 | 65576 | 66667 |
| 0.01 | 0.146 | 0.209 | 8982 | 14086 | 15000 | 8570 | 19673 | 20000 |
| 0.03 | 0.438 | 0.605 | 2994 | 4874 | 5000 | 2857 | 6807 | 6667 |
| 0.1 | 1.46 | 1.94 | 898 | 1518 | 1500 | 857 | 2120 | 2000 |
| 0.3 | 4.38 | 5.62 | 299 | 525 | 500 | 286 | 733 | 667 |
| 1 | 14.6 | 18.0 | 90 | 163 | 150 | 86 | 228 | 200 |
| 3 | 43.8 | 52.2 | 30 | 57 | 50 | 29 | 79 | 67 |
| 6 | 87.5 | 100.5 | 15 | 29 | 25 | 14 | 41 | 33 |
| 10 | 146 | 161 | 9 | 18 | 15 | 9 | 26 | 20 |
| 1. Linear PK was assumed for the prediction of human exposure as a conservative approach<br>2. Monkey NOAEL (150 mg/kg IV weekly for 1 month): $AUC_{0-7d}$ = 1310 μg•day/mL, $C_{max}$ = 2950 μg/mL<br>3. Rat NOAEL (200 mg/kg IV weekly for 1 month): $AUC_{0-7d}$ = 1250 μg•day/mL, $C_{max}$ = 4120 μg/mL Abbreviations: NOAEL-No-observed-adverse-effect-level; AUC-Area under curve; $AUC_{0-7d-}$ Area under curve from zero to 7 days; C Maximum observed drug concentration max - | | | | | | | | |

**B. Human Dosing**

Exemplary human single-ascending doses (SAD) and multiple-ascending doses (MAD) that consider CD28 target saturation and NOAEL safety margins as described above were designed for intravenous and subcutaneous administration to human subjects. Two single SC doses of 1 and 6 mg/kg are to be administered to human subjects to examine the absorption of SC dosing of ICOSL vIgD-Fc fusion proteins in humans.

In a single-ascending dose protocol, human subjects are to be administered the exemplary ICOSL vIgD-Fc fusion protein, N52H/N57Y/Q100R as set forth in SEQ ID NO: 36, fused to an inert Fc (e.g. containing mutations L234A, L235E and G237Ain a human IgG1 Fc, e.g. set forth in SEQ ID NO:42), or placebo either intravenously (IV) or subcutaneously (SC), as a single dose according to the dosing regimen exemplified in Table 30. At dose levels above 1 mg/kg, a single SC dose of keyhole limpet haemocyanin (KLH) is added to assess the effect of exemplary ICOSL vIgD-Fc fusion protein on the immune response.

| Table 30. SAD Proposed Dose Escalation | | | |
|---|---|---|---|
| **Cohort** | **ICOSL vIgD-Fc fusion proteins Dose (mg/kg)** | **ICOSL vIgD-Fc fusion proteins Dosing Route** | **KLH administration** |
| A1 | 0.001 | IV | - |
| A2 | 0.003 | IV | - |
| A3 | 0.01 | IV | - |
| A4 | 0.03 | IV | - |
| A5 | 0.1 | IV | - |
| A6 | 0.3 | IV | - |

(continued)

| Table 30. SAD Proposed Dose Escalation | | | |
|---|---|---|---|
| **Cohort** | **ICOSL vIgD-Fc fusion proteins Dose (mg/kg)** | **ICOSL vIgD-Fc fusion proteins Dosing Route** | **KLH administration** |
| A7 | 1 | IV | 1 mg |
| A8 | 1 | SC | 1 mg |
| A9 | 3 | IV | 1 mg |
| A10 | 6 | IV | 1 mg |
| A11 | 6 | SC | 1 mg |
| A12 | 10 | IV | 1 mg |

For a multiple ascending dose protocol, subjects are to receive 4 weekly doses for 4 weeks (4 doses in total) of placebo or the exemplary ICOSL vIgD-Fc fusion protein as outlined in **Table 31.** A single dose of KLH is proposed on day 2 to assess the effect of exemplary ICOSL vIgD-Fc fusion protein on the immune response.

| Table 31. MAD Proposed Dose Escalation | | | |
|---|---|---|---|
| **Cohort** | | | |
| | **ICOSL vIgD-Fc fusion proteins (mg/kg)** | **ICOSL vIgD-Fc fusion proteins (Dosing Route)** | **KLH Immunization** |
| B1 | 0.3 | SC | 1 mg |
| B2 | 1 | SC | 1 mg |
| B3 | 3 | SC | 1 mg |
| B4 | 6 | SC | 1 mg |

**EXAMPLE 19**

**Assessment of Variant ICOSL-IgV Fc in Models of Connective Tissue Diseases, e.g. Sjögren's Syndrome Model**

The exemplary variant ICOSL IgV-Fc containing a variant ICOSL IgV (N52H/ N57Y/Q100R; SEQ ID NO: 36) fused to an inert Fc (containing mutations L234A, L235E and G237A in a human IgG1 Fc, e.g. set forth in SEQ ID NO:42) was assessed for activity in models assessing effector mechanisms that underly Sjögren's Syndrome (SjS) and other connective tissue diseases, including systemic lupus erythematosus (SLE).

**A. PBMC stimulation assay**

For in vitro primary cell assays, PBMCs from healthy donors and from subjects with SjS were stimulated with artificial antigen presenting cells (K562 cells expressing CD80, CD86, ICOS ligand, and anti-CD3 (OKT3)), at a 20:1 ratio of cells to artificial APCs and in the presence of 100 nM of the variant ICOSL IgV-Fc or, for comparison, the CD28-only inhibitor, abatacept, the ICOS pathway inhibitor, prezalumab (AMG-557; anti-ICOSL, Creative Biolabs), or a combination of abatacept and prezalumab. Fc alone was used as a control. Supernatants were collected after a 48 hour incubation, and assayed for production of proinflammatory cytokines, including IFN-$\gamma$ and IL-2. Compared to abatacept, prezalumab or combination abatacept + prezalumab, the variant ICOSL IgV-Fc demonstrated superior suppression of pro-inflammatory cytokine (e.g. TNF-$\alpha$, IFN-$\gamma$, IL-1$\beta$, IL-2, IL-6, IL-17A, GM-CSF) release from stimulated healthy donor or SjS patient PBMCs. Exemplary results for IL-2 response are shown in **FIG. 19A** (SjS PBMC) and in **FIG. 19B** (healthy donor) and exemplary results for IFN-$\gamma$ response are shown in **FIG. 19C** (SjS PBMC) and in **FIG. 19D** (healthy donor). As shown in **FIGS. 19E** (IFN-$\gamma$) and **19F** (IL-2), results from matched donors showed that the variant ICOSL IgV-Fc consistently suppressed cytokine production to a greater extent than abatacept.

In a similar study as described above, PBMCs from healthy donors and from subjects with SjS or SLE were stimulated with artificial antigen presenting cells (K562 cells expressing CD80, CD86, ICOS ligand, and anti-CD3 (OKT3)) under conditions generally as described above, including in the presence of 100 nM of the variant ICOSL IgV-Fc or, for comparison, the CD28-only inhibitor abatacept, the ICOS pathway inhibitor prezalumab, or a combination of abatacept + prezalumab, and assayed for production of proinflammatory cytokines, including IFN-γ, IL-6 and IL-17A. Exemplary results for IFN-γ, IL-6 and IL-17A responses are shown in **FIG. 19G,** although similar trends were observed for all cytokines measured **(FIG. 19H and FIG. 19I** healthy donors PBMCs shown in right panel of **FIG. 19H** and **FIG. 19I**). Together, the results demonstrated statistically significant superiority to prezalumab, abatacept, or a combination of prezalumab+abatacept for the majority of donors and analytes tested. Together, the results demonstrate that the variant ICOSL IgV-Fc inhibited cytokine production from human SjS and SLE patents PBMCs in vitro more potentaly than single CD28 or ICOS pathway inhibitors.

RNA was isolated from patient PBMC samples generated in the assay described above, and gene expression was assessed. For comparison, RNA was also isolated from PBMC samples from patients with with psoriatic arthritis (PsA; see Example 20). Genes that were significantly downregulated across SjS, SLE and PsA are shown in **FIG. 19J.** The gene analysis of ICOSL IgV-Fc in PsA, SjS and SLE stimulated patient populations indicates that ICOSL IgV-Fc can significantly down-regulate pathogenic genes and/or pathways implicated across many autoimmune diseases. The genes significantly downregulated in response to the variant ICOSL IgV-Fc across PsA, SjS and SLE stimulated patient populations provide a gene signature for activity of the variant molecule.

**B. Human follicular B helper T (B-Tfh) Cell Co-Cultures**

The effect of exemplary variant ICOSL IgV-Fc was further evaluated for the ability to suppress proliferation and antibody responses in human B and Tfh cells. Autologous circulating human B and Tfh cells isolated from PBMCs from healthy donors were mixed with artificial APCs (K562/CD80) and soluble OKT3, and and in the presence of titrations of the variant ICOSL IgV-Fc or, for comparison, abatacept, prezalumab,the combination of abatacept + prezalumab, or belatacept. Co-Cultures were assessed on Day 7 for cellular activation/proliferation.

As shown in **FIG. 19K,** the variant ICOSL IgV-Fc suppressed T cell proliferation, CD40L+ T cell recovery and B cell proliferation, and is involved in B cell differentiation as reflected by the increase in percent of naive B cells, and parallel decrease in activated B cells, during the co-culture. The IC50 values for effects on cellular activation/proliferation are summarized in **FIG. 19L.**

In a similar study as above, supernatants from co-cultures were collected at Day 8, diluted to 1:10 and assayed for immunoglobulin secretion. Secretion of human IgG1, IgG3, IgA and IgM was substantially reduced in the presence of the exemplary variant ICOSL IgV-Fc **(FIG. 19M).** Data are representative of results from 8 donors.

RNA isolated from a B and Tfh cell co-culture generally as described above was analyzed for relative gene expression levels by next generation sequencing for genes involved in B-T cell collaboration. Expression values in Reads Per Kilobase of transcript, per Million mapped reads (RPKM) were plotted on a relative scale from 0 to 1 for each gene between treatment groups, with degree of red reflecting higher expression values and degree of blue representing lower values for each gene **(FIG. 19N).** In **FIG. 19N,** the list of genes was sorted by level of inhibition by the variant ICOSL IgV-Fc (left to right).

**C. In vivo model of Sjogren's syndrome**

The effect of the exemplary variant ICOSL IgV-Fc was compared to abatacept on inhibiting an antibody response in vivo following administration in mouse immunization models in which keyhole limpet hemocyanin (KLH) or sheep red blood cells (SRBC) were administered intraperitoneally (IP) to mice. For KLH immunization, 0.25 mg KLH was administered on Day 1, and on Day 13 as a boost. For SRBC immunization, 100 pL of washed citrated SRBC was administered on Day 0 and boost groups were dosed again on Day 14. Antibody production in the presence of the variant ICOSL IgV-Fc or abatacept also was compared in human B cell/T cell co-cultures. The variant ICOSL IgV-Fc inhibitied antibody production in both mouse immunization models. Suppressed proliferation and antibody production was also observed in human B cell/T cell co-cultures.

The effect of the exemplary variant ICOSL IgV-Fc also was assessed in a mouse model of SjS involving anti-PD-L1 antibody-mediated acceleration of sialadenitis in NOD mice. PD-L1 blockade accelerates leukocyte infiltration and caspase-3 activation in the submandibular gland (SMG), production of antinuclear and anti-M3 muscarinic acetylcholine receptor (M3R) autoantibodies and impairment of saliva secretion, indicative of accelerated development and onset of SjS, see e.g. Zhou et al. (2016) Sci. Rep. 6:39105). Six week old NOD/ShiLtJ mice were treated intraperitoneally with 0.1 mg of a blocking anti-mouse PD-L1 antibody (to induce inflammation in the salivary glands, as described in Zhou et al.) along with Fc control (0.3 mg), abatacept (0.5 mg), or the variant ICOSL IgV-Fc (0.5 mg) test articles on days 0, 2, 4, and 6. On day 8, submandibular glands were collected, sectioned, and stained with hematoxylin and eosin (H&E) stain and scored for inflammation on a scale of 0 to 3. In anti-PD-L1-treated NOD mice in this study, the variant ICOSL IgV-Fc suppressed sialadenitis, as assessed by SMG histology, greater than abatacept alone **(FIGS. 20A-20B).**

A similar study using the anti-PD-L1 mAb-induced NOD mouse model of Sjogren's syndrome was carried out as described above, except that submandibular glands, pancreas and blood/serum were harvested at Day 10. In this study, wild-type ICOSL-Fc (0.5 mg) was also administered as a test article for comparison. Representative histology H&E images are shown in **FIG. 20C** . The percent incidence of Sialadenitis is shown in **FIG. 20D,** which demonstrates that variant ICOSL IgV-Fc reduced the incidence and severity of sialadenitis. Individual SMG scores are shown in **FIG. 20E** (statistical significance was determined using the Kruskal-Wallis test (* p.05, ** p<0.01, ****p.0001. The naive group was not powered for statistical comparisons). The variant also reduced the severity of insulitis in this model, as demonstrated by histology on pancreas that had been collected at Day 10. Results of glucose measurements in blood/serum collected at Day 10 showed that the variant ICOSL IgV-Fc reduced blood glucose levels. The results demonstrated that variant ICOSL IgV-Fc reduced the incidence and severity of sialadenitis and insulitis.

Anti-Ro-52 and anti-La antibodies were assessed by ELISA (Signosis, Santa Clara, CA) in diluted serum that had been collected on Day 10. As shown in **FIG. 20F,** the variant ICOSL IgV-Fc suppressed the production of serum autoantibodies anti-RO 52 and anti-La. Statistically significant differences were observed between the variant ICOSL IgV-Fc group and comparator treatments, as determined using an uncorrected non-parametric Dunn's test.

RNA was isolated from SMG that had been collected on Day 10 and was analyzed for relative gene expression levels by next generation sequencing. **FIG. 20G** sets forth RPKM for exemplary genes. Statistical diffrences in RNA expression of genes were noted between the variant ICOSL IgV-Fc and Fc control groups, as determined using a non-parametric Kruskal-Wallis test (*p<0.05, **p<0.01)

**D. In vivo model of systemic lupus erythematosus (SLE)**

The effect of the exemplary variant ICOSL IgV-Fc also was assessed in a mouse model of SLE involving alloactivation of donor CD4+ T cells by recipient antigen-presenting cells leading to a cGvHD disease with symptoms resembling SLE (Klarquist & Jansse, 2015, J. Vis. Exp. (105):e53319). Splenocyte suspensions from female I-$A^{bm12}$B6(C)-*H2*-Ab1$^{bm12}$/KhEgJ ('bm12') mice were injected IP into an equivalent number (1:1) of 9 week-old female C57BL/6NJ recipient mice on Day 0. H2-Ab1$^{bm12}$ differs from H2-Ab1$^{b}$ by 3 nucleotides, resulting in alteration of three amino acids in the β-chain of the MHC I-A molecule. Starting on Day 0, recipient mice were treated twice a week for 11 weeks with 400 μg variant ICOSL IgV-or abatacept, or a molar-matched amount (250 μg) of Fc control protein. At study termination on Day 82, germinal center (B220+CD19+GL7+CD95+) B cells and follicular helper (CD4+CD45+CD3+PD1+CD185+) T cells were enumerated by flow cytometry. Anti-double stranded DNA IgG titers were measured on Day 14 using the Mouse Anti-dsDNA IgG Antibody Assay kit from Chondrex (Redmond WA).

As shown in **FIG. 20H,** exemplary variant ICOSL IgV-Fc treated mice demonstrated decreased levels of GC B and CD4+Tfh cells per spleen, and decreased serum anti-dsDNA compared to Fc control. Statistically significant differences were observed beween the variant ICOSL IgV-Fc and the Fc control group for GC B and Tfh using an unpaired t-test, and for anti-dsDNA concentrations using an uncorrected Fisher's least significance difference test (*p<0.05, **p<0.01, and ****p<0.0001).

These results further support the observation that a dual CD28/ICOS antagonist, such as the exemplary tested variant ICOSL IgV-Fc, is superior to CD28 or ICOS costimulatory pathway inhibitors, administered individually or in combination, in human in vitro and/or mouse in vivo translational studies.

**EXAMPLE 20**

**Assessment of Variant ICOSL-IgV Fc in Arthritis Model**

The exemplary variant ICOSL IgV-Fc containing a variant ICOSL IgV (N52H/ N57Y/Q100R; SEQ ID NO: 36) fused to an inert Fc (containing mutations L234A, L235E and G237A in a human IgG1 Fc, e.g. set forth in SEQ ID NO:42) was assessed for activity in models assessing effector mechanisms that underly rheumatoid arthritis (RA) or psoriatic arthritis (PsA).

**A. PBMC stimulation assay**

For in vitro primary cell assays, PBMCs from healthy donor (HD), RA, and PsA subjects or Th17-skewed T cell cultures were stimulated with artificial antigen presenting cells (K562 cells expressing CD80, CD86, ICOSL, and anti-CD3 (OKT3)), at a 20:1 ratio of cells to artificial APCs and in the presence of 100 nM of the variant ICOSL IgV-Fc or, for comparison, the CD28-only inhibitor, abatacept, the ICOS pathway inhibitor, prezalumab (AMG-557; anti-ICOSL, Creative Biolabs), and a combination of abatacept and prezalumab. Fc alone was used as a control.

Supernatants were collected after a 48-hour incubation for assessment of cytokine production. The variant ICOSL IgV-Fc demonstrated superior suppression of pro-inflammatory cytokine (e.g. TNF-α, IFN-γ, IL-1β, IL-2, IL-6, IL-17A, GM-CSF, RANKL) release from stimulated healthy and patient PBMCs, and suppressed T cell proliferation in Th17-skewed cultures compared to abatacept, prezalumab, and combination of abatacept and prezalumab. Exemplary results from cells from co-cultures with PBMCs from RA, PsA or HD are shown for IFN-γ **(FIG. 21A),** IL-2 **(FIG. 21B),** TNF-α **(FIG. 21C)** and IL-17A **(FIG. 21D).** The percent inhibition of cytokine secretion relative to Fc control was determined by the formula ((Fc control value - Exp value)/Fc control value))*100, and is shown in **FIG. 21E.** The results show that the variant ICOSL IgV-Fc demonstrated greater cytokine inhibition than observed with abatacept or prezalumab alone or combined.

RNA was isolated from patient PBMC samples generated in the assay described above, and gene expression was assessed by RNA sequencing. For assesement of genes differentially reduced by variant ICOSL IgV-Fc the cutoff criteria was FDR≤0.05, LOG FC<-0.5. For assessment of genes differentially reduced by abatacept the cutoff criteria was FDR≤ 0.3, LOG FC <-0.5. As shown in **FIG. 22A,** more genes associated with disease pathogenesis were significantly downregulated by ICOSL IgV-Fc than abatacept alone or in combination with prezalumab. ICOS-inhibition (ICOSL IgV-Fc or abatacept plus prezalumab) were required for down regulation of IL-17A and IL-17F. In this study, significant gene reduction and/or modulation by ICOSL IgV-Fc vs comparators was observed for: Th17-associated effector molecules **(FIG. 22B),** costimulatory molecules **(FIG. 22C)** and inflammatory TH1 or TH2-associated effector molecules **(FIG. 22D).**

### B. TH17-Skewed Activity

Suppression of Th17-skewed cells by ICOSL IgV-Fc was further evaluated and compared to CD28 or ICOS only inhibitors. Human PBMCs underwent primary stimulation with aAPCs generally as described above, except under conditions for no skewing (K562/OKT3/CD80 + CD86 + IL-2) or Th17 Skewing (K562/OKT3/ICOSL + Th17 skewing media (Cat#CDK003, R&D Systems)), and then were re-fed with cytokines and allowed to rest for approximately 10-14 days. Rested cells then underwent a secondary stimulation in a mixed lymphocyte reaction (MLR) using up to 100,000 T cells with 10,000 allogenic LPS-activated monocyte-derived dentritic cells with 3,200 pM variant ICOSL IgV-Fc or comparator protein.

Four days post-stimulation, cells were analyzed by flow cytometry for ICOS or CD28 expression as shown in **(FIG. 23A),** which shows altered CD28 and ICOS expression in TH17-skewed cells including following restimulation. Assessment of T cell proliferation **(FIG. 23B)** and IL-17A cytokine secretion was measured by ELISA **(FIG. 23C)** in stimulated cells showed that the variant ICOSL IgV-Fc exhibited greater suppression of TH17-skewed cells versus CD218 or ICOS only inhibitors.

### C. In vivo Model of Collagen-Induced Arthritis (CIA)

The effect of the exemplary variant ICOSL IgV-Fc was compared to abatacept in vivo following administrationin a collagen-induced arthritis (CIA) model in which male DBA/1 mice were immunized with bovine collagen in Freund's adjuvant on days 0 and 18. The administration of the variant ICOSL IgV-Fc starting at or after the collagen boost on Day 18 consistently resulted in significant disease reduction in the mouse CIA model (including paw inflammation, serum cytokines, and anti-collagen antibodies), matching or exceeding that the activity of abatacept.

In a similar study, mice were treated intraperitoneally (IP) every 3 days for a total of 5 doses (Q3Dx5) with vehicle (Dulbecco's phosphate buffered saline/DPBS), Fc control, abatacept (Orencia™), or exemplary variant ICOSL IgV-Fc or abatacept in a semi-therapeutic regimen, starting before administration of the collagen boost on Day 18. PBS and Fc alone were used as controls. Administration of ICOSL IgV-Fc significantly reduced paw inflammation and structural damage **(FIG. 24A).** Toluidine blue staining of decalcified paws demonstrated absence of inflammation, pannus, cartilage damage and bone resorption when treated with exemplary variant ICOSL IgV-Fc **(FIG. 24B).** Specifically, in PBS-treated animals (top, left panel), the staining showed severe diffuse inflammatory infiltrate with severe edema with total or near total destruction of joint architecture, with multifocal chrondrocyte loss and full thickness defects in cortical bone with destruction of joint architecture in a PBS-treated animal. In Fc control-treated animals (top, right panel), the staining showed devere diffuse inflammatory infiltrate with severe edema with extensive destruction of joint architecture, with marked chondrocyte loss and full thickness defects in cortical bone with distortion of the cortical surface. In abatacept-treated animals (bottom, left panel), the staining showed moderate to severe inflammatory infiltrate with marked destruction of joint architecture, minimal to mild disruption of structure and some areas of bone resorption in an abatacept-treated animal. In animals treated with variant ICOSL IgV-Fc (bottom, right panel), the staining showed absence of inflammation, pannus, cartilage damage or bone resorption.

Popliteal LNs were harvested and processed into single cell suspensions and surface stained for various markers including: $T_{FH}$ cells (CD3, CD4, CXCR5, and PD-1); germinal center (GC) B-cells (CD19, GL-7, CD95). For intracellular cytokine staining, cells were stimulated for 5 hours with PMA/Ionomycin in the presence of Golgi Stop and Golgi Plug. Cells were then surface stained for CD3, CD4, fixed, permeabilized and stained for IFN-gamma or IL-17A, and analyzed by flow cytometry. Administration of exemplary variant ICOSL IgV-Fc significantly reduced the number of cytokines as measured by the percent of CD4+ or total live cells **(FIG. 24C).** The concentration of total anti-mouse Type II collagen IgG antibody was significantly reduced in the sera of mice immunized with exemplary variant ICOSL IgV-Fc, as determined by ELISA (Chondrex, Cat#2036T) **(FIG. 24D).**

Percent inhibition of cytokine was determined for individual mice from the Fc control, Abatacept, or exemplary variant ICOSL IgV-Fc -treated mice relative to mice treated with vehicle (DPBS) using the following formula: ((Ave DPBS - Exp Animal)/Ave DPBS))*100. The number of cytokines present in the serum was significantly reduced in mice immunized with exemplary variant ICOSL IgV-Fc **(FIG. 24E).**

These results are consistent with an observation that immunosuppressive activity of a dual CD28/ICOS antagonist, such as the tested variant ICOSL IgV-Fc, is superior to CD28 or ICOS costimulatory pathway inhibitors, administered individually or in combination, in human in vitro and/or mouse in vivo translational studies, and support its use for treatment of inflammatory diseases including rheumatoid, psoriatic, and juvenile idiopathic arthritis.

**EXAMPLE 21**

**Administration of Variant ICOSL-IgV Fc in Healthy Subjects**

Healthy adult subjects were administered the exemplary ICOSL vIgD-Fc fusion protein, N52H/N57Y/Q100R as set forth in SEQ ID NO: 36, fused to an inert Fc (containing mutations L234A, L235E and G237A in a human IgG1 Fc, e.g. set forth in SEQ ID NO:42). Both a single ascending dose study (SAD) and a multiple ascending dose (MAD) study were performed. Safety, tolerability, pharmacokinetics (PK), and pharmacodynamics (PD) were analyzed.

*A. Single Ascending Dose Study (SAD)*

The subjects (n=72) were divided among ten intravenous (IV) cohorts and two subcutaneous (SC) cohorts. Subjects in the IV cohorts received a single dose of the ICOSL vIgD-Fc fusion protein (dosed at either 1 μg/kg, 3 μg/kg, 6 μg/kg, 12 μg/kg, 30 μg/kg, 0.1 mg/kg, 0.3 mg/kg, 1 mg/kg, 3 mg/kg, or 10 mg/kg) or placebo by a single intravenous infusion on day 1 and were monitored for an additional 28 days. Subjects in the SC cohorts received a single dose of the fusion protein (either 1 mg/kg or 3 mg/kg) or placebo by subcutaneous injection on day 1 and were monitored for an additional 28 days.

The PK of the ICOSL vIgD-Fc fusion protein was assessed in all cohorts by monitoring the concentration of the variant ICOSL vIgD-Fc in serum at various time points over 28 days. The concentration of the ICOSL vIgD-Fc fusion protein in serum was measured by enzyme-linked immunosorbent assay (ELISA), using an anti-human ICOSL capture antibody and a mouse anti-human IgG-HRP antibody conjugate to detect captured antigen. For all dose cohorts, the mean concentration of the exemplary fusion protein was determined at each time point and plotted over time (IV cohorts shown in **FIG. 25A;** all cohorts shown in **FIG. 33).** The mean estimated half-life was 4.3 - 8.6 days over 1 - 10 mg/kg, and SC bioavailability was -60% at 3 mg/kg.

The CD4+ target saturation was also assessed in all cohorts over 28 days.

Serial whole blood samples (1 mL) were collected predose and postdose at 10 min, 2 hr, 24 hr (1 day), 3, 7, 14, 21, and 28 days after the end of administration. Whole blood samples were lysed and the cell pellet was divided into 3 aliquots. A saturating concentration (400 ng/mL) of the exemplary variant ICOSL IgV-Fc was added to one of the aliquots followed by incubation with human Fc block to reduce nonspecific binding. Target engagement and sub-populations of the circulating lymphocytes were quantified using by flow cytometry.

For calculation of target saturation, median fluorescence intensity (MFI) of CD4+ cells that stained positively for the anti-human IgG (vs. FMO control) were reported. Target saturation was determined using the following formula:

$$\%\ Target\ Saturation = \left(\frac{MFI_{test\ sample}}{MFI_{saturated\ sample}}\right) \times 100\% .$$

In all subjects who received the exemplary variant ICOSL IgV-Fc, the target saturation of the exemplary variant ICOSL IgV-Fc on CD4+ T cells and on CD8+ T cells from each blood sample were consistently similar across SAD dose groups.

Binding of the variant ICOSL vIgD-Fc to CD4+ cells was detected by co-staining cells with an anti-CD4 antibody and an anti-human IgG Fc antibody (IV cohorts shown in **FIG. 25B;** all SAD cohorts shown in **FIG. 34** ).

Following single IV dose administration of the exemplary variant ICOSL IgV-Fc, target saturation of variant ICOSL IgV-Fc in CD4+ T cells peaked at the end of infusion. The mean maximum target saturation increased from 22.9% at 0.001 mg/kg to 99.2% at 0.1 mg/kg.

The mean duration of high target saturation (>95%) increased in a dose-dependent manner from 1.39 days at 0.03 mg/kg to at least 28 days at 10 mg/kg. After 28 days, the mean target saturation was low (< 10 %) at doses below 1 mg/kg and ranged from 58.1 % at 1 mg/kg to 99.4% at 10 mg/kg.

Following 1 and 3 mg/kg subcutaneous dose administration, the mean time to reach high target saturation (> 95%) was 1 and 0.083 days, respectively. High target saturation (> 95%) was maintained on average for 14.7 days and at least 28 days with the mean target saturation at 28 days post-dose calculated at 35.8% and 97.7% for 1 and 3 mg/kg, respectively.

The results showed that IV infusion of the ICOSL vIgD-Fc fusion protein achieved sustained high-level target saturation.

The ability of the ICOSL vIgD-Fc fusion protein to exert dose-dependent immunomodulation in healthy subjects was assessed. To measure subjects' humoral response, subjects were given a single subcutaneous (SC) injection of 1 mg of keyhole limpet hemocyanin (KLH), a strong immunostimulant, on day 1 (IV cohorts) or day 2 (SC cohorts) of the study. Antibody response was assessed by measuring the ratio of anti-KLH IgG and IgM antibodies in serum, before and after exposure to KLH. Blood samples for detecting anti-KLH antibodies were collected predose, 14, 21 and 28 days postdose. Serum levels of IgG and IgM antibodies were quantitatively measured by ELISA, using KLH to capture anti-KLH antibodies, and an HRP-conjugated secondary antibody specific to either IgG or IgM. The anti-KLH IgG and IgM response rates, calculated as the percentage of subjects manifesting an increase in antibody concentration of >2-fold above baseline level, were 100% and 69% in placebo group, respectively.

The anti-KLH IgG response rates were lower in variant ICOSL vIgD-Fc treated groups than the placebo group. The anti-KLH IgM response rates were also reduced in variant ICOSL vIgD-Fc treated groups. **Table 34** below shows the incidence of elevated anti-KLH antibody response by treatment. As shown in **FIG. 35,** changes in anti-KLH IgG or IgM in all variant ICOSL IgV-Fc treatment groups were lower than the placebo group, indicating inhibition of antigen-specific antibody responses by the exemplary variant ICOSL IgV-Fc.

**Table 34:** Incidence of elevated anti-KLH antibody response (>2-fold of baseline) by SAD treatment.

| Treatment (Total N) | Elevated anti-KLH IgG at any timepoint | Elevated anti-KLH IgM at any timepoint |
|---|---|---|
| Placebo (N=16) | 16 (100%) | 11 (69%) |
| 1 mg/kg (N=4) | 2 (50%) | 2 (50%) |
| 3 mg/kg (N=4) | 1 (25%) | 1 (25%) |
| 10 mg kg (N=4) | 0 (0%) | 1 (25%) |
| 1 mg/kg, SC (N=4) | 1 (25%) | 0 (0%) |
| 3 mg/kg, SC (N=4) | 1 (25%) | 1 (25%) |

The results revealed that ICOSL vIgD-Fc fusion protein was able to suppress antibody response to KLH at doses of 1 mg/kg, 3 mg/kg, and 10 mg/kg in IV and SC cohorts (IV cohorts shown in **FIG. 26A).**

A separate assay was performed to measure the subjects' antigen-dependent T cell response.

Serial whole blood samples for ex vivo staphylococcal enterotoxin B stimulation were collected predose and postdose at 2 hr, 24 hr (1 day), 3, 7, 14, and 28 days after the end of administration. Subjects' T cells were cultured *ex vivo* and IL-2 production by the cells was measured for 3 days following exposure to staphylococcal enterotoxin B (SEB) and treatment with the exemplary fusion protein.

The results showed that IL-2 production by T cells was inhibited by the fusion protein at doses of 0.03 mg/kg and higher **(FIG. 26B).** Baseline-adjusted SEB induced IL-2 was reduced by exemplary variant ICOSL IgV-Fc treatment as early as 2 hr postdose, with recovery time increasing with increasing exemplary variant ICOSL IgV-Fc dose. The duration and the magnitude of the inhibition by the exemplary variant ICOSL IgV-Fc was dose dependent. The maximum inhibition of IL-2 by the exemplary variant ICOSL IgV-Fc increased with increasing dose from dose 0.001 mg/kg (-13%) to 0.03 mg/kg (-49.5%) and remained approximately -50% at doses above 0.03 mg/kg. Similarly, the duration of inhibition (defined as time ≤ -25%) also increased with increasing doses from 1.08 day at 0.003 mg/kg to 19.9 days at 10 mg/kg. These results are consistent with an observation that the variant ICOSL vIgD-Fc mediated dose-dependent immunomodulation following single dose IV administration to human subjects.

Together the results showed that the ICOSL vIgD-Fc fusion protein was well tolerated in the subjects at all dose levels, including up to the highest 10 mg/kg dose level. No grade 3 or grade 4 adverse events occurred. Additionally, no dose-related laboratory changes, infusion reactions, cytokine release syndrome (CRS), or anaphylaxis were observed in the study.

These results are consistent with an observation that the exemplary ICOSL vIgD-Fc fusion protein is well tolerated in healthy subjects, exhibits dose-dependent immunomodulatory activity, and does not result in CRS or immunogenicity at the doses tested.

*B. Multiple Ascending Dose Study (MAD)*

The MAD study included 2 repeated weekly IV dose cohorts (0.3 or 1 mg/kg Q1W, total 4 doses) and 1 repeated every-two-weeks IV dose cohort (1 mg/kg Q2W, total 2 doses). A total number of 24 subjects (8 subjects per dose cohort) were randomized to receive the exemplary variant ICOSL IgV-Fc or placebo at a 6:2 ratio.

Subjects were followed for 49 day (MAD) to assess safety, PK, target saturation on T cells, circulating cytokines and PD, the latter based on suppression of IgG responses to keyhole limpet hemocyanin (KLH).

The PK of the ICOSL vIgD-Fc fusion protein was assessed in all MAD cohorts by monitoring the concentration of the variant ICOSL vIgD-Fc in serum at various time points over 28 or 49 days. The concentration of the ICOSL vIgD-Fc fusion protein in serum was measured by enzyme-linked immunosorbent assay (ELISA), using an anti-human ICOSL capture antibody and a mouse anti-human IgG-HRP antibody conjugate to detect captured antigen. **FIG. 36** shows the pharmacodynamics of each MAD cohort. Minimal to modest accumulation was observed with repeat IV dosing.

To assess target saturation of the exemplary variant ICOSL IgV-Fc, the amount of the exemplary variant ICOSL IgV-Fc on circulating CD4+ and CD8+ T cells was determined according to the methods described above for the SAD study, except that serial whole blood samples (1 mL) were collected predose and postdose at 4 hr, 24 hr (1 day), 2 day after the end of infusion of each dose, and 7 and 28 days Q1W cohorts after the end of infusion of the fourth dose, and 8, 9, 14, and 35 days for Q2W cohort after the end of infusion of the second dose.

In all subjects who received the exemplary variant ICOSL IgV-Fc, the target saturation of the exemplary variant ICOSL IgV-Fc on CD4+ T cells and on CD8+ T cells from each blood sample were consistently similar across dose groups. **FIG. 37** summarizes target saturation in CD4+ T cells over time.

Following 4 (Q1W, for 4 weeks) intravenous dose administrations at 0.3 and 1 mg/kg or 2 administrations (Q2W, for 4 weeks) at 1 mg/kg, high target saturation (>95%) was achieved after dose administration and generally maintained for 26.8 to 39 days. At the last scheduled timepoint (49 days post the first dose), the mean target saturation decreased to 19.5, 26.6, and 91.7% for 0.3 mg/kg Q1W, 1 mg/kg Q2W, and 1 mg/kg Q1W, respectively.

These results show that multiple IV infusions of the ICOSL vIgD-Fc fusion protein achieve sustained high-level target saturation.

The ability of the ICOSL vIgD-Fc fusion protein to exert dose-dependent immunomodulation in healthy subjects receiving multiple doses of the fusion protein was assessed.

All MAD cohorts received a subcutaneous administration of 1 mg Keyhole Limpet Hemocyanin (KLH) within 2 hours following variant ICOSL IgV-Fc or placebo administration on day 1. Blood samples for anti-KLH antibody were collected predose, 7, 14, 21, 28, and 49 days post the first dose.

As shown in **FIG. 38,** changes in anti-KLH IgG or IgM in all variant ICOSL IgV-Fc treatment groups were lower than the placebo group, indicating inhibition of antigen-specific antibody responses by the exemplary variant ICOSL IgV-Fc. **Table 35** below shows the incidence of elevated anti-KLH antibody response by treatment.

**Table 35:** Incidence of elevated anti-KLH antibody response (>2-fold of baseline) by MAD treatment.

| Treatment (Total N) | Elevated anti-KLH IgG at any timepoint | Elevated anti-KLH IgM at any timepoint |
|---|---|---|
| 0.3 mg/kg, Q1W (N=5) | 4 (75%) | 1 (20%) |
| 1 mg/kg, Q1W (N=4) | 2 (50%) | 1 (25%) |
| 1 mg/kg, Q2W (N=5) | 1 (20%) | 3 (60%) |

Note: 4 subjects (1 in 0.3 mg/kg Q1W, 2 in 1 mg.kg Q1W and 1 in 1 mg/kg Q2W) were excluded because these subjects didn't receive the full planned doses.

The results revealed that multiple doses of ICOSL vIgD-Fc fusion protein were able to suppress antibody response to KLH.

An assay was performed to measure antigen-dependent T cell response in the MAD cohort.

Serial whole blood samples for ex vivo staphylococcal enterotoxin B (SEB) stimulation were collected predose prior to each dose (0, 7, 14, 21 days), 2 days after the first and fourth doses (3 and 23 days), 28 and 49. Subjects' T cells were cultured *ex vivo* and IL-2 production by the cells was measured for 3 days following exposure to staphylococcal enterotoxin B (SEB) and treatment with the exemplary fusion protein.

After 4 weekly doses of the exemplary variant ICOSL IgV-Fc, duration of inhibition increased from 25.1 days at 0.3 mg/kg to 44.5 days at 1 mg/kg. These results are consistent with an observation that the variant ICOSL vIgD-Fc mediated dose-dependent immunomodulation following multiple IV administrations to human subjects.

Together, these results are consistent with an observation that the exemplary variant ICOSL IgV-Fc fusion protein is well tolerated in healthy subjects, exhibits dose-dependent immunomodulatory activity, and does not result in CRS or immunogenicity when administered as multiple doses.

## EXAMPLE 22

### Administration of Variant ICOSL-IgV Fc in Subjects with Steroid-Resistant or Steroid-Refractory Acute Graft Versus Host Disease (aGVHD)

Adults receiving an allogeneic stem cell transplant (*e.g.*, a first allogeneic stem cell transplant) with Grade II-IV aGVHD per Mount Sinai Acute GVHD international Consortium (MAGIC) criteria (Harris et al. 2016) that are resistant or refractory to an immunosuppressant, such as corticosteroids (*e.g.*, glucocorticoids) or cyclosporine, are administered a single intravenous dose of the variant ICOSL vIgD-Fc fusion protein, N52H/N57Y/Q100R as set forth in SEQ ID NO: 36, fused to an inert Fc (e.g. containing mutations L234A, L235E and G237Ain a human IgG1 Fc, e.g. set forth in SEQ ID NO:42), in combination with a salvage therapy. The administration is part of a clinical study that is conducted in two parts including dose escalation and dose expansion. Dose escalation proceeds by using an accelerated titration design. Subjects are administered a single intravenous infusion of one of the following doses and evaluated as part of the dose escalations study: 1 μg/kg, 10 μg/kg, 30 μg/kg, 0.1 mg/kg, 0.3 mg/kg, 6 mg/kg, 10 mg/kg, and 20 mg/kg. Thereafter, a selected dose level(s) is evaluated in an expansion cohort of 10 subject; if ≥ 25% (n=3) subjects achieve response, 15 additional subjects are enrolled (Simon two-stage design). In some cases, subjects may continue therapies administered for prophylaxis, continue or increase glucocorticoids and/or add another salvage therapy. Responders are considered for glucocorticoid taper.

The dosing protocol described above is based on the predicted human PK profile and CD28 target saturation from non-clinical data, and a safety margin for the predicted human exposure based on the NOAEL toxicology studies.

A further dosing protocol is designed based on the studies in healthy human subjects as described in Example 21. A starting dose of 0.3 mg/kg is deemed feasible based on observed clinical safety data, PK and target saturation following the single or multiple IV doses in the healthy subjects in the study in Example 21. Dose escalation proceeds as described above using an accelerated titration design. Subjects are administered a single intravenous infusion of one of the following doses or an intermediate dose between any of the following doses (up to 20 mg/kg) and evaluated as part of the dose escalations study: 0.3 mg/kg, 1 mg/kg, 3 mg/kg, 6 mg/kg, 10 mg/kg, 15 mg/kg, and 20 mg/kg. Thereafter, a selected dose level(s) is evaluated in an expansion cohort of 10 subject; if ≥ 25% (n=3) subjects achieve response, 15 additional subjects are enrolled (Simon two-stage design). In some cases, subjects may continue therapies administered for prophylaxis, continue or increase glucocorticoids and/or add another salvage therapy. Responders are considered for glucocorticoid taper.

Safety is assessed over 28 days, based on the incidence, severity and seriousness of adverse events. Efficacy is assessed for endpoints that include objective response rate, duration of response, relapse-free survival, failure-free survival, event-free survival, non-relapse mortality, malignancy relapse/progression, overall survival, and glucocorticoid use.

The incidence and titer of anti-drug antibodies is assessed. Serum concentration of the variant ICOSL vIgD-Fc is measured and pharmacokinetic (PK) parameters are estimated. Pharmacodynamic (PD) endpoints are assessed and include target saturation and immunophenotyping of circulating leucocytes, and may include quantification of circulating cytokines, immunoglobulins, acture phase reactants, and soluble forms of the targeted pathway receptor, evaluation of changes in mRNA expression in circulating leucocytes, evaluation of risk alleles, and correlates of response.

## EXAMPLE 23

### In Vitro Assessment of Variant ICOSL-IgV Fc via Patient Derived PBMCs

The effect of the exemplary variant ICOSL IgV-Fc containing a variant ICOSL IgV (N52H/ N57Y/Q100R; SEQ ID NO: 36) fused to an inert Fc (containing mutations L234A, L235E and G237A in a human IgG1 Fc, e.g. set forth in SEQ ID NO:42) on cytokine release was evaluated.

PBMCs isolated from patients with Crohn's Disease (CD) or Ulcerative Colitis (UC) were stimulated at a 20:1 ratio with artificial antigen presenting cells (K562 cells expressing cell surface anti-CD3 (OKT3), CD80, CD86, and ICOSL), in the presence of 100 nM of the exemplary variant ICOSL IgV-Fc, single pathway antagonists prezalumab or abatacept, or a combination of both prezalumab or abatacept. Supernatants were collected after 48 hours and cytokine concentrations were analyzed as shown in **FIG. 27A.** TNF-alpha, IFNγ, IL-6, and IL-12p70 release from patient cells treated with exemplary variant ICOSL IgV-Fc were downregulated compared to cells treated with either single pathway antagonists, a combination of both, or an Fc control antibody.

Percent inhibition of cytokine release was calculated with respect to the Fc control treatment using the following formula : ((Fc control value - Experimental value)/Fc control value))*100. As shown in **FIG. 27B,** the exemplary variant ICOSL IgV-Fc demonstrated a greater cytokine inhibition than was observed following treatment with either abatacept or prezalumab.

**EXAMPLE 24**

**Assessment of Multiple Dosing of Variant ICOSL-IgV Fc in a T Cell-Induced Mouse Model of Colitis**

The effect of the exemplary variant ICOSL IgV-Fc containing a variant ICOSL IgV (N52H/ N57Y/Q100R; SEQ ID NO: 36) fused to an inert Fc (containing mutations L234A, L235E and G237A in a human IgG1 Fc, e.g. set forth in SEQ ID NO:42) on disease progression and severity in a CD4+CD45RBhigh T cell-induced murine model of colitis was evaluated.

CD4+CD45RBhigh donor cells were enriched by negative selection from spleen cell suspensions obtained from male BALB/C donor mice. On Day 0, 0.3 million CD4+CD45RBhigh Treg depleted donor cells or CD4+ cells were injected intravenously into immunodeficient C.B17 (SCID) male mice to induce colitis or to serve as a control group, respectively. The mice were divided into six treatment groups and treated with exemplary variant ICOSL-IgV Fc, Fc only, or a vehicle control on Day 0 and/or throughout the study as indicated in **Table 32.**

**Table 32: Treatment Regimens**

| Group | # mice | Cells injected | Treatment | Dose | Route | Frequency |
|---|---|---|---|---|---|---|
| 1 | 12 | CD4+ | Vehicle | - | i.p. in 0.1 mL | 2x/week (Day 0-41) |
| 2 | 16 | CD4+CD45RBhigh | Fc Control | 260 μg | i.p. in 0.1 mL | 2x/week (Day 0-41) |
| 3 | 12 | CD4+CD45RBhigh | Variant ICOSL IgV-Fc | 400 μg | i.p. in 0.1 mL | 2x/week (Day 0-41) |
| 4 | 12 | CD4+CD45RBhigh | Variant ICOSL IgV-Fc | 400 μg | i.p. in 0.1 mL | 1x/week (Day 0) |
| 5 | 12 | CD4+CD45RBhigh | Variant ICOSL IgV-Fc | 400 μg | i.p. in 0.1 mL | 1x/week (Day 14) |
| 6 | 12 | CD4+CD45RBhigh | Variant ICOSL IgV-Fc | 400 μg | i.p. in 0.1 mL | 2x/week (Day 14-41) |

Body weight and stool score measurements were taken each day starting on Day 0. Blood was collected for analysis on Days 13, 20, and 27. After the study was terminated on Day 42 or 43, mesenteric lymph node, colon, and small intestine were collected for cytometric analysis, determination of length and weight, as well as histological analysis. The distal 1/3 of colon was maintained as an *ex vivo* culture to assess cytokine secretion in complement with cytokine measurements taken from serum. Disease activity index (DAI) was calculated from body weight and stool scores (body weight score + stool score) as previously described in **Example 16.** Statistical analysis of DAI of the exemplary variant ICOSL-IgV Fc treated group with the Fc control group was assessed using a two-way ANOVA.

Changes in body weight, stool scores, and DAI results are shown in **FIG. 28A,** Improvement in overall DAI as well as in body weight and stool score was seen with exemplary variant ICOSL IgV-Fc (N52H/N57Y/Q100R) treatment, including treatment with a single dose on Day 0 or Day 14.

**FIG 28B** shows the result of flow cytometric analyses for T cell count. As shown, treatment with exemplary variant ICOSL IgV-Fc was also shown to contribute to a suppression of effector memory T cells (Tern) both in the blood and mesenteric lymph nodes.

A decrease in the serum concentration of proinflammatory cytokines TNFα, IL-6, IFNγ, and IL-12p70 also was observed following treatment with the exemplary variant ICOSL-IgV-Fc **(FIG. 28C).**

Macroscopic and microscopic assessment of the colon can be seen in **FIG. 28D** and **FIGS. 28E-28F,** respectively. As shown in **FIG. 28D,** analysis revealed that all therapeutic treatment conditions showed a reduced weight:length ratio and had lower combined histological scores compared to the Fc control treated group. Histological analysis confirmed efficacy of the exemplary variant ICOSL IgV-Fc in reducing colitis. Histological analysis of mice treated with repeated doses of the variant ICOSL IgV-Fc are shown in **FIG. 28E** in the two right panels, compared to the vehicle only, no colitis treated group (top left panel) or Fc control treated group (bottom left panel). Mice treated by repeat dose from Day 0 to the end of the study had no pathological colon findings (top right panel), and 4/12 mice treated by delayed repeat dose from Day 14 to end of study had no signs of colitis (bottom right panel). **FIG. 28F** shows higher magnification histological images of those in FIG. 28E. None of the mice in the treatment groups had their muscularis layer of colon affected by inflammation. These results are consistent with the finding that the variant ICOSL IgV-Fc completely prevents development of colitis even with delayed repeat dose administration. Mice treated with a single dose variant ICOSL IgV-Fc at day 0 or day 14 also exhibited milder colitis than Fc control-treated mice.

These data support exemplary variant ICOSL IgV-Fc as a therapeutic candidate for the treatment of inflammatory bowel disorders, such as colitis.

**EXAMPLE 25**

**Assessment of Intravitreal Administration of Variant ICOSL-IgV Fc in Experimental Autoimmune Uveitis**

The effect of intravitreal injections of the exemplary variant ICOSL IgV-Fc containing a variant ICOSL IgV (N52H/ N57Y/Q100R; SEQ ID NO: 36) fused to an inert Fc (containing mutations L234A, L235E and G237A in a human IgG1 Fc, e.g. set forth in SEQ ID NO:42) on experimental autoimmune uveitis (EAU) was evaluated.

Twenty (20) female Lewis rats were divided into 5 treatment groups of 4 animals each. Two (2) experimental rounds were performed with 2 animals per treatment per round. On day zero, EAU was induced in all animals via a subcutaneous injection of 30 μg of bovine interphotoreceptor retinoid binding protein (IRBP) peptide R16 in 200 μl complete Freund's adjuvant (100 μl to each hip). Animals in groups 1-4 (n = 4 animals per group, total n = 16) were treated with PBS, Fc control, steroid, or exemplary variant ICOSL IgV-Fc by intraocular injection into the right vitreous on days 10 and 12. Animals in group 5 (n = 4) were treated with exemplary variant ICOSL IgV-Fc on days 8 and 10. Treatment regimens are summarized in **Table 33.**

| **Table 33: Treatment regimens** | | | | |
|---|---|---|---|---|
| **Group** | **Treatment** | **Dose** | **Route** | **Frequency** |
| 1 | Vehicle | 1x PBS | Intravitreal injection in 3-3.5 μι. | 2x (days 10/12) |
| 2 | Fc Control (SEQ ID NO: 42) | 40 μg | Intravitreal injection in 3-3.5 μι. | 2x (days 10/12) |
| 3 | Triamcinolone Acetonide (Steroid) | 120 μg | Intravitreal injection in 3-3.5 μι. | 2x (days 10/12) |
| 4 | Variant ICOSL IgV-Fc (SEQ ID NO: 36) | 80 μg | Intravitreal injection in 3-3.5 μι. | 2x (days 10/12) |
| 5 | Variant ICOSL IgV-Fc (SEQ ID NO: 36) | 80 μg | Intravitreal injection in 3-3.5 μι. | 2x (days 8/10) |

Injections were performed under anesthesia (i.p. ketamine/Xylazine and topical tetracaine). Eyes were cleaned with 5% betadine and pupils dilated with 2.5% phenylephrine. Injections were performed under direct visualization using a glass needle and a dissection microscope. Intravitreal placement of the study compound was verified for each injection.

Animals were weighed at baseline and on all days when anesthesia was administered. Unmasked clinical scores of both eyes were obtained on days 0, 7, 8, 9, 10, 11, 12, 13, and 14. Scores were assigned by observation of unanesthetized animals using an established score system (Agarwal and Caspi, 2004). Optical coherence tomography (OCT) images were obtained on days 7, 11, and 14. Images were scored by graders masked to treatment and day. Scores were assigned using an image analysis protocol adapted from (Pepple and Wang 2016). On day 14, the rats were euthanized and eyes were collected for histology. Histologic sections (n = 3 per eye) were scored by a grader masked to eye and treatment condition using an established score system (Dick and Forrester, 1994). Median or mean clinical scores (masked or unmasked), masked OCT scores, and histology scores were determined for the treated (right) and untreated (left) eyes for each treatment group.

Compared to Fc control treatment, treatment with the exemplary variant ICOSL IgV-Fc starting on day 8 demonstrated significant suppression of inflammation by daily unmasked clinical score on days 10, 11, 12 ($p<0.05$), and 13 ($p<0.01$) **(FIGS. 29A 29E).** Treatment with variant ICOSL IgV-Fc on day 8/10 delayed onset of disease, as seen by a difference in clinical score on all days except the final point (day 14), which was 4 days after the last dose. Additionally, clinical inflammation scored by masked OCT was significantly lower in variant ICOSL IgV-Fc treated eyes compared to Fc control eyes on both days 11 ($p<0.05$) and 14 ($p<0.01$) **(FIGS. 30A-30C).** Histologic score of inflammation in variant ICOSL IgV-Fc treated eyes was also significantly lower than Fc control treated eyes ($p<0.01$) **(FIG. 31).**

Treatment with variant ICOSL IgV-Fc starting on day 10 prevented progression of uveitis score and decreased final inflammation scores when compared to PBS and control treatment **(FIGS. 29A-31).** Unmasked clinical score decreased in the ICOSL IgV-Fc day 10/12 treated eyes the day after intravitreal injection.

A systemic effect in the fellow, untreated eye (left eye) was also observed in variant ICOSL IgV-Fc day 8/10 and day 10/12 treated animals. A similar systemic effect was also observed in the fellow, untreated eyes of steroid treated animals **(FIGS. 29A-29B, 30A, and 31)** . Treatment with variant ICOSL IgV-Fc on day 8/10 was more effective than Fc control in suppressing EAU.

Steroid treated animals showed a mean weight loss of 12.7% on day 14 **(FIG. 32).** Treatment with variant ICOSL IgV-Fc or control (vehicle, Fc control), did not result in weight loss (3% and 2% mean weight gain, respectively).

Local treatment with the dual CD28/ICOS antagonist variant ICOSL IgV-Fc suppressed EAU in Lewis rats.

Furthermore, intravitreal injection of variant ICOSL IgV-Fc was well-tolerated without the weight loss noted with steroid treatment. These data support variant ICOSL IgV-Fc as a therapeutic candidate for the treatment of inflammatory eye disorders, such as uveitis.

## EXAMPLE 26

### Administration of Variant ICOSL-IgV Fc in Subjects with SARS-CoV-2 Infection (COVID-19)

Subjects aged 13 years or older with confirmed SAR-CoV-2 infection (COVID-19) and severe pneumonia, acute respiratory distress syndrome (ARDS), sepsis, or septic shock attributed to COVID-19 infection are administered one or more intravenous doses of the variant ICOSL vIgD-Fc fusion protein, N52H/N57Y/Q100R as set forth in SEQ ID NO: 36, fused to an inert Fc (e.g. containing mutations L234A, L235E and G237A in a human IgG1 Fc, e.g. set forth in SEQ ID NO:42). Subjects are administered an intravenous infusion(s) of up to 10 mg/kg per dose, and observed for up to 56 days after the last dose.

Clinical outcomes and assessments are recorded, including but not limited to: **(1)** status based on the seven-category ordinal scale (7 - Death; 6 - Hospitalized, requiring ECMO, invasive mechanical ventilation, or both; 5 - Hospitalized, requiring nasal high-flow $O_2$ therapy, noninvasive mechanical ventilation, or both; 4 - Hospitalized, requiring supplemental oxygen; 3 - Hospitalized, not requiring supplemental oxygen; 2 - Not hospitalized, but unable to resume normal activities; 1 - Not hospitalized, with resumption of normal activities); **(2)** respiratory status (level of oxygen supplementation (L/min); PaO2 (partial pressure of oxygen) / FiO2 (fraction of inspired oxygen) ratio; SaO2 (peripheral oxygen saturation); radiographic abnormalities); **(3)** overall status (assessed based on SOFA (sepsis-related organ failure) score, NEWS2 (national early warning score 2), use of vasoactive agents, and/or organ-specific laboratory abnormalities); **(4)** laboratory values potentially associated with disease severity, including, e.g., serum ferritin, procalcitonin, D-dimer, C-reactive protein (CRP), total leukocyte count, differential lymphocytic count and neutrophil to lymphocyte ratio; and **(5)** serious adverse events related to treatment with the exemplary variant ICOSL IgV-Fc fusion protein. Blood samples to assess pharmacokinetics (PK) and pharmacodynamics (PD) are collected at intervals when practical. PD may include, but is not limited to, circulating cytokines and/or levels of antibodies against SARS-CoV-2.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

**SEQUENCE TABLE**

| SEQ ID NO | SEQUENCE | Description |
|---|---|---|
| 1 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQNLTV GSQTGNDIGE RDKITENPVS TGEKNAAT | ICOSLG(B7RP1, CD275, ICOSL, B7-H2) ECD |
| 2 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV E | ICOSL WT IgV |
| 3 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SV | IgV domain |
| 4 | MRLGSPGLLF LLFSSLRADT QEKEVRAMVG SDVELSCACP EGSRFDLNDV YVYWQTSESK TVVTYHIPQN SSLENVDSRY RNRALMSPAG MLRGDFSLRL FNVTPQDEQK FHCLVLSQSL GFQEVLSVEV TLHVAANFSV PVVSAPHSPS QDELTFTCTS INGYPRPNVY WINKTDNSLL DQALQNDTVF LNMRGLYDVV SVLRIARTPS VNIGCCIENV LLQQNLTVGS QTGNDIGERD KITENPVSTG EKNAATWSIL AVLCLLVVVA VAIGWVCRDR CLQHSYAGAW AVSPETELTG HV | ICOSLG(B7RP1, CD275, ICOSL, B7-H2) |

(continued)

| SEQ ID NO | SEQUENCE | Description |
|---|---|---|
| 5 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQNL | ICOSL WT ECD Truncation 2 |
| 6 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQNLTV GSQ | ICOSL WT ECD Truncation 3 |
| 7 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQN | ICOSL WT ECD Truncation 4 |
| 8 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQ | ICOSL WT ECD Truncation 5 |

(continued)

| SEQ ID NO | SEQUENCE | Description |
|---|---|---|
| 9 | DTQEKEVRAM VGSDVELSCA CPEGSRFDLN DVYVYWQTSE<br>SKTVVTYHIP QNSSLENVDS<br>RYRNRALMSP AGMLRGDFSL RLFNVTPQDE QKFHCLVLSQ<br>SLGFQEVLSV EVTLHVAANF<br>SVPVVSAPHS PSQDELTFTC TSINGYPRPN VYWINKTDNS<br>LLDQALQNDT VFLNMRGLYD<br>VVSVLRIART PSVNIGCCIE NVLL | ICOSL WT ECD Truncation 6 |
| 10 | DTQEKEVRAM VGSDVELSCA CPEGSRFDLN DVYVYWQTSE<br>SKTVVTYHIP QNSSLENVDS<br>RYRNRALMSP AGMLRGDFSL RLFNVTPQDE QKFHCLVLSQ<br>SLGFQEVLSV EVTLHVAANF<br>SVPVVSAPHS PSQDELTFTC TSINGYPRPN VYWINKTDNS<br>LLDQALQNDT VFLNMRGLYD<br>VVSVLRIART PSVNIGCCIE N | ICOSL WT ECD Truncation 7 |
| 11 | DTQEKEVRAM VGSDVELSCA CPEGSRFDLN DVYVYWQTSE<br>SKTVVTYHIP QNSSLENVDS<br>RYRNRALMSP AGMLRGDFSL RLFNVTPQDE QKFHCLVLSQ<br>SLGFQEVLSV EVTLHVAANF<br>SVPVVSAPHS PSQDELTFTC TSINGYPRPN VYWINKTDNS<br>LLDQALQNDT VFLNMRGLYD<br>VVSVLRIART PSVNIGCCIE NVLLQQNLT | ICOSL WT ECD Truncation 8 |
| 12 | DTQEKEVRAM VGSDVELSCA CPEGSRFDLN DVYVYWQTSE<br>SKTVVTYHIP QNSSLENVDS<br>RYRNRALMSP AGMLRGDFSL RLFNVTPQDE QKFHCLVLSQ<br>SLGFQEVLSV EVTLHVAANF<br>SVPVVSAPHS PSQDELTFTC TSINGYPRPN VYWINKTDNS<br>LLDQALQNDT VFLNMRGLYD<br>VVSVLRIART PSVNIGCCIE NVLLQQAL | ICOSL N207A ECD Truncation 2 |

(continued)

| SEQ ID NO | SEQUENCE | Description |
|---|---|---|
| 13 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQGL | ICOSL N207G ECD Truncation 2 |
| 14 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQNG | ICOSL L208G ECD Truncation 2 |
| 15 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQALTV GSQ | ICOSL N207A ECD Truncation 3 |
| 16 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQGLTV GSQ | ICOSL N207G ECD Truncation 3 |
| 17 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS | ICOSL L208G ECD Truncation 3 |

(continued)

| SEQ ID NO | SEQUENCE | Description |
|---|---|---|
| | LLDQALQNDT VFLNMRGLYD<br>VVSVLRIART PSVNIGCCIE NVLLQQNGTV GSQ | |
| 18 | DTQEKEVRAM VGSDVELSCA CPEGSRFDLN DVYVYWQTSE<br>SKTVVTYHIP QNSSLENVDS<br>RYRNRALMSP AGMLRGDFSL RLFNVTPQDE QKFHCLVLSQ<br>SLGFQEVLSV EVTLHVAANF<br>SVPVVSAPHS PSQDELTFTC TSINGYPRPN VYWINKTDNS<br>LLDQALQNDT VFLNMRGLYD<br>VVSVLRIART PSVNIGCCIE NVLLQQA | ICOSL N207A ECD Truncation 4 |
| 19 | DTQEKEVRAM VGSDVELSCA CPEGSRFDLN DVYVYWQTSE<br>SKTVVTYHIP QNSSLENVDS<br>RYRNRALMSP AGMLRGDFSL RLFNVTPQDE QKFHCLVLSQ<br>SLGFQEVLSV EVTLHVAANF<br>SVPVVSAPHS PSQDELTFTC TSINGYPRPN VYWINKTDNS<br>LLDQALQNDT VFLNMRGLYD<br>VVSVLRIART PSVNIGCCIE NVLLQQG | ICOSL N207G ECD Truncation 4 |
| 20 | DTQEKEVRAM VGSDVELSCA CPEGSRFDLN DVYVYWQTSE<br>SKTVVTYHIP QNSSLENVDS<br>RYRNRALMSP AGMLRGDFSL RLFNVTPQDE QKFHCLVLSQ<br>SLGFQEVLSV EVTLHVAANF<br>SVPVVSAPHS PSQDELTFTC TSINGYPRPN VYWINKTDNS<br>LLDQALQNDT VFLNMRGLYD<br>VVSVLRIART PSVNIGCCIE NVLLQQALT | ICOSL N207A ECD Truncation 8 |
| 21 | DTQEKEVRAM VGSDVELSCA CPEGSRFDLN DVYVYWQTSE<br>SKTVVTYHIP QNSSLENVDS<br>RYRNRALMSP AGMLRGDFSL RLFNVTPQDE QKFHCLVLSQ<br>SLGFQEVLSV EVTLHVAANF<br>SVPVVSAPHS PSQDELTFTC TSINGYPRPN VYWINKTDNS<br>LLDQALQNDT VFLNMRGLYD<br>VVSVLRIART PSVNIGCCIE NVLLQQGLT | ICOSL N207G ECD Truncation 8 |

(continued)

| SEQ ID NO | SEQUENCE | Description |
|---|---|---|
| 22 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQNGT | ICOSL L208G ECD Truncation 8 |
| 23 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQAG | ICOSL N207A/L208G ECD Truncation 2 |
| 24 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQGG | ICOSL N207G/L208G ECD Truncation 2 |
| 25 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQAGTV GSQ | ICOSL N207A/L208G ECD Truncation 3 |
| 26 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ | ICOSL N207G/L208G ECD Truncation 3 |

(continued)

| SEQ ID NO | SEQUENCE | Description |
|---|---|---|
| | SLGFQEVLSV EVTLHVAANF<br>SVPVVSAPHS PSQDELTFTC TSINGYPRPN VYWINKTDNS<br>LLDQALQNDT VFLNMRGLYD<br>VVSVLRIART PSVNIGCCIE NVLLQQGGTV GSQ | |
| 27 | DTQEKEVRAM VGSDVELSCA CPEGSRFDLN DVYVYWQTSE<br>SKTVVTYHIP QNSSLENVDS<br>RYRNRALMSP AGMLRGDFSL RLFNVTPQDE QKFHCLVLSQ<br>SLGFQEVLSV EVTLHVAANF<br>SVPVVSAPHS PSQDELTFTC TSINGYPRPN VYWINKTDNS<br>LLDQALQNDT VFLNMRGLYD<br>VVSVLRIART PSVNIGCCIE NVLLQQAGT | ICOSL N207A/L208G ECD Truncation 8 |
| 28 | DTQEKEVRAM VGSDVELSCA CPEGSRFDLN DVYVYWQTSE<br>SKTVVTYHIP QNSSLENVDS<br>RYRNRALMSP AGMLRGDFSL RLFNVTPQDE QKFHCLVLSQ<br>SLGFQEVLSV EVTLHVAANF<br>SVPVVSAPHS PSQDELTFTC TSINGYPRPN VYWINKTDNS<br>LLDQALQNDT VFLNMRGLYD<br>VVSVLRIART PSVNIGCCIE NVLLQQGGT | ICOSL N207G/L208G ECD Truncation 8 |
| 29 | DTQEKEVRAM VGSDVELSCA CPEGSRFDLN DVYVYWQTSE<br>SKTVVTYHIP QNSSLENVDS<br>RYRNRALMSP AGMLRGDFSL RLFNVTPQDE QKFHCLVLSQ<br>SLGFQEVLSV EVTLHVAANF<br>SVPVVSAPHS PSQDELTFTC TSINGYPRPN VYWINKTDNS<br>LLDQALQNDT VFLNMRGLYD<br>VVSVLRIART PSVNIGCCIE NVLLQQALTV GSQTGNDIGE<br>RDKITENPVS TGEKNAAT | ICOSL N207A Full ECD |

(continued)

| SEQ ID NO | SEQUENCE | Description |
|---|---|---|
| 30 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQGLTV GSQTGNDIGE RDKITENPVS TGEKNAAT | ICOSL N207G Full ECD |
| 31 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQNGTV GSQTGNDIGE RDKITENPVS TGEKNAAT | ICOSL L208G Full ECD |
| 32 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQAGTV GSQTGNDIGE RDKITENPVS TGEKNAAT | ICOSL N207A/L208G Full ECD |
| 33 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQGGTV GSQTGNDIGE RDKITENPVS TGEKNAAT | ICOSL N207G/L208G Full ECD |

(continued)

| SEQ ID NO | SEQUENCE | Description |
|---|---|---|
| 34 | DTQEKEVRAM VGSDVELSCA CPEGSRFDLN DVYVYWQTSE SKTVVTYHIP QHSSLEYVDS RYRNRALMSP AGMLRGDFSL RLFNVTPQDE QKFHCLVLSR SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQNLTV GSQTGNDIGE RDKITENPVS TGEKNAAT | ICOSL v39 ECD |
| 35 | DTQEKEVRAM VGSDVELSCA CPEGSRFDLN DVYVYWQTSE SKTVVTYHIP QHSSLEYVDS RYRNRALMSP AGMLRGDFSL RLFNVTPQDE QKFHCLVLSR SLGFQEVLSV E | ICOSL v39 IgV |
| 36 | DTQEKEVRAM VGSDVELSCA CPEGSRFDLN DVYVYWQTSE SKTVVTYHIP QHSSLEYVDS RYRNRALMSP AGMLRGDFSL RLFNVTPQDE QKFHCLVLSR SLGFQEVLSV EVTLHVAANF SV | ICOSL v39 IgV |
| 37 | EPKSCDKTHT CPPCPAPELL GGPSVFLFPP KPKDTLMISR TPEVTCVVVD VSHEDPEVKF NWYVDGVEVH NAKTKPREEQ YNSTYRVVSV LTVLHQDWLN GKEYKCKVSN KALPAPIEKT ISKAKGQPRE PQVYTLPPSR EEMTKNQVSL TCLVKGFYPS DIAVEWESNG QPENNYKTTP PVLDSDGSFF LYSKLTVDKS RWQQGNVFSC SVMHEALHNH YTQKSLSLSP GK | IgG1 Fc |
| 38 | EPKSSDKTHT CPPCPAPELL GGPSVFLFPP KPKDTLMISR TPEVTCVVVD VSHEDPEVKF NWYVDGVEVH NAKTKPREEQ YNSTYRVVSV LTVLHQDWLN GKEYKCKVSN KALPAPIEKT ISKAKGQPRE PQVYTLPPSR DELTKNQVSL TCLVKGFYPS DIAVEWESNG QPENNYKTTP PVLDSDGSFF LYSKLTVDKS RWQQGNVFSC SVMHEALHNH YTQKSLSLSP G | Fc with C220S/E356D/M358L/K44 7del |

(continued)

| SEQ ID NO | SEQUENCE | Description |
|---|---|---|
| 39 | EPKSSDKTHT CPPCPAPEAE GAPSVFLFPP KPKDTLMISR TPEVTCVVVD VSHEDPEVKF NWYVDGVEVH NAKTKPREEQ YNSTYRVVSV LTVLHQDWLN GKEYKCKVSN KALPAPIEKT ISKAKGQPRE PQVYTLPPSR EEMTKNQVSL TCLVKGFYPS DIAVEWESNG QPENNYKTTP PVLDSDGSFF LYSKLTVDKS RWQQGNVFSC SVMHEALHNH YTQKSLSLSP G | Fc with C220S/L234A/L235E/G23 7A/K447del |
| 40 | EPKSSDKTHT CPPCPAPEAE GAPSVFLFPP KPKDTLMISR TPEVTCVVVD VSHEDPEVKF NWYVDGVEVH NAKTKPREEQ YNSTYRVVSV LTVLHQDWLN GKEYKCKVSN KALPAPIEKT ISKAKGQPRE PQVYTLPPSR EEMTKNQVSL TCLVKGFYPS DIAVEWESNG QPENNYKTTP PVLDSDGSFF LYSKLTVDKS RWQQGNVFSC SVMHEALHNH YTQKSLSLSP GK | Fc with C220S/L234A/L235E/G23 7A |
| 41 | EPKSSDKTHT CPPCPAPEAE GAPSVFLFPP KPKDTLMISR TPEVTCVVVD VSHEDPEVKF NWYVDGVEVH NAKTKPREEQ YNSTYRVVSV LTVLHQDWLN GKEYKCKVSN KALPAPIEKT ISKAKGQPRE PQVYTLPPSR DELTKNQVSL TCLVKGFYPS DIAVEWESNG QPENNYKTTP PVLDSDGSFF LYSKLTVDKS RWQQGNVFSC SVMHEALHNH YTQKSLSLSP GK | Fc region |
| 42 | EPKSSDKTHT CPPCPAPEAE GAPSVFLFPP KPKDTLMISR TPEVTCVVVD VSHEDPEVKF NWYVDGVEVH NAKTKPREEQ YNSTYRVVSV LTVLHQDWLN GKEYKCKVSN KALPAPIEKT ISKAKGQPRE PQVYTLPPSR DELTKNQVSL TCLVKGFYPS DIAVEWESNG QPENNYKTTP PVLDSDGSFF LYSKLTVDKS RWQQGNVFSC SVMHEALHNH YTQKSLSLSP G | Fc region |

(continued)

| SEQ ID NO | SEQUENCE | Description |
|---|---|---|
| 43 | EPKSSDKTHT CPPCPAPELL GGPSVFLFPP KPKDTLMISR<br>TPEVTCVVVD VSHEDPEVKF<br>NWYVDGVEVH NAKTKPCEEQ YGSTYRCVSV LTVLHQDWLN<br>GKEYKCKVSN KALPAPIEKT<br>ISKAKGQPRE PQVYTLPPSR EEMTKNQVSL TCLVKGFYPS<br>DIAVEWESNG QPENNYKTTP<br>PVLDSDGSFF LYSKLTVDKS RWQQGNVFSC SVMHEALHNH<br>YTQKSLSLSP GK | Fc (C5S (C220S), R77C, (R292C), N82G (N297G), V87C (V302C)) |
| 44 | EPKSSDKTHT CPPCPAPPVA GPSVFLFPPK PKDTLMISRT<br>PEVTCVVVDV KHEDPEVKFN<br>WYVDGVEVHN AKTKPREEQY NSTYRVVSVL TVLHQDWLNG<br>KEYKCKVSNK ALPAPIEKTI<br>SKAKGQPREP QVYTLPPSRE EMTKNQVSLT CLVKGFYPSD<br>IAVEWESNGQ PENNYKTTPP<br>VLDSDGSFFL YSKLTVDKSR WQQGNVFSCS VMHEALHNHY<br>TQKSLSLSPG K | Fc with C220S/E233P/L234V/L235 A/G236del/S267K |
| 45 | VIHVTKEVKE VATLSCGHNV SVEELAQTRI YWQKEKKMVL<br>TMMSGDMNIW PEYKNRTIFD<br>ITNNLSIVIQ ALRPSDEGTY ECVVLKYEKD GFKREHLAEV<br>TLSVKADFPT PSISDFEIPT<br>SNIRRIICSA SGGFPEPHLS WLENGEELNA INTTVSQDPE<br>TELYAVSSKL DFNMTTNHSF<br>MCLIKYGHLR VNQTFNWNTT KQEHFPDN | CD80 v2 ECD |
| 46 | EPKSSDKTHT CPPCPAPELL GGPSVFLFPP KPKDTLMISR<br>TPEVTCVVVD VSHEDPEVKF<br>NWYVDGVEVH NAKTKPCEEQ YGSTYRCVSV LTVLHQDWLN<br>GKEYKCKVSN KALPAPIEKT<br>ISKAKGQPRE PQVYTLPPSR EEMTKNQVSL TCLVKGFYPS<br>DIAVEWESNG QPENNYKTTP<br>PVLDSDGSFF LYSKLTVDKS RWQQGNVFSC SVMHEALHNH<br>YTQKSLSLSP G | Fc (C5S (C220S), R77C, (R292C), N82G (N297G), V87C (V302C), L232del (K447del)) |

(continued)

| SEQ ID NO | SEQUENCE | Description |
|---|---|---|
| 47 | EPKSSDKTHT CPPCPAPPVA GPSVFLFPPK PKDTLMISRT PEVTCVVVDV KHEDPEVKFN WYVDGVEVHN AKTKPREEQY NSTYRVVSVL TVLHQDWLNG KEYKCKVSNK ALPAPIEKTI SKAKGQPREP QVYTLPPSRE EMTKNQVSLT CLVKGFYPSD IAVEWESNGQ PENNYKTTPP VLDSDGSFFL YSKLTVDKSR WQQGNVFSCS VMHEALHNHY TQKSLSLSPG | Fc with C220S/E233P/L234V/L235 A/G236del/S267K/K447del |
| 48 | EPKSSDKTHT CPPCPAPELL GGPSVFLFPP KPKDTLMISR TPEVTCVVVD VSHEDPEVKF NWYVDGVEVH NAKTKPREEQ YNSTYRVVSV LTVLHQDWLN GKEYKCKVSN KALPAPIEKT ISKAKGQPRE PQVYTLPPSR EEMTKNQVSL TCLVKGFYPS DIAVEWESNG QPENNYKTTP PVLDSDGSFF LYSKLTVDKS RWQQGNVFSC SVMHEALHNH YTQKSLSLSP GK | IgG1 Fc C220S |
| 49 | TKVDKTVERK CCVECPPCPA PPVAGPSVFL FPPKPKDTLM ISRTPEVTCV VVDVSHEDPE VQFNWYVDGV EVHNAKTKPR EEQFNSTFRV VSVLTVVHQD WLNGKEYKCK VSNKGLPAPI EKTISKTKGQ PREPQVYTLP PSREEMTKNQ VSLTCLVKGF YPSDIAVEWE SNGQPENNYK TTPPMLDSDG SFFLYSKLTV DKSRWQQGNV FSCSVMHEAL HNHYTQKSLS LSPGK | IgG2 Fc |
| 50 | ESKYGPPCPS CPAPEFLGGP SVFLFPPKPK DTLMISRTPE VTCVVVDVSQ EDPEVQFNWY VDGVEVHNAK TKPREEQFNS TYRVVSVLTV LHQDWLNGKE YKCKVSNKGL PSSIEKTISK AKGQPREPQV YTLPPSQEEM TKNQVSLTCL VKGFYPSDIA VEWESNGQPE NNYKTTPPVL DSDGSFFLYS RLTVDKSRWQ EGNVFSCSVM HEALHNHYTQ KSLSLSLGK | IgG4 Fc |

(continued)

| SEQ ID NO | SEQUENCE | Description |
|---|---|---|
| 51 | ESKYGPPCPP CPAPEFLGGP SVFLFPPKPK DTLMISRTPE VTCVVVDVSQ EDPEVQFNWY VDGVEVHNAK TKPREEQFNS TYRVVSVLTV LHQDWLNGKE YKCKVSNKGL PSSIEKTISK AKGQPREPQV YTLPPSQEEM TKNQVSLTCL VKGFYPSDIA VEWESNGQPE NNYKTTPPVL DSDGSFFLYS RLTVDKSRWQ EGNVFSCSVM HEALHNHYTQ KSLSLSLGK | IgG4 Fc S228P |
| 52 | GGGGS | 4GS Linker |
| 53 | GGGGSGGGGS | IgV-Fc linker 1 |
| 54 | GGGGSGGGGS GGGGS | IgV-IgV linker |
| 55 | EAAAK | EAAAK Linker |
| 56 | EAAAKEAAAK EAAAK | 3x EAAAK Linker |
| 57 | EAAAKEAAAK EAAAKEAAAK EAAAK | 5x EAAAK Linker |
| 58 | GSGGGGS | Linker |
| 59 | GGGGSGGGGS AAA | IgV-Fc linker 2 |
| 60 | DTQEKEVRAM VGSDVELSCA CPEGSRFDLN DVYVYWQTSE SKTVVTYHIP QHSSLEYVDS RYRNRALMSP AGMLRGDFSL RLFNVTPQDE QKFHCLVLSR SLGFQEVLSV EVTLHVAANF SVGGGGSGGG GSEPKSSDKT HTCPPCPAPE AEGAPSVFLF PPKPKDTLMI SRTPEVTCVV VDVSHEDPEV KFNWYVDGVE VHNAKTKPRE EQYNSTYRVV SVLTVLHQDW LNGKEYKCKV SNKALPAPIE KTISKAKGQP REPQVYTLPP SRDELTKNQV SLTCLVKGFY PSDIAVEWES NGQPENNYKT TPPVLDSDGS FFLYSKLTVD KSRWQQGNVF SCSVMHEALH NHYTQKSLSL SPG | Variant ICOSL IgV-Fc |

(continued)

| SEQ ID NO | SEQUENCE | Description |
|---|---|---|
| 61 | DTQEKEVRAM VGSDVELSCA CPEGSRFDLN DVYVYWQTSE SKTVVTYHIP QHSSLENVDS RYRNRALMSP AGMLRGDFSL RLFNVTPQDE QKFHCLVLSQ SLGFQEVLSV EVTLHVAANF SVPVVSAPHS PSQDELTFTC TSTNGYPRPN VYWINKTDNS LLDQALQNDT VFLNMRGLYD VVSVLRIART PSVNIGCCIE NVLLQQNLTV GSQTGNDIGE RDKITENPVS TGEKNAAT | ICOSL v27 ECD |
| 62 | DTQEKEVRAM VGSDVELSCA CPEGSRFDLN DVYVYWQTSE SKTVVTYHIP QHSSLEYVDS RYRNRALMSP AGMLRGDFSL RLFNVTPQDE QKFHCLVLSP SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQNLTV GSQTGNDIGE RDKITENPVS TGEKNAAT | ICOSL v5 ECD |
| 63 | DTQEKEVRAM VGSDVELSCA CPEGSRFDLN DVYVYWQTSE SKTVVTYHIP QHSSLEYVDS RYRNRALMSP AGMLRGDFSL RLFNVTPQDE QKFHCLVLSR SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCRIE NVLLQQNLTV GSQTGNDIGE RDKITENPVS TGEKNAAT | ICOSL v82 ECD |
| 64 | DTQEKEVRAM VGSDVELSCA CPEGSRFDLN DVYVYWQTSE SKTVVTYHIP QHSSLEYVDS RYRNRALMSP AGMLRGDFSL RLFNVTPQDE QKFHCLVLSR SLGFQEVLSV EVTLHVAANF SVPVVSAPHS PSQDELTFTC TSINGYPRPN VYWINKTDNS LLDQALQNDT VSLNMRGLYD VVSVLRIART PSVNIGCCIE NVLLQQNLTV GSQTGNDIGE RDKITENPVS TGEKNAAT | ICOSL v47 ECD |

(continued)

| SEQ ID NO | SEQUENCE | Description |
|---|---|---|
| 65 | DTQEKEVRAM VGSDVELSCA CPEGSRFDLN DVYVYWQTSE SKTVVTYHIP QHSSLEYVDS RYRNRALMSP AGMLRGDFSL RLFNVTPQDE QKFHCLVLSP SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQNLTV GSQTGNDIGE RDKITENPVS TGEKNAAT | ICOSL v44 ECD |
| 66 | DTQEKEVRAM VGSDVELSCA CPEGSRFDLN DVYVYWQTSE SKTVVTYHIP QHSSLENVDS RYRNRALMSP AGMLRGDFSL RLFNVTPQDE QKFHCLVLSR SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQNLTV GSQTGNDIGE RDKITENPVS TGEKNAAT | ICOSL v41 ECD |
| 67 | DTQEKEVRAM VGSDVELSCA CPEGSRFDLN DVYVYWQTSE SKTVVTYHIP QHSSLEYVDS RYRNRALMSP AGMLRGDFSL RLFNVTPQDE QKFHCLVLSP SLGFQEVLSV EVTLHVAANF SV | ICOSL v44 IgV |
| 68 | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQNLTV GSQTGNDIGE RDKITENPVS TGEKNAATWS ILAVLCLLVV VAVAIGWVCR DRCLQHSYAG AWAVSPETEL TGHV | Mature ICOSLG(B7RP1, CD275, ICOSL, B7-H2) |

The present invention is further described by the following numbered embodiments:

1. A method of preventing or reducing acute graft versus host disease (aGVHD), the method comprising administering to the subject one or more doses of a variant ICOSL fusion protein in a treatment period, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO: 1, wherein each of the one or more doses of the variant ICOSL fusion protein is administered in an amount from at or about 0.1 mg/kg to at or about 20 mg/kg.

2. The method of embodiment 1, wherein the subject has previously received an allogeneic hematopoietic stem cell transplant (HSCT) and the aGVHD occurs in the subject after receiving the allogeneic HSCT.

3. The method of embodiment 1 or embodiment 2, wherein the subject has Grade II-IV aGVHD.

4. The method of any one of embodiments 1-3, wherein the aGVHD in the subject is resistant or refractory to treatment with an immunosuppressant.

5. The method of embodiment 4, wherein the immunosuppressant comprises a corticosteroid.

6. The method of embodiment 4 or embodiment 5, wherein the immunosuppressant comprises a cyclosporine.

7. A method of preventing or reducing inflammation secondary to a viral infection, the method comprising administering to the subject one or more doses of a variant ICOSL fusion protein in a treatment period, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1, wherein each dose of the one or more doses of the variant ICOSL fusion protein is administered in an amount from at or about 0.1 mg/kg to at or about 20 mg/kg.

8. The method of embodiment 7, wherein the virus is a coronavirus.

9. The method of embodiment 8, wherein the coronavirus is SARS-CoV-2 and the infection is COVID-19.

10. The method of any of embodiments 7-9, wherein the inflammation is associated with cytokine release syndrome (CRS).

11. The method of embodiment 10, wherein the CRS is a severe CRS or a grade 3 or higher CRS.

12. The method of any of embodiments 7-11, wherein at the time of or immediately prior to the administration the subject has severe pneumonia, acute respiratory distress syndrome (ARDS), sepsis or septic shock associated with or attributed to the viral infection.

13. A method of treating an autoimmune or inflammatory disease or condition in a subject, the method comprising administering to a subject having an autoimmune or inflammatory disease or condition one or more doses of a variant ICOSL fusion protein in a treatment period, said variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1, wherein each dose of the one or more doses of the variant ICOSL fusion protein is administered in an amount from at or about 0.001 mg/kg to at or about 20 mg/kg.

14. The method of any one of embodiments 1-13, wherein each dose of the one or more doses is administered in an amount from or from about 0.1 mg/kg to at or about 10 mg/kg.

15. The method of embodiment 14, wherein the autoimmune or inflammatory disease or condition is an acute condition.

16. The method of any one of embodiments 1-15, wherein only a single dose of the variant ICOSL fusion protein is administered to the subject.

17. The method of embodiment 14, wherein the autoimmune or inflammatory disease or condition is a chronic condition.

18. The method of embodiment 13, embodiment 14 or embodiment 17, wherein the inflammatory or autoimmune disease or condition is systemic lupus erythematosus (SLE).

19. The method of embodiment 13, embodiment 14 or embodiment 17, wherein the inflammatory or autoimmune disease or condition is Sjögren's Syndrome.

20. The method of embodiment 13, embodiment 14 or embodiment 17, wherein the inflammatory or autoimmune disease or condition is psoriatic arthritis.

21. The method of embodiment 13, embodiment 14 or embodiment 17, wherein the inflammatory or autoimmune disease or condition is rheumatoid arthritis.

22. The method of embodiment 13, embodiment 14 or embodiment 17, wherein the inflammatory or autoimmune disease or condition is Crohn's disease.

23. The method of embodiment 13, embodiment 14 or embodiment 17, wherein the inflammatory or autoimmune disease or condition is ulcerative colitis.

24. The method of any one of embodiments 1-15 and 17-23, wherein a multiple number of doses of the variant ICOSL fusion protein is administered to the subject.

25. The method of embodiment 24, wherein each of the multiple number of doses is administered no more than once weekly.

26. The method of embodiment 24 or embodiment 25, wherein each of the multiple number of doses is administered once a week (Q1W).

27. The method of embodiment 24 or embodiment 25, wherein each of the multiple number of doses is administered once every two weeks (Q2W).

28. The method of embodiment 24 or embodiment 25, wherein each of the multiple number of doses is administered once a month (Q4W).

29. The method of any one of embodiments 1-28, wherein each dose of the one or more doses is administered in an amount from at or about 0.3 mg/kg to at or about 10 mg/kg, at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 10 mg/kg, at or about 1 mg/kg to at or about 6 mg/kg, at or about 1 mg/kg to at or about 3 mg/kg, at or about 3 mg/kg to at or about 10mg/kg, at or about 3 mg/kg to at or about 6 mg/kg, or at or about 6 mg/kg to at or about 10 mg/kg.

30. The method of any one of embodiments 1-29, wherein each dose of the one or more doses is administered in an amount from at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 6 mg/kg, at or about 1 mg/kg to at or about 3 mg/kg, or at or about 3 mg/kg to at or about 6 mg/kg.

31. The method of any one of embodiments 1-30, wherein each dose of the one or more doses is administered in an amount of or of about 0.3 mg/kg.

32. The method of any one of embodiments 1-30, wherein each dose of the one or more doses is administered in an amount of or of about 1 mg/kg.

33. The method of any one of embodiments 1-30, wherein each dose of the one or more doses is administered in an amount of or of about 3 mg/kg.

34. The method of any one of embodiments 1-30, wherein each dose of the one or more doses is administered in an amount of or of about 6 mg/kg.

35. The method of any one of embodiments 1-30, wherein each dose of the one or more doses is administered in an amount of or of about 10 mg/kg.

36. The method of any one of embodiments 1-28, wherein each dose of the one or more doses is administered in an amount of or of about 15 mg/kg.

37. The method of any one of embodiments 1-28, wherein each dose of the one or more doses is administered in an amount of or of about 20 mg/kg.

38. The method of any one of embodiments 1-37, wherein the treatment period is repeated.

39. The method of any one of embodiments 1-38, wherein the administration is via subcutaneous administration.

40. The method of any one of embodiments 1-38, wherein the administration is via intravenous administration.

41. A method of treating an ocular autoimmune or inflammatory disease in a subject, the method comprising administering intravitreally a dose of a variant ICOSL fusion protein comprising a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO: 1.

42. The method of embodiment 41, wherein the ocular autoimmune or inflammatory disease is uveitis.

43. The method of embodiment 41 or embodiment 42, wherein the variant ICOSL fusion protein is administered at a dose of between about 0.01 mg to 10 mg, at or about 0.05 mg to 10 mg, at or about 0.1 mg to 10 mg, at or about 0.5 mg to 10 mg, at or about 1 mg to 10 mg, at or about 1.5 mg to 10 mg, at or about 2 mg to 10 mg, at or about 3 mg to 10 mg, at or about 4 mg to 10 mg, at or about 5 mg to 10 mg, at or about 6 mg to 10 mg, at or about 7 mg to 10 mg, at or about 8 mg to 10 mg, at or about 9 mg to 10 mg, inclusive.

44. The method of any of embodiments 41-43, wherein the variant ICOSL fusion protein is administered in a volume less than 0.2 mL, optionally less than 0.1 mL.

45. The method of any of embodiments 41-44, wherein the variant ICOSL fusion protein is administered in a volume of at or about 0.05 mL.

46. The method of any one of embodiments 1-45, wherein the ICOSL reference polypeptide comprises (i) the sequence of amino acids set forth in SEQ ID NO:32, (ii) a sequence of amino acids that has at least 95% sequence identity to SEQ ID NO:32; or (iii) a portion of (i) and/or (ii) comprising an IgV domain or an IgC domain or specific binding fragments thereof.

47. The method of any one of embodiments 1-46, wherein the variant ICOSL polypeptide comprises the IgV domain or a specific binding fragment thereof.

48. The method of any one of embodiments 1-47, wherein the IgV domain or specific binding fragment thereof is the only ICOSL portion of the variant ICOSL polypeptide or of the variant ICOSL fusion protein.

49. The method of any one of embodiments 1-48, wherein the ICOSL reference polypeptide comprises the sequence of amino acids set forth in SEQ ID NO:3

50. The method of any one of embodiments 1-48, wherein the ICOSL reference polypeptide consists of the sequence of amino acids set forth in SEQ ID NO:3

51. The method of any one of embodiments 1-50, wherein the variant ICOSL polypeptide has the sequence set forth in SEQ ID NO:36 or a sequence that exhibits at least at or about 90%, at least at or about 91%, at least at or about 92%, at least at or about 93%, at least at or about 94%, at least at or about 95%, at least at or about 96%, at least at or about 97%, at least at or about 98%, or at least at or about 99% sequence identity to the sequence set forth in SEQ ID NO:36 and comprises the one or more amino acid substitutions selected from N52H, N57Y and Q100R.

52. The method of any one of embodiments 1-51, wherein the variant ICOSL polypeptide has the sequence set forth in SEQ ID NO:36.

53. The method of any one of embodiments 1-52, wherein the multimerization domain is or comprises an Fc region of an immunoglobulin.

54. The method of embodiment 53, wherein the Fc region is a variant Fc region that exhibits reduced effector function compared to an Fc of a wildtype human immunoglobulin.

55. The method of embodiment 53 or embodiment 54, wherein the Fc region is a variant IgG1 Fc region comprising one or more amino acid substitutions compared to a wildtype human IgG1.

56. The method of embodiment 54 or embodiment 55, wherein the variant Fc region comprises one or more amino acid substitutions selected from N297G, E233P/L234V/L235A/G236del/S267K or L234A/L235E/G237A, wherein the residue is numbered according to the EU index of Kabat.

57. The method of any of embodiments 54-56, wherein the variant Fc region further comprises the amino acid substitution C220S, wherein the residues are numbered according to the EU index of Kabat.

58. The method of any of embodiments 53-57, wherein the Fc region comprises K447del, wherein the residue is numbered according to the EU index of Kabat.

59. A pharmaceutical composition comprising a variant ICOSL fusion protein for use in a method of preventing or reducing acute graft versus host disease (aGVHD) in a subject, wherein the variant ICOSL fusion protein is comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO: 1 .

60. Use of a variant ICOSL fusion protein in the formulation of a medicament for use in a method of preventing or reducing acute graft versus host disease (aGVHD), wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO: 1.

61. The pharmaceutical composition for use of embodiment 59 or the use of embodiment 60, wherein the method comprises the steps of administering one or more doses of the variant ICOSL fusion protein to a subject in a treatment period, wherein each of the one or more doses of the variant ICOSL fusion protein is administered in an amount from at or about 0.1 mg/kg to at or about 20 mg/kg.

62. The pharmaceutical composition for use of embodiment 59 or embodiment 61 or use of embodiment 60 or embodiment 61, wherein the aGvHD is Grade II-IV aGVHD.

63. The pharmaceutical composition for use of any one of embodiments 59, 61, and 62 or use of any one of embodiments 60, 61, and 62, wherein the aGVHD is resistant or refractory to treatment with an immunosuppressant.

64. The pharmaceutical composition for use of any one of embodiments 59 and 61-63, or use of any one of embodiments 60 and 61-63, wherein the immunosuppressant comprises a corticosteroid.

65. The pharmaceutical composition for use of embodiment 63 or embodiment 64 or use of embodiment 63 or embodiment 64, wherein the immunosuppressant comprises a cyclosporine.

66. A pharmaceutical composition comprising a variant ICOSL fusion protein for use in a method of preventing or reducing inflammation secondary to a viral infection in a subject, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO: 1.

67. Use of a variant ICOSL fusion protein in the formulation of a medicament for use in a method of preventing or reducing inflammation secondary to a viral infection, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1,.

68. The pharmaceutical composition for use of embodiment 66 or use of embodiment 67, wherein the method comprises the steps of administering one or more doses of the variant ICOSL fusion protein to a subject in a treatment period, wherein each of the one or more doses of the variant ICOSL fusion protein is administered in an amount from at or about 0.1 mg/kg to at or about 20 mg/kg.

69. The pharmaceutical composition for use of embodiment 66 or embodiment 68 or use of embodiment 67 or embodiment 68, wherein the virus is a coronavirus.

70. The pharmaceutical composition for use of embodiment 69 or use of embodiment 69, wherein the coronavirus is SARS-CoV-2 and the infection is COVID-19.

71. The pharmaceutical composition for use of any one of embodiments 66, 68, and 69 or use of any one of embodiments 67-69, wherein the inflammation is associated with cytokine release syndrome (CRS).

72. The pharmaceutical composition for use of embodiment 71 or use of embodiment 71, wherein the CRS is a severe CRS or a grade 3 or higher CRS.

73. The pharmaceutical composition for use of any one of embodiments 68, and 69-72 or use of any one of embodiments 68, and 69-72, wherein at the time of or immediately prior to the administration the subject has severe pneumonia, acute respiratory distress syndrome (ARDS), sepsis or septic shock associated with or attributed to the viral infection.

74. A pharmaceutical composition comprising a variant ICOSL fusion protein for use in treating an autoimmune or inflammatory disease or condition in a subject, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

75. Use of a variant ICOSL fusion protein in the formulation of a medicament for use in treating an autoimmune or inflammatory disease or condition, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

76. The pharmaceutical composition for use of embodiment 74 or use of embodiment 75, wherein the method comprises the steps of administering one or more doses of the variant ICOSL fusion protein to a subject in a treatment period, wherein each of the one or more doses of the variant ICOSL fusion protein is administered in an amount from at or about 0.001 mg/kg to at or about 20 mg/kg.

77. The pharmaceutical composition for use of any one of embodiments 61-65, 68-73, and 76 or use of any one of embodiments 61-65, 68-73, and 76, wherein each dose of the one or more doses is administered in an amount from or from about 0.1 mg/kg to at or about 10 mg/kg.

78. The pharmaceutical composition for use of embodiment 77 or the use of embodiment 77, wherein the autoimmune or inflammatory disease or condition is an acute condition.

79. The pharmaceutical composition for use of any one of embodiments 61-65, 68-73, and 76-78 or use of any one of embodiments 61-65, 68-73, and 76-78, wherein only a single dose of the variant ICOSL fusion protein is administered to the subject.

80. The pharmaceutical composition for use of embodiment 77 or use of embodiment 77, wherein the autoimmune or inflammatory disease or condition is a chronic disease or condition.

81. The pharmaceutical composition for use of any one of embodiments 74, 77, and 80 or use of any one of embodiments 75, 77 and 80, wherein the inflammatory or autoimmune disease or condition is systemic lupus erythematosus (SLE).

82. The pharmaceutical composition for use of any one of embodiments 74, 77, and 80 or use of any one of embodiments 75, 77 and 80, wherein the inflammatory or autoimmune disease or condition is Sjögren’s Syndrome.

83. The pharmaceutical composition for use of any one of embodiments 74, 77, and 80 or use of any one of embodiments 75, 77 and 80, wherein the inflammatory or autoimmune disease or condition is psoriatic arthritis.

84. The pharmaceutical composition for use of any one of embodiments 74, 77, and 80 or use of any one of embodiments 75, 77 and 80, wherein the inflammatory or autoimmune disease or condition is rheumatoid arthritis.

85. The pharmaceutical composition for use of any one of embodiments 74, 77, and 80 or use of any one of embodiments 75, 77 and 80, wherein the inflammatory or autoimmune disease or condition is Crohn’s disease.

86. The pharmaceutical composition for use of any one of embodiments 74, 77, and 80 or use of any one of embodiments 75, 77 and 80, wherein the inflammatory or autoimmune disease or condition is ulcerative colitis.

87. The pharmaceutical composition for use of any one of embodiments 61-65, 68-73, and 76-78 or use of any one of embodiments 61-65, 68-73, and 76-78, wherein a multiple number of doses of the variant ICOSL fusion protein is administered to the subject.

88. The pharmaceutical composition for use of embodiment 87 or the use of embodiment 87, wherein each of the multiple number of doses is administered no more than once weekly.

89. The pharmaceutical composition for use of embodiment 87 or embodiment 88 or the use of embodiment 87 or embodiment 88, wherein each of the multiple number of doses is administered once a week (Q1W).

90. The pharmaceutical composition for use of embodiment 87 or embodiment 88 or the use of embodiment 87 or embodiment 88, wherein each of the multiple number of doses is administered once every two weeks (Q2W).

91. The pharmaceutical composition for use of embodiment 87 or embodiment 88 or the use of embodiment 87 or embodiment 88, wherein each of the multiple number of doses is administered once a month (Q4W).

92. The pharmaceutical composition for use of any one of embodiments 61-65, 68-73, 76-78, and 87-91 or use of any one of embodiments 61-65, 68-73, 76-78, and 87-91, wherein each dose of the one or more doses is administered in an amount from at or about 0.3 mg/kg to at or about 10 mg/kg, at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 10 mg/kg, at or about 1 mg/kg to at or about 6 mg/kg, at or about 1 mg/kg to at or about 3 mg/kg, at or about 3 mg/kg to at or about 10mg/kg, at or about 3 mg/kg to at or about 6 mg/kg, or at or about 6 mg/kg to at or about 10 mg/kg.

93. The pharmaceutical composition for use of any one of embodiments 61-65, 68-73, 76-78, and 87-92 or use of any one of embodiments 61-65, 68-73, 76-78, and 87-92, wherein each dose of the one or more doses is administered in an amount from at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 6 mg/kg, at or about 1 mg/kg to at or about 3 mg/kg, or at or about 3 mg/kg to at or about 6 mg/kg.

94. The pharmaceutical composition for use of any one of embodiments 61-65, 68-73, 76-78, and 87-93 or use of any one of embodiments 61-65, 68-73, 76-78, and 87-93, wherein each dose of the one or more doses is administered in an amount of or of about 0.3 mg/kg.

95. The pharmaceutical composition for use of any one of embodiments 61-65, 68-73, 76-78, and 87-93 or use of any one of embodiments 61-65, 68-73, 76-78, and 87-93, wherein each dose of the one or more doses is administered in an amount of or of about 1 mg/kg.

96. The pharmaceutical composition for use of any one of embodiments 61-65, 68-73, 76-78, and 87-93 or use of any one of embodiments 61-65, 68-73, 76-78, and 87-93, wherein each dose of the one or more doses is administered in an amount of or of about 3 mg/kg.

97. The pharmaceutical composition for use of any one of 61-65, 68-73, 76-78, and 87-93 or use of any one of embodiments 61-65, 68-73, 76-78, and 87-93, wherein each dose of the one or more doses is administered in an amount of or of about 6 mg/kg.

98. The pharmaceutical composition for use of any one of embodiments 61-65, 68-73, 76-78, and 87-93 or use of any one of embodiments 61-65, 68-73, 76-78, and 87-93, wherein each dose of the one or more doses is administered in an amount of or of about 10 mg/kg.

99. The pharmaceutical composition for use of any one of embodiments 61-65, 68-73, 76-78, and 87-91 or use of any one of embodiments 61-65, 68-73, 76-78, and 87-91, wherein each dose of the one or more doses is administered in an amount of or of about 15 mg/kg.

100. The pharmaceutical composition for use of any one of embodiments 61-65, 68-73, 76-78, and 87-91 or use of any one of embodiments 61-65, 68-73, 76-78, and 87-91, wherein each dose of the one or more doses is administered in an amount of or of about 20 mg/kg.

101. The pharmaceutical composition for use of any one of embodiments 61-65, 68-73, 76-78, and 87-100 or use of any one of embodiments 61-65, 68-73, 76-78, and 87-100, wherein the treatment period is repeated.

102. The pharmaceutical composition for use of any one of embodiments 61-65, 68-73, 76-78, and 87-101 or use of any one of embodiments 61-65, 68-73, 76-78, and 87-101, wherein the variant ICOSL fusion protein is administered subcutaneously.

103. The pharmaceutical composition for use of any one of embodiments 61-65, 68-73, 76-78, and 87-101 or use of any one of embodiments 61-65, 68-73, 76-78, and 87-101, wherein the variant ICOSL fusion protein is administered intravenously.

104. A pharmaceutical composition comprising a variant ICOSL fusion protein for use in a method of treating an ocular autoimmune or inflammatory disease in a subject, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

105. Use of a variant ICOSL fusion protein in the formulation of a medicament for use in a method of treating an ocular autoimmune or inflammatory disease in a subject, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

106. The pharmaceutical composition for use of embodiment 104 or use of embodiment 105, wherein the ocular autoimmune or inflammatory disease is uveitis.

107. The pharmaceutical composition for use of embodiment 104 or embodiment 106 or use embodiment 105 or embodiment 106, wherein the method comprises steps of administering one or more doses of the variant ICOSL fusion protein to a subject, wherein each of the one or more doses of the variant ICOSL fusion protein is administered at a dose of between about 0.01 mg to 10 mg, at or about 0.05 mg to 10 mg, at or about 0.1 mg to 10 mg, at or about 0.5 mg to 10 mg, at or about 1 mg to 10 mg, at or about 1.5 mg to 10 mg, at or about 2 mg to 10 mg, at or about 3 mg to 10 mg, at or about 4 mg to 10 mg, at or about 5 mg to 10 mg, at or about 6 mg to 10 mg, at or about 7 mg to 10 mg, at or about 8 mg to 10 mg, at or about 9 mg to 10 mg, inclusive.

108. The pharmaceutical composition for use of embodiment 107 or use of embodiment 107, wherein the variant ICOSL fusion protein is administered in a volume less than 0.2 mL, optionally less than 0.1 mL.

109. The pharmaceutical composition for use of embodiment 107 or embodiment 108 or use of embodiment 107 or embodiment 108, wherein the variant ICOSL fusion protein is administered in a volume of at or about 0.05 mL.

110. The pharmaceutical composition for use of any one of embodiments 59, 61-65, 66, 68-73-, 74, 76-103, 104, and 106-109 or use of any one of embodiments 60, 61-65, 67, 68-73, 75, 76-103, 105, and 106 107-109, wherein the ICOSL reference polypeptide comprises (i) the sequence of amino acids set forth in SEQ ID NO:32, (ii) a sequence of amino acids that has at least 95% sequence identity to SEQ ID NO:32; or (iii) a portion of (i) and/or (ii) comprising an IgV domain or an IgC domain or specific binding fragments thereof.

111. The pharmaceutical composition for use of any one of embodiments 59, 61-65, 66, 68-73-, 74, 76-103, 104, and 106-110 or use of any one of embodiments 60, 61-65, 67, 68-73, 75, 76-103, and 105-110, wherein the variant ICOSL polypeptide comprises the IgV domain or a specific binding fragment thereof.

112. The pharmaceutical composition for use of any one of embodiments 59, 61-65, 66, 68-73-, 74, 76-103, 104, and 106-111 or use of any one of embodiments 60, 61-65, 67, 68-73, 75, 76-103, and 105-111, wherein the IgV domain or specific binding fragment thereof is the only ICOSL portion of the variant ICOSL polypeptide or of the variant ICOSL fusion protein.

113. The pharmaceutical composition for use of any one of embodiments 59, 61-65, 66, 68-73-, 74, 76-103, 104, and 106-112 or use of any one of embodiments 60, 61-65, 67, 68-73, 75, 76-103, and 105-112, wherein the ICOSL reference polypeptide comprises the sequence of amino acids set forth in SEQ ID NO:3

114. The pharmaceutical composition for use of any one of embodiments 59, 61-65, 66, 68-73-, 74, 76-103, 104, and 106-113 or use of any one of embodiments 60, 61-65, 67, 68-73, 75, 76-103, and 105-113, wherein the ICOSL reference polypeptide consists of the sequence of amino acids set forth in SEQ ID NO:3

115. The pharmaceutical composition for use of any one of embodiments 59, 61-65, 66, 68-73-, 74, 76-103, 104, and 106-114 or use of any one of embodiments 60, 61-65, 67, 68-73, 75, 76-103, and 105-114, wherein the variant ICOSL polypeptide has the sequence set forth in SEQ ID NO:36 or a sequence that exhibits at least at or about 90%, at least at or about 91%, at least at or about 92%, at least at or about 93%, at least at or about 94%, at least at or about 95%, at least at or about 96%, at least at or about 97%, at least at or about 98%, or at least at or about 99% sequence identity to the sequence set forth in SEQ ID NO:36 and comprises the one or more amino acid substitutions selected from N52H, N57Y and Q100R.

116. The pharmaceutical composition for use of any one of embodiments 59, 61-65, 66, 68-73-, 74, 76-103, 104, and 106-115 or use of any one of embodiments 60, 61-65, 67, 68-73, 75, 76-103, and 105-115, wherein the variant ICOSL polypeptide has the sequence set forth in SEQ ID NO:36.

117. The pharmaceutical composition for use of any one of embodiments 59, 61-65, 66, 68-73-, 74, 76-103, 104, and 106-116 or use of any one of embodiments 60, 61-65, 67, 68-73, 75, 76-103, and 105-116, wherein the multimerization domain is or comprises an Fc region of an immunoglobulin.

118. The pharmaceutical composition for use of embodiment 117 or use of embodiment 117, wherein the Fc region is a variant Fc region that exhibits reduced effector function compared to an Fc of a wildtype human immunoglobulin.

119. The pharmaceutical composition for use of embodiment 117 or embodiment 118 or use of embodiment 117 or embodiment 118, wherein the Fc region is a variant IgG1 Fc region comprising one or more amino acid substitutions compared to a wildtype human IgG1.

120. The pharmaceutical composition for use of embodiment 118 or embodiment 119 or use of embodiment 118 or embodiment 119, wherein the variant Fc region comprises one or more amino acid substitutions selected from N297G, E233P/L234V/L235A/G236del/S267K or L234A/L235E/G237A, wherein the residue is numbered according to the EU index of Kabat.

121. The pharmaceutical composition for use of any one of embodiments 118-120 or use of any one of embodiments 118-120, wherein the variant Fc region further comprises the amino acid substitution C220S, wherein the residues are numbered according to the EU index of Kabat.

122. The pharmaceutical composition for use of any one of embodiments 117-121 or use of any one of embodiments 117-121, wherein the Fc region comprises K447del, wherein the residue is numbered according to the EU index of Kabat.

## Claims

1. A pharmaceutical composition comprising a variant ICOSL fusion protein for use in a method of preventing or reducing acute graft versus host disease (aGVHD) in a subject, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO: 1.

2. The pharmaceutical composition for use of claim 1, wherein the aGVHD is Grade II-IV aGVHD, optionally wherein the aGVHD is resistant or refractory to treatment with an immunosuppressant, further optionally wherein the immunosuppressant comprises a corticosteroid or a cyclosporine.

3. A pharmaceutical composition comprising a variant ICOSL fusion protein for use in a method of preventing or reducing inflammation secondary to a viral infection in a subject, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO: 1.

4. The pharmaceutical composition for use of claim 3, wherein:

   a) the virus is a coronavirus, optionally wherein the coronavirus is SARS-CoV-2 and the infection is COVID-19; and/or
   b) the inflammation is associated with cytokine release syndrome (CRS), optionally wherein the CRS is a severe CRS or a grade 3 or higher CRS;and/orc) at the time of or immediately prior to the administration the subject has severe pneumonia, acute respiratory distress syndrome (ARDS), sepsis or septic shock associated with or attributed to the viral infection.

5. The pharmaceutical composition for use of claims 1 or 3, wherein the method comprises the steps of administering one or more doses of the variant ICOSL fusion protein to a subject in a treatment period, wherein each of the one or more doses of the variant ICOSL fusion protein is administered in an amount from at or about 0.001 mg/kg to at or about 20 mg/kg or from at or about 0.1 mg/kg to at or about 20 mg/kg, optionally wherein each dose of the one or more doses is administered in an amount from or from about 0.1 mg/kg to at or about 10 mg/kg.

6. The pharmaceutical composition for use of any one of claims 1-5, wherein only a single dose of the variant ICOSL fusion protein is administered to the subj ect.

7. The pharmaceutical composition for use of any one of claims 1-5,
wherein a multiple number of doses of the variant ICOSL fusion protein is administered to the subject, optionally wherein:

a) each of the multiple number of doses is administered no more than once weekly;
b) each of the multiple number of doses is administered once a week (Q1W);
c) each of the multiple number of doses is administered once every two weeks (Q2W); or
d) each of the multiple number of doses is administered once a month (Q4W).

**8.** The pharmaceutical composition for use of any one of claims 1-5 or 7, wherein each dose of the one or more doses is administered in an amount from at or about 0.3 mg/kg to at or about 10 mg/kg, at or about 0.3 mg/kg to at or about 6 mg/kg, at or about 0.3 mg/kg to at or about 3 mg/kg, at or about 0.3 mg/kg to at or about 1 mg/kg, at or about 1 mg/kg to at or about 10 mg/kg, at or about 1 mg/kg to at or about 6 mg/kg, at or about 1 mg/kg to at or about 3 mg/kg, at or about 3 mg/kg to at or about 10mg/kg, at or about 3 mg/kg to at or about 6 mg/kg, or at or about 6 mg/kg to at or about 10 mg/kg.

**9.** The pharmaceutical composition for use of any one of claims 1-5 or 7-8, wherein each dose of the one or more doses is administered:

a) in an amount of or of about 0.3 mg/kg;
b) in an amount of or of about 1 mg/kg;
c) in an amount of or of about 3 mg/kg;
d) in an amount of or of about 6 mg/kg;
e) in an amount of or of about 10 mg/kg;
f) in an amount of or of about 15 mg/kg; or
g) in an amount of or of about 20 mg/kg.

**10.** The pharmaceutical composition for use of any one of claims 1-9, wherein:

a) the treatment period is repeated; and/or
b) the variant ICOSL fusion protein is administered subcutaneously or wherein the variant ICOSL fusion protein is administered intravenously.

**11.** A pharmaceutical composition comprising a variant ICOSL fusion protein for use in a method of treating an ocular autoimmune or inflammatory disease in a subject, wherein the variant ICOSL fusion protein comprises a variant ICOSL polypeptide linked to a multimerization domain, wherein the variant ICOSL polypeptide is an ICOSL extracellular domain or a portion thereof comprising an IgV domain or a specific binding fragment thereof and comprises one or more amino acid substitutions in a reference ICOSL polypeptide corresponding to amino acid substitutions selected from N52H, N57Y and Q100R with reference to the sequence set forth in SEQ ID NO:1.

**12.** The pharmaceutical composition for use of claim 11, wherein the ocular autoimmune or inflammatory disease is uveitis.

**13.** The pharmaceutical composition for use of claim 11 or claim 12 wherein the method comprises steps of administering one or more doses of the variant ICOSL fusion protein to a subject, wherein each of the one or more doses of the variant ICOSL fusion protein is administered at a dose of between about 0.01 mg to 10 mg, at or about 0.05 mg to 10 mg, at or about 0.1 mg to 10 mg, at or about 0.5 mg to 10 mg, at or about 1 mg to 10 mg, at or about 1.5 mg to 10 mg, at or about 2 mg to 10 mg, at or about 3 mg to 10 mg, at or about 4 mg to 10 mg, at or about 5 mg to 10 mg, at or about 6 mg to 10 mg, at or about 7 mg to 10 mg, at or about 8 mg to 10 mg, at or about 9 mg to 10 mg, inclusive.

**14.** The pharmaceutical composition for use of claim 13, wherein the variant ICOSL fusion protein is administered in a volume less than 0.2 mL, optionally less than 0.1 mL, for example wherein the variant ICOSL fusion protein is administered in a volume of at or about 0.05 mL.

**15.** The pharmaceutical composition for use of any one of claims 1-14, wherein:

a) the ICOSL reference polypeptide comprises (i) the sequence of amino acids set forth in SEQ ID NO:32, (ii) a sequence of amino acids that has at least 95% sequence identity to SEQ ID NO:32; or (iii) a portion of (i) and/or (ii) comprising an IgV domain or an IgC domain or specific binding fragments thereof;
b) the variant ICOSL polypeptide comprises the IgV domain or a specific binding fragment thereof; and/or

c) the IgV domain or specific binding fragment thereof is the only ICOSL portion of the variant ICOSL polypeptide or of the variant ICOSL fusion protein.

**16.** The pharmaceutical composition for use of any one of claims 1-15, wherein:

a) the ICOSL reference polypeptide comprises or consists of the sequence of amino acids set forth in SEQ ID NO:3; and/or
b) the variant ICOSL polypeptide has the sequence set forth in SEQ ID NO:36 or a sequence that exhibits at least at or about 90%, at least at or about 91%, at least at or about 92%, at least at or about 93%, at least at or about 94%, at least at or about 95%, at least at or about 96%, at least at or about 97%, at least at or about 98%, or at least at or about 99% sequence identity to the sequence set forth in SEQ ID NO:36 and comprises the one or more amino acid substitutions selected from N52H, N57Y and Q100R.

**17.** The pharmaceutical composition for use of any one of claims 1-16, wherein the multimerization domain is or comprises an Fc region of an immunoglobulin, optionally wherein:

a)

i) the Fc region is a variant Fc region that exhibits reduced effector function compared to an Fc of a wildtype human immunoglobulin; and/or
ii) the Fc region is a variant IgG1 Fc region comprising one or more amino acid substitutions compared to a wildtype human IgG1; and optionally

b)

i) the variant Fc region comprises one or more amino acid substitutions selected from N297G, E233P/L234V/L235A/G236del/S267K or L234A/L235E/G237A, wherein the residue is numbered according to the EU index of Kabat; and/or
ii) the variant Fc region further comprises the amino acid substitution C220S, wherein the residues are numbered according to the EU index of Kabat; and/or

c) the Fc region comprises K447del, wherein the residue is numbered according to the EU index of Kabat.

FIG. 1A

**Soluble vIgD (e.g. vIgD-Fc)**

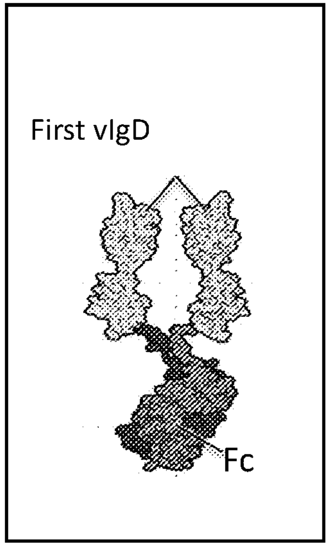

FIG. 1B

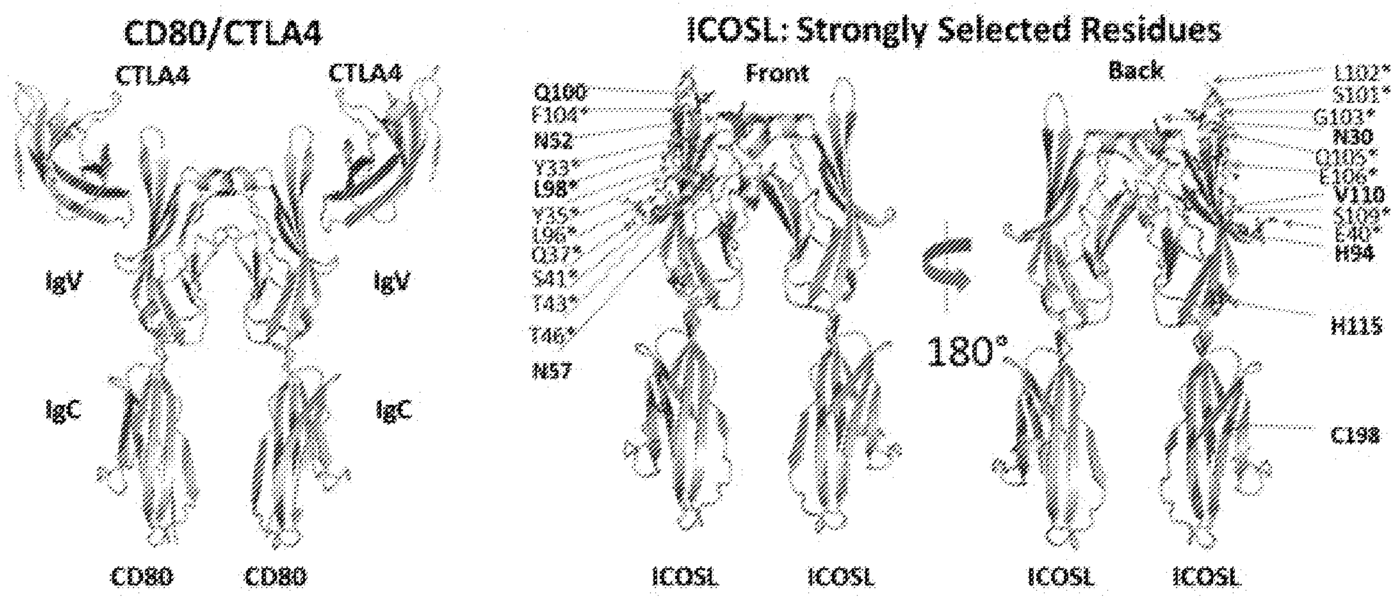
CD80/CTLA4
CTLA4
CTLA4
IgV
IgV
IgC
IgC
CD80
CD80
ICOSL: Strongly Selected Residues
Front
Back
Q100
F104*
N52
Y33*
L98*
Y35*
L96*
Q37*
S41*
T43*
T46*
N57
180°
L102*
S101*
G103*
N30
Q105*
E106*
V110
S109*
E40*
H94
H115
C198
ICOSL
ICOSL
ICOSL
ICOSL

FIG. 2A

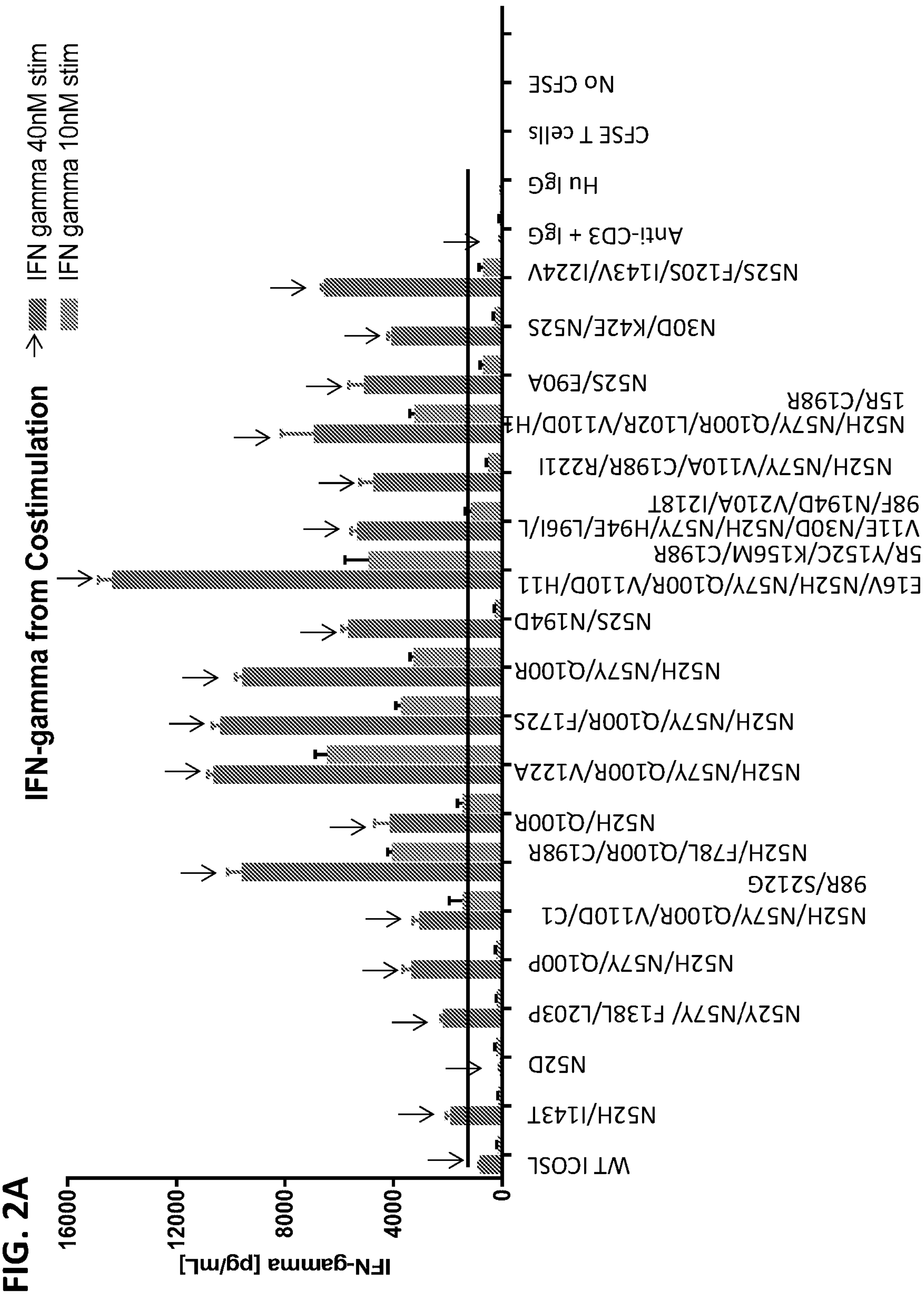
IFN-gamma from Costimulation
IFN gamma 40nM stim
IFN gamma 10nM stim
IFN-gamma [pg/mL]
16000
12000
8000
4000
0
WT ICOSL
N52H/I143T
N52D
N52Y/N57Y/ F138L/L203P
N52H/N57Y/Q100P
N52H/N57Y/Q100R/V110D/C198R/S212G
N52H/F78L/Q100R/C198R
N52H/Q100R
N52H/N57Y/Q100R/V122A
N52H/N57Y/Q100R/F172S
N52H/N57Y/Q100R
N52S/N194D
E16V/N52H/N57Y/Q100R/V110D/H115R/Y152C/K156M/C198R
V11E/N30D/N52H/N57Y/H94E/L96I/L98F/N194D/V210A/I218T
N52H/N57Y/V110A/C198R/R221I
N52H/N57Y/Q100R/L102R/V110D/H115R/C198R
N52S/E90A
N30D/K42E/N52S
N52S/F120S/I143V/I224V
Anti-CD3 + IgG
Hu IgG
CFSE T cells
No CFSE

FIG. 2B

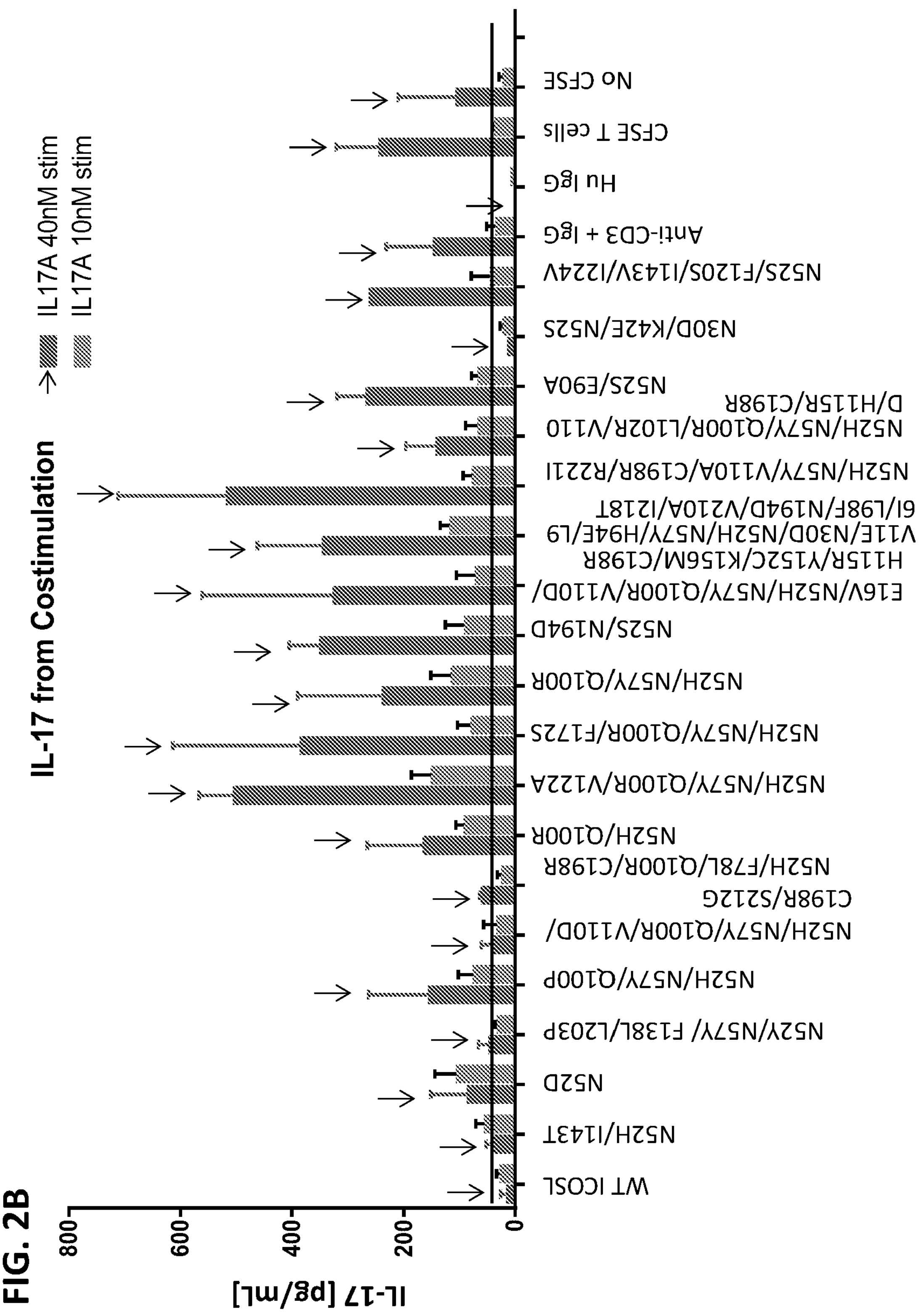
IL-17 from Costimulation
IL17A 40nM stim
IL17A 10nM stim
IL-17 [pg/mL]
0
200
400
600
800
WT ICOSL
N52H/I143T
N52D
N52Y/N57Y/ F138L/L203P
N52H/N57Y/Q100P
N52H/N57Y/Q100R/V110D/
C198R/S212G
N52H/F78L/Q100R/C198R
N52H/Q100R
N52H/N57Y/Q100R/V122A
N52H/N57Y/Q100R/F172S
N52H/N57Y/Q100R
N52S/N194D
E16V/N52H/N57Y/Q100R/V110D/
H115R/Y152C/K156M/C198R
V11E/N30D/N52H/N57Y/H94E/L9
6I/L98F/N194D/V210A/I218T
N52H/N57Y/V110A/C198R/R221I
N52H/N57Y/Q100R/L102R/V110
D/H115R/C198R
N52S/E90A
N30D/K42E/N52S
N52S/F120S/I143V/I224V
Anti-CD3 + IgG
Hu IgG
CFSE T cells
No CFSE

FIG. 3A

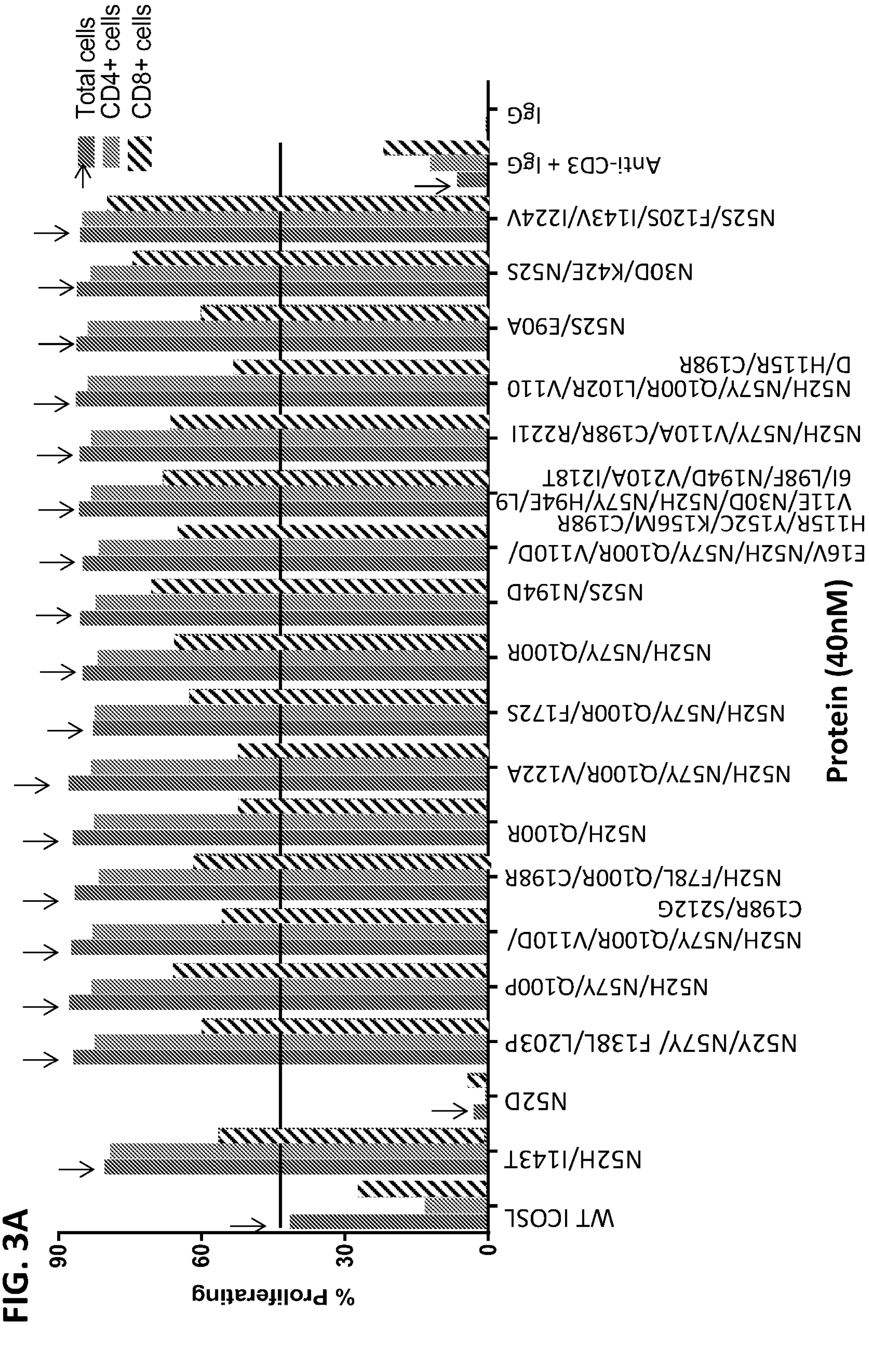
Total cells
CD4+ cells
CD8+ cells
% Proliferating
90
60
30
0
WT ICOSL
N52H/I143T
N52D
N52Y/N57Y/ F138L/L203P
N52H/N57Y/Q100P
N52H/N57Y/Q100R/V110D/ C198R/S212G
N52H/F78L/Q100R/C198R
N52H/Q100R
N52H/N57Y/Q100R/V122A
N52H/N57Y/Q100R/F172S
N52H/N57Y/Q100R
N52S/N194D
E16V/N52H/N57Y/Q100R/V110D/ H115R/Y152C/K156M/C198R
V11E/N30D/N52H/N57Y/H94E/L96I/L98F/N194D/V210A/I218T
N52H/N57Y/V110A/C198R/R221I
N52H/N57Y/Q100R/L102R/V110D/H115R/C198R
N52S/E90A
N30D/K42E/N52S
N52S/F120S/I143V/I224V
Anti-CD3 + IgG
IgG
Protein (40nM)

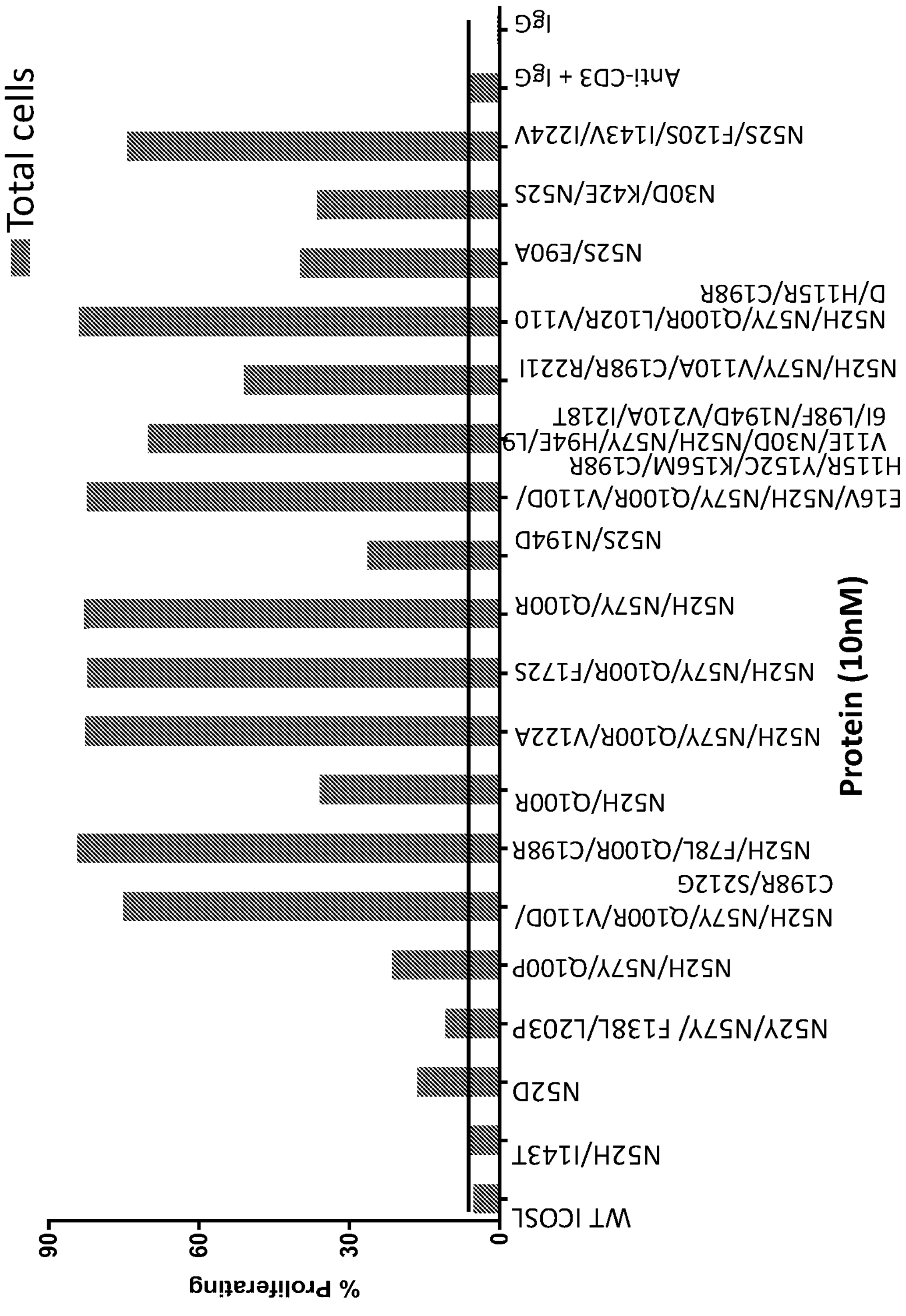
Total cells
% Proliferating
0
30
60
90
Protein (10nM)
WT ICOSL
N52H/I143T
N52D
N52Y/N57Y/ F138L/L203P
N52H/N57Y/Q100P
N52H/N57Y/Q100R/V110D/
C198R/S212G
N52H/F78L/Q100R/C198R
N52H/Q100R
N52H/N57Y/Q100R/V122A
N52H/N57Y/Q100R/F172S
N52H/N57Y/Q100R
N52S/N194D
E16V/N52H/N57Y/Q100R/V110D/
H115R/Y152C/K156M/C198R
V11E/N30D/N52H/N57Y/H94E/L9
6I/L98F/N194D/V210A/I218T
N52H/N57Y/V110A/C198R/R221I
N52H/N57Y/Q100R/L102R/V110
D/H115R/C198R
N52S/E90A
N30D/K42E/N52S
N52S/F120S/I143V/I224V
Anti-CD3 + IgG
IgG

FIG. 3B

FIG. 4

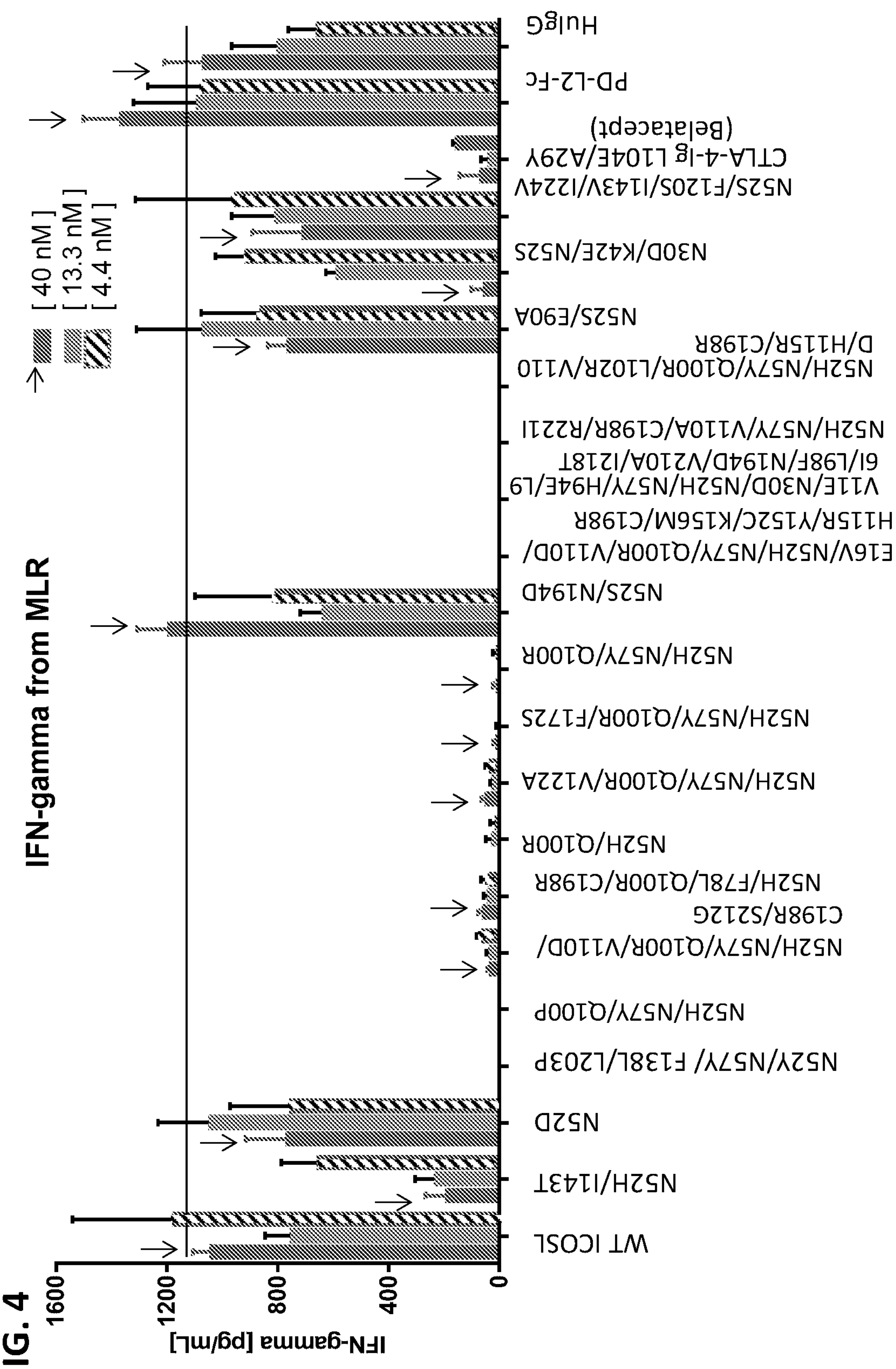
IFN-gamma from MLR
IFN-gamma [pg/mL]
0
400
800
1200
1600
[ 40 nM ]
[ 13.3 nM ]
[ 4.4 nM ]
WT ICOSL
N52H/I143T
N52D
N52Y/N57Y/ F138L/L203P
N52H/N57Y/Q100P
N52H/N57Y/Q100R/V110D/
C198R/S212G
N52H/F78L/Q100R/C198R
N52H/Q100R
N52H/N57Y/Q100R/V122A
N52H/N57Y/Q100R/F172S
N52H/N57Y/Q100R
N52S/N194D
E16V/N52H/N57Y/Q100R/V110D/
H115R/Y152C/K156M/C198R
V11E/N30D/N52H/N57Y/H94E/L9
6I/L98F/N194D/V210A/I218T
N52H/N57Y/V110A/C198R/R221I
N52H/N57Y/Q100R/L102R/V110
D/H115R/C198R
N52S/E90A
N30D/K42E/N52S
N52S/F120S/I143V/I224V
CTLA-4-Ig L104E/A29Y
(Belatacept)
PD-L2-Fc
HuIgG

FIG. 5A

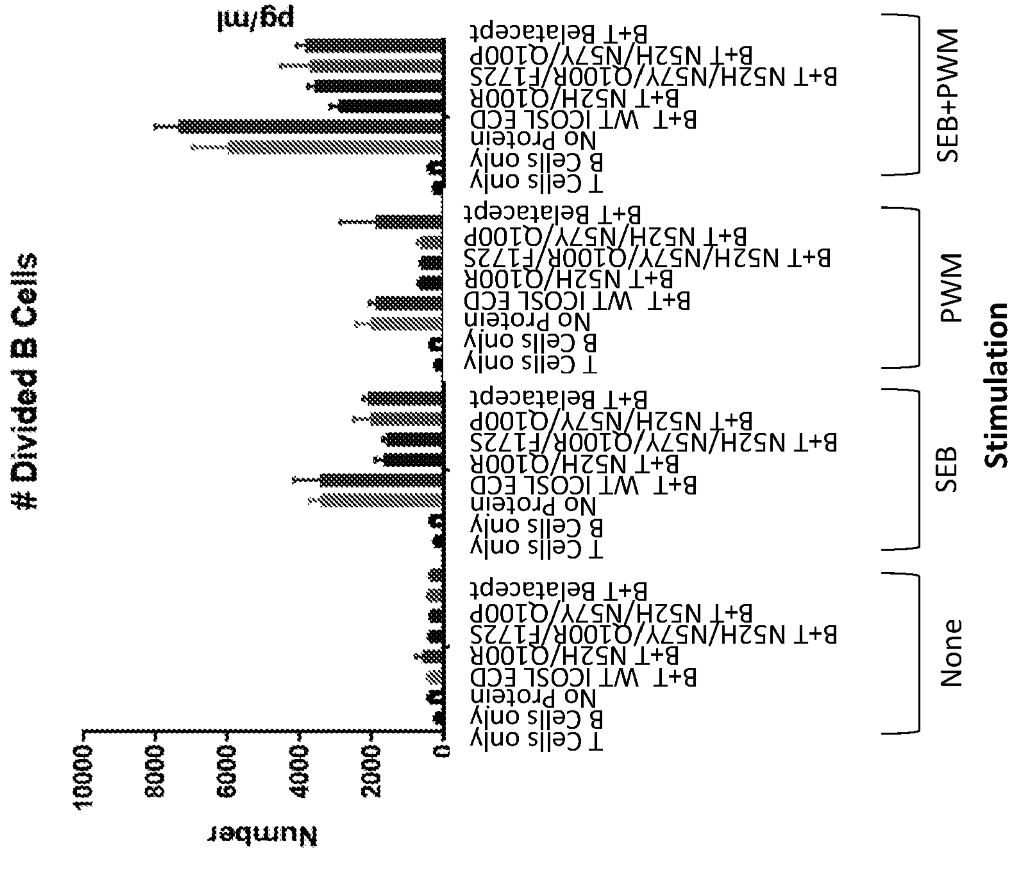
# Divided B Cells
Number
10000
8000
6000
4000
2000
0
T Cells only
B Cells only
No Protein
B+T WT ICOSL ECD
B+T N52H/Q100R
B+T N52H/N57Y/Q100R/F172S
B+T N52H/N57Y/Q100P
B+T Belatacept
None
SEB
PWM
SEB+PWM
Stimulation

FIG. 5B

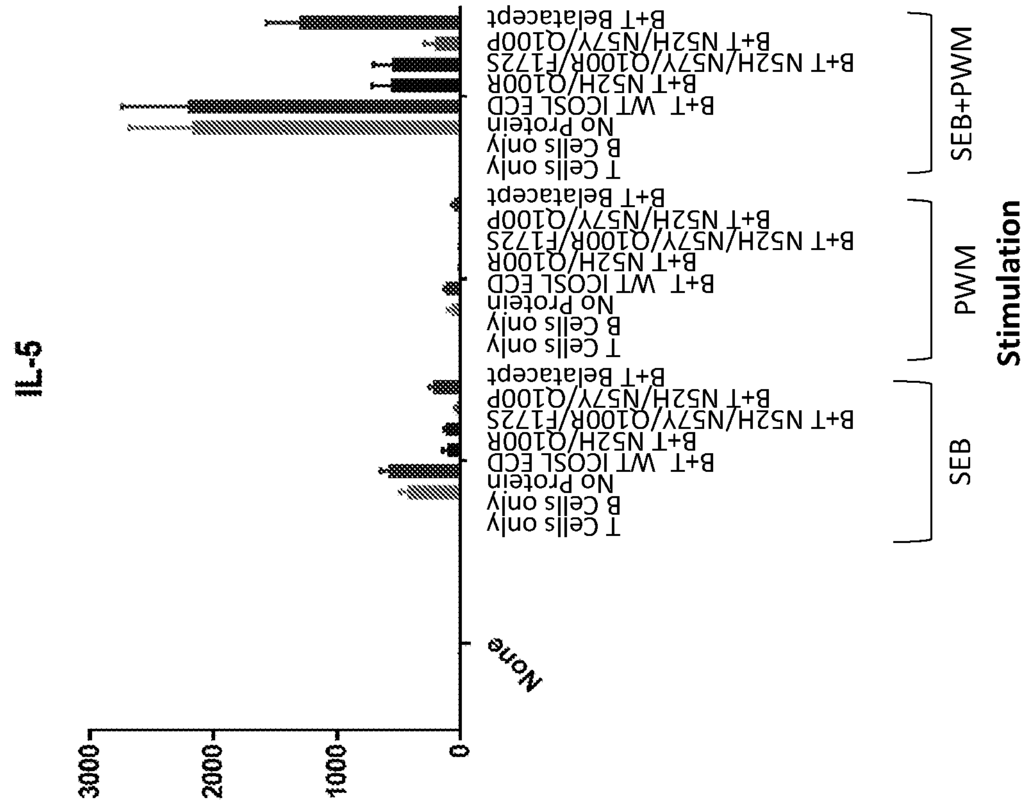
IL-5
pg/ml
3000
2000
1000
0
None
T Cells only
B Cells only
No Protein
B+T WT ICOSL ECD
B+T N52H/Q100R
B+T N52H/N57Y/Q100R/F172S
B+T N52H/N57Y/Q100P
B+T Belatacept
SEB
PWM
SEB+PWM
Stimulation

FIG. 5C

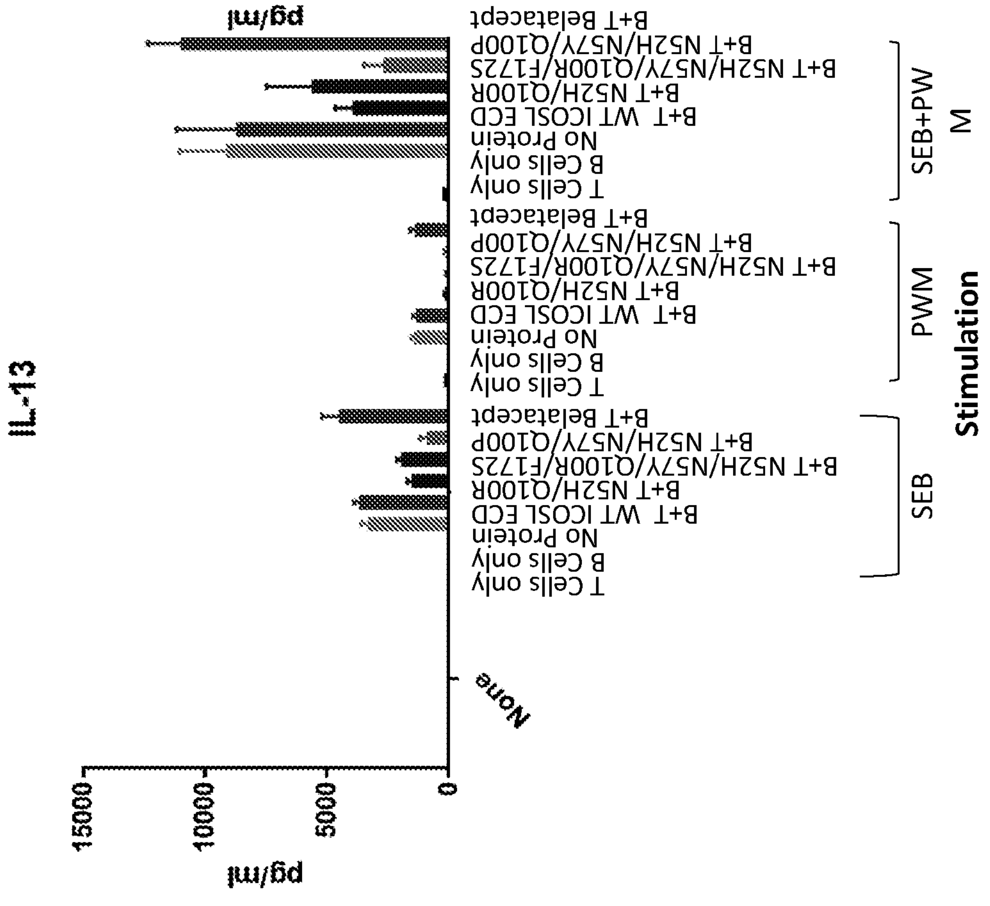
IL-13
pg/ml
15000
10000
5000
0
None
T Cells only
B Cells only
No Protein
B+T WT ICOSL ECD
B+T N52H/Q100R
B+T N52H/N57Y/Q100R/F172S
B+T N52H/N57Y/Q100P
B+T Belatacept
SEB
PWM
SEB+PW M
Stimulation

FIG. 5D

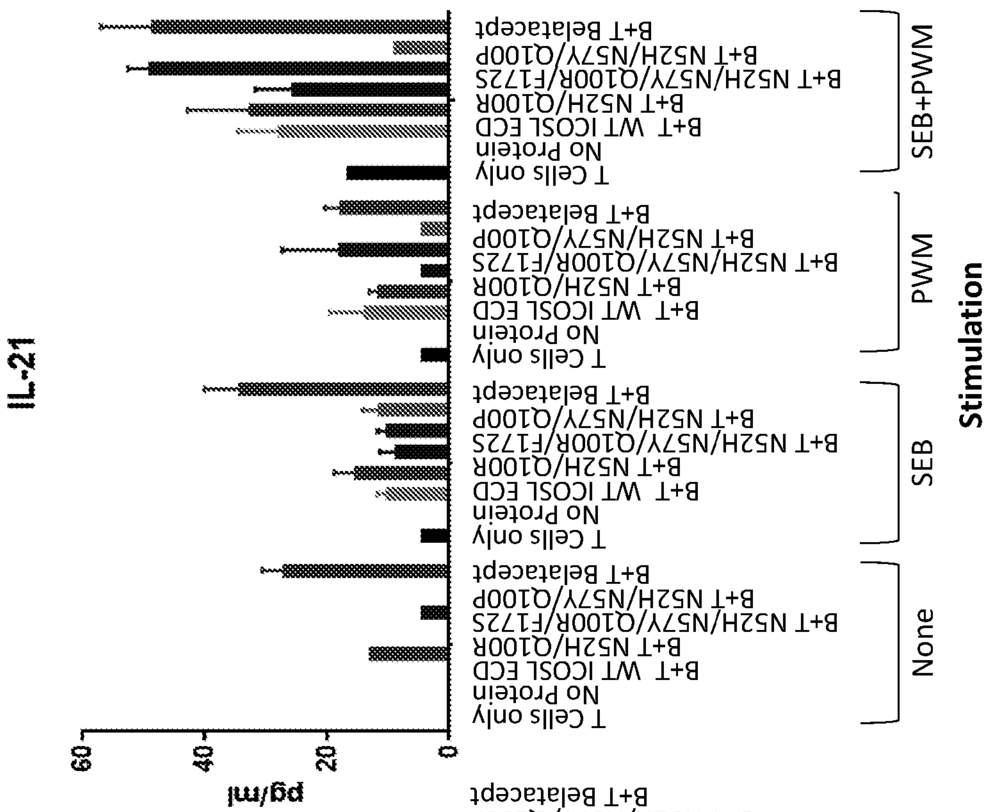
IL-21
pg/ml
60
40
20
0
T Cells only
No Protein
B+T WT ICOSL ECD
B+T N52H/Q100R
B+T N52H/N57Y/Q100R/F172S
B+T N52H/N57Y/Q100P
B+T Belatacept
None
SEB
PWM
SEB+PWM
Stimulation

FIG. 6A

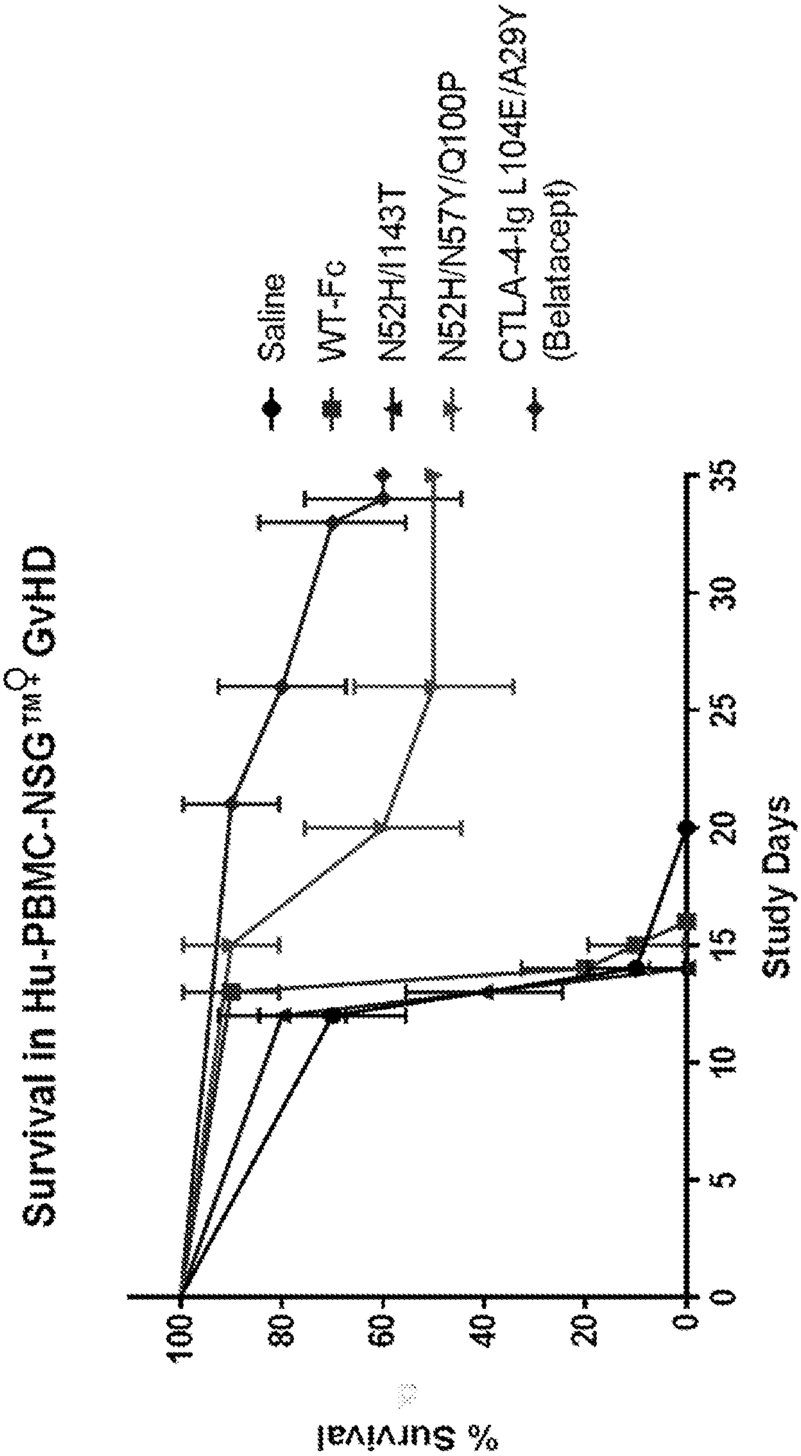
Survival in Hu-PBMC-NSG™♀ GvHD
% Survival
100
80
60
40
20
0
0
5
10
15
20
25
30
35
Study Days
Saline
WT-Fc
N52H/I143T
N52H/N57Y/Q100P
CTLA-4-Ig L104E/A29Y
(Belatacept)

FIG. 6B

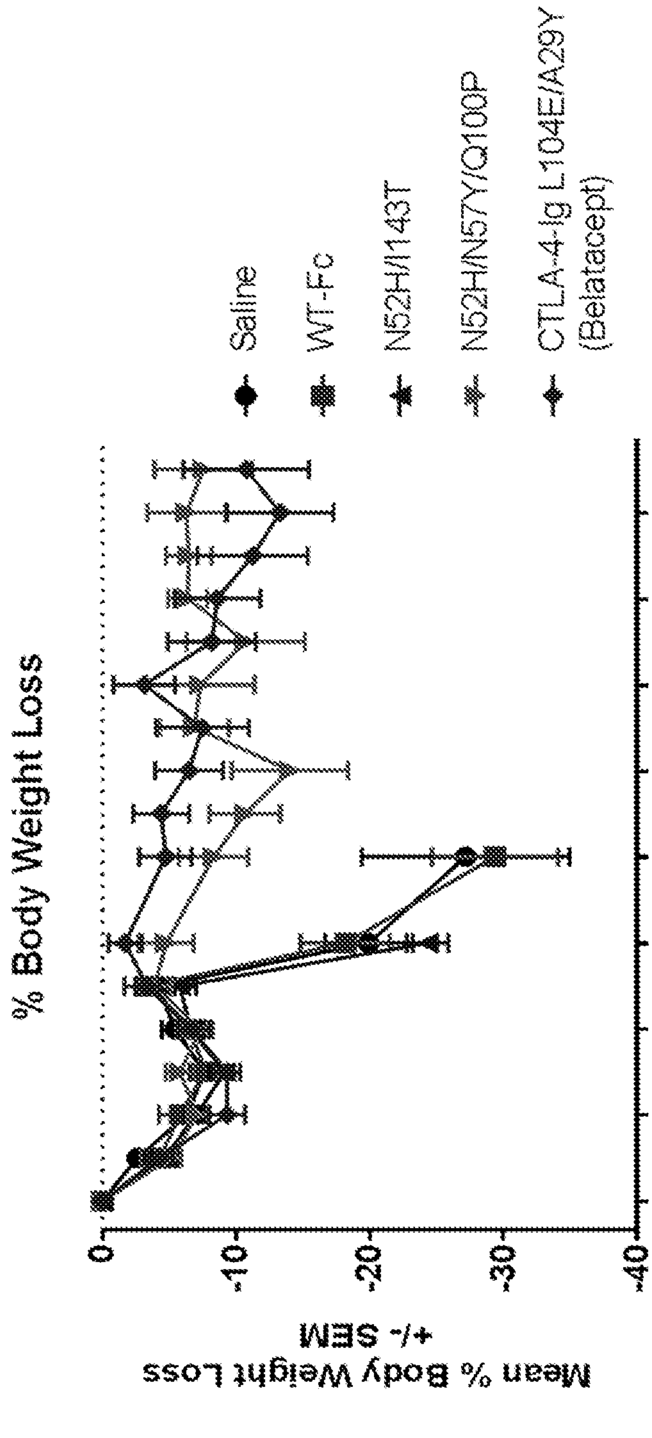
% Body Weight Loss
Mean % Body Weight Loss +/- SEM
0
-10
-20
-30
-40
-1
2
7
12
14
19
23
28
33
Study Days
Saline
WT-Fc
N52H/I143T
N52H/N57Y/Q100P
CTLA-4-Ig L104E/A29Y (Belatacept)

FIG. 6C

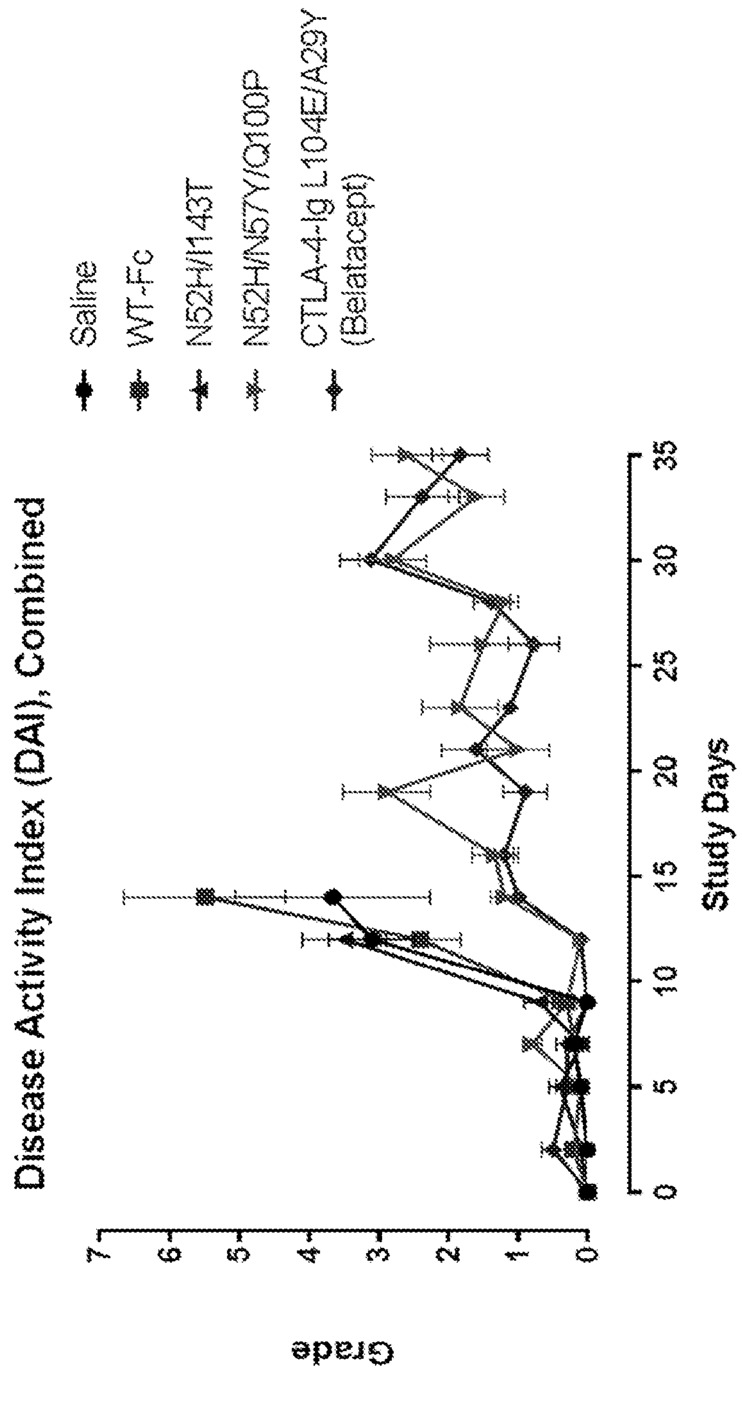
Disease Activity Index (DAI), Combined
Grade
Study Days
Saline
WT-Fc
N52H/I143T
N52H/N57Y/Q100P
CTLA-4-Ig L104E/A29Y
(Belatacept)
0
1
2
3
4
5
6
7
0
5
10
15
20
25
30
35

FIG. 6D

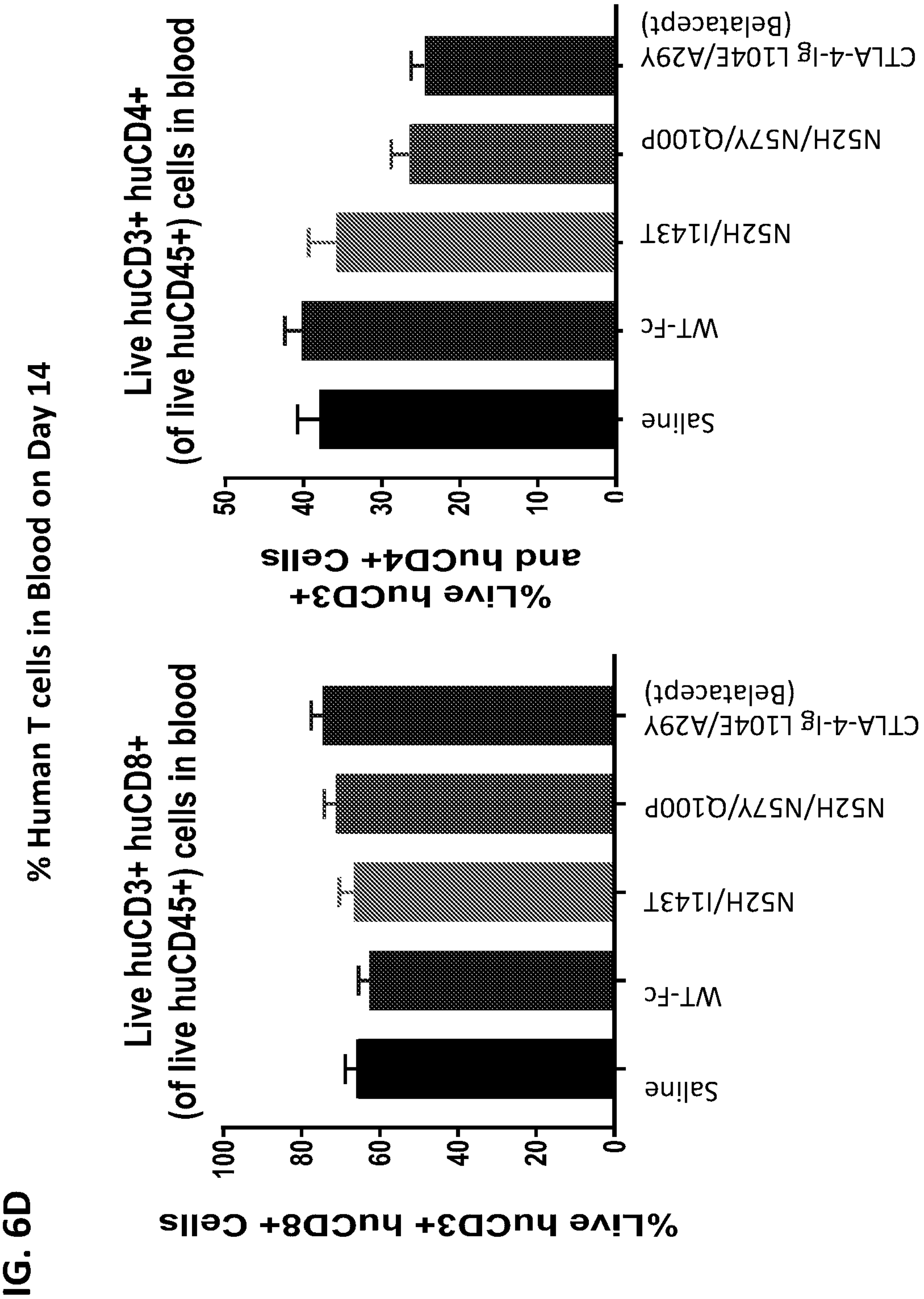
% Human T cells in Blood on Day 14
Live huCD3+ huCD8+
(of live huCD45+) cells in blood
%Live huCD3+ huCD8+ Cells
100
80
60
40
20
0
Saline
WT-Fc
N52H/I143T
N52H/N57Y/Q100P
CTLA-4-Ig L104E/A29Y
(Belatacept)
Live huCD3+ huCD4+
(of live huCD45+) cells in blood
%Live huCD3+
and huCD4+ Cells
50
40
30
20
10
0
Saline
WT-Fc
N52H/I143T
N52H/N57Y/Q100P
CTLA-4-Ig L104E/A29Y
(Belatacept)

FIG. 6E

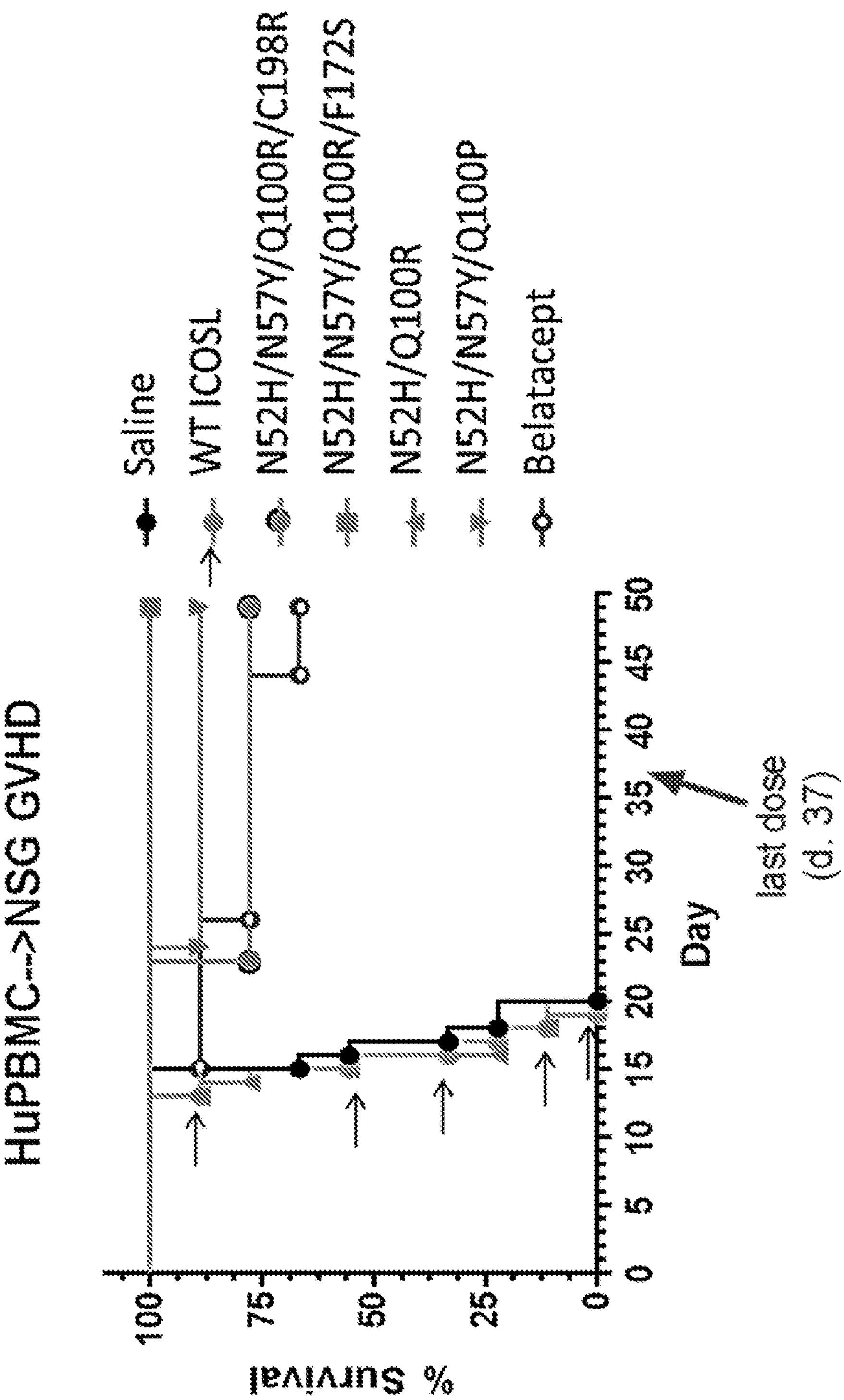
HuPBMC-->NSG GVHD
% Survival
100
75
50
25
0
0
5
10
15
20
25
30
35
40
45
50
Day
last dose
(d. 37)
Saline
WT ICOSL
N52H/N57Y/Q100R/C198R
N52H/N57Y/Q100R/F172S
N52H/Q100R
N52H/N57Y/Q100P
Belatacept

FIG. 6F

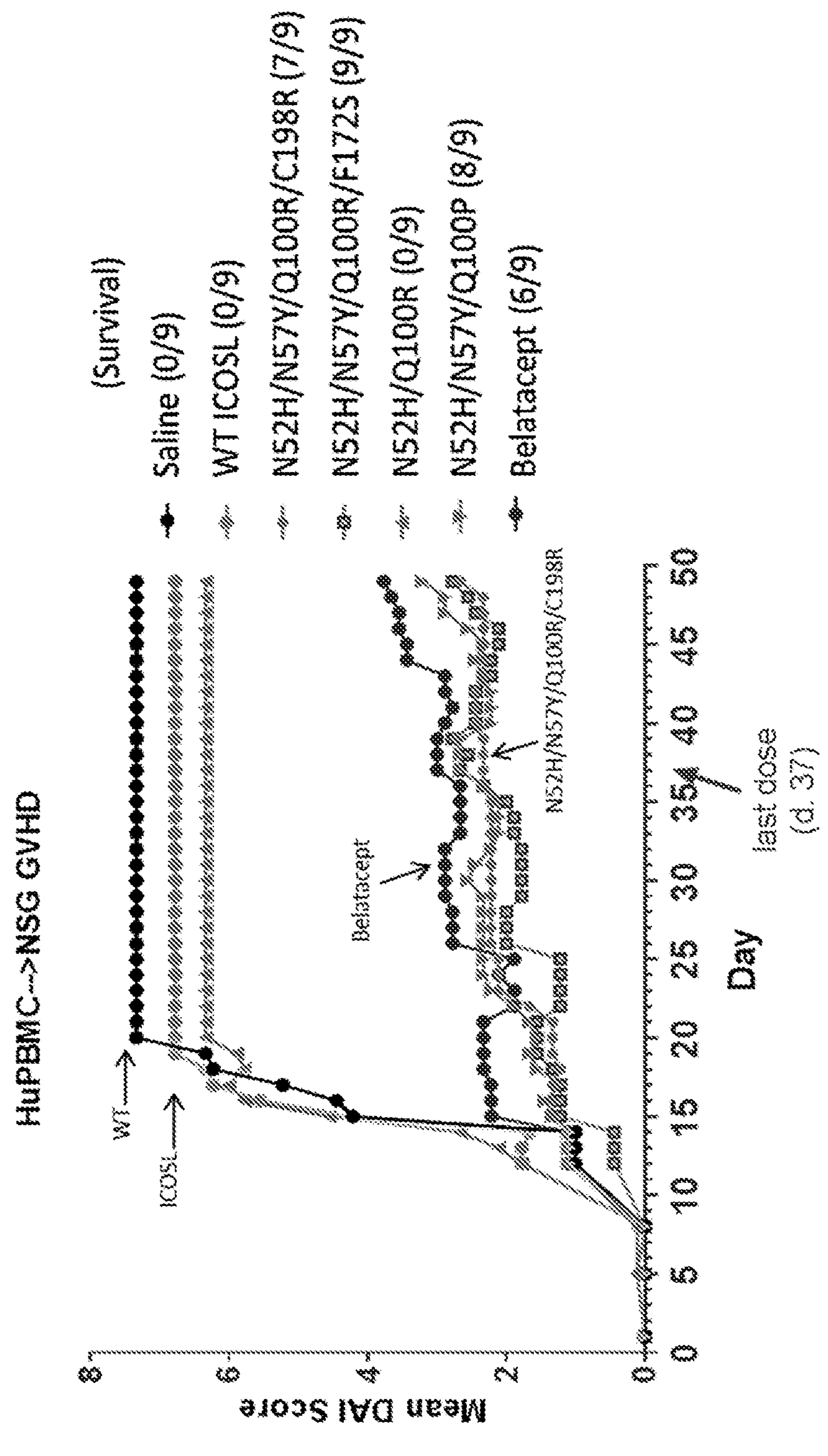
HuPBMC-->NSG GVHD
Mean DAI Score
Day
WT
ICOSL
Belatacept
N52H/N57Y/Q100R/C198R
last dose
(d. 37)
(Survival)
Saline (0/9)
WT ICOSL (0/9)
N52H/N57Y/Q100R/C198R (7/9)
N52H/N57Y/Q100R/F172S (9/9)
N52H/Q100R (0/9)
N52H/N57Y/Q100P (8/9)
Belatacept (6/9)

FIG. 7

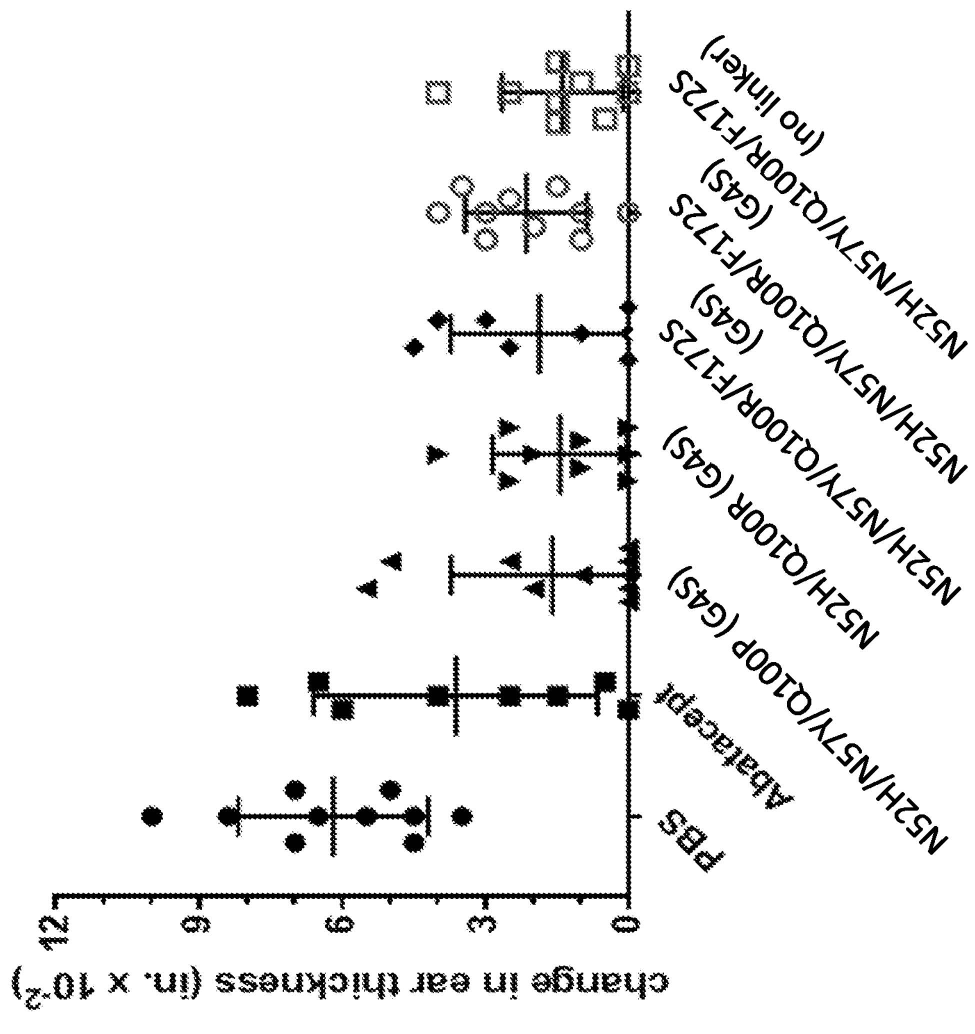
change in ear thickness (in. x $10^{-2}$)
12
9
6
3
0
PBS
Abatacept
N52H/N57Y/Q100P (G4S)
N52H/Q100R (G4S)
N52H/N57Y/Q100R (G4S)
N52H/N57Y/Q100R/F172S (G4S)
N52H/N57Y/Q100R/F172S (no linker)

FIG. 8

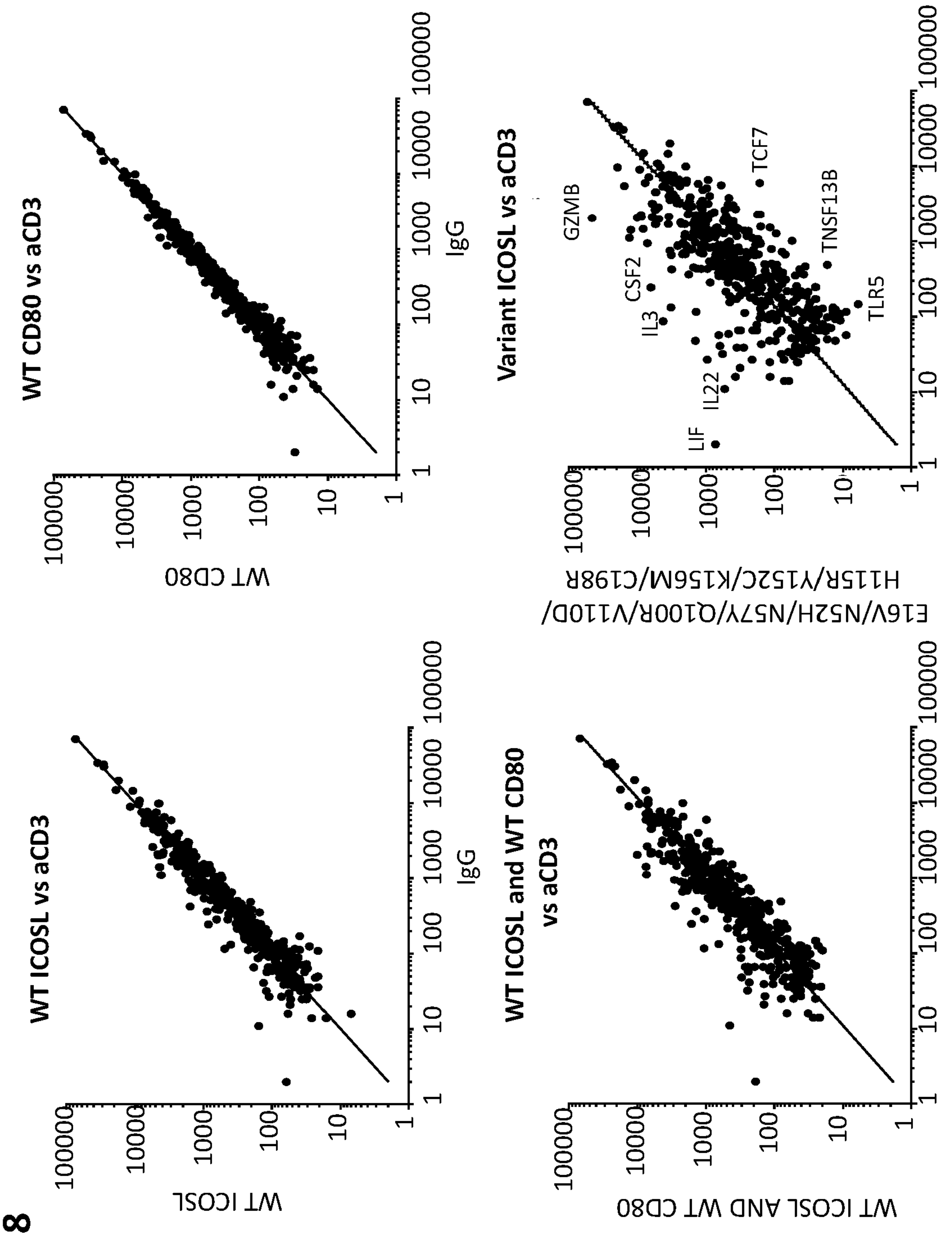
WT ICOSL vs aCD3
WT ICOSL
IgG
WT CD80 vs aCD3
WT CD80
IgG
WT ICOSL and WT CD80 vs aCD3
WT ICOSL AND WT CD80
IgG
Variant ICOSL vs aCD3
E16V/N52H/N57Y/Q100R/V110D/ H115R/Y152C/K156M/C198R
IgG
GZMB
CSF2
IL3
IL22
LIF
TCF7
TNSF13B
TLR5
1
10
100
1000
10000
100000

FIG. 9

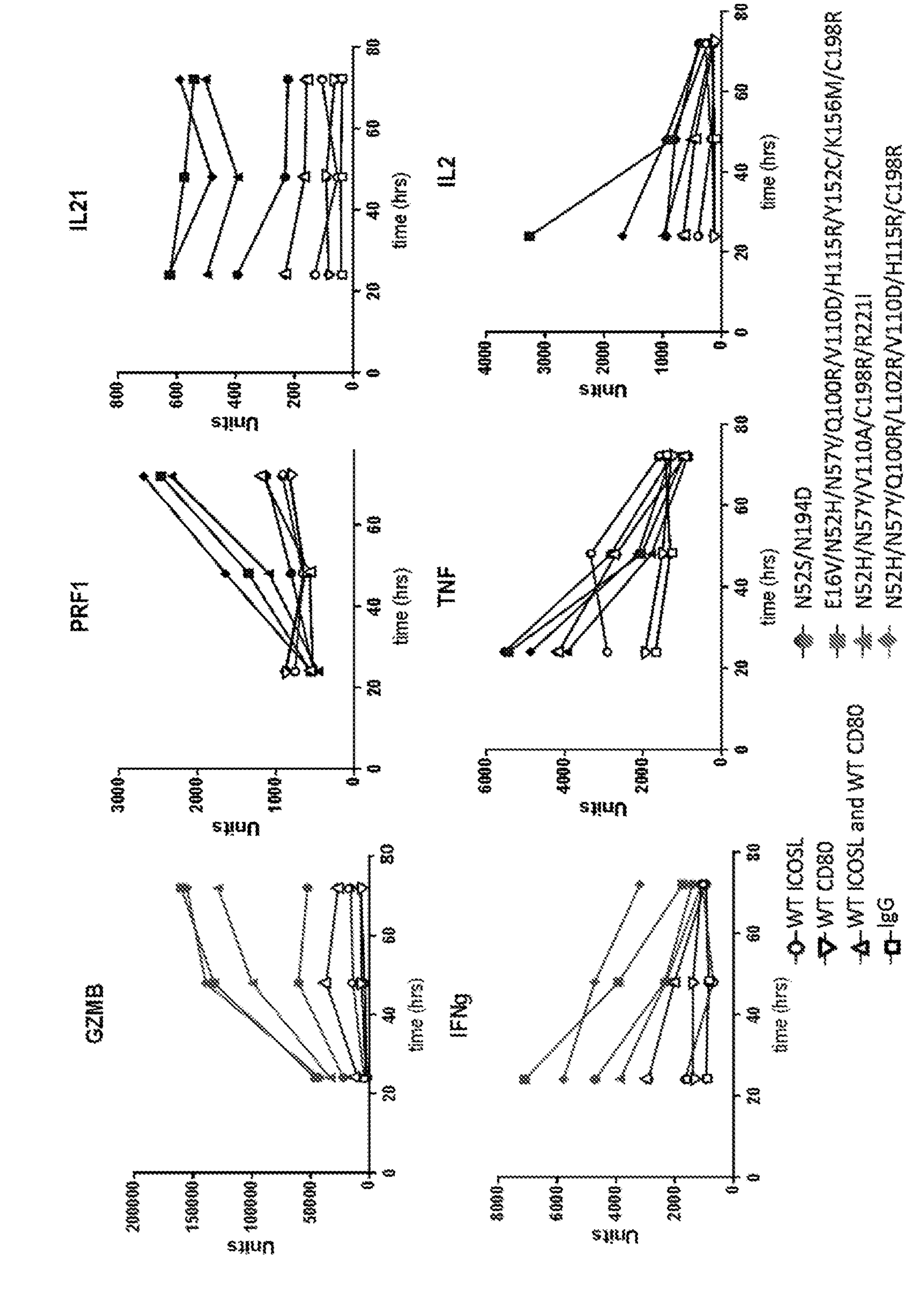
GZMB
PRF1
IL21
IFNg
TNF
IL2
Units
time (hrs)
WT ICOSL
WT CD80
WT ICOSL and WT CD80
IgG
N52S/N194D
E16V/N52H/N57Y/Q100R/V110D/H115R/Y152C/K156M/C198R
N52H/N57Y/V110A/C198R/R221I
N52H/N57Y/Q100R/L102R/V110D/H115R/C198R

FIG. 10A

ICOSL variant ECD (SEQ ID NO: 1) with N52H/N57Y/Q100R/F172S

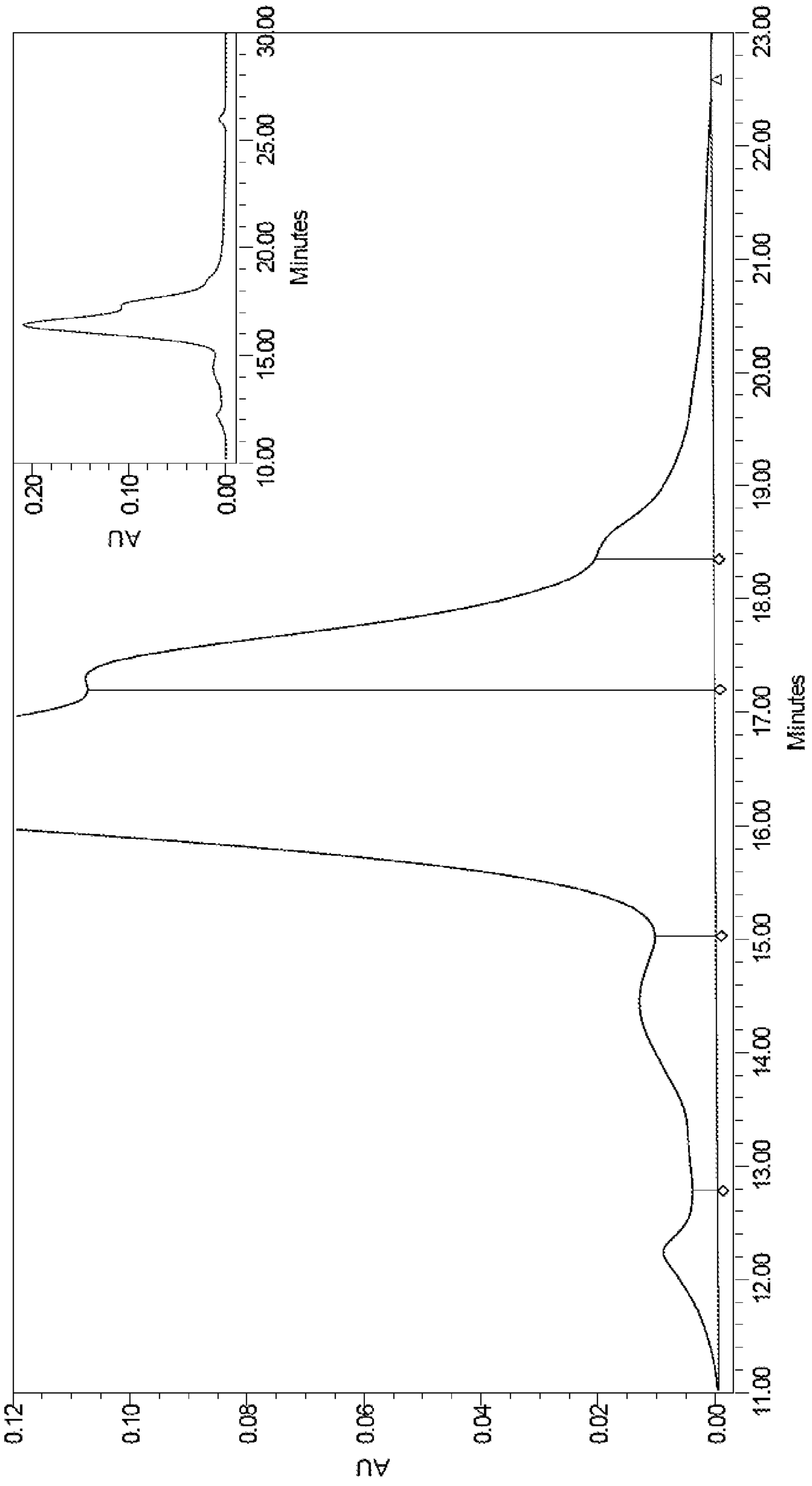

FIG. 10B

ICOSL variant truncation #4 (SEQ ID NO: 7) with N52H/N57Y/Q100R/F172S

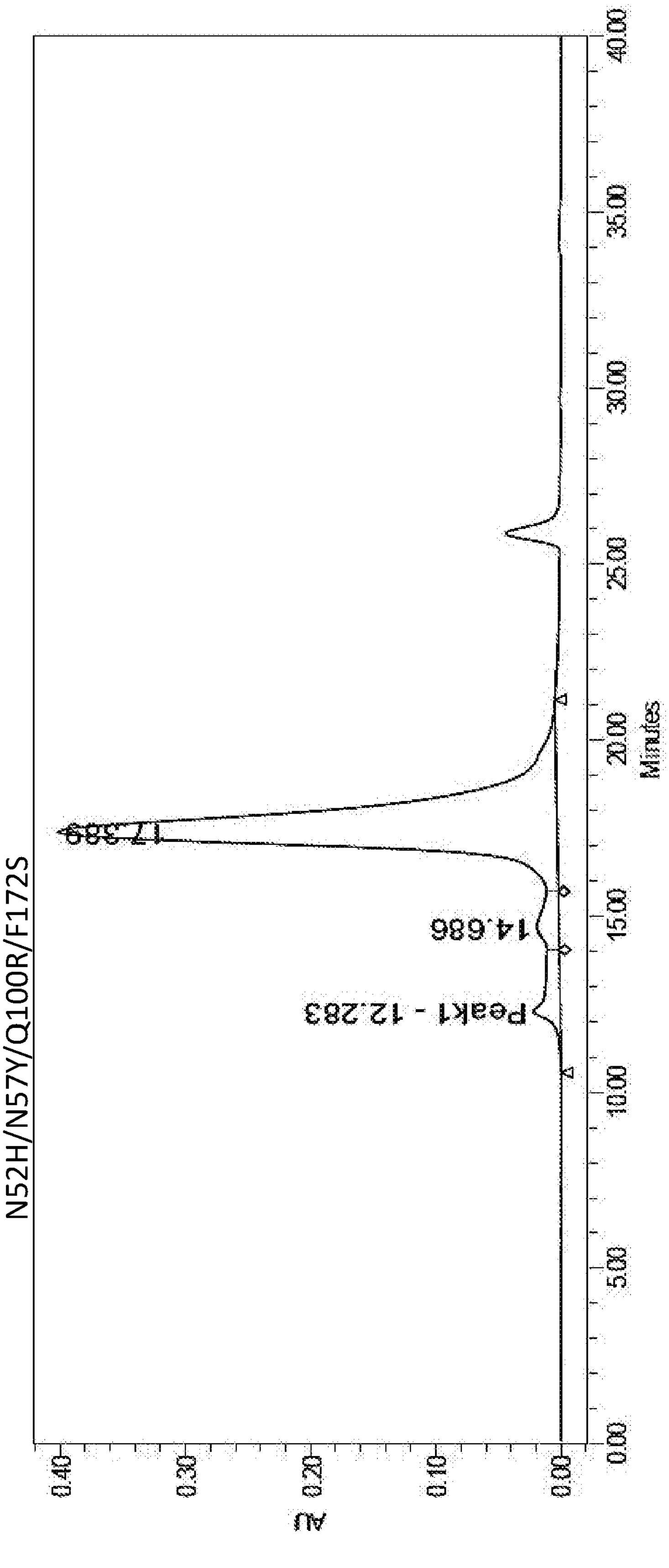

**FIG. 10C**

ICOSL variant truncation #5 (SEQ ID NO: 8) with N52H/N57Y/Q100R/F172S

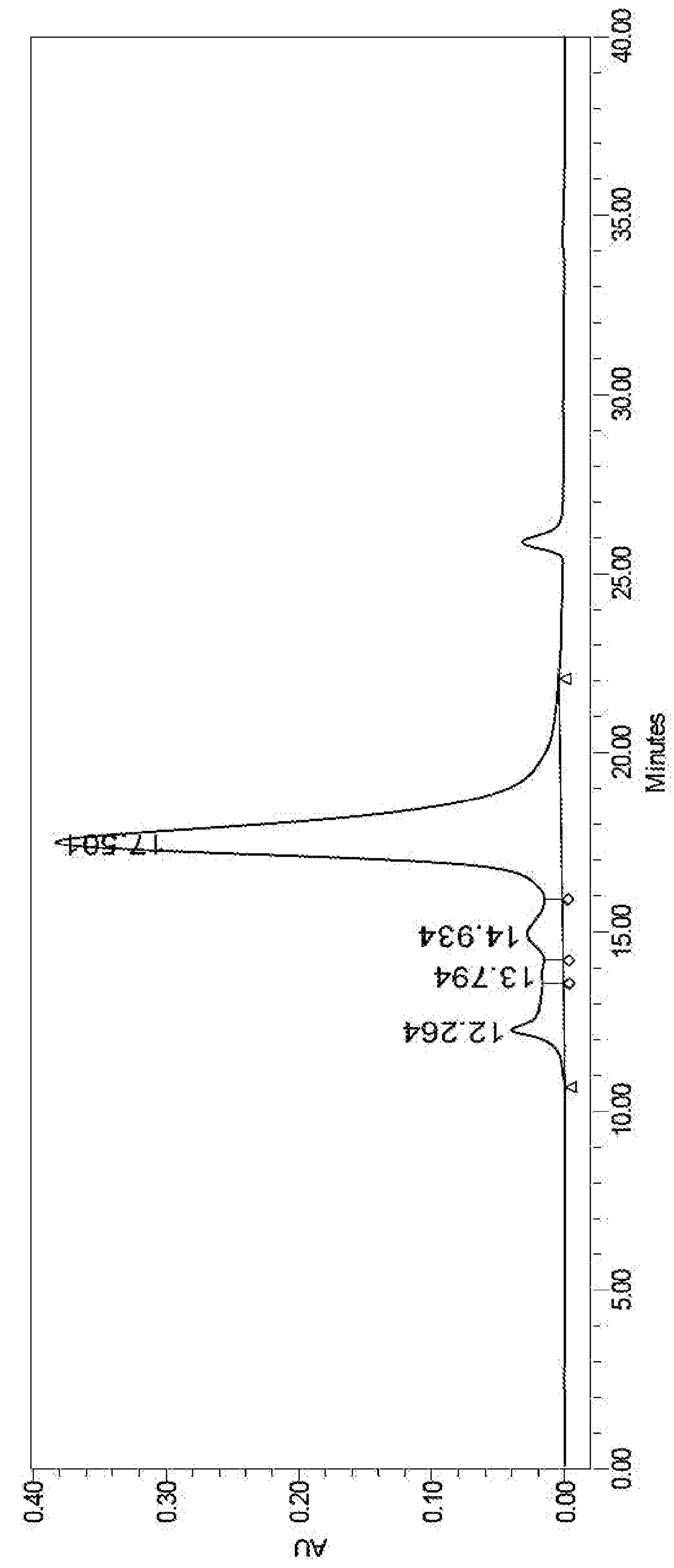

**FIG. 10D**

ICOSL variant truncation #6 (SEQ ID NO: 9) with N52H/N57Y/Q100R/F172S

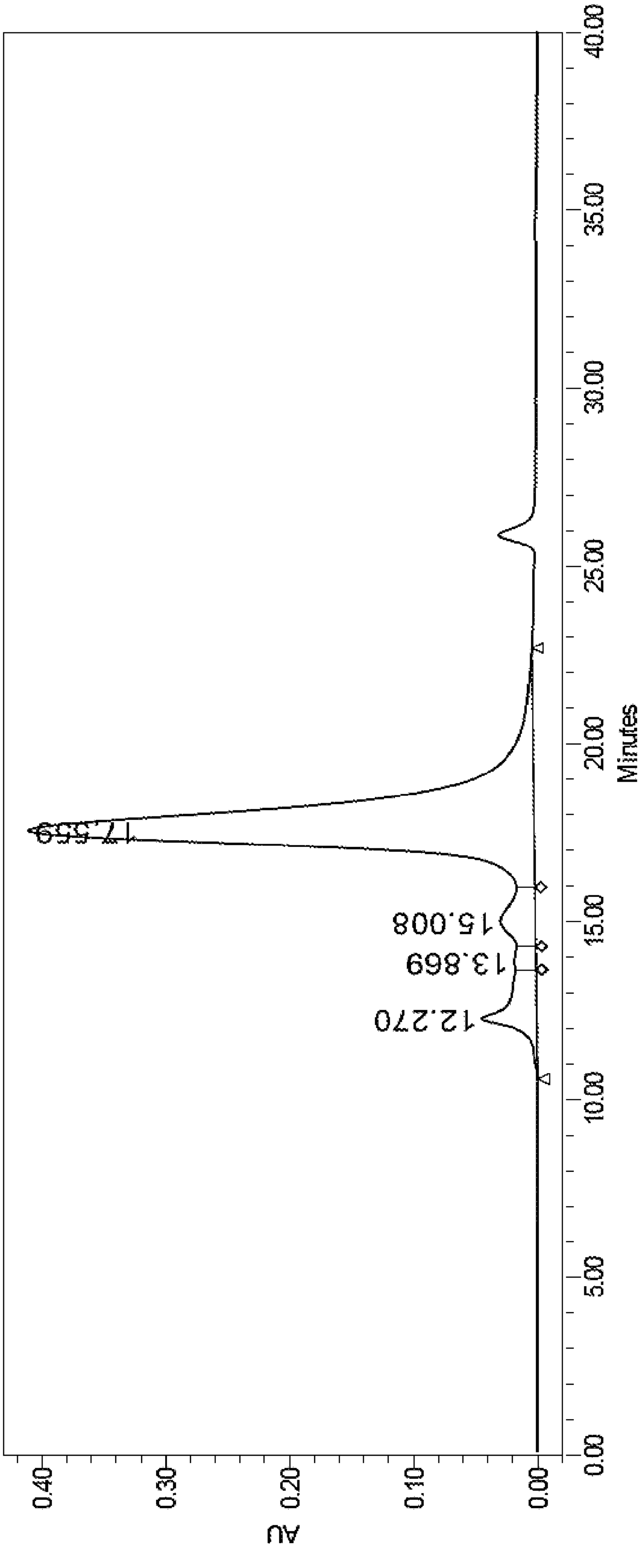

**FIG. 10E**

ICOSL variant truncation #8 with mutations N207G/L208G (SEQ ID NO: 28) and with N52H/N57Y/Q100R/F172S

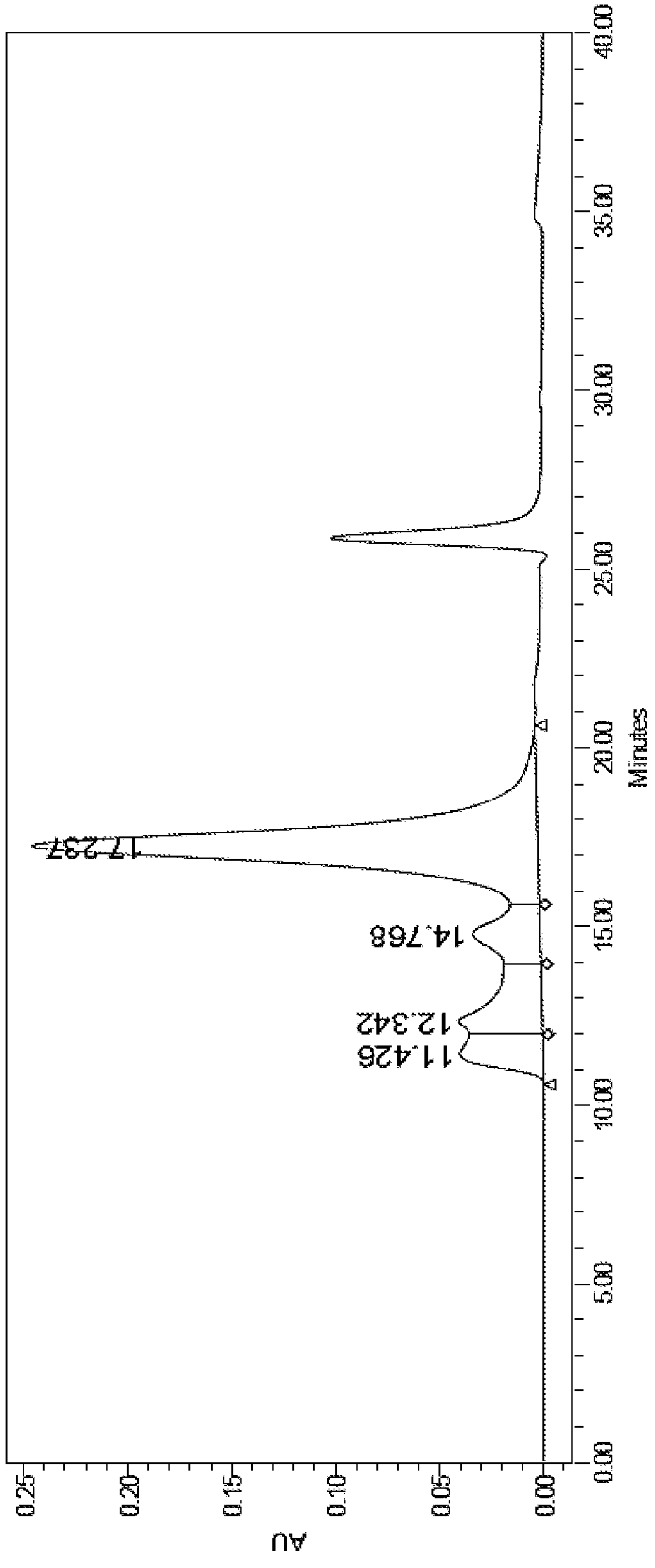

**FIG. 10F**

ICOSL variant ECD with mutations N207G/L208G (SEQ ID NO: 33) with N52H/N57Y/Q100R/F172S

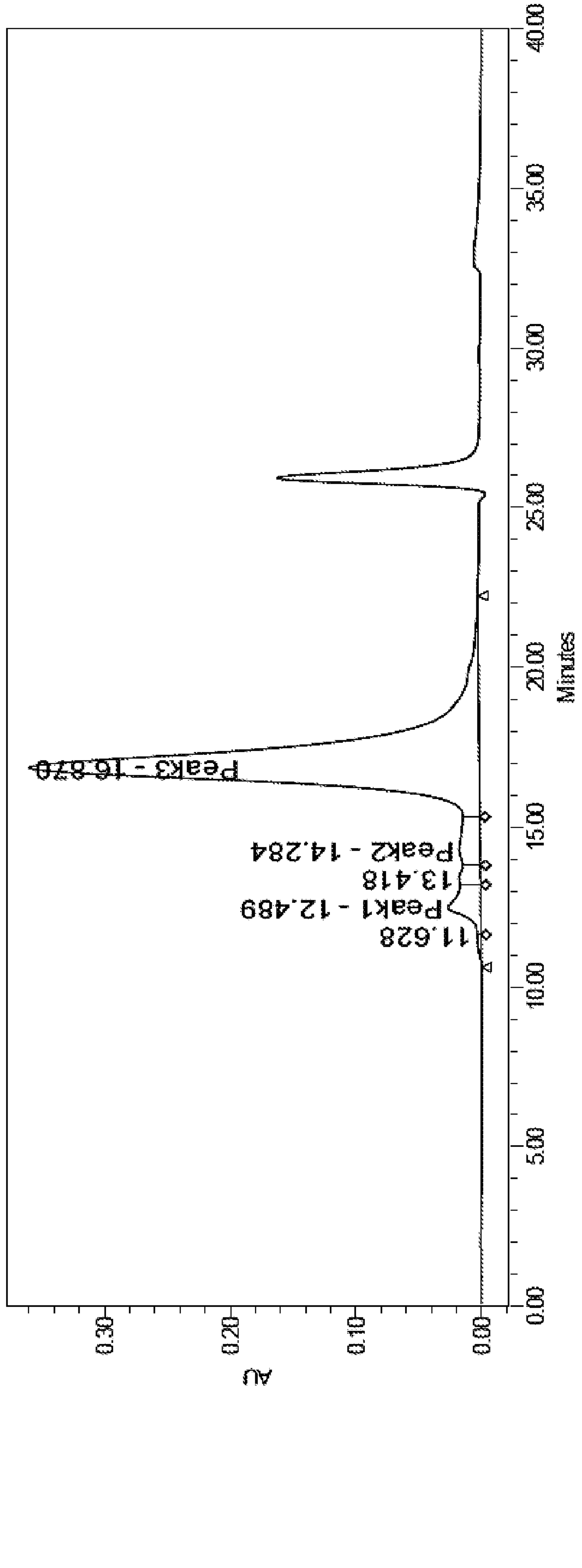

FIG. 10G

ICOSL variant IgV (SEQ ID NO: 3) with N52H/N57Y/Q100R/F172S

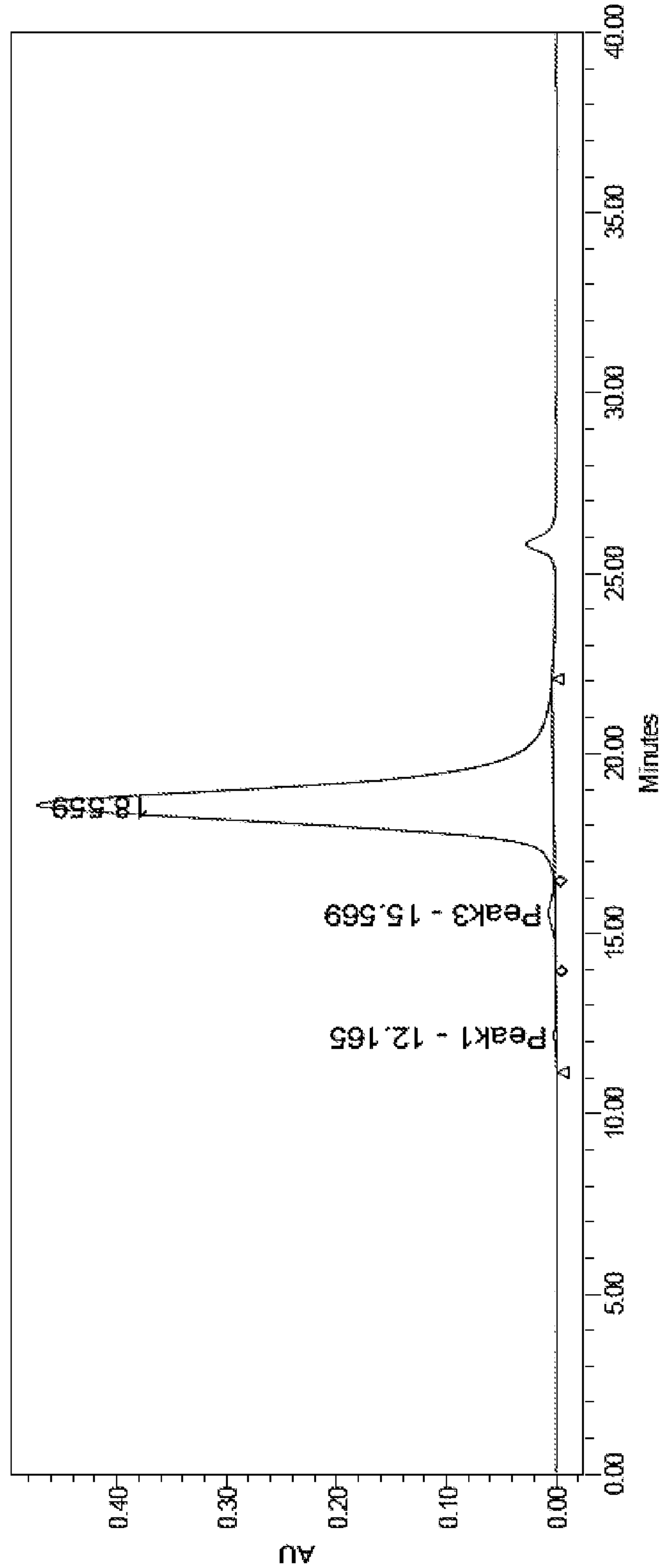

FIG. 10H

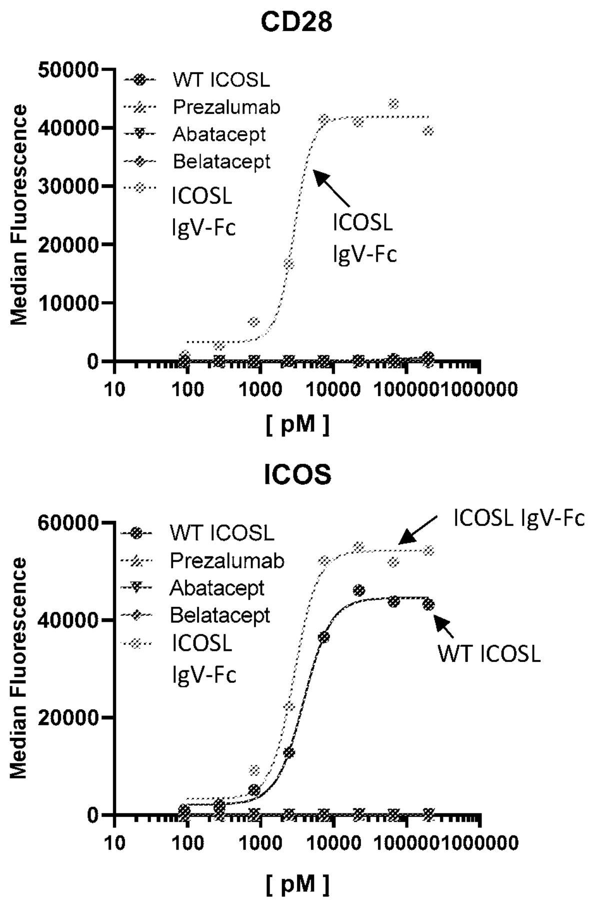


FIG. 10I

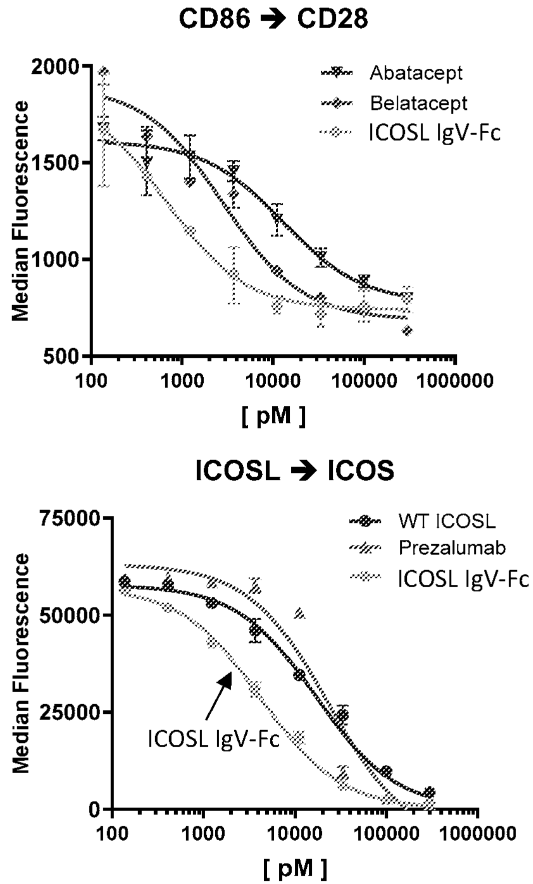


FIG. 10J

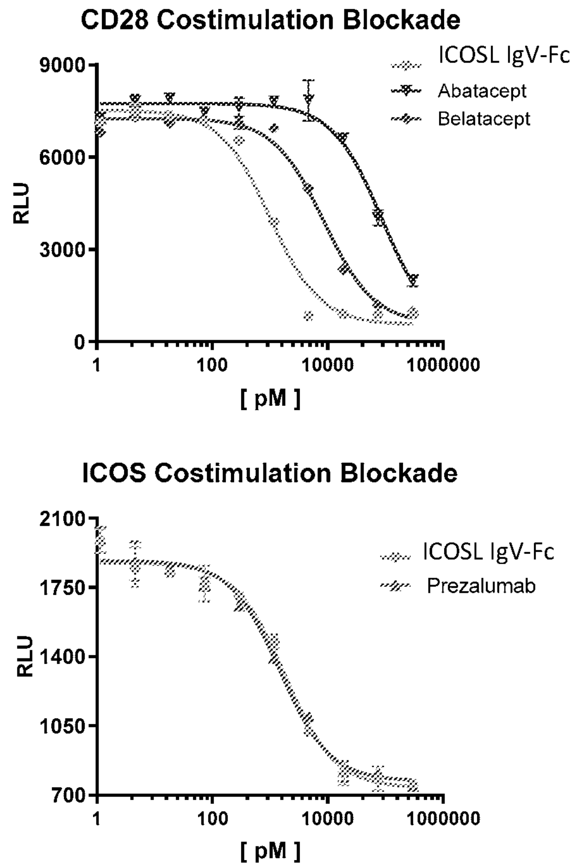

FIG. 11A

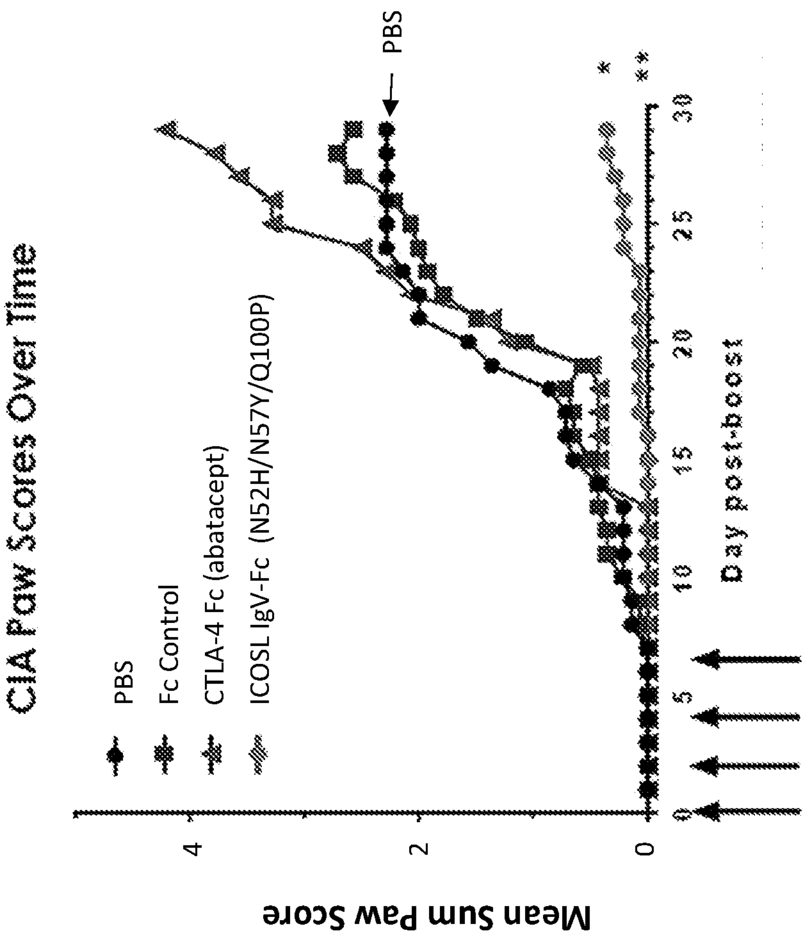
CIA Paw Scores Over Time
PBS
Fc Control
CTLA-4 Fc (abatacept)
ICOSL IgV-Fc (N52H/N57Y/Q100P)
Mean Sum Paw Score
0
2
4
Day post-boost
0
5
10
15
20
25
30
Doses
PBS
*
**

FIG. 11B

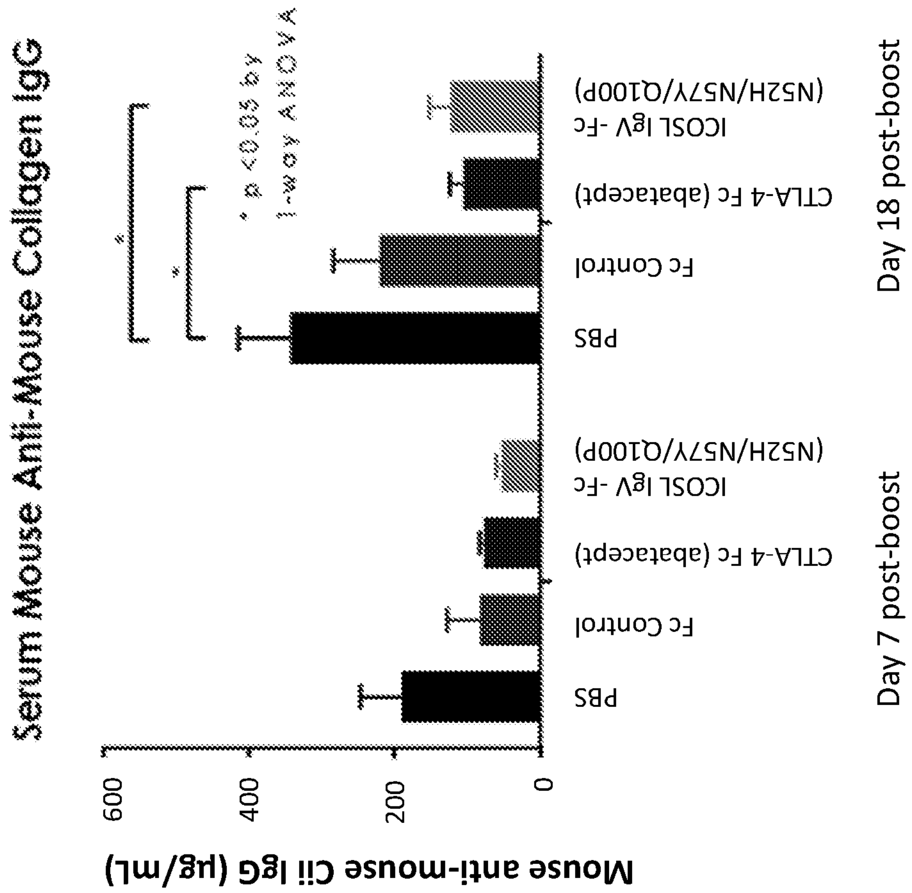
Serum Mouse Anti-Mouse Collagen IgG
Mouse anti-mouse Cii IgG (µg/mL)
600
400
200
0
PBS
Fc Control
CTLA-4 Fc (abatacept)
ICOSL IgV -Fc
(N52H/N57Y/Q100P)
Day 7 post-boost
Day 18 post-boost
*
* p <0.05 by
1-way ANOVA

FIG. 11C

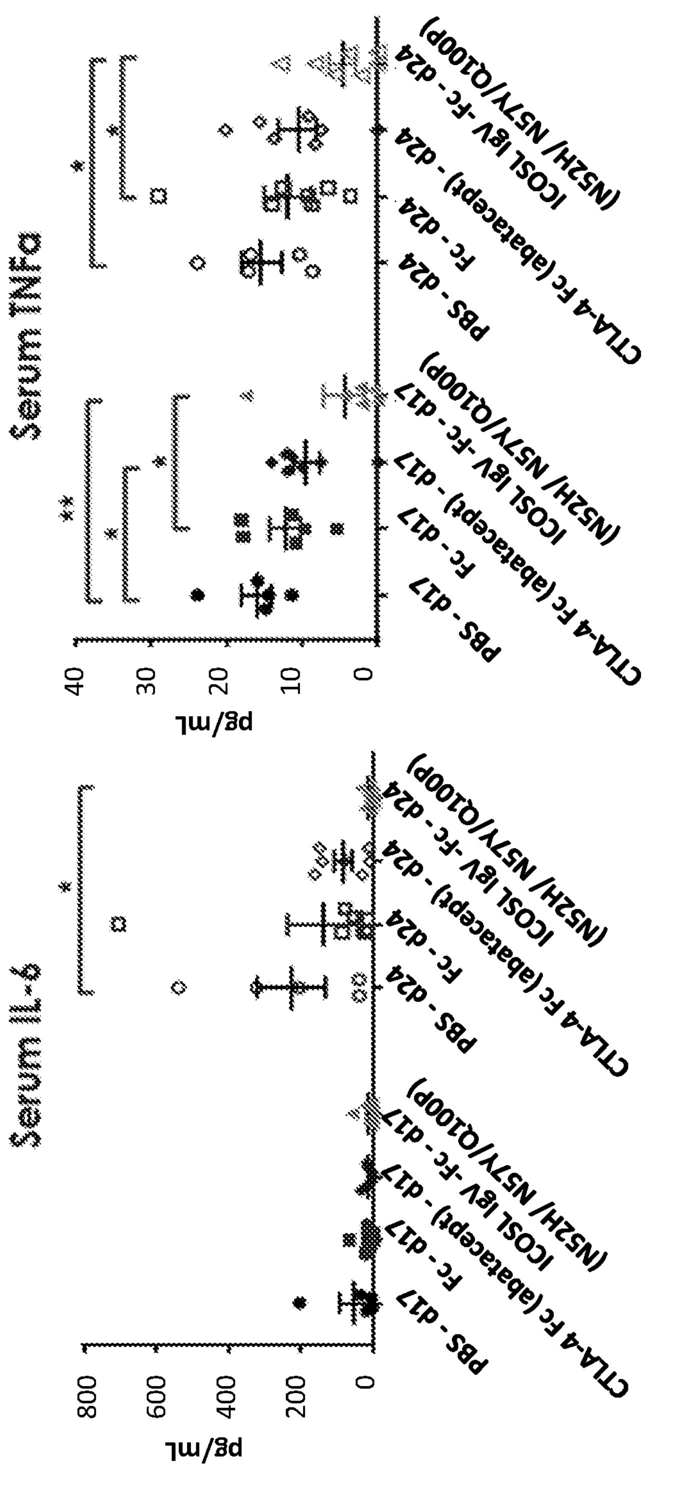
Serum IL-6
Serum TNFα
pg/mL
800
600
400
200
0
40
30
20
10
0
*
**
PBS - d17
Fc - d17
CTLA-4 Fc (abatacept) - d17
ICOSL IgV -Fc - d17
(N52H/ N57Y/Q100P)
PBS - d24
Fc - d24
CTLA-4 Fc (abatacept) - d24
ICOSL IgV -Fc - d24
(N52H/ N57Y/Q100P)

FIG. 11D

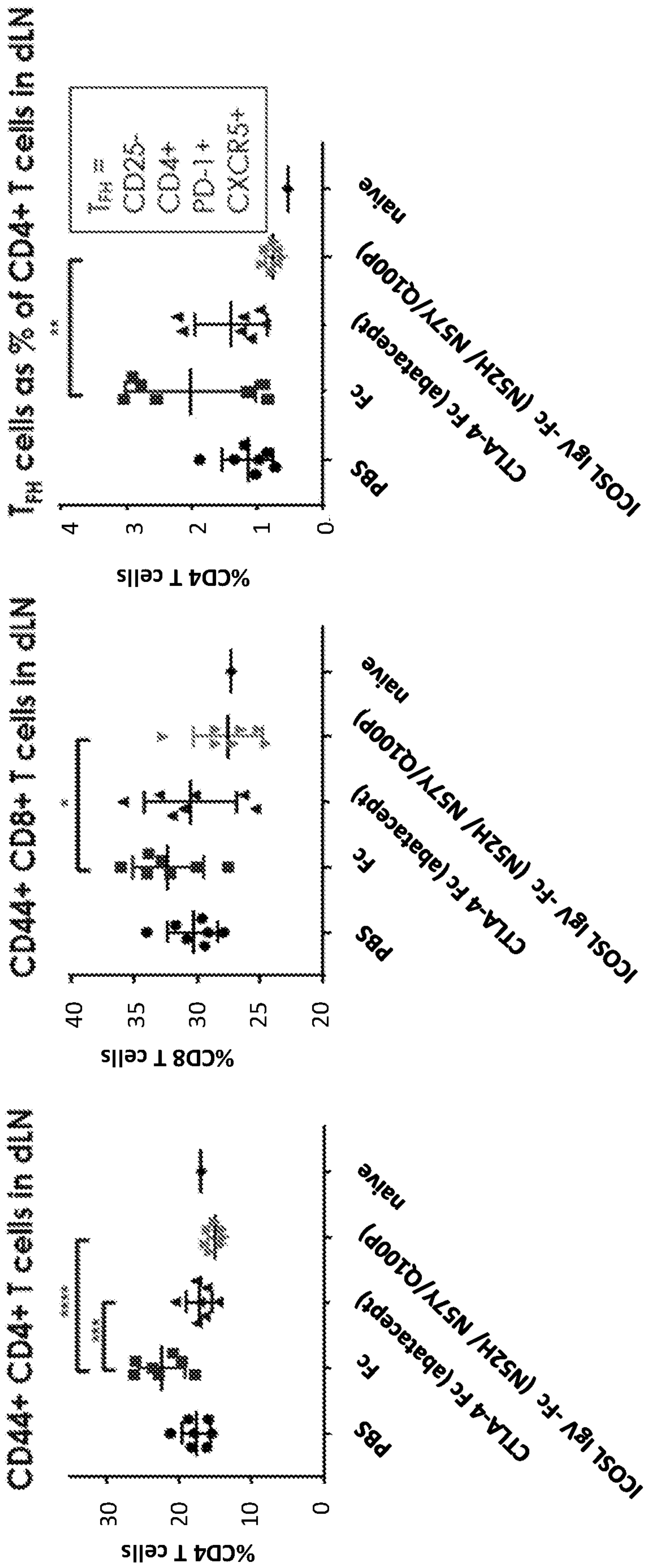
CD44+ CD4+ T cells in dLN
%CD4 T cells
CD44+ CD8+ T cells in dLN
%CD8 T cells
$T_{FH}$ cells as % of CD4+ T cells in dLN
%CD4 T cells
$T_{FH}$ = CD25- CD4+ PD-1+ CXCR5+
PBS
Fc
CTLA-4 Fc (abatacept)
ICOSL IgV -Fc (N52H/ N57Y/Q100P)
naive

FIG. 11E

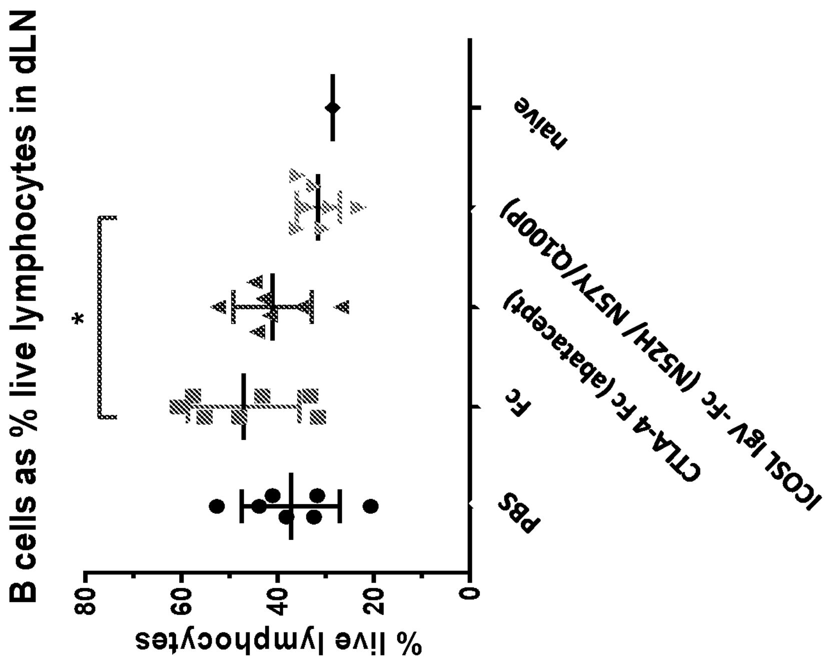
B cells as % live lymphocytes in dLN
% live lymphocytes
80
60
40
20
0
*
PBS
Fc
CTLA-4 Fc (abatacept)
ICOSL IgV -Fc (N52H/ N57Y/Q100P)
naive

FIG. 12A

FIG. 12B

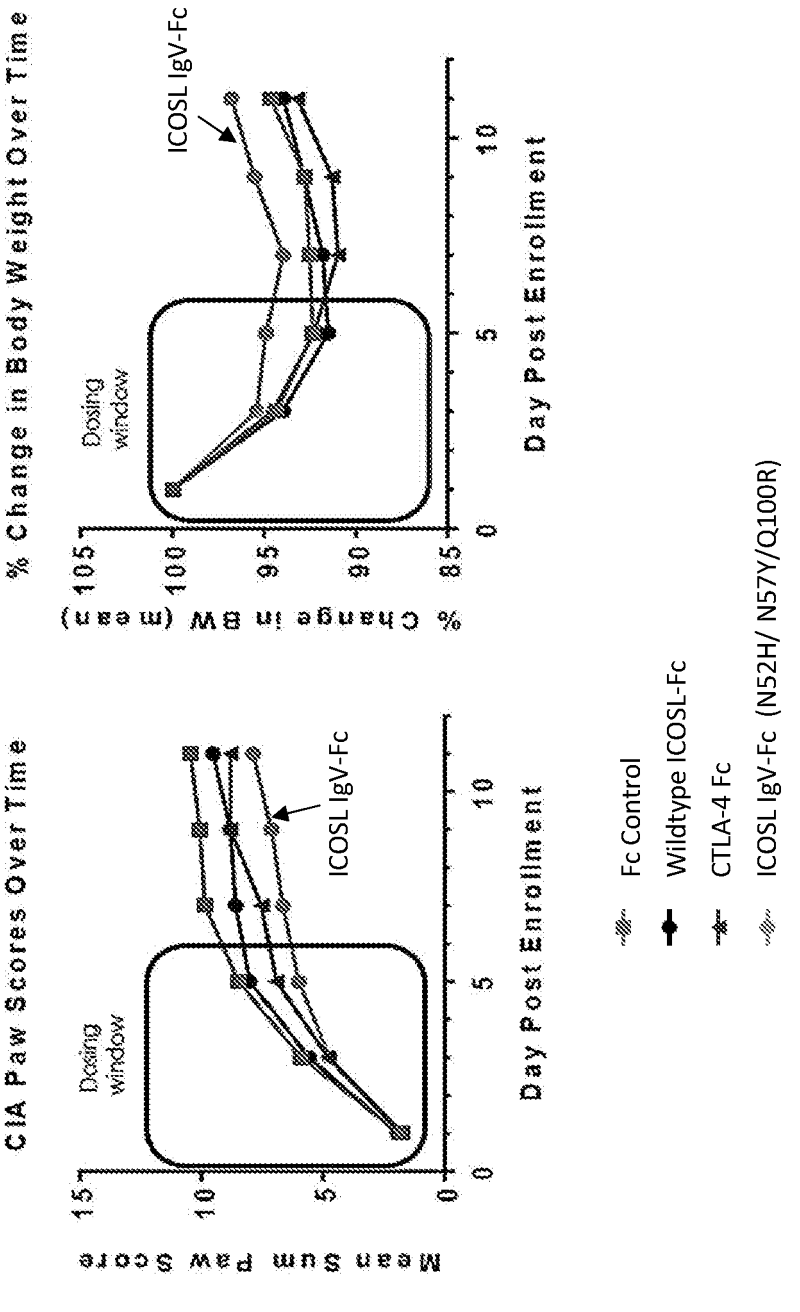

FIG. 12C

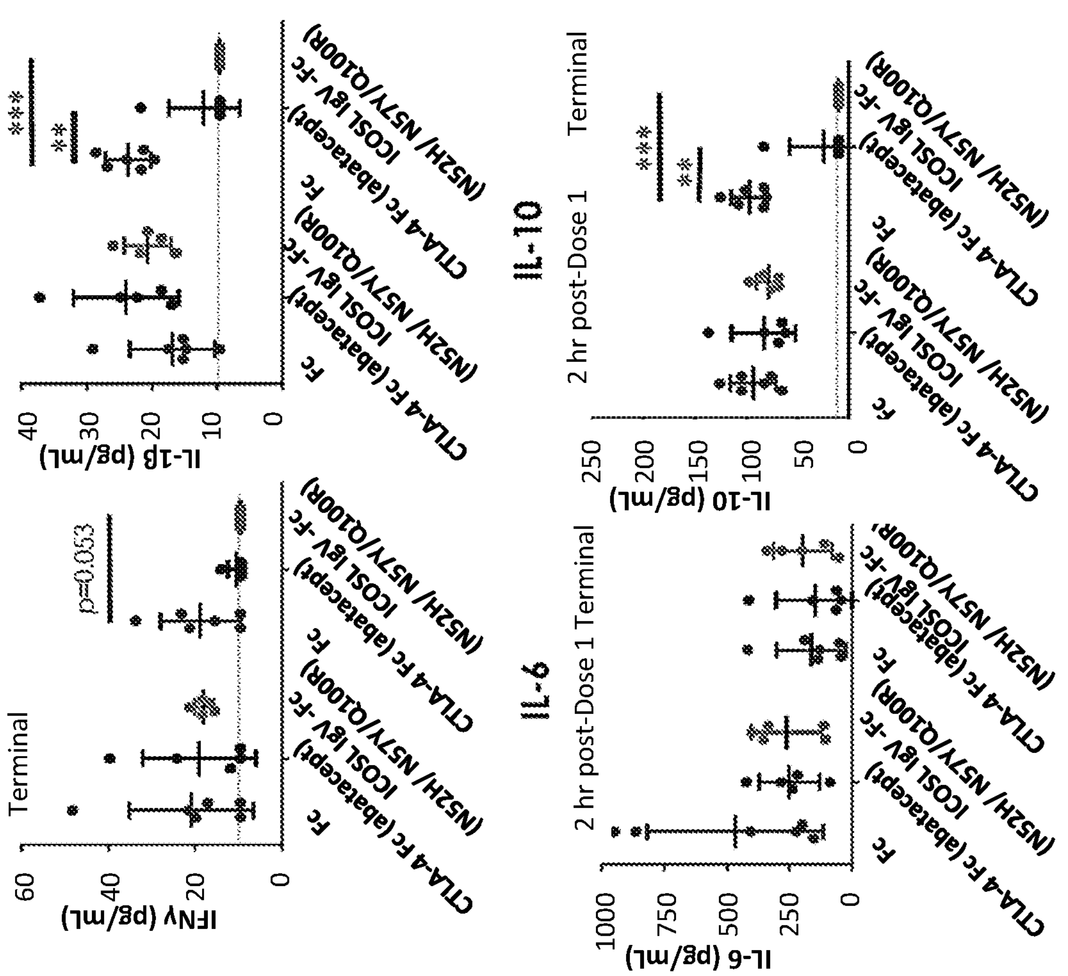
IFNγ
2 hr post-Dose 1
Terminal
IFNγ (pg/mL)
60
40
20
0
p=0.053
Fc
CTLA-4 Fc (abatacept)
ICOSL IgV -Fc
(N52H/ N57Y/Q100R)
IL-1β
2 hr post-Dose 1
Terminal
IL-1β (pg/mL)
40
30
20
10
0
***
**
IL-6
2 hr post-Dose 1 Terminal
IL-6 (pg/mL)
1000
750
500
250
0
IL-10
2 hr post-Dose 1
Terminal
IL-10 (pg/mL)
250
200
150
100
50
0
***
**

FIG. 12D

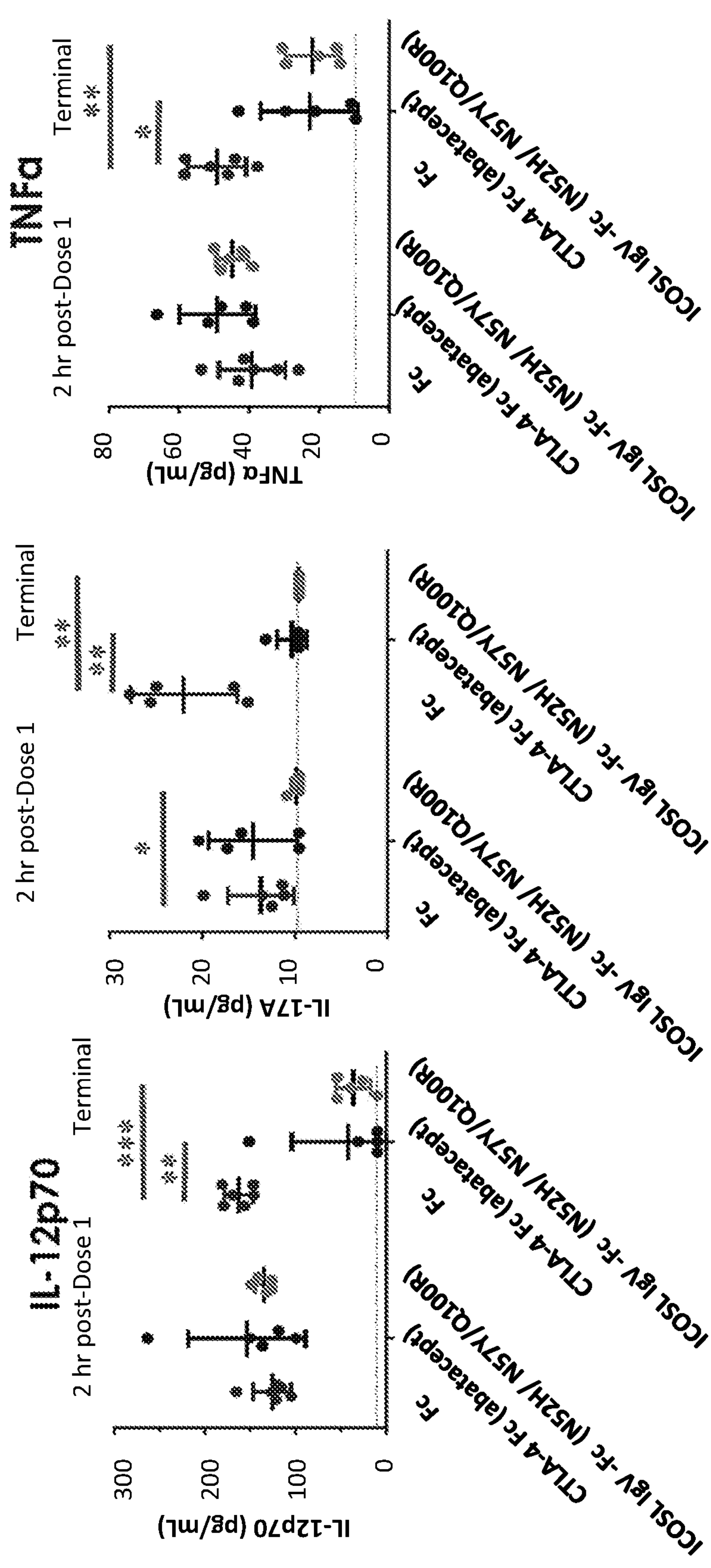
IL-12p70
2 hr post-Dose 1
Terminal
IL-12p70 (pg/mL)
300
200
100
0
***
**
IL-17A
IL-17A (pg/mL)
30
20
10
0
*
**
**
TNFα
TNFα (pg/mL)
80
60
40
20
0
*
**
Fc
CTLA-4 Fc (abatacept)
ICOSL IgV -Fc (N52H/ N57Y/Q100R)

FIG. 13A

FIG. 13B

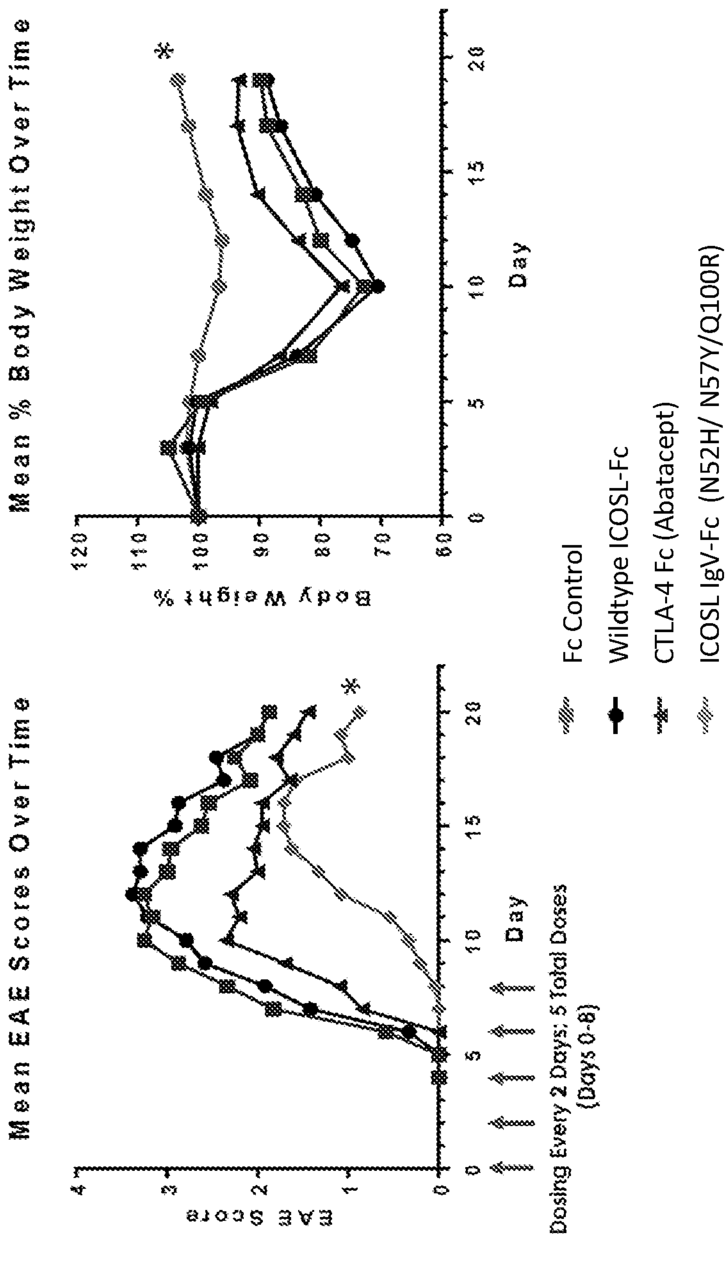

FIG. 13C

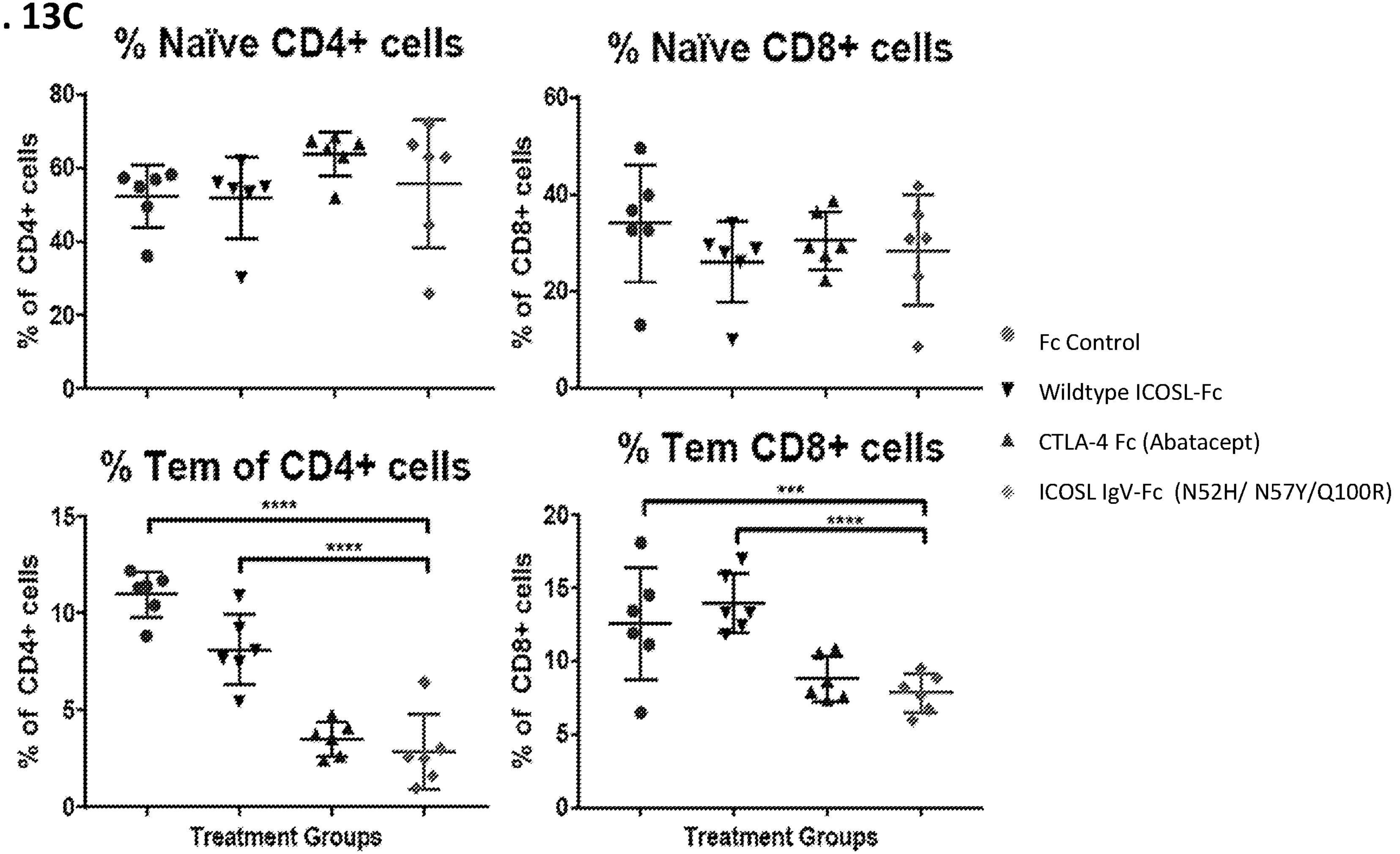
% Naïve CD4+ cells
% of CD4+ cells
80
60
40
20
0
% Naïve CD8+ cells
% of CD8+ cells
60
40
20
0
% Tem of CD4+ cells
% of CD4+ cells
15
10
5
0
****
****
Treatment Groups
% Tem CD8+ cells
% of CD8+ cells
20
15
10
5
0
***
****
Treatment Groups
Fc Control
Wildtype ICOSL-Fc
CTLA-4 Fc (Abatacept)
ICOSL IgV-Fc (N52H/ N57Y/Q100R)

FIG. 13D

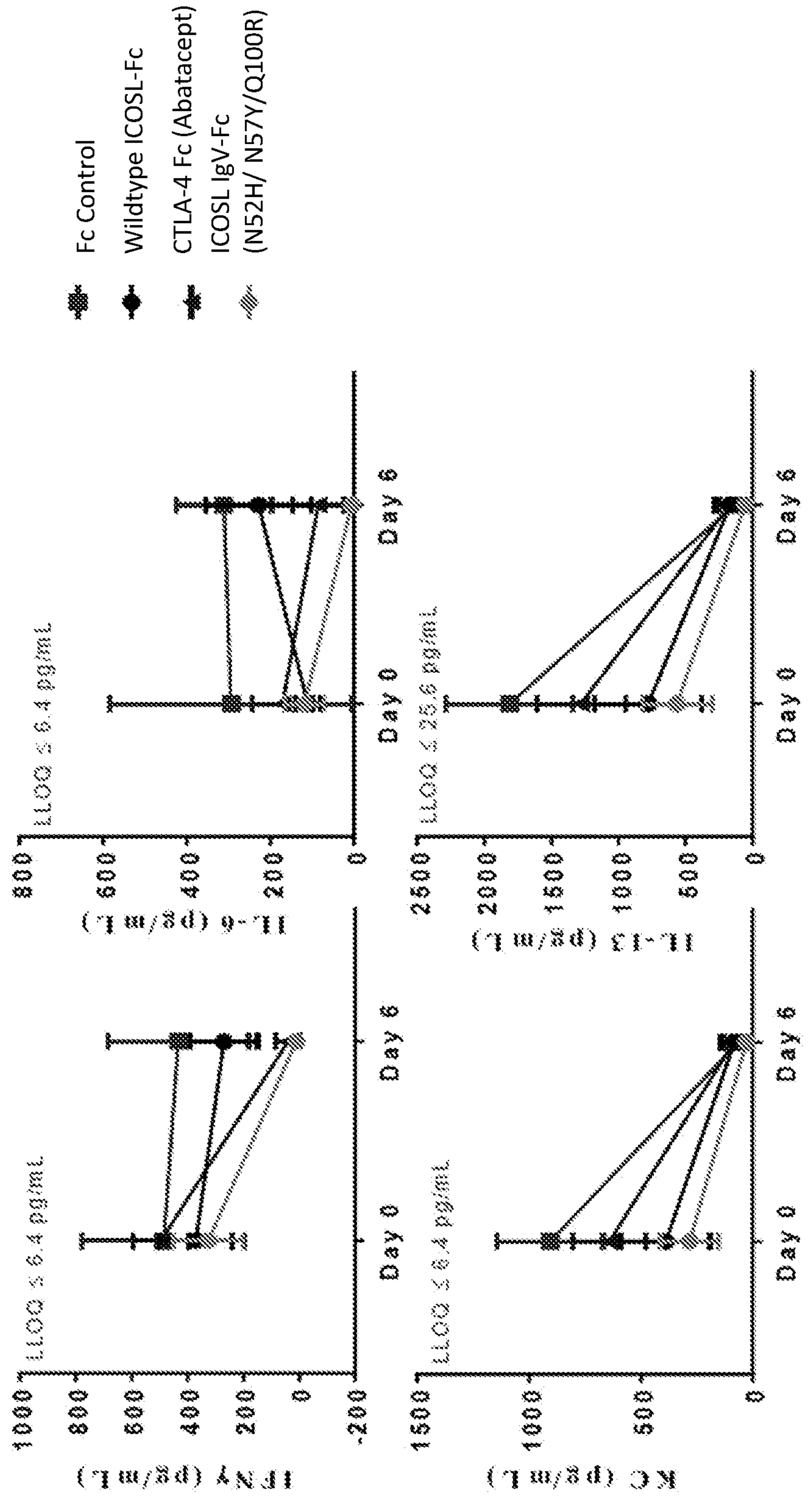
Fc Control
Wildtype ICOSL-Fc
CTLA-4 Fc (Abatacept)
ICOSL IgV-Fc
(N52H/ N57Y/Q100R)
IFNγ (pg/mL)
LLOQ ≤ 6.4 pg/mL
IL-6 (pg/mL)
LLOQ ≤ 6.4 pg/mL
KC (pg/mL)
LLOQ ≤ 6.4 pg/mL
IL-13 (pg/mL)
LLOQ ≤ 25.6 pg/mL
Day 0
Day 6

FIG. 14A

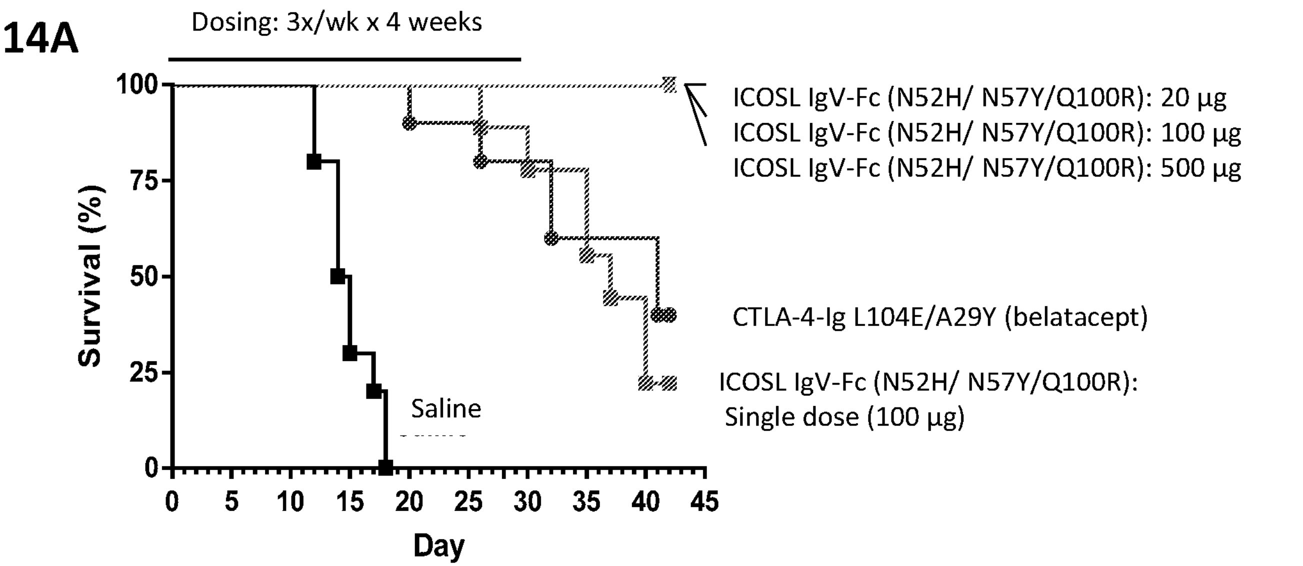


FIG. 14B

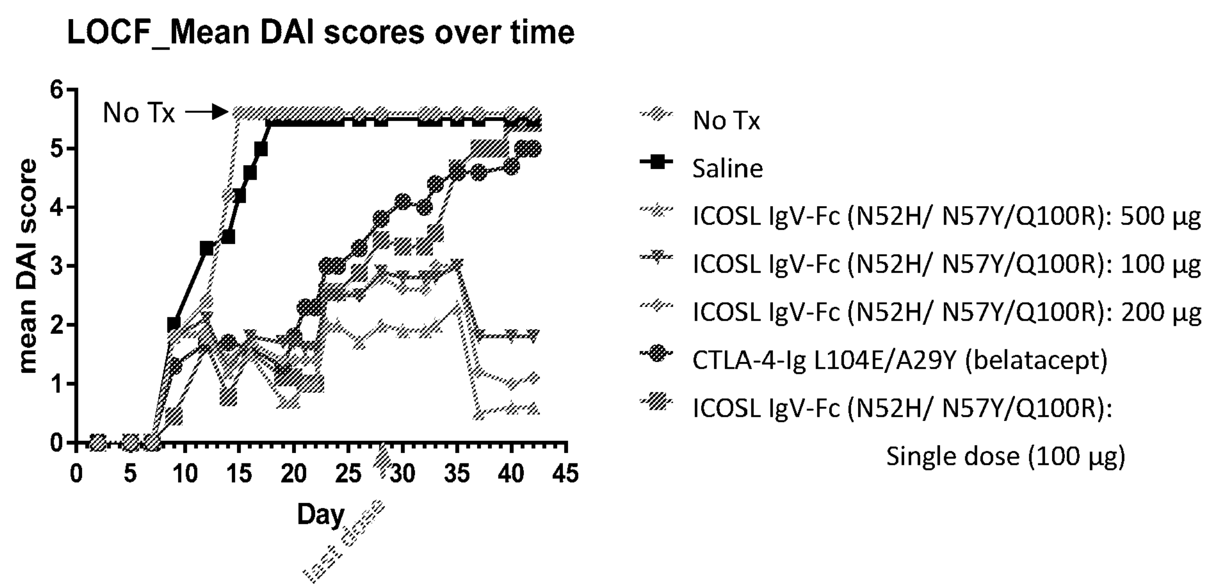

FIG. 15A

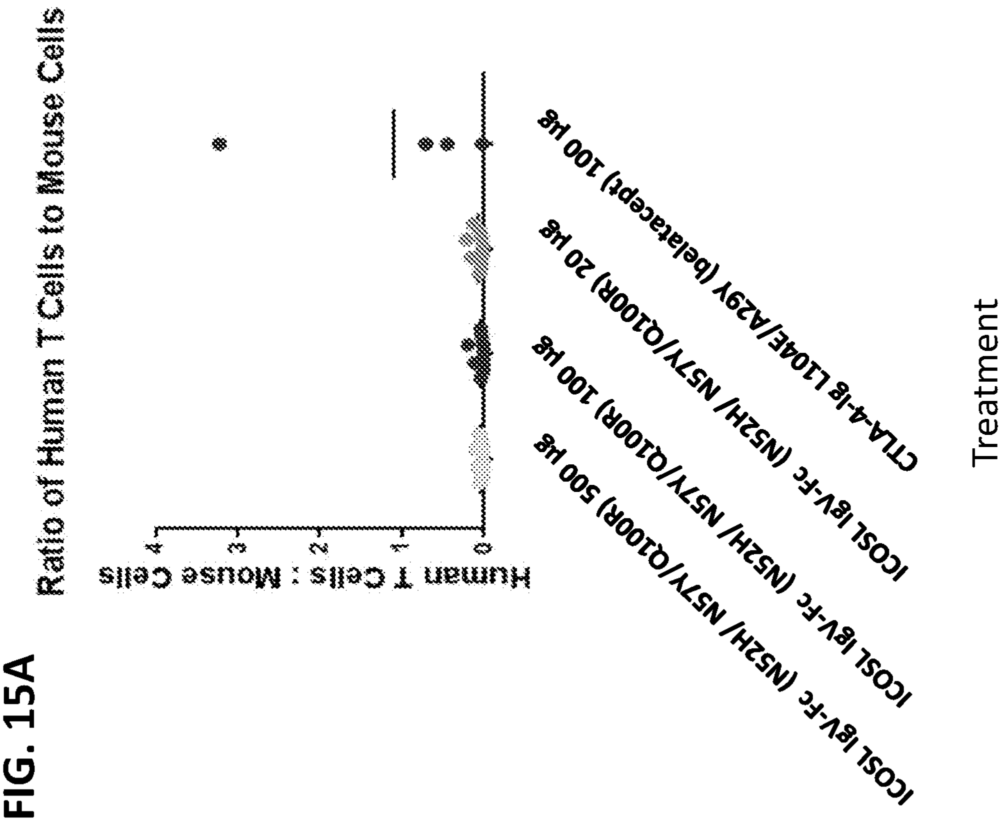


FIG. 15B

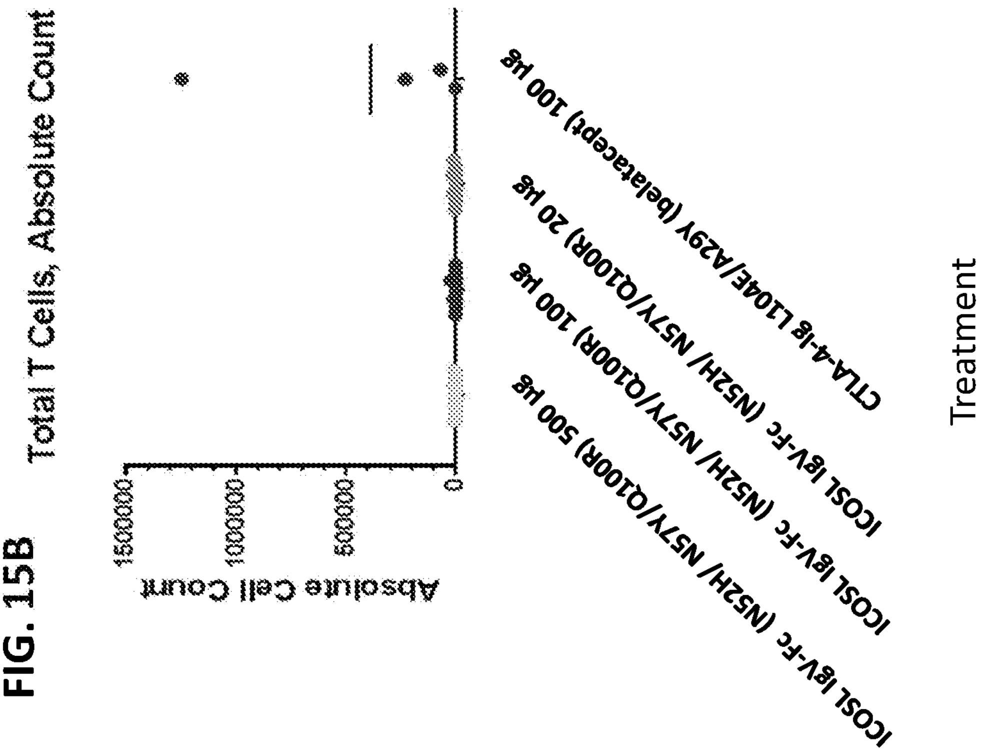

FIG. 15D

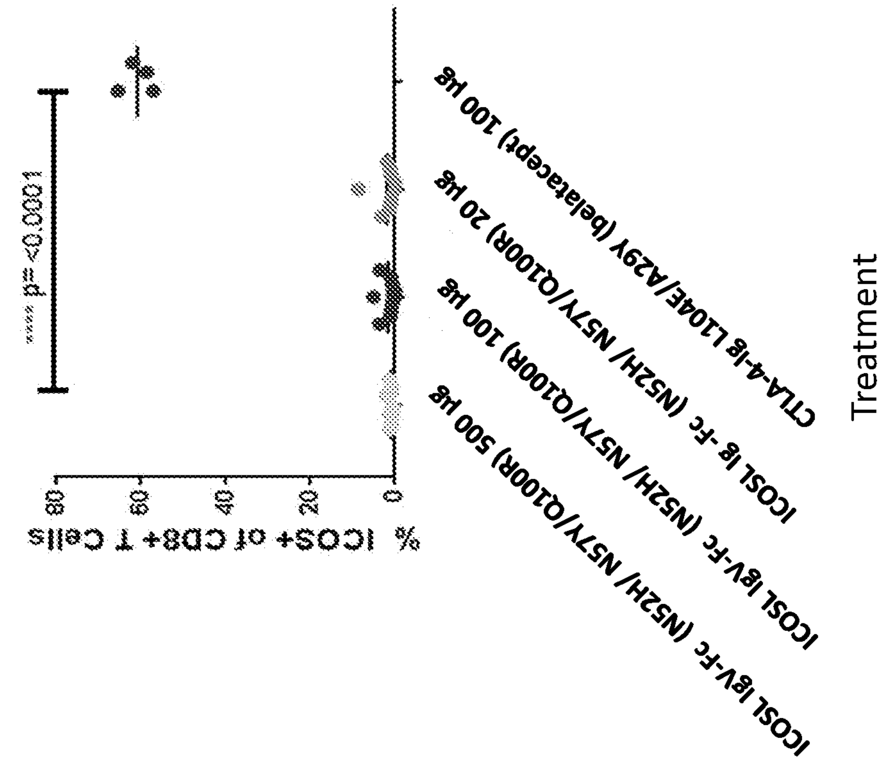


FIG. 15C

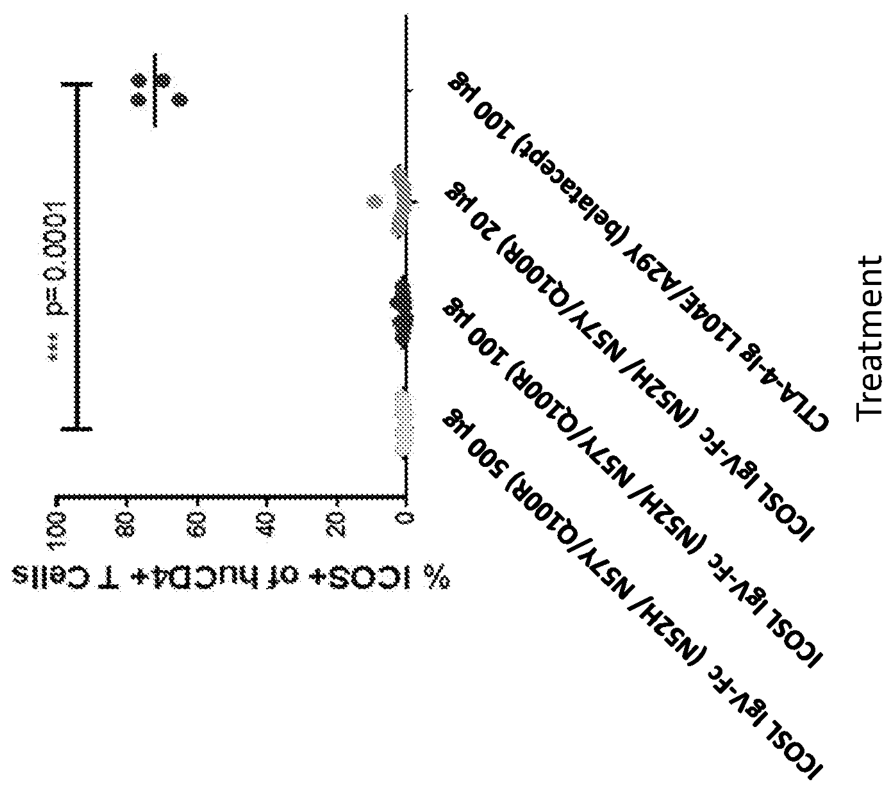

FIG. 15E

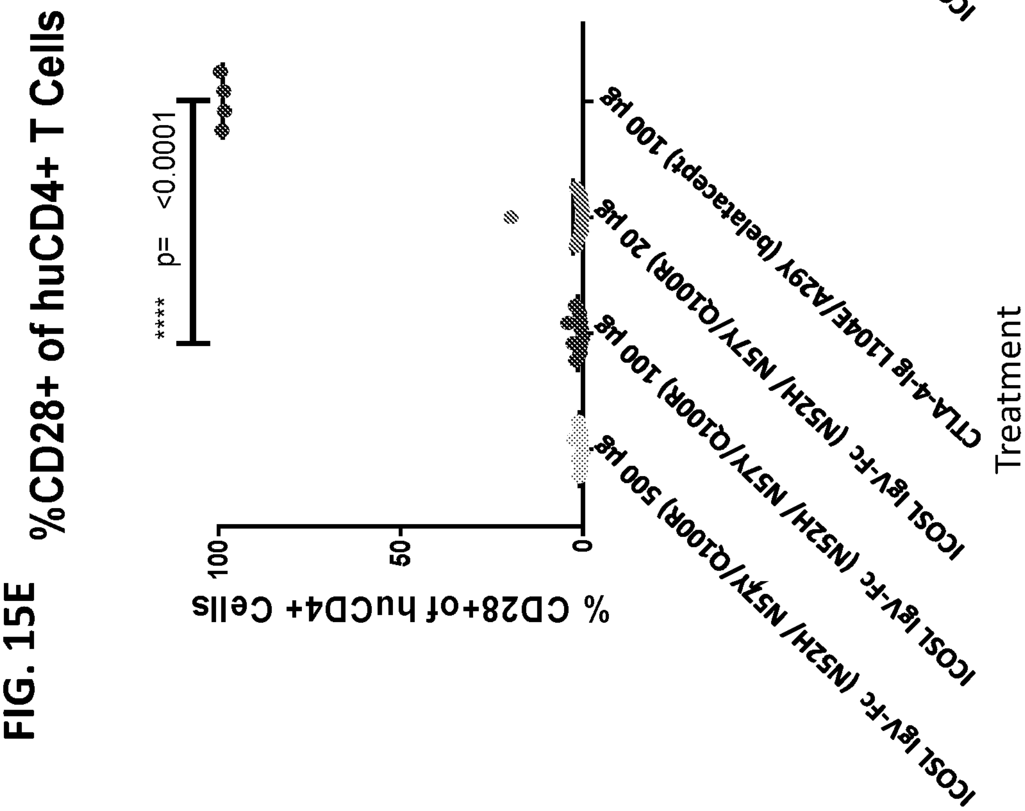


FIG. 15F

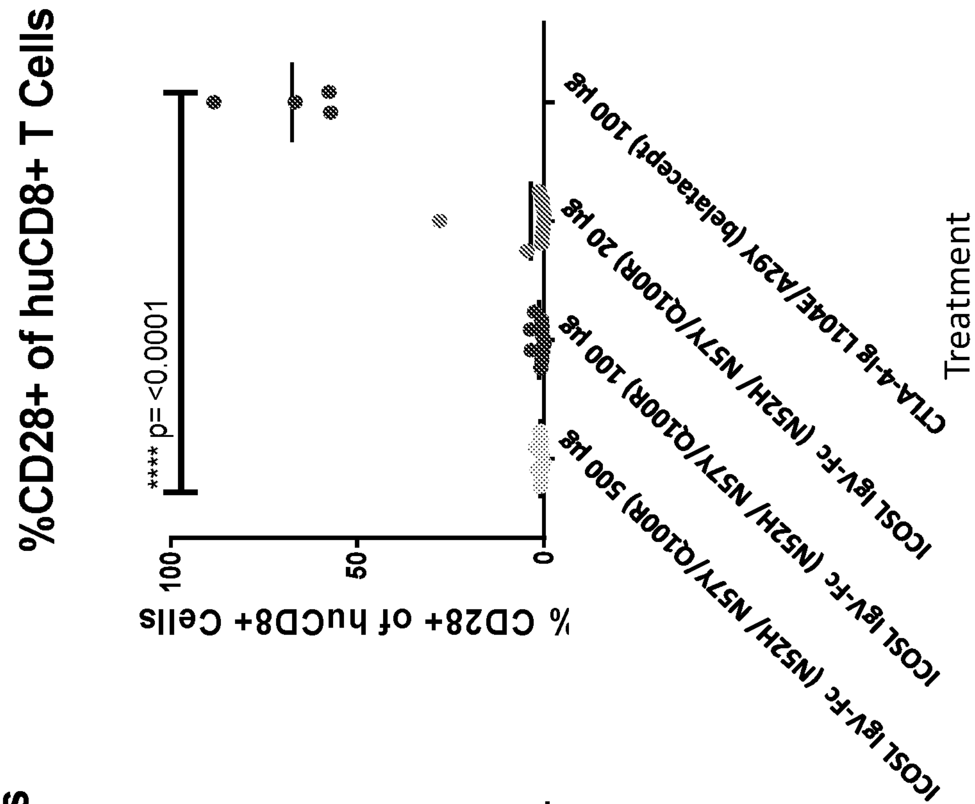

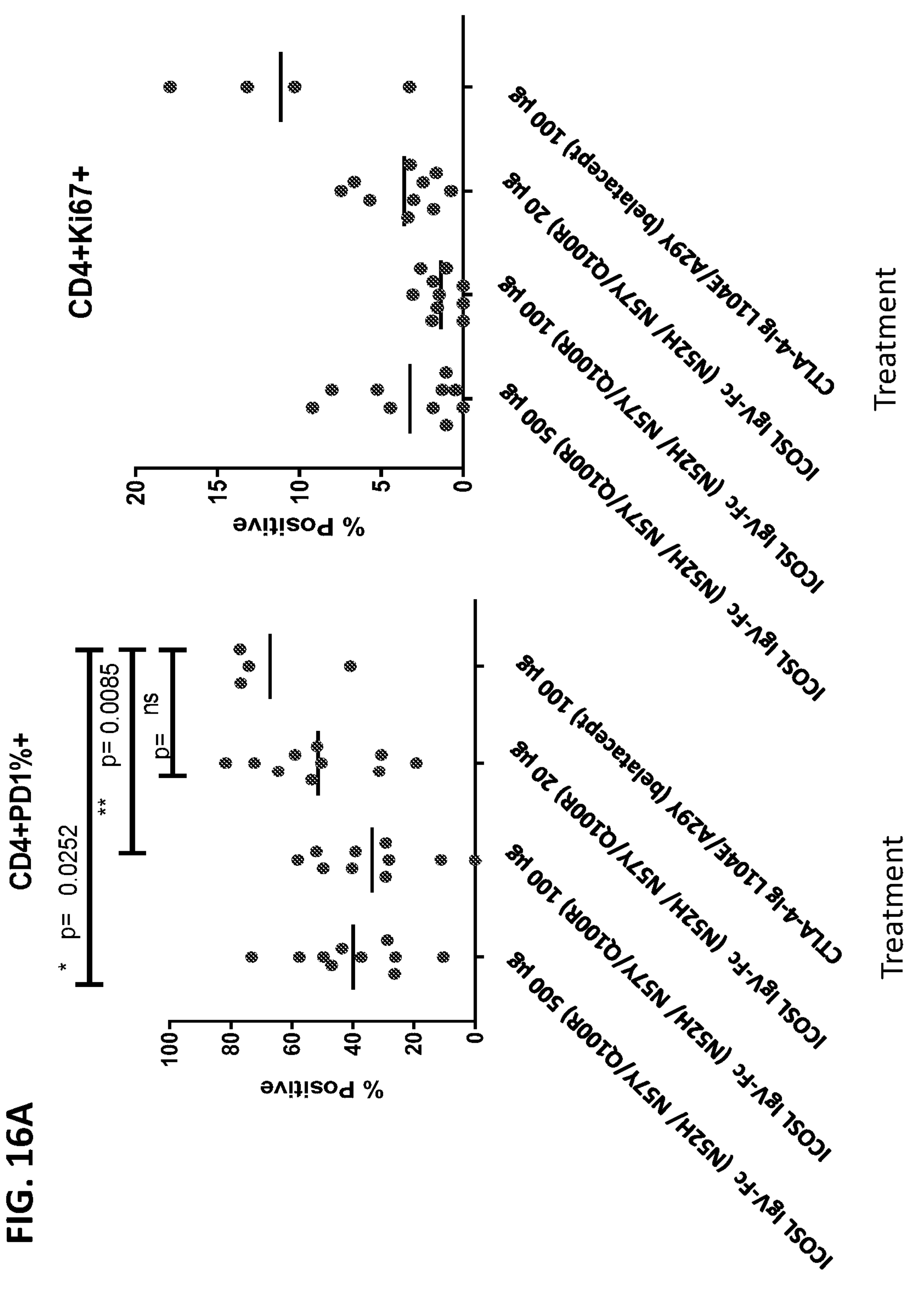
CD4+PD1%+
% Positive
100
80
60
40
20
0
* p= 0.0252
** p= 0.0085
p= ns
CD4+Ki67+
% Positive
20
15
10
5
0
ICOSL IgV-Fc (N52H/ N57Y/Q100R) 500 µg
ICOSL IgV-Fc (N52H/ N57Y/Q100R) 100 µg
ICOSL IgV-Fc (N52H/ N57Y/Q100R) 20 µg
CTLA-4-Ig L104E/A29Y (belatacept) 100 µg
Treatment

FIG. 16A

FIG. 16B

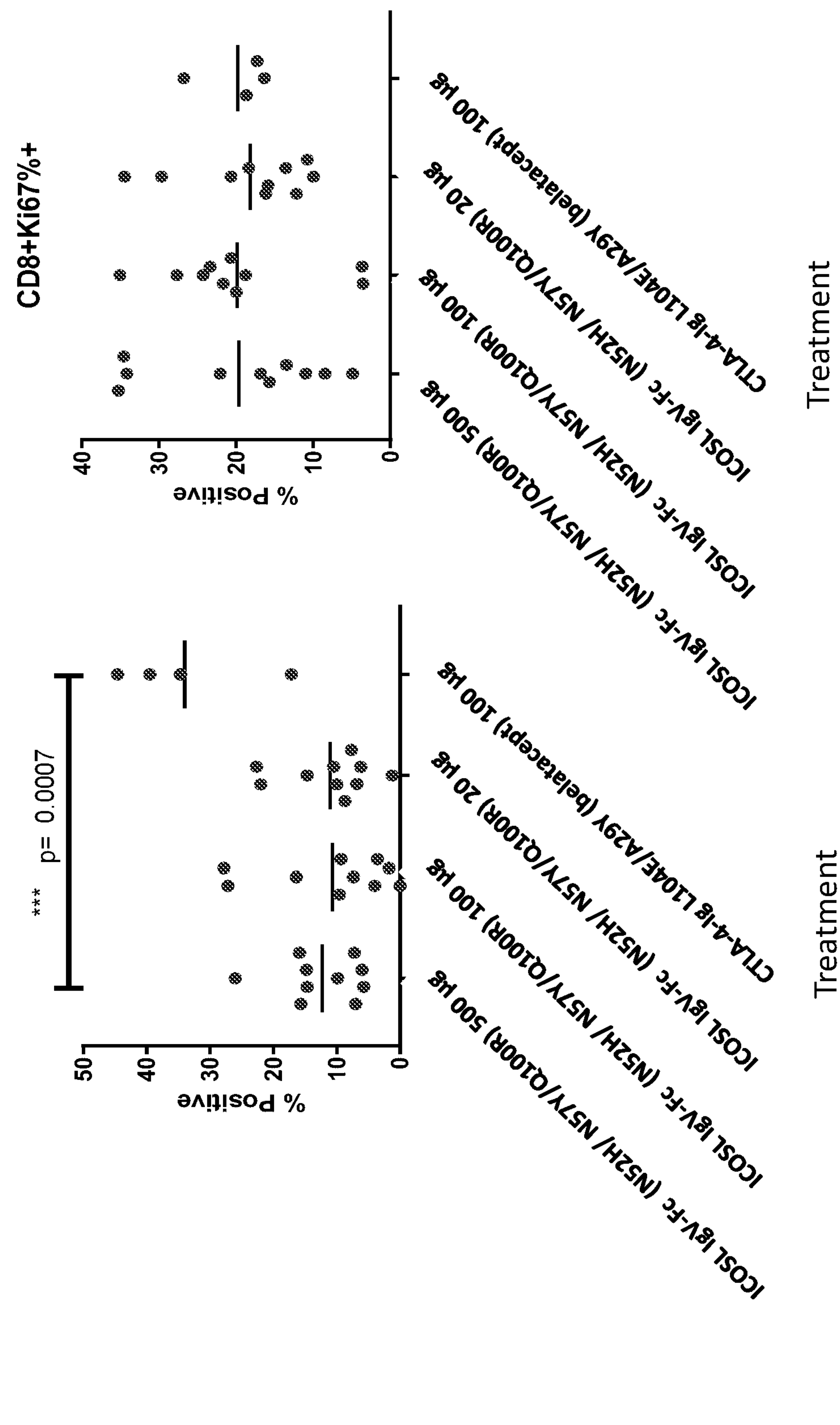
CD8+PD1%+
*** p= 0.0007
% Positive
50
40
30
20
10
0
ICOSL IgV-Fc (N52H/ N57Y/Q100R) 500 µg
ICOSL IgV-Fc (N52H/ N57Y/Q100R) 100 µg
ICOSL IgV-Fc (N52H/ N57Y/Q100R) 20 µg
CTLA-4-Ig L104E/A29Y (belatacept) 100 µg
Treatment
CD8+Ki67%+
% Positive
40
30
20
10
0
ICOSL IgV-Fc (N52H/ N57Y/Q100R) 500 µg
ICOSL IgV-Fc (N52H/ N57Y/Q100R) 100 µg
ICOSL IgV-Fc (N52H/ N57Y/Q100R) 20 µg
CTLA-4-Ig L104E/A29Y (belatacept) 100 µg
Treatment

FIG. 16C

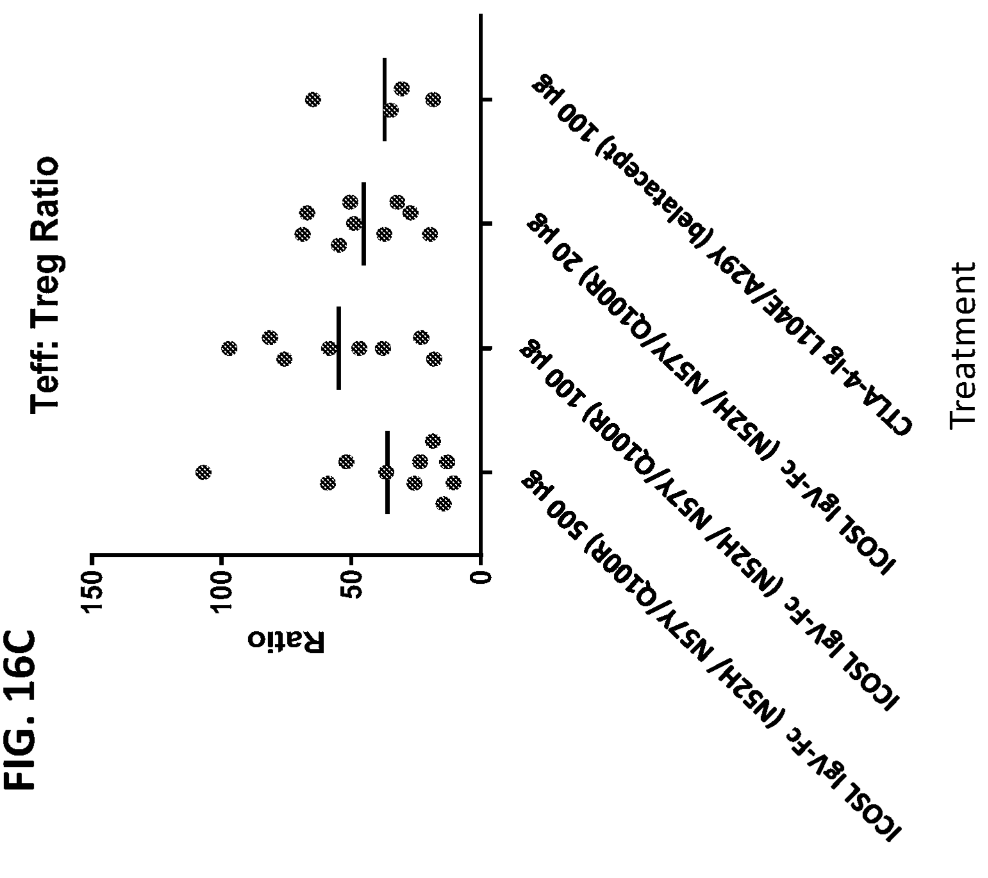
Teff: Treg Ratio
Ratio
150
100
50
0
ICOSL IgV-Fc (N52H/ N57Y/Q100R) 500 µg
ICOSL IgV-Fc (N52H/ N57Y/Q100R) 100 µg
ICOSL IgV-Fc (N52H/ N57Y/Q100R) 20 µg
CTLA-4-Ig L104E/A29Y (belatacept) 100 µg
Treatment

FIG. 17A

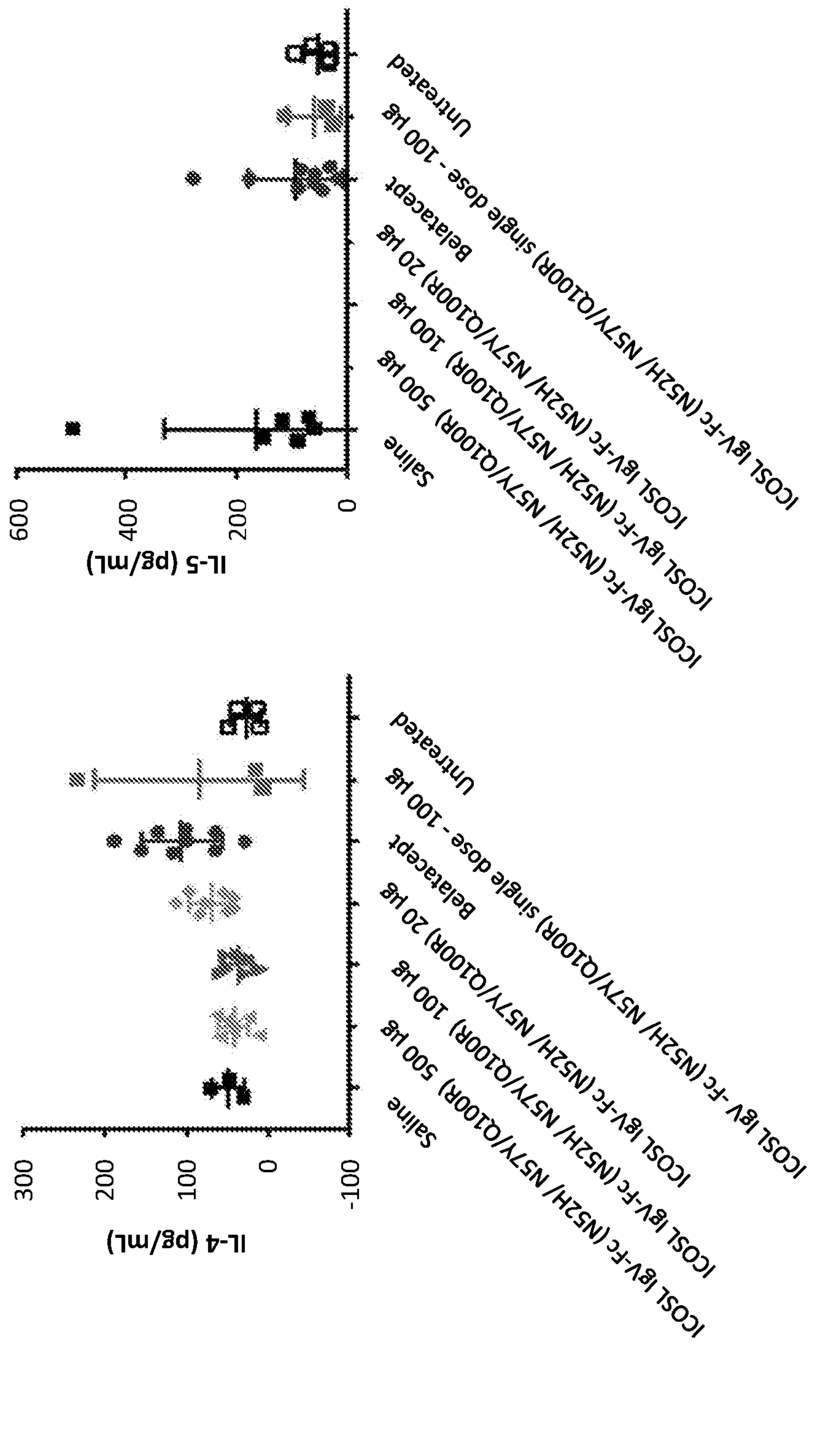
IL-4 (pg/mL)
300
200
100
0
-100
IL-5 (pg/mL)
600
400
200
0
Saline
ICOSL IgV-Fc (N52H/ N57Y/Q100R) 500 µg
ICOSL IgV-Fc (N52H/ N57Y/Q100R) 100 µg
ICOSL IgV-Fc (N52H/ N57Y/Q100R) 20 µg
Belatacept
ICOSL IgV -Fc (N52H/ N57Y/Q100R) single dose - 100 µg
Untreated

FIG. 17B

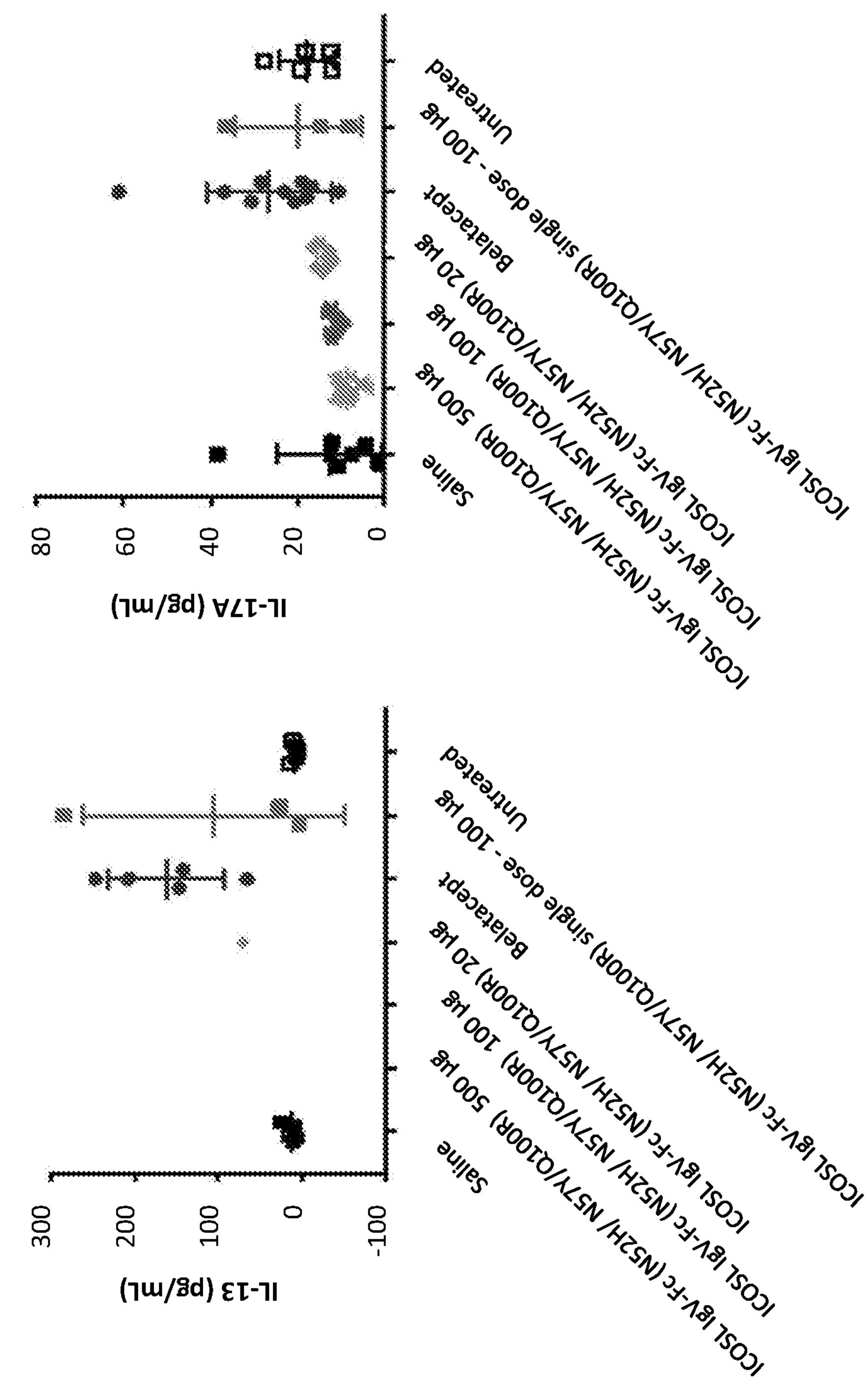
IL-13 (pg/mL)
300
200
100
0
-100
IL-17A (pg/mL)
80
60
40
20
0
Saline
ICOSL IgV-Fc (N52H/ N57Y/Q100R) 500 µg
ICOSL IgV-Fc (N52H/ N57Y/Q100R) 100 µg
ICOSL IgV-Fc (N52H/ N57Y/Q100R) 20 µg
Belatacept
ICOSL IgV-Fc (N52H/ N57Y/Q100R) single dose - 100 µg
Untreated

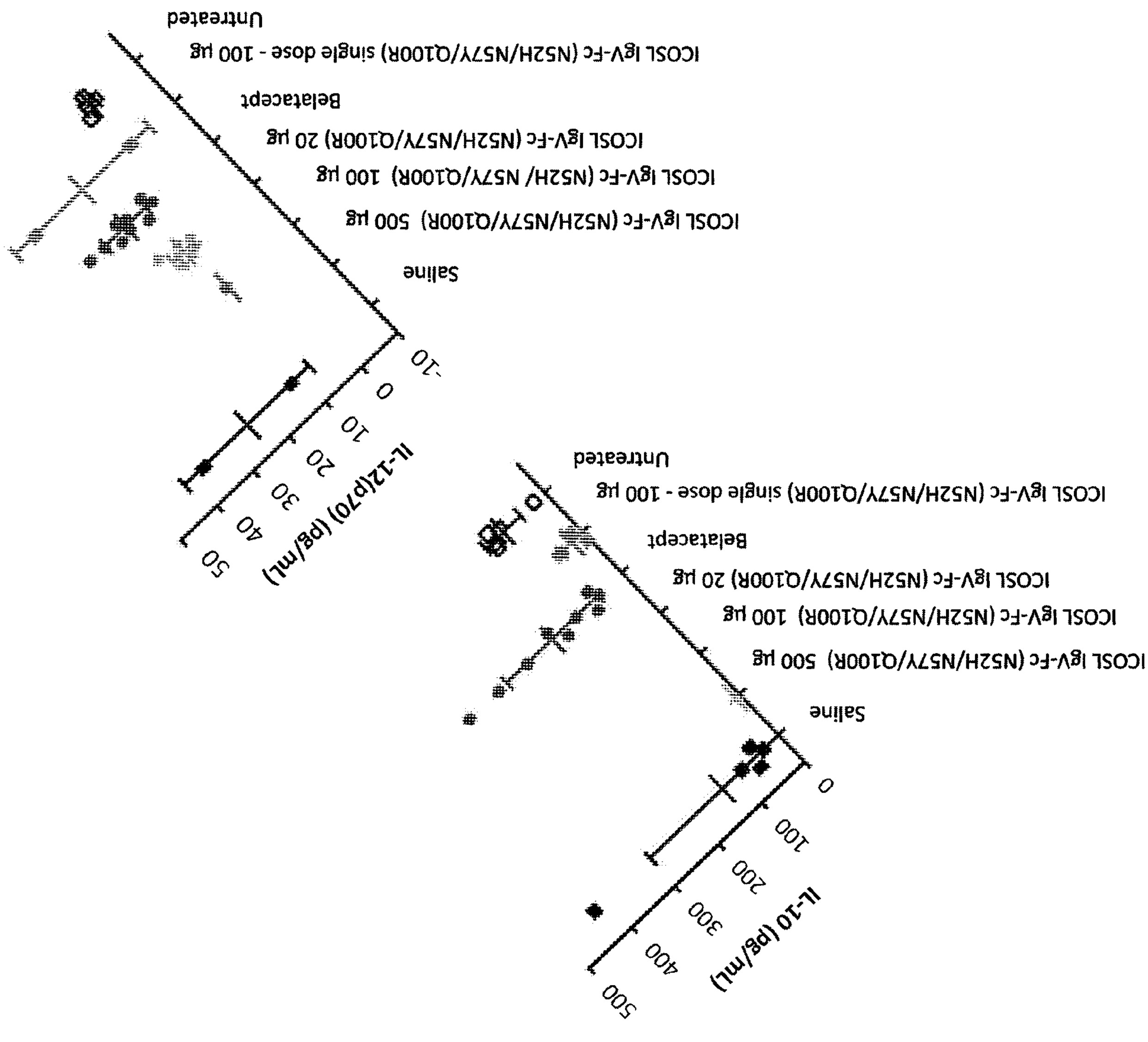
Untreated
ICOSL IgV-Fc (N52H/N57Y/Q100R) single dose - 100 µg
Belatacept
ICOSL IgV-Fc (N52H/N57Y/Q100R) 20 µg
ICOSL IgV-Fc (N52H/ N57Y/Q100R) 100 µg
ICOSL IgV-Fc (N52H/N57Y/Q100R) 500 µg
Saline
-10
0
10
20
30
40
50
IL-12(p70) (pg/mL)
Untreated
ICOSL IgV-Fc (N52H/N57Y/Q100R) single dose - 100 µg
Belatacept
ICOSL IgV-Fc (N52H/N57Y/Q100R) 20 µg
ICOSL IgV-Fc (N52H/N57Y/Q100R) 100 µg
ICOSL IgV-Fc (N52H/N57Y/Q100R) 500 µg
Saline
0
100
200
300
400
500
IL-10 (pg/mL)

FIG. 17C

FIG. 17D

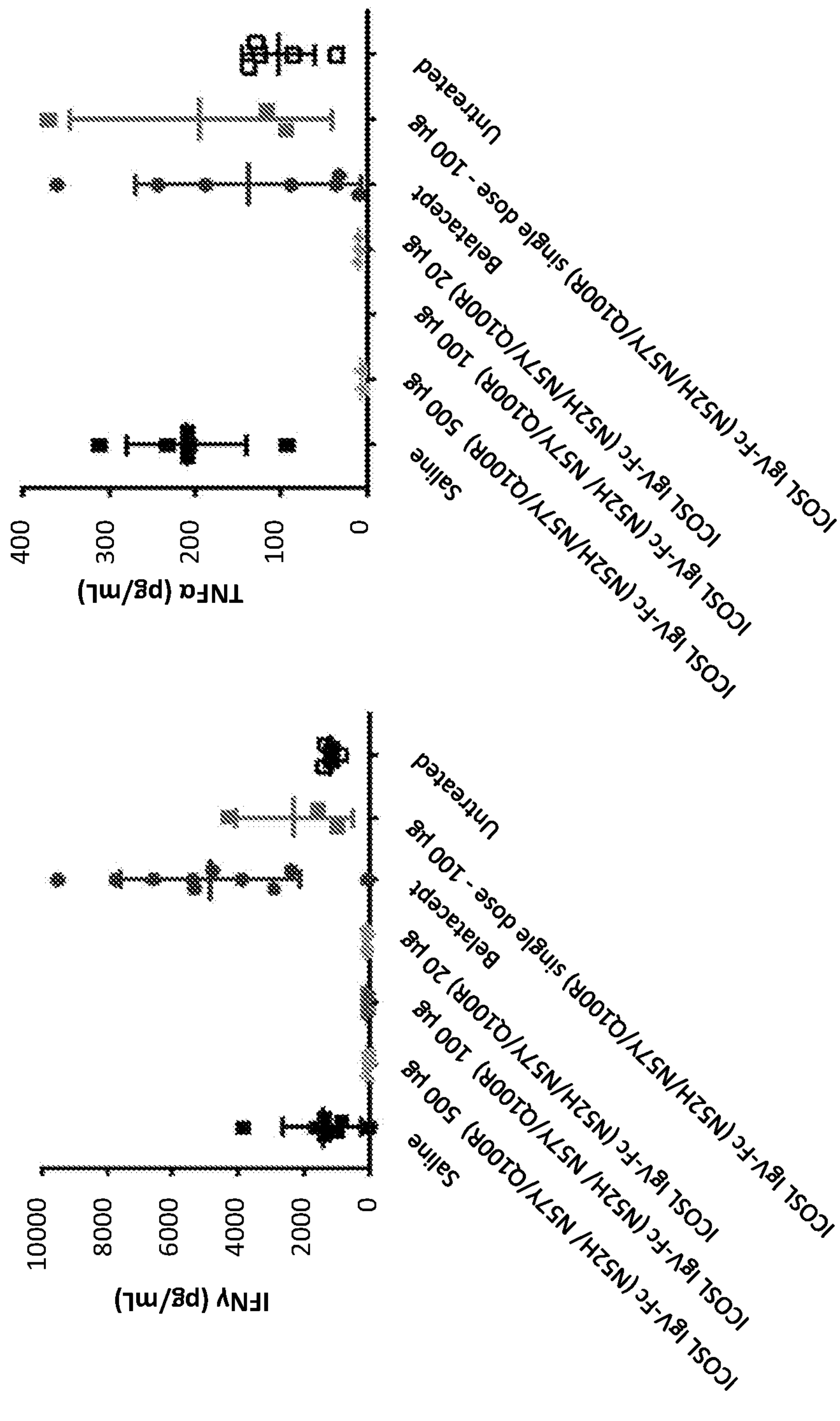
IFNγ (pg/mL)
10000
8000
6000
4000
2000
0
TNFα (pg/mL)
400
300
200
100
0
Saline
ICOSL IgV-Fc (N52H/N57Y/Q100R) 500 µg
ICOSL IgV-Fc (N52H/ N57Y/Q100R) 100 µg
ICOSL IgV-Fc (N52H/N57Y/Q100R) 20 µg
Belatacept
ICOSL IgV-Fc (N52H/N57Y/Q100R) single dose - 100 µg
Untreated

FIG. 17E

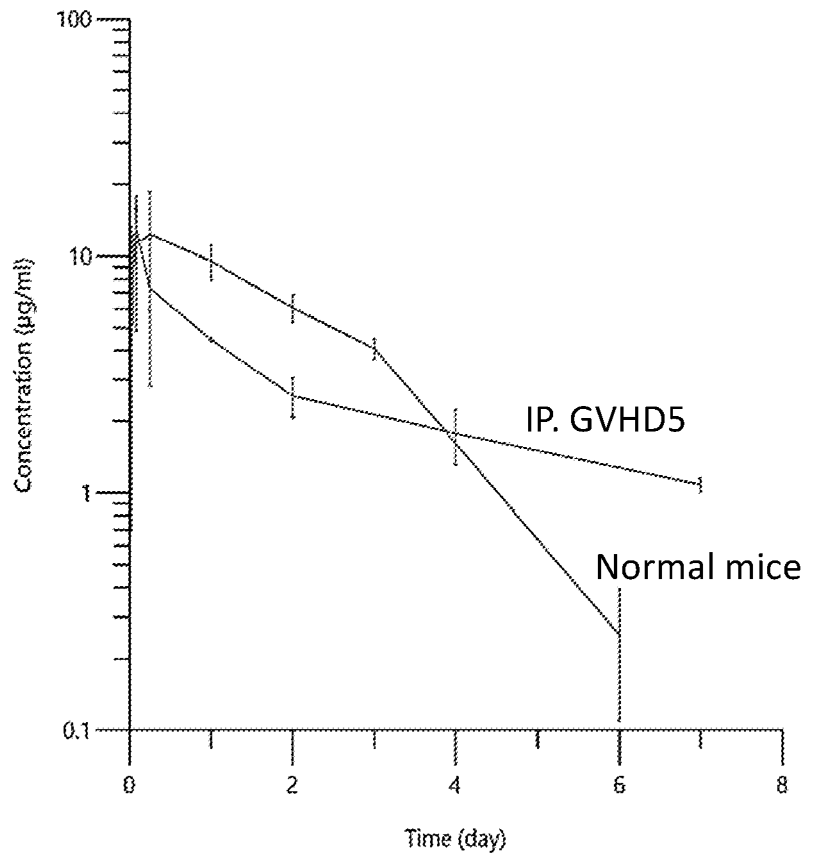


| Study | Dose (µg) | Cmax (µg/mL) | T1/2 (day) | AUC (day*µg/mL) |
|---|---|---|---|---|
| GVHD5 | 100 | 12.94 | 4.02 | 18.97 |
| Normal Mice | 100 | 12.30 | 0.84 | 30.04 |

FIG. 18A

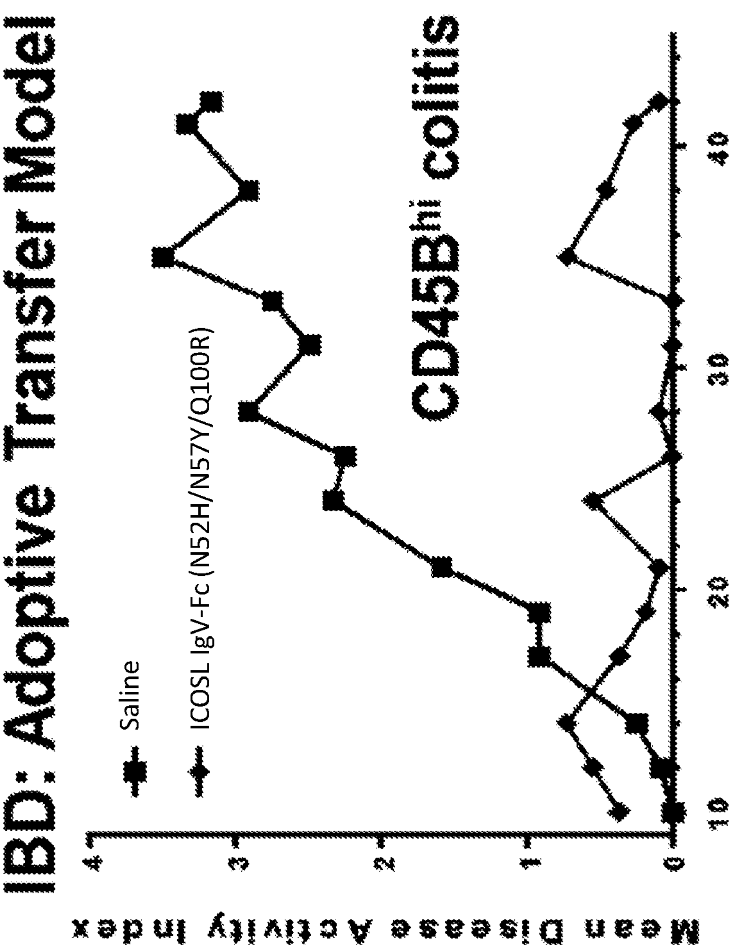
IBD: Adoptive Transfer Model
Saline
ICOSL IgV-Fc (N52H/N57Y/Q100R)
CD45B$^{hi}$ colitis
Mean Disease Activity Index
0
1
2
3
4
10
20
30
40
Day

FIG. 18B

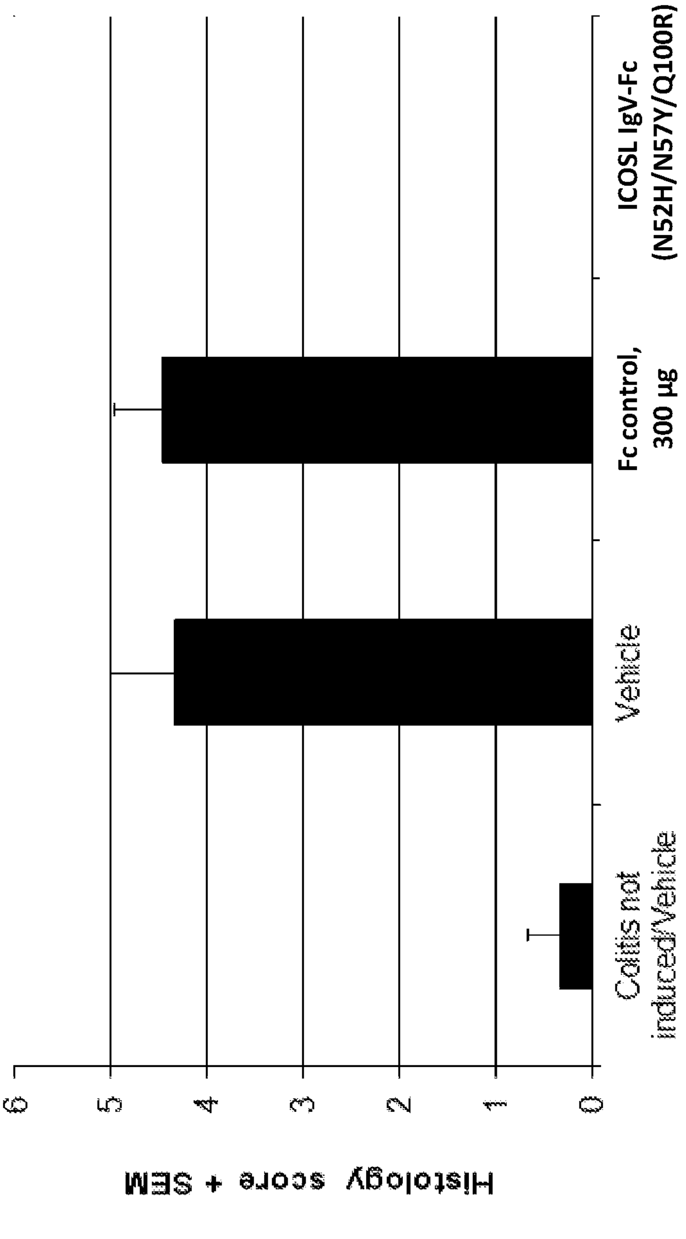
Histology score + SEM
0
1
2
3
4
5
6
Colitis not induced/Vehicle
Vehicle
Fc control, 300 µg
ICOSL IgV-Fc (N52H/N57Y/Q100R) 400 µg

FIG. 19A

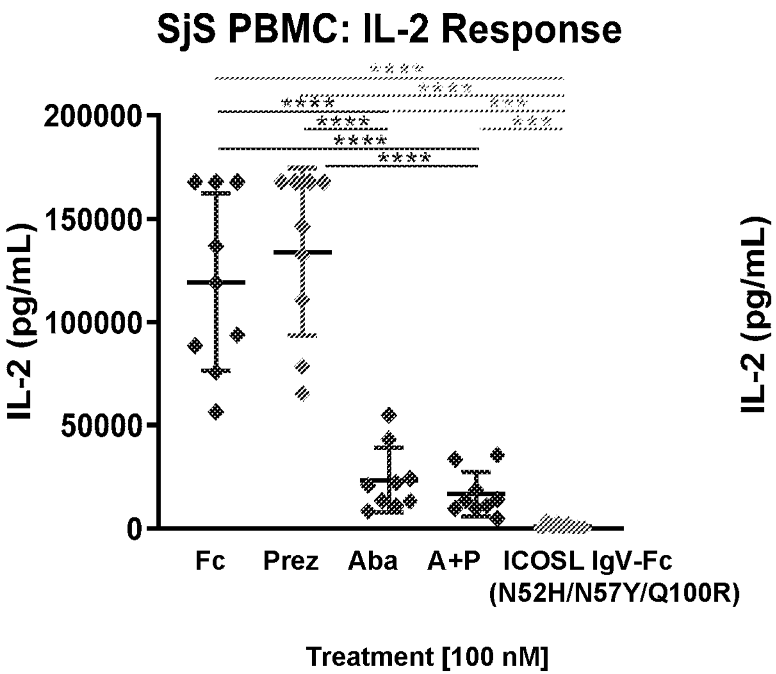


FIG. 19B

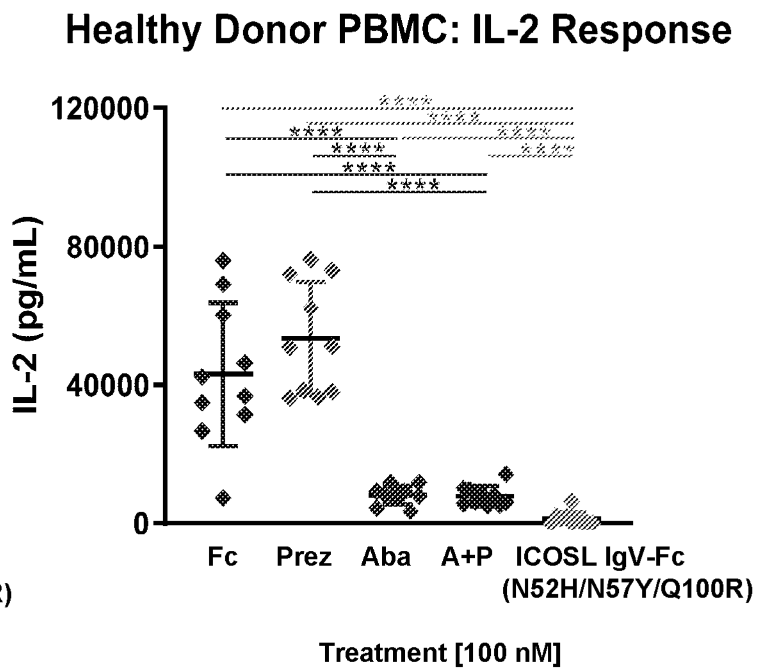

FIG. 19C

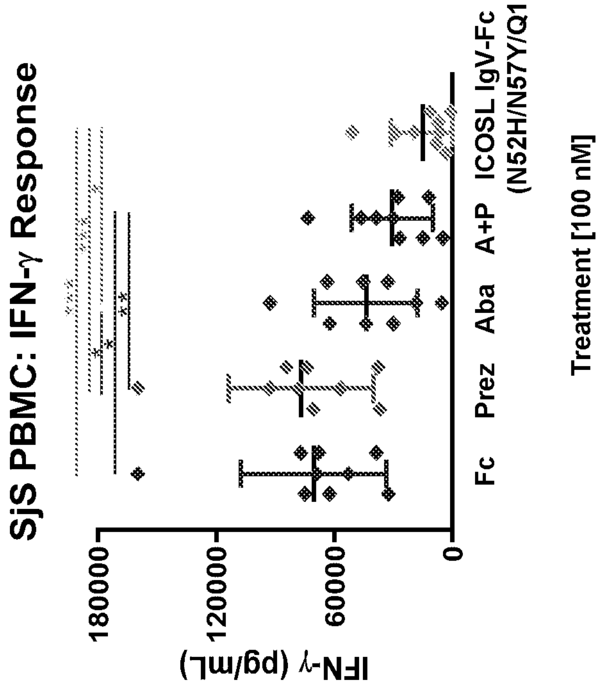


FIG. 19D

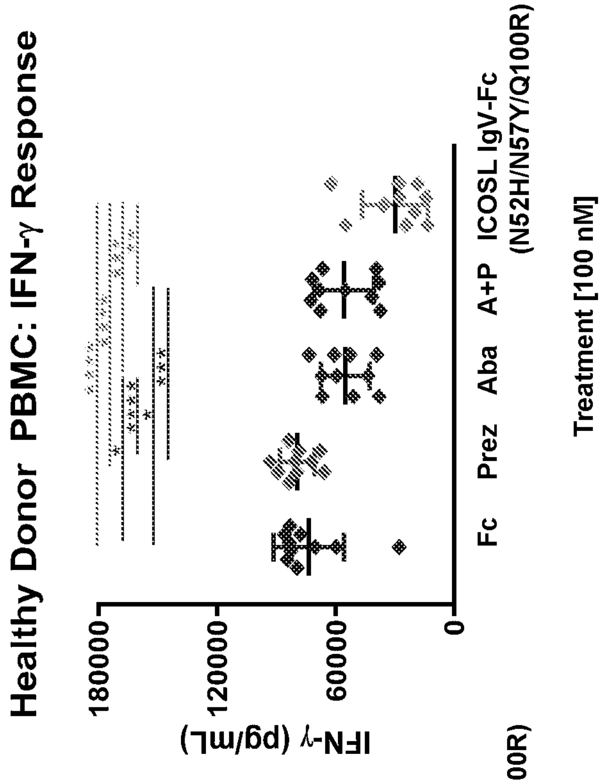

FIG. 19E

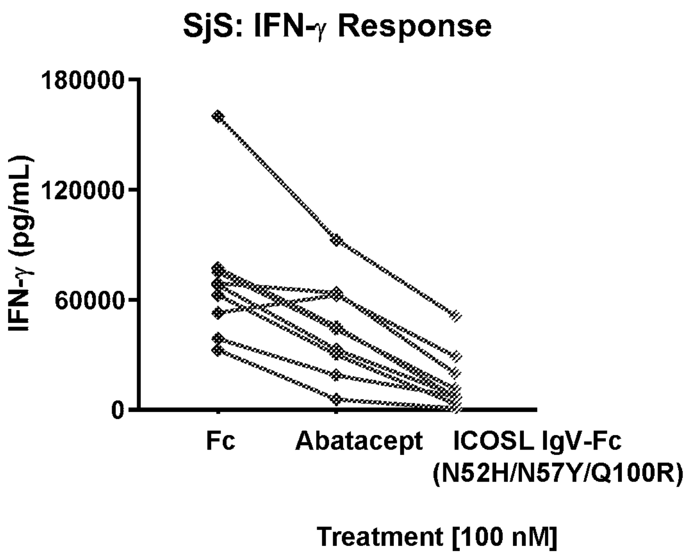


FIG. 19F

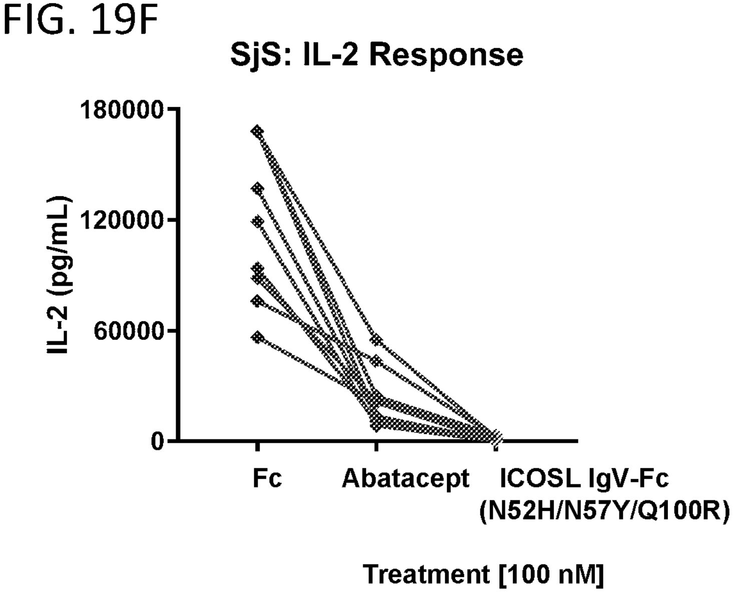

FIG. 19G

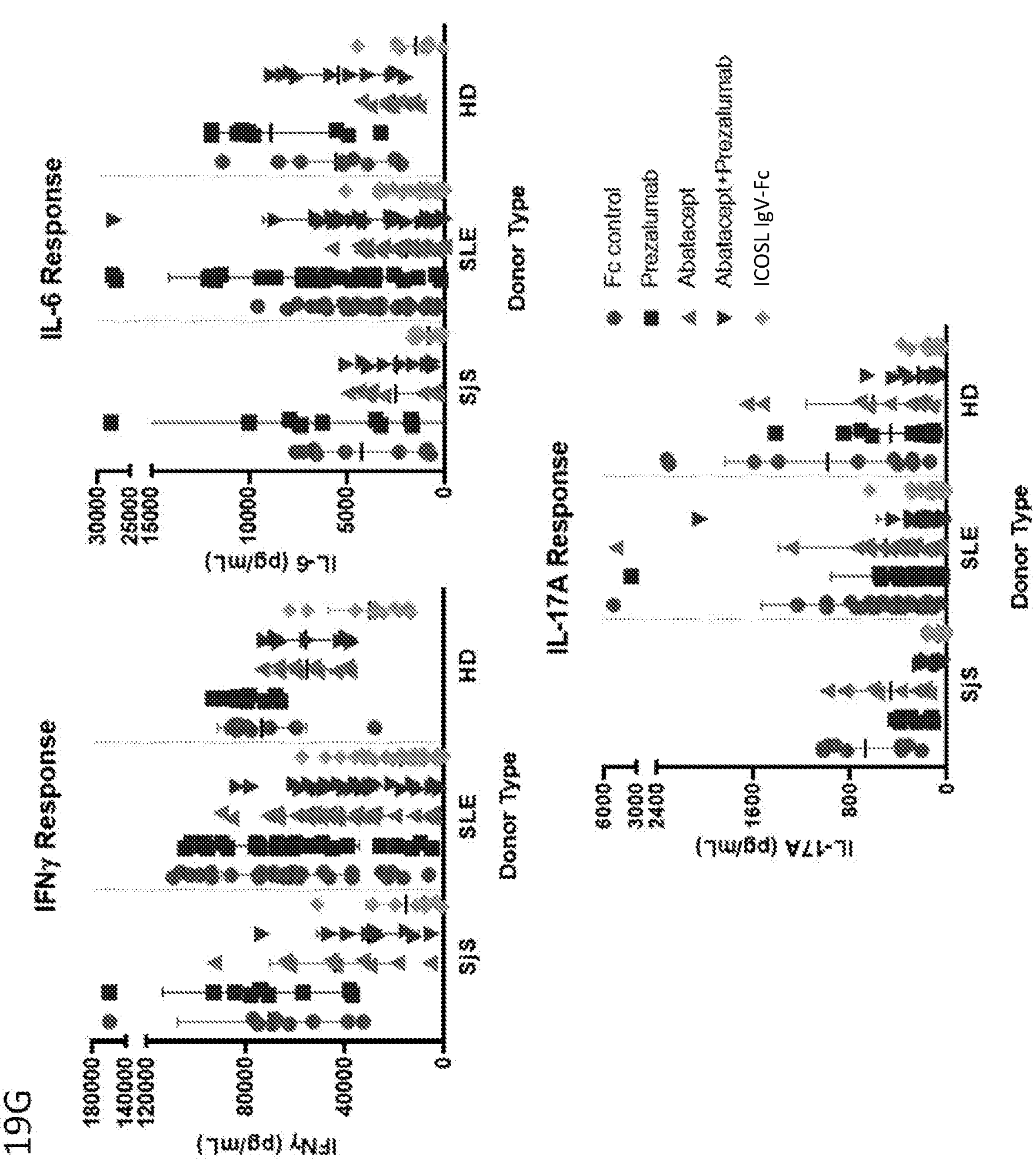
IFNγ Response
IFNγ (pg/mL)
180000
140000
120000
80000
40000
0
SjS
SLE
HD
Donor Type
IL-6 Response
IL-6 (pg/mL)
30000
25000
15000
10000
5000
0
SjS
SLE
HD
Donor Type
IL-17A Response
IL-17A (pg/mL)
6000
3000
2400
1600
800
0
SjS
SLE
HD
Donor Type
Fc control
Prezalumab
Abatacept
Abatacept+Prezalumab
ICOSL IgV-Fc

FIG. 19H

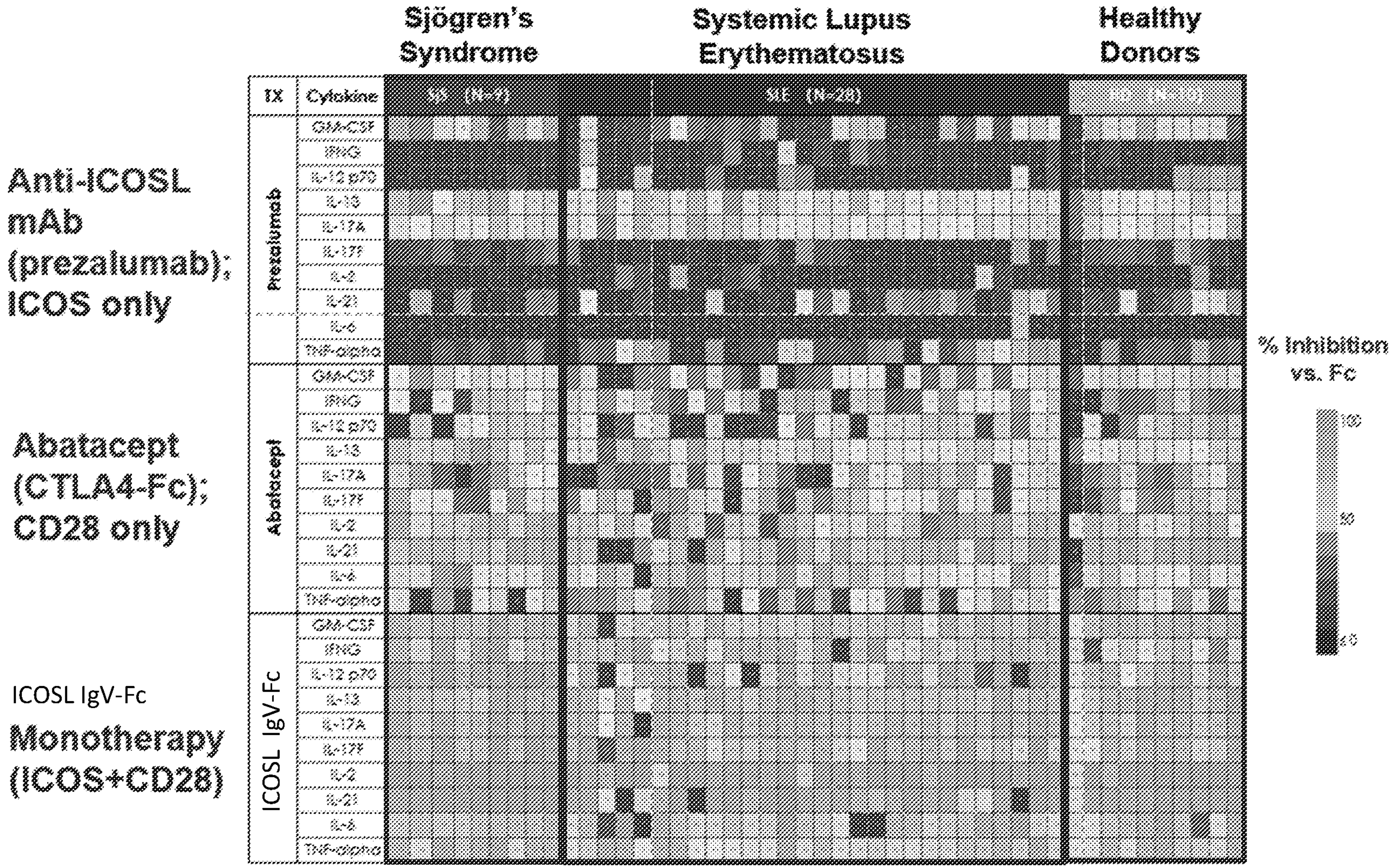
Sjögren's Syndrome
Systemic Lupus Erythematosus
Healthy Donors
TX
Cytokine
Anti-ICOSL mAb (prezalumab); ICOS only
Prezalumab
Abatacept (CTLA4-Fc); CD28 only
Abatacept
ICOSL IgV-Fc Monotherapy (ICOS+CD28)
ICOSL IgV-Fc
GM-CSF
IFNG
IL-12 p70
IL-13
IL-17A
IL-17F
IL-2
IL-21
IL-6
TNF-alpha
% Inhibition vs. Fc
100
50
0

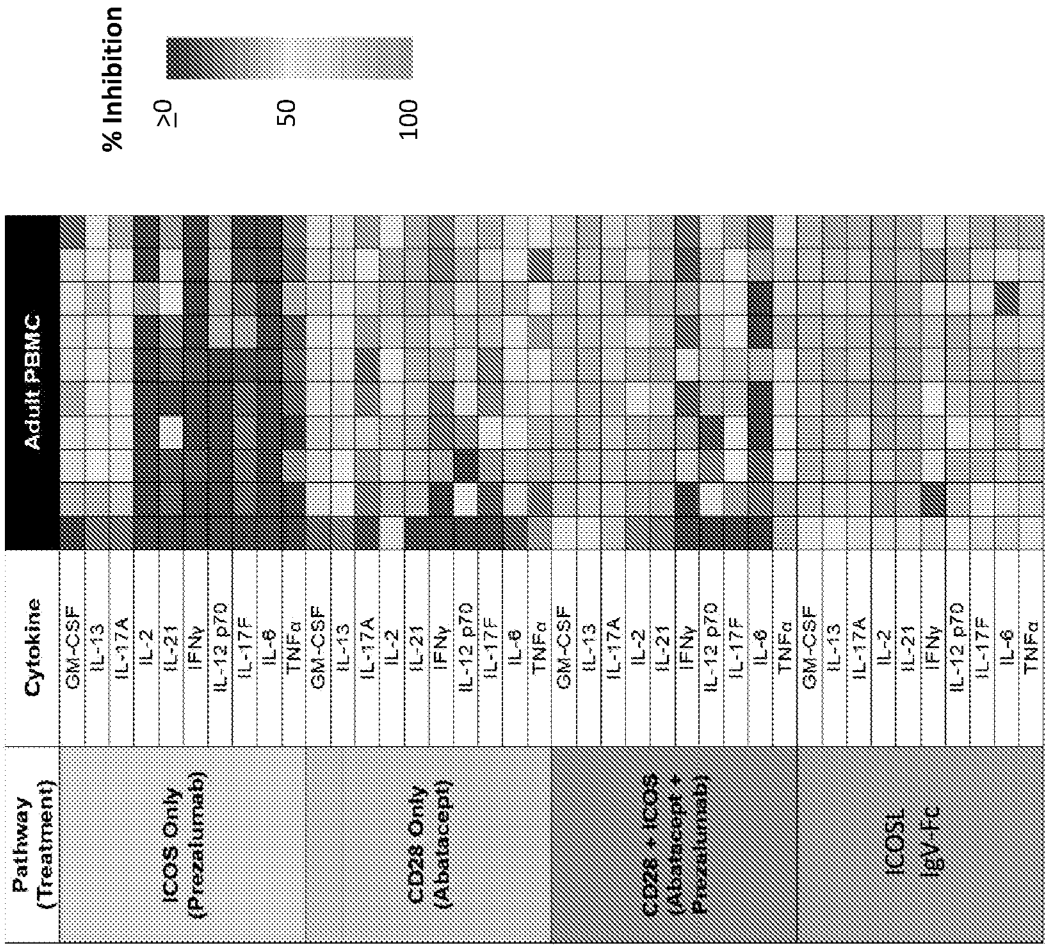
% Inhibition
≥0
50
100
Pathway (Treatment)
Cytokine
Adult PBMC
ICOS Only (Prezalumab)
CD28 Only (Abatacept)
CD28 + ICOS (Abatacept + Prezalumab)
ICOSL IgV-Fc
GM-CSF
IL-13
IL-17A
IL-2
IL-21
IFNγ
IL-12 p70
IL-17F
IL-6
TNFα
GM-CSF
IL-13
IL-17A
IL-2
IL-21
IFNγ
IL-12 p70
IL-17F
IL-6
TNFα
GM-CSF
IL-13
IL-17A
IL-2
IL-21
IFNγ
IL-12 p70
IL-17F
IL-6
TNFα
GM-CSF
IL-13
IL-17A
IL-2
IL-21
IFNγ
IL-12 p70
IL-17F
IL-6
TNFα

FIG. 19I

FIG. 19J

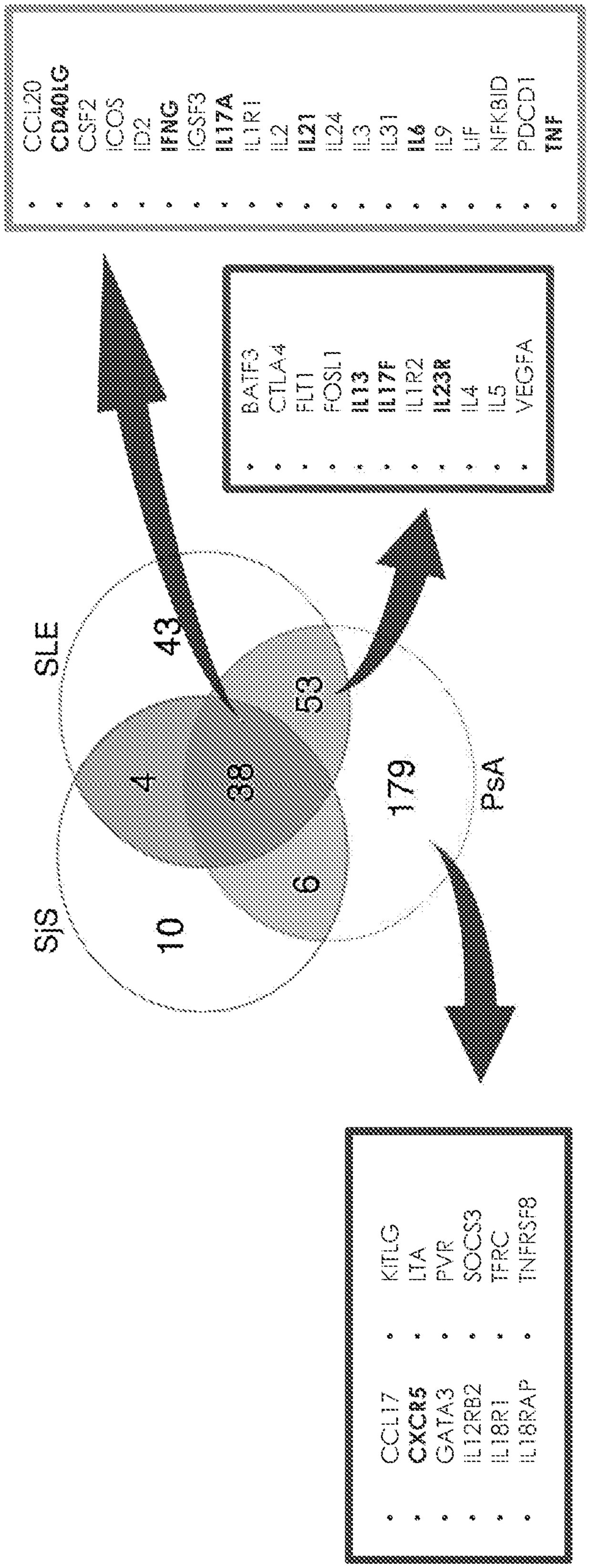
SjS
SLE
PsA
10
4
43
38
6
53
179
CCL20
CD40LG
CSF2
ICOS
ID2
IFNG
IGSF3
IL17A
IL1R1
IL2
IL21
IL24
IL3
IL31
IL6
IL9
LIF
NFKBID
PDCD1
TNF
BATF3
CTLA4
FLT1
FOSL1
IL13
IL17F
IL1R2
IL23R
IL4
IL5
VEGFA
CCL17
CXCR5
GATA3
IL12RB2
IL18R1
IL18RAP
KITLG
LTA
PVR
SOCS3
TFRC
TNFRSF8

FIG. 19K

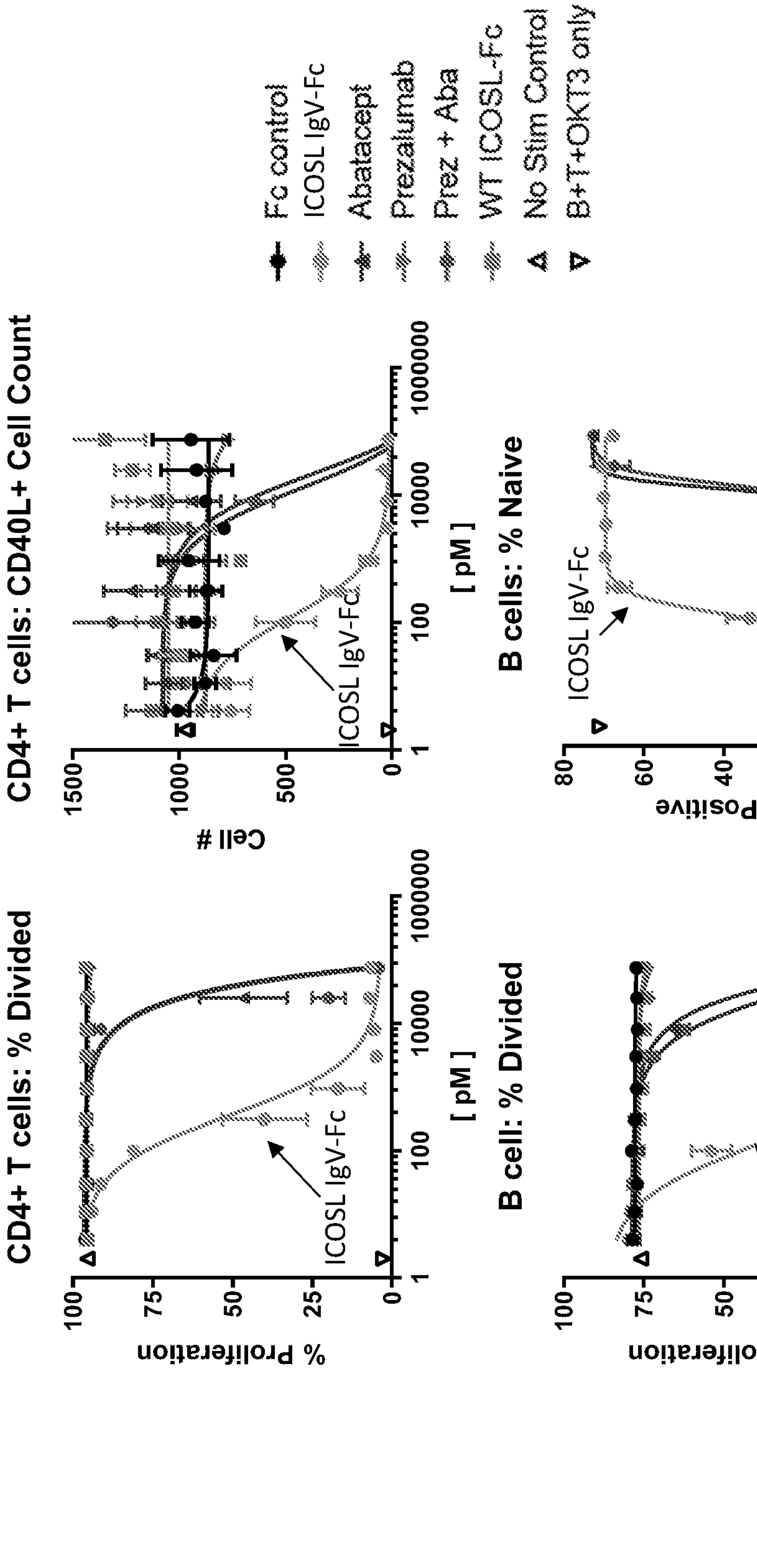
CD4+ T cells: % Divided
% Proliferation
ICOSL IgV-Fc
[ pM ]
CD4+ T cells: CD40L+ Cell Count
Cell #
B cell: % Divided
B cells: % Naive
% Positive
Fc control
ICOSL IgV-Fc
Abatacept
Prezalumab
Prez + Aba
WT ICOSL-Fc
No Stim Control
B+T+OKT3 only

FIG. 19L

| Parameter | D# | B-cell | IC50s (pM) Fc1.1 | WT ICOSL | Preza | Aba | Aba + Prez | Bela | ICOSL IgV-Fc |
|---|---|---|---|---|---|---|---|---|---|
| T cell % Prolif | A | Naïve | ND | ND | >75000 | >75000 | >75000 | [illegible] | [illegible] |
| | B | Naïve | ND | - | ND | [illegible] | [illegible] | [illegible] | [illegible] |
| | D | Total | - | - | - | 5.3 | - | - | 1.2 |
| | E | Total | ND | - | ND | ND | ND | 8.2 | 1.6 |
| T cell Recovery | A | Naïve | ND | >75000 | ND | 52377 | 17275 | [illegible] | 71 |
| | B | Naïve | ND | - | 36873 | [illegible] | [illegible] | [illegible] | 11 |
| | D | Total | - | - | - | 1.31 | - | - | 1.04 |
| | E | Total | ND | - | ND | [illegible] | [illegible] | [illegible] | 1.4 |
| CD40L+ T cell Recovery | A | Naïve | ND | ND | ND | 21511 | 13115 | [illegible] | [illegible] |
| | B | Naïve | [illegible] | - | [illegible] | [illegible] | [illegible] | [illegible] | 17 |
| | D | Total | - | - | - | [illegible] | - | - | 1.03 |
| | E | Total | ND | - | ND | ND | ND | [illegible] | 1.3 |
| B cell % Prolif | A | Naïve | ND | >75000 | ND | >75000 | 43219 | [illegible] | [illegible] |
| | B | Naïve | ND | - | ND | [illegible] | [illegible] | [illegible] | [illegible] |
| | D | Total | - | - | - | [illegible] | - | - | [illegible] |
| | E | Total | ND | - | ND | [illegible] | [illegible] | [illegible] | 1.4 |
| B cell % Naïve | A | Naïve | 14518 | [illegible] | [illegible] | [illegible] | [illegible] | [illegible] | [illegible] |
| | B | Naïve | ND | - | [illegible] | [illegible] | [illegible] | [illegible] | [illegible] |
| | D | Total | - | - | - | [illegible] | - | - | 1.3 |
| | E | Total | ND | - | 5 | [illegible] | 1.2 | 4 | 1.6 |
| B cell % Activated (Non-Class-Switched) | A | Naïve | ND | [illegible] | [illegible] | 29381 | 44841 | [illegible] | [illegible] |
| | B | Naïve | ND | - | [illegible] | [illegible] | [illegible] | [illegible] | [illegible] |
| | D | Total | - | - | - | [illegible] | - | - | 1.07 |
| | E | Total | ND | - | [illegible] | [illegible] | [illegible] | [illegible] | 1.4 |

( ND ) Not Determined ( - ) Not assayed

Worse — Better

FIG. 19M

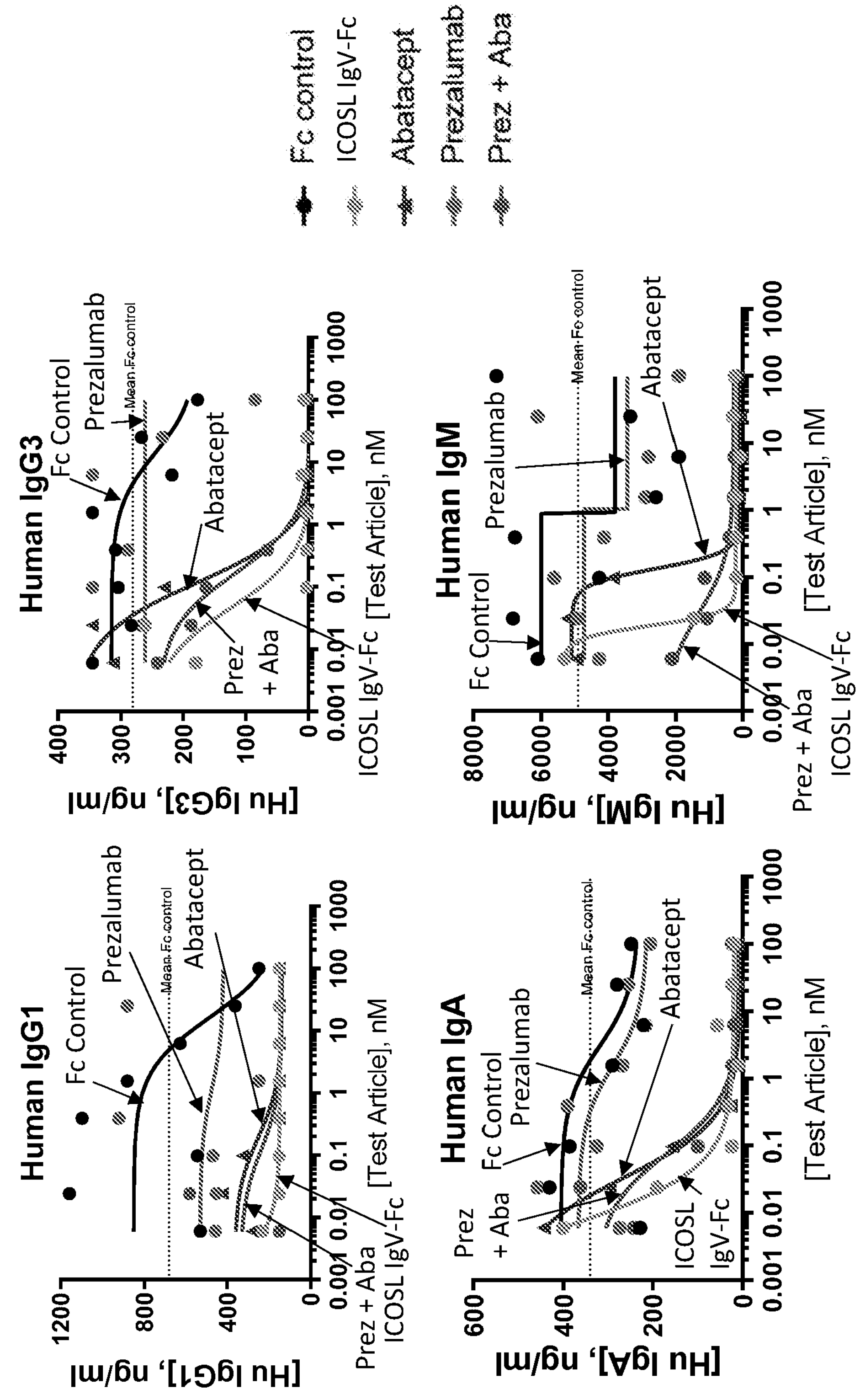
Human IgG1
[Hu IgG1], ng/ml
Fc Control
Prezalumab
Abatacept
Mean Fc control
Prez + Aba
ICOSL IgV-Fc
[Test Article], nM
Human IgG3
[Hu IgG3], ng/ml
Prez
+ Aba
Human IgA
[Hu IgA], ng/ml
ICOSL
IgV-Fc
Human IgM
[Hu IgM], ng/ml
Fc control
ICOSL IgV-Fc
Abatacept
Prezalumab
Prez + Aba

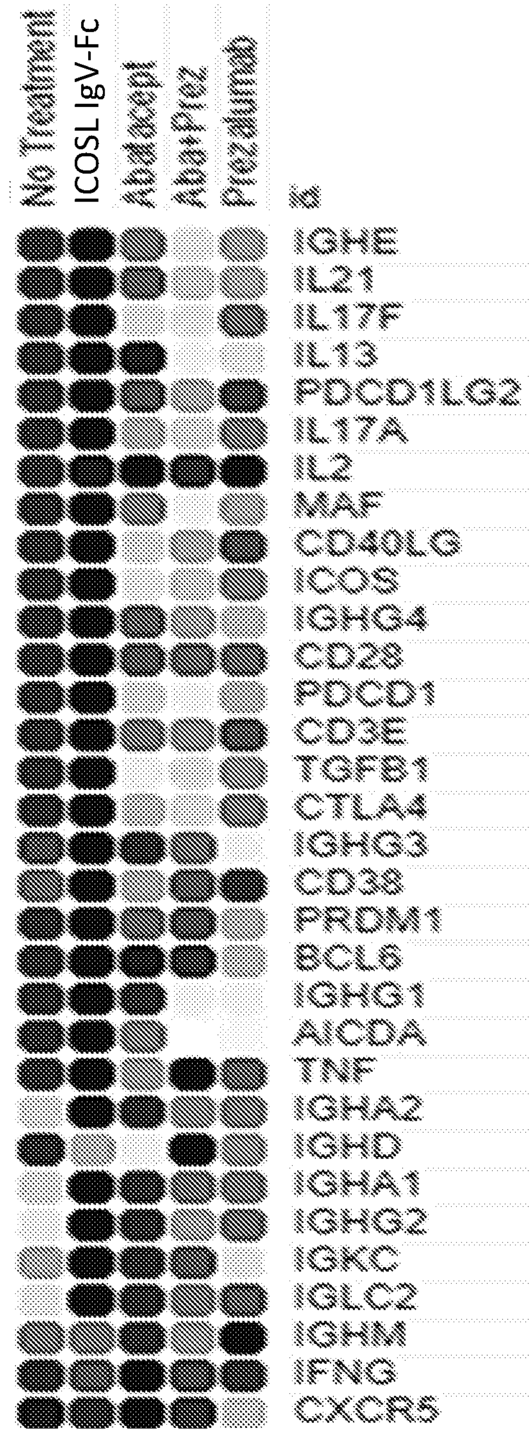
No Treatment
ICOSL IgV-Fc
Abatacept
Aba+Prez
Prezalumab
id
IGHE
IL21
IL17F
IL13
PDCD1LG2
IL17A
IL2
MAF
CD40LG
ICOS
IGHG4
CD28
PDCD1
CD3E
TGFB1
CTLA4
IGHG3
CD38
PRDM1
BCL6
IGHG1
AICDA
TNF
IGHA2
IGHD
IGHA1
IGHG2
IGKC
IGLC2
IGHM
IFNG
CXCR5

FIG. 19N

FIG. 20A

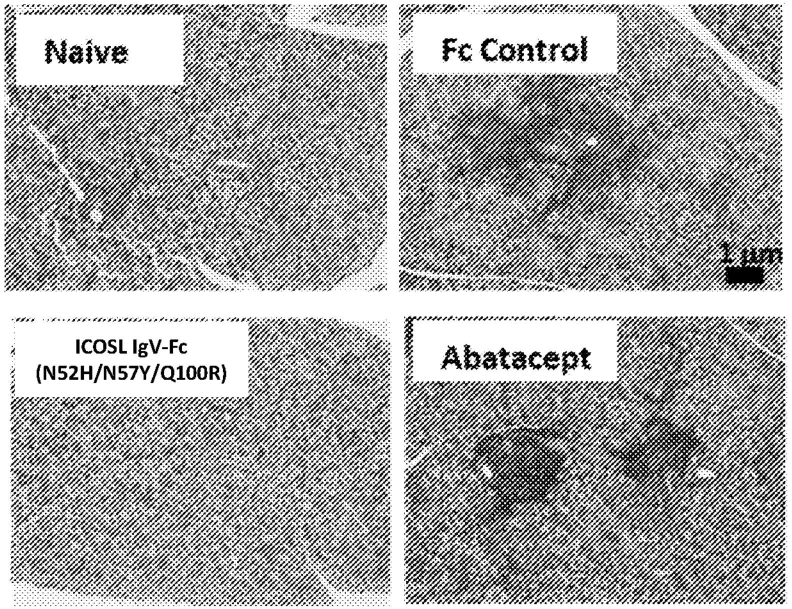


FIG. 20B

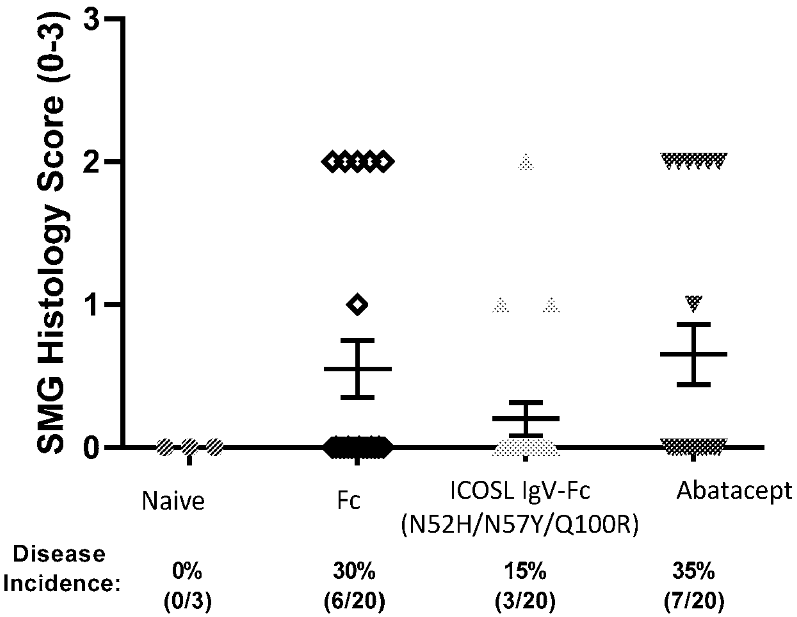

FIG. 20C

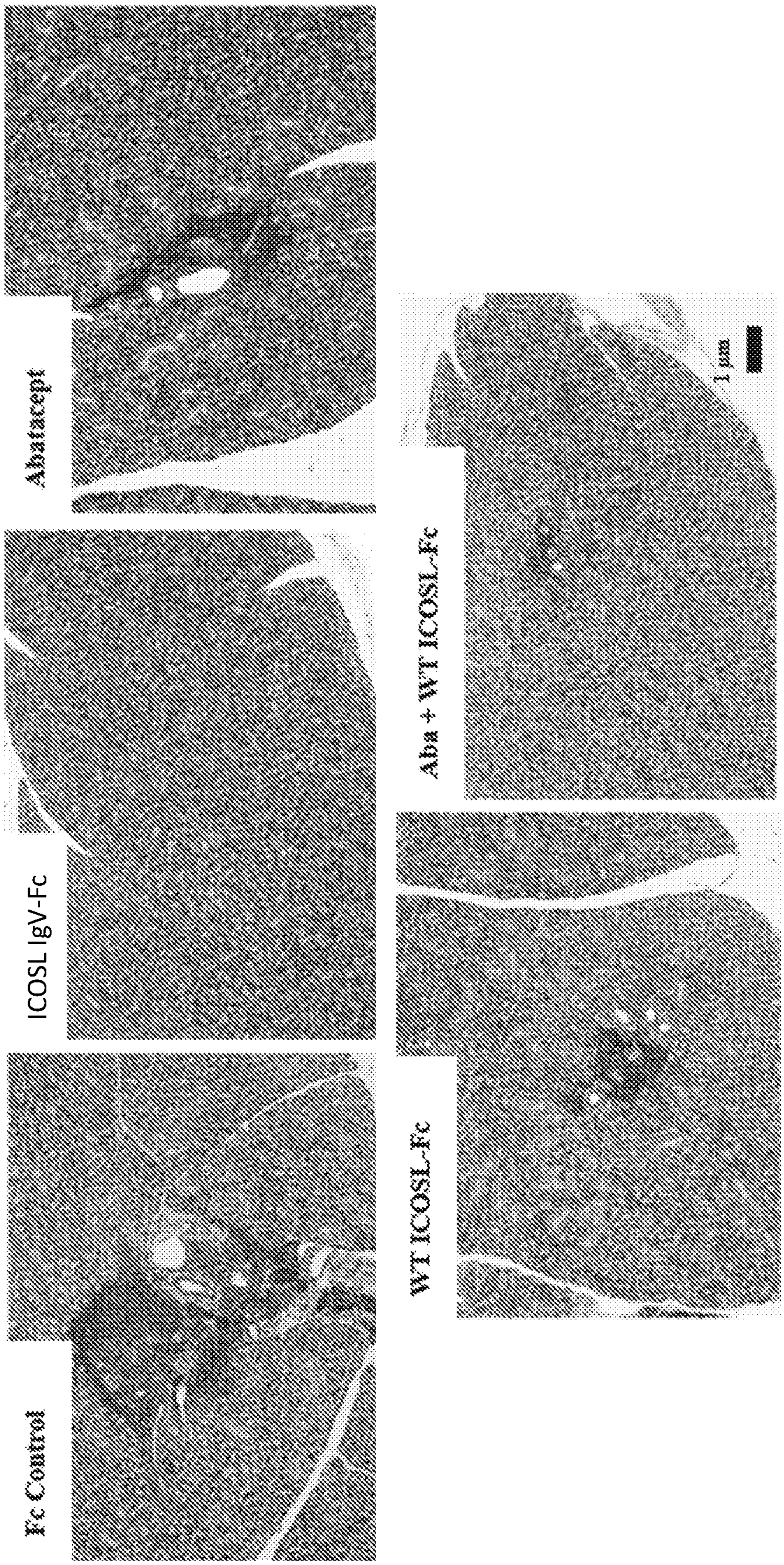
Fc Control
ICOSL IgV-Fc
Abatacept
WT ICOSL-Fc
Aba + WT ICOSL-Fc
1 μm

FIG. 20D

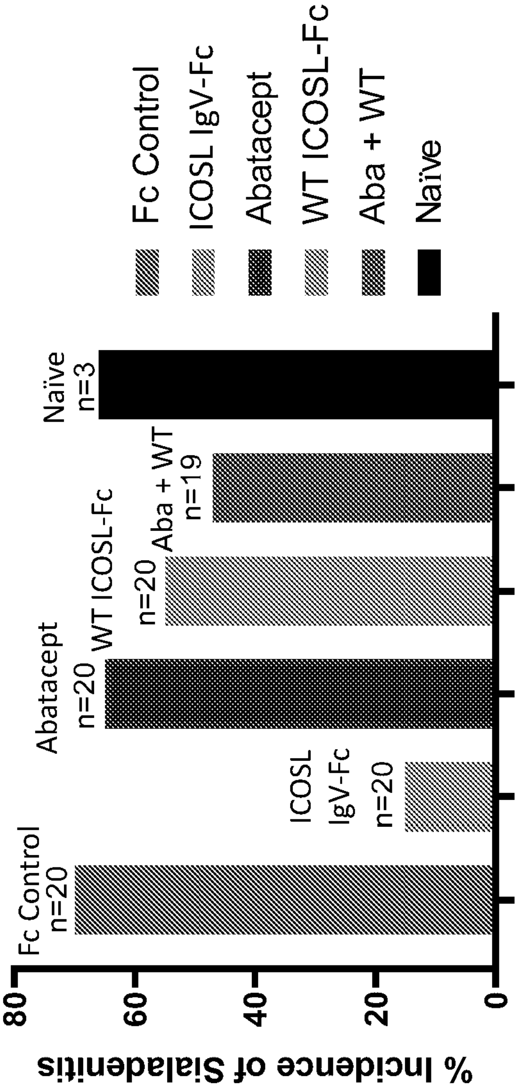


FIG. 20E

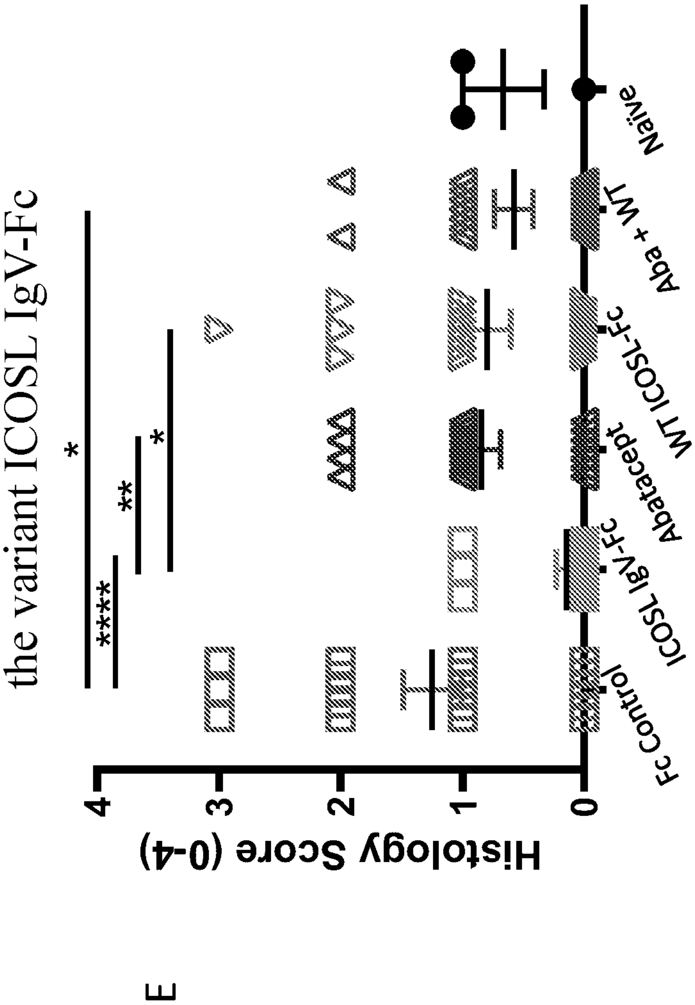

FIG. 20F

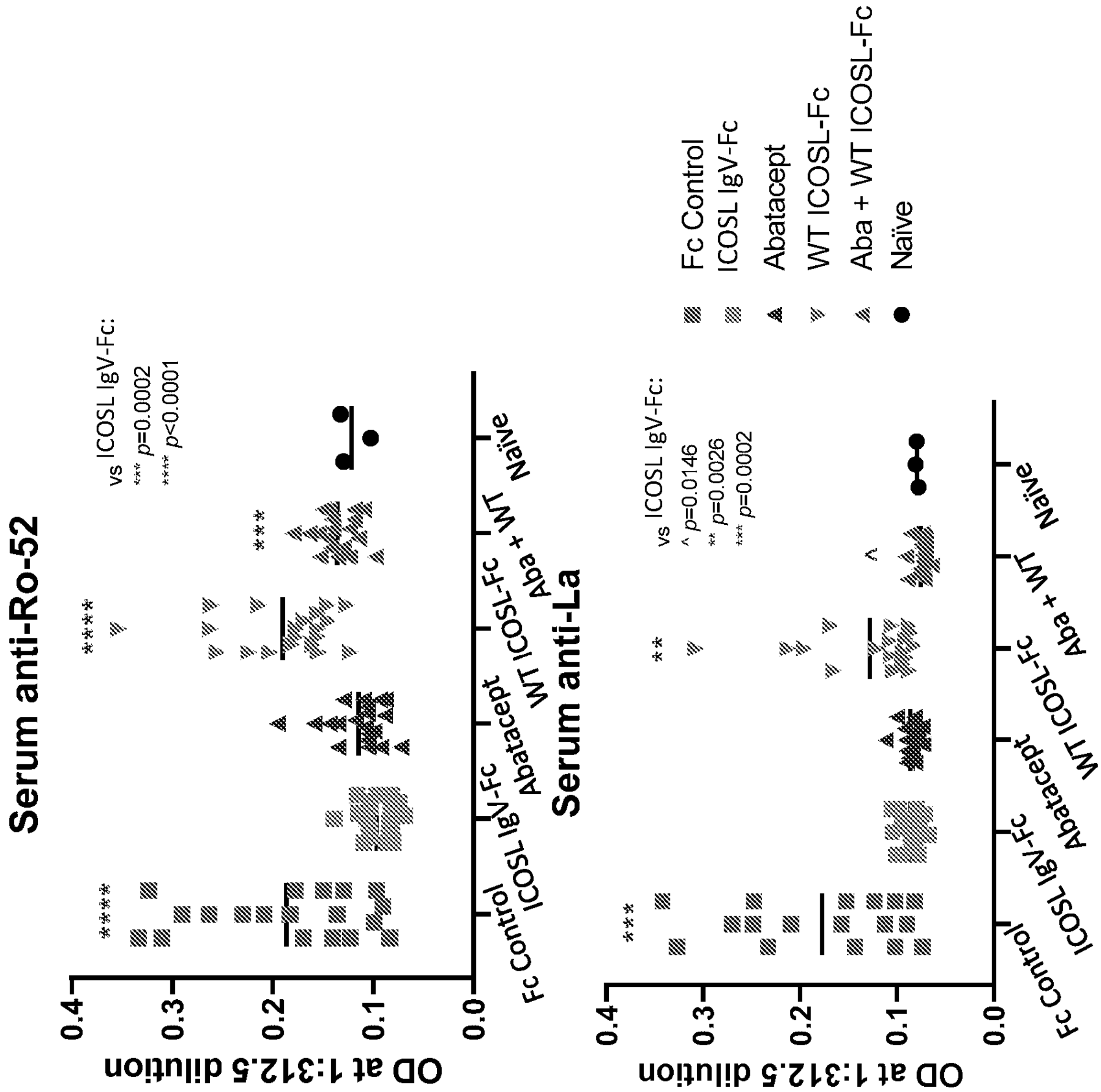
Serum anti-Ro-52
OD at 1:312.5 dilution
0.0
0.1
0.2
0.3
0.4
Fc Control
ICOSL IgV-Fc
Abatacept
WT ICOSL-Fc
Aba + WT
Naïve
vs ICOSL IgV-Fc:
*** p=0.0002
**** p<0.0001
Serum anti-La
OD at 1:312.5 dilution
0.0
0.1
0.2
0.3
0.4
Fc Control
ICOSL IgV-Fc
Abatacept
WT ICOSL-Fc
Aba + WT
Naïve
vs ICOSL IgV-Fc:
^ p=0.0146
** p=0.0026
*** p=0.0002
Fc Control
ICOSL IgV-Fc
Abatacept
WT ICOSL-Fc
Aba + WT ICOSL-Fc
Naïve

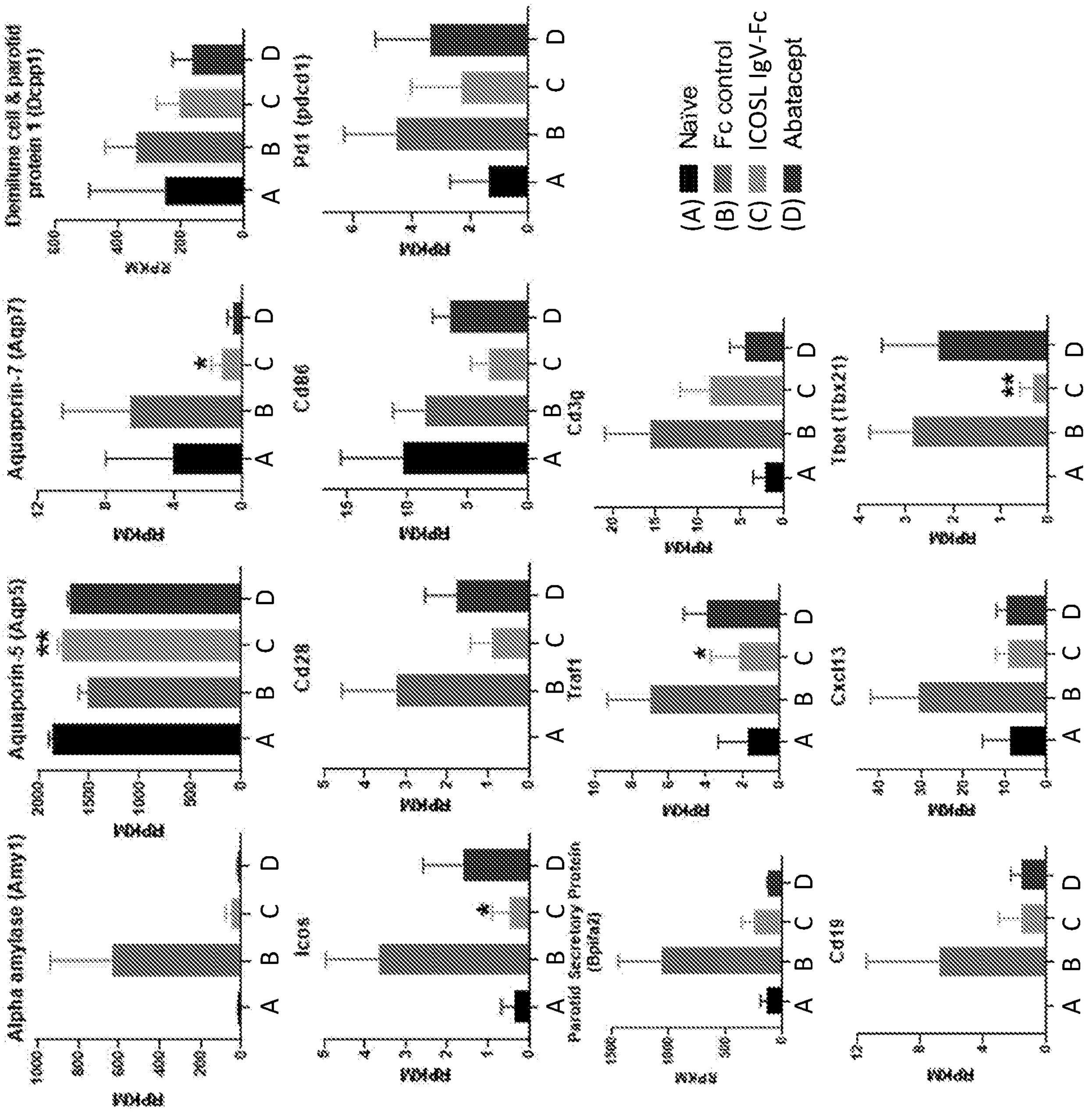
Alpha amylase (Amy1)
Aquaporin-5 (Aqp5)
Aquaporin-7 (Aqp7)
Demilune cell & parotid protein 1 (Dcpp1)
Icos
Cd28
Cd86
Pd1 (pdcd1)
Parotid Secretory Protein (Bpifa2)
Traf1
Cd3g
Cd19
Cxcl13
Tbet (Tbx21)
RPKM
(A) Naive
(B) Fc control
(C) ICOSL IgV-Fc
(D) Abatacept

FIG. 20G

FIG. 20H

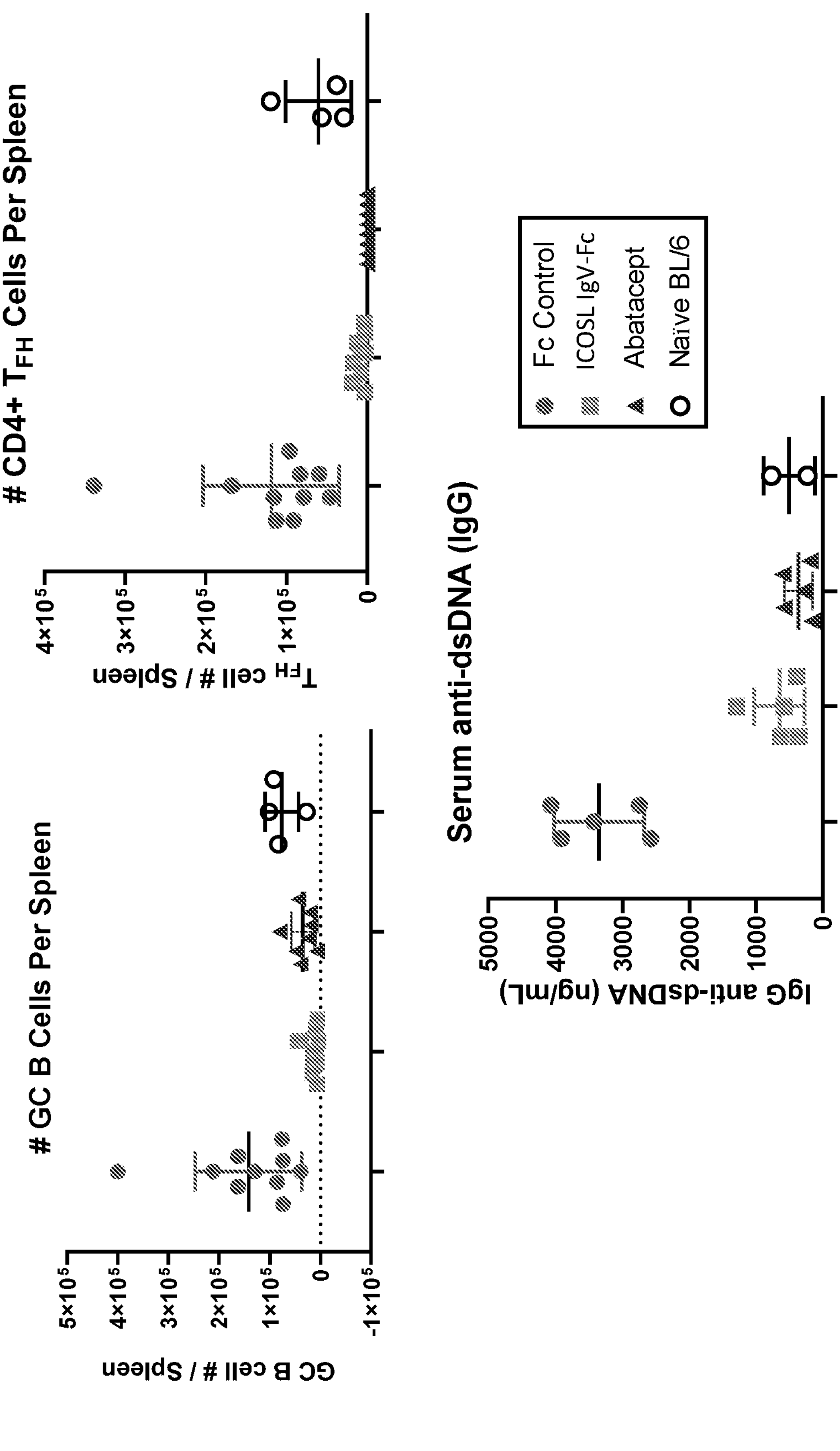
# GC B Cells Per Spleen
GC B cell # / Spleen
5×10⁵
4×10⁵
3×10⁵
2×10⁵
1×10⁵
0
-1×10⁵
# CD4+ T$_{FH}$ Cells Per Spleen
T$_{FH}$ cell # / Spleen
4×10⁵
3×10⁵
2×10⁵
1×10⁵
0
Serum anti-dsDNA (IgG)
IgG anti-dsDNA (ng/mL)
5000
4000
3000
2000
1000
0
Fc Control
ICOSL IgV-Fc
Abatacept
Naïve BL/6

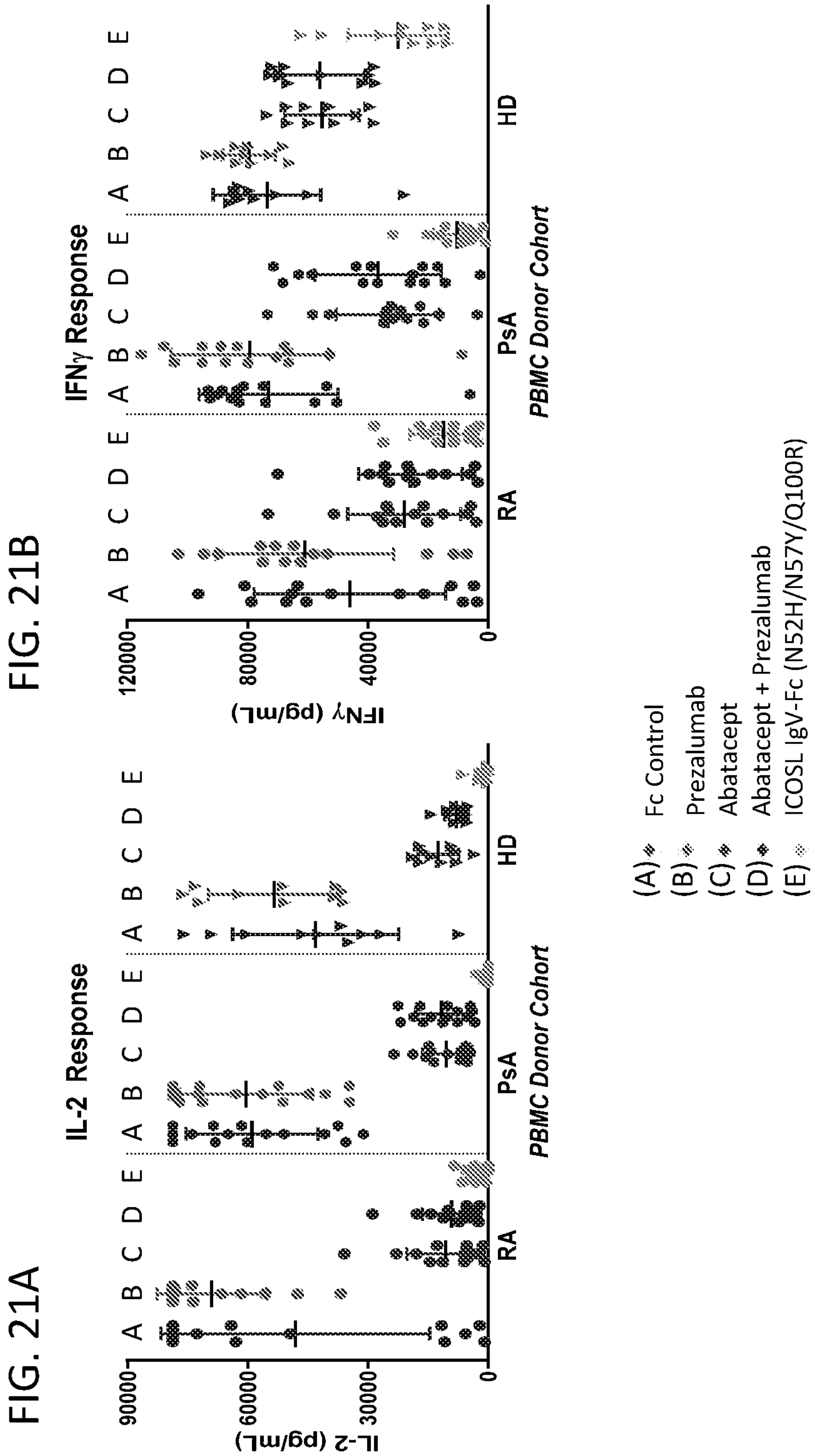
FIG. 21A
IL-2 Response
IL-2 (pg/mL)
90000
60000
30000
0
A B C D E
RA
PsA
HD
PBMC Donor Cohort
FIG. 21B
IFNγ Response
IFNγ (pg/mL)
120000
80000
40000
0
(A) Fc Control
(B) Prezalumab
(C) Abatacept
(D) Abatacept + Prezalumab
(E) ICOSL IgV-Fc (N52H/N57Y/Q100R)

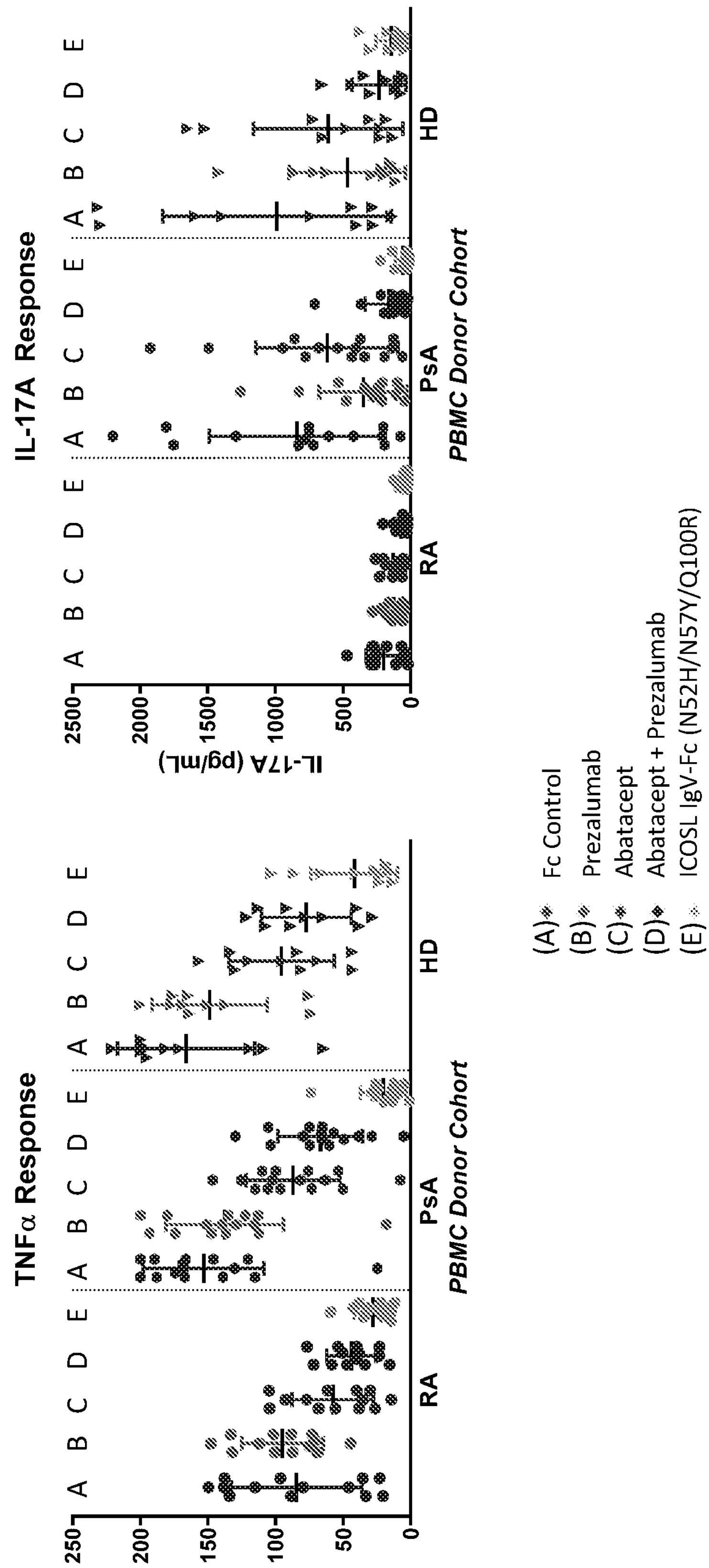


FIG. 21C

FIG. 21D

FIG. 21E

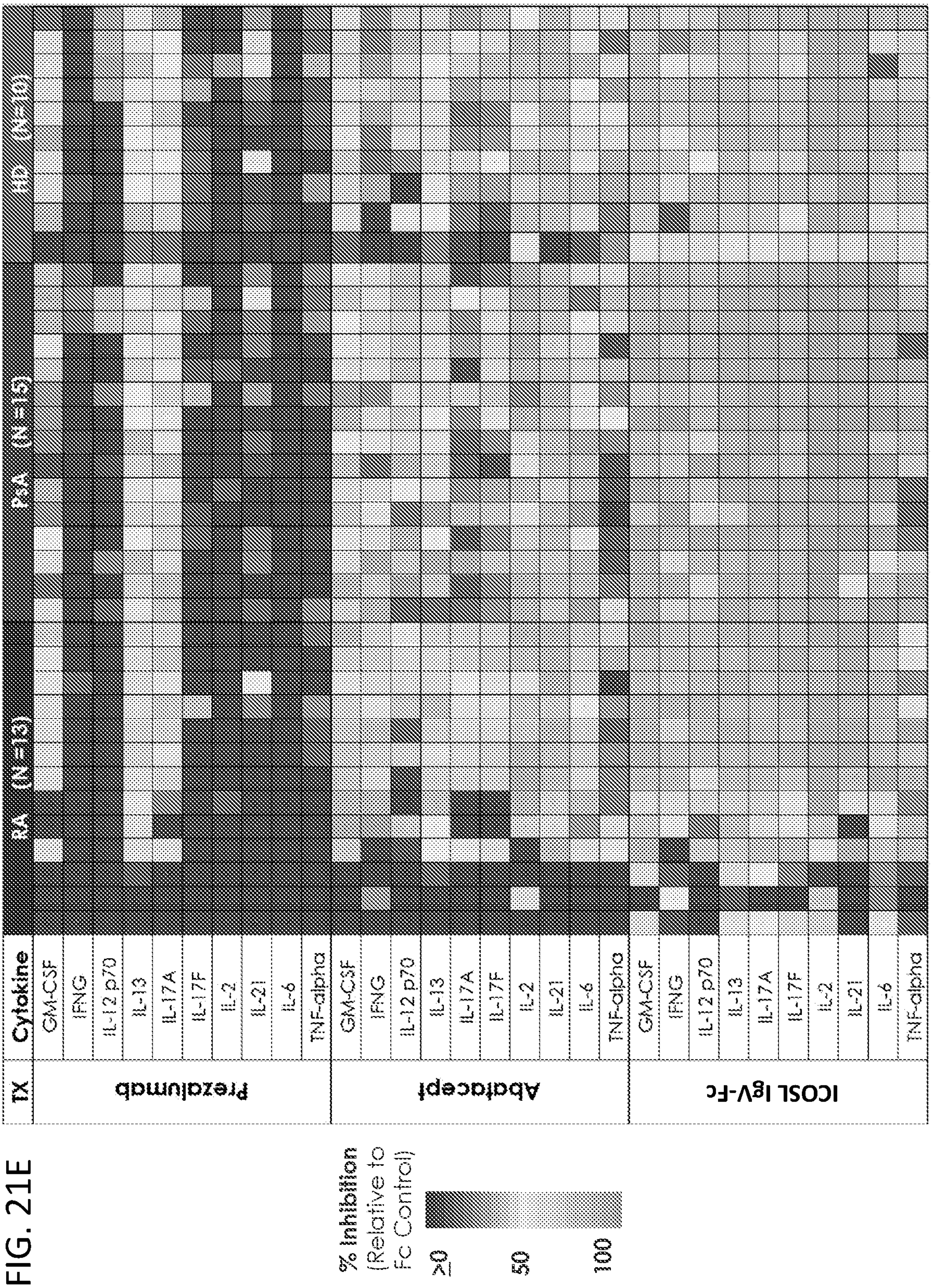
TX
Cytokine
RA (N =13)
PsA (N =15)
HD (N=10)
Prezalumab
Abatacept
ICOSL IgV-Fc
GM-CSF
IFNG
IL-12 p70
IL-13
IL-17A
IL-17F
IL-2
IL-21
IL-6
TNF-alpha
% Inhibition
(Relative to
Fc Control)
≥0
50
100

FIG. 22A

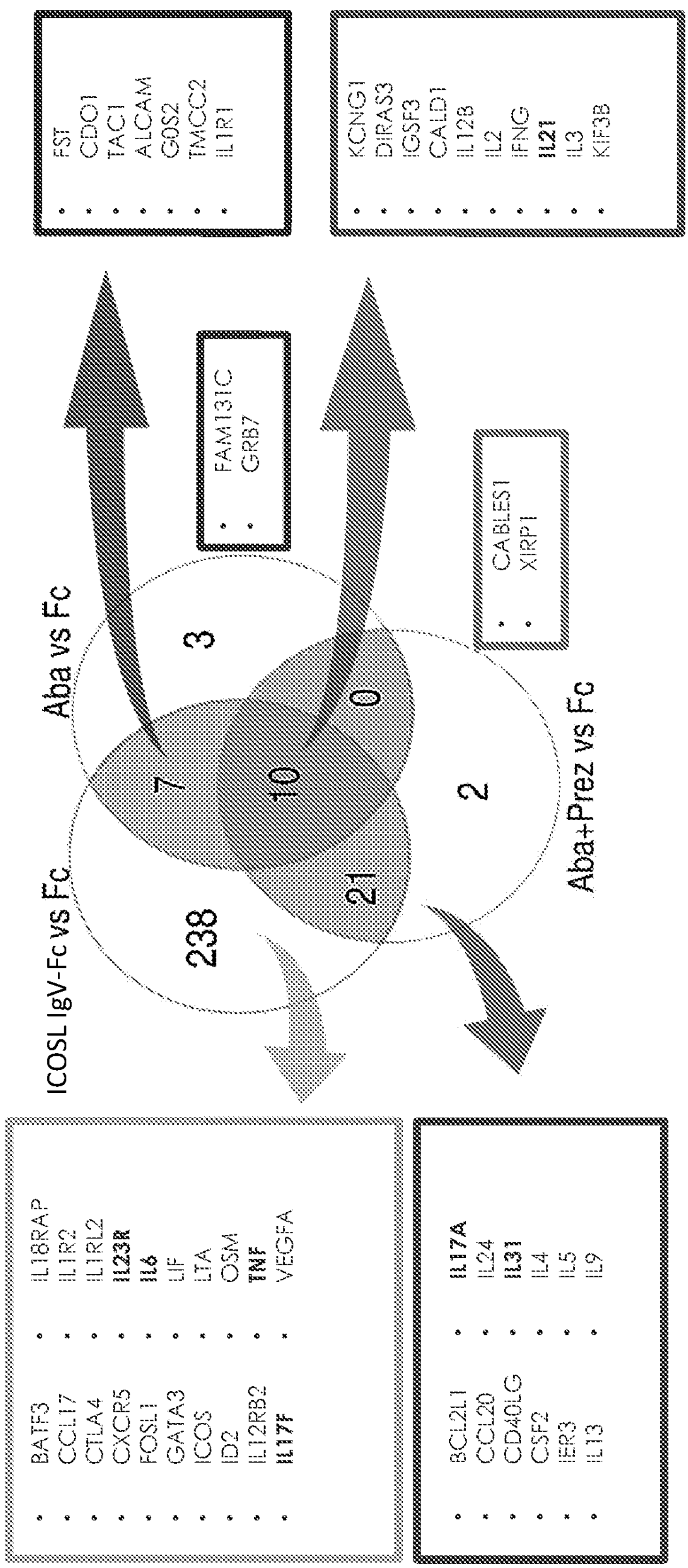
ICOSL IgV-Fc vs Fc
Aba vs Fc
Aba+Prez vs Fc
238
7
10
21
3
0
2
BATF3
CCL17
CTLA4
CXCR5
FOSL1
GATA3
ICOS
ID2
IL12RB2
IL17F
IL18RAP
IL1R2
IL1RL2
IL23R
IL6
LIF
LTA
OSM
TNF
VEGFA
BCL2L1
CCL20
CD40LG
CSF2
IER3
IL13
IL17A
IL24
IL31
IL4
IL5
IL9
FAM131C
GRB7
CABLES1
XIRP1
FST
CDO1
TAC1
ALCAM
G0S2
TMCC2
IL1R1
KCNG1
DIRAS3
IGSF3
CALD1
IL12B
IL2
IFNG
IL21
IL3
KIF3B

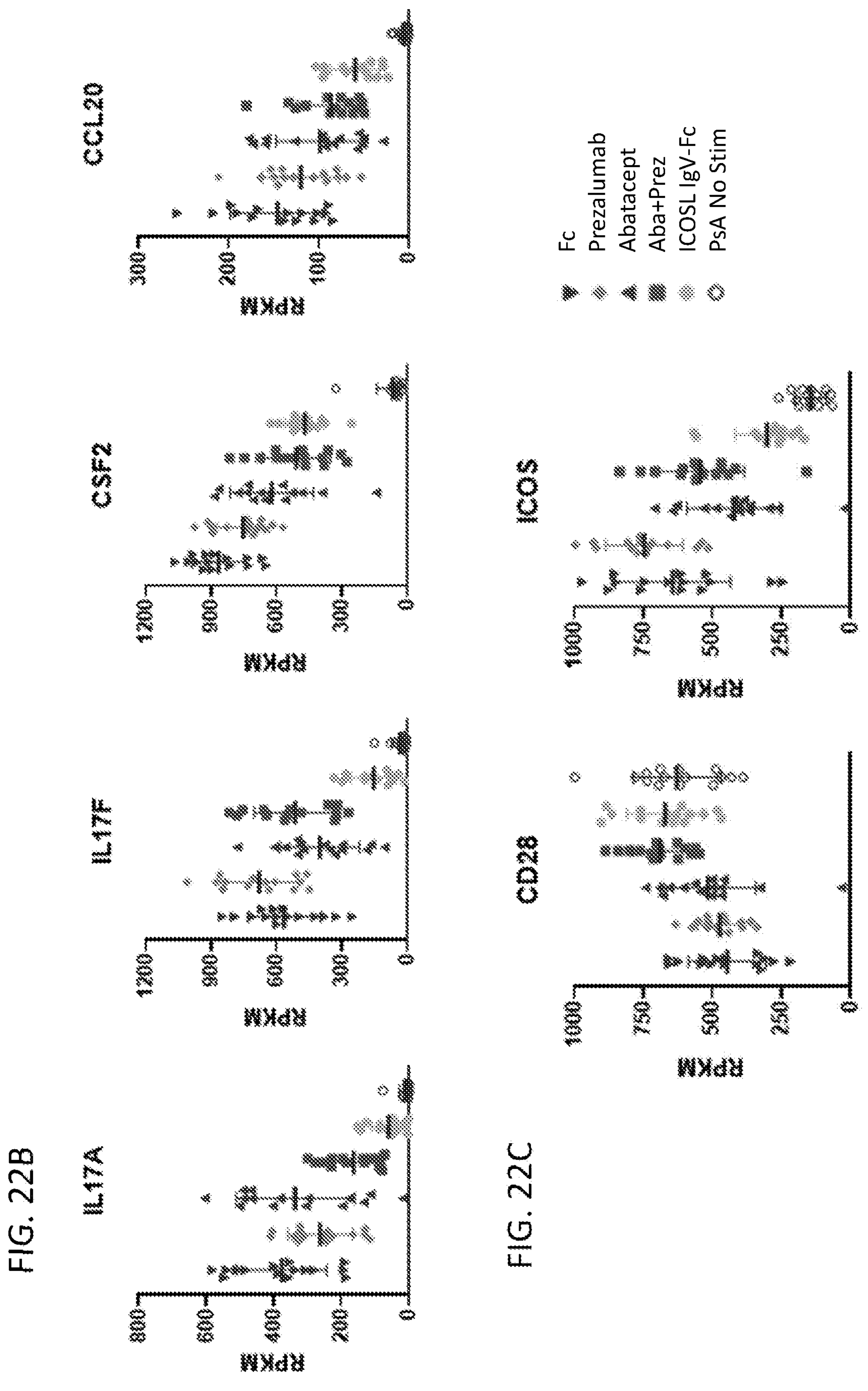


FIG. 22B

FIG. 22C

FIG. 22D

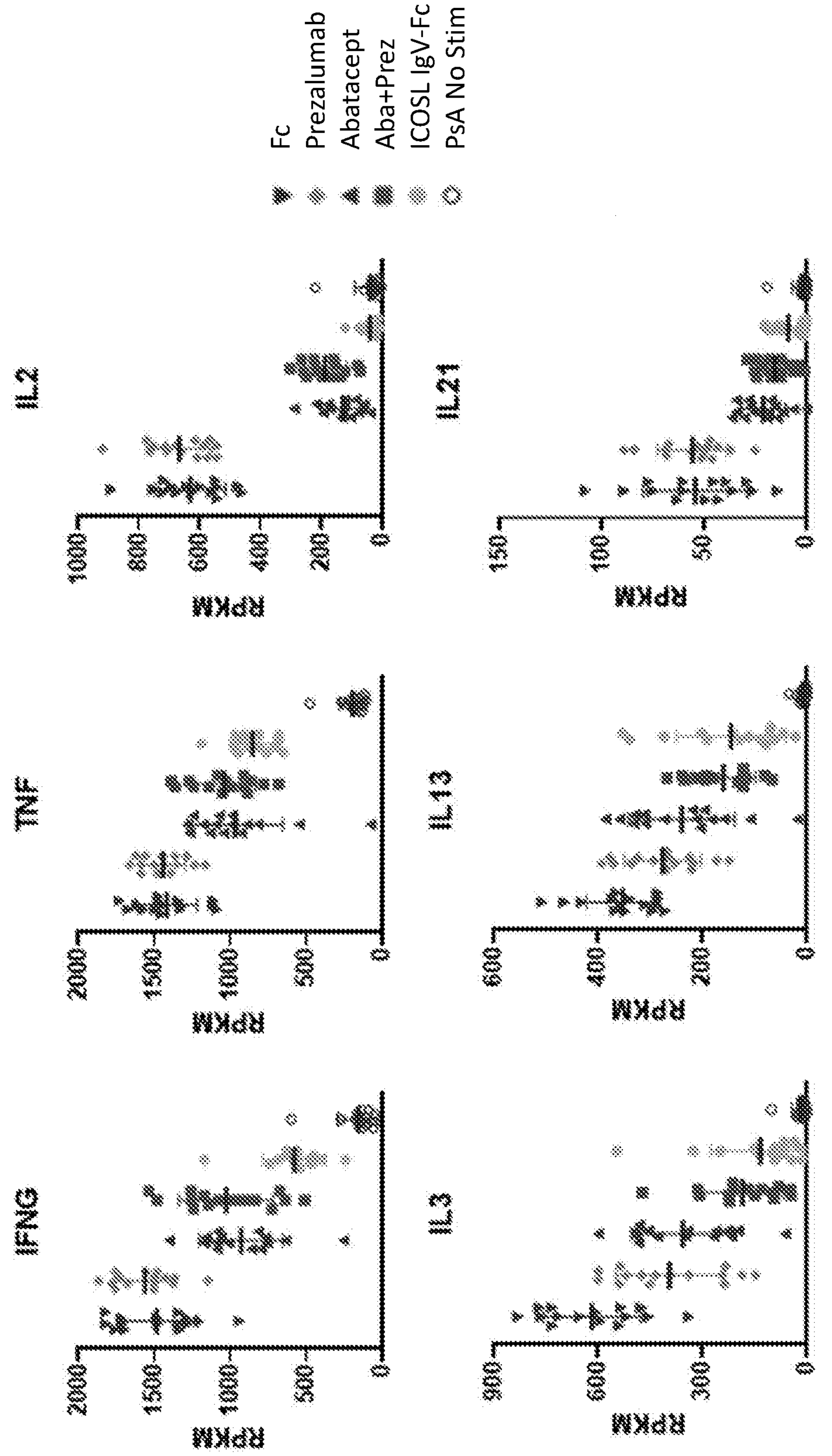
IFNG
TNF
IL2
IL3
IL13
IL21
RPKM
Fc
Prezalumab
Abatacept
Aba+Prez
ICOSL IgV-Fc
PsA No Stim

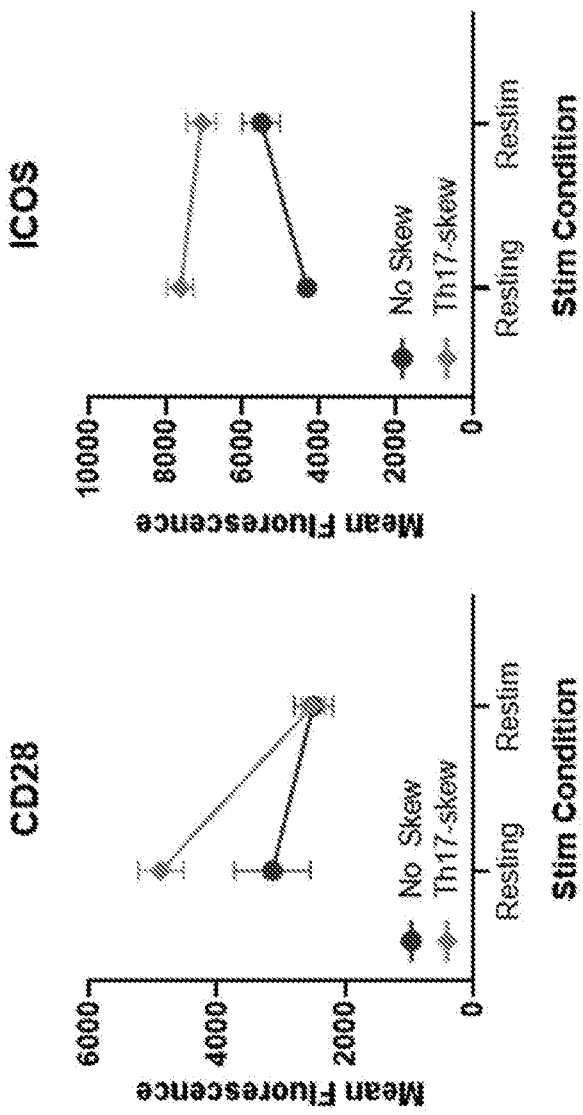
CD28
ICOS
Mean Fluorescence
Stim Condition
Resting
Restim
No Skew
Th17-skew
0
2000
4000
6000
8000
10000

FIG. 23A

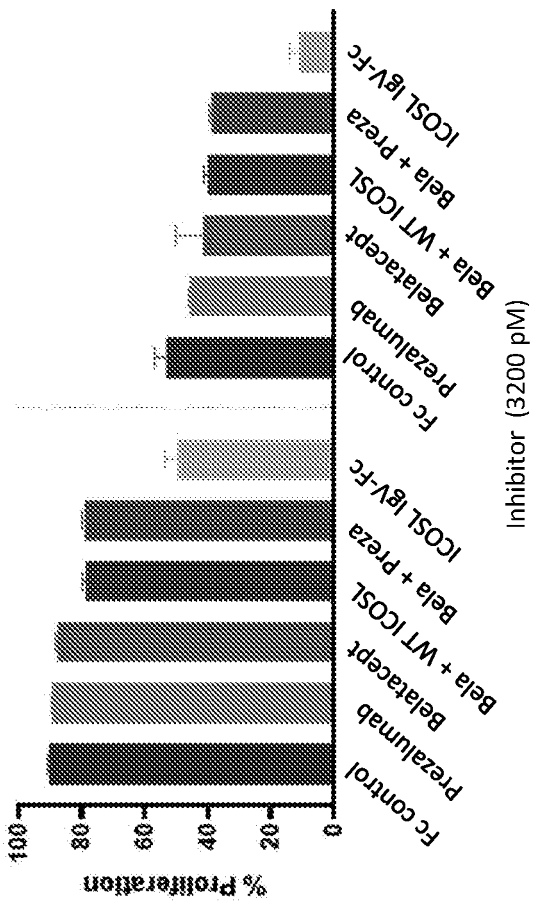


FIG. 23B

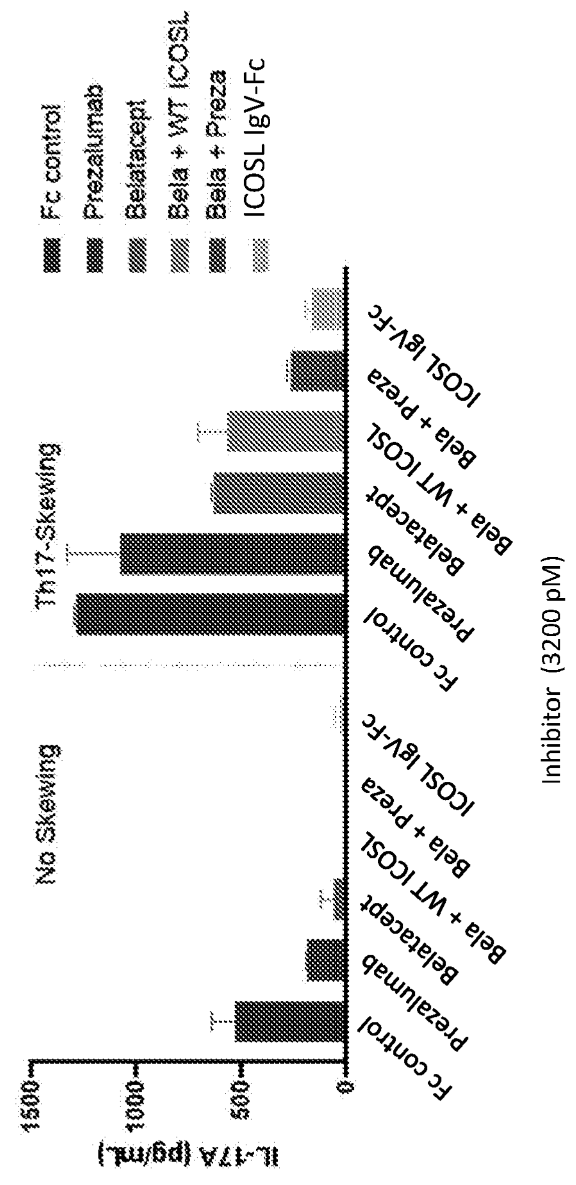


FIG. 23C

FIG. 24A

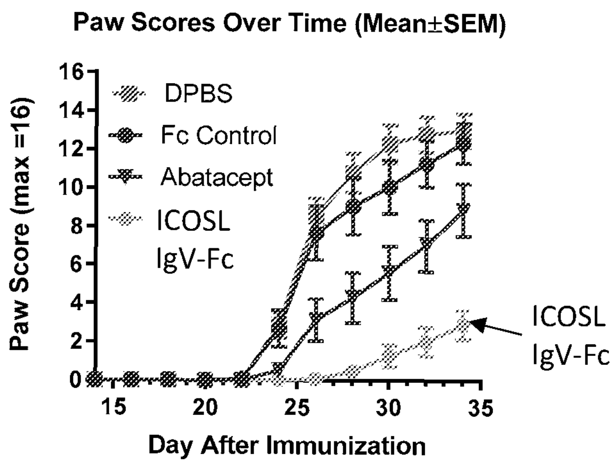


Paw Scores Over Time (P values)

| | DPBS | Fc Control | Abatacept | ICOSL IgV-Fc |
|---|---|---|---|---|
| DPBS | - | - | 0.0006 | <0.0001 |
| Fc Control | - | - | 0.0172 | <0.0001 |
| Abatacept | - | - | - | 0.0016 |
| ICOSL IgV-Fc | - | - | - | - |

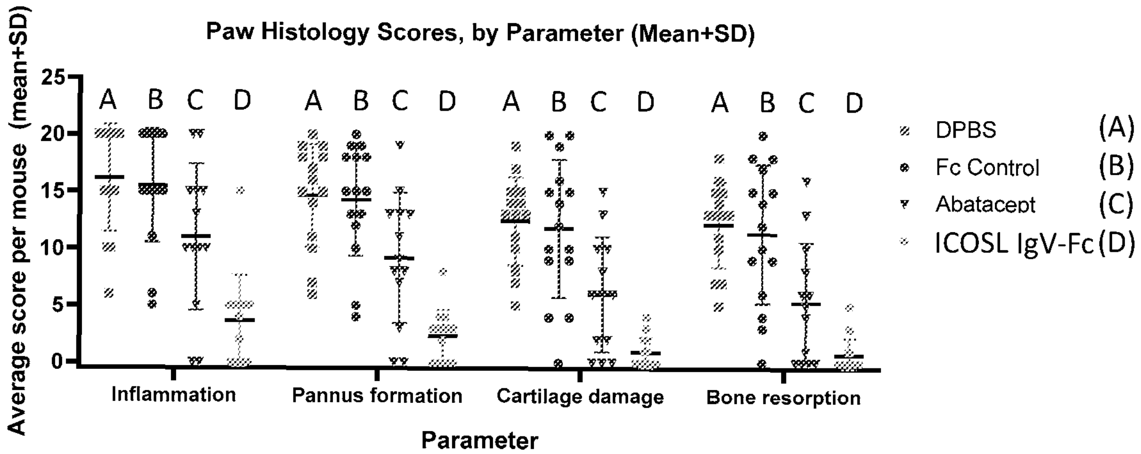

FIG. 24B

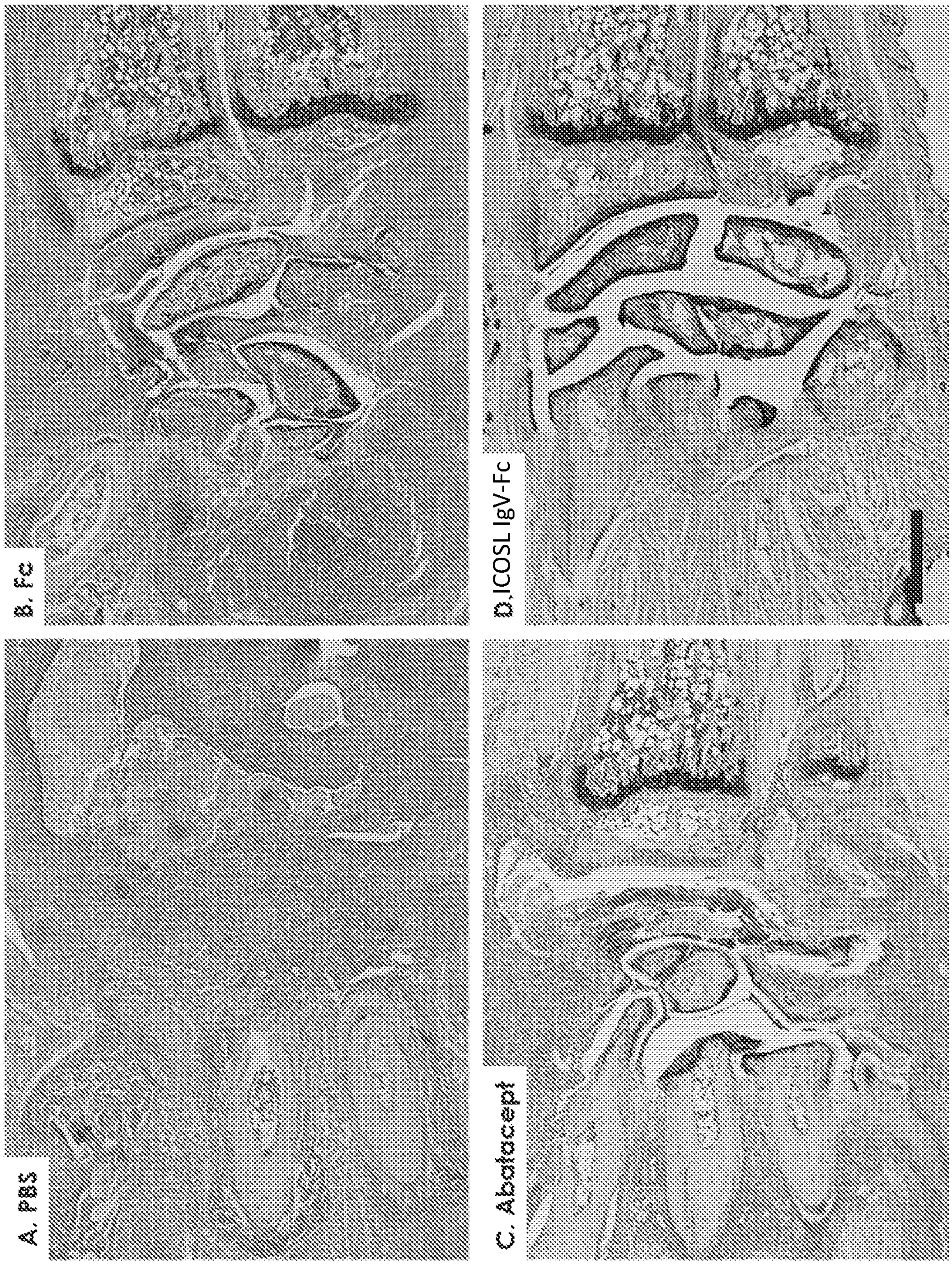

FIG. 24C

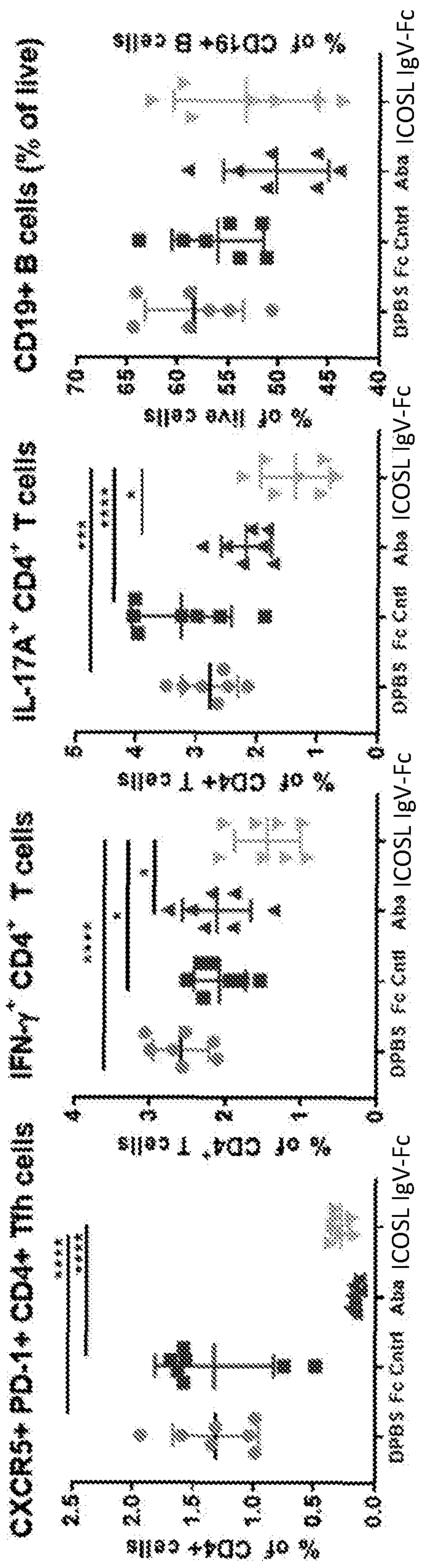


FIG. 24D

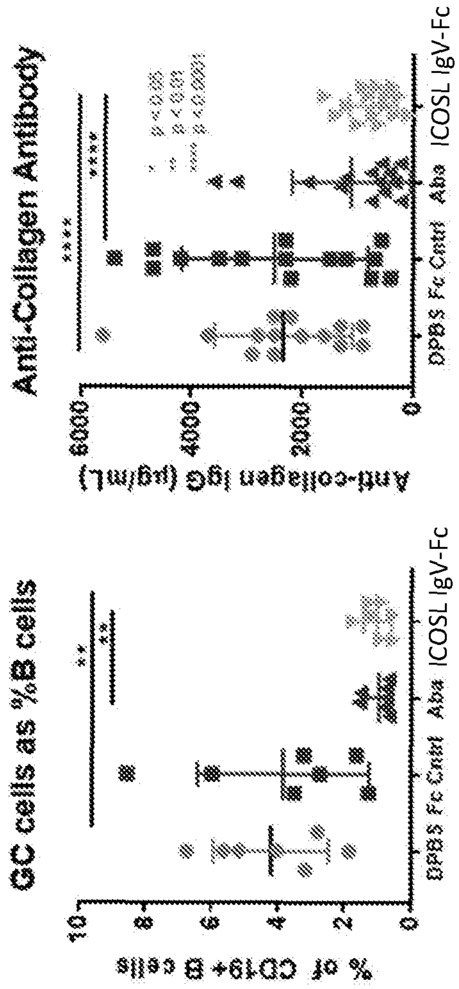

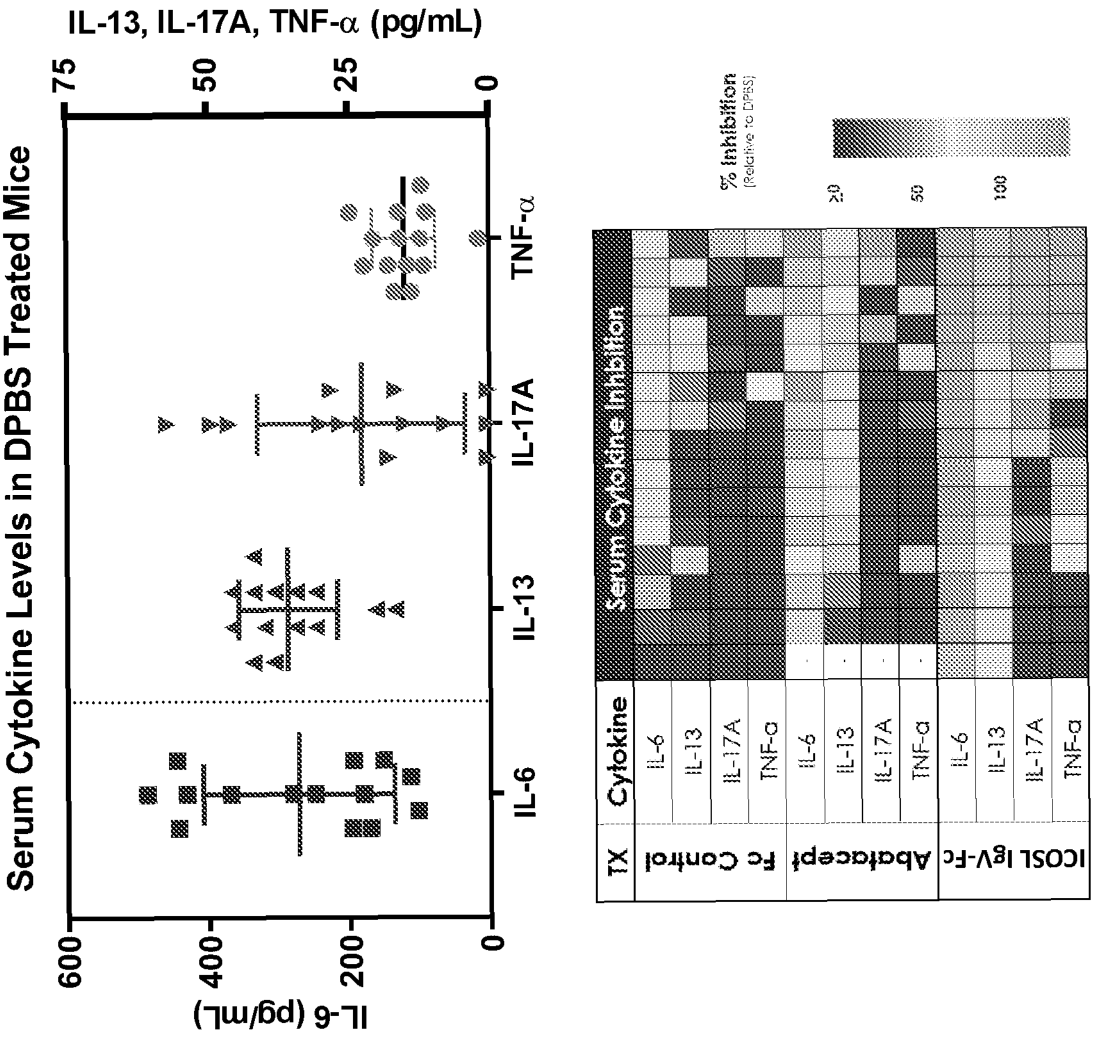
Serum Cytokine Levels in DPBS Treated Mice
IL-6 (pg/mL)
600
400
200
0
IL-13, IL-17A, TNF-α (pg/mL)
75
50
25
0
IL-6
IL-13
IL-17A
TNF-α
Serum Cytokine Inhibition
TX
Cytokine
Fc Control
Abatacept
ICOSL IgV-Fc
IL-6
IL-13
IL-17A
TNF-α
% Inhibition
(Relative to DPBS)
≥0
50
100

FIG. 24E

FIG. 25B

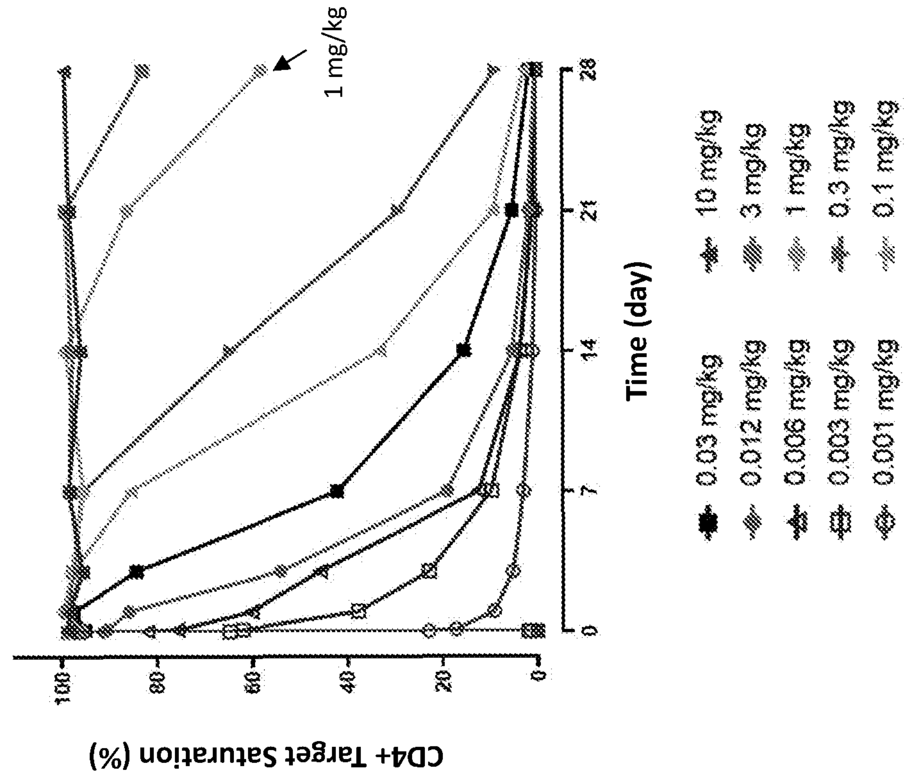


FIG. 25A

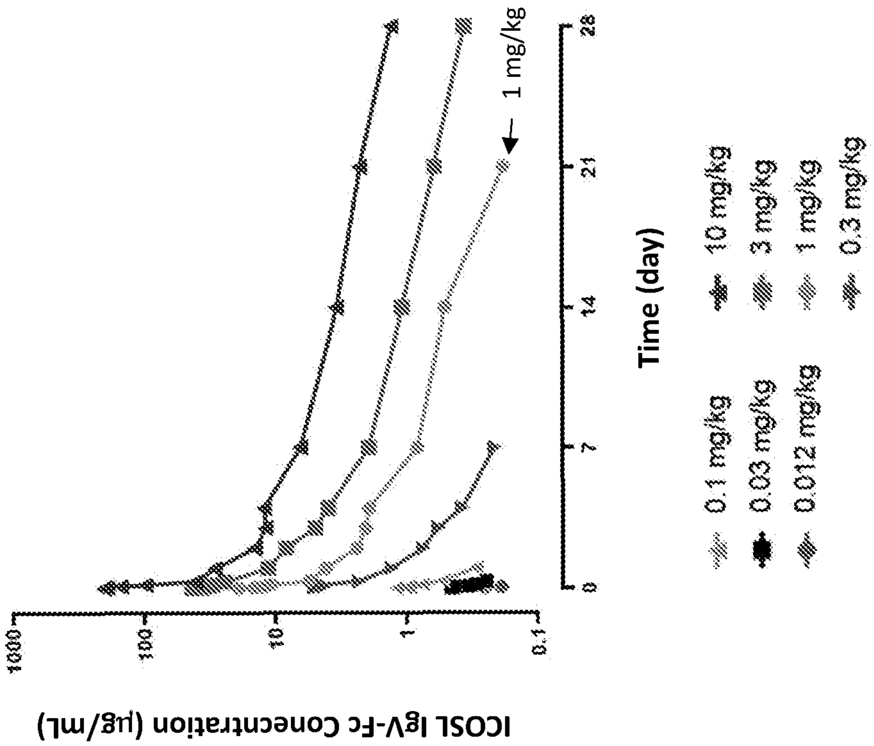

FIG. 26A

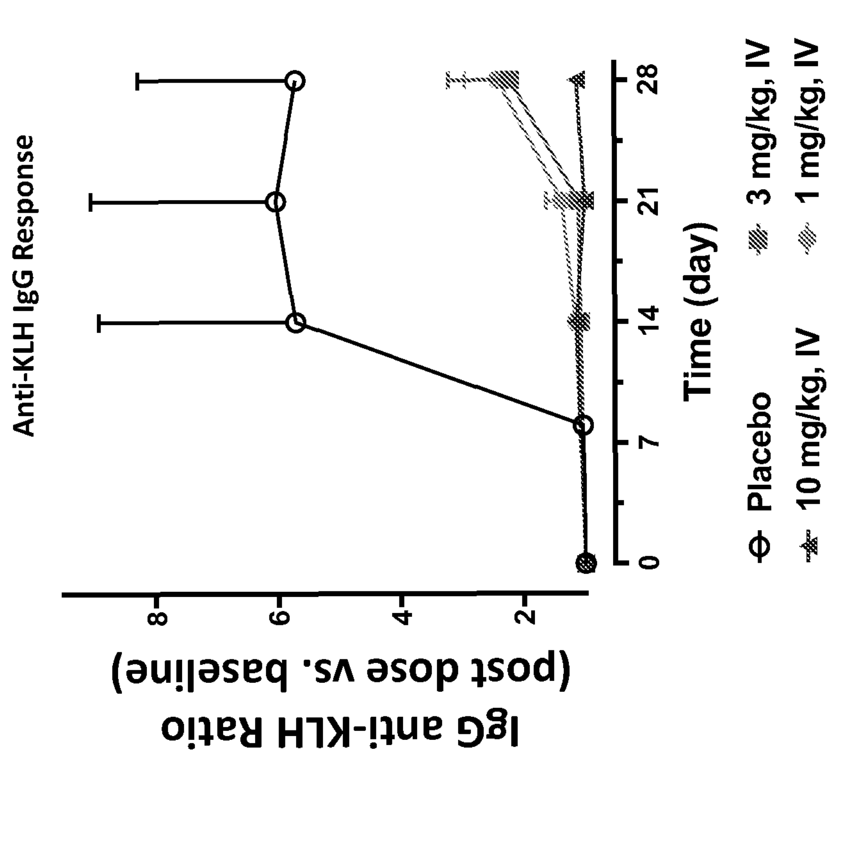


FIG. 26B

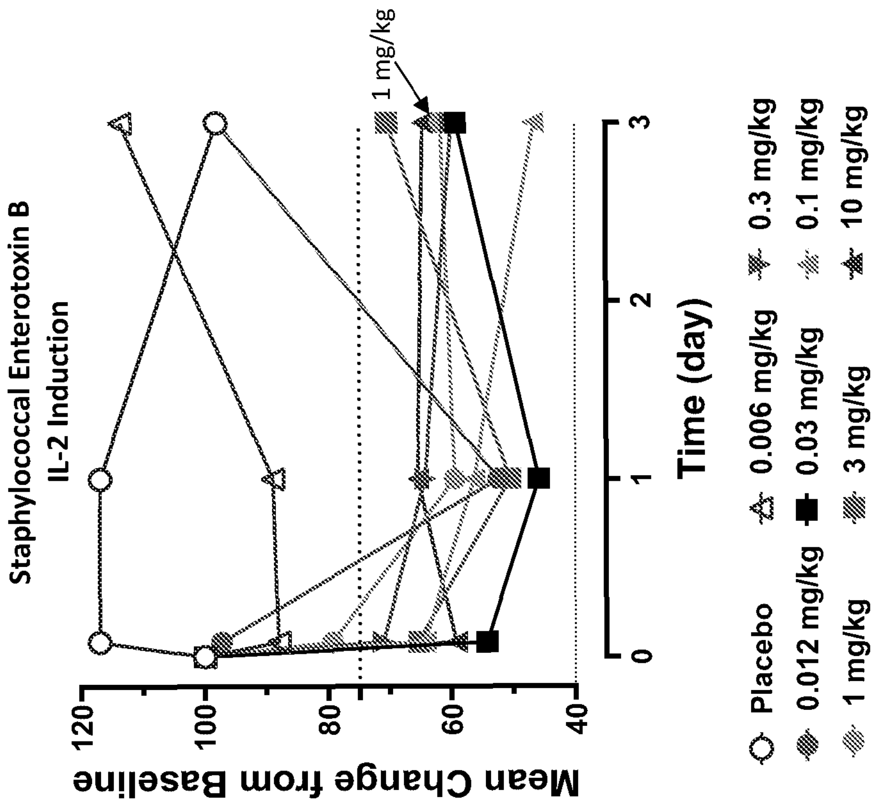

FIG. 27A

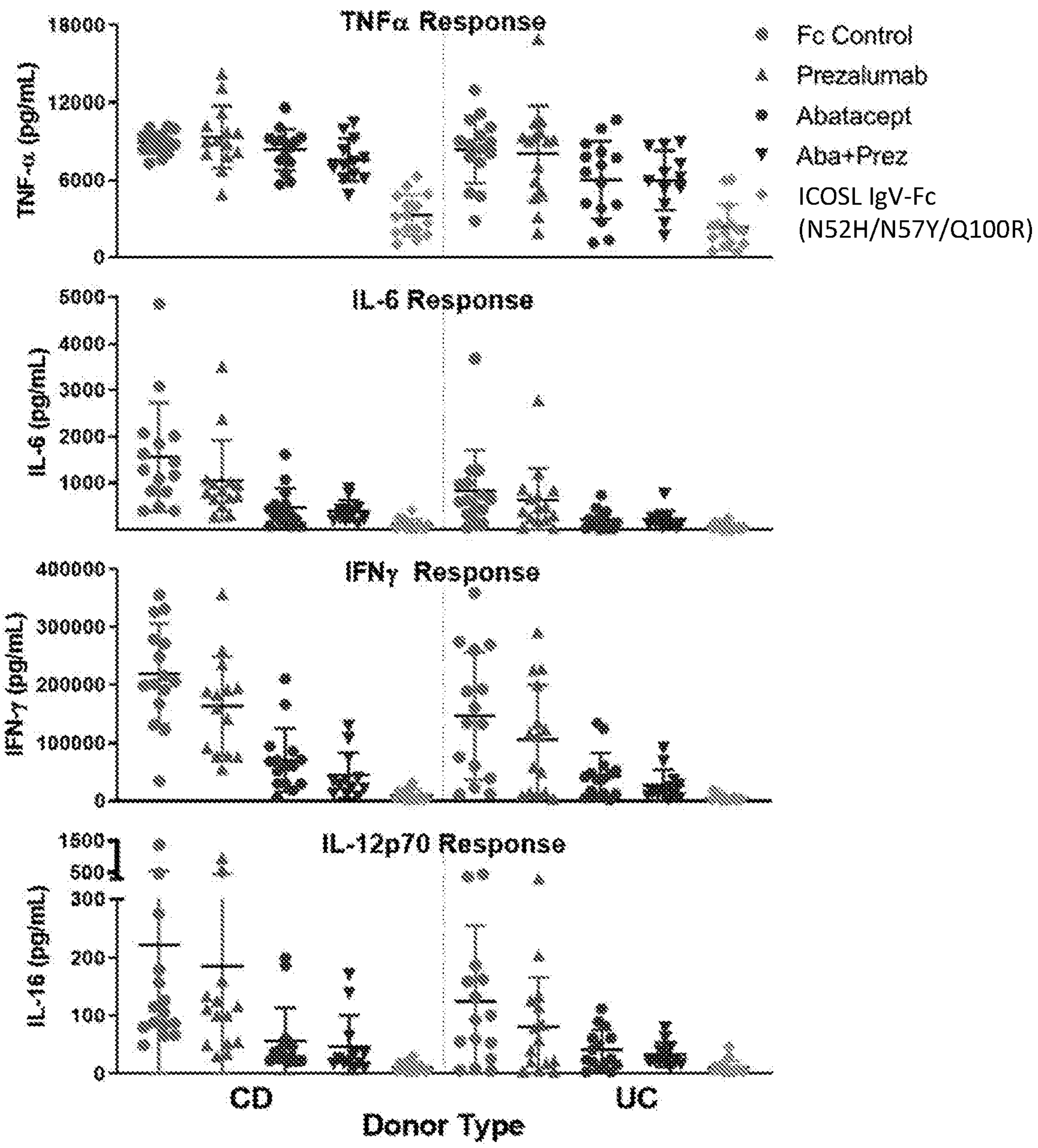
TNFα Response
TNF-α (pg/mL)
18000
12000
6000
0
IL-6 Response
IL-6 (pg/mL)
5000
4000
3000
2000
1000
IFNγ Response
IFN-γ (pg/mL)
400000
300000
200000
100000
0
IL-12p70 Response
IL-16 (pg/mL)
1500
500
300
200
100
0
CD
UC
Donor Type
Fc Control
Prezalumab
Abatacept
Aba+Prez
ICOSL IgV-Fc
(N52H/N57Y/Q100R)

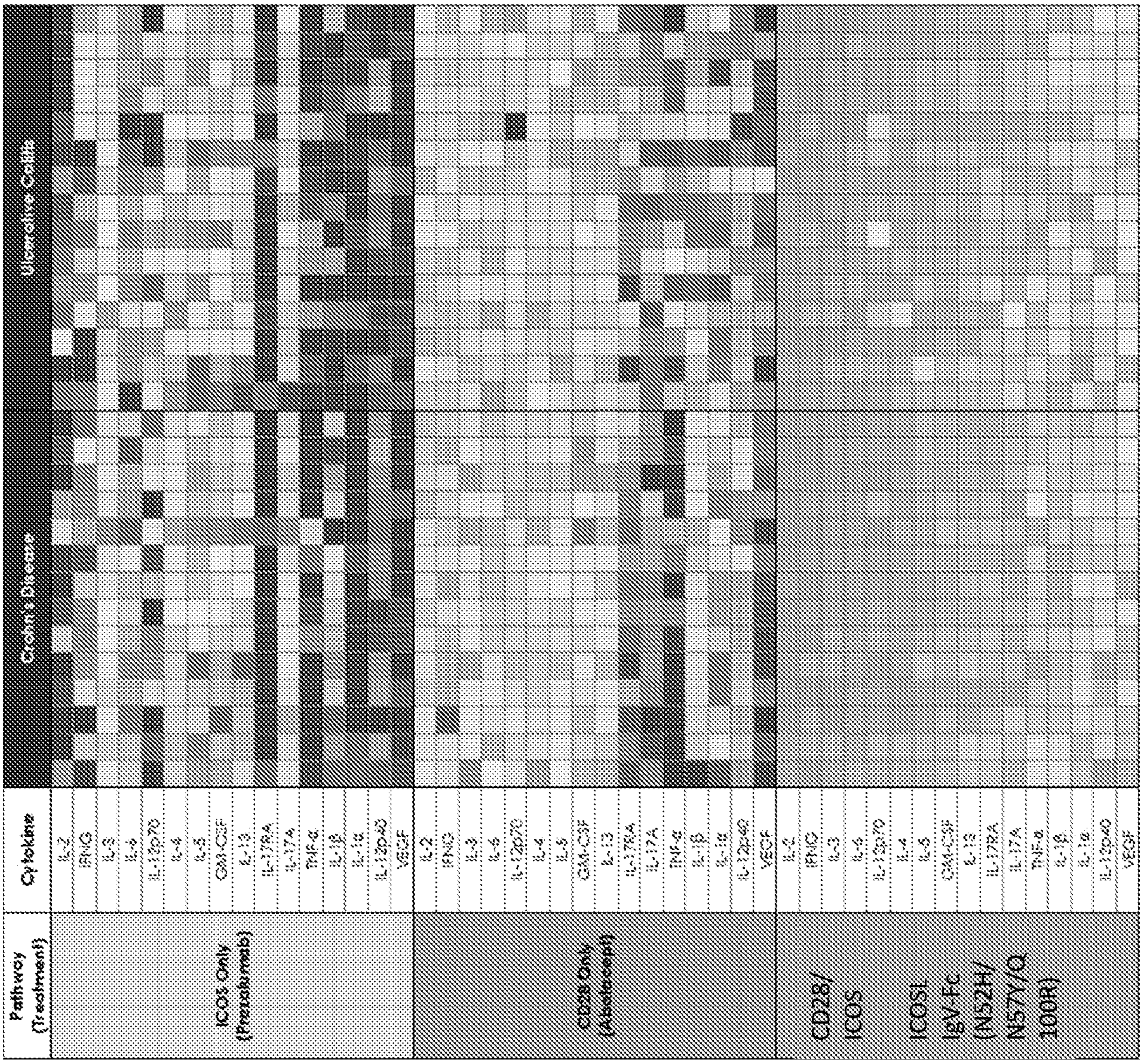
Pathway (Treatment)
Cytokine
Crohn's Disease
Ulcerative Colitis
ICOS Only (Prezalumab)
CD28 Only (Abatacept)
CD28/ ICOS
ICOSL IgV-Fc (N52H/ N57Y/Q 100R)
IL-2
IFNG
IL-3
IL-6
IL-12p70
IL-4
IL-5
GM-CSF
IL-13
IL-17RA
IL-17A
TNF-α
IL-1β
IL-1α
IL-12p40
VEGF

FIG. 27B

FIG. 28A

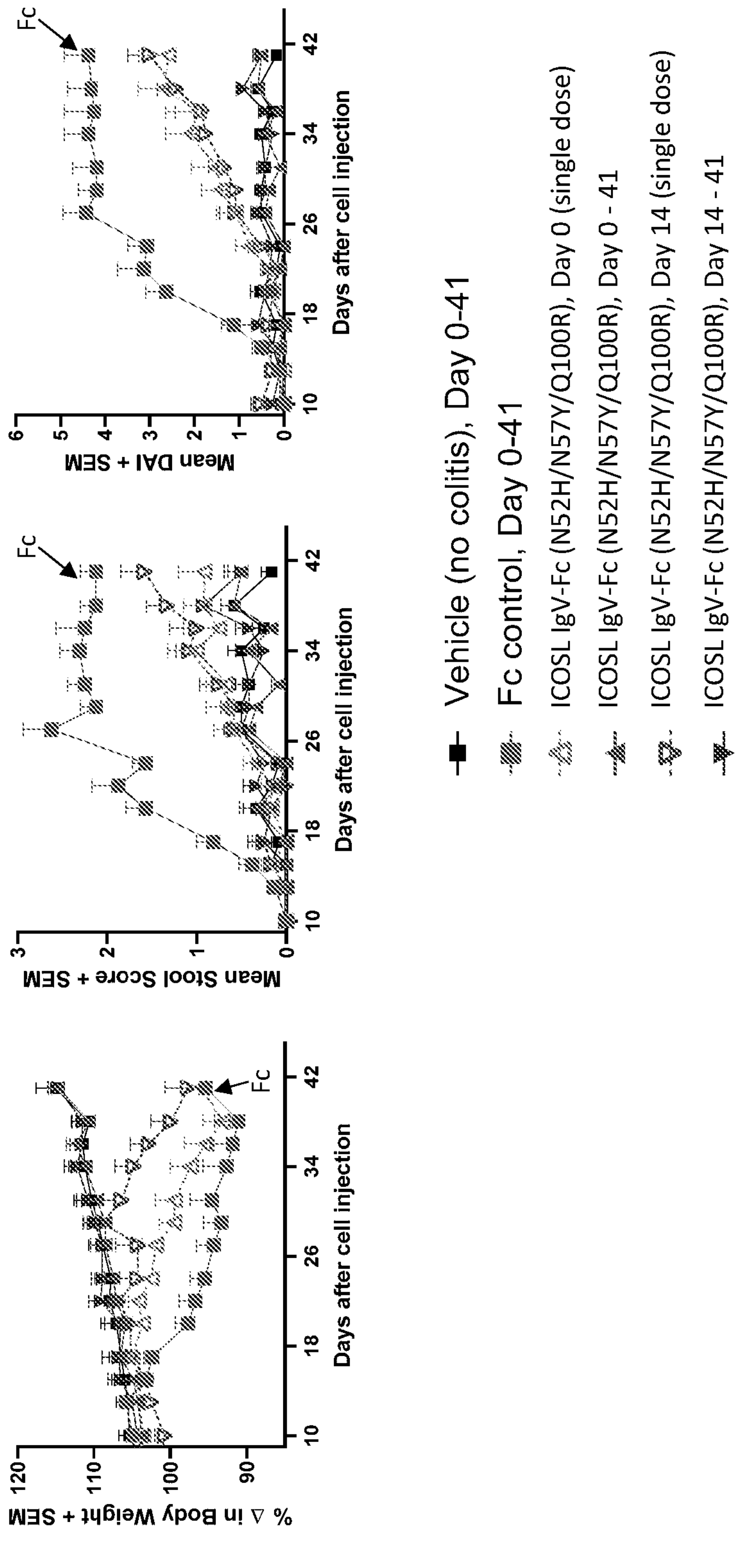
% Δ in Body Weight + SEM
Mean Stool Score + SEM
Mean DAI + SEM
Days after cell injection
Fc
Vehicle (no colitis), Day 0-41
Fc control, Day 0-41
ICOSL IgV-Fc (N52H/N57Y/Q100R), Day 0 (single dose)
ICOSL IgV-Fc (N52H/N57Y/Q100R), Day 0 - 41
ICOSL IgV-Fc (N52H/N57Y/Q100R), Day 14 (single dose)
ICOSL IgV-Fc (N52H/N57Y/Q100R), Day 14 - 41

FIG. 28B

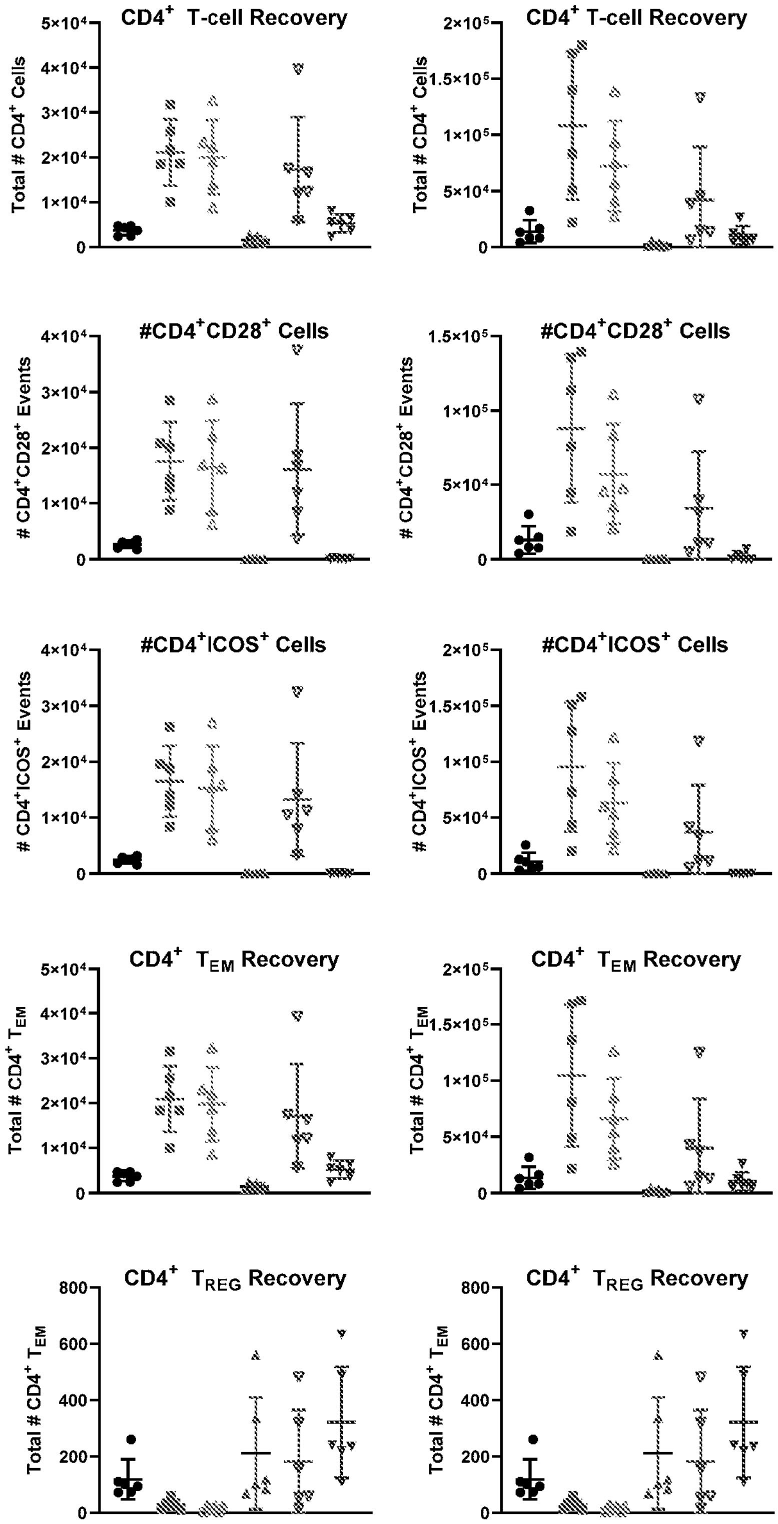
CD4+ T-cell Recovery
Total # CD4+ Cells
#CD4+CD28+ Cells
# CD4+CD28+ Events
#CD4+ICOS+ Cells
# CD4+ICOS+ Events
CD4+ TEM Recovery
Total # CD4+ TEM
CD4+ TREG Recovery

FIG. 28C

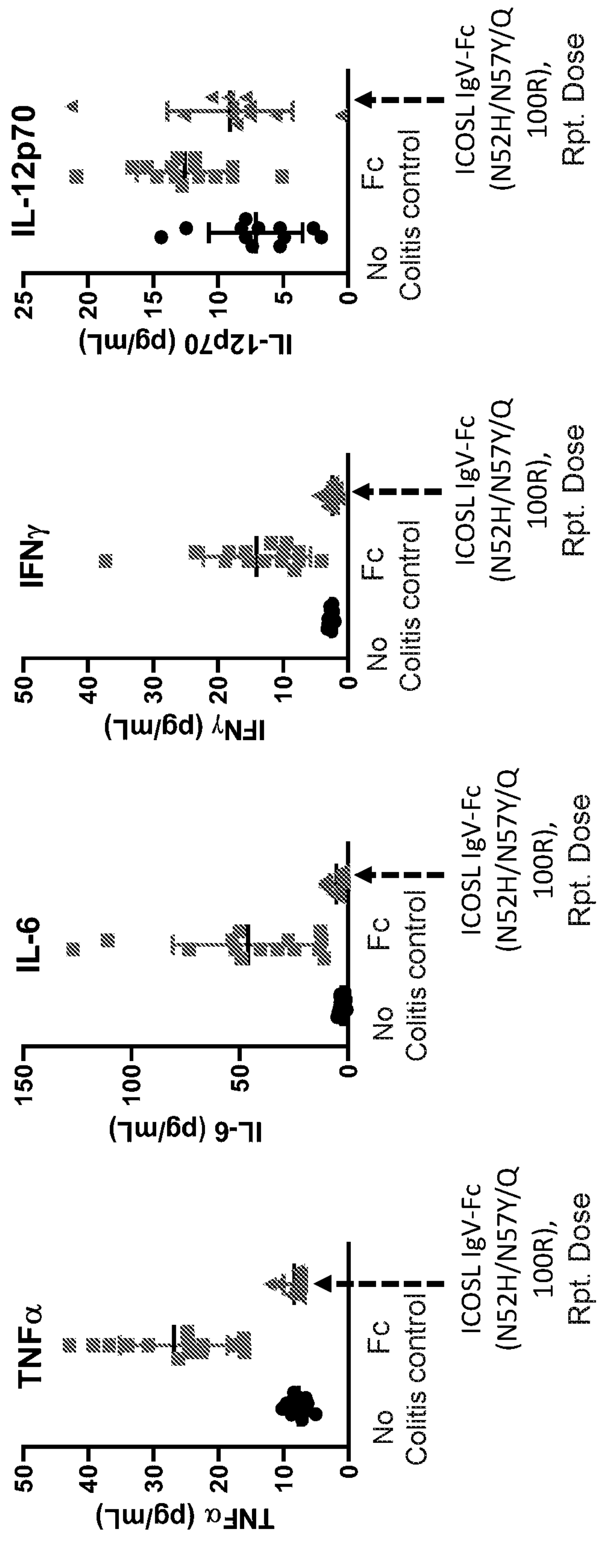
TNFα
TNFα (pg/mL)
IL-6
IL-6 (pg/mL)
IFNγ
IFNγ (pg/mL)
IL-12p70
IL-12p70 (pg/mL)
No Colitis control
Fc
ICOSL IgV-Fc (N52H/N57Y/Q100R), Rpt. Dose

FIG. 28D

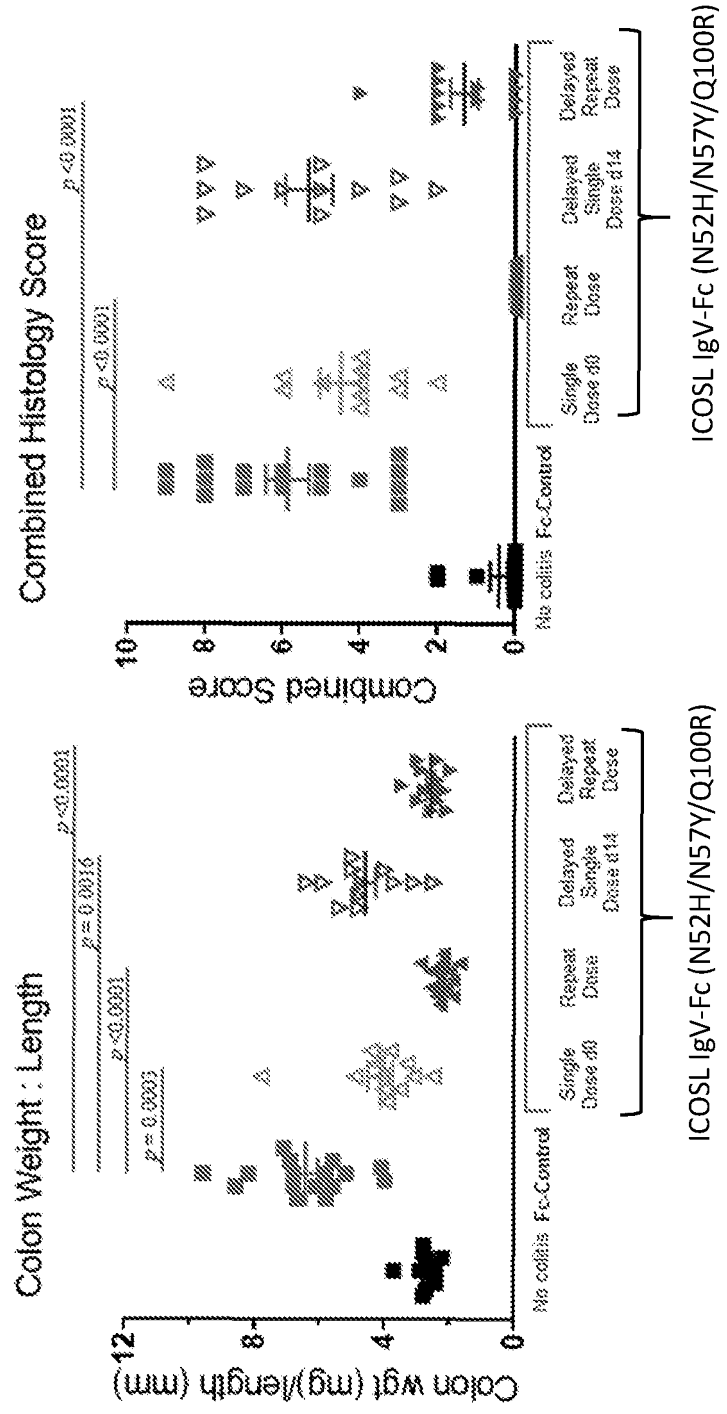
Colon Weight : Length
Colon wgt (mg)/length (mm)
12
8
4
0
p = 0.0003
p <0.0001
p = 0.0016
p <0.0001
No colitis
Fc-Control
Single Dose d0
Repeat Dose
Delayed Single Dose d14
Delayed Repeat Dose
ICOSL IgV-Fc (N52H/N57Y/Q100R)
Combined Histology Score
Combined Score
10
8
6
4
2
0
p <0.0001
p <0.0001
No colitis
Fc-Control
Single Dose d0
Repeat Dose
Delayed Single Dose d14
Delayed Repeat Dose
ICOSL IgV-Fc (N52H/N57Y/Q100R)

FIG. 28E

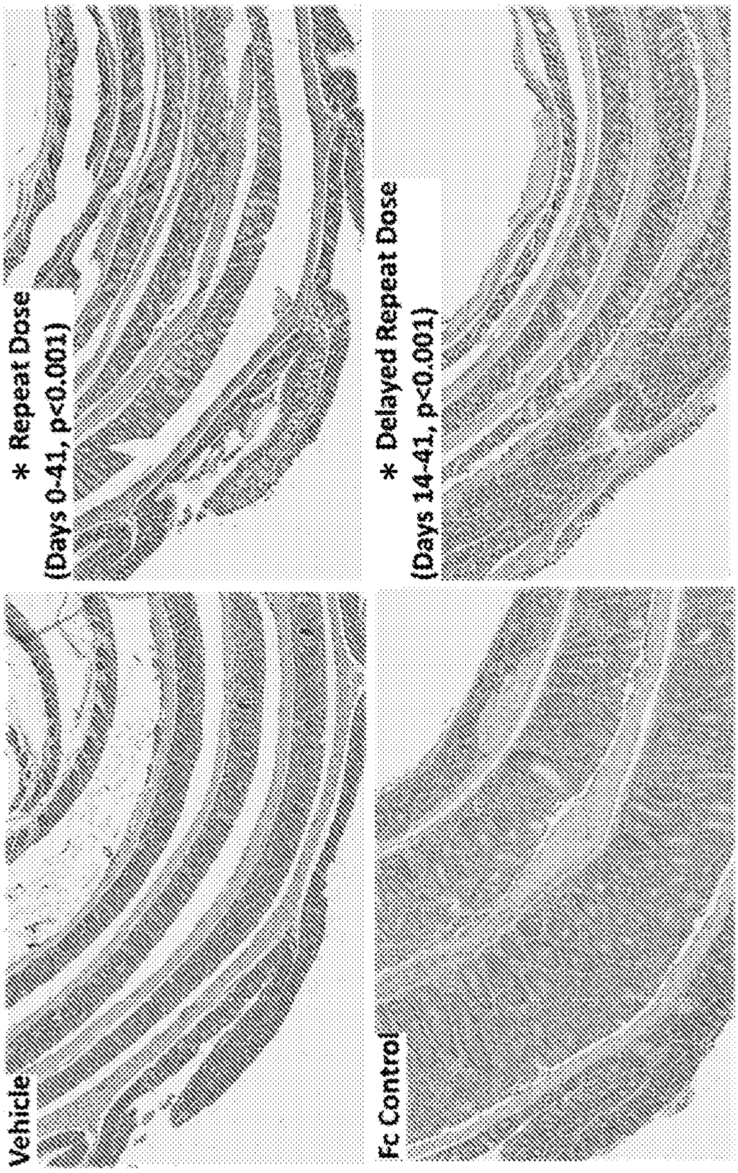


* ICOSL IgV-Fc
(N52H/N57Y/Q100R)

FIG. 28F

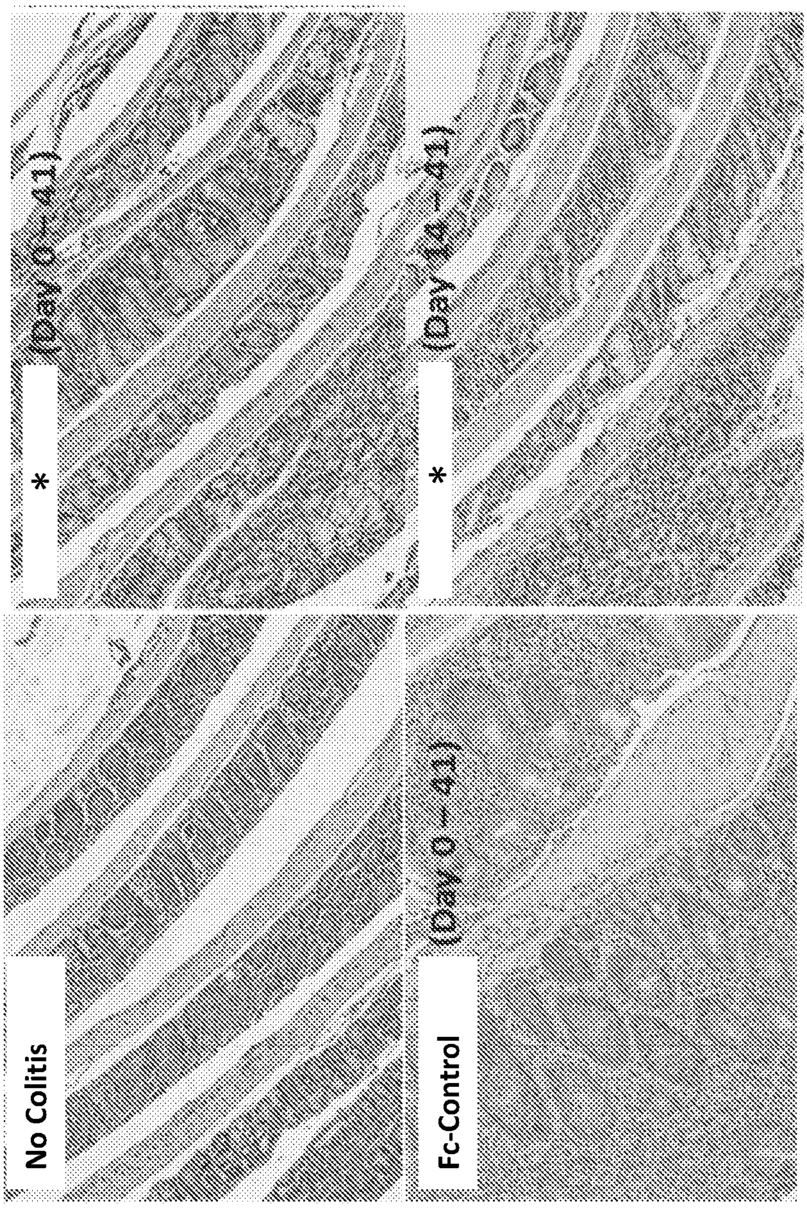


* ICOSL IgV-Fc
(N52H/N57Y/Q100R)

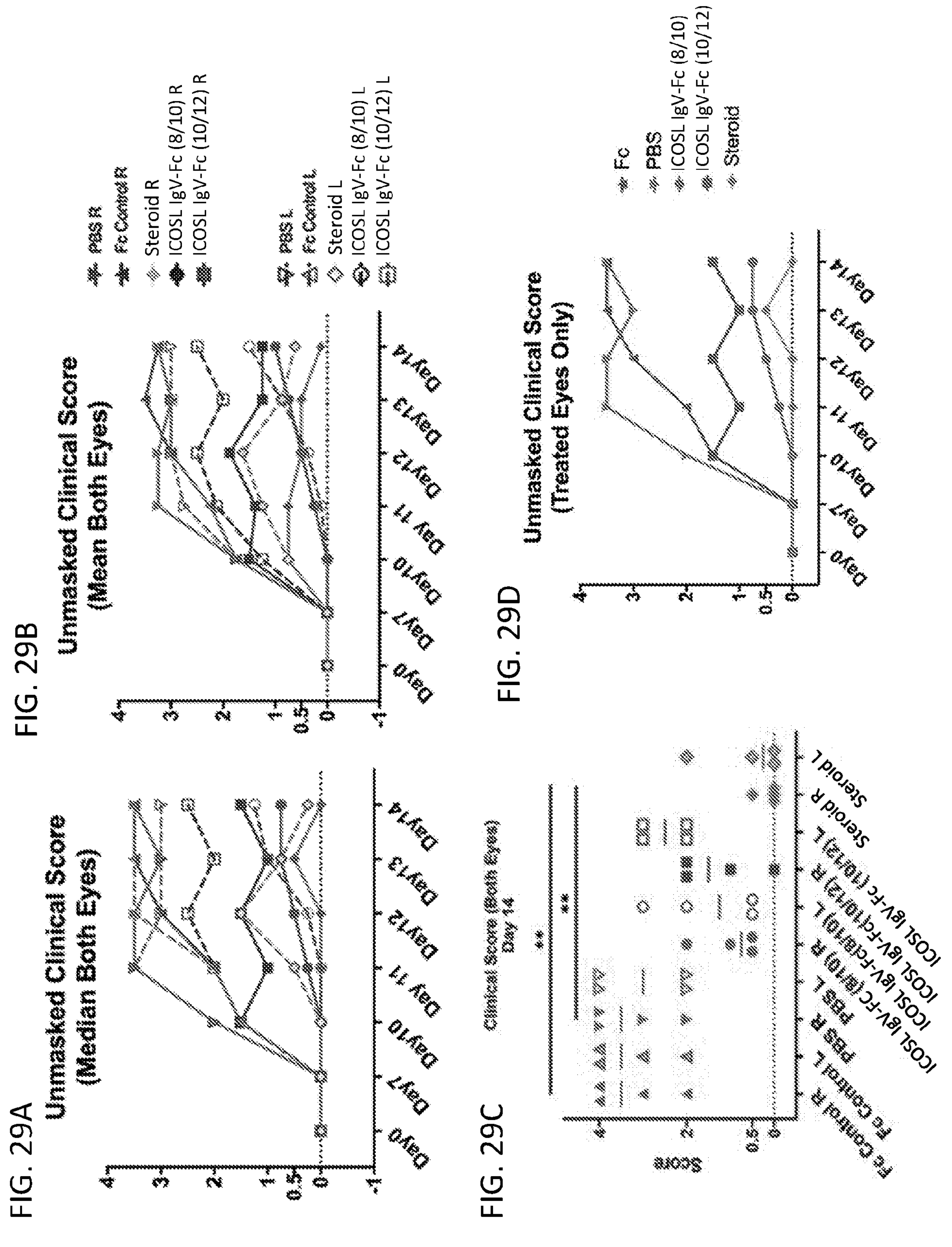


FIG. 29A

FIG. 29B

FIG. 29C

FIG. 29D

FIG. 29E

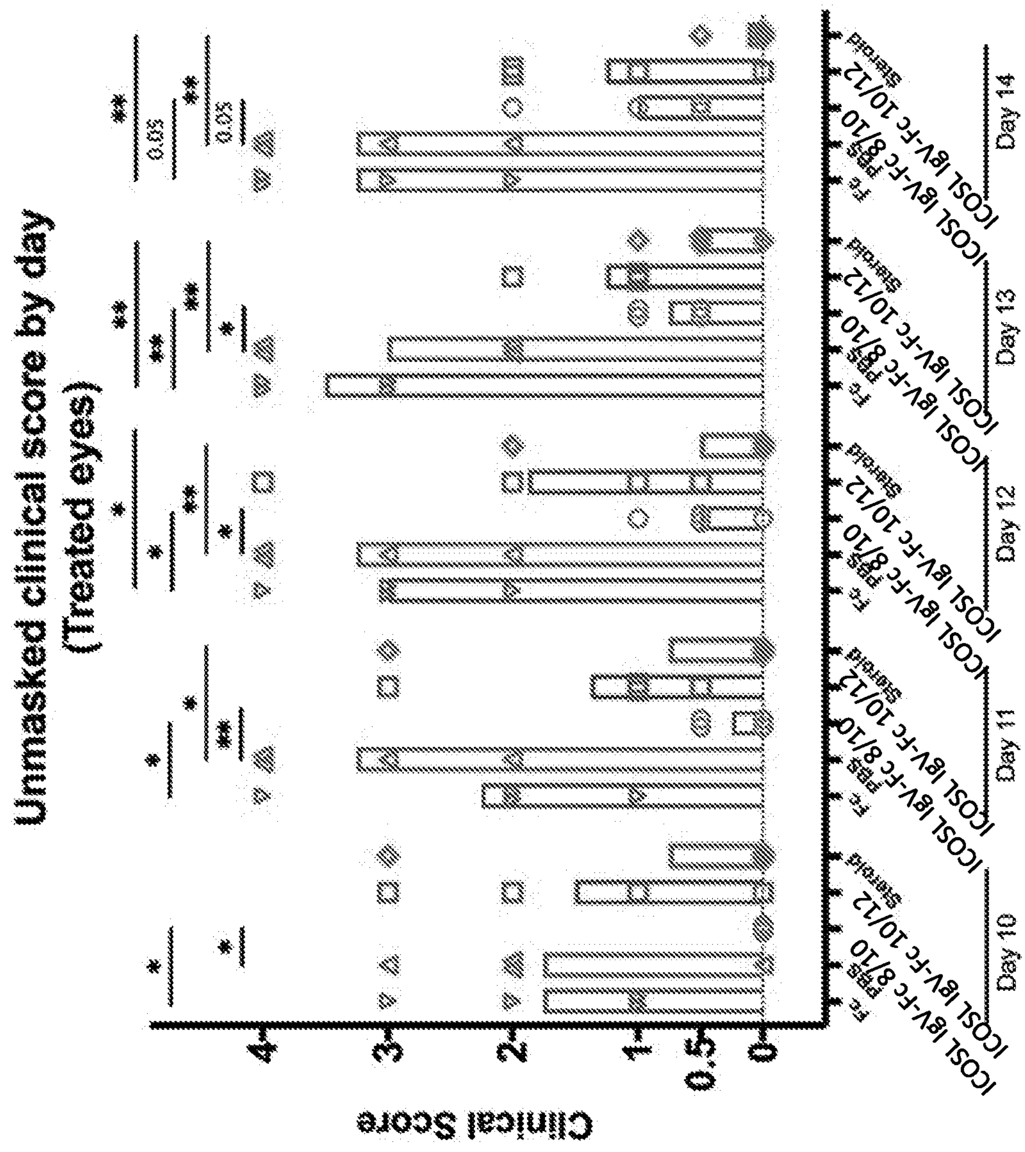

Unmasked clinical score by day
(Treated eyes)
Clinical Score
0
0.5
1
2
3
4
*
**
0.05
Fc
PBS
ICOSL IgV-Fc 8/10
ICOSL IgV-Fc 10/12
Steroid
Day 10
Day 11
Day 12
Day 13
Day 14

FIG. 30A

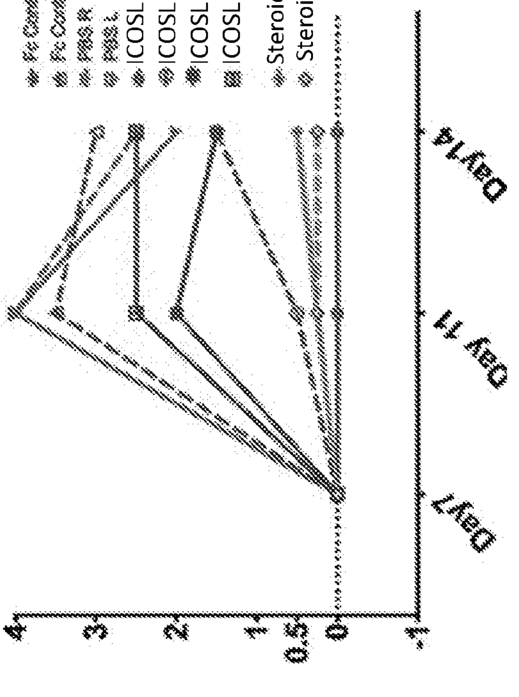


FIG. 30B

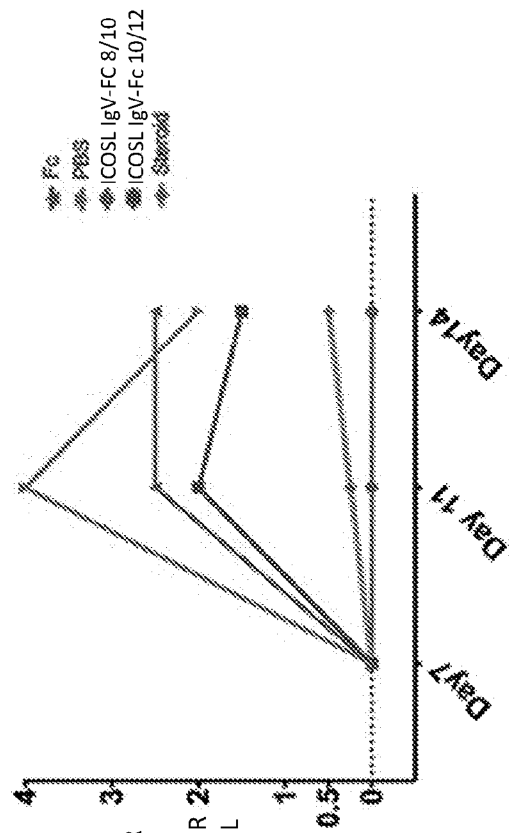


FIG. 30C

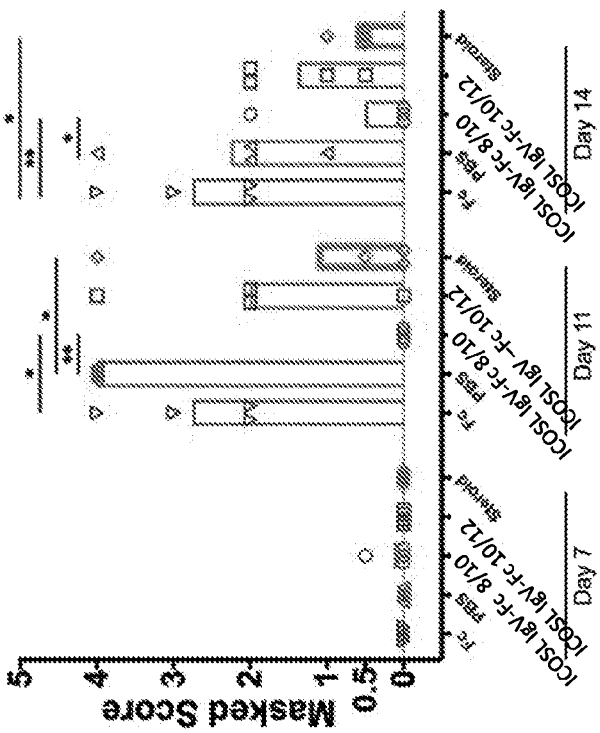

FIG. 31

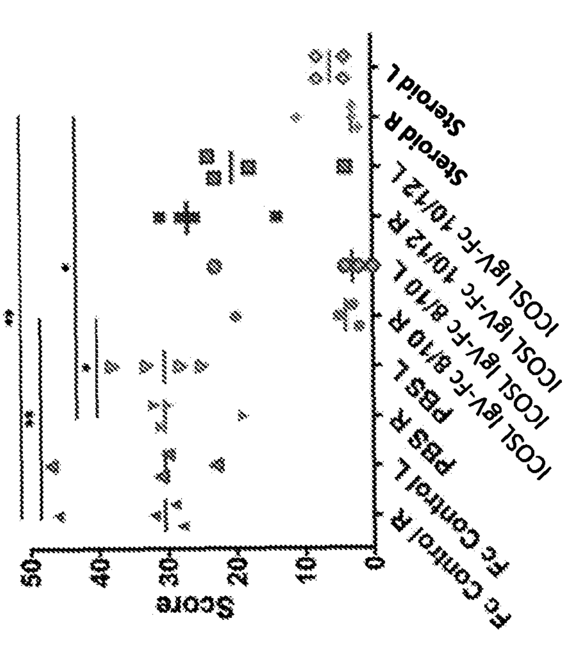


FIG. 32

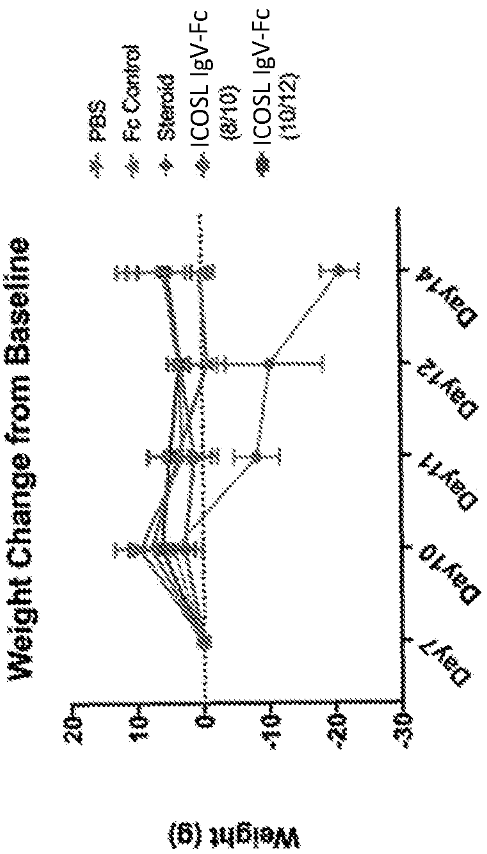


FIG. 33

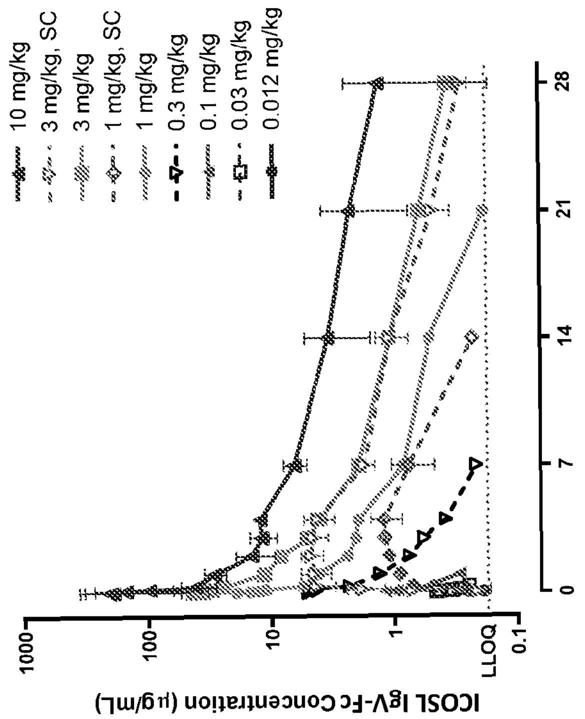

FIG. 34

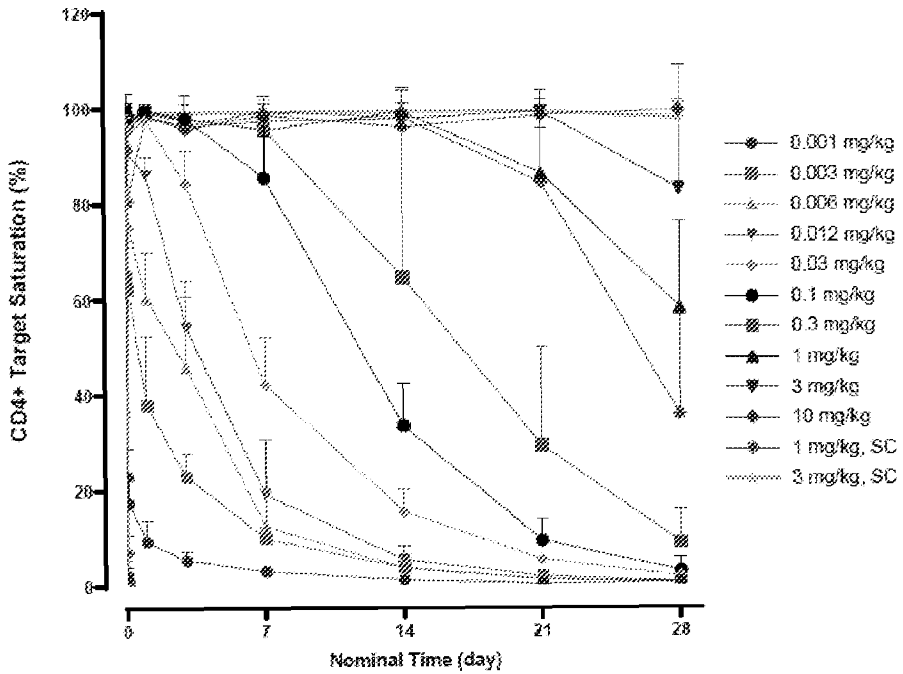
CD4+ Target Saturation (%)
120
100
80
60
40
20
0
0
7
14
21
28
Nominal Time (day)
0.001 mg/kg
0.003 mg/kg
0.006 mg/kg
0.012 mg/kg
0.03 mg/kg
0.1 mg/kg
0.3 mg/kg
1 mg/kg
3 mg/kg
10 mg/kg
1 mg/kg, SC
3 mg/kg, SC

FIG. 35

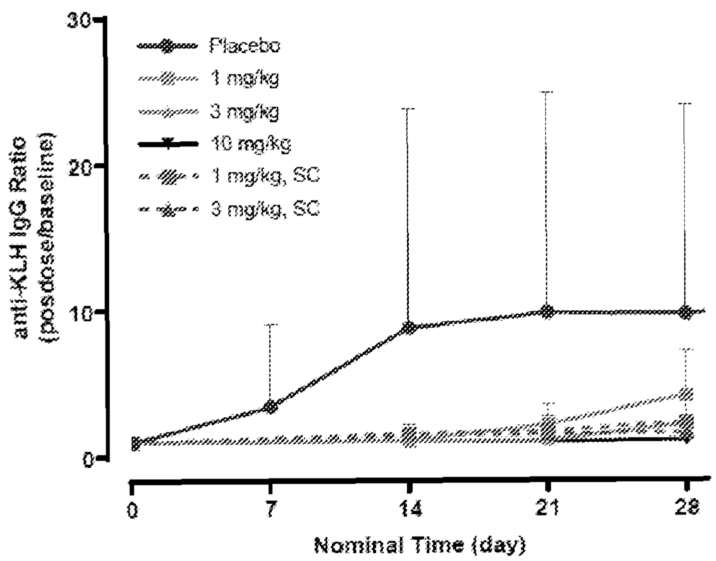
anti-KLH IgG Ratio (posdose/baseline)
30
20
10
0
0
7
14
21
28
Nominal Time (day)
Placebo
1 mg/kg
3 mg/kg
10 mg/kg
1 mg/kg, SC
3 mg/kg, SC

FIG. 36

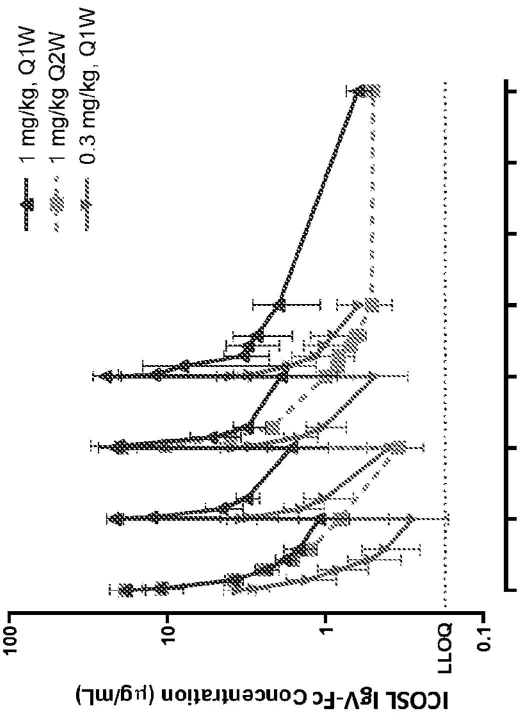
1 mg/kg, Q1W
1 mg/kg Q2W
0.3 mg/kg, Q1W
ICOSL IgV-Fc Concentration (µg/mL)
100
10
1
0.1
LLOQ
0
7
14
21
28
35
42
49
Time (day)

FIG. 37

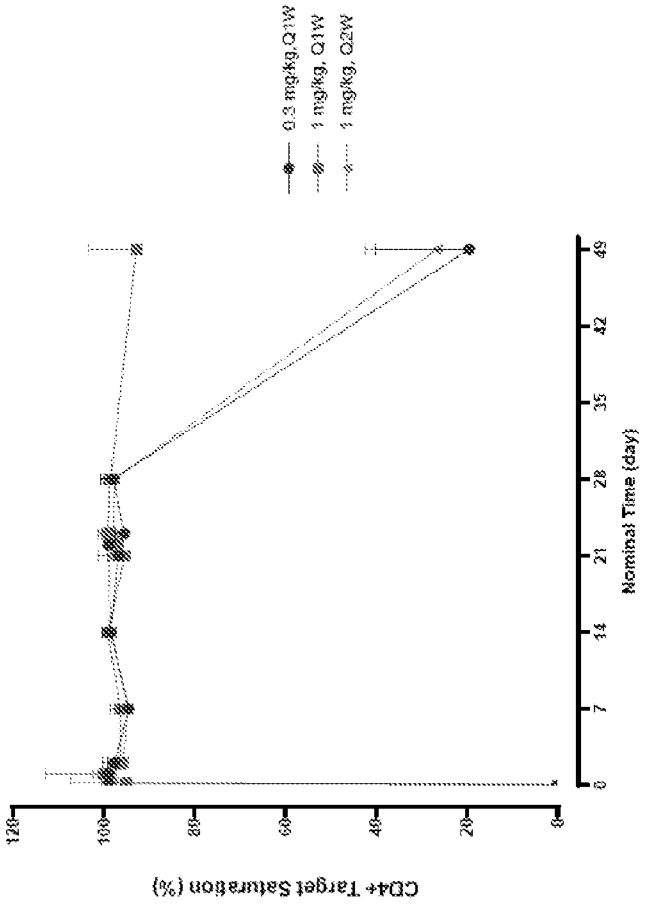
0.3 mg/kg,Q1W
1 mg/kg, Q1W
1 mg/kg, Q2W
CD4+ Target Saturation (%)
120
100
80
60
40
20
0
0
7
14
21
28
35
42
49
Nominal Time (day)

FIG. 38

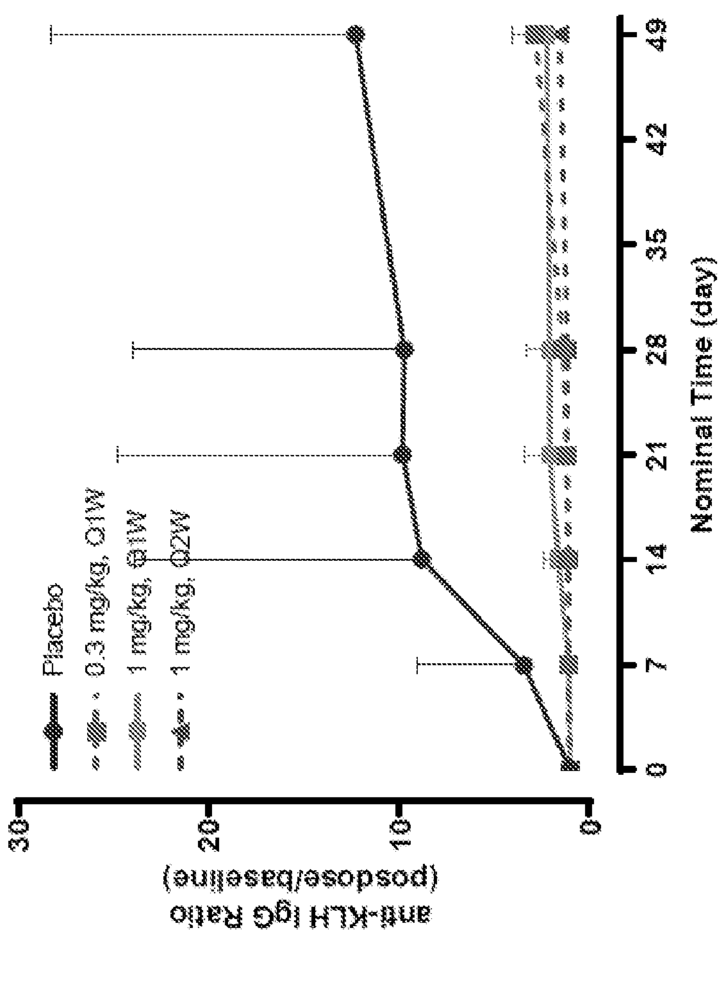
Placebo
0.3 mg/kg, Q1W
1 mg/kg, Q1W
1 mg/kg, Q2W
anti-KLH IgG Ratio (posdose/baseline)
30
20
10
0
0
7
14
21
28
35
42
49
Nominal Time (day)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62835488
- US 62855830
- US 62931212
- US 62945071
- US 62962832
- US 62987854
- WO 2013130683 A
- WO 9310151 A
- WO 2005063816 A
- US 20060024298 A
- US 5457035 A
- US 5731168 A
- WO 9850431 A
- WO 200042072 A
- WO 2006019447 A
- WO 2012125850 A
- WO 2015107026 A
- US 20160017041 A
- US 5500362 A
- US 5821337 A
- WO 2006029879 A
- WO 2005100402 A
- US 6737056 B
- US 7332581 B
- WO 2008092117 A
- WO 2000042072 A
- WO 2004056312 A
- US 20050014934 A1, Hinton
- US 7371826 B
- US 5648260 A
- US 5624821 A
- WO 9429351 A
- US 6194551 B
- WO 9951642 A
- US 8911726 B
- WO 0130843 A
- US 20020168714 A
- US 5698530 A
- US 6998252 B
- US 5443964 A
- US 7247615 B
- US 7368116 B
- US 6143290 A
- US 6596535 B
- US 6855317 B
- US 6936257 B
- US 7125717 B
- US 7378087 B
- US 7550296 B
- WO 2009076524 A
- WO 2012141984 A
- WO 2016011083 A
- US 5385839 A
- US 5168062 A
- US 5538848 A
- US 5925517 A
- US 6174670 B
- US 6329144 B
- US 6326145 B
- US 6635427 B
- US 7510687 B
- US 20160271218 A


### Non-patent literature cited in the description


- **YAO et al.** *Immunity,* 2011, vol. 34, 729-740
- **WEKERLE et al.** *Transpl. Int.,* 2012, vol. 25, 139-50
- **MASSON.** *Cochrane Database Sys. Rev.,* 2014
- **SINGH.** *Cochrane Database Sys. Rev.,* 2017
- **WIKENHEISER.** *Front. Immunol.,* 2016, vol. 7, 304
- **GARIN et al.** *Eur J Haematol,* 1996, vol. 56, 119-23
- **SCHMIDT et al.** *J. Clin. Invest.,* 1996, vol. 97, 2027-37
- **YANG et al.** *Chin J Microbiol Immunol,* 2005, vol. 25, 248-251
- **SCARSI et al.** *J. Rheumatol.,* 2011, vol. 38, 2105-11
- **MOU et al.** *Am J Transplant,* 2014, vol. 14, 2460-6
- **ZABINSKA et al.** *J Immunol Res.,* 2016, vol. 2016, 1058165
- **PIANTONI et al.** *Lupus,* 2018, vol. 27, 143-149
- **LARSEN et al.** *American Journal of Transplantation,* 2005, vol. 5, 443-453
- **LINSLEY et al.** *Immunity,* 1994, vol. 1, 793-801
- **WANG et al.** *Cancer Immunol Res.,* September 2014, vol. 2 (9), 846-56

- **KABAT, E.A. et al.** Sequences of Proteins of Immunological interest. US Department of Health and Human Services, 1991
- **LI ; KURLANDER.** *J Transl Med.,* 2010, vol. 8, 104
- **WU et al.** *Immunol Lett,* 15 April 2008, vol. 117 (1), 57-62
- **MILONE et al.** *Molecular Therapy,* 2009, vol. 17, 1453-1464
- **PEPER et al.** *Journal of Immunological Methods,* 2014, vol. 405, 192-198
- **ZHAO ; JI.** *Exp Cell Res.,* 01 January 2016, vol. 340 (1), 132-138
- **PEREZ DE LA LASTRA et al.** *Immunology,* April 1999, vol. 96 (4), 663-670
- **LINSLEY et al.** *Immunity,* 1994, vol. 1 (9), 793-801
- *Nucleic Acids Research,* January 2013, vol. 41 (D1), D36-42
- **BUSCH ; SASSONE-CORSI.** *Trends Genetics,* 1990, vol. 6, 36-40
- **GENTZ et al.** *Science,* 1989, vol. 243, 1695-1699
- **RIDGWAY et al.** *Protein Engineering,* 1996, vol. 9, 617-621
- **SHIELDS et al.** *J Biol. Chem.,* 2001, vol. 9 (2), 6591-6604
- **RAVETCH ; KINET.** *Annu. Rev. Immunol.,* 1991, vol. 9, 457-492
- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Nat'l Acad. Sci. USA,* 1998, vol. 95, 652-656
- **GAZZANO-SANTORO et al.** *J. Immunol. Methods,* 1996, vol. 202, 163
- **CRAGG, M. S. et al.** *Blood,* 2003, vol. 101, 1045-1052
- **CRAGG, M. S. ; M. J. GLENNIE.** *Blood,* 2004, vol. 103, 2738-2743
- **PETKOVA, S. B. et al.** *Int'l. Immunol.,* 2006, vol. 18 (12), 1759-1769
- *Current Opinion in Biotechnology,* 2009, vol. 20 (6), 685-691
- **SHIELDS et al.** *J. Biol. Chem.,* 2001, vol. 9 (2), 6591-6604
- **DUNCAN ; WINTER.** *Nature,* 1988, vol. 322, 738-40
- **IDUSOGIE et al.** *J. Immunol.,* 2000, vol. 164, 4178-4184
- **LABRIJIN et al.** *Nat. Biotechnol.,* 2009, vol. 27 (8), 767-71
- **GUERRA et al.** *J. Virol.,* 2006, vol. 80, 985-98
- **TARTAGLIA et al.** *AIDS Research and Human Retroviruses,* 1992, vol. 8, 1445-47
- **GHERADI et al.** *J. Gen. Virol.,* 2005, vol. 86, 2925-36
- **MAYR et al.** *Infection,* 1975, vol. 3, 6-14
- **HU et al.** *J. Virol.,* 2001, vol. 75, 10300-308
- **MOLIN et al.** *J. Virol.,* 1998, vol. 72, 8358-61
- **NARUMI et al.** *Am J. Respir. Cell Mol. Biol.,* 1998, vol. 19, 936-41
- **MERCIER et al.** *Proc. Natl. Acad. Sci. USA,* 2004, vol. 101, 6188-93
- **BUCHSCHER et al.** *J. Virol.,* 1992, vol. 66, 2731-39
- **JOHANN et al.** *J. Virol.,* 1992, vol. 66, 1635-40
- **SOMMERFELT et al.** *Virology,* 1990, vol. 176, 58-59
- **WILSON et al.** *J. Virol.,* 1989, vol. 63, 2374-78
- **MILLER et al.** *J. Virol.,* 1991, vol. 65, 2220-24
- **MILLER et al.** *Mol. Cell Biol.,* 1990, vol. 10, 4239
- **KOLBERG.** *NIH Res.,* 1992, vol. 4, 43
- **COMETTA et al.** *Hum. Gene Ther.,* 1991, vol. 2, 215
- **PFEIFER et al.** *Annu. Rev. Genomics Hum. Genet.,* 2001, vol. 2, 177-211
- **ZUFFEREY et al.** *J. Virol.,* 1998, vol. 72, 9873
- **MIYOSHI et al.** *J. Virol.,* 1998, vol. 72, 8150
- **PHILPOTT ; THRASHER.** *Human Gene Therapy,* 2007, vol. 18, 483
- **ENGELMAN et al.** *J. Virol.,* 1995, vol. 69, 2729
- **NIGHTINGALE et al.** *Mol. Therapy,* 2006, vol. 13, 1121
- **BROWN et al.** *J. Virol.,* 1999, vol. 73, 9011
- **MCWILLIAMS et al.** *J. Virol.,* 2003, vol. 77, 11150
- **POWELL et al.** *J. Virol.,* 1996, vol. 70, 5288
- **ADORN et al.** *Blood,* 2018, 132-355
- **SHENDURE et al.** *Nat. Rev. Genet.,* 2004, vol. 5, 335-44
- **NOWROUSIAN.** *Euk. Cell,* 2010, vol. 9 (9), 1300-1310
- **MILLER, K.D.** *Current Protocols in Cytometry,* July 2008, 4.7.1-4.7.30
- **BIASINI et al.** *Nucleic Acids Res.,* 2014, vol. 42, W252-8
- **PEACH et al.** *J Exp Med,* 1994, vol. 180, 2049-58
- **CHATTOPADHYAY et al.** *J Immunol,* 2006, vol. 177, 3920-9